# EUROPÄISCHE PATENTSCHRIFT

(11) **EP 2 885 286 B1**
(45) Veröffentlichungstag und Bekanntmachung des Hinweises auf die Patenterteilung: **22.03.2017**
(21) Anmeldenummer: 13748072.9
(22) Anmeldetag: 13.08.2013
(51) Int. Cl.: C07D 401/14, C07D 413/14, C07D 401/04, C07D 401/10, C07D 401/12, C07D 403/10, C07D 403/14, C07D 409/10, C07D 413/04, C07D 413/10, C07D 417/10, C07D 243/02, A61K 31/55, A61P 35/00, A61P 31/12

(54) **2,3-BENZODIAZEPINE**
2,3-BENZODIAZEPINES
2,3-BENZODIAZÉPINES

(30) Priorität: 16.08.2012 DE 102012214602; 08.02.2013 DE 102013202104
(43) Veröffentlichungstag der Anmeldung: 24.06.2015
(73) Patentinhaber: Bayer Pharma Aktiengesellschaft, 13353 Berlin (DE)
(72) Erfinder: SIEGEL, Stephan, 10779 Berlin (DE); BÄURLE, Stefan, 10245 Berlin (DE); CLEVE, Arwed, 10707 Berlin (DE); HAENDLER, Bernard, 13465 Berlin (DE); FERNANDEZ-MONTALVAN, Amaury Ernesto, 10437 Berlin (DE); MÖNNING, Ursula, 15569 Woltersdorf (DE); KRAUSE, Sabine, 13158 Berlin (DE); LEJEUNE, Pascale, 13469 Berlin (DE); SCHMEES, Norbert, 13469 Berlin (DE); BUSEMANN, Matthias, 13467 Berlin (DE); HOLTON, Simon, 12159 Berlin (DE); KUHNKE, Joachim, 14482 Potsdam (DE)
(74) Vertreter: BIP Patents
(86) Internationale Anmeldenummer: PCT/EP2013/066931
(87) Internationale Veröffentlichungsnummer: WO 2014/026997

(56) Entgegenhaltungen:
- WO-A1-02/50044
- WO-A1-2007/077469
- WO-A1-2008/124075
- WO-A2-2011/143669
- CHUNG CHUN-WA ET AL: "Discovery and characterization of small molecule inhibitors of the BET family bromodomains", JOURNAL OF MEDICINAL CHEMISTRY , Bd. 54, Nr. 11 13. Mai 2011 (2011-05-13), Seiten 3827-3838, XP008164769, ISSN: 0022-2623, DOI: 10.1021/JM200108T Gefunden im Internet: URL:http://pubs.acs.org/doi/pdf/10.1021/jm 200108t [gefunden am 2013-09-11] in der Anmeldung erwähnt

## Beschreibung

Die vorliegende Erfindung betrifft BET-proteininhibitorische, insbesondere BRD4-inhibitorische substituierte Phenyl-2,3-Benzodiazepine, pharmazeutische Mittel enthaltend die erfindungsgemäßen Verbindungen sowie die erfindungsgemäßen Verbindungen zur prophylaktischen und therapeutischen Verwendung bei hyperproliferativen Erkrankungen, insbesondere bei Tumorerkrankungen. Desweiteren betrifft diese Erfindung BET-Proteininhibitoren zur Verwendung in benignen Hyperplasien, atherosklerotische Erkrankungen, Sepsis, Autoimmunerkrankungen, Gefäßerkrankungen, viralen Infektionen, in neurodegenerativen Erkrankungen, in inflammatorischen Erkrankungen, in atherosklerotischen Erkrankungen und in der männlichen Fertilitätskontrolle.

Die humane BET-Familie (bromodomain and extra C-terminal domain family) hat vier Mitglieder (BRD2, BRD3, BRD4 und BRDT), die zwei verwandte Bromodomänen und eine extraterminale Domäne enthalten (Wu und Chiang, J. Biol. Chem., 2007, 282:13141-13145). Die Bromodomänen sind Proteinregionen, die acetylierte Lysinreste erkennen. Solche acetylierten Lysine findet man oft am N-terminalen Ende von Histonen (z. B. Histon 3 oder Histon 4) und sie sind Merkmale für eine offene Chromatin-Struktur und aktive Gentranskription (Kuo und Allis, Bioessays, 1998, 20:615-626). Die verschiedenen Acetylierungsmuster, die durch BET Proteine in Histonen erkannt werden, wurden genau untersucht (Umehara et al., J. Biol. Chem., 2010, 285:7610-7618; Filippakopoulos et al., Cell, 2012, 149:214-231). Zusätzlich können Bromodomänen weitere acetylierte Proteine erkennen. Zum Beispiel bindet BRD4 an RelA, was zur Stimulierung von NF-κB und transkriptioneller Aktivität von inflammatorischen Genen führt (Huang et al., Mol. Cell. Biol., 2009, 29:1375-1387; Zhang et al., J. Biol. Chem., 2012, 287: 28840-28851; Zou et al., Oncogene, 2013, doi:10.1038/onc.2013.179). Die extraterminale Domäne von BRD2, BRD3 und BRD4 interagiert mit mehreren Proteinen, die eine Rolle in der Chromatinmodulierung und der Regulation der Genexpression haben (Rahman et al., Mol. Cell. Biol., 2011, 31:2641-2652). Mechanistisch spielen BET-Proteine eine wichtige Rolle im Zellwachstum und im Zellcyclus. Sie sind mit mitotischen Chromosomen assoziiert, was eine Funktion im epigenetischen Gedächtnis nahelegt (Dey et al., Mol. Biol. Cell, 2009, 20:4899-4909; Yang et al., Mol. Cell. Biol., 2008, 28:967-976). BRD4 ist für die post-mitotische Reaktivierung von Gentranskription wichtig (Zhao et al., Nat. Cell. Biol., 2011, 13:1295-1304). Es wurde gezeigt, dass BRD4 essentiell ist für die Transkriptionselongation und für die Rekrutierung des Elongationskomplexes P-TEFb, der aus CDK9 und Cyclin T1 besteht, was zur Aktivierung der RNA Polymerase II führt (Yang et al., Mol. Cell, 2005, 19:535-545; Schröder et al., J. Biol. Chem., 2012, 287:1090-1099). Folglich wird die Expression von Genen stimuliert, die in der Zellproliferation involviert sind, wie zum Beispiel c-Myc und Aurora B (You et al., Mol. Cell. Biol., 2009, 29:5094-5103; Zuber et al., Nature, 2011, 478:524-528). BRD2 und BRD3 binden an transkribierte Gene in hyperacetylierten Chromatinbereichen und fördern die Transkription durch RNA Polymerase II (LeRoy et al., Mol. Cell, 2008, 30:51-60).

Der Knock-down von BRD4 bzw. die Hemmung der Interaktion mit acetylierten Histonen in verschiedenen Zelllinien führen zu einem G1-Arrest und zum Zelltod durch Apoptose (Mochizuki et al., J. Biol. Chem., 2008, 283:9040-9048; Mertz et al., Proc. Natl. Acad. Sci. USA, 2011, 108:16669-16674). Es wurde auch gezeigt, dass BRD4 an Promotorregionen von mehreren Genen, die in der G1-Phase aktiviert werden, wie zum Beispiel Cyclin D1 und D2, bindet (Mochizuki et al., J. Biol. Chem., 2008, 283:9040-9048). Zusätzlich wurde eine Hemmung der Expression von c-Myc, ein essentieller Faktor in der Zellproliferation, nach BRD4-Inhibition nachgewiesen (Dawson et al., Nature, 2011, 478:529-533; Delmore et al., Cell, 2011, 146:1-14; Mertz et al., Proc. Natl. Acad. Sci. USA, 2011, 108:16669-16674).

BRD2 und BRD4 Knockout-Mäuse sterben früh während der Embryogenese (Gyuris et al., Biochim. Biophys. Acta, 2009, 1789:413-421; Houzelstein et al., Mol. Cell. Biol., 2002, 22:3794-3802). Heterozygote BRD4 Mäuse haben verschiedene Wachstumsdefekte, die auf eine reduzierte Zellproliferation zurückzuführen sind (Houzelstein et al., Mol. Cell. Biol., 2002, 22:3794-3802). BET-Proteine spielen eine wichtige Rolle in verschiedenen Tumorarten. Die Fusion zwischen den BET-Proteinen BRD3 oder BRD4 und NUT, einem Protein, das normalerweise nur im Hoden exprimiert wird, führt zu einer aggressiven Form des Plattenepithelkarzinoms, genannt NUT midline carcinoma (French, Cancer Genet. Cytogenet., 2010, 203:16-20). Das Fusionsprotein verhindert Zelldifferenzierung und fördert Proliferation (Yan et al., J. Biol. Chem., 2011, 286:27663-27675; Grayson et al., 2013, doi:10-1038/onc.2013.126). Das Wachstum von davon abgeleiteten in vivo Modellen wird durch einen BRD4-Inhibitor gehemmt (Filippakopoulos et al., Nature, 2010, 468:1067-1073). Ein Screening für therapeutische Targets in einer akuten myeloiden Leukämiezelllinie (AML) zeigte, dass BRD4 eine wichtige Rolle in diesem Tumor spielt (Zuber et al., Nature, 2011, doi:10.1038). Die Reduktion der BRD4-Expression führt zu einem selektiven Arrest des Zellcyclus und zur Apoptose. Die Behandlung mit einem BRD4-Hemmer verhindert die Proliferation eines AML-Xenografts in vivo. Eine Amplifizierung der DNA-Region die das BRD4-Gen enthält wurde in primären Brusttumoren nachgewiesen (Kadota et al., Cancer Res, 2009, 69:7357-7365). Auch für BRD2 gibt es Daten bezüglich einer Rolle in Tumoren. Eine transgene Maus, die BRD2 selektiv in B-Zellen hochexprimiert, entwickelt B-Zell Lymphome und Leukämien (Greenwall et al., Blood, 2005, 103:1475-1484).

BET-Proteine sind auch an viralen Infektionen beteiligt. BRD4 bindet an das E2 Protein von verschiedenen Papillomaviren und ist wichtig für das Überleben der Viren in latent infizierten Zellen (Wu et al., Genes Dev., 2006, 20:2383-2396; Vosa et al., J. Virol., 2012, 86:348-357; McBride und Jang, Viruses, 2013, 5:1374-1394). Auch das Herpesvirus, das für das Kaposi-Sarkom verantwortlich ist, interagiert mit verschiedenen BET-Proteinen, was für die Krankheitsbeständigkeit wichtig ist (Viejo-Borbolla et al., J. Virol., 2005, 79:13618-13629; You et al., J. Virol., 2006, 80:8909-8919). Durch Bindung an P-TEFb spielt BRD4 auch eine wichtige Rolle in der Replikation von HIV (Bisgrove et al., Proc. Natl Acad. Sci. USA, 2007, 104:13690-13695).

BET-Proteine sind zusätzlich an Inflammationsprozessen beteiligt. BRD2-hypomorphe Mäuse zeigen eine reduzierte Inflammation im Fettgewebe (Wang et al., Biochem. J., 2009, 425:71-83). Auch die Infiltration von Makrophagen in weißem Fettgewebe ist in BRD2-defizienten Mäusen reduziert (Wang et al., Biochem. J., 2009, 425:71-83). Es wurde auch gezeigt, dass BRD4 eine Reihe von Genen reguliert, die in der Inflammation involviert sind. In LPS-stimulierten Makrophagen verhindert ein BRD4-Inhibitor die Expression von inflammatorischen Genen, wie zum Beispiel IL-1 oder IL-6 (Nicodeme et al., Nature, 2010, 468:1119-1123).

BET-Proteine regulieren auch die Expression des ApoA1-Gens, das eine wichtige Rolle in Atherosklerose und in inflammatorischen Prozessen spielt (Chung et al., J. Med. Chem, 2011, 54:3827-3838). Apolipoprotein A1 (ApoA1) ist ein Hauptbestandteil von High Density Lipoproteine (HDL) und erhöhte Expression von ApoA1 führt zu erhöhten Blut-Cholesterin Werten (Degoma und Rader, Nat. Rev. Cardiol., 2011, 8:266-277). Erhöhte HDL-Werte sind mit einem erniedrigten Risiko für Atherosklerose verbunden (Chapman et al., Eur. Heart J., 2011, 32:1345-1361).

### Stand der Technik

Die bei der Betrachtung des strukturellen Standes der Technik angewendete Nomenklatur wird durch die nachfolgende Abbildung verdeutlicht:

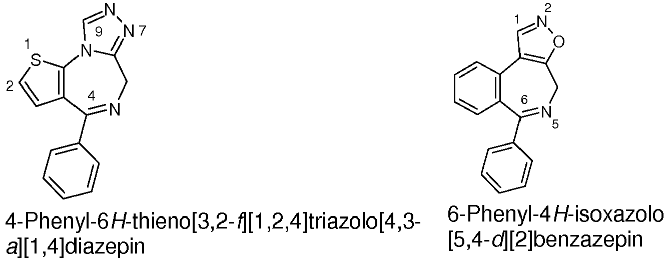

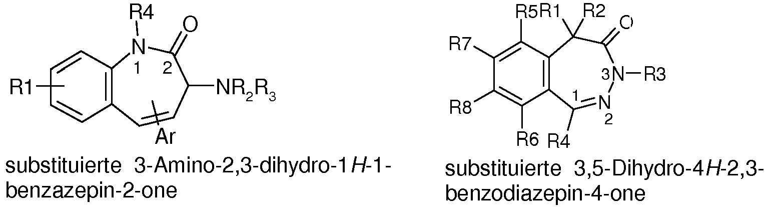

Bezogen auf die chemische Struktur wurden bisher nur sehr wenige Typen von BRD4-Inhibitoren beschrieben (Chun-Wa Chung et al., Progress in Medicinal Chemistry 2012, 51, 1-55).

Die ersten publizierten BRD4-Inhibitoren sind Phenyl-thieno-triazolo-1,4-diazepine (4-Phenyl-6*H-*thieno[3,2-*f*] [1,2,4]triazolo[4,3-*a*] [1,4]diazepine) wie in WO2009/084693 (Mitsubishi Tanabe Pharma Corporation) und mit der Verbindung JQ1 in WO2011/143669 (Dana Farber Cancer Institute) beschrieben. Der Ersatz der Thieno- durch eine Benzo-Einheit führt auch zu aktiven Inhibitoren (J. Med. Chem. 2011, 54, 3827 - 3838; E. Nicodeme et al., Nature 2010, 468, 1119). Diese und eine weitere Publikation zeigen auf, dass die an das 1,4-Benzodiazepin-oder Thieno-1,4-diazepin-Ringsystem ankondensierte Pyrazol-Einheit aktiv an der Bindung des Targetproteins BRD4, beteiligt ist (P. Filippakopoulos et al., Nature 2010, 468, 1067). Weitere 4-Phenyl-6*H-*thieno[3,2-*f*] [1,2,4]triazolo[4,3-*a*][1,4]diazepine und verwandte Verbindungen mit alternativen Ringen als Fusionspartner anstelle der Benzo-Einheit werden generisch adressiert oder direkt beschrieben in WO2012/075456 (Constellation Pharmaceuticals). WO2012/075383 (Constellation Pharmaceuticals) beschreibt 6-substituierte 4*H*-isoxazolo[5,4-*d*] [2]benzazepine und 4*H-*Isoxazolo[3,4-*d*][2]benzazepine einschließlich von Verbindungen, die an Position 6 optional substituiertes Phenyl aufweisen, als BRD4-Inhibitoren und auch Analoga mit alternativen heterozyklischen Fusionspartnern anstelle der Benzo-Einheit, z.B. Thieno- oder Pyridoazepine. Als eine andere strukturelle Klasse von BRD4 Inhibitoren werden 7-Isoxazolochinoline und verwandte Chinolon-Derivative (WO2011/054843, Bioorganic & Medicinal Chemistry Letters 22 (2012) 2963-2967, GlaxoSmithKline) beschrieben.

In WO94/26718 bzw. EP0703222A1 (Yoshitomi Pharmaceutical Industries) werden substituierte 3-Amino-2,3-dihydro-1*H*-1-benzazepin-2-one oder die entsprechenden 2-Thione und Analoga, in denen die Benzo-Einheit durch alternative monocyclische Systeme ersetzt ist und in denen das 2-Keton oder das 2-Thion zusammen mit dem substituierten Stickstoffatom des Azepinringes einen Heterozyklus bilden kann, als CCK- und Gastrin-Antagonisten für die Therapie von Erkrankungen des ZNS, wie Angstzustände und Depressionen, sowie von Erkrankungen der Bauchspeicheldrüse und von gastrointestinalen Geschwüren, beschrieben. Liganden des Gastrin- und des Cholecystokinin Rezeptors sind in WO2006/051312 (James Black Foundation) beschrieben. Sie umfassen auch substituierte 3,5-Dihydro-4*H*-2,3-benzodiazepin-4-one, die sich von den erfindungsgemässen Verbindungen hauptsächlich durch die obligatorische Oxogruppe in Position 4 und durch eine obligatorische, eine Carbonylgruppe enthaltende Alkylkette in Position 5 unterscheiden. Schließlich werden substituierte 3,5-Dihydro-4*H*-2,3-benzodiazepin-4-one auch als AMPA-Antagonisten in WO97/34878 (Cocensys Inc.) beschrieben. Trotz eines sehr breiten generischen Anspruchs in Bezug auf die möglichen Substitutionsmuster am Benzodiazepin-Gerüst sind die Ausführungsbeispiele auf einen engen Ausschnitt begrenzt.

Wünschenswert wäre es deshalb neue Verbindungen zu finden, die prophylaktische und therapeutische Eigenschaften aufweisen.

Somit ist es Aufgabe der vorliegenden Erfindung Verbindungen und pharmazeutische Mittel enthaltend diese Verbindungen bereitzustellen, die für eine prophylaktische und therapeutische Verwendung bei hyperproliferativen Erkrankungen, insbesondere bei Tumorerkrankungen sowie als BET-Proteininhibitoren bei viralen Infektionen, bei neurodegenerativen Erkrankungen, bei inflammatorischen Erkrankungen, bei atherosklerotischen Erkrankungen und bei der männlichen Fertilitätskontrolle zur Anwendung kommen.

Die erfindungsgemässen Verbindungen sind neue Phenyl-2,3-benzodiazepine (1-Phenyl-4,5-dihydro-3*H*-2,3-benzodiazepine) und Heteroaryl-2,3-benzodiazepine (1-Heteroaryl-4,5-dihydro-3*H*-2,3-benzodiazepine), die am Benzodiazepingerüst keine Fusion mit einer zweiten heterozyklischen Einheit, speziell einem Isoxazol oder Triazol aufweisen, und überraschenderweise dennoch BRD4-Inhibitoren sind.

Desweiteren unterscheiden sich die erfindungsgemässen Verbindungen von bekannten 2,3-Benzodiazepinen wie den zahlreichen publizierten AMPA-Antagonisten (WO0198280, Annovis Inc.; WO 9728135, Schering AG; für eine Übersicht siehe Med. Res. Rev. 2007, 27(2), 239-278) oder von analogen Diazepinen, bei denen die Benzo-Einheit durch eine andere monocyclische Einheit ersetzt ist, durch ihr Substitutionsmuster an der Phenylgruppe oder an der Benzo-Einheit bzw. einer anderen monocyclischen Einheit: Mindestens ein Substituent an der Phenylgruppe oder an der Benzo-Einheit ist zyklisch ((hetero)aromatisch, (hetero)zyklisch) oder ist neu an der entsprechenden Position wie z.B. Trifluormethoxy oder Alkylaminosulfonylphenyl an der Benzo-Einheit.

Die erfindungsgemässen Verbindungen unterscheiden sich auch von den bekannten psychopharmakologischen 2,3-Benzodiazepin-Derivaten, die Hemmer des Adenosin-Transporters und des MT2-Rezeptors sind (WO2008/124075, Teva Pharm).

Die strukturell nächstliegenden Verbindungen des Standes der Technik sind nicht im Zusammenhang mit der Prophylaxe und Therapie von Tumorerkrankungen offenbart. Ausgehend vom oben beschriebenen Stand der Technik bestand keine Veranlassung, die Strukturen des Standes der Technik so abzuwandeln, dass Strukturen erhalten werden, die für die Prophylaxe und Therapie von Tumorerkrankungen geeignet sind.

Es wurde nun gefunden, dass Verbindungen der allgemeinen Formel (I-A) 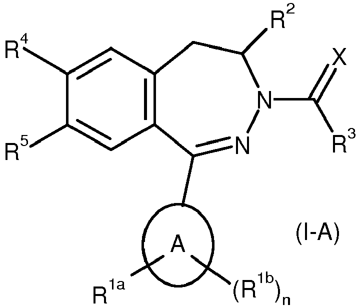 in der
- X: für ein Sauerstoff- oder Schwefelatom steht, und
- A: für einen monozyklischen Heteroarylring mit 5 oder 6 Ringatomen oder für einen Phenylring steht, und
- R^{1a}: für Wasserstoff, Halogen, Cyano, Carboxy, Amino oder Aminosulfonyl steht,
oder
für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino-, C₁-C₆-Alkylamino-C₁-C₆-Alkyl-, *N*-(Heterocyclyl)-C₁-C₆₋Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
oder
für einen monozyklischen Heterocyclylrest mit 3 bis 8 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, Phenyl-, Halophenyl-, Phenyl-C₁-C₆-Alkyl-, Pyridinyl-, -C(=O)-NR⁶R⁷, -C(=O)-R⁸, -S(=O)₂-NR⁶R⁷, -S(=O)-R⁹, -S(=O)₂-R⁹,-NH-S(=O)₂-R⁹, und/oder einem monocyclischen Heterocyclylrest mit 3
bis 8 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 3 bis 8 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden, mit Halogen, C₁-C₃-Alkyl und/oder C₁-C₃-Alkoxy-substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, C₁-C₃-Alkylcarbonyl, -C(=O)NH₂, C₁-C₃-Alkylaminocarbonyl-, C₃-C₆-Cycloalkylaminocarbonyl-, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl-, und/oder einem monocyclischen Heterocyclylrest mit 3 bis 8 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl und/oder C₁-C₃-Alkoxy-,
und
- n: für 0, 1 oder 2 steht, und
- R^{1b}: für Halogen, Hydroxy, Cyano, Nitro und/oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl-Rest und/oder einen monocyclischen Heterocyclylrest mit 3 bis 8 Ringatomen steht, und
- R²: für einen C₁-C₃-Alkyl- oder Trifluormethyl- oder einen C₃- oder C₄-Cycloalkylrest steht, und
- R³: für Cyclopropyl-, C₁-C₃-Alkyl-, C₁-C₃-Alkoxy-, Amino-, Cyclopropylamino- oder C₁-C₃-Alkylamino- steht, und
- R⁴ und R⁵: unabhängig voneinander stehen für Wasserstoff, Hydroxy, Cyano, Nitro, Amino, Aminocarbonyl-, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die gegebenenfalls ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, monocyclischem Heterocyclyl- mit 3 bis 8 Ringatomen und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl,
oder
für C₃-C₁₀-Cycloalkyl-, das gegebenenfalls ein- oder mehrfach, gleich oder verschieden substituiert sein kann mit Halogen, Amino, Hydroxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, und/oder einem monocyclischen Heterocyclylrest mit 3 bis 8 Ringatomen,
oder
für monozyklisches Heteroaryl- mit 5 oder 6 Ringatomen, das gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl, und/oder einem monocyclischen Heterocyclylrest mit 3 bis 8 Ringatomen,
oder
für monozyklisches Heterocyclyl- mit 3 bis 8 Ringatomen, das gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, und/oder einem monocyclischen Heterocyclylrest mit 3 bis 8 Ringatomen,
oder
für Phenyl-, das gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl-, C₁-C₆-Alkylaminosulfonyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen, und
- R⁶ und R⁷: unabhängig voneinander für Wasserstoff, C₁-C₃-Alkyl-, Cyclopropyl-, oder Di-C₁-C₃-Alkyl-Amino-C₁-C₃-Alkyl- stehen, und
- R⁸: für Hydroxy, C₁-C₆-Alkyl-, Halogen-C₁-C₃-Alkyl-, Hydroxy-C₁-C₃-Alkyl-, C₁-C₃-Alkoxy-C₁-C₃-Alkyl-, C₃-C₈-Cycloalkyl-, Phenyl-, monocyclisches Heterocyclyl- mit 3 bis 8 Ringatomen oder monocyclisches Heteroaryl- mit 5 oder 6 Ringatomen steht, worin Phenyl-, Heteroaryl- und Heterocyclyl- gegebenenfalls ein- oder zweifach substituiert sein können durch Halogen, C₁-C₃-Alkoxy- oder C₁-C₃-Alkyl-, und
- R⁹: für C₁-C₆-Alkyl- steht,
sowie deren Polymorphe, Enantiomere, Diastereomere, Razemate, Tautomere, Solvate, physiologisch verträglichen Salze und Solvate dieser Salze, besonders gut für eine Vielzahl prophylaktischer und therapeutischer Verwendungen geeignet sind, insbesondere bei hyperproliferativen Erkrankungen, bei Tumorerkrankungen sowie als BET-Proteininhibitoren bei viralen Infektionen, bei neurodegenerativen Erkrankungen, bei inflammatorischen Erkrankungen, bei atherosklerotischen Erkrankungen und bei der männlichen Fertilitätskontrolle.

Ein Gegenstand der vorliegenden Erfindung sind daher Verbindungen der allgemeinen Formel (I-A), wie oben beschrieben, mit der Maßgabe, dass,
wenn A für einen Phenylring steht und R⁴ und R⁵ unabhängig voneinander stehen für Wasserstoff, Hydroxy, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino- oder C₁-C₆-Alkylaminocarbonyl-, stehen, oder für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die gegebenenfalls ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, monocyclischem Heterocyclyl- mit 3 bis 8 Ringatomen und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl,
R^{1a} nicht für Wasserstoff, Halogen, Cyano, Carboxy, Amino oder Aminosulfonyl, oder für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,

Überraschenderweise hemmen die erfindungsgemäßen Verbindungen die Interaktion zwischen BET-Proteinen, insbesondere BRD4 und einem acetylierten Histon 4 Peptid und inhibieren das Wachstum von Krebszellen. Sie stellen damit neue Strukturen für die Therapie von menschlichen und tierischen Erkrankungen dar, insbesondere von Krebserkrankungen.

Der Erfindung liegen folgende Definitionen zu Grunde:

### Alkyl:

Alkyl steht für einen linearen oder verzweigten, gesättigten, monovalenten Kohlenwasserstoffrest mit in der Regel 1 bis 6 (C₁-C₆-Alkyl), bevorzugt 1 bis 3 Kohlenstoffatomen (C₁-C₃-Alkyl). Beispielhaft seien genannt:
Methyl-, Ethyl-, Propyl-, Butyl-, Pentyl-, Hexyl-, iso-Propyl-, iso-Butyl-, sec-Butyl, tert-Butyl-, iso-Pentyl-, 2-Methylbutyl-, 1-Methylbutyl-, 1-Ethylpropyl-, 1,2-Dimethylpropyl, neo-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- 1,2-Dimethylbutyl-.
   Bevorzugt sind ein Methyl-, Ethyl-, Propyl-, Isopropyl- oder tert-Butylrest.

### Cycloalkyl:

Cycloalkyl steht für einen mono- oder bicyclischen, gesättigten, monovalenten Kohlenwasserstoffrest mit in der Regel 3 bis 10 (C₃-C₁₀-Cycloalkyl-), bevorzugt 3 bis 8 (C₃-C₈-Cycloalkyl-), und besonders bevorzugt 3 bis 7 (C₃-C₇-Cycloalkyl-) Kohlenstoffatomen.

Beispielhaft und bevorzugt für monocyclische Cycloalkylreste seien genannt:
Cyclopropyl-, Cyclobutyl-, Cyclopentyl-, Cyclohexyl- und Cycloheptyl-. Besonders bevorzugt ist ein Cyclopropyl-, Cylopentyl- oder ein Cyclohexylrest. Beispielhaft für bicyclische Cycloalkylreste seien genannt: Perhydropentalenyl-, Decalinyl-.

### Phenylalkyl:

Unter Phenyl-C₁-C₆-alkyl- ist eine Gruppe zu verstehen, die zusammengesetzt ist aus einem gegebenenfalls substituierten Phenylrest und einer C₁-C₆-Alkyl-Gruppe, und die über die C₁-C₆-Alkyl-Gruppe an den Rest des Moleküls gebunden ist. Der Alkylrest hat hierbei die oben unter Alkyl angegebenen Bedeutungen. Bevorzugt ist Phenyl-C₁-C₃-alkyl-.
Beispielhaft seien genannt Benzyl-, Phenethyl-, Phenylpropyl-, Phenylpentyl-, wobei Benzylbesonders bevorzugt ist.

### Alkoxy:

Alkoxy- steht für einen linearen oder verzweigten, gesättigten Alkyletherrest der Formel -O-Alkyl mit in der Regel 1 bis 6 (C₁-C₆-Alkoxy-), bevorzugt 1 bis 3 (C₁-C₃-Alkoxy) Kohlenstoffatomen. Beispielhaft und bevorzugt seien genannt:
Methoxy-, Ethoxy-, n-Propoxy-, Isopropoxy-, *tert*-Butoxy-, n-Pentyloxy- und n-Hexyloxy-.

### Alkoxyalkyl

Alkoxyalkyl- steht für einen mit Alkoxy substituierten Alkylrest. C₁-C₃-Alkoxy-C₁-C₃-Alkyl- bedeutet dabei, dass die Bindung an das übrige Molekül über den Alkylteil erfolgt..

### Alkoxyalkoxy

Alkoxyalkoxy- steht für einen mit Alkoxy substituierten Alkoxyrest. C₁-C₃-Alkoxy-C₂-C₃-Alkoxy- bedeutet dabei, dass die Bindung an das übrige Molekül über den inneren C₂-C₃-Alkoxylteil erfolgt.

### Oxo

Unter Oxo, einer Oxogruppe oder einem Oxo-Substituenten ist ein doppelt gebundenes SauerstoffAtom =O zu verstehen. Oxo kann an Atome geeigneter Valenz gebunden sein, beispielsweise an ein gesättigtes Kohlenstoff-Atom oder an Schwefel.
Bevorzugt ist die Bindung an Kohlenstoff unter Bildung einer Carbonyl-Gruppe -C(=O)-. Bevorzugt ist weiterhin die Bindung zweier doppelt gebundenener Sauerstoffatome an ein Schwefelatom unter Bildung einer Sulfonyl-Gruppe -S(=O)₂-.

### Alkylamino

Alkylamino- steht für einen Aminorest mit einem oder zwei (unabhängig voneinander gewählten) Alkylsubstituenten mit in der Regel 1 bis 6 (C₁-C₆-Alkylamino-), bevorzugt 1 bis 3 Kohlenstoffatomen (C₁-C₃-Alkylamino-).
(C₁-C₃)-Alkylamino- steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.
Beispielhaft seien genannt:
Methylamino-, Ethylamino-, n-Propylamino-, Isopropylamino-, *tert*-Butylamino-, n-Pentylamino-, n-Hexylamino-, *N,N*-Dimethylamino-, *N,N*-Diethylamino-, *N*-Ethyl-*N*-methylamino-, *N*-Methyl-N-n-propylamino-, *N*-Isopropyl-*N*-n-propylamino-, *N*-*tert*-Butyl-*N*-methylamino-, *N*-Ethyl-*N*-n-pentylamino- und *N*-n-Hexyl-*N*-methylamino-.

### Alkylaminocarbonyl

Alkylaminocarbonyl- steht für die Gruppe Alkylamino-C(=O)- mit einem oder zwei (unabhängig voneinander gewählten) Alkylsubstituenten mit in der Regel 1 bis 6 (C₁-C₆-Alkylanünocarbonyl-), bevorzugt 1 bis 3 Kohlenstoffatomen (C₁-C₃-Alkylaminocarbonyl-).

### Cycloalkylaminocarbonyl

Cycloalkylaminocarbonyl- steht für die Gruppe Cycloalkyl-NH-C(=O)- mit einem Cycloalkylsubstituenten, in der Regel bestehend aus 3 bis 6 (C₃-C₆-Cycloalkylaminocarbonyl-) Kohlenstoffatomen.
Beispielhaft und bevorzugt seien genannt:
Cyclopropylaminocarbonyl- und Cyclopentylaminocarbonyl-.

### Alkylcarbonyl

Alkylcarbonyl- steht für die Gruppe -C(=O)-Alkyl mit in der Regel 1 bis 6 (C₁-C₆-Alkylcarbonyl-), bevorzugt 1 bis 4, und besonders bevorzugt 1 bis 3 Kohlenstoffatomen im Alkylteil. Beispielhaft genannt sind Acetyl- und Propanoyl-.

### Alkylcarbonylamino

Alkylcarbonylamino- steht für die Gruppe Alkyl-C(=O)-NH- mit in der Regel 1 bis 6 (C₁-C₆-Alkylcarbonylamino-), bevorzugt 1 bis 4, und besonders bevorzugt 1 bis 3 Kohlenstoffatomen im Alkylteil.

### Alkoxycarbonyl

Alkoxycarbonyl- steht für die Gruppe -C(=O)-O-Alkyl mit in der Regel 1 bis 6 (C₁-C₆-Alkoxycarbonyl-), bevorzugt 1 bis 4, und besonders bevorzugt 1 bis 3 Kohlenstoffatomen im Alkylteil.

Beispielhaft seien genannt:
Methoxycarbonyl-, Ethoxycarbonyl-, Propoxycarbonyl-, Isopropoxycarbonyl-, *tert*-Butoxycarbonyl-, n-Pentyloxycarbonyl- und n-Hexyloxycarbonyl-.

### Alkylsulfonyl

Alkylsulfonyl- steht für einen linearen oder verzweigten, gesättigten Rest der Formel -S(=O)₂-Alkyl mit in der Regel 1 bis 6 (C₁-C₆-Alkylsulfonyl-), bevorzugt -1 bis 3 (C₁-C₃-Alkylsulfonyl-) Kohlenstoffatomen.
Beispielhaft und bevorzugt seien genannt:
Methylsulfonyl-, Ethylsulfonyl-, Propylsulfonyl-.

### Alkylsulfinyl

Alkylsulfinyl- steht für einen linearen oder verzweigten, gesättigten Rest der Formel -S(=O)-Alkyl mit in der Regel 1 bis 6 (C₁-C₆-Alkylsulfinyl-), bevorzugt -1 bis 3 (C₁-C₃-Alkylsulfinyl-) Kohlenstoffatomen.
Beispielhaft und bevorzugt seien genannt:
Methylsulfinyl-, Ethylsulfinyl-, Propylsulfinyl-.

### Alkylsulfonylamino

Alkylsulfonylamino- steht für einen linearen oder verzweigten, gesättigten Rest der Formel -NH-S(=O)₂-Alkyl mit 1 bis 3 (C₁-C₃-Alkylsulfonyl-) Kohlenstoffatomen in der Alkylgruppe. Beispielhaft und bevorzugt seien genannt:
Methylsulfonylamino-, Ethylsulfonylamino-, Propylsulfonylamino-.

### Alkylaminosulfonyl

Alkylaminosulfonyl- steht für die Gruppe Alkylamino-S(=O)₂- mit einem oder zwei (unabhängig voneinander gewählten) Alkylsubstituenten mit in der Regel 1 bis 6 (C₁-C₆-Alkylaminosulfonyl-), bevorzugt 1 bis 3 Kohlenstoffatomen.
Beispielhaft und bevorzugt seien genannt:
Methylaminosulfonyl-, Ethylaminosulfonyl-, Dimethylaminosulfonyl-.

### Cycloalkylaminosulfonyl

Cycloalkylaminosulfonyl- steht für die Gruppe Cycloalkyl-NH-S(=O)₂- mit einem Cycloalkylsubstituenten, in der Regel bestehend aus 3 bis 6 (C₃-C₆-Cycloalkylaminosulfonyl-) Kohlenstoffatomen.
Beispielhaft und bevorzugt sei genannt:
Cyclopropylaminosulfonyl-.

### Heteroatome

Unter Heteroatomen sind Sauerstoff-, Stickstoff- und Schwefel-Atome zu verstehen.

### Heteroaryl

Heteroaryl bedeutet ein monovalentes, monocyclisches aromatisches Ringsystem mit 5 oder 6 Ringatomen, wovon mindestens eines ein Heteroatom ist. Als Heteroatome können Stickstoffatome, Sauerstoffatome und/oder Schwefelatome vorkommen. Die Bindungsvalenz kann sich an einem beliebigen aromatischen Kohlenstoffatom oder an einem Stickstoffatom befinden.

Ein monocyclischer Heteroarylrest gemäß der vorliegenden Erfindung hat 5 oder 6 Ringatome.

Heteroarylreste mit 5 Ringatomen umfassen beispielsweise die Ringe:
Thienyl-, Thiazolyl-, Furyl-, Pyrrolyl-, Oxazolyl-, Imidazolyl-, Pyrazolyl-, Isoxazolyl-, Isothiazolyl-, Oxadiazolyl-, Triazolyl-, Tetrazolyl- und Thiadiazolyl-.

Heteroarylreste mit 6 Ringatomen umfassen beispielsweise die Ringe:
Pyridyl-, Pyridazinyl-, Pyrimidinyl-, Pyrazinyl- und Triazinyl-.

### Heterocyclyl

Heterocyclyl bedeutet ein nicht aromatisches monocyclisches Ringsystem mit mindestens einem Heteroatom oder einer Heterogruppe. Als Heteroatome können Stickstoffatome, Sauerstoffatome und/oder Schwefelatome vorkommen. Als Heterogruppen können -S(=O)-, -S(=O)₂- oder -N⁺(O⁻)-vorkommen.
Ein monocyclischer Heterocyclylring gemäß der vorliegenden Erfindung kann 3 bis 8, bevorzugt 5 bis 8 oder 4 bis 7, besonders bevorzugt 5 oder 6 Ringatome aufweisen.

Beispielhaft und bevorzugt für monocyclische Heterocyclylreste mit 3 Ringatomen seien genannt: Aziridinyl-.

Beispielhaft und bevorzugt für monocyclische Heterocyclylreste mit 4 Ringatomen seien genannt: Azetidinyl-, Oxetanyl-.

Beispielhaft und bevorzugt für monocyclische Heterocyclylreste mit 5 Ringatomen seien genannt: Pyrrolidinyl-, Imidazolidinyl- , Pyrazolidinyl-, Pyrrolinyl-, Dioxolanyl- und Tetrahydrofuranyl-. Beispielhaft und bevorzugt für monocyclische Heterocyclylreste mit 6 Ringatomen seien genannt: Piperidinyl-, Piperazinyl-, Morpholinyl-, Dioxanyl-, Tetrahydropyranyl- und Thiomorpholinyl-.

Beispielhaft und bevorzugt für monocyclische Heterocyclylreste mit 7 Ringatomen seien genannt: Azepanyl-, Oxepanyl-, 1,3-Diazepanyl-, 1,4-Diazepanyl-.

Beispielhaft und bevorzugt für monocyclische Heterocyclylreste mit 8 Ringatomen seien genannt: Oxocanyl-, Azocanyl-.
Bevorzugt sind 5- bis 8- und 4 bis 7-gliedrige, monocyclische gesättigte Heterocyclylreste mit bis zu zwei Heteroatomen aus der Reihe O, N und S.
Besonders bevorzugt sind Morpholinyl-, Piperidinyl-, Piperazinyl- und Pyrrolidinyl-.

### N-Heterocyclyl

*N*-Heterocyclyl bedeutet ein nicht aromatisches cyclisches Ringsystem mit mindestens einem Stickstoffatom als Heteroatom, das über das Stickstoffatom an den Rest des Moleküls gebunden ist.

### Halogen

Die Bezeichnung Halogen umfasst Fluor, Chlor, Brom und Iod.
Bevorzugt sind Fluor und Chlor.

### Halo

Halo steht für Halogen und umfasst Fluor, Chlor und Brom und bezeichnet einen mit Fluor, Chlor oder Brom substituierten Rest, wie zum Beispiel Halophenyl, welches einen ein- oder mehrfach, gleich oder verschieden mit Fluor, Chlor und/oder Brom substituierten Phenylrest bedeutet.

### Halogenalkyl

Halogenalkyl steht für einen Alkylrest mit mindestens einem Halogensubstituenten.
Ein Halogen-C₁-C₆-Alkylrest ist ein Alkylrest mit 1-6 Kohlenstoffatomen und mindestens einem Halogensubstituenten. Sind mehrere Halogensubstituenten enthalten, so können diese auch verschieden sein. Bevorzugt sind Fluor-C₁-C₃-Alkyl-Reste.
Beispielhaft und bevorzugt seien weiterhin genannt:
Trifluormethyl-, 2,2,2-Trifluorethyl-, Pentafluorethyl-, 4,4,5,5,5-Pentafluorpentyl- oder 3,3,4,4,5,5,5-Heptafluorpentylgruppe.
   Besonders bevorzugt sind Trifluormethyl-, 2,2-Difluorethyl und 2,2,2-Trifluorethyl.

### Halogenalkoxy

Halogenalkoxy steht für einen Alkoxyrest mit mindestens einem Halogensubstituenten.
Ein Halogen-C₁-C₆-Alkoxyrest ist ein Alkoxyrest mit 1-6 Kohlenstoffatomen und mindestens einem Halogensubstituenten. Sind mehrere Halogensubstituenten enthalten, so können diese auch verschieden sein .Bevorzugt sind Fluor-C₁-C₃-alkoxy-Reste.
Beispielhaft und besonders bevorzugt seien genannt:
Difluormethoxy-, Trifluormethoxy- oder 2,2,2-Trifluorethoxy-.

### Hydroxylalkyl

Hydroxyalkyl steht für einen Alkylrest mit mindestens einem Hydroxysubstituenten.
Ein Hydroxy-C₁-C₆-Alkylrest ist ein Alkylrest mit 1-6 Kohlenstoffatomen und mindestens einem Hydroxysubstituenten. Bevorzugt ist Hydroxy-C₁-C₃-Alkyl-.

Bevorzugt sind solche Verbindungen der allgemeinen Formel (I-A), in der
- X: für ein Sauerstoffatom steht,
- A: für einen Phenyl- oder Pyridylring steht,
- R^{1a}: für Wasserstoff, Halogen, Cyano, Carboxy, Amino oder Aminosulfonyl steht,
oder
für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino-, C₁-C₆-Alkylamino-C₁-C₆-Alkyl-, *N*-(Heterocyclyl)-C₁₋C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
oder
für einen monozyklischen Heterocyclylrest mit 3 bis 8 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl-, Phenyl-, Halophenyl-, Phenyl-C₁-C₆-Alkyl-, Pyridinyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 3 bis 8 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 3 bis 8 Ringatomen und/oder mit einem monocyclischen Heteroarylrest mit 5 oder 6 Ringatomen und/oder mit einem Phenylrest, der seinerseits gegebenenfalls ein- oder mehrfach, gleich oder verschieden mit Halogen, C₁-C₃-Alkyl und/oder C₁-C₃-Alkoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, C₁-C₃-Alkylcarbonyl-, -C(=O)NH₂, C₁-C₃-Alkylaminocarbonyl-, C₃-C₆-Cycloalkylaminocarbonyl-, C₁-C₃-Alkylsulfinyl-, C₁-C₃-Alkylsulfonyl-, - S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl-, und/oder einem monocyclischen Heterocyclylrest mit 3 bis 8 Ringatomen und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl und/oder C₁-C₃-Alkoxy-, und
- n: für 0, 1 oder 2 steht, und
- R^{1b}: für Halogen, Hydroxy, Cyano, Nitro und/oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl-Rest und/oder einen monocyclischen Heterocyclylrest mit 3 bis 8 Ringatomen steht, und
- R²: für Methyl steht, und
- R³: für Cyclopropyl-, C₁-C₃-Alkyl-, C₁-C₃-Alkoxy-, Amino-, Cyclopropylamino- oder C₁-C₃-Alkylamino- steht, und
- R⁴ und R⁵: unabhängig voneinander stehen für Wasserstoff, Hydroxy, Cyano, Nitro, Amino, Aminocarbonyl-, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die gegebenenfalls ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino- oder Amino-C₁-C₆-Alkyl-, einem monocyclischen Heterocyclylrest mit 3 bis 8 Ringatomen und/oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl-,
oder
für einen C₃-C₁₀-Cycloalkylrest, der gegebenenfalls ein- oder mehrfach, gleich oder verschieden substituiert sein kann mit Halogen, Amino, Hydroxy, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, und/oder einem monocyclischen Heterocyclylrest mit 3 bis 8 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl-, und/oder einem monocyclischen Heterocyclylrest mit 3 bis 8 Ringatomen,
oder
für einen monozyklischen Heterocyclylrest mit 3 bis 8 Ringatomen, der gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Hydroxy-C₁-C₆-Alkyl-, Halogen-C₁-C₆-Alkyl-, Halogen-C₁-C₆-Alkoxy-, C₃-C₁₀-Cycloalkyl-, und/oder einem monocyclischen Heterocyclylrest mit 3 bis 8 Ringatomen,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl, C₁-C₆-Alkylaminosulfonyl-, C₁-C₆-Alkylamino-C₁-C₆-Alkyl, Hydroxy-C₁-C₆-Alkyl-, Halogen-C₁-C₆-Alkyl-, Halogen-C₁-C₆-Alkoxy-, C₃-C₁₀-Cycloalkyl-, und/oder einem monocyclischen Heterocyclylrest mit 3 bis 8 Ringatomen, und
- R⁶ und R⁷: unabhängig voneinander für Wasserstoff, C₁-C₃-Alkyl-, Cyclopropyl-, oder Di-C₁-C₃-Alkyl-Amino-C₁-C₃-Alkyl- stehen, und
- R⁸: für Hydroxy, C₁-C₆-Alkyl-, Halogen-C₁-C₃-Alkyl-, Hydroxy-C₁-C₃-Alkyl-, C₁-C₃-Alkoxy-C₁-C₃-Alkyl-, C₃-C₈-Cycloalkyl-, Phenyl, monocyclisches Heterocyclyl- mit 3 bis 8 Ringatomen oder monocyclisches Heteroaryl- mit 5 oder 6 Ringatomen steht, und
- R⁹: für C₁-C₆-Alkyl- steht,
mit der Maßgabe, dass,
wenn A für einen Phenylring steht und R⁴ und R⁵ unabhängig voneinander stehen für Wasserstoff, Hydroxy, Amino, Aminocarbonyl-, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die gegebenenfalls ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino- oder Amino-C₁-C₆-Alkyl-, einem monocyclischen Heterocyclylrest mit 3 bis 8 Ringatomen und/oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl-,
R^{1a} nicht für Wasserstoff, Halogen, Cyano, Carboxy, Amino oder Aminosulfonyl, oder für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
sowie deren Polymorphe, Enantiomere, Diastereomere, Razemate, Tautomere, Solvate, physiologisch verträglichen Salze und Solvate dieser Salze.

Mehr bevorzugt sind solche Verbindungen der allgemeinen Formel (I-A), in der
- X: für ein Sauerstoffatom steht,
- A: für einen Phenyl- oder Pyridylring steht,
- R^{1a}: für Wasserstoff, Halogen, Cyano, Carboxy, Amino oder Aminosulfonyl steht,
oder
für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, Phenyl-, Halophenyl-, Phenyl-C₁-C₆-Alkyl-, Pyridinyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen und/oder mit einem monocyclischen Heteroarylrest mit 5 oder 6 Ringatomen und/oder mit einem Phenylrest, der seinerseits gegebenenfalls ein- oder mehrfach, gleich oder verschieden mit Halogen, C₁-C₃-Alkyl- und/oder C₁-C₃-Alkoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, C₁-C₃-Alkylcarbonyl-, -C(=O)NH₂, C₁-C₃-Alkylaminocarbonyl-, C₃-C₆-Cycloalkylaminocarbonyl-, C₁-C₃-Alkylsulfinyl-, C₁-C₃-Alkylsulfonyl-, - S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl, und/oder einem monocyclischen Heterocyclylrest mit 4 bis 7 Ringatomen und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl- und/oder C₁-C₃-Alkoxy-, und
- n: für 0 oder 1 steht, und
- R^{1b}: für Halogen, Hydroxy, Cyano, Nitro oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl-Rest, und/oder einen monocyclischen Heterocyclylrest mit 4 bis 7 Ringatomen steht, und
- R²: für Methyl steht, und
- R³: für Cyclopropyl-, C₁-C₃-Alkyl-, C₁-C₃-Alkoxy-, Amino-, Cyclopropylamino- oder C₁-C₃-Alkylamino- steht, und
- R⁴ und R⁵: unabhängig voneinander stehen für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, einem monocyclischen Heterocyclylrest mit 4 bis 7 Ringatomen, und/oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl-,
oder
für einen C₃-C₁₀-Cycloalkylrest, der gegebenenfalls ein- oder mehrfach, gleich oder verschieden substituiert sein kann mit Halogen, Amino, Hydroxy, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, und/oder einem monocyclischen Heterocyclylrest mit 4 bis 7 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl-, und/oder einem monocyclischen Heterocyclylrest mit 4 bis 7 Ringatomen,
oder
für einen monozyklischen Heterocyclylrest mit 4 bis 7 Ringatomen, der gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, und/oder einem monocyclischen Heterocyclylrest mit 4 bis 7 Ringatomen,
oder
- R⁶ und R⁷: für einen Phenylrest, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl-, C₁-C₆-Alkylaminosulfonyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, und/oder einem monocyclischen Heterocyclylrest mit 4 bis 7 Ringatomen, und unabhängig voneinander für Wasserstoff, C₁-C₃-Alkyl-, Cyclopropyl-, oder Di-C₁-C₃-Alkyl-Amino-C₁-C₃-Alkyl- stehen, und
- R⁸: für Hydroxy, C₁-C₆-Alkyl-, Halogen-C₁-C₃-Alkyl-, Hydroxy-C₁-C₃-Alkyl-, C₁-C₃-Alkoxy-C₁-C₃-Alkyl-, C₃-C₈-Cycloalkyl-, Phenyl-, monocyclisches Heterocyclyl- mit 5 oder 6 Ringatomen steht, und
- R⁹: für C₁-C₆-Alkyl- steht,
mit der Maßgabe, dass,
wenn A für einen Phenylring steht und R⁴ und R⁵ unabhängig voneinander stehen für Wasserstoff, Hydroxy, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, einem monocyclischen Heterocyclylrest mit 4 bis 7 Ringatomen, und/oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl-,
R^{1a} nicht für Wasserstoff, Halogen, Cyano, Carboxy, Amino oder Aminosulfonyl, oder für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
sowie deren Polymorphe, Enantiomere, Diastereomere, Razemate, Tautomere, Solvate, physiologisch verträglichen Salze und Solvate dieser Salze.

Noch mehr bevorzugt sind solche Verbindungen der allgemeinen Formel (I-A), in der
- X: für ein Sauerstoffatom steht,
- A: für einen Phenyl- oder Pyridylring steht,
- R^{1a}: für Wasserstoff, Halogen, Cyano, Carboxy, Amino oder Aminosulfonyl steht,
oder
für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, Phenyl-, Halophenyl-, Phenyl-C₁-C₆-Alkyl-, Pyridinyl-, -C(=O)-NR⁶R⁷, -C(=O)-_{R}⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Halogen, C₁-C₃-Alkyl und/oder C₁-C₃-Alkoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, C₁-C₃-Alkylcarbonyl-, -C(=O)NH₂, C₁-C₃-Alkylaminocarbonyl-, C₃-C₆-Cycloalkylaminocarbonyl-, C₁-C₃-Alkylsulfinyl-, C₁-C₃-Alkylsulfonyl-, - S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl-, und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl- und/oder C₁-C₃-Alkoxy-, und
- n: für 0 oder 1 steht, und
- R^{1b}: für Halogen, Hydroxy, Cyano, Nitro oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl-Rest und/oder einen monocyclischen Heterocyclylrest mit 4 bis 7 Ringatomen steht, und
- R²: für Methyl steht, und
- R³: für Methyl oder C₁-C₃-Alkylamino- steht, und
- R⁴ und R⁵: unabhängig voneinander stehen für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, einem monocyclischen Heterocyclylrest mit 4 bis 7 Ringatomen, und/oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl,
oder
für einen C₃-C₁₀-Cycloalkylrest, der gegebenenfalls ein- oder mehrfach, gleich oder verschieden substituiert sein kann mit Halogen, Amino, Hydroxy, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, und/ oder einem monocyclischen Heterocyclylrest mit 4 bis 7 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, und/oder einem monocyclischen Heterocyclylrest mit 4 bis 7 Ringatomen,
oder
für einen monozyklischen Heterocyclylrest mit 4 bis 7 Ringatomen, der gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, und/oder einem monocyclischen Heterocyclylrest mit 4 bis 7 Ringatomen, oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl, C₁-C₆-Alkylaminosulfonyl-, C₁-C₆-Alkylamino-C₁-C₆-Alkyl, Hydroxy-C₁-C₆-Alkyl-, Halogen-C₁-C₆-Alkyl-, Halogen-C₁-C₆-Alkoxy-, C₃-C₁₀-Cycloalkyl-, und/oder einem monocyclischen Heterocyclylrest mit 4 bis 7 Ringatomen, und
- R⁶ und R⁷: unabhängig voneinander für Wasserstoff, C₁-C₃-Alkyl-, Cyclopropyl-, oder Di-C₁-C₃-Alkyl-Amino-C₁-C₃-Alkyl- stehen, und
- R⁸: für Hydroxy, C₁-C₆-Alkyl-, Halogen-C₁-C₃-Alkyl-, Hydroxy-C₁-C₃-Alkyl-, C₁-C₃-Alkoxy-C₁-C₃-Alkyl-, C₃-C₈-Cycloalkyl-, monocyclisches Heterocyclyl- mit 5 oder 6 Ringatomen steht, und
- R⁹: für C₁-C₆-Alkyl- steht,
mit der Maßgabe, dass,
wenn A für einen Phenylring und R⁴ und R⁵ unabhängig voneinander stehen für Wasserstoff, Hydroxy, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, einem monocyclischen Heterocyclylrest mit 4 bis 7 Ringatomen, und/oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl,
R^{1a} nicht Wasserstoff, Halogen, Cyano, Carboxy, Amino oder Aminosulfonyl, oder für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
sowie deren Polymorphe, Enantiomere, Diastereomere, Razemate, Tautomere, Solvate, physiologisch verträglichen Salze und Solvate dieser Salze.

Besonders bevorzugt sind solche Verbindungen der allgemeinen Formel (I-A), in der
- X: für ein Sauerstoffatom steht,
- A: für einen Phenyl- oder Pyridylring steht,
- R^{1a}: für Wasserstoff, Halogen, Cyano, Carboxy, Amino oder Aminosulfonyl steht,
oder
für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino-, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, Phenyl-, Halophenyl-, Phenyl-C₁-C₆-Alkyl-, Pyridinyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Halogen, C₁-C₃-Alkyl und/oder C₁-C₃-Alkoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, C₁-C₃-Alkylcarbonyl-, -C(=O)NH₂, C₁-C₃-Alkylaminocarbonyl-, C₃-C₆-Cycloalkylaminocarbonyl-, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl-, und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl und/oder C₁-C₃-Alkoxy-, und
- n: für 0 oder 1 steht, und
- R^{1b}: für Halogen, Hydroxy, Cyano, Nitro oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl-Rest und/oder einen monocyclischen Heterocyclylrest mit 4 bis 7 Ringatomen steht, und
- R²: für für Methyl steht, und
- R³: für Methyl oder C₁-C₃-Alkylamino- steht, und
- R⁴: für Wasserstoff, Chlor, Cyano, Methoxy-, Ethoxy- oder Difluormethoxy- steht, und
- R⁵: für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl- steht,
oder
für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl- steht, die ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, einem monocyclischen Heterocyclylrest mit 4 bis 7 Ringatomen, und/oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl,
oder
für einen C₃-C₁₀-Cycloalkylrest steht, der gegebenenfalls ein- oder mehrfach, gleich oder verschieden substituiert sein kann mit Halogen, Amino, Hydroxy, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, und/oder einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl-, und/oder einem monocyclischen Heterocyclylrest mit 4 bis 7 Ringatomen,
oder
für einen monozyklischen Heterocyclylrest mit 4 bis 7 Ringatomen steht, der gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, und/oder einem monocyclischen Heterocyclylrest mit 4 bis 7 Ringatomen,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl-, C₁-C₆-Alkylaminosulfonyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, und/oder einem monocyclischen Heterocyclylrest mit 4 bis 7 Ringatomen, und
- R⁶ und R⁷: unabhängig voneinander für Wasserstoff, C₁-C₃-Alkyl-, Cyclopropyl-, oder Di-C₁-C₃-Alkyl-Amino-C₁-C₃-Alkyl- stehen, und
- R⁸: für Hydroxy, C₁-C₆-Alkyl-, Hydroxy-C₁-C₃-Alkyl-, Trifluormethyl-, Pyrrolidinyl-, Morpholinyl- oder Piperidinyl- steht, und
- R⁹: für C₁-C₆-Alkyl- steht,
mit der Maßgabe, dass,
wenn A für Phenyl steht, R⁴ für Wasserstoff, Chlor, Methoxy-, Ethoxy- oder Difluormethoxysteht und R⁵ für Wasserstoff, Hydroxy, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl- steht,
oder für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl- steht, die ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, einem monocyclischen Heterocyclylrest mit 4 bis 7 Ringatomen, und/oder einem
monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl,
R^{1a} nicht für Wasserstoff, Halogen, Cyano, Carboxy, Amino oder Aminosulfonyl, oder für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino-, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy- , Halogen-C₁-C₆-Alkyl-, Halogen-C₁-C₆-Alkoxy-, C₁-C₆-Alkylcarbonyl- oder C₁-C₆-Alkoxycarbonyl-Rest steht,
sowie deren Polymorphe, Enantiomere, Diastereomere, Razemate, Tautomere, Solvate, physiologisch verträglichen Salze und Solvate dieser Salze.

Ganz besonders bevorzugt sind solche Verbindungen der allgemeinen Formel (I-A), in der
- X: für ein Sauerstoffatom steht,
- A: für einen Phenyl- oder Pyridylring steht,
- R^{1a}: für Wasserstoff, Halogen, Cyano, Carboxy, Amino oder Aminosulfonyl steht,
oder
für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, Phenyl-, Halophenyl-, Phenyl-C₁-C₆-Alkyl-, Pyridinyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Halogen, C₁-C₃-Alkyl und/oder C₁-C₃-Alkoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, C₁-C₃-Alkylcarbonyl-, -C(=O)NH₂, C₁-C₃-Alkylaminocarbonyl-, C₃-C₆-Cycloalkylaminocarbonyl-, C₁-C₃-Alkylsulfinyl-, C₁-C₃-Alkylsulfonyl-, - S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl-, und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl und/oder C₁-C₃-Alkoxy-, und
- n: für 0 oder 1 steht, und
- R^{1b}: für Fluor steht, und
- R²: für für Methyl steht, und
- R³: für Methyl oder C₁-C₃-Alkylamino- steht, und
- R⁴: für Wasserstoff, Chlor, Cyano, Methoxy-, Ethoxy- oder Difluormethoxy- steht, und
- R⁵: für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl- steht,
oder
für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl- steht, die ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, einem monocyclischen Heterocyclylrest mit 4 bis 7 Ringatomen, oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl,
für einen C₃-C₁₀-Cycloalkylrest steht, der gegebenenfalls ein- oder mehrfach, gleich oder verschieden substituiert sein kann mit Halogen, Amino, Hydroxy, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, und/oder einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl-, und/oder einem monocyclischen Heterocyclylrest mit 4 bis 7 Ringatomen,
oder
für einen monozyklischen Heterocyclylrest mit 4 bis 7 Ringatomen steht, der gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Hydroxy-C₁-C₆-Alkyl-, Halogen-C₁-C₆-Alkyl-, Halogen-C₁-C₆-Alkoxy-, C₃-C₁₀-Cycloalkyl-, und/oder einem monocyclischen Heterocyclylrest mit 4 bis 7 Ringatomen,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl, C₁-C₆-Alkylaminosulfonyl-, C₁-C₆-Alkylamino-C₁-C₆-Alkyl, Hydroxy-C₁-C₆-Alkyl-, Halogen-C₁-C₆-Alkyl-, Halogen-C₁-C₆-Alkoxy-, C₃-C₁₀Cycloalkyl-, und/oder einem monocyclischen Heterocyclylrest mit 4 bis 7 Ringatomen, und
- R⁶ und R⁷: unabhängig voneinander für Wasserstoff, C₁-C₃-Alkyl-, Cyclopropyl-, oder Di-C₁-C₃-Alkyl-Amino-C₁-C₃-Alkyl- stehen, und
- R⁸: für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
- R⁹: für C₁-C₃-Alkyl- steht,
mit der Maßgabe, dass,
wenn A für Phenyl steht, R⁴ für Wasserstoff, Chlor, Methoxy-, Ethoxy- oder Difluormethoxysteht und R⁵ für Wasserstoff, Hydroxy, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl- steht,
oder für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl- steht, die ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, einem monocyclischen Heterocyclylrest mit 4 bis 7 Ringatomen, oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl,
R^{1a} nicht für Wasserstoff, Halogen, Cyano, Carboxy, Amino oder Aminosulfonyl oder für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino, C₁-C₆-Alkylamino-C₁-C₆-Alkyl-, *N*-(Heterocyclyl)-C₁-C₆-Alkyl-, *N-*(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
sowie deren Polymorphe, Enantiomere, Diastereomere, Razemate, Tautomere, Solvate, physiologisch verträglichen Salze und Solvate dieser Salze.

Ebenfalls ganz besonders bevorzugt sind solche Verbindungen der allgemeinen Formel (I-A), in der
- X: für ein Sauerstoffatom steht,
- A: für einen Phenyl- oder Pyridylring steht,
- R^{1a}: für Wasserstoff, Halogen, Cyano, Carboxy, Amino oder Aminosulfonyl steht,
oder
für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino-, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonylrest,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, Phenyl-, Halophenyl-, Phenyl-C₁-C₆-Alkyl-, Pyridinyl-,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, -C(=O)-NR⁶R⁷, -C(=O)-R⁸, -S(=O)₂-NR⁶R⁷, -S(=O)-R⁹, -S(=O)₂-R⁹ oder -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Halogen, C₁-C₃-Alkyl- und/oder C₁-C₃-Alkoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁C₆-Alkyl-, C₁-C₆-Alkylamino-C₁-C₆-Alkyl-, C₁-C₃-Alkylcarbonyl-, -C(=O)NH₂, C₁-C₃-Alkylaminocarbonyl-, C₃-C₆-Cycloalkylaminocarbonyl-, C₁-C₃-Alkylsulfinyl-, C₁-C₃-Alkylsulfonyl, - S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl-, und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl- und/oder C₁-C₃-Alkoxy-, und
- n: für 0 oder 1 steht, und
- R^{1b}: für Fluor steht, und
- R²: für Methyl steht, und
- R³: für Methyl oder C₁-C₃-Alkylamino- steht, und
- R⁴: für Wasserstoff, Chlor, Cyano, Methoxy-, Ethoxy- oder Difluormethoxy- steht, und
- R⁵: für Chlor, Hydroxy, Amino, C₁-C₆-Alkyl-, Methoxy-, Ethoxy-, Difluormethoxy-, Trifluormethoxy-, C₁-C₃-Alkylcarbonylamino-, Cyclopropyl-, Pyridinyl-, Morpholinyl-, Piperidinyl-, gegebenenfalls ein- oder mehrfach mit Methyl substituiertes Piperazinyl-, Pyrazolyl-, oder für gegebenenfalls mit C₁-C₃-Alkylaminosulfonyl- substituiertes Phenyl, steht, oder für C₁-C₃-Alkoxy- steht, welches mit Morpholinyl-, Pyrrolidinyl-, Piperazinyl- oder Pyridinyl- substituiert sein kann, worin das Piperazinyl- oder Pyridinyl- selbst mit C₁-C₃-Alkyl- substituiert sein kann, und
- R⁶ und R⁷: unabhängig voneinander für Wasserstoff, C₁-C₃-Alkyl-, Cyclopropyl-, oder Di-C₁-C₃-Alkyl-Amino-C₁-C₃-Alkyl- stehen, und
- R⁸: für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
- R⁹: für C₁-C₃-Alkyl- steht,
mit der Maßgabe, dass,
wenn A für Phenyl steht, R⁴ für Wasserstoff, Chlor, Methoxy-, Ethoxy- oder Difluormethoxy- steht und R⁵ für Chlor, Hydroxy, Amino, C₁-C₆-Alkyl-, Methoxy-, Ethoxy, Difluormethoxy-, Trifluormethoxy-, C₁-C₃-Alkylcarbonylamino- steht,
oder für C₁-C₃-Alkoxy-, welches mit Morpholinyl-, Pyrrolidinyl-, Piperazinyl- oder Pyridinyl-substituiert sein kann, worin das Piperazinyl- oder Pyridinyl- selbst mit C₁-C₃-Alkyl- substituiert sein kann,
R^{1a} nicht für für Wasserstoff, Halogen, Cyano, Carboxy, Amino oder Aminosulfonyl, oder für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino-, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonylrest steht,
sowie deren Polymorphe, Enantiomere, Diastereomere, Razemate, Tautomere, Solvate, physiologisch verträglichen Salze und Solvate dieser Salze.

Ausserordentlich bevorzugt sind solche Verbindungen der allgemeinen Formel (I-A), in der
- X: für ein Sauerstoffatom steht,
- A: für einen Phenyl- oder Pyridylring steht,
- R^{1a}: für Wasserstoff oder Chlor steht,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, C₁-C₆-Alkylcarbonyl-, C₁-C₆-Alkylsulfonyl-, Halogen-C₁-C₆-Alkyl-, Halogen-C₁-C₆-Alkoxy-, C₃-C₁₀-Cycloalkyl-, Phenyl-, Halophenyl-, Phenyl-C₁-C₃-Alkyl-, Pyridinyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Halogen, C₁-C₃-Alkyl und/oder C₁-C₃-Alkoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, C₁-C₃-Alkylcarbonyl-, -C(=O)NH₂, C₁-C₃-Alkylaminocarbonyl-, C₃-C₆-Cycloalkylaminocarbonyl-, C₁-C₃-Alkylsulfinyl-, C₁-C₃-Alkylsulfonyl, -S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl-, und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl- und/oder C₁-C₃-Alkoxy-, und
- n: für 0 oder 1 steht, und
- R^{1b}: für Fluor steht, und
- R²: für Methyl steht, und
- R³: für Methyl oder C₁-C₃-Alkylamino- steht, und
- R⁴: für Wasserstoff, Chlor, Cyano, Methoxy-, Ethoxy- oder Difluormethoxy- steht, und
- R⁵: für Chlor, Hydroxy, Amino, C₁-C₆-Alkyl-, Methoxy-, Ethoxy-, Difluormethoxy-, Trifluormethoxy-, C₁-C₃-Alkylcarbonylamino-, Cyclopropyl-, Pyridinyl-, Morpholinyl-, Piperidinyl-, gegebenenfalls ein- oder mehrfach mit Methyl substituiertes Piperazinyl-, Pyrazolyl-, oder für gegebenenfalls mit C₁-C₃-Alkylaminosulfonyl- substituiertes Phenyl, steht, oder für C₁-C₃-Alkoxy-steht, welches mit Morpholinyl-, Pyrrolidinyl-, Piperazinyl- oder Pyridinyl-substituiert sein kann, worin das Piperazinyl- oder Pyridinyl- selbst mit C₁-C₃-Alkyl- substituiert sein kann, und
- R⁶ und R⁷: unabhängig voneinander für Wasserstoff, C₁-C₃-Alkyl-, Cyclopropyl-, oder Di-C₁-C₃-Alkyl-Amino-C₁-C₃-Alkyl- stehen, und
- R⁸: für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
- R⁹: für C₁-C₃-Alkyl- steht,
mit der Maßgabe, dass,
wenn A für Phenyl steht, R⁴ für Wasserstoff, Chlor, Methoxy-, Ethoxy- oder Difluormethoxysteht, und R⁵ für Chlor, Hydroxy, Amino, C₁-C₆-Alkyl-, Methoxy-, Ethoxy-, Difluormethoxy-, Trifluormethoxy-, C₁-C₃-Alkylcarbonylamino- steht, oder für C₁-C₃-Alkoxy- steht, welches mit Morpholinyl-, Pyrrolidinyl-, Piperazinyl- oder Pyridinyl- substituiert sein kann, worin das Piperazinyl- oder Pyridinyl- selbst mit C₁-C₃-Alkyl- substituiert sein kann,
R^{1a} nicht für Wasserstoff oder Chlor steht,
sowie deren Polymorphe, Enantiomere, Diastereomere, Razemate, Tautomere, Solvate, physiologisch verträglichen Salze und Solvate dieser Salze.

Besonders interessante Verbindungen der allgemeinen Formel (I-A) sind solche, in der
- X: für ein Sauerstoffatom steht,
- A: für einen Phenyl- oder 3-Pyridylring steht,
- R^{1a}: für Wasserstoff, Chlor, Morpholinyl-, Dioxidothiomorpholinyl- oder Tetrazolyl- steht, oder für gegebenfalls ein- oder mehrfach, gleich oder verschieden mit Fluor, Chlor, Oxo, Hydroxy, Cyano, Nitro, C₁-C₃-Alkyl-, C₁-C₃-Alkoxy-, C₁-C₃-Alkoxy-C₁-C₃-Alkyl-, Hydroxy-C₁-C₃-Alkyl-, C₃-C₆-Cycloalkyl-, Phenyl-C₁-C₃-Alkyl-, Trifluormethyl-, Trifluormethoxy-, Phenyl-, Fluorphenyl-, Pyridinyl-, -C(=O)-NR⁶R⁷, -C(=O)-R⁸, -S(=O)₂-NR⁶R⁷, -S(=O)-R⁹, -S(=O)₂-R⁹ und/oder -NH-S(=O)₂-R⁹ substituiertes Isoxazolyl-, Pyrazolyl-, Thienyl-, Thiazolyl-, Imidazolyl-, Triazolyl-, Azinanyl-, Piperazinyl-, Pyrrolyl-, Pyrrolidinyl-, Piperidinyl-, Piperazinyl-, Diazepanyl-, Oxazinanyl-, Oxazolidinyl-, Morpholinyl-, Azolidinyl-, Azetidinyl-, Phenyl-, Pyridinyl- oder Pyrimidinyl- steht, oder für Phenyl steht, welches mit Morpholinyl- oder Halothienyl- substituiert ist,
- n: für 0 oder 1 steht,
- R^{1b}: für Fluor,
- R²: für Methyl steht,
- R³: für Methyl oder C₁-C₃-Alkylamino- steht, und
- R⁴: für Wasserstoff, Chlor, Cyano, Methoxy-, Ethoxy- oder Difluormethoxysteht, und
- R⁵: für Chlor, Hydroxy, Amino, C₁-C₆-Alkyl-, Methoxy-, Ethoxy-, Difluormethoxy-, Trifluormethoxy-, C₁-C₃-Alkylcarbonylamino-, Cyclopropyl-, Pyridinyl-, Morpholinyl-, Piperidinyl-, gegebenenfalls ein- oder mehrfach mit Methyl substituiertes Piperazinyl-, Pyrazolyl-, oder für gegebenenfalls mit C₁-C₃-Alkylaminosulfonyl- substituiertes Phenyl, steht, oder für C₁-C₃-Alkoxy- steht, welches mit Morpholinyl-, Pyrrolidinyl-, Piperazinyl- oder Pyridinyl- substituiert sein kann, und das Piperazinyl- oder Pyridinyl- selbst mit C₁-C₃-Alkyl- substituiert sein kann, steht, und
- R⁶ und R⁷: unabhängig voneinander für Wasserstoff, C₁-C₃-Alkyl-, Cyclopropyl- oder Di-C₁-C₃-Alkyl-Amino-C₁-C₃-Alkyl- steht, und
- R⁸: für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
- R⁹: für C₁-C₃-Alkyl- steht,
mit der Maßgabe, dass,
wenn A für Phenyl steht, R⁴ für Wasserstoff, Chlor, Methoxy-, Ethoxy- oder Difluormethoxysteht und R⁵ für Chlor, Hydroxy, Amino, C₁-C₆-Alkyl-, Methoxy-, Ethoxy-, Difluormethoxy-, Trifluormethoxy-, C₁-C₃-Alkylcarbonylamino- steht,
oder für C₁-C₃-Alkoxy-, welches mit Morpholinyl-, Pyrrolidinyl-, Piperazinyl- oder Pyridinyl-substituiert sein kann, und das Piperazinyl- oder Pyridinyl- selbst mit C₁-C₃-Alkyl- substituiert sein kann,
R^{1a} nicht für Wasserstoff oder Chlor steht,
sowie deren Polymorphe, Enantiomere, Diastereomere, Razemate, Tautomere, Solvate, physiologisch verträglichen Salze und Solvate dieser Salze.

Ganz besonders interessant sind solche Verbindungen der allgemeinen Formel (I-A), in der
- X: für ein Sauerstoffatom steht,
- A: für einen Phenyl- oder 3-Pyridylring steht,
- R^{1a}: für Wasserstoff, Chlor, Morpholinyl-, Dioxidothiomorpholinyl- oder Tetrazolyl- steht, oder für gegebenfalls ein- oder mehrfach, gleich oder verschieden mit Fluor, Chlor, Oxo, Hydroxy, Cyano, Nitro, C₁-C₃-Alkyl-, C₁-C₃-Alkoxy-, C₁-C₃-Alkoxy-C₁-C₃-Alkyl-, Hydroxy-C₁-C₃-Alkyl-, Cyclopropyl-, Phenyl-C₁-C₃-Alkyl-, Trifluormethyl-, Trifluormethoxy-, Phenyl-, Fluorphenyl-, Pyridinyl-,-C(=O)-NR⁶R⁷, -C(=O)-R⁸, -S(=O)₂-NR⁶R⁷, -S(=O)-R⁹, -S(=O)₂-R⁹ und/oder -NH-S(=O)₂-R⁹ substituiertes Isoxazolyl-, Pyrazolyl-, Thienyl-, Thiazolyl-, Imidazolyl-, Triazolyl-, Azinanyl-, Piperazinyl-, Pyrrolyl-, Pyrrolidinyl-, Piperidinyl-, Piperazinyl-, Diazepanyl-, Oxazinanyl-, Oxazolidinyl-, Morpholinyl-, Azolidinyl-, Azetidinyl-, Phenyl-, Pyridinyl- oder Pyrimidinyl- steht, oder für Phenyl steht, welches mit Morpholinyl- oder Halothienyl- substituiert ist,
- n: für 0 oder 1 steht,
- R^{1b}: für Fluor,
- R²: für Methyl- steht,
- R³: für Methylamino- steht,
- R⁴: für Wasserstoff, Chlor, Cyano, Methoxy-, Ethoxy- oder Difluormethoxysteht, und
- R⁵: für Chlor, Hydroxy, Amino, C₁-C₆-Alkyl-, Methoxy-, Ethoxy-, Difluormethoxy-, Trifluormethoxy-, C₁-C₃-Alkylcarbonylamino-, Cyclopropyl-, Pyridinyl-, Morpholinyl-, Piperidinyl-, gegebenenfalls ein- oder mehrfach mit Methyl substituiertes Piperazinyl-, Pyrazolyl-, oder für gegebenenfalls mit C₁-C₃-Alkylaminosulfonyl- substituiertes Phenyl, steht, oder für C₁-C₃-Alkoxy- steht, welches mit Morpholinyl-, Pyrrolidinyl-, Piperazinyl- oder Pyridinyl- substituiert sein kann, worin das Piperazinyl- oder Pyridinyl- selbst mit C₁-C₃-Alkyl- substituiert sein kann, steht, und
- R⁶ und R⁷: unabhängig voneinander für Wasserstoff, C₁-C₃-Alkyl-, Cyclopropyl- oder Di-C₁-C₃-Alkyl-Amino-C₁-C₃-Alkyl- steht, und
- R⁸: für Hydroxy, C₁-C₃-Alkyl-, Hydroxy-C₁-C₃-Alkyl-, Trifluormethyl-, Pyrrolidinyl-, Morpholinyl- oder Piperidinyl- steht,und
- R⁹: für C₁-C₃-Alkyl- steht,
mit der Maßgabe, dass,
wenn A für Phenyl steht, R⁴ für Wasserstoff, Chlor, Methoxy-, Ethoxy- oder Difluormethoxysteht und R⁵ für Chlor, Hydroxy, Amino, C₁-C₆-Alkyl-, Methoxy-, Ethoxy-, Difluormethoxy-, Trifluormethoxy-, C₁-C₃-Alkylcarbonylamino- steht,
oder für C₁-C₃-Alkoxy-, welches mit Morpholinyl-, Pyrrolidinyl-, Piperazinyl- oder Pyridinyl-substituiert sein kann, worin das Piperazinyl- oder Pyridinyl- selbst mit C₁-C₃-Alkyl-substituiert sein kann,
R^{1a} nicht für Wasserstoff oder Chlor steht,
sowie deren Polymorphe, Enantiomere, Diastereomere, Razemate, Tautomere, Solvate, physiologisch verträglichen Salze und Solvate dieser Salze,

Die Maßgabe umfasst aber nicht solche Verbindungen der allgemeinen Formel (I-A), in der A für Phenyl steht und R⁴ für Wasserstoff oder Chlor steht und R⁵ für Trifluormethoxy steht, und R^{1a} für Chlor steht,
und solche Verbindungen der allgemeinen Formel (I-A), in der
A für Phenyl steht und R⁴ für Wasserstoff steht und R⁵ für C₁-C₃-Alkoxy, welches mit Morpholinyl-, Pyrrolidinyl-, Piperazinyl- oder Pyridyl- substituiert sein kann, und das Piperazinyl-oder Pyridinyl- selbst mit C₁-C₃-Alkyl- substituiert sein kann, steht, und R^{1a} für Chlor steht.

Hierunter zu verstehen sind zum Beispiel Verbindungen der hergestellten Ausführungsbeispiele Nr. 1; 15; 32; 33; 164; 164.1; 164.2; 165; 166 und 167.

Gleichfalls interessant sind auch solche Verbindungen der allgemeinen Formel (I-A), in welcher
- X: für ein Sauerstoff- oder Schwefelatom steht, und
- A: für einen monozyklischen Heteroarylring mit 5 oder 6 Ringatomen oder für einen Phenylring steht, und
- R^{1a}: steht
a) für Wasserstoff, Halogen, Cyano, Carboxy, Amino oder Aminosulfonyl, oder
b) für einen C₁-C₆-Alkoxy-, C₁-C₃-Alkoxy-C₁-C₃-Alkyl-, C₁-C₃-Alkoxy-C₁-C₃-Alkoxy-, C₁-C₆-Alkylamino-, C₁-C₆-Alkylcarbonylamino, C₁-C₆-Alkylamino-C₁-C₆-Alkyl-, *N*-Heterocyclyl-C₁-C₆-Alkyl-, *N*-Heterocyclyl-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonylrest, oder
c) für einen monozyklischen Heterocyclylrest mit 3 bis 8 Ringatomen, der ein- oder mehrfach, gleich oder verschieden substituiert sein kann mit Hydroxy, Halogen, Amino, Oxo, Carboxy und/oder einem C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen, oder
d) für einen monozyklischen Heteroarylrest mit 5 oder 6 Ringatomen, der ein- oder mehrfach, gleich oder verschieden substituiert sein kann mit Halogen, Hydroxy, Amino, Cyano, Nitro, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen und/oder einem monocyclischen Heteroarylrest mit 5 oder 6 Ringatomen, oder
e) für einen Phenylrest, der ein- oder mehrfach, gleich oder verschieden substituiert sein kann mit Halogen, Amino, Hydroxy, Cyano, Nitro, Carboxy und/oder einem C₁-C₆-Alkyl, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, Hydroxy-C₁-C₆-Alkyl-, C₃-C₁₀-Cycloalkyl- und/oder einem monocyclischen Heterocyclylrest mit 3 bis 8 Ringatomen, und
- R^{1b}: für Halogen, Hydroxy, Cyano, Nitro und/oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen steht, und
- n: 0-2, und
- R²: für einen C₁-C₃-Alkyl- oder Trifluormethyl- oder einen C₃- oder C₄-Cycloalkylrest steht, und
- R³: für einen Cyclopropyl-, C₁-C₃-Alkyl-, C₁-C₃-Alkoxy-, Amino-, Cyclopropylamino- oder einen C₁-C₃-Alkylaminorest steht, und
- R⁴, R⁵: stehen unabhängig voneinander
i) für Wasserstoff, Hydroxy, Amino, Aminocarbonyl, Fluor, Chlor, Brom oder einen Trifluormethyl-, C₁-C₆-Alkyl-, C₁-C₆-Alkoxy-, C₁-C₆-Alkylamino-, C₁-C₆-Alkylcarbonylamino- oder C₁-C₆-Alkylaminocarbonylrest, wobei der Alkylteil eines Restes unter i) ein- oder mehrfach, gleich oder verschieden substituiert sein kann mit Amino, Fluor, Hydroxy, Carboxy oder einem Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, oder einem monocyclischen Heterocyclylrest mit 3 bis 6 Ringatomen, oder
ii) für Cyano oder Nitro oder
iii) für einen C₁-C₆-Alkylaminosulfonyl-, Halogen-C₁-C₆-Alkyl- oderHalogen-C₁-C₆-Alkoxyrest, wobei der Alkylteil eines Restes unter iii) ein- oder mehrfach, gleich oder verschieden substituiert sein kann mit Amino, Fluor, Hydroxy, Carboxy oder einem Hydroxy-C₁-C₆-Alkyl-, C₁-C₆-Alkoxy-, C₁-C₆-Alkylamino, Amino-C₁-C₆-Alkyl-, C₁-C₆-Alkoxy-C₁-C₆-Alkyl- oder einem monocyclischen Heterocyclylrest mit 3 bis 6 Ringatomen, oder
iv) für einen C₃-C₁₀-Cycloalkylrest, der gegebenenfalls ein- oder mehrfach, gleich oder verschieden substituiert sein kann mit Halogen, Amino, Hydroxy, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl- oder Halogen-C₁-C₆-Alkoxyrest, oder
v) für einen monozyklischen Heteroarylrest mit 5 oder 6 Ringatomen, der ein- oder mehrfach, gleich oder verschieden substituiert sein kann mit Halogen, Amino, Hydroxy, Cyano, Nitro, Carboxy und/oder einem C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen, oder
vi) für einen monozyklischen Heterocyclylrest mit 3 bis 8 Ringatomen, der gegebenenfalls ein- oder mehrfach, gleich oder verschieden substituiert sein kann mit Halogen, Amino, Hydroxy, Cyano, Oxo, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Hydroxy-C₁-C₆-Alkyl-, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen, oder
vii) für einen Phenylrest, der gegebenenfalls ein- oder mehrfach, gleich oder verschieden substituiert sein kann mit Halogen, Amino, Hydroxy, Cyano, Nitro, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl, C₁-C₆-Alkylaminosulfonyl-, C₁-C₆-Alkylamino-C₁-C₆-Alkyl, Hydroxy-C₁-C₆-Alkyl-, Halogen-C₁-C₆-Alkyl- oder Halogen-C₁-C₆-Alkoxy, C₃-C₁₀-Cycloalkyl-und/oder einem monocyclischen Heterocyclylrest mit 3 bis 8 Ringatomen,
mit der Maßgabe, dass, wenn A für einen Phenylrest und R⁴ und R⁵ für einen Rest unter i) stehen, R^{1a} für einen Rest unter c), d) oder e) steht, sowie deren, Enantiomere, Diastereomere, Razemate, Tautomere, Solvate, physiologisch verträglichen Salze und Solvate dieser Salze.

Eine bevorzugte Untergruppe davon sind Verbindungen der Formel (I-A),
in welcher
- X: für ein Sauerstoff- oder Schwefelatom steht, und
- A: für einen monozyklischen Heteroarylring mit 5 oder 6 Ringatomen oder für einen Phenylring steht, und
- R^{1a}: steht
a) für einen monozyklischen Heterocyclylrest mit 3 bis 8 Ringatomen, der ein- oder mehrfach, gleich oder verschieden substituiert sein kann mit Hydroxy, Halogen, Amino, Oxo, Carboxy und/oder einem C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen, oder
b) für einen monzyklischen Heteroarylrest mit 5 oder 6 Ringatomen, der ein- oder mehrfach, gleich oder verschieden substituiert sein kann mit Halogen, Hydroxy, Amino, Cyano, Nitro, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen und/oder einem monocyclischen Heteroarylrest mit 5 oder 6 Ringatomen, oder
c) für einen Phenylrest, der ein- oder mehrfach, gleich oder verschieden substituiert sein kann mit Halogen, Amino, Hydroxy, Cyano, Nitro, Carboxy und/oder einem C₁-C₆-Alkyl, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, Hydroxy-C₁-C₆-Alkyl-, C₃-C₁₀-Cycloalkyl- und/oder einem monocyclischen Heterocyclylrest mit 3 bis 8 Ringatomen, und
- R^{1b}: für Halogen, Hydroxy, Cyano, Nitro und/oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen steht, und
- n: 0-2, und
- R²: für einen C₁-C₃-Alkyl- oder Trifluormethyl- oder einen C₃- oder C₄-Cycloalkylrest steht, und
- R³: für einen Cyclopropyl-, C₁-C₃-Alkyl-, C₁-C₃-Alkoxy-, Amino-, Cyclopropylamino- oder einen C₁-C₃-Alkylaminorest steht, und
- R⁴, R⁵: stehen unabhängig voneinander
i) für Wasserstoff, Hydroxy, Amino, Aminocarbonyl, Fluor, Chlor, Brom oder einen Trifluormethyl-, C₁-C₆-Alkyl-, C₁-C₆-Alkoxy-, C₁-C₆-Alkylamino-, C₁-C₆-Alkylcarbonylamino- oder C₁-C₆-Alkylaminocarbonylrest, wobei der Alkylteil eines Restes unter i) ein- oder mehrfach, gleich oder verschieden substituiert sein kann mit Amino, Fluor, Hydroxy, Carboxy oder einem Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, oder einem monocyclischen Heterocyclylrest mit 3 bis 6 Ringatomen, oder
ii) für Cyano oder Nitro oder
iii) für einen C₁-C₆-Alkylaminosulfonyl-, Halogen-C₁-C₆-Alkyl- oder Halogen-C₁-C₆-Alkoxyrest, wobei der Alkylteil eines Restes unter iii) ein- oder mehrfach, gleich oder verschieden substituiert sein kann mit Amino, Fluor, Hydroxy, Carboxy oder einem Hydroxy-C₁-C₆-Alkyl-, C₁-C₆-Alkoxy-, C₁-C₆-Alkylamino, Amino-C₁-C₆-Alkyl-, C₁-C₆-Alkoxy-C₁-C₆-Alkyl- oder einem monocyclischen Heterocyclylrest mit 3 bis 6 Ringatomen, oder
iv) für einen C₃-C₁₀-Cycloalkylrest, der ein- oder mehrfach, gleich oder verschieden substituiert sein kann mit Halogen, Amino, Hydroxy, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl- oder Halogen-C₁-C₆-Alkoxyrest, oder
v) für einen monzyklischen Heteroarylrest mit 5 oder 6 Ringatomen, der ein- oder mehrfach, gleich oder verschieden substituiert sein kann mit Halogen, Amino, Hydroxy, Cyano, Nitro, Carboxy und/oder einem C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen, oder
vi) für einen monozyklischen Heterocyclylrest mit 3 bis 8 Ringatomen, der ein- oder mehrfach, gleich oder verschieden substituiert sein kann mit Halogen, Amino, Hydroxy, Cyano, Oxo, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Hydroxy-C₁-C₆-Alkyl-, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen, oder
vii) für einen Phenylrest, der ein- oder mehrfach, gleich oder verschieden substituiert sein kann mit Halogen, Amino, Hydroxy, Cyano, Nitro, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl, C₁-C₆-Alkylaminosulfonyl-, C₁-C₆-Alkylamino-C₁-C₆-Alkyl, Hydroxy-C₁-C₆-Alkyl-, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen,
sowie deren Enantiomere, Diastereomere, Razemate, Tautomere, Solvate, physiologisch verträglichen Salze und Solvate dieser Salze.

Eine weiter Untergruppe davon sind Verbindungen der Formel (I-A), in welcher
- X: für ein Sauerstoff- oder Schwefelatom steht, und
- A: für einen monozyklischen Heteroarylring mit 5 oder 6 Ringatomen oder für einen Phenylring steht, und
- R^{1a}: steht
a) für Wasserstoff, Halogen, Cyano, Carboxy, Amino oder Aminosulfonyl, oder
b) für einen C₁-C₆-Alkoxy-, C₁-C₃-Alkoxy-C₁-C₃-Alkyl-, C₁-C₃-Alkoxy-C₁-C₃-Alkoxy-, C₁-C₆-Alkylamino-, C₁-C₆-Alkylcarbonylamino, C₁-C₆-Alkylamino-C₁-C₆-Alkyl-, *N*-Heterocyclyl-C₁-C₆-Alkyl-, *N*-Heterocyclyl-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonylrest, oder
c) für einen monozyklischen Heterocyclylrest mit 3 bis 8 Ringatomen, der ein- oder mehrfach, gleich oder verschieden substituiert sein kann mit Hydroxy, Halogen, Amino, Oxo, Carboxy und/oder einem C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen, oder
d) für einen monzyklischen Heteroarylrest mit 5 oder 6 Ringatomen, der ein- oder mehrfach, gleich oder verschieden substituiert sein kann mit Halogen, Hydroxy, Amino, Cyano, Nitro, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen und/oder einem monocyclischen Heteroarylrest mit 5 oder 6 Ringatomen, oder
e) für einen Phenylrest, der ein- oder mehrfach, gleich oder verschieden substituiert sein kann mit Halogen, Amino, Hydroxy, Cyano, Nitro, Carboxy und/oder einem C₁-C₆-Alkyl, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, Hydroxy-C₁-C₆-Alkyl-, C₃-C₁₀-Cycloalkyl- und/oder einem monocyclischen Heterocyclylrest mit 3 bis 8 Ringatomen, und
- R^{1b}: für Halogen, Hydroxy, Cyano, Nitro und/oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen steht, und
- n: 0-2, und
- R²: für einen C₁-C₃-Alkyl- oder Trifluormethyl- oder einen C₃- oder C₄-Cycloalkylrest steht, und
- R³: für einen Cyclopropyl-, C₁-C₃-Alkyl-, C₁-C₃-Alkoxy-, Amino-, Cyclopropylamino-oder einen C₁-C₃-Alkylaminorest steht, und
- R⁴, R⁵: stehen unabhängig voneinander
i) für Cyano oder Nitro oder
ii) für einen C₁-C₆-Alkylaminosulfonyl-, Halogen-C₁-C₆-Alkyl- oder Halogen-C₁-C₆-Alkoxyrest, wobei der Alkylteil eines Restes unter ii) ein- oder mehrfach, gleich oder verschieden substituiert sein kann mit Amino, Fluor, Hydroxy, Carboxy oder einem Hydroxy-C₁-C₆-Alkyl-, C₁-C₆-Alkoxy-, C₁-C₆-Alkylamino, Amino-C₁-C₆-Alkyl-, C₁-C₆-Alkoxy-C₁-C₆-Alkyl- oder einem monocyclischen Heterocyclylrest mit 3 bis 6 Ringatomen, oder
iii) für einen C₃-C₁₀-Cycloalkylrest, der ein- oder mehrfach, gleich oder verschieden substituiert sein kann mit Halogen, Amino, Hydroxy, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl- oder Halogen-C₁-C₆-Alkoxyrest, oder
iv) für einen monzyklischen Heteroarylrest mit 5 oder 6 Ringatomen, der ein- oder mehrfach, gleich oder verschieden substituiert sein kann mit Halogen, Amino, Hydroxy, Cyano, Nitro, Carboxy und/oder einem C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen, oder
v) für einen monozyklischen Heterocyclylrest mit 3 bis 8 Ringatomen, der ein- oder mehrfach, gleich oder verschieden substituiert sein kann mit Halogen, Amino, Hydroxy, Cyano, Oxo, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Hydroxy-C₁-C₆-Alkyl-, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen, oder
vi) für einen Phenylrest, der ein- oder mehrfach, gleich oder verschieden substituiert sein kann mit Halogen, Amino, Hydroxy, Cyano, Nitro, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl, C₁-C₆-Alkylaminosulfonyl-, C₁-C₆-Alkylamino-C₁-C₆-Alkyl, Hydroxy-C₁-C₆-Alkyl-, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen,
sowie deren Enantiomere, Diastereomere, Razemate, Tautomere, Solvate, physiologisch verträglichen Salze und Solvate dieser Salze.

Eine weitere Untergruppe davon sind auch Verbindungen der Formel (I-A), in welcher
- X: für ein Sauerstoffatom steht, und
- A: für einen monozyklischen Heteroarylring mit 5 oder 6 Ringatomen oder für einen Phenylring steht, und
- R^{1a}: steht
a) für Wasserstoff oder Halogen, oder
b) für einen monozyklischen Heterocyclylrest mit 3 bis 8 Ringatomen, der ein- oder mehrfach, gleich oder verschieden substituiert sein kann mit Hydroxy, Fluor, Oxo, Carboxy und/oder einem C₁-C₆-Alkyl-, Hydroxy-C₁-C₆-Alkyl-, Halogen-C₁-C₆-Alkyl- oder Halogen-C₁-C₆-Alkoxyrest, oder
c) für einen monzyklischen Heteroarylrest mit 5 oder 6 Ringatomen, der ein- oder mehrfach, gleich oder verschieden substituiert sein kann mit Halogen, Hydroxy, Carboxy und/oder einem C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heteroarylrest mit 5 oder 6 Ringatomen, oder
d) für einen Phenylrest, der ein- oder mehrfach, gleich oder verschieden substituiert sein kann mit Halogen, Hydroxy, Carboxy und/oder einem C₁-C₆-Alkyl, C₁-C₆-Alkoxy-, Halogen-C₁-C₆-Alkyl- oder Halogen-C₁-C₆-Alkoxyrest, und
- R^{1b}: für Halogen, Cyano und/oder für einen C₁-C₆-Alkyl-, C₁-C₆-Alkoxy-, Halogen-C₁-C₆-Alkyl- oder Halogen-C₁-C₆-Alkoxyrest, und
- n: 0-2, und
- R²: für einen C₁-C₃-Alkyl- oder Trifluormethylrest steht, und
- R³: für einen C₁-C₃-Alkyl- oder einen C₁-C₃-Alkylaminorest steht, und
- R⁴, R⁵: stehen unabhängig voneinander
i) für Wasserstoff oder Fluor, Chlor, Brom oder für einen C₁-C₆-Alkoxyrest, wobei der Alkylteil des C₁-C₆-Alkoxyrestes ein- oder mehrfach, gleich oder verschieden substituiert sein kann mit einem monocyclischen Heterocyclylrest mit 3 bis 6 Ringatomen, oder
ii) für einen Halogen-C₁-C₆-Alkyl- oder Halogen-C₁-C₆-Alkoxyrest, oder
iii) für einen C₃-C₁₀-Cycloalkylrest, der ein- oder mehrfach, gleich oder verschieden substituiert sein kann mit Halogen, Hydroxy, Carboxy und/oder einem C₁-C₆-Alkylrest-, oder
iv) für einen monzyklischen Heteroarylrest mit 5 oder 6 Ringatomen, der ein- oder mehrfach, gleich oder verschieden substituiert sein kann mit Halogen, Hydroxy, oder einem C₁-C₆-Alkyl-, C₁-C₆-Alkoxy- oder Hydroxy-C₁-C₆-Alkylrest, oder
v) für einen monozyklischen Heterocyclylrest mit 3 bis 8 Ringatomen, der ein- oder mehrfach, gleich oder verschieden substituiert sein kann mit Halogen, Hydroxy, Carboxy und/oder einem C₁-C₆-Alkylrest-, oder
vi) für einen Phenylrest, der ein- oder mehrfach, gleich oder verschieden substituiert sein kann mit Halogen, Cyano, Carboxy und/oder einem C₁-C₆-Alkyl-, C₁-C₆-Alkoxy, Hydroxy-C₁-C₆-Alkyl- oder C₁-C₆-Alkylaminosulfonylrest,
mit der Maßgabe, dass, wenn A für einen Phenylrest und R⁴ und R⁵ für einen Rest unter i) stehen, R^{1a} für einen Rest unter b), c) oder d) steht,
sowie deren Enantiomere, Diastereomere, Razemate, Tautomere, Solvate, physiologisch verträglichen Salze und Solvate dieser Salze.

Eine weitere Untergruppe davon sind auch Verbindungen der Formel (I-A), in welcher
- X: für ein Sauerstoffatom steht, und
- A: für einen monozyklischen Heteroarylring mit 5 oder 6 Ringatomen oder für einen Phenylring steht, und
- R^{1a}: steht
a) für einen monozyklischen Heterocyclylrest mit 3 bis 8 Ringatomen, der ein- oder mehrfach, gleich oder verschieden substituiert sein kann mit Hydroxy, Fluor, Oxo, Carboxy und/oder einem C₁-C₆-Alkyl-, Hydroxy-C₁-C₆-Alkyl-, Halogen-C₁-C₆-Alkyl- oder Halogen-C₁-C₆-Alkoxyrest, oder
c) für einen monzyklischen Heteroarylrest mit 5 oder 6 Ringatomen, der ein- oder mehrfach, gleich oder verschieden substituiert sein kann mit Halogen, Hydroxy, Carboxy und/oder einem C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heteroarylrest mit 5 oder 6 Ringatomen, oder
d) für einen Phenylrest, der ein- oder mehrfach, gleich oder verschieden substituiert sein kann mit Halogen, Hydroxy, Carboxy und/oder einem C₁-C₆-Alkyl, C₁-C₆-Alkoxy-, Halogen-C₁-C₆-Alkyl- oder Halogen-C₁-C₆-Alkoxyrest, und
- R^{1b}: für Halogen, Cyano und/oder für einen C₁-C₆-Alkyl-, C₁-C₆-Alkoxy-, Halogen-C₁-C₆-Alkyl- oder Halogen-C₁-C₆-Alkoxyrest, und
- n: 0-2, und
- R²: für einen C₁-C₃-Alkyl- oder Trifluormethylrest steht, und
- R³: für einen C₁-C₃-Alkyl- oder einen C₁-C₃-Alkylaminorest steht, und
- R⁴, R⁵: stehen unabhängig voneinander
i) für Wasserstoff oder Fluor, Chlor, Brom oder für einen C₁-C₆-Alkoxyrest, wobei der Alkylteil des C₁-C₆-Alkoxyrestes ein- oder mehrfach, gleich oder verschieden substituiert sein kann mit einem monocyclischen Heterocyclylrest mit 3 bis 6 Ringatomen, oder
ii) für einen Halogen-C₁-C₆-Alkyl- oder Halogen-C₁-C₆-Alkoxyrest, oder
iii) für einen C₃-C₁₀-Cycloalkylrest, der ein- oder mehrfach, gleich oder verschieden substituiert sein kann mit Halogen, Hydroxy, Carboxy und/oder einem C₁-C₆-Alkylrest-, oder
iv) für einen monzyklischen Heteroarylrest mit 5 oder 6 Ringatomen, der ein- oder mehrfach, gleich oder verschieden substituiert sein kann mit Halogen, Hydroxy, oder einem C₁-C₆-Alkyl-, C₁-C₆-Alkoxy- oder Hydroxy-C₁-C₆-Alkylrest, oder
v) für einen monozyklischen Heterocyclylrest mit 3 bis 8 Ringatomen, der ein- oder mehrfach, gleich oder verschieden substituiert sein kann mit Halogen, Hydroxy, Carboxy und/oder einem C₁-C₆-Alkylrest-, oder
vi) für einen Phenylrest, der ein- oder mehrfach, gleich oder verschieden substituiert sein kann mit Halogen, Cyano, Carboxy und/oder einem C₁-C₆-Alkyl-, C₁-C₆-Alkoxy, Hydroxy-C₁-C₆-Alkyl-, oder C₁-C₆-Alkylaminosulfonylrest,
sowie deren Enantiomere, Diastereomere, Razemate, Tautomere, Solvate, physiologisch verträglichen Salze und Solvate dieser Salze.

Eine weitere Untergruppe davon sind auch Verbindungen der Formel (I-A), in welcher
- X: für ein Sauerstoffatom steht, und
- A: für einen monozyklischen Heteroarylring mit 5 oder 6 Ringatomen oder für einen Phenylring steht, und
- R^{1a}: steht
a) für Wasserstoff oder Halogen, oder
b) für einen monozyklischen Heterocyclylrest mit 3 bis 8 Ringatomen, der ein- oder mehrfach, gleich oder verschieden substituiert sein kann mit Hydroxy, Fluor, Oxo, Carboxy und/oder einem C₁-C₆-Alkyl-, Hydroxy-C₁-C₆-Alkyl-, Halogen-C₁-C₆-Alkyl- oder Halogen-C₁-C₆-Alkoxyrest, oder
c) für einen monzyklischen Heteroarylrest mit 5 oder 6 Ringatomen, der ein- oder mehrfach, gleich oder verschieden substituiert sein kann mit Halogen, Hydroxy, Carboxy und/oder einem C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heteroarylrest mit 5 oder 6 Ringatomen, oder
d) für einen Phenylrest, der ein- oder mehrfach, gleich oder verschieden substituiert sein kann mit Halogen, Hydroxy, Carboxy und/oder einem C₁-C₆-Alkyl, C₁-C₆-Alkoxy-, Halogen-C₁-C₆-Alkyl- oder Halogen-C₁-C₆-Alkoxyrest, und
- R^{1b}: für Halogen, Cyano und/oder für einen C₁-C₆-Alkyl-, C₁-C₆-Alkoxy-, Halogen-C₁-C₆-Alkyl- oder Halogen-C₁-C₆-Alkoxyrest, und
- n: 0-2, und
- R²: für einen C₁-C₃-Alkyl- oder Trifluormethylrest steht, und
- R³: für einen C₁-C₃-Alkyl- oder einen C₁-C₃-Alkylaminorest steht, und
- R⁴, R⁵: stehen unabhängig voneinander
i) für einen Halogen-C₁-C₆-Alkyl- oder Halogen-C₁-C₆-Alkoxyrest, oder
ii) für einen C₃-C₁₀-Cycloalkylrest, der ein- oder mehrfach, gleich oder verschieden substituiert sein kann mit Halogen, Hydroxy, Carboxy und/oder einem C₁-C₆-Alkylrest-, oder
iii) für einen monzyklischen Heteroarylrest mit 5 oder 6 Ringatomen, der ein- oder mehrfach, gleich oder verschieden substituiert sein kann mit Halogen, Hydroxy, oder einem C₁-C₆-Alkyl-, C₁-C₆-Alkoxy- oder Hydroxy-C₁-C₆-Alkylrest, oder
iv) für einen monozyklischen Heterocyclylrest mit 3 bis 8 Ringatomen, der ein- oder mehrfach, gleich oder verschieden substituiert sein kann mit Halogen, Hydroxy, Carboxy und/oder einem C₁-C₆-Alkylrest-, oder
v) für einen Phenylrest, der ein- oder mehrfach, gleich oder verschieden substituiert sein kann mit Halogen, Cyano, Carboxy und/oder einem C₁-C₆-Alkyl-, C₁-C₆-Alkoxy, Hydroxy-C₁-C₆-Alkyl-, oder C₁-C₆-Alkylaminosulfonylrest,
sowie deren Enantiomere, Diastereomere, Razemate, Tautomere, Solvate, physiologisch verträglichen Salze und Solvate dieser Salze.

Eine weitere Untergruppe davon sind Verbindungen der Formel (I-A), in welcher
- X: für ein Sauerstoffatom steht, und
- A: für einen monozyklischen Heteroarylring mit 6 Ringatomen oder für einen Phenylring steht, und
- R^{1a}: steht
a) für Wasserstoff oder Halogen, oder
b) für einen monozyklischen Heterocyclylrest mit 4 bis 6 Ringatomen, oder
c) für einen monzyklischen Heteroarylrest mit 5 oder 6 Ringatomen, der ein- oder mehrfach, gleich oder verschieden substituiert sein kann mit Halogen, Carboxy und/oder einem C₁-C₆-Alkyl-, C₁-C₆-Alkoxy-C₁-C₆-Alkyl-, C₃-C₁₀-Cycloalkyl- und/oder einem monocyclischen Heteroarylrest mit 6 Ringatomen, oder
d) für einen Phenylrest, der ein- oder mehrfach substituiert sein kann mit Carboxy, und
- n: für 0 steht, und
- R²: für einen C₁-C₃-Alkylrest steht, und
- R³: für einen C₁-C₃-Alkylaminorest steht, und
- R⁴, R⁵: stehen unabhängig voneinander
i) für Wasserstoff, Chlor oder einen C₁-C₆-Alkoxyrest, wobei der Alkylteil des C₁-C₆-Alkoxyrestes ein- oder mehrfach, gleich oder verschieden substituiert sein kann mit einem monocyclischen Heterocyclylrest mit 3 bis 6 Ringatomen, oder
ii) für einen Halogen-C₁-C₆-Alkoxyrest, oder
iii) für einen C₃-C₁₀-Cycloalkylrest, oder
iv) für einen monzyklischen Heteroarylrest mit 6 Ringatomen, oder
v) für einen monozyklischen Heterocyclylrest mit 3 bis 6 Ringatomen, der ein- oder mehrfach, gleich oder verschieden substituiert sein kann mit einem C₁-C₆-Alkylrest, oder
vi) für einen Phenylrest, der ein- oder mehrfach, gleich oder verschieden substituiert sein kann mit einem C₁-C₆-Alkylaminosulfonylrest,
mit der Maßgabe, dass, wenn A für einen Phenylrest und R⁴ und R⁵ für einen Rest unter i) stehen,
R^{1a} für einen Rest unter b), c) oder d) steht,
sowie deren Enantiomere, Diastereomere, Razemate, Tautomere, Solvate, physiologisch verträglichen Salze und Solvate dieser Salze.

Eine weitere Untergruppe davon sind Verbindungen gemäß der Formel (I-A), in welcher
- X: für ein Sauerstoffatom steht, und
- A: für einen Pyridyl- oder für einen Phenylring steht, und
- R^{1a}: steht
a) für Wasserstoff oder Chlor, oder
b) für einen Morpholinylrest, oder
c) für einen Isooxazolyl-, Pyrazolyl-, Tetrazolyl-, Thienyl- oder Pyridinylrest, der ein- oder mehrfach, gleich oder verschieden substituiert sein kann mit Chlor, Carboxy und/oder einem C₁-C₃-Alkyl-, C₁-C₃-Alkoxy-C₁-C₃-Alkyl-, C₃-C₆-Cycloalkyl- und/oder einem Pyridinylrest, oder
d) für einen Phenylrest, der substituiert sein kann mit Carboxy, und
- n: für 0 steht, und
- R²: für einen Methylrest steht, und
- R³: für einen Methylaminorest steht, und
- R⁴: für Wasserstoff, Chlor oder einen C₁-C₃-Alkyoxyrest steht, und
- R⁵: steht
i) für Wasserstoff, Chlor oder einen C₁-C₃-Alkoxyrest, wobei der Alkylteil des C₁-C₃-Alkoxyrestes ein- oder mehrfach, gleich oder verschieden substituiert sein kann mit einem Morpholinyl- oder Pyrrolidinylrest, oder
ii) für einen Trifluormethoxyrest, oder
iii) für einen Cyclopropylrest, oder
iv) für einen Pyridinylrest, oder
v) für einen Morpholinyl-, Piperazinyl- oder Piperidinylrest, der ein- oder mehrfach, gleich oder verschieden substituiert sein kann mit einem C₁-C₃-Alkylrest,
mit der Maßgabe, dass, wenn A für einen Phenylrest und R⁴ und R⁵ für Wasserstoff, Chlor oder einen C₁-C₃-Alkoxyrest stehen, R^{1a} für einen Rest unter b), c) oder d) steht,
sowie deren Enantiomere, Diastereomere, Razemate, Tautomere, Solvate, physiologisch verträglichen Salze und Solvate dieser Salze.

Eine weitere Untergruppe davon sind Verbindungen gemäß der Formel (I-A), in welcher
- X: für ein Sauerstoffatom steht, und
- A: für einen Phenylring steht, und
- R^{1a}: steht
a) für Chlor, oder
b) für einen Morpholinylrest, oder
c) für einen Isooxazolyl-, Pyrazolyl-, Thienyl- oder Pyridinylrest, der ein- oder mehrfach, gleich oder verschieden substituiert sein kann mit Chlor, Carboxy und/oder einem C₁-C₃-Alkyl-, C₁-C₃-Alkoxy-C₁-C₃-Alkyl-, C₃-C₆-Cycloalkyl- und/oder einem Pyridinylrest, und
- n: für 0 steht, und
- R²: für einen C₁-C₃-Alkylrest steht, und
- R³: für einen C₁-C₃-Alkylaminorest steht, und
- R⁴: für Wasserstoff oder einen Methoxyrest steht, und
- R⁵: steht
i) für einen Methoxyrest, oder
ii) für einen Trifluormethoxyrest, oder
iii) für einen Cyclopropylrest, oder
iv) für einen Pyridinylrest, oder
v) für einen Morpholinylrest,
mit der Maßgabe, dass, wenn R⁴ für Wasserstoff oder einen Methoxyrest steht und R⁵ für einen Methoxyrest steht, R^{1a} für einen Rest unter b) oder c) steht,
sowie deren Enantiomere, Diastereomere, Razemate, Tautomere, Solvate, physiologisch verträglichen Salze und Solvate dieser Salze.

In der allgemeinen Formel I-A kann n für 0, 1 oder 2 stehen, wobei (R^{1b})ₙ auch gleichbedeutend stehen kann für R^{1b} und R^{1c}, und wobei R^{1b} und R^{1c} dann unabhängig voneinander für Wasserstoff, Halogen, Hydroxy, Cyano, Nitro und/oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl-Rest und/oder einen monocyclischen Heterocyclylrest mit 3 bis 8 Ringatomen stehen können.

Gegenstand der vorliegenden Erfindung sind somit Verbindungen der allgemeinen Formel (I) 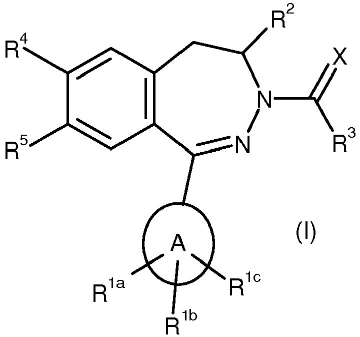 in der
- X: für ein Sauerstoff- oder Schwefelatom steht, und
- A: für einen monozyklischen Heteroarylring mit 5 oder 6 Ringatomen oder für einen Phenylring steht, und
- R^{1a}: für Wasserstoff, Halogen, Cyano, Carboxy, Amino oder Aminosulfonyl steht,
oder
für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino-, C₁-C₆-Alkyl-amino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
oder
für einen monozyklischen Heterocyclylrest mit 3 bis 8 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃**-**C₁₀-Cycloalkyl-**,** Phenyl-, Halophenyl-, Phenyl-C₁-C₆-Alkyl-, Pyridinyl-, -NR⁶C(=O)-R⁹, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder einem monocyclischen Heterocyclylrest mit 3 bis
8 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 3 bis 8 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden, mit Halogen, C₁-C₃-Alkyl und/oder C₁-C₃-Alkoxy-substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, -C(=O)NR⁶R⁷, -C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl- und/oder einem monocyclischen Heterocyclylrest mit 3 bis 8 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl und/oder C₁-C₃-Alkoxy-, und
- R^{1b} und R^{1c}: unabhängig voneinander für Wasserstoff, Halogen, Hydroxy, Cyano, Nitro und/oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-CycloalkylRest und/oder einen monocyclischen Heterocyclylrest mit 3 bis 8 Ringatomen stehen, und
- R²: für einen C₁-C₃-Alkyl- oder Trifluormethyl- oder einen C₃- oder C₄-Cycloalkylrest steht, und
- R³: für Cyclopropyl-, C₁-C₃-Alkyl-, C₁-C₃-Alkoxy-, Amino-, Cyclopropylamino- oder C₁-C₃-Alkylamino- steht, und

- R⁴ und R⁵: unabhängig voneinander stehen für Wasserstoff, Hydroxy, Cyano, Nitro, Amino, Aminocarbonyl-, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylamino-carbonyl- oder C₁-C₆-Alkylaminosulfonyl-,
oder
für C₁-C₆-Alkyl-, C₁-C₆-Alkoxy-, C₁-C₆-Alkylamino-, C₁-C₆-Alkylcarbonyl amino-, C₁-C₆-Alkylaminocarbonyl- oder C₁-C₆-Alkylaminosulfonyl-, die gegebenenfalls ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, monocyclischem Heterocyclyl- mit 3 bis 8 Ringatomen und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl,
oder
für C₃-C₁₀-Cycloalkyl-, das gegebenenfalls ein- oder mehrfach, gleich oder verschieden substituiert sein kann mit Halogen, Amino, Hydroxy, Carboxy, C₁-C₆-Alkyl-, C₁-C₆-Alkoxy-, C₁-C₆-Alkoxy-C₁-C₆-Alkyl-, C₁-C₆-Alkyl-amino-, Amino-C₁-C₆-Alkyl-, C₁-C₆-Alkylamino-C₁-C₆-Alkyl, Halogen-C₁-C₆- Alkyl-, Halogen-C₁-C₆-Alkoxy-, und/oder einem monocyclischen Heterocyclylrest mit 3 bis 8 Ringatomen,
oder
für monozyklisches Heteroaryl- mit 5 oder 6 Ringatomen, das gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷, und/oder einem monocyclischen Heterocyclylrest mit 3 bis 8 Ringatomen,
oder
für monozyklisches Heterocyclyl- mit 3 bis 8 Ringatomen, das gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷ und/oder einem monocyclischen Heterocyclylrest mit 3 bis 8 Ringatomen,
oder
für Phenyl-, das gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl-, C₁-C₆-Alkylaminosulfonyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-und/oder einem monocyclischen Heterocyclylrest mit 3 bis 8 Ringatomen, und
- R⁶ und R⁷: unabhängig voneinander für Wasserstoff, C₁-C₃-Alkyl-, Cyclopropyl-, Di-C₁-C₃-Alkyl-Amino-C₁-C₃-Alkyl- oder Fluorpyridyl- stehen, und
- R⁸: für Hydroxy, C₁-C₆-Alkyl-, Halogen-C₁-C₃-Alkyl-, Hydroxy-C₁-C₃-Alkyl-, C₁-C₃-Alkoxy-C₁-C₃-Alkyl-, C₃-C₈-Cycloalkyl-, Phenyl-, monocyclisches Heterocyclyl- mit 3 bis 8 Ringatomen oder monocyclisches Heteroaryl- mit 5 oder 6 Ringatomen steht, worin Phenyl-, Heteroaryl- und Heterocyclylgegebenenfalls ein- oder zweifach substituiert sein können durch Halogen, C₁-C₃-Alkoxy- oder C₁-C₃-Alkyl-, und
- R⁹: für Wasserstoff, C₁-C₆-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze,
mit der Maßgabe, dass,
wenn A für einen Phenylring steht und R⁴ und R⁵ unabhängig voneinander stehen für Wasserstoff, Hydroxy, Cyano, Nitro, Amino, Aminocarbonyl-, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, oder für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die gegebenenfalls ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, monocyclischem Heterocyclyl- mit 3 bis 8 Ringatomen und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl,
R^{1a} nicht für Wasserstoff, Halogen, Cyano, Carboxy, Amino oder Aminosulfonyl, oder für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino-, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht.
Die Maßgabe umfasst aber nicht solche Verbindungen der allgemeinen Formel (I), in der A für Phenyl steht und R⁴ für Wasserstoff, Fluor, Chlor oder Brom steht und R⁵ für C₁-C₆-Alkoxy-, das ein- oder mehrfach, gleich oder verschieden mit Halogen substituiert ist, steht, und R^{1a} für Halogen steht,
und auch nicht solche Verbindungen der allgemeinen Formel (I), in der A für Phenyl steht und R⁴ für Wasserstoff steht und R⁵ für C₁-C₆-Alkoxy steht, welches ein- oder mehrfach, gleich oder verschieden mit einem monocyclischen Heterocyclylrest mit 3 bis 8 Ringatomen, und/oder einem monocyclischen Heteroarylrest mit 5 oder 6 Ringatomen substituiert ist, worin die genannten monocyclischen Heterocyclyl- und Heteroarylreste ihrerseits gegebenenfalls einfach substituiert sein können durch C₁-C₃-Alkyl, und R^{1a} für Halogen steht.
Damit umfasst die vorliegende Erfindung zum Beispiel auch Verbindungen der hergestellten Ausführungsbeispiele Nr. 1; 15; 32; 33; 164; 164.2; 165; 166 und 167.

Bevorzugt sind solche Verbindungen der allgemeinen Formel (I) in der
- X: für ein Sauerstoffatom steht, und
- A: für einen Phenyl- oder Pyridylring steht, und
- R^{1a}: für Wasserstoff, Halogen, Cyano, Carboxy, Amino oder Aminosulfonyl steht, oder
für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino, C₁-C₆-Alkyl-amino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, Phenyl-, Halophenyl-, Phenyl-C₁-C₆-Alkyl-, Pyridinyl-, -NR⁶C(=O)R⁹, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹ und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen und/oder mit einem monocyclischen Heteroarylrest mit 5 oder 6 Ringatomen und/oder mit einem Phenylrest, der seinerseits gegebenenfalls ein- oder mehrfach, gleich oder verschieden mit Halogen, C₁-C₃-Alkyl- und/oder C₁-C₃-Alkoxy-substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl- und/oder einem monocyclischen Heterocyclylrest mit 4 bis 7 Ringatomen und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl- und/oder C₁-C₃-Alkoxy-, und
- R^{1b} und R^{1c}: unabhängig voneinander für Wasserstoff, Halogen, Hydroxy, Cyano, Nitro oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl-Rest, und/oder einen monocyclischen Heterocyclylrest mit 4 bis 7 Ringatomen stehen, und
- R²: für Methyl-, Ethyl- oder Isopropyl- steht, und
- R³: für Cyclopropyl-, C₁-C₃-Alkyl-, C₁-C₃-Alkoxy-, Amino-, Cyclopropylamino- oder C₁-C₃-Alkylamino- steht, und
- R⁴ und R⁵: unabhängig voneinander stehen für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die gegebenenfalls ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, monocyclischem Heterocyclyl- mit 4 bis 7 Ringatomen, und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl-,
oder
für einen C₃-C₁₀-Cycloalkylrest, der gegebenenfalls ein- oder mehrfach, gleich oder verschieden substituiert sein kann mit Halogen, Amino, Hydroxy, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, und/oder einem monocyclischen Heterocyclylrest mit 4 bis 7 Ringatomen,
oder
für monozyklisches Heteroaryl- mit 5 oder 6 Ringatomen, das gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷ und/oder einem monocyclischen Heterocyclylrest mit 4 bis 7 Ringatomen,
oder
für monozyklisches Heterocyclyl- mit 4 bis 7 Ringatomen, das gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷ und/oder einem monocyclischen Heterocyclylrest mit 4 bis 7 Ringatomen,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl-, C₁-C₆-Alkylaminosulfonyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cyclo-alkyl-, und/oder einem monocyclischen Heterocyclylrest mit 4 bis 7 Ringatomen, und
- R⁶ und R⁷: unabhängig voneinander für Wasserstoff, C₁-C₃-Alkyl-, Cyclopropyl-, Di-C₁-C₃-Alkyl-Amino-C₁-C₃-Alkyl- oder Fluorpyridyl stehen, und
- R⁸: für Hydroxy, C₁-C₆-Alkyl-, Halogen-C₁-C₃-Alkyl-, Hydroxy-C₁-C₃-Alkyl-, C₁-C₃-Alkoxy-C₁-C₃-Alkyl-, C₃-C₈-Cycloalkyl-, Phenyl-, monocyclisches Heterocyclyl- mit 5 oder 6 Ringatomen steht, und
- R⁹: für Wasserstoff, C₁-C₆-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze,
mit der Maßgabe, dass, wenn A für einen Phenylring steht und R⁴ und R⁵ unabhängig voneinander stehen für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, oder für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die gegebenfalls ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, monocyclischem Heterocyclyl- mit 4 bis 7 Ringatomen, und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl-,
R^{1a} nicht für Wasserstoff, Halogen, Cyano, Carboxy, Amino oder Aminosulfonyl, oder für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino, C₁-C₆-Alkylamino-C₁-C₆-Alkyl-, *N-*(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht.
Die Maßgabe umfasst aber nicht solche Verbindungen der allgemeinen Formel (I), in der A für Phenyl steht und R⁴ für Wasserstoff, Fluor, Chlor oder Brom steht und R⁵ für C₁-C₆-Alkoxy-, das ein- oder mehrfach, gleich oder verschieden mit Halogen substituiert ist, steht, und R^{1a} für Halogen steht,
und auch nicht solche Verbindungen der allgemeinen Formel (I), in der A für Phenyl steht und R⁴ für Wasserstoff steht und R⁵ für C₁-C₆-Alkoxy steht, welches ein- oder mehrfach, gleich oder verschieden mit einem monocyclischen Heterocyclylrest mit 4 bis 7 Ringatomen, und/oder einem monocyclischen Heteroarylrest mit 5 oder 6 Ringatomen substituiert ist, worin die genannten monocyclischen Heterocyclyl- und Heteroarylreste ihrerseits gegebenenfalls einfach substituiert sein können durch C₁-C₃-Alkyl, und R^{1a} für Halogen steht.
Damit umfasst die vorliegende Erfindung zum Beispiel auch Verbindungen der hergestellten Ausführungsbeispiele Nr. 1; 15; 32; 33; 164; 164.2; 165; 166 und 167.

Besonders bevorzugt sind solche Verbindungen der allgemeinen Formel (I), in der
- X: für ein Sauerstoffatom steht, und
- A: für einen Phenyl- oder Pyridylring steht, und
- R^{1a}: für Wasserstoff, Halogen, Cyano, Carboxy, Amino oder Aminosulfonyl steht, oder
für einen C₁-C₆-Alkoxy-, C₁-C₃-Alkoxy-C₁-C₃-Alkyl-, C₁-C₃-Alkoxy-C₂-C₃-Alkoxy-, C₁-C₃-Alkylamino-, C₁-C₃-Alkylcarbonylamino, C₁-C₃-Alkyl-amino-C₁-C₃-Alkyl-, Hydroxy-C₁-C₃-Alkyl-, Fluor-C₁-C₃-Alkyl-, Fluor-C₁-C₃-Alkoxy-, C₁-C₃-Alkylcarbonyl- oder C₁-C₄-Alkoxycarbonyl-Rest steht,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, Phenyl-, Halophenyl-, Phenyl-C₁-C₃-Alkyl-, Pyridinyl-, -NR⁶C(=O)-R⁹,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹ und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Fluor, Chlor, Brom, Methyl- und/oder Methoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl, C₁-C₃-Alkoxy-, C₁-C₂-Alkoxy-C₁-C₂-Alkyl-, C₁-C₃-Alkylamino-, Amino-C₁-C₃-Alkyl-, -C(=O)NR⁶R⁷, -C(=O)R⁸, C₁-C₃-Alkylsulfinyl-, C₁-C₃-Alkylsulfonyl-, -S(=O)₂NH₂, C₁-C₃-Alkyl-sulfonylamino-, C₁-C₃-Alkylaminosulfonyl-, C₃-C₆-Cycloalkylamino-sulfonyl-, Fluor-C₁-C₃-Alkyl-, Fluor-C₁-C₃-Alkoxy-, Hydroxy-C₁-C₃-Alkyl-, C₃-C₆-Cycloalkyl- und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Fluor, Chlor, Brom, Methyl- und/oder Methoxy-, und
- R^{1b}: für Wasserstoff, Halogen, Hydroxy, Cyano, Nitro oder für einen C₁-C₃-Alkyl-, C₁-C₃-Alkoxy-, Fluor-C₁-C₃-Alkyl- oder Fluor-C₁-C₃-Alkoxy-Rest steht, und
- R^{1c}: für Wasserstoff, Fluor, Chlor, Brom oder Cyano steht, und
- R²: für Methyl-, Ethyl- oder Isopropyl- steht, und
- R³: für Cyclopropyl-, C₁-C₃-Alkyl-, C₁-C₃-Alkoxy-, Cyclopropylamino- oder C₁-C₃-Alkylamino- steht, und
- R⁴ und R⁵: unabhängig voneinander stehen für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylamino-carbonyl- oder C₁-C₆-Alkylaminosulfonyl-,
oder
für C₁-C₃-Alkyl-, C₁-C₃-Alkoxy-, C₁-C₃-Alkylamino-, die ein- oder mehrfach, gleich oder verschieden substituiert sein können mit Halogen, Amino, Hydroxy, Carboxy, Hydroxy-C₁-C₃-Alkyl-, C₁-C₃-Alkoxy-, C₁-C₃-Alkylamino-, Amino-C₁-C₃-Alkyl-, monocyclischem Heterocyclyl-mit 4 bis 7 Ringatomen, und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl,
oder
für einen C₃-C₇-Cycloalkylrest, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen,
oder
für monozyklisches Heteroaryl- mit 5 oder 6 Ringatomen, das gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷ und/oder einem monocyclischen Heterocyclylrest mit 4 bis 7 Ringatomen,
oder
für monozyklisches Heterocyclyl- mit 4 bis 7 Ringatomen, das gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷ und/oder einem monocyclischen Heterocyclylrest mit 4 bis 7 Ringatomen,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylamino-carbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl- und/oder einem monocyclischen Heterocyclylrest mit 4 bis 7 Ringatomen, und
- R⁶ und R⁷: unabhängig voneinander für Wasserstoff, C₁-C₃-Alkyl-, Cyclopropyl-, Di-C₁-C₃-Alkyl-Amino-C₁-C₃-Alkyl- oder Fluorpyridyl- stehen, und
- R⁸: für Hydroxy, C₁-C₆-Alkyl-, Hydroxy-C₁-C₃-Alkyl-, Fluor-C₁-C₃-Alkyl-, C₁-C₃-Alkoxy-C₁-C₃-Alkyl-, C₃-C₈-Cycloalkyl- oder monocyclisches Heterocyclyl- mit 5 oder 6 Ringatomen steht, und
- R⁹: für Wasserstoff, C₁-C₆-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze,
mit der Maßgabe, dass,
wenn A für einen Phenylring und R⁴ und R⁵ unabhängig voneinander stehen für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, oder für C₁-C₃-Alkyl-, C₁-C₃-Alkoxy-, C₁-C₃-Alkylamino-, die gegebenenfalls ein- oder mehrfach, gleich oder verschieden substituiert sein können mit Halogen, Amino, Hydroxy, Carboxy, Hydroxy-C₁-C₃-Alkyl-, C₁-C₃-Alkoxy-, C₁-C₃-Alkylamino-, Amino-C₁-C₃-Alkyl-, monocyclischem Heterocyclylmit 4 bis 7 Ringatomen, und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl,
R^{1a} nicht für Wasserstoff, Halogen, Cyano, Carboxy, Amino oder Aminosulfonyl, oder für einen C₁-C₆-Alkoxy-, C₁-C₃-Alkoxy-C₁-C₃-Alkyl-, C₁-C₃-Alkoxy-C₂-C₃-Alkoxy-, C₁-C₃-Alkylamino-, C₁-C₃-Alkylcarbonylamino, C₁-C₃-Alkylamino-C₁-C₃-Alkyl-, Hydroxy-C₁-C₃-Alkyl-, Fluor-C₁-C₃-Alkyl-, Fluor-C₁-C₃-Alkoxy-, C₁-C₃-Alkylcarbonyl- oder C₁-C₄-Alkoxycarbonyl-Rest steht.
Die Maßgabe umfasst aber nicht solche Verbindungen der allgemeinen Formel (I), in der A für Phenyl steht und R⁴ für Wasserstoff, Fluor, Chlor oder Brom steht und R⁵ für C₁-C₃-Alkoxy-, das ein- oder mehrfach, gleich oder verschieden mit Halogen substituiert ist, steht, und R^{1a} für Halogen steht,
und auch nicht solche Verbindungen der allgemeinen Formel (I), in der A für Phenyl steht und R⁴ für Wasserstoff steht und R⁵ für C₁-C₃-Alkoxy steht, welches ein- oder mehrfach, gleich oder verschieden mit einem monocyclischen Heterocyclylrest mit 4 bis 7 Ringatomen, und/oder einem monocyclischen Heteroarylrest mit 5 oder 6 Ringatomen substituiert ist, worin die genannten monocyclischen Heterocyclyl- und Heteroarylreste ihrerseits gegebenenfalls einfach substituiert sein können durch C₁-C₃-Alkyl, und R^{1a} für Halogen steht.
Damit umfasst die vorliegende Erfindung zum Beispiel auch Verbindungen der hergestellten Ausführungsbeispiele Nr. 1; 15; 32; 33; 164; 164.2; 165; 166 und 167.

Besonders bevorzugt sind ferner solche Verbindungen der allgemeinen Formel (I), in der
- X: für ein Sauerstoffatom steht, und
- A: für einen Phenyl- oder Pyridylring steht, und
- R^{1a}: für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, Phenyl-, Halophenyl-, Phenyl-C₁-C₃-Alkyl-, Pyridinyl-, -NR⁶C(=O)-R⁹,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹ und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Fluor, Chlor, Brom, Methyl- oder Methoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl, C₁-C₃-Alkoxy-, C₁-C₂-Alkoxy-C₁-C₂-Alkyl-, C₁-C₃-Alkylamino-, Amino-C₁-C₃-Alkyl-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkyl-sulfonylamino-, C₁-C₃-Alkylaminosulfonyl-, C₃-C₆-Cycloalkylamino-sulfonyl-, Fluor-C₁-C₃-Alkyl-, Fluor-C₁-C₃-Alkoxy-, Hydroxy-C₁-C₃-Alkyl-, C₃-C₆-Cycloalkyl- und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Fluor, Chlor, Brom, Methyl- oder Methoxy-, und
- R^{1b}: für Wasserstoff, Halogen, Hydroxy, Cyano, Nitro oder für einen C₁-C₃-Alkyl-, C₁-C₃-Alkoxy-, Fluor-C₁-C₃-Alkyl- oder Fluor-C₁-C₃-Alkoxy-Rest steht, und
- R^{1c}: für Wasserstoff, Fluor, Chlor, Brom oder Cyano steht, und
- R²: für Methyl-, Ethyl- oder Isopropyl- steht, und
- R³: für Cyclopropyl-, C₁-C₃-Alkyl-, C₁-C₃-Alkoxy-, Cyclopropylamino- oder C₁-C₃-Alkylamino- steht, und
- R⁴ und R⁵: unabhängig voneinander stehen für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₃-Alkyl-, C₁-C₃-Alkoxy-, C₁-C₃-Alkylamino-, die ein- oder mehrfach, gleich oder verschieden substituiert sein können mit Halogen, Amino, Hydroxy, Carboxy, Hydroxy-C₁-C₃-Alkyl-, C₁-C₃-Alkoxy-, C₁-C₃-Alkylamino-, Amino-C₁-C₃-Alkyl-, monocyclischem Heterocyclyl- mit 4 bis 7 Ringatomen, und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl,
oder
für einen C₃-C₇-Cycloalkylrest, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen,
oder
für monozyklisches Heteroaryl- mit 5 oder 6 Ringatomen, das gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für monozyklisches Heterocyclyl- mit 4 bis 7 Ringatomen, das gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylaminocarbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen, und
- R⁶und R⁷: unabhängig voneinander für Wasserstoff, C₁-C₃-Alkyl-, Cyclopropyl-, Di-C₁-C₃-Alkyl-Amino-C₁-C₃-Alkyl- oder Fluorpyridyl- stehen, und
- R⁸: für Hydroxy, C₁-C₆-Alkyl-, Hydroxy-C₁-C₃-Alkyl-, Fluor-C₁-C₃-Alkyl-, C₁-C₃-Alkoxy-C₁-C₃-Alkyl-, C₃-C₈-Cycloalkyl- oder monocyclisches Heterocyclyl- mit 5 oder 6 Ringatomen steht, und
- R⁹: für Wasserstoff, C₁-C₆-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze.

Besonders bevorzugt sind ferner solche Verbindungen der allgemeinen Formel (I), in der
- X: für ein Sauerstoffatom steht, und
- A: für einen Phenyl- oder Pyridylring steht, und
- R^{1a}: für Wasserstoff, Halogen, Cyano, Carboxy, Amino oder Aminosulfonyl steht, oder
für einen C₁-C₆-Alkoxy-, C₁-C₃-Alkoxy-C₁-C₃-Alkyl-, C₁-C₃-Alkoxy-C₂-C₃-Alkoxy-, C₁-C₃-Alkylamino-, C₁-C₃-Alkylcarbonylamino, C₁-C₃-Alkyl-amino-C₁-C₃-Alkyl-, Hydroxy-C₁-C₃-Alkyl-, Fluor-C₁-C₃-Alkyl-, Fluor-C₁-C₃-Alkoxy-, C₁-C₃-Alkylcarbonyl- oder C₁-C₄-Alkoxycarbonyl-Rest steht,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, Phenyl-, Halophenyl-, Phenyl-C₁-C₃-Alkyl-, Pyridinyl-, -NR⁶C(=O)-R⁹,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Fluor, Chlor, Brom, Methyl- und/oder Methoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl, C₁-C₃-Alkoxy-, C₁-C₂-Alkoxy-C₁-C₂-Alkyl-, C₁-C₃-Alkylamino-, Amino-C₁-C₃-Alkyl-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkyl-sulfonylamino-, C₁-C₃-Alkylaminosulfonyl-, C₃-C₆-Cycloalkylamino-sulfonyl-, Fluor-C₁-C₃-Alkyl-, Fluor-C₁-C₃-Alkoxy-, Hydroxy-C₁-C₃-Alkyl-, C₃-C₆-Cycloalkyl- und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Fluor, Chlor, Brom, Methyl- und/oder Methoxy-, und
- R^{1b}: für Wasserstoff, Halogen, Hydroxy, Cyano, Nitro oder für einen C₁-C₃-Alkyl-, C₁-C₃-Alkoxy-, Fluor-C₁-C₃-Alkyl- oder Fluor-C₁-C₃-Alkoxy-Rest steht, und
- R^{1c}: für Wasserstoff, Fluor, Chlor, Brom oder Cyano steht, und
- R²: für Methyl-, Ethyl- oder Isopropyl- steht, und
- R³: für Cyclopropyl-, C₁-C₃-Alkyl-, C₁-C₃-Alkoxy-, Cyclopropylamino- oder C₁-C₃-Alkylamino- steht, und
- R⁴: für einen C₃-C₇-Cycloalkylrest steht, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen,
oder
für monozyklisches Heteroaryl- mit 5 oder 6 Ringatomen steht, das gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für monozyklisches Heterocyclyl- mit 4 bis 7 Ringatomen steht, das gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁C₂-Alkoxy-C₁-C₂-Alkyl-, C₁-C₃-Alkylamino-, Amino-C₁-C₃-Alkyl-, C₁-C₃-Alkylaminocarbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen, und
- R⁵: für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkyl-aminosulfonyl- steht, und
- R⁶und R⁷: unabhängig voneinander für Wasserstoff, C₁-C₃-Alkyl-, Cyclopropyl-, Di-C₁-C₃-Alkyl-Amino-C₁-C₃-Alkyl- oder Fluorpyridyl- stehen, und
- R⁸: für Hydroxy, C₁-C₆-Alkyl-, Hydroxy-C₁-C₃-Alkyl-, Fluor-C₁-C₃-Alkyl-, C₁-C₃-Alkoxy-C₁-C₃-Alkyl-, C₃-C₈-Cycloalkyl- oder monocyclisches Heterocyclyl- mit 5 oder 6 Ringatomen steht, und
- R⁹: für Wasserstoff, C₁-C₆-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze.

Besonders bevorzugt sind ferner solche Verbindungen der allgemeinen Formel (I), in der
- X: für ein Sauerstoffatom steht, und
- A: für einen Phenyl- oder Pyridylring steht, und
- R^{1a}: für Wasserstoff, Halogen, Cyano, Carboxy, Amino oder Aminosulfonyl steht, oder
für einen C₁-C₆-Alkoxy-, C₁-C₃-Alkoxy-C₁-C₃-Alkyl-, C₁-C₃-Alkoxy-C₂-C₃-Alkoxy-, C₁-C₃-Alkylamino-, C₁-C₃-Alkylcarbonylamino, C₁-C₃-Alkyl-amino-C₁-C₃_Alkyl-, Hydroxy-C₁-C₃-Alkyl-, Fluor-C₁-C₃-Alkyl-, Fluor-C₁-C₃-Alkoxy-, C₁-C₃-Alkylcarbonyl- oder C₁-C₄-Alkoxycarbonyl-Rest steht,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, Phenyl-, Halophenyl-, Phenyl-C₁-C₃-Alkyl-, Pyridinyl-, -NR⁶C(=O)-R⁹,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Fluor, Chlor, Brom, Methyl- und/oder Methoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl, C₁-C₃-Alkoxy-, C₁-C₂-Alkoxy-C₁-C₂-Alkyl-, C₁-C₃-Alkylamino-, Amino-C₁-C₃-Alkyl-, -C(=O)NR⁶R⁷, -C(=O)R⁸, C₁-C₃-Alkylsulfinyl-, C₁-C₃-Alkylsulfonyl-, -S(=O)₂NH₂, C₁-C₃-Alkyl-sulfonylamino-, C₁-C₃-Alkylaminosulfonyl-, C₃-C₆-Cycloalkylamino-sulfonyl-, Fluor-C₁-C₃-Alkyl-, Fluor-C₁-C₃-Alkoxy-, Hydroxy-C₁-C₃-Alkyl-, C₃-C₆-Cycloalkyl- und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Fluor, Chlor, Brom, Methyl- und/oder Methoxy-, und
- R^{1b}: für Wasserstoff, Halogen, Hydroxy, Cyano, Nitro oder für einen C₁-C₃-Alkyl-, C₁-C₃-Alkoxy-, Fluor-C₁-C₃-Alkyl- oder Fluor-C₁-C₃-Alkoxy-Rest steht, und
- R^{1c}: für Wasserstoff, Fluor, Chlor, Brom oder Cyano steht, und
- R²: für Methyl-, Ethyl- oder Isopropyl- steht, und
- R³: für Cyclopropyl-, C₁-C₃-Alkyl-, C₁-C₃-Alkoxy-, Cyclopropylamino- oder C₁-C₃-Alkylamino- steht, und
- R⁴: für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkyl-aminosulfonyl- steht, und
- R⁵: für einen C₃-C₇-Cycloalkylrest steht, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen,
oder
für monozyklisches Heteroaryl- mit 5 oder 6 Ringatomen steht, das gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für monozyklisches Heterocyclyl- mit 4 bis 7 Ringatomen steht, das gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylamino-carbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen, und
- R⁶ und R⁷: unabhängig voneinander für Wasserstoff, C₁-C₃-Alkyl-, Cyclopropyl-, Di-C₁-C₃-Alkyl-Amino-C₁-C₃-Alkyl- oder Fluorpyridyl- stehen, und
- R⁸: für Hydroxy, C₁-C₆-Alkyl-, Hydroxy-C₁-C₃-Alkyl-, Fluor-C₁-C₃-Alkyl-, C₁-C₃-Alkoxy-C₁-C₃-Alkyl-, C₃-C₈-Cycloalkyl- oder monocyclisches Heterocyclyl- mit 5 oder 6 Ringatomen steht, und
- R⁹: für Wasserstoff, C₁-C₆-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze.

Ganz besonders interessant sind solche Verbindungen der allgemeinen Formel (I), in der
- X: für ein Sauerstoffatom steht, und
- A: für einen Phenyl- oder 3-Pyridylring steht, und
- R^{1a}: für Wasserstoff oder Chlor steht,
oder
für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl-, die gegebenenfalls ein- oder mehrfach, gleich oder verschieden substituiert sein können mit Fluor, Chlor, Cyano, Nitro, Hydroxy, Oxo, C₁-C₃-Alkyl-, C₁-C₃-Alkoxy-, C₁-C₂-Alkoxy-C₁-C₂-Alkyl-, Hydroxy-C₁-C₃-Alkyl-, Dimethylamino-, Trifluormethyl-, Difluorethyl, Trifluorethyl-, Trifluormethoxy-, Cyclopropyl-, Phenyl-, Fluorphenyl-, Phenyl-C₁-C₃-Alkyl-, Pyridinyl-, -NR⁶C(=O)-R⁹, -C(=O)-NR⁶R⁷, -C(=O)-R⁸, -S(=O)₂-NR⁶R⁷, -S(=O)-R⁹, -S(=O)₂-R⁹ und/oder -NH-S(=O)₂-R⁹ steht,
oder
für Tetrazolyl- steht,
oder
für Isoxazolyl-, Pyrazolyl-, Thienyl-, Thiazolyl-, Imidazolyl-, Triazolyl-, Pyrrolyl-, Oxadiazolyl-, Pyridinyl- oder Pyrimidinyl- steht, die gegebenenfalls ein- oder mehrfach, gleich oder verschieden substituiert sein können mit Fluor, Chlor, Hydroxy, Amino, Cyano, Nitro, C₁-C₃-Alkyl-, C₁-C₃-Alkoxy-, C₁-C₂-Alkoxy-C₁-C₂-Alkyl-, Dimethylamino-, Trifluormethyl-, Difluorethyl-, Trifluorethyl-, Trifluormethoxy-, Cyclopropyl, Pyridinyl-, Phenyl-, Fluorphenyl-, -C(=O)-NR⁶R⁷, -C(=O)-R⁸, -S(=O)₂-NR⁶R⁷, -S(=O)-R⁹, -S(=O)₂-R⁹ und/oder -NH-S(=O)₂-R⁹,
oder
- R^{1b}: für einen Phenylrest steht, der gegebenenfalls ein- oder mehrfach, gleich oder verschieden substituiert sein kann mit Fluor, Chlor, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Dimethylamino-, -C(=O)NR⁶R⁷, -C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Trifluormethyl-, Difluorethyl-, Trifluorethyl-, Trifluormethoxy-, Hydroxy-C₁-C₃-Alkyl-, Cyclopropyl, Chlorthienyl, Morpholino- und/oder Pyridinyl-, und für Wasserstoff, Fluor, Brom oder Cyano steht,
- R^{1c}: für Wasserstoff oder Brom steht, und
- R²: für Methyl-, Ethyl- oder Isopropyl- steht, und
- R³: für Cyclopropyl-, Methyl-, Ethyl-, Methoxy-, Ethoxy-, Cyclopropylamino-, Methylamino- oder Ethylamino- steht, und
- R⁴ und R⁵: unabhängig voneinander stehen für Wasserstoff, Hydroxy, Cyano, Amino, Chlor, C₁-C₆-Alkyl-, Methoxy-, Ethoxy- oder C₁-C₃-Alkylcarbonylamino-,
oder
für Difluormethoxy- oder Trifluormethoxy-, oder für C₁-C₃-Alkoxy-, welches mit Pyridinyl-, Morpholinyl-, Pyrrolidinyl- oder Piperazinyl- substituiert sein kann,
worin Pyridinyl- und Piperazinyl- ihrerseits gegebenenfalls substituiert sein können mit C₁-C₃-Alkyl-,
oder
für Cyclopropyl-,
oder
für Pyridinyl-, Pyrazolyl-, Triazolyl-, Isoxazolyl-, die gegebenenfalls ein- oder mehrfach, gleich oder verschieden substituiert sein können mit Hydroxy und/oder Methyl-,
oder
für Pyrrolidinyl-, Morpholinyl-, Piperidinyl-, Piperazinyl-, Oxazolidinyl-, Thiomorpholinyl-, die gegebenenfalls ein- oder mehrfach substituiert sein können mit Oxo, Methyl- und/oder -S(=O)₂R⁹,
oder
für gegebenenfalls mit C₁-C₃-Alkylaminosulfonyl- oder Fluor substituiertes Phenyl, und
- R⁶und R⁷: unabhängig voneinander für Wasserstoff, C₁-C₃-Alkyl-, Cyclopropyl-, Di-C₁-C₃-Alkyl-Amino-C₁-C₃-Alkyl- oder Fluorpyridyl stehen, und
- R⁸: für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
- R⁹: für C₁-C₄-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze,
mit der Maßgabe, dass,
wenn A für einen Phenylring steht und R⁴ und R⁵ unabhängig voneinander stehen für Wasserstoff, Hydroxy, Cyano, Amino, Chlor, C₁-C₆-Alkyl-, Methoxy-, Ethoxy- oder C₁-C₃-Alkylcarbonylamino-, oder für Difluormethoxy- oder Trifluormethoxy-, oder für C₁-C₃-Alkoxy-, welches mit Pyridinyl-, Morpholinyl-, Pyrrolidinyl- oder Piperazinyl- substituiert sein kann,
worin Pyridinyl- und Piperazinyl- ihrerseits gegebenenfalls mit C₁-C₃-Alkyl- substituiert sein können,
R^{1a} nicht für Wasserstoff oder Chlor steht.
Die Maßgabe umfasst aber nicht solche Verbindungen der allgemeinen Formel (I), in der A für Phenyl steht und R⁴ für Wasserstoff oder Chlor steht und R⁵ für Trifluormethoxy steht, und R^{1a} für Chlor steht,
und auch nicht solche Verbindungen der allgemeinen Formel (I), in der A für Phenyl steht und R⁴ für Wasserstoff steht und R⁵ für C₁-C₃-Alkoxy steht, welches mit Morpholinyl-, Pyrrolidinyl-, Piperazinyl- oder Pyridyl- substituiert ist, worin das Piperazinyl- und Pyridinyl- selbst durch C₁-C₃-Alkyl- substituiert sein kann, und R^{1a} für Chlor steht.
Damit umfasst die vorliegende Erfindung zum Beispiel auch Verbindungen der hergestellten Ausführungsbeispiele Nr. 1; 15; 32; 33; 164; 164.2; 165; 166 und 167.

Ganz besonders interessant sind ferner solche Verbindungen der allgemeinen Formel (I), in der
- X: für ein Sauerstoffatom steht, und
- A: für einen Phenyl- oder 3-Pyridylring steht, und
- R^{1a}: für Wasserstoff oder Chlor steht,
oder
für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl- steht, die gegebenenfalls ein- oder mehrfach, gleich oder verschieden substituiert sein können mit Fluor, Hydroxy, Oxo, C₁-C₃-Alkyl-, Methoxy, Hydroxy-C₁-C₃-Alkyl-, Dimethylamino-, Difluorethyl-, Trifluorethyl-, Benzyl-, -NR⁶C(=O)-R⁹, -C(=O)-NR⁶R⁷, -C(=O)-R⁸, und/oder -S(=O)₂-R⁹,
oder
für Tetrazolyl- steht,
oder
für Isoxazolyl-, Pyrazolyl-, Thienyl-, Thiazolyl-, Imidazolyl-, Triazolyl-, Pyrrolyl-, Oxadiazolyl-, Pyridinyl- oder Pyrimidinyl- steht, die gegebenenfalls ein- oder mehrfach, gleich oder verschieden substituiert sein können mit Fluor, Chlor, Hydroxy, Cyano, C₁-C₂-Alkyl-, Methoxy-, Methoxymethyl-, Trifluormethyl-, Cyclopropyl, Pyridinyl-, Phenyl-, Fluorphenyl- und/oder -C(=O)-R⁸,
oder
für einen Phenylrest steht, der gegebenenfalls ein- oder mehrfach, gleich oder verschieden substituiert sein kann mit Fluor, Chlor, Hydroxy, Cyano, Nitro, Carboxy, C₁-C₃-Alkyl-, Methoxy-, -C(=O)NR⁶R⁷, -C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cyclo-alkylaminosulfonyl-, Trifluormethyl-, Trifluormethoxy-, Hydroxy-C₁-C₃-Alkyl-, Cyclopropyl-, Chlorthienyl und/oder Morpholino-, und
- R^{1b}: für Wasserstoff, Fluor, Brom oder Cyano steht, und
- R^{1c}: für Wasserstoff oder Brom steht, und
- R²: für Methyl-, Ethyl- oder Isopropyl- steht, und
- R³: für Cyclopropyl-, Methyl-, Ethyl-, Methoxy-, Ethoxy-, Cyclopropylamino-, Methylamino- oder Ethylamino- steht, und
- R⁴ und R⁵: unabhängig voneinander stehen für Wasserstoff, Hydroxy, Cyano, Amino, Chlor, C₁-C₆-Alkyl-, Methoxy-, Ethoxy- oder C₁-C₃-Alkylcarbonylamino-,
oder
für Difluormethoxy- oder Trifluormethoxy-, oder für C₁-C₃-Alkoxy-, welches mit Pyridinyl-, Morpholinyl-, Pyrrolidinyl- oder Piperazinyl- substituiert sein kann,
worin Pyridinyl- und Piperazinyl- ihrerseits gegebenenfalls substituiert sein können mit C₁-C₃-Alkyl-,
oder
für Cyclopropyl-,
oder
für Pyridinyl-, Pyrazolyl-, Triazolyl- oder Isoxazolyl-, die gegebenenfalls ein- oder mehrfach, gleich oder verschieden substituiert sein können mit Hydroxy und/oder Methyl-,
oder
für Pyrrolidinyl-, Morpholinyl-, Piperidinyl-, Piperazinyl-, Oxazolidinyl-, Thiomorpholinyl-, die gegebenenfalls ein- oder mehrfach substituiert sein können mit Methyl-, Oxo und/oder -S(=O)₂R⁹,
oder
für gegebenenfalls mit C₁-C₃-Alkylaminosulfonyl- oder Fluor substituiertes Phenyl, und
- R⁶und R⁷: unabhängig voneinander für Wasserstoff, C₁-C₃-Alkyl-, Cyclopropyl-, Di-C₁-C₃-Alkyl-Amino-C₁-C₃-Alkyl- oder Fluorpyridyl stehen, und
- R⁸: für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
- R⁹: für C₁-C₄-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze,
mit der Maßgabe, dass,
wenn A für einen Phenylring steht und R⁴ und R⁵ unabhängig voneinander stehen für Wasserstoff, Hydroxy, Cyano, Amino, Chlor, C₁-C₆-Alkyl-, Methoxy-, Ethoxy- oder C₁-C₃-Alkylcarbonylamino-, oder für Difluormethoxy- oder Trifluormethoxy-, oder für C₁-C₃-Alkoxy-, welches mit Pyridinyl-, Morpholinyl-, Pyrrolidinyl- oder Piperazinyl- substituiert sein kann,
worin Pyridinyl- und Piperazinyl- ihrerseits gegebenenfalls mit C₁-C₃-Alkyl- substituiert sein können,
R^{1a} nicht für Wasserstoff oder Chlor steht.
Die Maßgabe umfasst aber nicht solche Verbindungen der allgemeinen Formel (I), in der A für Phenyl steht und R⁴ für Wasserstoff oder Chlor steht und R⁵ für Trifluormethoxy steht, und R^{1a} für Chlor steht,
und auch nicht solche Verbindungen der allgemeinen Formel (I), in der A für Phenyl steht und R⁴ für Wasserstoff steht und R⁵ für C₁-C₃-Alkoxy steht, welches mit Morpholinyl-, Pyrrolidinyl-, Piperazinyl- oder Pyridyl- substituiert ist, worin das Piperazinyl- und Pyridinyl- selbst durch C₁-C₃-Alkyl- substituiert sein kann, und R^{1a} für Chlor steht.
Damit umfasst die vorliegende Erfindung zum Beispiel auch Verbindungen der hergestellten Ausführungsbeispiele Nr. 1; 15; 32; 33; 164; 164.2; 165; 166 und 167.

Überaus interessant sind solche Verbindungen der allgemeinen Formel (I), in der
- X: für ein Sauerstoffatom steht, und
- A: für einen Phenylring steht, und
- R^{1a}: für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl- steht, die gegebenenfalls ein- oder mehrfach, gleich oder verschieden substituiert sein können mit Fluor, Hydroxy, Oxo, C₁-C₃-Alkyl-, Methoxy, Hydroxy-C₁-C₃-Alkyl-, Dimethylamino-, Difluorethyl-, Trifluorethyl-, Benzyl-, -NR⁶C(=O)-R⁹, -C(=O)-NR⁶R⁷, -C(=O)-R⁸, und/oder -S(=O)₂-R⁹,
oder
für Tetrazolyl- steht,
oder für Isoxazolyl-, Pyrazolyl-, Thienyl-, Thiazolyl-, Imidazolyl-, Triazolyl-, Pyrrolyl-, Oxadiazolyl-, Pyridinyl- oder Pyrimidinyl- steht, die gegebenenfalls ein- oder mehrfach, gleich oder verschieden substituiert sein können mit Fluor, Chlor, Hydroxy, Cyano, C₁-C₂-Alkyl-, Methoxy-, Methoxymethyl-, Trifluormethyl-, Cyclopropyl, Pyridinyl-, Phenyl-, Fluorphenyl- und/oder -C(=O)-R⁸,
oder
für einen Phenylrest steht, der gegebenenfalls ein- oder mehrfach, gleich oder verschieden substituiert sein kann mit Fluor, Chlor, Hydroxy, Cyano, Nitro, Carboxy, C₁-C₃-Alkyl-, Methoxy-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonyl-amino-, C₁-C₃-Alkylaminosulfonyl-, C₃-C₆-Cycloalkylaminosulfonyl-, Trifluormethyl-, Trifluormethoxy-, Hydroxy-C₁-C₃-Alkyl-, Cyclopropyl-, Chlorthienyl- und/oder Morpholino-,
- R^{1b}: für Wasserstoff, Fluor, Brom oder Cyano steht, und
- R^{1c}: für Wasserstoff steht, und
- R²: für Methyl- oder Ethyl- steht, und
- R³: für Methylamino- steht, und
- R⁴ und R⁵: unabhängig voneinander für Wasserstoff, Hydroxy, Cyano, Chlor, C₁-C₆-Alkyl-, Methoxy-, Ethoxy- oder C₁-C₃-Alkylcarbonylamino-,
oder
für Difluormethoxy- oder Trifluormethoxy- stehen, und
- R⁶und R⁷: unabhängig voneinander für Wasserstoff, C₁-C₃-Alkyl-, Cyclopropyl- oder Di-C₁-C₃-Alkyl-Amino-C₁-C₃-Alkyl- stehen, und
- R⁸: für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
- R⁹: für C₁-C₄-Alkyl- oder C₁-C₄-Alkoxy- steht,
sowie deren Polymorphe, Tautomere, Solvate, physiologisch verträglichen Salze und Solvate dieser Salze, und
wobei das Stereozentrum, welches durch das an R² gebundene Kohlenstoffatom des Benzodiazepin-Gerüsts repräsentiert wird, entweder racemisch oder überwiegend oder vollständig in der (*S*)-Konfiguration vorliegt.

Überaus interessant sind ferner solche Verbindungen der allgemeinen Formel (I), in der
- X: für ein Sauerstoffatom steht, und
- A: für einen Phenylring steht, und
- R^{1a}: für Wasserstoff oder Chlor steht, und
- R^{1b}: für Wasserstoff, Fluor, Brom oder Cyano steht, und
- R^{1c}: für Wasserstoff steht, und
- R²: für Methyl- oder Ethyl- steht, und
- R³: für Methylamino- steht, und
- R⁴: für Cyclopropyl- steht,
oder
für Pyridinyl-, Pyrazolyl-, Triazolyl- oder Isoxazolyl- steht, die gegebenenfalls ein- oder mehrfach, gleich oder verschieden substituiert sein können mit Hydroxy und/oder Methyl-,
oder
für Pyrrolidinyl-, Morpholinyl-, Piperidinyl-, Piperazinyl-, Oxazolidinyl- oder Thiomorpholinyl- steht, die gegebenenfalls ein- oder mehrfach substituiert sein können mit Methyl-, Oxo und/oder -S(=O)₂R⁹,
oder
für gegebenenfalls mit C₁-C₃-Alkylaminosulfonyl- oder Fluor substituiertes Phenyl- steht, und
- R⁵: für Wasserstoff, Hydroxy, Cyano, Chlor, C₁-C₆-Alkyl-, Methoxy-, Ethoxy- oder C₁-C₃-Alkylcarbonylamino-,
oder
für Difluormethoxy- oder Trifluormethoxy- steht, und
- R⁶und R⁷: unabhängig voneinander für Wasserstoff, C₁-C₃-Alkyl-, Cyclopropyl- oder Di-C₁-C₃-Alkyl-Amino-C₁-C₃-Alkyl- stehen, und
- R⁸: für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
- R⁹: für C₁-C₄-Alkyl- oder C₁-C₄-Alkoxy- steht,
sowie deren Polymorphe, Tautomere, Solvate, physiologisch verträglichen Salze und Solvate dieser Salze, und
wobei das Stereozentrum, welches durch das an R² gebundene Kohlenstoffatom des Benzodiazepin-Gerüsts repräsentiert wird, entweder racemisch oder überwiegend oder vollständig in der (*S*)-Konfiguration vorliegt.

Überaus interessant sind ferner solche Verbindungen der allgemeinen Formel (I), in der
- X: für ein Sauerstoffatom steht, und
- A: für einen Phenylring steht, und
- R^{1a}: für Wasserstoff oder Chlor steht, und
- R^{1b}: für Wasserstoff, Fluor, Brom oder Cyano steht, und
- R^{1c}: für Wasserstoff steht, und
- R²: für Methyl- oder Ethyl- steht, und
- R³: für Methylamino- steht, und
- R⁴: für Wasserstoff, Chlor, Methoxy- oder Ethoxy-,
oder
für Difluormethoxy- oder Trifluormethoxy- steht, und
- R⁵: für Cyclopropyl- steht,
oder
für Pyridinyl- oder Pyrazolyl- steht, die gegebenenfalls ein- oder mehrfach, mit Methyl- substituiert sein können,
oder
für Morpholinyl-, Piperidinyl-, Piperazinyl- oder Thiomorpholinyl- steht, die gegebenenfalls ein- oder mehrfach substituiert sein können mit Methyl-, Oxo und/oder -S(=O)₂R⁹,
oder
für mit C₁-C₃-Alkylaminosulfonyl- substituiertes Phenyl steht, und
- R⁶und R⁷: unabhängig voneinander für Wasserstoff, C₁-C₃-Alkyl-, Cyclopropyl- oder Di-C₁-C₃-Alkyl-Amino-C₁-C₃-Alkyl- stehen, und
- R⁸: für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
- R⁹: für C₁-C₄-Alkyl- oder C₁-C₄-Alkoxy- steht,
sowie deren Polymorphe, Tautomere, Solvate, physiologisch verträglichen Salze und Solvate dieser Salze, und
wobei das Stereozentrum, welches durch das an R² gebundene Kohlenstoffatom des Benzodiazepin-Gerüsts repräsentiert wird, entweder racemisch oder überwiegend oder vollständig in der (*S*)-Konfiguration vorliegt.

Überaus interessant sind weiterhin solche Verbindungen der allgemeinen Formel (I), in der
- X: für ein Sauerstoffatom steht, und
- A: für einen Phenylring steht, und
- R^{1a}: für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl- steht, die gegebenenfalls ein- oder mehrfach, gleich oder verschieden substituiert sein können mit Fluor, Hydroxy, Oxo, C₁-C₃-Alkyl-, Methoxy, Hydroxy-C₁-C₃-Alkyl-, Dimethylamino-, Difluorethyl-, Trifluorethyl-, Benzyl-, -NR⁶C(=O)-R⁹, -C(=O)-NR⁶R⁷, -C(=O)-R⁸ und/oder -S(=O)₂-R⁹,
oder
für Isoxazolyl- oder Pyrazolyl- steht, die gegebenenfalls ein- oder mehrfach, gleich oder verschieden mit C₁-C₂-Alkyl-substituiert sein können, und
- R^{1b}: für Wasserstoff, Fluor, Brom oder Cyano steht, und
- R^{1c}: für Wasserstoff steht, und
- R²: für Methyl- steht, und
- R³: für Methylamino- steht, und
- R⁴ und R⁵: unabhängig voneinander für Wasserstoff, Hydroxy, Cyano, Chlor, C₁-C₆-Alkyl-, Methoxy-, Ethoxy- oder C₁-C₃-Alkylcarbonylamino-,
oder
für Difluormethoxy- oder Trifluormethoxy- stehen, und
- R⁶und R⁷: unabhängig voneinander für Wasserstoff, C₁-C₃-Alkyl-, Cyclopropyl- oder Di-C₁-C₃-Alkyl-Amino-C₁-C₃-Alkyl- stehen, und
- R⁸: für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
- R⁹: für C₁-C₄-Alkyl- oder C₁-C₄-Alkoxy- steht,
sowie deren Polymorphe, Tautomere, Solvate, physiologisch verträglichen Salze und Solvate dieser Salze, und
wobei das Stereozentrum, welches durch das an R² gebundene Kohlenstoffatom des Benzodiazepin-Gerüsts repräsentiert wird, entweder racemisch oder überwiegend oder vollständig in der (*S*)-Konfiguration vorliegt.

Überaus interessant sind weiterhin solche Verbindungen der allgemeinen Formel (I), in der
- X: für ein Sauerstoffatom steht, und
- A: für einen Phenylring steht, und
- R^{1a}: für Piperazinyl-, Pyrrolidinyl-, Piperidinyl-, Morpholinyl-, Thiomorpholinyl- oder Azetidinyl- steht, die gegebenenfalls ein- oder mehrfach, gleich oder verschieden substituiert sein können mit Hydroxy, Oxo, C₁-C₃-Alkyl-, Methoxy-, Dimethylamino-, Difluorethyl-, Trifluorethyl-, -NR⁶C(=O)-R⁹, -C(=O)-NR⁶R⁷ und/oder -C(=O)-R⁸, und
- R^{1b}: für Wasserstoff, Fluor, Brom oder Cyano steht, und
- R^{1c}: für Wasserstoff steht, und
- R²: für Methyl- steht, und
- R³: für Methylamino- steht, und
- R⁴ und R⁵: unabhängig voneinander für Wasserstoff, Chlor, Methoxy- oder Ethoxy-,
oder
für Difluormethoxy- oder Trifluormethoxy- stehen, und
- R⁶und R⁷: unabhängig voneinander für Wasserstoff oder C₁-C₃-Alkyl- stehen, und
- R⁸: für Methyl- steht, und
- R⁹: für Methyl- steht,
sowie deren Polymorphe, Tautomere, Solvate, physiologisch verträglichen Salze und Solvate dieser Salze, und
wobei das Stereozentrum, welches durch das an R² gebundene Kohlenstoffatom des Benzodiazepin-Gerüsts repräsentiert wird, entweder racemisch oder überwiegend oder vollständig in der (*S*)-Konfiguration vorliegt.

Außerordentlich bevorzugt sind folgende Verbindungen:
- (±)-1-(4-Chlorphenyl)-*N*,4-dimethyl-8-(trifluormethoxy)-4,5-dihydro-3*H*-2,3-benzodiazepin-3-carboxamid
- (4*S*)-1-(4-Chlorphenyl)-*N*,4-dimethyl-8-(trifluormethoxy)-4,5-dihydro-3*H*-2,3-benzodiazepin-3-carboxamid
- (4*R*)-1-(4-Chlorphenyl)-*N*,4-dimethyl-8-(trifluormethoxy)-4,5-dihydro-3*H*-2,3-benzodiazepin-3-carboxamid
- (±)-1-[4-(3,5-Dimethyl-4-isoxazolyl)phenyl]-7,8-dimethoxy-*N*,4-dimethyl-4,5-dihydro-3*H-*2,3-benzodiazepin-3-carboxamid
- (4*R*)-1-[4-(3,5-Dimethyl-4-isoxazolyl)phenyl]-7,8-dimethoxy-*N*,4-dimethyl-4,5-dihydro-3*H*-2,3-benzodiazepin-3-carboxamid
- (4*S*)-1-[4-(3,5-Dimethyl-4-isoxazolyl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4-(1*H*-pyrazol-3-yl)phenyl]-4,5-dihydro-3*H*-2,3-benzodiazepin-3-carboxamid
- (±)-1-[4-(2-Chlorpyridin-3-yl)phenyl]-7,8-dimethoxy-*N*,4-dimethyl-4,5-dihydro-3*H*-2,3-benzodiazepin-3-carboxamid
- (±)-5-(4-{7,8-Dimethoxy-4-methyl-3-[(methylamino)carbonyl]-4,5-dihydro-3*H*-2,3-benzodiazepin-1-yl}phenyl)thiophen-2-carbonsäure
- (±)-4'-{7,8-Dimethoxy-4-methyl-3-[(methylamino)carbonyl]-4,5-dihydro-3*H*-2,3-benzodiazepin-1-yl }biphenyl-2-carbonsäure
- (±)-7,8-Dimethoxy-*N*,4-dimethyl-1-[4-(morpholin-4-yl)phenyl]-4,5-dihydro-3*H*-2,3-benzodiazepin-3-carboxamid
- (±)-1-(4-Chlorphenyl)-*N*,4-dimethyl-8-(pyridin-4-yl)-4,5-dihydro-3*H*-2,3-benzodiazepin-3-carboxamid
- (±)-1-(4-Chlorphenyl)-8-cyclopropyl-*N*,4-dimethyl-4,5-dihydro-3*H*-2,3-benzodiazepin-3-carboxamid
- (±)-1-(4-Chlorphenyl)-*N*,4-dimethyl-8-{4-[(methylamino)sulfonyl]phenyl}-4,5-dihydro-3*H*-2,3-benzodiazepin-3-carboxamid
- (±)-1-(4-Chlorphenyl)-*N*,4-dimethyl-8-(morpholin-4-yl)-4,5-dihydro-3*H*-2,3-benzodiazepin-3-carboxamid
- (4*S*)-1-(4-Chlorphenyl)-*N*,4-dimethyl-8-(morpholin-4-yl)-4,5-dihydro-3*H*-2,3-benzodiazepin-3-carboxamid
- (4*R*)-1-(4-Chlorphenyl)-*N*,4-dimethyl-8-(morpholin-4-yl)-4,5-dihydro-3*H*-2,3-benzodiazepin-3-carboxamid
- (±)-1-(4-Chlorphenyl)-*N*,4-dimethyl-8-(4-methylpiperazin-1-yl)-4,5-dihydro-3*H*-2,3-benzodiazepin-3-carboxamid
- (±)-1-(4-Chlorphenyl)-*N*,4-dimethyl-8-(piperidin-1-yl)-4,5-dihydro-3*H*-2,3-benzodiazepin-3-carboxamid
- (±)-8-Methoxy-*N*,4-dimethyl-1-(pyridin-3-yl)-4,5-dihydro-3*H*-2,3-benzodiazepin-3-carboxamid
- (±)-7-Chlor-1-(4-chlorphenyl)-*N*,4-dimethyl-8-(trifluormethoxy)-4,5-dihydro-3*H*-2,3-benzodiazepin-3-carboxamid
- 7-Chlor-1-(4-chlorphenyl)-*N*,4-dimethyl-8-(trifluormethoxy)-4,5-dihydro-3*H*-2,3-benzodiazepin-3-carboxamid, Enantiomer 1
- (4*S*)-1-[4-(3,5-Dimethyl-1*H*-pyrazol-1-yl)phenyl]-8-methoxy-*N*,4-dimethyl-4,5-dihydro-3*H*-2,3-benzodiazepin-3-carboxamid
- (4*S*)-8-Methoxy-*N*,4-dimethyl-1-[4-(morpholin-4-yl)phenyl]-4,5-dihydro-3*H*-2,3-benzodiazepin-3-carboxamid
- (4*S*)-1-[4-(4-Isoxazolyl)phenyl]-8-methoxy-*N*,4-dimethyl-4,5-dihydro-3*H*-2,3-benzodiazepin-3-carboxamid
- (4*S*)-8-Methoxy-*N*,4-dimethyl-1-[4-(1-methyl-1H-pyrazol-5-yl)phenyl]-4,5-dihydro-3*H-*2,3-benzodiazepin-3-carboxamid
- (4*S*)-1-[4-(3,5-Dimethyl-4-isoxazolyl)phenyl]-8-methoxy-*N*,4-dimethyl-4,5-dihydro-3*H-*2,3-benzodiazepin-3-carboxamid
- (4*S*)-8-Methoxy-*N*,4-dimethyl-1-[4-(1-methyl-1*H*-pyrazol-4-yl)phenyl]-4,5-dihydro-3*H-*2,3-benzodiazepin-3-carboxamid
- (4*S*)-8-Methoxy-*N*,4-dimethyl-1-[4-(1,3,5-trimethyl-1*H*-pyrazol-4-yl)phenyl]-4,5-dihydro-3*H*-2,3-benzodiazepin-3-carboxamid
- (4*S*)-8-Methoxy-*N*,4-dimethyl-1-[4-(1*H*-pyrazol-5-yl)phenyl]-4,5-dihydro-3*H*-2,3-benzodiazepin-3-carboxamid
- (4*S*)-1-[4-(3-Cyclopropyl-5-ethyl-1*H*-pyrazol-1-yl)phenyl]-8-methoxy-*N*,4-dimethyl-4,5-dihydro-3*H*-2,3-benzodiazepin-3-carboxamid
- (4*S*)-1-[4-(5-Cyclopropyl-3-ethyl-1*H*-pyrazol-1-yl)phenyl]-8-methoxy-*N*,4-dimethyl-4,5-dihydro-3*H*-2,3-benzodiazepin-3-carboxamid
- (4*S*)-8-Methoxy-1-{4-[3-(methoxymethyl)-5-methyl-1*H*-pyrazol-1-yl]phenyl}-*N*,4-dimethyl-4,5-dihydro-3*H*-2,3-benzodiazepin-3-carboxamid
- (4*S*)-8-Methoxy-1-{4-[5-(methoxymethyl)-3-methyl-1*H*-pyrazol-1-yl]phenyl}-*N*,4-dimethyl-4,5-dihydro-3*H*-2,3-benzodiazepin-3-carboxamid
- (4*S*)-1-{4-[5-Cyclopropyl-3-(pyridin-2-yl)-1*H*-pyrazol-1-yl]phenyl}-8-methoxy-*N*,4-dimethyl-4,5-dihydro-3*H*-2,3-benzodiazepin-3-carboxamid
- (4*S*)-1-{4-[3-Cyclopropyl-5-(pyridin-2-yl)-1*H*-pyrazol-1-yl]phenyl}-8-methoxy-*N*,4-dimethyl-4,5-dihydro-3*H*-2,3-benzodiazepin-3-carboxamid
- (4*S*)-8-Methoxy-*N*,4-dimethyl-1-[4-(1*H*-tetrazol-1-yl)phenyl]-4,5-dihydro-3*H*-2,3-benzodiazepin-3-carboxamid
- (±)-1-[3-(3,5-Dimethylisoxazol-4-yl)phenyl]-7,8-dimethoxy-*N*,4-dimethyl-4,5-dihydro-3*H-*2,3-benzodiazepin-3-carboxamid
- (±)-1-(4-Chlorphenyl)-*N*,4-dimethyl-8-[2-(morpholin-4-yl)ethoxy]-4,5-dihydro-3*H*-2,3-benzodiazepin-3-carboxamid
- (±)-1-(4-Chlorphenyl)-*N*,4-dimethyl-8-[2-(pyrrolidin-1-yl)ethoxy]-4,5-dihydro-3*H*-2,3-benzodiazepin-3-carboxamid
- (±)-7,8-Dimethoxy-*N*,4-dimethyl-1-[4-(2-oxooxazolidin-3-yl)phenyl]-4,5-dihydro-3*H*-2,3-benzodiazepin-3-carboxamid
- (4*S*)-7,8-Dimethoxy-*N*,4-dimethyl-1-[4-(2-oxooxazolidin-3-yl)phenyl]-4,5-dihydro-3*H*-2,3-benzodiazepin-3-carboxamid
- (±)-7,8-Dimethoxy-*N*,4-dimethyl-1-[4-(2-oxopiperidin-1-yl)phenyl]-4,5-dihydro-3*H*-2,3-benzodiazepin-3-carboxamid
- (±)-1-[4-(4-Benzyl-2-oxopiperazin-1-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4-(4-methyl-2-oxo-1,4-diazepan-1-yl)phenyl]-4,5-dihydro-3*H*-2,3-benzodiazepin-3-carboxamid
- (±)-7,8-Dimethoxy-*N*,4-dimethyl-1-[4-(2-oxo-1,3-oxazinan-3-yl)phenyl]-4,5-dihydro-3*H-*2,3-benzodiazepin-3-carboxamid
- (±)-7,8-Dimethoxy-*N*,4-dimethyl-1-[4-(2-oxopyrrolidin-1-yl)phenyl]-4,5-dihydro-3*H*-2,3-benzodiazepin-3-carboxamid
- (±)-7,8-Dimethoxy-*N*,4-dimethyl-1-[4-(3-oxomorpholin-4-yl)phenyl]-4,5-dihydro-3*H*-2,3-benzodiazepin-3-carboxamid
- (4*S*)-7,8-Dimethoxy-*N*,4-dimethyl-1-[4-(3-oxomorpholin-4-yl)phenyl]-4,5-dihydro-3*H*-2,3-benzodiazepin-3-carboxamid
- (±)-7,8-Dimethoxy-*N*,4-dimethyl-1-[4-(2-methyl-5-oxomorpholin-4-yl)phenyl]-4,5-dihydro-3*H*-2,3-benzodiazepin-3-carboxamid (Stereoisomerengemisch)
- (±)-7,8-Dimethoxy-*N*,4-dimethyl-1-[4-(2-methyl-3-oxomorpholin-4-yl)phenyl]-4,5-dihydro-3*H*-2,3-benzodiazepin-3-carboxamid (Stereoisomerengemisch)
- (±)-7,8-Dimethoxy-*N*,4-dimethyl-1-[4-(4-methyl-2-oxopiperazin-1-yl)phenyl]-4,5-dihydro-3*H*-2,3-benzodiazepin-3-carboxamid
- (4*S*)-7,8-Dimethoxy-*N*,4-dimethyl-1-[4-(4-methyl-2-oxopiperazin-1-yl)phenyl]-4,5-dihydro-3*H*-2,3-benzodiazepin-3-carboxamid
- (4*R*)-7,8-Dimethoxy-*N*,4-dimethyl-1-[4-(4-methyl-2-oxopiperazin-1-yl)phenyl]-4,5-dihydro-3*H*-2,3-benzodiazepin-3-carboxamid
- (±)-7,8-Dimethoxy-*N*,4-dimethyl-1-[4-(2-oxopiperazin-1-yl)phenyl]-4,5-dihydro-3*H*-2,3-benzodiazepin-3-carboxamid
- (±)-7,8-Dimethoxy-*N*,4-dimethyl-1-[4-(1-methyl-1*H*-1,2,3-triazol-4-yl)phenyl]-4,5-dihydro-3*H*-2,3-benzodiazepin-3-carboxamid
- (4*S*)-7,8-Dimethoxy-*N*,4-dimethyl-1-[4-(1-methyl-1*H*-1,2,3-triazol-4-yl)phenyl]-4,5-dihydro-3*H*-2,3-benzodiazepin-3-carboxamid
- (±)-1-[4-(2,4-Dimethylthiazol-5-yl)phenyl]-7,8-dimethoxy-*N*,4-dimethyl-4,5-dihydro-3*H-*2,3-benzodiazepin-3-carboxamid
- (4*S*)-1-[4-(2,4-Dimethylthiazol-5-yl)phenyl]-7,8-dimethoxy-*N*,4-dimethyl-4,5-dihydro-3*H-*2,3-benzodiazepin-3-carboxamid
- (4*R*)-1-[4-(2,4-Dimethylthiazol-5-yl)phenyl]-7,8-dimethoxy-*N*,4-dimethyl-4,5-dihydro-3*H-*2,3-benzodiazepin-3-carboxamid
- (±)-1-[4-(1,2-Dimethyl-1*H*-imidazol-5-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-{4-[2-(trifluormethyl)pyridin-3-yl]phenyl}-4,5-dihydro-3*H*-2,3-benzodiazepin-3-carboxamid
- (±)-1-[4-(6-Hydroxypyridin-3-yl)phenyl]-7,8-dimethoxy-*N*,4-dimethyl-4,5-dihydro-3*H*-2,3-benzodiazepin-3-carboxamid
- (4*S*)-1-[4-(6-Hydroxypyridin-3-yl)phenyl]-7,8-dimethoxy-*N*,4-dimethyl-4,5-dihydro-3*H-*2,3-benzodiazepin-3-carboxamid
- (±)-7,8-Dimethoxy-*N*,4-dimethyl-1-{4-[6-(trifluormethyl)pyridin-3-yl]phenyl}-4,5-dihydro-3*H*-2,3-benzodiazepin-3-carboxamid
- (±)-7,8-Dimethoxy-*N*,4-dimethyl-1-[4-(1,3,5-trimethyl-1*H*-pyrazol-4-yl)phenyl]-4,5-dihydro-3*H*-2,3-benzodiazepin-3-carboxamid
- (4*R*)-7,8-Dimethoxy-*N*,4-dimethyl-1-[4-(1,3,5-trimethyl-1*H*-pyrazol-4-yl)phenyl]-4,5-dihydro-3*H*-2,3-benzodiazepin-3-carboxamid
- (4*S*)-7,8-Dimethoxy-*N*,4-dimethyl-1-[4-(1,3,5-trimethyl-1*H*-pyrazol-4-yl)phenyl]-4,5-dihydro-3*H*-2,3-benzodiazepin-3-carboxamid
- (±)-1-[4-(Isoxazol-4-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[3-(1,3,5-trimethyl-1*H*-pyrazol-4-yl)phenyl]-4,5-dihydro-3*H*-2,3-benzodiazepin-3-carboxamid
- (±)-7,8-Dimethoxy-*N*,4-dimethyl-1-[3-(1-methyl-1*H*-pyrazol-4-yl)phenyl]-4,5-dihydro-3*H-*2,3-benzodiazepin-3-carboxamid
- (±)-7,8-Dimethoxy-*N*,4-dimethyl-1-[3-(1-methyl-1*H*-pyrazol-5-yl)phenyl]-4,5-dihydro-3*H-*2,3-benzodiazepin-3-carboxamid
- (±)-1-[4-Fluoro-3-(1,3,5-trimethyl-1*H*-pyrazol-4-yl)phenyl]-7,8-dimethoxy-*N*,4-dimethyl-4,5-dihydro-3*H*-2,3-benzodiazepin-3-carboxamid
- (±)-1-[4-Fluoro-3-(1-methyl-1*H*-pyrazol-4-yl)phenyl]-7,8-dimethoxy-*N*,4-dimethyl-4,5-dihydro-3*H*-2,3-benzodiazepin-3-carboxamid
- (±)-1-[3-(3,5-Dimethylisoxazol-4-yl)-4-fluorophenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-(3'-nitrobiphenyl-4-yl)-4,5-dihydro-3*H*-2,3-benzodiazepin-3-carboxamid
- (±)-1-(Biphenyl-4-yl)-7,8-dimethoxy-*N*,4-dimethyl-4,5-dihydro-3*H*-2,3-benzodiazepin-3-carboxamid
- (±)-1-(2',4'-Dichlorbiphenyl-4-yl)-7,8-dimethoxy-*N*,4-dimethyl-4,5-dihydro-3*H*-2,3-benzodiazepin-3-carboxamid
- (±)-1-(4'-Fluorbiphenyl-4-yl)-7,8-dimethoxy-*N*,4-dimethyl-4,5-dihydro-3*H*-2,3-benzodiazepin-3-carboxamid
- (±)-1-(4'-Chlorbiphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-(4'-methylbiphenyl-4-yl)-4,5-dihydro-3*H*-2,3benzodiazepin-3-carboxamid
- (±)-7,8-Dimethoxy-1-(4'-methoxybiphenyl-4-yl)-*N*,4-dimethyl-4,5-dihydro-3*H*-2,3-benzodiazepin-3-carboxamid
- (±)-7,8-Dimethoxy-1-[4-(6-methoxypyridin-3-yl)phenyl]-*N*,4-dimethyl-4,5-dihydro-3*H-*2,3-benzodiazepin-3-carboxamid
- (±)-7,8-Dimethoxy-*N*,4-dimethyl-1-[4'-(methylsulfinyl)biphenyl-4-yl]-4,5-dihydro-3*H*-2,3-benzodiazepin-3-carboxamid
- (±)-7,8-Dimethoxy-*N*,4-dimethyl-1-{2'-[(methylsulfonyl)amino]biphenyl-4-yl}-4,5-dihydro-3*H*-2,3-benzodiazepin-3-carboxamid
- (±)-7,8-Dimethoxy-*N*,4-dimethyl-1-[2'-(methylsulfonyl)biphenyl-4-yl]-4,5-dihydro-3*H*-2,3-benzodiazepin-3-carboxamid
- (±)-7,8-Dimethoxy-*N*,4-dimethyl-1-{4'-[(methylsulfonyl)amino]biphenyl-4-yl}-4,5-dihydro-3*H*-2,3-benzodiazepin-3-carboxamid
- (±)-7,8-Dimethoxy-*N*,4-dimethyl-1-{3'-[(methylsulfonyl)amino]biphenyl-4-yl}-4,5-dihydro-3*H*-2,3-benzodiazepin-3-carboxamid
- (±)-7,8-Dimethoxy-*N*,4-dimethyl-1-(2'-methylbiphenyl-4-yl)-4,5-dihydro-3*H*-2,3-benzodiazepin-3-carboxamid
- (±)-7,8-Dimethoxy-*N*,4-dimethyl-1-[3'-(methylsulfonyl)biphenyl-4-yl]-4,5-dihydro-3*H*-2,3-benzodiazepin-3-carboxamid
- (±)-7,8-Dimethoxy-1-[4-(2-methoxypyrimidin-5-yl)phenyl]-*N*,4-dimethyl-4,5-dihydro-3*H-*2,3-benzodiazepin-3-carboxamid
- (±)-1-(3'-Cyan-4'-fluorbiphenyl-4-yl)-7,8-dimethoxy-*N*,4-dimethyl-4,5-dihydro-3*H*-2,3-benzodiazepin-3-carboxamid
- (±)-7,8-Dimethoxy-1-[4-(2-methoxypyridin-3-yl)phenyl]-*N*,4-dimethyl-4,5-dihydro-3*H-*2,3-benzodiazepin-3-carboxamid
- (±)-1-(3'-Carbamoylbiphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4'-(pyrrolidin-1-ylcarbonyl)biphenyl-4-yl]-4,5-dihydro-3*H*-2,3-benzodiazepine-3-carboxamide
- (±)-7,8-Dimethoxy-*N*,4-dimethyl-1-[4'-(morpholin-4-ylcarbonyl)biphenyl-4-yl]-4,5-dihydro-3*H*-2,3-benzodiazepin-3-carboxamid
- (±)-7,8-Dimethoxy-1-[4-(5-methoxypyridin-3-yl)phenyl]-*N*,4-dimethyl-4,5-dihydro-3*H-*2,3-benzodiazepin-3-carboxamid
- (±)-7,8-Dimethoxy-*N*,4-dimethyl-1-[4-(5-methylpyridin-3-yl)phenyl]-4,5-dihydro-3*H*-2,3-benzodiazepin-3-carboxamid
- (±)-7,8-Dimethoxy-*N*,4-dimethyl-1-[4-(4-methylpyridin-3-yl)phenyl]-4,5-dihydro-3*H*-2,3-benzodiazepin-3-carboxamid
- (±)-1-[4'-(Cyclopropylcarbamoyl)biphenyl-4-yl]-7,8-dimethoxy-*N*,4-dimethyl-4,5-dihydro-3*H*-2,3-benzodiazepin-3-carboxamid
- (±)-1-[4-(3-Fluorpyridin-4-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[3'-(trifluormethyl)biphenyl-4-yl]-4,5-dihydro-3*H*-2,3-benzodiazepin-3-carboxamid
- (±)-7,8-Dimethoxy-*N*,4-dimethyl-1-[4'-(trifluormethyl)biphenyl-4-yl]-4,5-dihydro-3*H*-2,3-benzodiazepin-3-carboxamid
- (±)-7,8-Dimethoxy-1-(3'-methoxybiphenyl-4-yl)-*N*,4-dimethyl-4,5-dihydro-3*H*-2,3-benzodiazepin-3-carboxamid
- (±)-1-[4'-(5-Chlorthien-2-yl)biphenyl-4-yl]-7,8-dimethoxy-*N*,4-dimethyl-4,5-dihydro-3*H-*2,3-benzodiazepin-3-carboxamid
- (±)-1-(3'-Fluorbiphenyl-4-yl)-7,8-dimethoxy-*N*,4-dimethyl-4,5-dihydro-3*H*-2,3-benzodiazepin-3-carboxamid
- (±)-7,8-Dimethoxy-1-(2'-methoxybiphenyl-4-yl)-*N*,4-40dimethyl-4,5-dihydro-3*H*-2,3-benzodiazepin-3-carboxamid
- (±)-7,8-Dimethoxy-*N*,4-dimethyl-1-[2'-(trifluormethyl)biphenyl-4-yl]-4,5-dihydro-3*H*-2,3-benzodiazepin-3-carboxamid
- (±)-1-(2'-Chlorbiphenyl-4-yl)-7,8-dimethoxy-*N*,4-dimethyl-4,5-dihydro-3*H*-2,3-benzodiazepin-3-carboxamid
- (±)-1-(2'-Fluorbiphenyl-4-yl)-7,8-dimethoxy-*N*,4-dimethyl-4,5-dihydro-3*H*-2,3-benzodiazepin-3-carboxamid
- (±)-1-[4'-(Hydroxymethyl)biphenyl-4-yl]-7,8-dimethoxy-*N*,4-dimethyl-4,5-dihydro-3*H-*2,3-benzodiazepin-3-carboxamid
- (±)-7,8-Dimethoxy-*N*,4-dimethyl-1-[4'-(trifluormethoxy)biphenyl-4-yl]-4,5-dihydro-3*H-*2,3-benzodiazepin-3-carboxamid
- (±)-7,8-Dimethoxy-*N*,4-dimethyl-1-[3'-(pyrrolidin-1-ylcarbonyl)biphenyl-4-yl]-4,5-dihydro-3*H*-2,3-benzodiazepin-3-carboxamid
- (±)-7,8-Dimethoxy-*N*,4-dimethyl-1-[3'-(piperidin-1-ylcarbonyl)biphenyl-4-yl]-4,5-dihydro-3*H*-2,3-benzodiazepin-3-carboxamid
- (±)-7,8-Dimethoxy-*N*,4-dimethyl-1-[3'-(morpholin-4-ylcarbonyl)biphenyl-4-yl]-4,5-dihydro-3*H*-2,3-benzodiazepin-3-carboxamid
- (±)-1-[3'-(Cyclopropylcarbamoyl)biphenyl-4-yl]-7,8-dimethoxy-*N*,4-dimethyl-4,5-dihydro-3*H*-2,3-benzodiazepin-3-carboxamid
- (±)-1-(2',4'-Difluorbiphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4-(1-methyl-1*H*-pyrazol-5-yl)phenyl]-4,5-dihydro-3H-2,3-benzodiazepin-3-carboxamid
- (±)-7,8-Dimethoxy-*N*,4-dimethyl-1-(4'-nitrobiphenyl-4-yl)-4,5-dihydro-3*H*-2,3-benzodiazepin-3-carboxamid
- (±)-7,8-Dimethoxy-*N*,4-dimethyl-1-[4-(pyridin-3-yl)phenyl]-4,5-dihydro-3*H*-2,3-benzodiazepin-3-carboxamid
- (±)-7,8-Dimethoxy-1-[4-(4-methoxypyridin-3-yl)phenyl]-*N*,4-dimethyl-4,5-dihydro-3*H-*2,3-benzodiazepin-3-carboxamid
- (±)-1-(3'-Cyanbiphenyl-4-yl)-7,8-dimethoxy-*N*,4-dimethyl-4,5-dihydro-3*H*-2,3-benzodiazepin-3-carboxamid
- (±)-1-(4'-Cyanbiphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[2'-(trifluormethoxy)biphenyl-4-yl]-4,5-dihydro-3*H-*2,3-benzodiazepin-3-carboxamid
- (±)-7,8-Dimethoxy-*N*,4-dimethyl-1-[4'-(methylsulfonyl)biphenyl-4-yl]-4,5-dihydro-3*H*-2,3-benzodiazepin-3-carboxamid
- (±)-1-(2'-Cyanbiphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4'-(morpholin-4-yl)biphenyl-4-yl]-4,5-dihydro-3*H-*2,3-benzodiazepin-3-carboxamid
- (±)-7,8-Dimethoxy-*N*,4-dimethyl-1-[4-(pyrimidin-5-yl)phenyl]-4,5-dihydro-3*H*-2,3-benzodiazepin-3-carboxamid
- (±)-1-[2'-(Hydroxymethyl)biphenyl-4-yl]-7,8-dimethoxy-*N*,4-dimethyl-4,5-dihydro-3*H-*2,3-benzodiazepie-3-carboxamid
- (±)-1-(3'-{[2-(Dimethylamino)ethyl]carb-amoyl}biphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-(3'-sulfamoylbiphenyl-4-yl)-4,5-dihydro-3*H*-2,3-benzodiazepin-3-carboxamid
- (±)-7,8-Dimethoxy-*N*,4-dimethyl-1-[4'-(methylsulfamoyl)biphenyl-4-yl]-4,5-dihydro-3*H-*2,3-benzodiazepin-3-carboxamid
- (±)-7,8-Dimethoxy-*N*,4-dimethyl-1-[4-(1-methyl-1*H*-pyrrol-2-yl)phenyl]-4,5-dihydro-3*H-*2,3-benzodiazepin-3-carboxamid
- (±)-7,8-Dimethoxy-*N*,4-dimethyl-1-[4-(6-methylpyridin-3-yl)phenyl]-4,5-dihydro-3*H*-2,3-benzodiazepin-3-carboxamid
- (±)-1-[4'-(Cyclopropylsulfamoyl)biphenyl-4-yl]-7,8-dimethoxy-*N*,4-dimethyl-4,5-dihydro-3*H*-2,3-benzodiazepin-3-carboxamid
- (±)-1-(3'-Fluor-5'-hydroxybiphenyl-4-yl)-7,8-dimethoxy-*N*,4-dimethyl-4,5-dihydro-3*H*-2,3-benzodiazepin-3-carboxamid
- (±)-1-(3'-Fluor-5'-methylbiphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[3'-(methylsulfamoyl)biphenyl-4-yl]-4,5-dihydro-3*H-*2,3-benzodiazepin-3-carboxamid
- (±)-1-[4-(5-Fluorpyridin-3-yl)phenyl]-7,8-dimethoxy-*N*,4-dimethyl-4,5-dihydro-3*H*-2,3-benzodiazepin-3-carboxamid
- (±)-1-[4-(4-Fluorpyridin-3-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4-(2-methylpyridin-3-yl)phenyl]-4,5-dihydro-3*H*-2,3-benzodiazepin-3-carboxamid
- (±)-7,8-Dimethoxy-1-[4-(2-methoxypyridin-4-yl)phenyl]-*N*,4-dimethyl-4,5-dihydro-3*H-*2,3-benzodiazepin-3-carboxamid
- (±)-1-[4-(5-Cyanpyridin-3-yl)phenyl]-7,8-dimethoxy-*N*,4-dimethyl-4,5-dihydro-3*H*-2,3-benzodiazepin-3-carboxamid
- (4*R*)-7,8-Dimethoxy-*N*,4-dimethyl-1-[4-(morpholin-4-yl)phenyl]-4,5-dihydro-3*H*-2,3-benzodiazepin-3-carboxamid
- (4*S*)-7,8-Dimethoxy-*N*,4-dimethyl-1-[4-(morpholin-4-yl)phenyl]-4,5-dihydro-3*H*-2,3-benzodiazepin-3-carboxamid
- (4*S*)-7,8-Dimethoxy-*N*,4-dimethyl-1-[4-(4-methylpiperazin-1-yl)phenyl]-4,5-dihydro-3*H-*2,3-benzodiazepin-3-carboxamid
- (±)-1-[4-(Azetidin-1-yl)phenyl]-7,8-dimethoxy-*N*,4-dimethyl-4,5-dihydro-3*H*-2,3-benzodiazepin-3-carboxamid
- (4*R*)-1-[4-(Azetidin-1-yl)phenyl]-7,8-dimethoxy-*N*,4-dimethyl-4,5-dihydro-3*H*-2,3-benzodiazepin-3-carboxamid
- (4*S*)-1-[4-(Azetidin-1-yl)phenyl]-7,8-dimethoxy-*N*,4-dimethyl-4,5-dihydro-3*H*-2,3-benzodiazepin-3-carboxamid
- (±)-1-[4-(3-Fluorazetidin-1-yl)phenyl]-7,8-dimethoxy-*N*,4-dimethyl-4,5-dihydro-3*H*-2,3-benzodiazepin-3-carboxamid
- (4*R*)-1-[4-(3-Fluorazetidin-1-yl)phenyl]-7,8-dimethoxy-*N*,4-dimethyl-4,5-dihydro-3*H*-2,3-benzodiazepin-3-carboxamid
- (4*S*)-1-[4-(3-Fluorazetidin-1-yl)phenyl]-7,8-dimethoxy-*N*,4-dimethyl-4,5-dihydro-3*H*-2,3-benzodiazepin-3-carboxamid
- (±)-1-[4-(4-Hydroxypiperidin-1-yl)phenyl]-7,8-dimethoxy-*N*,4-dimethyl-4,5-dihydro-3*H-*2,3-benzodiazepin-3-carboxamid
- (4*S*)-1-[4-(4-Hydroxypiperidin-1-yl)phenyl]-7,8-dimethoxy-*N*,4-dimethyl-4,5-dihydro-3*H-*2,3-benzodiazepin-3-carboxamid
- (±)-7,8-Dimethoxy-*N*,4-dimethyl-1-[4-(piperazin-1-yl)phenyl]-4,5-dihydro-3*H*-2,3-benzodiazepin-3-carboxamid
- (4*S*)-7,8-Dimethoxy-*N*,4-dimethyl-1-[4-(piperazin-1-yl)phenyl]-4,5-dihydro-3*H*-2,3-benzodiazepin-3-carboxamid
- (±)-7,8-Dimethoxy-*N*,4-dimethyl-1-[4-(4-methylpiperazin-1-yl)phenyl]-4,5-dihydro-3*H-*2,3-benzodiazepin-3-carboxamid
- (±)-1-[4-(4-Acetylpiperazin-1-yl)phenyl]-7,8-dimethoxy-*N*,4-dimethyl-4,5-dihydro-3*H*-2,3-benzodiazepin-3-carboxamid
- (4*R*)-1-[4-(4-Acetylpiperazin-1-yl)phenyl]-7,8-dimethoxy-*N*,4-dimethyl-4,5-dihydro-3*H-*2,3-benzodiazepin-3-carboxamid
- (4*S*)-1-[4-(4-Acetylpiperazin-1-yl)phenyl]-7,8-dimethoxy-*N*,4-dimethyl-4,5-dihydro-3*H-*2,3-benzodiazepin-3-carboxamid
- (±)-7,8-Dimethoxy-*N*,4-dimethyl-1-{4-[4-(trifluoracetyl)piperazin-1-yl]phenyl}-4,5-dihydro-3*H*-2,3-benzodiazepin-3-carboxamid
- (±)-1- {4-[4-(2-Hydroxy-2-methylpropanoyl)piperazin-1-yl]phenyl}-7,8-dimethoxy-*N*,4-dimethyl-4,5-dihydro-3*H*-2,3-benzodiazepin-3-carboxamid
- (±)-7,8-Dimethoxy-*N*,4-dimethyl-1-{4-[4-(methylsulfonyl)piperazin-1-yl]phenyl}-4,5-dihydro-3*H*-2,3-benzodiazepin-3-carboxamid
- (4*S*)-1-[4-(1,1-Dioxidothiomorpholin-4-yl)phenyl]-7,8-diniethoxy-*N,*4-dimethyl-4,5-dihydro-3*H*-2,3-benzodiazepin-3-carboxamid
- (±)-7,8-Dimethoxy-*N*,4-dimethyl-1-[4-(3-oxopiperazin-1-yl)phenyl]-4,5-dihydro-3*H*-2,3-benzodiazepin-3-carboxamid
- (±)-7,8-Dimethoxy-*N*,4-dimethyl-1-[4-(4-methyl-3-oxopiperazin-1-yl)phenyl]-4,5-dihydro-3*H*-2,3-benzodiazepin-3-carboxamid
- (±)-7,8-Dimethoxy-*N*,4-dimethyl-1-[4-(piperidin-1-yl)phenyl]-4,5-dihydro-3*H*-2,3-benzodiazepin-3-carboxamid
- (±)-7,8-Dimethoxy-*N*,4-dimethyl-1-[3-(morpholin-4-yl)phenyl]-4,5-dihydro-3*H*-2,3-benzodiazepin-3-carboxamid
- (±)-1-[3-(3,3-Difluorazetidin-1-yl)phenyl]-7,8-dimethoxy-*N*,4-dimethyl-4,5-dihydro-3*H-*2,3-benzodiazepin-3-carboxamid
- (±)-1-[3-(Azetidin-1-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[3-(4-methylpiperazin-1-yl)phenyl]-4,5-dihydro-3*H-*2,3-benzodiazepin-3-carboxamid
- (±)-1-[4-Fluor-3-(morpholin-4-yl)phenyl]-7,8-dimethoxy-*N*,4-dimethyl-4,5-dihydro-3*H-*2,3-benzodiazepin-3-carboxamid
- (±)-1-[3-(3,3-Difluorazetidin-1-yl)-4-fluorphenyl]-7,8-dimethoxy-*N*,4-dimethyl-4,5-dihydro-3*H*-2,3-benzodiazepin-3-carboxamid
- (±)-1-[4-Fluor-3-(4-hydroxypiperidin-1-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4-(5-methyl-3-phenyl-1*H*-pyrazol-1-yl)phenyl]-4,5-dihydro-3*H*-2,3-benzodiazepin-3-carboxamid
- (4*S*)-7,8-Dimethoxy-*N*,4-dimethyl-1-[4-(5-methyl-3-phenyl-1*H*-pyrazol-1-yl)phenyl]-4,5-dihydro-3*H*-2,3-benzodiazepin-3-carboxamid
- (±)-1-[4-(5-Cyclopropyl-3-phenyl-1*H*-pyrazol-1-yl)phenyl]-7,8-dimethoxy-*N*,4-dimethyl-4,5-dihydro-3*H*-2,3-benzodiazepin-3-carboxamid
- (4*S*)-1-[4-(5-Cyclopropyl-3-phenyl-1*H*-pyrazol-1-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-{4-[3-phenyl-5-(trifluormethyl)-1*H*-pyrazol-1-yl]phenyl}-4,5-dihydro-3*H*-2,3-benzodiazepin-3-carboxamid
- (±)-1-{4-[3-(4-Fluorphenyl)-1*H*-pyrazol-1-yl]phenyl}-7,8-dimethoxy-*N*,4-dimethyl-4,5-dihydro-3*H*-2,3-benzodiazepin-3-carboxamid
- (4*S*)-1-{4-[3-(4-Fluorphenyl)-1*H*-pyrazol-1-yl]phenyl}-7,8-dimethoxy-*N*,4-dimethyl-4,5-dihydro-3*H*-2,3-benzodiazepin-3-carboxamid
- (4*R*)-1-{4-[3-(4-Fluorphenyl)-1*H*-pyrazol-1-yl]phenyl}-7,8-dimethoxy-*N*,4-dimethyl-4,5-dihydro-3*H*-2,3-benzodiazepin-3-carboxamid
- (±)-7,8-Dimethoxy-*N*,4-dimethyl-1-{4-[5-methyl-3-(trifluormethyl)-1*H*-pyrazol-1-yl]phenyl}-4,5-dihydro-3*H*-2,3-benzodiazepin-3-carboxamid
- (±)-7,8-Dimethoxy-*N*,4-dimethyl-1-[4-(1*H*-1,2,4-triazol-1-yl)phenyl]-4,5-dihydro-3*H*-2,3-benzodiazepin-3-carboxamid
- (±)-7,8-Dimethoxy-*N*,4-dimethyl-1-[4-(5-methyl-1*H*-1,2,4-triazol-1-yl)phenyl]-4,5-dihydro-3*H*-2,3-benzodiazepin-3-carboxamid
- (±)-1-[4-(3,5-Dimethyl-1*H*-1,2,4-triazol-1-yl)phenyl]-7,8-dimethoxy-*N*,4-dimethyl-4,5-dihydro-3*H*-2,3-benzodiazepin-3-carboxamid
- (±)-8-tert-Butyl-1-[4-(3,5-dimethylisoxazol-4-yl)phenyl]-*N*,4-dimethyl-4,5-dihydro-3*H-*2,3-benzodiazepin-3-carboxamid
- (±)-7-Chlor-1-[4-(3,5-dimethylisoxazol-4-yl)phenyl]-*N*,4-dimethyl-8-(trifluormethoxy)-4,5-dihydro-3*H*-2,3-benzodiazepin-3-carboxamid
- (4*S*)-7-Chlor-1-[4-(3,5-dimethylisoxazol-4-yl)phenyl]-*N*,4-dimethyl-8-(trifluormethoxy)-4,5-dihydro-3*H*-2,3-benzodiazepin-3-carboxamid
- (4*R*)-7-Chlor-1-[4-(3,5-dimethylisoxazol-4-yl)phenyl]-*N*,4-dimethyl-8-(trifluormethoxy)-4,5-dihydro-3*H*-2,3-benzodiazepin-3-carboxamid
- (±)-8-Chlor-1-[4-(3,5-dimethylisoxazol-4-yl)phenyl]-7-methoxy-*N*,4-dimethyl-4,5-dihydro-3*H*-2,3-benzodiazepin-3-carboxamid
- (4*S*)-8-Chlor-1-[4-(3,5-dimethylisoxazol-4-yl)phenyl]-7-methoxy-N,4-dimethyl-4,5-dihydro-3*H*-2,3-benzodiazepin-3-carboxamid
- (4*R*)-8-Chlor-1-[4-(3,5-dimethylisoxazol-4-yl)phenyl]-7-methoxy-*N*,4-dimethyl-4,5-dihydro-3*H*-2,3-benzodiazepin-3-carboxamid
- (±)-1-[4-(3,5-Dimethylisoxazol-4-yl)phenyl]-*N*,4,8-trimethyl-4,5-dihydro-3*H*-2,3-benzodiazepin-3-carboxamid
- (±)-7,8-Bis(difluormethoxy)-1-[4-(3,5-dimethylisoxazol-4-yl)phenyl]-*N*,4-dimethyl-4,5-dihydro-3*H*-2,3-benzodiazepin-3-carboxamid
- (±)-1-[4-(3,5-Dimethylisoxazol-4-yl)phenyl]-7,8-diethoxy-*N*,4-dimethyl-4,5-dihydro-3*H-*2,3-benzodiazepin-3-carboxamid
- (±)-7-(Difluormethoxy)-1-[4-(3,5-dimethylisoxazol-4-yl)phenyl]-8-methoxy-*N*,4-dimethyl-4,5-dihydro-3*H*-2,3-benzodiazepin-3-carboxamid
- (4*S*)-7-(Difluormethoxy)-1-[4-(3,5-dimethylisoxazol-4-yl)phenyl]-8-methoxy-*N*,4-dimethyl-4,5-dihydro-3*H*-2,3-benzodiazepin-3-carboxamid
- (4*R*)-7-(Difluormethoxy)-1-[4-(3,5-dimethylisoxazol-4-yl)phenyl]-8-methoxy-*N*,4-dimethyl-4,5-dihydro-3*H*-2,3-benzodiazepin-3-carboxamid
- (±)-7-(Difluormethoxy)-8-methoxy-*N*,4-dimethyl-1-[4-(4-methylpiperazin-1-yl)phenyl]-4,5-dihydro-3*H*-2,3-benzodiazepin-3-carboxamid
- (4*R*)-7-(Difluormethoxy)-8-methoxy-*N*,4-dimethyl-1-[4-(4-methylpiperazin-1-yl)phenyl]-4,5-dihydro-3*H*-2,3-benzodiazepin-3-carboxamid
- (4*S*)-7-(Difluormethoxy)-8-methoxy-*N*,4-dimethyl-1-[4-(4-methylpiperazin-1-yl)phenyl]-4,5-dihydro-3*H*-2,3-benzodiazepin-3-carboxamid
- (±)-1-(4-Chlorphenyl)-*N*,4-dimethyl-8-[3-(4-methylpiperazin-1-yl)propoxyl-4,5-dihydro-3*H*-2,3-benzodiazepin-3-carboxamid
- (4*S*)-1-(4-Chlorphenyl)-*N*,4-dimethyl-8-[3-(4-methylpiperazin-1-yl)propoxy]-4,5-dihydro-3*H*-2,3-benzodiazepin-3-carboxamid
- (±)-1-(4-Chlorphenyl)-*N*,4-dimethyl-8-[3-(morpholin-4-yl)propoxy]-4,5-dihydro-3*H*-2,3-benzodiazepin-3-carboxamid
- (±)-1-(4-Chlorphenyl)-*N*,4-dimethyl-8-[2-(4-methylpiperazin-1-yl)ethoxy]-4,5-dihydro-3*H-*2,3-benzodiazepin-3-carboxamid
- (±)-1-(4-Chlorphenyl)-*N*,4-dimethyl-8-[(6-methylpyridin-2-yl)methoxy]-4,5-dihydro-3*H-*2,3-benzodiazepin-3-carboxamid
- (±)-1-[4-(3,5-Dimethylisoxazol-4-yl)phenyl]-8-hydroxy-*N*,4-dimethyl-4,5-dihydro-3*H*-2,3-benzodiazepin-3-carboxamid
- (±)-1-[4-(3,5-Dimethylisoxazol-4-yl)phenyl]-*N*,4-dimethyl-8-[3-(morpholin-4-yl)propoxy]-4,5-dihydro-3*H*-2,3-benzodiazepin-3-carboxamid
- (±)-7-Cyan-1-[4-(3,5-dimethylisoxazol-4-yl)phenyl]-8-methoxy-*N*,4-dimethyl-4,5-dihydro-3*H*-2,3-benzodiazepin-3-carboxamid
- (±)-8-Acetamido-*N*,4-dimethyl-1-[4-(morpholin-4-yl)phenyl]-4,5-dihydro-3*H*-2,3-benzodiazepin-3-carboxamid
- (±)-8-Acetamido-*N*,4-dimethyl-1-[4-(4-methylpiperazin-1-yl)phenyl]-4,5-dihydro-3*H*-2,3-benzodiazepin-3-carboxamid
- (±)-8-Acetamido-1-[4-(3,5-dimethylisoxazol-4-yl)phenyl]-*N*,4-dimethyl-4,5-dihydro-3*H-*2,3-benzodiazepin-3-carboxamid
- (±)-1-(4-Chlorphenyl)-8-(3,5-dimethyl-1*H*-pyrazol-1-yl)-*N*,4-dimethyl-4,5-dihydro-3*H-*2,3-benzodiazepin-3-carboxamid
- (±)-1-[4-(3,5-Dimethylisoxazol-4-yl)phenyl]-8-(3,5-dimethyl-1*H*-pyrazol-1-yl)-*N*,4-dimethyl-4,5-dihydro-3*H*-2,3-benzodiazepin-3-carboxamid
- (±)-1-[4-(3,5-Dimethylisoxazol-4-yl)phenyl]-*N*,4-dimethyl-8-(morpholin-4-yl)-4,5-dihydro-3*H*-2,3-benzodiazepin-3-carboxamid
- (4*R*)-1-[4-(3,5-Dimethylisoxazol-4-yl)phenyl]-*N*,4-dimethyl-8-(morpholin-4-yl)-4,5-dihydro-3*H*-2,3-benzodiazepin-3-carboxamid
- (4*S*)-1-[4-(3,5-Dimethylisoxazol-4-yl)phenyl]-*N*,4-dimethyl-8-(morpholin-4-yl)-4,5-dihydro-3*H*-2,3-benzodiazepin-3-carboxamid
- (4*S*)-8-Methoxy-*N*,4-dimethyl-1-[4-(3-oxomorpholin-4-yl)phenyl]-4,5-dihydro-3*H*-2,3-benzodiazepin-3-carboxamid
- (±)-1-{4-[4-(2-Hydroxyethyl)piperazin-1-yl]phenyl}-7,8-dimethoxy-N,4-dimethyl-4,5-dihydro-3*H*-2,3-benzodiazepin-3-carboxamid
- (±)-7,8-Dimethoxy-1-[4-(4-methoxypiperidin-1-yl)phenyl]-N,4-dimethyl-4,5-dihydro-3*H-*2,3-benzodiazepin-3-carboxamid
- (4*R*)-7,8-Dimethoxy-1-[4-(4-methoxypiperidin-1-yl)phenyl]-N,4-dimethyl-4,5-dihydro-3*H-*2,3-benzodiazepin-3-carboxamid
- (4*S*)-7,8-Dimiethoxy-1-[4-(4-methoxypiperidin-1-yl)phenyl]-N,4-dimethyl-4,5-dihydro-3*H-*2,3-benzodiazepin-3-carboxamid
- (±)-1-{4-[4-(Dimethylamino)piperidin-1-yl]phenyl}-7,8-dimethoxy-N,4-dimethyl-4,5-dihydro-3*H*-2,3-benzodiazepin-3-carboxamid
- (4*R*)-1-{4-[4-(Dimethylamino)piperidin-1-yl]phenyl}-7,8-dimethoxy-N,4-dimethyl-4,5-dihydro-3*H*-2,3-benzodiazepin-3-carboxamid
- (4*S*)-1-{4-[4-(Dimethylamino)piperidin-1-yl]phenyl}-7,8-dimethoxy-N,4-dimethyl-4,5-dihydro-3*H*-2,3-benzodiazepin-3-carboxamid
- (±)-1-[4-(3,3-Difluorazetidin-1-yl)phenyl]-7,8-dimethoxy-N,4-dimethyl-4,5-dihydro-3H-2,3-benzodiazepin-3-carboxamid
- (±)-1-[4-(4-Acetamidopiperidin-1-yl)phenyl]-7,8-dimethoxy-N,4-dimethyl-4,5-dihydro-3H-2,3-benzodiazepin-3-carboxamid
- (±)-1-{4-[4-(2-Hydroxyethyl)piperidin-1-yl]phenyl-7,8-dimethoxy-N,4-dimethyl-4,5-dihydro-3H-2,3-benzodiazepin-3-carboxamid
- (±)-1-[4-(3-Hydroxyazetidin-1-yl)phenyl]-7,8-dimethoxy-N,4-dimethyl-4,5-dihydro-3H-2,3-benzodiazepin-3-carboxamid
- (±)-1-[4-(3-Hydroxy-3-methylazetidin-1-yl)phenyl]-7,8-dimethoxy-N,4-dimethyl-4,5-dihydro-3H-2,3-benzodiazepin-3-carboxamid
- (4*R*)-1-[4-(3-Hydroxy-3-methylazetidin-1-yl)phenyl]-7,8-dimethoxy-N,4-dimethyl-4,5-dihydro-3H-2,3-benzodiazepin-3-carboxamid
- (4*S*)-1-[4-(3-Hydroxy-3-methylazetidin-1-yl)phenyl]-7,8-dimethoxy-N,4-dimethyl-4,5-dihydro-3H-2,3-benzodiazepin-3-carboxamid
- (±)-1-[4-(4-Isopropylpiperazin-1-yl)phenyl]-7,8-dimethoxy-N,4-dimethyl-4,5-dihydro-3H-2,3-benzodiazepin-3-carboxamid
- (±)-7,8-Dimethoxy-1-[4-(3-methoxyazetidin-1-yl)phenyl]-N,4-dimethyl-4,5-dihydro-3H-2,3-benzodiazepin-3-carboxamid
- (±)-1-[4-(4-Hydroxy-4-methylpiperidin-1-yl)phenyl]-7,8-dimethoxy-N,4-dimethyl-4,5-dihydro-3H-2,3-benzodiazepin-3-carboxamid
- (4*R*)-1-[4-(4-Hydroxy-4-methylpiperidin-1-yl)phenyl]-7,8-dimethoxy-N,4-dimethyl-4,5-dihydro-3H-2,3-benzodiazepin-3-carboxamid
- (4*S*)-1-[4-(4-Hydroxy-4-methylpiperidin-1-yl)phenyl]-7,8-dimethoxy-N,4-dimethyl-4,5-dihydro-3H-2,3-benzodiazepin-3-carboxamid
- (±)-7,8-Dimethoxy-N,4-dimethyl-1-{4-[4-(methylcarbamoyl)piperidin-1-yl]phenyl}-4,5-dihydro-3H-2,3-benzodiazepin-3-carboxamid
- (4*S*)-7,8-Dimethoxy-N,4-dimethyl-1-{4-[4-(methylcarbamoyl)piperidin-1-yl]phenyl}-4,5-dihydro-3H-2,3-benzodiazepin-3-carboxamid
- (*4R*)-7,8-Dimethoxy-N,4-dimethyl-1-{4-[4-(methylcarbamoyl)piperidin-1-yl]phenyl}-4,5-dihydro-3H-2,3-benzodiazepin-3-carboxamid
- (±)-1-{4-[(3S)-3-Hydroxypyrrolidin-1-yl]phenyl}-7,8-dimethoxy-N,4-dimethyl-4,5-dihydro-3H-2,3-benzodiazepin-3-carboxamid
- (4S)-1-{4-[(3S)-3-Hydroxypyrrolidin-1-yl]phenyl}-7,8-dimethoxy-N,4-dimethyl-4,5-dihydro-3H-2,3-benzodiazepin-3-carboxamid
- (±)-tert-Butyl-(1-{4-[7,8-dimethoxy-4-methyl-3-(methylcarbamoyl)-4,5-dihydro-3H-2,3-benzodiazepin-1-yl]phenyl}-4-methylpiperidin-4-yl)carbamat
- (±)-1-{4-[(2S,5R)-2,5-Dimethylpiperazin-1-yl]phenyl}-7,8-dimethoxy-N,4-dimethyl-4,5-dihydro-3H-2,3-benzodiazepin-3-carboxamid
- (±)-1-{4-[4-(2,2-Difluorethyl)piperazin-1-yl]phenyl}-7,8-dimethoxy-N,4-dimethyl-4,5-dihydro-3H-2,3-benzodiazepin-3-carboxamid
- (4S)-7,8-Dimethoxy-N,4-dimethyl-1-[4-(3-oxopiperazin-1-yl)phenyl]-4,5-dihydro-3H-2,3-benzodiazepin-3-carboxamid
- (4S)-1-{4-[(2R,6S)-2,6-Dimethylmorpholin-4-yl]phenyl}-7,8-dimethoxy-N,4-dimethyl-4,5-dihydro-3H-2,3-benzodiazepin-3-carboxamid
- (±)-7,8-Dimethoxy-N,4-dimethyl-1-[4-(4-oxopiperidin-1-yl)phenyl]-4,5-dihydro-3H-2,3-benzodiazepin-3-carboxamid
- (±)-7,8-Dimethoxy-N,4-dimethyl-1-{4-[4-(2,2,2-trifluorethyl)piperazin-1-yl]phenyl}-4,5-dihydro-3H-2,3-benzodiazepin-3-carboxamid
- (4S)-1-{4-[(3R,SS)-3,5-Dimethylpiperazin-1-yl]phenyl}-7,8-dimethoxy-N,4-dimethyl-4,5-dihydro-3H-2,3-benzodiazepin-3-carboxamid
- (±)-7,8-Dihydroxy-N,4-dimethyl-1-[4-(4-methylpiperazin-1-yl)phenyl]-4,5-dihydro-3H-2,3-benzodiazepin-3-carboxamid
- (±)-7,8-Diethoxy-N,4-dimethyl-1-[4-(4-methylpiperazin-1-yl)phenyl]-4,5-dihydro-3H-2,3-benzodiazepin-3-carboxamid
- (±)-4-Ethyl-7,8-dimethoxy-N-methyl-1-[4-(4-methylpiperazin-1-yl)phenyl]-4,5-dihydro-3H-2,3-benzodiazepin-3-carboxamid
- (4S)-4-Ethyl-7,8-dimethoxy-N-methyl-1-[4-(4-methylpiperazin-1-yl)phenyl]-4,5-dihydro-3H-2,3-benzodiazepin-3-carboxamid
- (±)-4-Ethyl-7,8-dimethoxy-N-methyl-1-[4-(piperazin-1-yl)phenyl]-4,5-dihydro-3H-2,3-benzodiazepin-3-carboxamid
- (4S)-4-Ethyl-7,8-dimethoxy-N-methyl-1-[4-(piperazin-1-yl)phenyl]-4,5-dihydro-3H-2,3-benzodiazepin-3-carboxamid
- (4R)-4-Ethyl-7,8-dimethoxy-N-methyl-1-[4-(piperazin-1-yl)phenyl]-4,5-dihydro-3H-2,3-benzodiazepin-3-carboxamid
- (±)-4-Ethyl-7,8-dimethoxy-N-methyl-1-{4-[4-(methylsulfonyl)piperazin-1-yl]phenyl}-4,5-dihydro-3H-2,3-benzodiazepin-3-carboxamid
- (4S)-4-Ethyl-7,8-dimethoxy-N-methyl-1-{4-[4-(methylsulfonyl)piperazin-1-yl]phenyl}-4,5-dihydro-3H-2,3-benzodiazepin-3-carboxamid
- (4R)-4-Ethyl-7,8-dimethoxy-N-methyl-1-{4-[4-(methylsulfonyl)piperazin-1-yl]phenyl}-4,5-dihydro-3H-2,3-benzodiazepin-3-carboxamid
- (±)-4-Ethyl-1-[4-(3-fluorazetidin-1-yl)phenyl]-7,8-dimethoxy-N-methyl-4,5-dihydro-3H-2,3-benzodiazepin-3-carboxamid
- (±)-1-[4-(4-Acetylpiperazin-1-yl)phenyl]-4-ethyl-7,8-dimethoxy-N-methyl-4,5-dihydro-3H-2,3-benzodiazepine-3-carboxamid
- (+)-1-[4-(1,1-Dioxidothiomorpholin-4-yl)phenyl]-4-ethyl-7,8-dimethoxy-N-methyl-4,5-dihydro-3H-2,3-benzodiazepin-3-carboxamid
- (±)-4-Ethyl-1-[4-(4-hydroxypiperidin-1-yl)phenyl]-7,8-dimethoxy-N-methyl-4,5-dihydro-3H-2,3-benzodiazepin-3-carboxamid
- (±)-4-Ethyl-7,8-dimethoxy-N-methyl-1-[4-(morpholin-4-yl)phenyl]-4,5-dihydro-3H-2,3-benzodiazepin-3-carboxamid
- (4S)-4-Ethyl-7,8-dimethoxy-N-methyl-1-[4-(morpholin-4-yl)phenyl]-4,5-dihydro-3H-2,3-benzodiazepin-3-carboxamid
- (±)-8-Chlor,-1-[4-(1,1-dioxidothiomorpholin-4-yl)phenyl]-N,4-dimethyl-7-(trifluormethoxy)-4,5-dihydro-3H-2,3-benzodiazepin-3-carboxamid
- (±)-8-Chlor-N,4-dimethyl-1-[4-(piperazin-1-yl)phenyl]-7-(trifluormethoxy)-4,5-dihydro-3H-2,3-benzodiazepin-3-carboxamid
- (±)-8-Chlor-1-[4-(4-hydroxypiperidin-1-yl)phenyl]-N,4-dimethyl-7-(trifluormethoxy)-4,5-dihydro-3H-2,3-benzodiazepin-3-carboxamid
- (±)-8-Chlor-N,4-dimethyl-1-[4-(4-methylpiperazin-1-yl)phenyl]-7-(trifluormethoxy)-4,5-dihydro-3H-2,3-benzodiazepin-3-carboxamid
- (±)-8-Chlor-N,4-dimethyl-1-{4-[4-(methylsulfonyl)piperazin-1-yl]phenyl}-7-(trifluormethoxy)-4,5-dihydro-3H-2,3-benzodiazepin-3-carboxamid
- (±)-8-(1,1-Dioxidothiomorpholin-4-yl)-1-[4-(1,1-dioxidothiomorpholin-4-yl)phenyl]-N,4-dimethyl-7-(trifluormethoxy)-4,5-dihydro-3H-2,3-benzodiazepin-3-carboxamid
- (±)-1-(4-Chlorphenyl)-8-(1,1-dioxidothiomorpholin-4-yl)-N,4-dimethyl-7-(trifluormethoxy)-4,5-dihydro-3H-2,3-benzodiazepin-3-carboxamid
- (±)-1-[4-(4-Acetylpiperazin-1-yl)phenyl]-N,4-dimethyl-7-(trifluormethoxy)-4,5-dihydro-3H-2,3-benzodiazepin-3-carboxamid
- (±)-1-{4-[4-(2-Hydroxyethyl)piperazin-1-yl]phenyl}-N,4-dimethyl-7-(trifluormethoxy)-4,5-dihydro-3H-2,3-benzodiazepin-3-carboxamid
- (±)-8-Methoxy-N,4-dimethyl-1-[4-(4-methylpiperazin-1-yl)phenyl]-7-(trifluormethoxy)-4,5-dihydro-3H-2,3-benzodiazepin-3-carboxamid
- (±)-4-Ethyl-7,8-dimethoxy-N-methyl-1-4-(1,3,5-trimethyl-1H-pyrazol-4-yl)phenyl]-4,5-dihydro-3H-2,3-benzodiazepin-3-carboxamid
- (±)-4-Ethyl-1-(4'-fluorbiphenyl-4-yl)-7,8-dimethoxy-N-methyl-4,5-dihydro-3H-2,3-benzodiazepin-3-carboxamid
- (±)-4-Ethyl-7,8-dimethoxy-N-methyl-1-4-(1-methyl-1H-1,2,3-triazol-4-yl)phenyl]-4,5-dihydro-3H-2,3-benzodiazepin-3-carboxamid
- (±)-1-[4-(3,5-Dimethyl-1,2-oxazol-4-yl)phenyl]-4-ethyl-7,8-dimethoxy-N-methyl-4,5-dihydro-3H-2,3-benzodiazepin-3-carboxamid
- (±)-4-Isopropyl-7,8-dimethoxy-N-methyl-1-[4-(1,3,5-trimethyl-1H-pyrazol-4-yl)phenyl]-4,5-dihydro-3H-2,3-benzodiazepin-3-carboxamid
- (±)-4-Ethyl-7,8-dimethoxy-N-methyl-1-[4-(2-oxopyrrolidin-1-yl)phenyl]-4,5-dihydro-3H-2,3-benzodiazepin-3-carboxamid
- (±)-4-Ethyl-7,8-dimethoxy-N-methyl-1-[4-(3-oxomorpholin-4-yl)phenyl]-4,5-dihydro-3H-2,3-benzodiazepin-3-carboxamid
- (±)-4-Ethyl-7,8-dimethoxy-N-methyl-1-[4-(2-oxopiperidin-1-yl)phenyl]-4,5-dihydro-3H-2,3-benzodiazepin-3-carboxamid
- (±)-4-Ethyl-7,8-dimethoxy-N-methyl-1-[4-(2-oxo-1,3-oxazolidin-3-yl)phenyl]-4,5-dihydro-3H-2,3-benzodiazepin-3-carboxamid
- (4S)-7,8-Dimethoxy-N,4-dimethyl-1-[4-(4-methyl-2-oxo-1,4-diazepan-1-yl)phenyl]-4,5-dihydro-3H-2,3-benzodiazepin-3-carboxamid
- (±)-1-(4-Chlorphenyl)-N,4-dimethyl-7-(2-oxo-1,3-oxazolidin-3-yl)-4,5-dihydro-3H-2,3-benzodiazepin-3-carboxamid
- (4S)-1-(4-Chlorphenyl)-N,4-dimethyl-7-(2-oxo-1,3-oxazolidin-3-yl)-4,5-dihydro-3H-2,3-benzodiazepin-3-carboxamid
- (±)-1-(4-Chlorphenyl)-N,4-dimethyl-7-(2-oxopiperidin-1-yl)-4,5-dihydro-3H-2,3-benzodiazepin-3-carboxamid
- (±)-1-(4-Chlorphenyl)-N,4-dimethyl-7-(3-oxomorpholin-4-yl)-4,5-dihydro-3H-2,3-benzodiazepin-3-carboxamid
- (±)-1-(4-Chlorphenyl)-N,4-dimethyl-7-(morpholin-4-yl)-4,5-dihydro-3H-2,3-benzodiazepin-3-carboxamid
- (±)-1-(4-Chlorphenyl)-N,4-dimethyl-7-(pyrrolidin-1-yl)-4,5-dihydro-3H-2,3-benzodiazepin-3-carboxamid
- (±)-1-(4-Chlorphenyl)-7-(1,1-dioxidothiomorpholin-4-yl)-N,4-dimethyl-4,5-dihydro-3H-2,3-benzodiazepin-3-carboxamid
- (±)-1-(4-Chlorphenyl)-N,4-dimethyl-7-(4-methylpiperazin-1-yl)-4,5-dihydro-3H-2,3-benzodiazepin-3-carboxamid
- (±)-N,4-Dimethyl-7-(4-methylpiperazin-1-yl)-1-[4-(4-methylpiperazin-1-yl)phenyl]-4,5-dihydro-3H-2,3-benzodiazepin-3-carboxamid
- (±)-N,4-Dimethyl-7-(4-methyl-3-oxopiperazin-1-yl)-1-[4-(4-methyl-3-oxopiperazin-1-yl)phenyl]-4,5-dihydro-3H-2,3-benzodiazepin-3-carboxamid
- (±)-1-(4-Chlorphenyl)-7-(4-fluorphenyl)-N,4-dimethyl-4,5-dihydro-3H-2,3-benzodiazepin-3-carboxamid
- (±)-1-(4-Chlorphenyl)-N,4-dimethyl-7-(pyridin-4-yl)-4,5-dihydro-3H-2,3-benzodiazepin-3-carboxamid
- (±)-1-(4-Chlorphenyl)-7-(6-hydroxypyridin-3-yl)-N,4-dimethyl-4,5-dihydro-3H-2,3-benzodiazepin-3-carboxamid
- (±)-1-(4-Chlorphenyl)-7-(3,5-dimethyl-1,2-oxazol-4-yl)-N,4-dimethyl-4,5-dihydro-3H-2,3-benzodiazepin-3-carboxamid
- (±)-1-(4-Chlorphenyl)-N,4-dimethyl-7-(1-methyl-1H-1,2,3-triazol-4-yl)-4,5-dihydro-3H-2,3-benzodiazepin-3-carboxamid
- (4R)-1-[4-(4-Hydroxypiperidin-1-yl)phenyl]-7,8-dimethoxy-N,4-dimethyl-4,5-dihydro-3H-2,3-benzodiazepin-3-carboxamid
- (4R)-7,8-Dimethoxy-N,4-dimethyl-1-[4-(4-methylpiperazin-1-yl)phenyl]-4,5-dihydro-3H-2,3-benzodiazepin-3-carboxamid
- (±)-1-[4-(1,1-Dioxido-1,2-thiazolidin-2-yl)phenyl]-7,8-dimethoxy-N,4-dimethyl-4,5-dihydro-3H-2,3-benzodiazepin-3-carboxamid
- (±)-1-{7,8-Dimethoxy-4-methyl-1-[4-(4-methylpiperazin-1-yl)phenyl]-4,5-dihydro-3H-2,3-benzodiazepin-3-yl}ethanon
- 1-{(4S)-7,8-Dimethoxy-4-methyl-1-[4-(4-methylpiperazin-1-yl)phenyl]-4,5-dihydro-3H-2,3-benzodiazepin-3-yl}ethanon
- (±)-1-{1-[4-(3,5-Dimethyl-1,2-oxazol-4-yl)phenyl]-7,8-dimethoxy-4-methyl-4,5-dihydro-3H-2,3-benzodiazepin-3-yl}ethanon
- (±)-7,8-Dimethoxy-N,4-dimethyl-1-[4-(5-methyl-1,3,4-oxadiazol-2-yl)phenyl]-4,5-dihydro-3H-2,3-benzodiazepin-3-carboxamid
- (±)-7,8-Dimethoxy-N,4-dimethyl-1-[4-(3-methyl-1,2,4-oxadiazol-5-yl)phenyl]-4,5-dihydro-3H-2,3-benzodiazepin-3-carboxamid
- (±)-N,4-Dimethyl-8-(trifluormethoxy)-1-[4-(1,3,5-trimethyl-1H-pyrazol-4-yl)phenyl]-4,5-dihydro-3H-2,3-benzodiazepin-3-carboxamid
- (4S)-N,4-Dimethyl-8-(trifluormethoxy)-1-[4-(1,3,5-trimethyl-1H-pyrazol-4-yl)phenyl]-4,5-dihydro-3H-2,3-benzodiazepin-3-carboxamid
- (4R)-N,4-Dimethyl-8-(trifluormethoxy)-1-[4-(1,3,5-trimethyl-1H-pyrazol-4-yl)phenyl]-4,5-dihydro-3H-2,3-benzodiazepin-3-carboxamid
- (±)-N,4-Dimethyl-1-[4-(4-methyl-1-piperazinyl)phenyl]-8-(trifluormethoxy)-4,5-dihydro-3H-2,3-benzodiazepin-3-carboxamid
- (4R)-N,4-Dimethyl-1-[4-(4-methyl-1-piperazinyl)phenyl]-8-(trifluormethoxy)-4,5-dihydro-3H-2,3-benzodiazepin-3-carboxamid
- (4S)-N,4-Dimethyl-1-[4-(4-methyl-1-piperazinyl)phenyl]-8-(trifluormethoxy)-4,5-dihydro-3H-2,3-benzodiazepin-3-carboxamid
- (±)-1-[4-(4-Hydroxy-1-piperidinyl)phenyl]-N,4-dimethyl-8-(trifluormethoxy)-4,5-dihydro-3H-2,3-benzodiazepin-3-carboxamid
- (±)-1-[2,4-Dibrom-5-(4-methylpiperazin-1-yl)phenyl]-7,8-dimethoxy-N,4-dimethyl-4,5-dihydro-3H-2,3-benzodiazepin-3-carboxamid
- (4S)-1-[3-Brom-4-(4-methylpiperazin-1-yl)phenyl]-7,8-dimethoxy-N,4-dimethyl-4,5-dihydro-3H-2,3-benzodiazepin-3-carboxamid
- (4S)-1-[3-Cyan-4-(4-methylpiperazin-1-yl)phenyl]-7,8-dimethoxy-N,4-dimethyl-4,5-dihydro-3H-2,3-benzodiazepin-3-carboxamid
- (±)-7,8-Dimethoxy-4-methyl-1-[4-(4-methylpiperazin-1-yl)phenyl]-3-(1-oxopropyl)-4,5-dihydro-3H-2,3-benzodiazepin
- (±)-3-(Cyclopropylcarbonyl)-7,8-dimethoxy-4-methyl-1-[4-(4-methylpiperazin-1-yl)phenyl]-4,5-dihydro-3H-2,3-benzodiazepin
- (±)-N-Cyclopropyl-7,8-dimethoxy-4-methyl-1-[4-(4-methylpiperazin-1-yl)phenyl]-4,5-dihydro-3H-2,3-benzodiazepin
- (±)-7,8-Dimethoxy-N,4-dimethyl-1-[4-(4-methylpiperazin-1-yl)phenyl]-4,5-dihydro-3H-2,3-benzodiazepin-3-carbothioamid
- Methyl-(±)-7,8-dimethoxy-4-methyl-1-[4-(4-methylpiperazin-1-yl)phenyl]-4,5-dihydro-3H-2,3-benzodiazepin-3-carboxylat
- Ethyl-(±)-7,8-dimethoxy-4-methyl-1-[4-(4-methylpiperazin-1-yl)phenyl]-4,5-dihydro-3H-2,3-benzodiazepin-3-carboxylat
- (±)-N-Ethyl-7,8-dimethoxy-4-methyl-1-[4-(4-methylpiperazin-1-yl)phenyl]-4,5-dihydro-3H-2,3-benzodiazepin-3-carboxamid
- (4S)-N-Ethyl-7,8-dimethoxy-4-methyl-1-[4-(4-methylpiperazin-1-yl)phenyl]-4,5-dihydro-3H-2,3-benzodiazepin-3-carboxamid

In der allgemeinen Formel (I-A) kann X für ein Sauerstoff- oder Schwefelatom stehen.

In der allgemeinen Formel (I-A) steht X bevorzugt für ein Sauerstoffatom.

In der allgemeinen Formel (I-A) kann A für einen monozyklischen Heteroarylring mit 5 oder 6 Ringatomen oder für einen Phenylring stehen.

In der allgemeinen Formel (I-A) steht A bevorzugt für einen monozyklischen Heteroarylring mit 6 Ringatomen oder für einen Phenylring.

In der allgemeinen Formel (I-A) steht A mehr bevorzugt für einen Pyridyl- oder für einen Phenylring.

In der allgemeinen Formel (I-A) steht A besonders bevorzugt für einen Phenylring.

In der allgemeinen Formel (I-A) kann R^{1a} auch stehen
a) für Wasserstoff, Halogen, Cyano, Carboxy, Amino oder Aminosulfonyl, oder
b) für einen C₁-C₆-Alkoxy-,C₁-C₃-Alkoxy-C₁-C₃-Alkyl-,C₁-C₃-Alkoxy-C₁-C₃-Alkoxy-, C₁-C₆-Alkylamino-,C₁-C₆-Alkylcarbonylamino,C₁-C₆-Alkylamino-C₁-C₆-Alkyl-, *N*-Heterocyclyl-C₁-C₆-Alkyl-,*N*-Heterocyclyl-C₁-C₆-Alkoxy-,Hydroxy-C₁-C₆-Alkyl-, Hydroxy-C₁-C₆-Alkoxy-,Halogen-C₁-C₆-Alkyl-,Halogen-C₁-C₆-Alkoxy-, C₁-C₆-Alkylcarbonyl- oder C₁-C₆-Alkoxycarbonylrest, oder
c) für einen monozyklischen Heterocyclylrest mit 3 bis 8 Ringatomen, der ein- oder mehrfach, gleich oder verschieden substituiert sein kann mit Hydroxy, Halogen, Amino, Oxo, Carboxy und/oder einem C₁-C₆-Alkyl-,C₁-C₆-Alkoxy-,C₁-C₆-Alkoxy-C₁-C₆-Alkyl-, Hydroxy-C₁-C₆-Alkyl-,C₁-C₆-Alkylamino-,Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen, oder
d) für einen monzyklischen Heteroarylrest mit 5 oder 6 Ringatomen, der ein- oder mehrfach, gleich oder verschieden substituiert sein kann mit Halogen, Hydroxy, Amino, Cyano, Nitro, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen und/oder einem monocyclischen Heteroarylrest mit 5 oder 6 Ringatomen, oder
e) für einen Phenylrest, der ein- oder mehrfach, gleich oder verschieden substituiert sein kann mit Halogen, Amino, Hydroxy, Cyano, Nitro, Carboxy und/oder einem C₁-C₆-Alkyl, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-,Halogen-C₁-C₆-Alkyl-, Halogen-C₁-C₆-Alkoxy-, Hydroxy-C₁-C₆-Alkyl-, C₃-C₁₀-Cycloalkyl- und/oder einem monocyclischen Heterocyclylrest mit 3 bis 8 Ringatomen.

In der allgemeinen Formel (I-A) steht R^{1a} bevorzugt
a) für einen monozyklischen Heterocyclylrest mit 3 bis 8 Ringatomen, der ein- oder mehrfach, gleich oder verschieden substituiert sein kann mit Hydroxy, Halogen, Amino, Oxo, Carboxy und/oder einem C₁-C₆-Alkyl-, C₁-C₆-Alkoxy-, C₁-C₆-Alkoxy-C₁-C₆-Alkyl-, Hydroxy-C₁-C₆-Alkyl-,C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen, oder
b) für einen monzyklischen Heteroarylrest mit 5 oder 6 Ringatomen, der ein- oder mehrfach, gleich oder verschieden substituiert sein kann mit Halogen, Hydroxy, Amino, Cyano, Nitro, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen und/oder einem monocyclischen Heteroarylrest mit 5 oder 6 Ringatomen, oder
c) für einen Phenylrest, der ein- oder mehrfach, gleich oder verschieden substituiert sein kann mit Halogen, Amino, Hydroxy, Cyano, Nitro, Carboxy und/oder einem C₁-C₆-Alkyl, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, Hydroxy-C₁-C₆-Alkyl-, C₃-C₁₀-Cycloalkyl- und/oder einem monocyclischen Heterocyclylrest mit 3 bis 8 Ringatomen.

In der allgemeinen Formel (I-A) steht R^{1a} auch bevorzugt
a) für Wasserstoff oder Halogen, oder
b) für einen monozyklischen Heterocyclylrest mit 3 bis 8 Ringatomen, der ein- oder mehrfach, gleich oder verschieden substituiert sein kann mit Hydroxy, Fluor, Oxo, Carboxy und/oder einem C₁-C₆-Alkyl-, Hydroxy-C₁-C₆-Alkyl-, Halogen-C₁-C₆-Alkyl- oder Halogen-C₁-C₆-Alkoxyrest, oder
c) für einen monzyklischen Heteroarylrest mit 5 oder 6 Ringatomen, der ein- oder mehrfach, gleich oder verschieden substituiert sein kann mit Halogen, Hydroxy, Carboxy und/oder einem C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heteroarylrest mit 5 oder 6 Ringatomen, oder
d) für einen Phenylrest, der ein- oder mehrfach, gleich oder verschieden substituiert sein kann mit Halogen, Hydroxy, Carboxy und/oder einem C₁-C₆-Alkyl,C₁-C₆-Alkoxy-, Halogen-C₁-C₆-Alkyl- oder Halogen-C₁-C₆-Alkoxyrest.

In der allgemeinen Formel (I-A) steht R^{1a} mehr bevorzugt
a) für Wasserstoff oder Halogen, oder
b) für einen monozyklischen Heterocyclylrest mit 4 bis 6 Ringatomen, oder
c) für einen monzyklischen Heteroarylrest mit 5 oder 6 Ringatomen, der ein- oder mehrfach, gleich oder verschieden substituiert sein kann mit Halogen, Carboxy und/oder einem C₁-C₆-Alkyl-, C₁-C₆-Alkoxy-C₁-C₆-Alkyl-, C₃-C₁₀-Cycloalkyl- und/oder einem monocyclischen Heteroarylrest mit 6 Ringatomen, oder
d) für einen Phenylrest, der ein- oder mehrfach substituiert sein kann mit Carboxy.

In der allgemeinen Formel (I-A) steht R^{1a} sehr bevorzugt
a) für Wasserstoff oder Chlor, oder
b) für einen Morpholinylrest, oder
c) für einen Isooxazolyl-, Pyrazolyl-, Tetrazolyl-, Thienyl- oder Pyridinylrest, der ein- oder mehrfach, gleich oder verschieden substituiert sein kann mit Chlor, Carboxy und/oder einem C₁-C₃-Alkyl-, C₁-C₃-Alkoxy-C₁-C₃-Alkyl-, C₃-C₆-Cycloalkyl- und/oder einem Pyridinylrest, oder
d) für einen Phenylrest, der substituiert sein kann mit Carboxy.

In der allgemeinen Formel (I-A) steht R^{1a} besonders bevorzugt
a) für Chlor, oder
b) für einen Morpholinylrest, oder
c) für einen Isooxazolyl-, Pyrazolyl-, Thienyl- oder Pyridinylrest, der ein- oder mehrfach, gleich oder verschieden substituiert sein kann mit Chlor, Carboxy und/oder einem C₁-C₃-Alkyl-, C₁-C₃-Alkoxy-C₁-C₃-Alkyl-, C₃-C₆-Cycloalkyl- und/oder einem Pyridinylrest.

In der allgemeinen Formel (I-A) kann R^{1b} stehen für Halogen, Hydroxy, Cyano, Nitro und/oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen, und n in der allgemeinen Formel (I-A) kann für 0 - 2 stehen.

In der allgemeinen Formel (I-A) steht R^{1b} bevorzugt für Halogen, Cyano und/oder für einen C₁-C₆-Alkyl-, C₁-C₆-Alkoxy-, Halogen-C₁-C₆-Alkyl- oder Halogen-C₁-C₆-Alkoxyrest, und n in der allgemeinen Formel (I-A) kann für 0 - 2 stehen

In der allgemeinen Formel (I-A) steht n bevorzugt für 0.

In der allgemeinen Formel (I-A) kann R² stehen für einen C₁-C₃-Alkyl- oder Trifluormethyl- oder einen C₃- oder C₄-Cycloalkylrest.

In der allgemeinen Formel (I-A) steht R² bevorzugt für einen C₁-C₃-Alkyl- oder Trifluormethylrest.

In der allgemeinen Formel (I-A) steht R² mehr bevorzugt für einen C₁-C₃-Alkylrest.

In der allgemeinen Formel (I-A) steht R² besonders bevorzugt für einen Methylrest.

In der allgemeinen Formel (I-A) kann R³ stehen für einen Cyclopropyl-, C₁-C₃-Alkyl-, C₁-C₃-Alkoxy-, Amino-, Cyclopropylamino- oder einen C₁-C₃-Alkylaminorest steht.

In der allgemeinen Formel (I-A) steht R³ bevorzugt für einen C₁-C₃-Alkyl- oder einen C₁-C₃-Alkylaminorest.

In der allgemeinen Formel (I-A) steht R³ bevorzugt für einen Methyl- oder einen C₁-C₃-Alkylaminorest.

In der allgemeinen Formel (I-A) steht R³ bevorzugt für einen Methylrest.

In der allgemeinen Formel (I-A) steht R³ mehr bevorzugt für einen C₁-C₃-Alkylaminorest.

In der allgemeinen Formel (I-A) steht R³ besonders bevorzugt für einen Methylaminorest.

In der allgemeinen Formel (I-A) können R⁴ und R⁵ unabhängig voneinander stehen
i) für Wasserstoff, Hydroxy, Amino, Aminocarbonyl, Fluor, Chlor, Brom oder einen Trifluormethyl-, C₁-C₆-Alkyl-, C₁-C₆-Alkoxy-, C₁-C₆-Alkylamino-, C₁-C₆-Alkylcarbonylamino- oder C₁-C₆-Alkylaminocarbonylrest, wobei der Alkylteil eines Restes unter i) ein- oder mehrfach, gleich oder verschieden substituiert sein kann mit Amino, Fluor, Hydroxy, Carboxy oder einem Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, oder einem monocyclischen Heterocyclylrest mit 3 bis 6 Ringatomen, oder
ii) für Cyano oder Nitro oder
iii) für einen C₁-C₆-Alkylaminosulfonyl-, Halogen-C₁-C₆-Alkyl- oder Halogen-C₁-C₆-Alkoxyrest, wobei der Alkylteil eines Restes unter iii) ein- oder mehrfach, gleich oder verschieden substituiert sein kann mit Amino,Fluor,Hydroxy,Carboxy oder einem Hydroxy-C₁-C₆-Alkyl-, C₁-C₆-Alkoxy-, C₁-C₆-Alkylamino, Amino-C₁-C₆-Alkyl-, C₁-C₆-Alkoxy-C₁-C₆-Alkyl- oder einem monocyclischen Heterocyclylrest mit 3 bis 6 Ringatomen, oder
iv) für einen C₃-C₁₀-Cycloalkylrest, der ein- oder mehrfach, gleich oder verschieden substituiert sein kann mit Halogen, Amino, Hydroxy, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl- oder Halogen-C₁-C₆-Alkoxyrest, oder
v) für einen monzyklischen Heteroarylrest mit 5 oder 6 Ringatomen, der ein- oder mehrfach, gleich oder verschieden substituiert sein kann mit Halogen, Amino, Hydroxy, Cyano, Nitro, Carboxy und/oder einem C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen, oder
vi) für einen monozyklischen Heterocyclylrest mit 3 bis 8 Ringatomen, der ein- oder mehrfach, gleich oder verschieden substituiert sein kann mit Halogen, Amino, Hydroxy, Cyano, Oxo, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Hydroxy-C₁-C₆-Alkyl-, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen, oder
vii) für einen Phenylrest, der ein- oder mehrfach, gleich oder verschieden substituiert sein kann mit Halogen, Amino, Hydroxy, Cyano, Nitro, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl, C₁-C₆-Alkylaminosulfonyl-, C₁-C₆-Alkylamino-C₁-C₆-Alkyl, Hydroxy-C₁-C₆-Alkyl-, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen.

In der allgemeinen Formel (I-A) stehen R⁴ und R⁵ bevorzugt unabhängig voneinander
i) für Cyano oder Nitro oder
ii) für einen C₁-C₆-Alkylaminosulfonyl-, Halogen-C₁-C₆-Alkyl- oder Halogen-C₁-C₆-Alkoxyrest, wobei der Alkylteil eines Restes unter iii) ein- oder mehrfach, gleich oder verschieden substituiert sein kann mit Amino, Fluor, Hydroxy, Carboxy oder einem Hydroxy-C₁-C₆-Alkyl-, C₁-C₆-Alkoxy-, C₁-C₆-Alkylamino, Amino-C₁-C₆-Alkyl-, C₁-C₆-Alkoxy-C₁-C₆-Alkyl- oder einem monocyclischen Heterocyclylrest mit 3 bis 6 Ringatomen, oder
iii) für einen C₃-C₁₀-Cycloalkylrest, der ein- oder mehrfach, gleich oder verschieden substituiert sein kann mit Halogen, Amino, Hydroxy, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl- oder Halogen-C₁-C₆-Alkoxyrest, oder
iv) für einen monzyklischen Heteroarylrest mit 5 oder 6 Ringatomen, der ein- oder mehrfach, gleich oder verschieden substituiert sein kann mit Halogen, Amino, Hydroxy, Cyano, Nitro, Carboxy und/oder einem C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen, oder
v) für einen monozyklischen Heterocyclylrest mit 3 bis 8 Ringatomen, der ein- oder mehrfach, gleich oder verschieden substituiert sein kann mit Halogen, Amino, Hydroxy, Cyano, Oxo, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Hydroxy-C₁-C₆-Alkyl-, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen, oder
vi) für einen Phenylrest, der ein- oder mehrfach, gleich oder verschieden substituiert sein kann mit Halogen, Amino, Hydroxy, Cyano, Nitro, Carboxy und/oder einem C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl, C₁-C₆-Alkylaminosulfonyl-, C₁-C₆-Alkylamino-C₁-C₆-Alkyl, Hydroxy-C₁-C₆-Alkyl-, Halogen-C₁-C₆-Alkyl- oder Halogen-C₁-C₆-Alkoxy, C₃-C₁₀-Cycloalkyl- und/oder einem monocyclischen Heterocyclylrest mit 3 bis 8 Ringatomen.

In der allgemeinen Formel (I-A) stehen R⁴ und R⁵ mehr bevorzugt unabhängig voneinander
i) für Wasserstoff oder Fluor, Chlor, Brom oder für einen C₁-C₆-Alkoxyrest, wobei der Alkylteil des C₁-C₆-Alkoxyrestes ein- oder mehrfach, gleich oder verschieden substituiert sein kann mit einem monocyclischen Heterocyclylrest mit 3 bis 6 Ringatomen, oder
ii) für einen Halogen-C₁-C₆-Alkyl- oder Halogen-C₁-C₆-Alkoxyrest, oder
iii) für einen C₃-C₁₀-Cycloalkylrest, der ein- oder mehrfach, gleich oder verschieden substituiert sein kann mit Halogen, Hydroxy, Carboxy und/oder einem C₁-C₆-Alkylrest-, oder
iv) für einen monzyklischen Heteroarylrest mit 5 oder 6 Ringatomen, der ein- oder mehrfach, gleich oder verschieden substituiert sein kann mit Halogen, Hydroxy, oder einem C₁-C₆-Alkyl-, C₁-C₆-Alkoxy- oder Hydroxy-C₁-C₆-Alkylrest, oder
v) für einen monozyklischen Heterocyclylrest mit 3 bis 8 Ringatomen, der ein- oder mehrfach, gleich oder verschieden substituiert sein kann mit Halogen, Hydroxy, Carboxy und/oder einem C₁-C₆-Alkylrest-, oder
vi) für einen Phenylrest, der ein- oder mehrfach, gleich oder verschieden substituiert sein kann mit Halogen, Cyano, Carboxy und/oder einem C₁-C₆-Alkyl-, C₁-C₆-Alkoxy, Hydroxy-C₁-C₆-Alkyl-, oder C₁-C₆-Alkylaminosulfonylrest.

In der allgemeinen Formel (I-A) stehen R⁴ und R⁵ mehr bevorzugt unabhängig voneinander
i) für Wasserstoff, Chlor oder einen C₁-C₆-Alkoxyrest, wobei der Alkylteil des C₁-C₆-Alkoxyrestes ein- oder mehrfach, gleich oder verschieden substituiert sein kann mit einem monocyclischen Heterocyclylrest mit 3 bis 6 Ringatomen, oder
ii) für einen Halogen-C₁-C₆-Alkoxyrest, oder
iii) für einen C₃-C₁₀-Cycloalkylrest, oder
iv) für einen monzyklischen Heteroarylrest mit 6 Ringatomen, oder
v) für einen monozyklischen Heterocyclylrest mit 3 bis 6 Ringatomen, der ein- oder mehrfach, gleich oder verschieden substituiert sein kann mit einem C₁-C₆-Alkylrest, oder
vi) für einen Phenylrest, der ein- oder mehrfach, gleich oder verschieden substituiert sein kann mit einem C₁-C₆-Alkylaminosulfonylrest.
In der allgemeinen Formel (I-A) steht R⁴ besonders bevorzugt für Wasserstoff, Chlor oder einen C₁-C₃-Alkyoxyrest.

In der allgemeinen Formel (I-A) steht R⁴ außerordentlich bevorzugt für Wasserstoff oder einen Methoxyrest.

In der allgemeinen Formel (I-A) steht R⁵ besonders bevorzugt
i) für Wasserstoff, Chlor oder einen C₁-C₃-Alkoxyrest, wobei der Alkylteil des C₁-C₃-Alkoxyrestes ein- oder mehrfach, gleich oder verschieden substituiert sein kann mit einem Morpholinyl- oder Pyrrolidinylrest, oder
ii) für einen Trifluormethoxyrest, oder
iii) für einen Cyclopropylrest, oder
iv) für einen Pyridinylrest, oder
v) für einen Morpholinyl-, Piperazinyl- oder Piperidinylrest, der ein- oder mehrfach, gleich oder verschieden substituiert sein kann mit einemC₁-C₃-Alkylrest.

In der allgemeinen Formel (I-A) steht R⁵ außerordentlich bevorzugt
i) für einen Methoxyrest, oder
ii) für einen Trifluormethoxyrest, oder
iii) für einen Cyclopropylrest, oder
iv) für einen Pyridinylrest, oder
v) für einen Morpholinylrest.

R⁴ und R⁵ können unabhängig voneinander für einen monozyklischen Heterocyclylrest stehen. Bevorzugt sind hierbei Heterocyclylreste mit mindestens zwei Heteroatomen.

In der allgemeinen Formel (I-A) stehen R⁶ und R⁷ ganz besonders bevorzugt für Wasserstoff, C₁-C₃-Alkyl-, Cyclopropyl-, oder Di-C₁-C₃-Alkyl-Amino-C₁-C₃-Alkyl-.

In der allgemeinen Formel (I-A) steht R⁸ ganz besonders bevorzugt für Hydroxy, C₁-C₃-Alkyl-, Hydroxy-C₁-C₃-Alkyl-, Trifluormethyl-, Pyrrolidinyl-, Morpholinyloder Piperidinyl-.

In der allgemeinen Formel (I-A) steht R⁹ ganz besonders bevorzugt für C₁-C₃-Alkyl-.

In der allgemeinen Formel (I) kann X für ein Sauerstoff- oder Schwefelatom stehen.

In der allgemeinen Formel (I) steht X bevorzugt für ein Sauerstoffatom.

In der allgemeinen Formel (I) kann A für einen monozyklischen Heteroarylring mit 5 oder 6 Ringatomen oder für einen Phenylring stehen.

In der allgemeinen Formel (I) steht A bevorzugt für einen monozyklischen Heteroarylring mit 6 Ringatomen oder für einen Phenylring.

In der allgemeinen Formel (I) steht A mehr bevorzugt für einen Pyridyl- oder für einen Phenylring.

In der allgemeinen Formel (I) steht A besonders bevorzugt für Pyrid-3-yl-.

In der allgemeinen Formel (I) steht A besonders bevorzugt für einen Phenylring.

In der allgemeinen Formel (I) kann R^{1a} stehen für Wasserstoff, Halogen, Cyano, Carboxy, Amino oder Aminosulfonyl,
oder
für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino-, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest,
oder
für einen monozyklischen Heterocyclylrest mit 3 bis 8 Ringatomen, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, Phenyl-, Halophenyl-, Phenyl-C₁-C₆-Alkyl-, Pyridinyl-, -NR⁶C(=O)-R⁹,-C(=O)-NR⁶R7, -C(=O)-R⁸, -S(=O)₂-NR⁶R⁷, -S(=O)-R⁹, -S(=O)₂-R⁹, -NH-S(=O)₂-R⁹, und/oder einem monocyclischen Heterocyclylrest mit 3 bis 8 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 3 bis 8 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden, mit Halogen, C₁-C₃-Alkyl und/oder C₁-C₃-Alkoxy- substituiert sein kann,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, -C(=O)NR⁶R⁷, -C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl- und/oder einem monocyclischen Heterocyclylrest mit 3 bis 8 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl und/oder C₁-C₃-Alkoxy-.

In der allgemeinen Formel (I) steht R^{1a} bevorzugt für Wasserstoff, Halogen, Cyano, Carboxy, Amino oder Aminosulfonyl,
oder
für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest,
oder
für einen monozyklischen Heterocyclylrest mit 4 bis 7 Ringatomen, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, Phenyl-, Halophenyl-, Phenyl-C₁-C₆-Alkyl-, Pyridinyl-, -NR⁶C(=O)R⁹, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, oder
für einen monozyklischen Heteroarylrest mit 5 oder 6 Ringatomen, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-,-C(=O)-NR⁶R⁷, -C(=O)-R⁸, -S(=O)₂-NR⁶R⁷, -S(=O)-R⁹, -S(=O)₂-R⁹, - NH-S(=O)₂-R⁹, und/oder mit einem monocyclischen Heterocyclylrest mit 4 bis 7 Ringatomen und/oder mit einem monocyclischen Heteroarylrest mit 5 oder 6 Ringatomen und/oder mit einem Phenylrest, der seinerseits gegebenenfalls ein- oder mehrfach, gleich oder verschieden mit Halogen, C₁-C₃-Alkyl- und/oder C₁-C₃-Alkoxy- substituiert sein kann,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl- und/oder einem monocyclischen Heterocyclylrest mit 4 bis 7 Ringatomen und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl- und/oder C₁-C₃-Alkoxy-.

In der allgemeinen Formel (I) steht R^{1a} besonders bevorzugt für Wasserstoff, Halogen, Cyano, Carboxy, Amino oder Aminosulfonyl,
oder
für einen C₁-C₆-Alkoxy-, C₁-C₃-Alkoxy-C₁-C₃-Alkyl-, C₁-C₃-Alkoxy-C₂-C₃-Alkoxy-, C₁-C₃-Alkylamino-, C₁-C₃-Alkylcarbonylamino, C₁-C₃-Alkylamino-C₁-C₃-Alkyl-, Hydroxy-C₁-C₃-Alkyl-, Fluor-C₁-C₃-Alkyl-, Fluor-C₁-C₃-Alkoxy-, C₁-C₃-Alkylcarbonyl- oder C₁-C₄-Alkoxycarbonyl-Rest, oder
für einen monozyklischen Heterocyclylrest mit 4 bis 7 Ringatomen, der gegebenenfalls ein- oder mehrfach, gleich oder verschieden substituiert sein kann mit Halogen, Cyano, Nitro, Hydroxy, Amino, Oxo, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, Phenyl-, Halophenyl-, Phenyl-C₁-C₃-Alkyl-, Pyridinyl-, -NR⁶C(=O)-R⁹,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Hydroxy, Amino, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Fluor, Chlor, Brom, Methyl- oder Methoxy- substituiert sein kann,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₃-Alkyl, C₁-C₃-Alkoxy-, C₁-C₂-Alkoxy-C₁-C₂-Alkyl-, C₁-C₃-Alkylamino-, Amino-C₁-C₃-Alkyl-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Fluor-C₁-C₃-Alkyl-, Fluor-C₁-C₃-Alkoxy-, Hydroxy-C₁-C₃-Alkyl-, C₃-C₆-Cycloalkyl- und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Fluor, Chlor, Brom, Methyl- oder Methoxy-.

In der allgemeinen Formel (I) steht R^{1a} ferner besonders bevorzugt für einen monozyklischen Heterocyclylrest mit 4 bis 7 Ringatomen, der gegebenenfalls ein- oder mehrfach, gleich oder verschieden substituiert sein kann mit Halogen, Cyano, Nitro, Hydroxy, Amino, Oxo, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, Phenyl-, Halophenyl-, Phenyl-C₁-C₃-Alkyl-, Pyridinyl-, -NR⁶C(=O)-R⁹,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Hydroxy, Amino, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Fluor, Chlor, Brom, Methyl- oder Methoxy- substituiert sein kann,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₃-Alkyl, C₁-C₃-Alkoxy-, C₁-C₂-Alkoxy-C₁-C₂-Alkyl-, C₁-C₃-Alkylamino-, Amino-C₁-C₃-Alkyl-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Fluor-C₁-C₃-Alkyl-, Fluor-C₁-C₃-Alkoxy-, Hydroxy-C₁-C₃-Alkyl-, C₃-C₆-Cycloalkyl- und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Fluor, Chlor, Brom, Methyl- oder Methoxy-.

In der allgemeinen Formel (I) steht R^{1a} ferner besonders bevorzugt für Wasserstoff, Halogen, Cyano, Carboxy, Amino oder Aminosulfonyl,
oder
für einen C₁-C₆-Alkoxy-, C₁-C₃-Alkoxy-C₁-C₃-Alkyl-, C₁-C₃-Alkoxy-C₂-C₃-Alkoxy-, C₁-C₃-Alkylamino-, C₁-C₃-Alkylcarbonylamino, C₁-C₃-Alkylamino-C₁-C₃-Alkyl-, Hydroxy-C₁-C₃-Alkyl-, Fluor-C₁-C₃-Alkyl-, Fluor-C₁-C₃-Alkoxy-, C₁-C₃-Alkylcarbonyl- oder C₁-C₄-Alkoxycarbonyl-Rest.

In der allgemeinen Formel (I) steht R^{1a} ferner besonders bevorzugt für einen monozyklischen Heterocyclylrest mit 4 bis 7 Ringatomen, der gegebenenfalls ein- oder mehrfach, gleich oder verschieden substituiert sein kann mit Halogen, Cyano, Nitro, Hydroxy, Amino, Oxo, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, Phenyl-, Halophenyl-, Phenyl-C₁-C₃-Alkyl-, Pyridinyl-, -NR⁶C(=O)-R⁹,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen.

In der allgemeinen Formel (I) steht R^{1a} ferner besonders bevorzugt für einen monozyklischen Heteroarylrest mit 5 oder 6 Ringatomen, der gegebenenfalls ein- oder mehrfach, gleich oder verschieden substituiert sein kann mit Halogen, Hydroxy, Amino, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Fluor, Chlor, Brom, Methyl- oder Methoxy- substituiert sein kann.

In der allgemeinen Formel (I) steht R^{1a} ferner besonders bevorzugt für einen Phenylrest, der gegebenenfalls ein- oder mehrfach, gleich oder verschieden substituiert sein kann mit Halogen, Amino, Hydroxy, Cyano, Nitro, Carboxy, C₁-C₃-Alkyl, C₁-C₃-Alkoxy-, C₁-C₂-Alkoxy-C₁-C₂-Alkyl-, C₁-C₃-Alkylamino-, Amino-C₁-C₃-Alkyl-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Fluor-C₁-C₃-Alkyl-, Fluor-C₁-C₃-Alkoxy-, Hydroxy-C₁-C₃-Alkyl-, C₃-C₆-Cycloalkyl- und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Fluor, Chlor, Brom, Methyl- oder Methoxy-.

In der allgemeinen Formel (I) steht R^{1a} ganz besonders bevorzugt für Wasserstoff oder Chlor, oder
für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl-, die gegebenenfalls ein- oder mehrfach, gleich oder verschieden substituiert sein können mit Fluor, Chlor, Cyano, Nitro, Hydroxy, Oxo, C₁-C₃-Alkyl-, C₁-C₃-Alkoxy-, C₁-C₂-Alkoxy-C₁-C₂-Alkyl-, Hydroxy-C₁-C₃-Alkyl-, Dimethylamino-, Trifluormethyl-, Difluorethyl-, Trifluorethyl-, Trifluormethoxy-, Cyclopropyl-, Phenyl-, Fluorphenyl-, Phenyl-C₁-C₃-Alkyl-, Pyridinyl-, -NR⁶C(=O)-R⁹, -C(=O)-NR⁶R⁷, -C(=O)-R⁸, -S(=O)₂-NR⁶R⁷, -S(=O)-R⁹, -S(=O)₂-R⁹ und/oder -NH-S(=O)₂-R⁹,
oder
für Tetrazolyl-,
oder
für Isoxazolyl-, Pyrazolyl-, Thienyl-, Thiazolyl-, Imidazolyl-, Triazolyl-, Pyrrolyl-, Oxadiazolyl-, Pyridinyl- oder Pyrimidinyl-, die gegebenenfalls ein- oder mehrfach, gleich oder verschieden substituiert sein können mit Fluor, Chlor, Hydroxy, Amino, Cyano, Nitro, C₁-C₃-Alkyl-, C₁-C₃-Alkoxy-, C₁-C₂-Alkoxy-C₁-C₂-Alkyl-, Dimethylamino-, Trifluormethyl-, Difluorethyl-, Trifluorethyl-, Trifluormethoxy-, Cyclopropyl, -C(=O)-NR⁶R⁷, -C(=O)-R⁸, -S(=O)₂-NR⁶R⁷, - S(=O)-R⁹, -S(=O)₂-R⁹, -NH-S(=O)₂-R⁹, Pyridinyl-, Phenyl-, und/oder Fluorphenyl-,
oder
für Phenyl-, das gegebenenfalls ein- oder mehrfach, gleich oder verschieden substituiert sein kann mit Fluor, Chlor, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Dimethylamino-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Trifluormethyl-, Difluorethyl-, Trifluorethyl-, Trifluormethoxy-, Hydroxy-C₁-C₃-Alkyl-, Cyclopropyl, Morpholino- und/oder Pyridinyl-.

In der allgemeinen Formel (I) steht R^{1a} ferner ganz besonders bevorzugt für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl-, die gegebenenfalls ein- oder mehrfach, gleich oder verschieden substituiert sein können mit Fluor, Chlor, Cyano, Nitro, Hydroxy, Oxo, C₁-C₃-Alkyl-, C₁-C₃-Alkoxy-, C₁-C₂-Alkoxy-C₁-C₂-Alkyl-, Hydroxy-C₁-C₃-Alkyl-, Dimethylamino-, Trifluormethyl-, Difluorethyl-, Trifluorethyl-, Trifluormethoxy-, Cyclopropyl-, Phenyl-, Fluorphenyl-, Phenyl-C₁-C₃-Alkyl-, Pyridinyl-, -NR⁶C(=O)-R⁹, -C(=O)-NR⁶R⁷, -C(=O)-R⁸, - S(=O)₂-NR⁶R⁷, -S(=O)-R⁹, -S(=O)₂-R⁹ und/oder -NH-S(=O)₂-R⁹,
oder
für Tetrazolyl-,
oder
für Isoxazolyl-, Pyrazolyl-, Thienyl-, Thiazolyl-, Imidazolyl-, Triazolyl-, Pyrrolyl-, Oxadiazolyl-, Pyridinyl- oder Pyrimidinyl-, die gegebenenfalls ein- oder mehrfach, gleich oder verschieden substituiert sein können mit Fluor, Chlor, Hydroxy, Amino, Cyano, Nitro, C₁-C₃-Alkyl-, C₁-C₃-Alkoxy-, C₁-C₂-Alkoxy-C₁-C₂-Alkyl-, Dimethylamino-, Trifluormethyl-, Difluorethyl-, Trifluorethyl-, Trifluormethoxy-, Cyclopropyl, -C(=O)-NR⁶R⁷, -C(=O)-R⁸, -S(=O)₂-NR⁶R⁷, - S(=O)-R⁹, -S(=O)₂-R⁹, -NH-S(=O)₂-R⁹, Pyridinyl-, Phenyl-, und/oder Fluorphenyl-,
oder
für Phenyl-, das gegebenenfalls ein- oder mehrfach, gleich oder verschieden substituiert sein kann mit Fluor, Chlor, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Dimethylamino-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Trifluormethyl-, Difluorethyl-, Trifluorethyl-, Trifluormethoxy-, Hydroxy-C₁-C₃-Alkyl-, Cyclopropyl, Morpholino- und/oder Pyridinyl-.

In der allgemeinen Formel (I) steht R^{1a} ferner ganz besonders bevorzugt für Wasserstoff oder Chlor.

In der allgemeinen Formel (I) steht R^{1a} ferner ganz besonders bevorzugt Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl-, die gegebenenfalls ein- oder mehrfach, gleich oder verschieden substituiert sein können mit Fluor, Chlor, Cyano, Nitro, Hydroxy, Oxo, C₁-C₃-Alkyl-, C₁-C₃-Alkoxy-, C₁-C₂-Alkoxy-C₁-C₂-Alkyl-, Hydroxy-C₁-C₃-Alkyl-, Dimethylamino-, Trifluormethyl-, Difluorethyl-, Trifluorethyl-, Trifluormethoxy-, Cyclopropyl-, Phenyl-, Fluorphenyl-, Phenyl-C₁-C₃-Alkyl-, Pyridinyl-, -NR⁶C(=O)-R⁹, -C(=O)-NR⁶R⁷, -C(=O)-R⁸, -S(=O)₂-NR⁶R⁷, -S(=O)-R⁹, - S(=O)₂-R⁹ und/oder -NH-S(=O)₂-R⁹.

In der allgemeinen Formel (I) steht R^{1a} ferner ganz besonders bevorzugt für Isoxazolyl-, Pyrazolyl-, Thienyl-, Thiazolyl-, Imidazolyl-, Triazolyl-, Pyrrolyl-, Oxadiazolyl-, Pyridinyl- oder Pyrimidinyl-, die gegebenenfalls ein- oder mehrfach, gleich oder verschieden substituiert sein können mit Fluor, Chlor, Hydroxy, Amino, Cyano, Nitro, C₁-C₃-Alkyl-, C₁-C₃-Alkoxy-, C₁-C₂-Alkoxy-C₁-C₂-Alkyl-, Dimethylamino-, Trifluormethyl-, Difluorethyl-, Trifluorethyl-, Trifluormethoxy-, Cyclopropyl, - C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, Pyridinyl-, Phenyl-, und/oder Fluorphenyl-.

In der allgemeinen Formel (I) steht R^{1a} ferner ganz besonders bevorzugt für Phenyl-, das gegebenenfalls ein- oder mehrfach, gleich oder verschieden substituiert sein kann mit Fluor, Chlor, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Dimethylamino-, -C(=O)NR⁶R⁷, C(=O)R⁸, C₁-C₃-Alkylsulfinyl-, C₁-C₃-Alkylsulfonyl-, - S(=O)₂NH₂, C₁-C₃-Alkylsulfonylamino-, C₁-C₃-Alkylaminosulfonyl-, C₃-C₆-Cycloalkylaminosulfonyl-, Trifluormethyl-, Difluorethyl-, Trifluorethyl-, Trifluormethoxy-, Hydroxy-C₁-C₃-Alkyl-, Cyclopropyl, Morpholino- und/oder Pyridinyl-.

In der allgemeinen Formel (I) steht R^{1a} ferner ganz besonders bevorzugt für Wasserstoff oder Chlor,
oder
für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl-, die gegebenenfalls ein- oder mehrfach, gleich oder verschieden substituiert sein können mit Fluor, Hydroxy, Oxo, C₁-C₃-Alkyl-, Methoxy, Hydroxy-C₁-C₃-Alkyl-, Dimethylamino-, Difluorethyl-, Trifluorethyl-, Benzyl-, - NR⁶C(=O)-R⁹, -C(=O)-NR⁶R⁷, -C(=O)-R⁸, und/oder -S(=O)₂-R⁹,
oder
für Tetrazolyl-,
oder
für Isoxazolyl-, Pyrazolyl-, Thienyl-, Thiazolyl-, Imidazolyl-, Triazolyl-, Pyrrolyl-, Oxadiazolyl-, Pyridinyl- oder Pyrimidinyl-, die gegebenenfalls ein- oder mehrfach, gleich oder verschieden substituiert sein können mit Fluor, Chlor, Hydroxy, Cyano, C₁-C₂-Alkyl-, Methoxy-, Methoxymethyl-, Trifluormethyl-, Cyclopropyl, -C(=O)-R⁸, Pyridinyl-, Phenyl-, und/oder Fluorphenyl-,
oder
für Phenyl-, das gegebenenfalls ein- oder mehrfach, gleich oder verschieden substituiert sein kann mit Fluor, Chlor, Hydroxy, Cyano, Nitro, Carboxy, C₁-C₃-Alkyl-, Methoxy-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Trifluormethyl-, Trifluormethoxy-, Hydroxy-C₁-C₃-Alkyl-, Cyclopropyl-, und/oder Morpholino-.

In der allgemeinen Formel (I) steht R^{1a} ferner ganz besonders bevorzugt für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl-, die gegebenenfalls ein- oder mehrfach, gleich oder verschieden substituiert sein können mit Fluor, Hydroxy, Oxo, C₁-C₃-Alkyl-, Methoxy, Hydroxy-C₁-C₃-Alkyl-, Dimethylamino-, Difluorethyl-, Trifluorethyl-, Benzyl-, -NR⁶C(=O)-R⁹, - C(=O)-NR⁶R⁷, -C(=O)-R⁸, und/oder -S(=O)₂-R⁹,
oder
für Tetrazolyl-,
oder
für Isoxazolyl-, Pyrazolyl-, Thienyl-, Thiazolyl-, Imidazolyl-, Triazolyl-, Pyrrolyl-, Oxadiazolyl-, Pyridinyl- oder Pyrimidinyl-, die gegebenenfalls ein- oder mehrfach, gleich oder verschieden substituiert sein können mit Fluor, Chlor, Hydroxy, Cyano, C₁-C₂-Alkyl-, Methoxy-, Methoxymethyl-, Trifluormethyl-, Cyclopropyl, -C(=O)-R⁸, Pyridinyl-, Phenyl-, und/oder Fluorphenyl-,
oder
für Phenyl-, das gegebenenfalls ein- oder mehrfach, gleich oder verschieden substituiert sein kann mit Fluor, Chlor, Hydroxy, Cyano, Nitro, Carboxy, C₁-C₃-Alkyl-, Methoxy-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Trifluormethyl-, Trifluormethoxy-, Hydroxy-C₁-C₃-Alkyl-, Cyclopropyl-, und/oder Morpholino-.

In der allgemeinen Formel (I) steht R^{1a} überaus bevorzugt für Piperazinyl-, Pyrrolidinyl-, Piperidinyl-, Diazepanyl-, Oxazinanyl-, Oxazolidinyl-, Morpholinyl-, Thiomorpholinyl-, Thiazolidinyl- oder Azetidinyl-, die gegebenenfalls ein- oder mehrfach, gleich oder verschieden substituiert sein können mit Fluor, Hydroxy, Oxo, C₁-C₃-Alkyl-, Methoxy, Hydroxy-C₁-C₃-Alkyl-, Dimethylamino-, Difluorethyl-, Trifluorethyl-, Benzyl-, -NR⁶C(=O)-R⁹, -C(=O)-NR⁶R⁷, -C(=O)-R⁸, und/oder -S(=O)₂-R⁹,
oder
für Tetrazolyl-,
oder
für Isoxazolyl-, Pyrazolyl-, Thienyl-, Thiazolyl-, Imidazolyl-, Triazolyl-, Pyrrolyl-, Oxadiazolyl-, Pyridinyl- oder Pyrimidinyl-, die gegebenenfalls ein- oder mehrfach, gleich oder verschieden substituiert sein können mit Fluor, Chlor, Hydroxy, Cyano, C₁-C₂-Alkyl-, Methoxy-, Methoxymethyl-, Trifluormethyl-, Cyclopropyl, -C(=O)-R⁸, Pyridinyl-, Phenyl-, und/oder Fluorphenyl-,
oder
für Phenyl-, das gegebenenfalls ein- oder mehrfach, gleich oder verschieden substituiert sein kann mit Fluor, Chlor, Hydroxy, Cyano, Nitro, Carboxy, C₁-C₃-Alkyl-, C₁-C₃-Methoxy-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Trifluormethyl-, Trifluormethoxy-, Hydroxy-C₁-C₃-Alkyl-, Cyclopropyl-, und/oder Morpholino-.

In der allgemeinen Formel (I) steht R^{1a} ferner überaus bevorzugt für Piperazinyl-, Pyrrolidinyl-, Piperidinyl-, Diazepanyl-, Oxazinanyl-, Oxazolidinyl-, Morpholinyl-, Thiomorpholinyl-, Thiazolidinyl- oder Azetidinyl-, die gegebenenfalls ein- oder mehrfach, gleich oder verschieden substituiert sein können mit Fluor, Hydroxy, Oxo, C₁-C₃-Alkyl-, Methoxy, Hydroxy-C₁-C₃-Alkyl-, Dimethylamino-, Difluorethyl-, Trifluorethyl-, Benzyl-, -NR⁶C(=O)-R⁹, -C(=O)-NR⁶R⁷, -C(=O)-R⁸, und/oder -S(=O)₂-R⁹.

In der allgemeinen Formel (I) steht R^{1a} ferner überaus bevorzugt für Isoxazolyl-, Pyrazolyl-, Thienyl-, Thiazolyl-, Imidazolyl-, Triazolyl-, Pyrrolyl-, Oxadiazolyl-, Pyridinyl- oder Pyrimidinyl-, die gegebenenfalls ein- oder mehrfach, gleich oder verschieden substituiert sein können mit Fluor, Chlor, Hydroxy, Cyano, C₁-C₂-Alkyl-, Methoxy-, Methoxymethyl-, Trifluormethyl-, Cyclopropyl, - C(=O)-R⁸, Pyridinyl-, Phenyl-, und/oder Fluorphenyl-.

In der allgemeinen Formel (I) steht R^{1a} ferner überaus bevorzugt für Phenyl-, das gegebenenfalls ein- oder mehrfach, gleich oder verschieden substituiert sein kann mit Fluor, Chlor, Hydroxy, Cyano, Nitro, Carboxy, C₁-C₃-Alkyl-, C₁-C₃-Methoxy-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Trifluormethyl-, Trifluormethoxy-, Hydroxy-C₁-C₃-Alkyl-, Cyclopropyl-, und/oder Morpholino-.

In der allgemeinen Formel (I) steht R^{1a} ferner überaus bevorzugt für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl-, die gegebenenfalls ein- oder mehrfach, gleich oder verschieden substituiert sein können mit Fluor, Hydroxy, Oxo, C₁-C₃-Alkyl-, Methoxy, Hydroxy-C₁-C₃-Alkyl-, Dimethylamino-, Difluorethyl-, Trifluorethyl-, Benzyl-, -NR⁶C(=O)-R⁹, -C(=O)-NR⁶R⁷, -C(=O)-R⁸, und/oder -S(=O)₂-R⁹,
oder
für Isoxazolyl- oder Pyrazolyl-, die gegebenenfalls ein- oder mehrfach, gleich oder verschieden mit C₁-C₂-Alkyl-substituiert sein können.

In der allgemeinen Formel (I) steht R^{1a} ferner überaus bevorzugt für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl-, die gegebenenfalls ein- oder mehrfach, gleich oder verschieden substituiert sein können mit Fluor, Hydroxy, Oxo, C₁-C₃-Alkyl-, Methoxy, Hydroxy-C₁-C₃-Alkyl-, Dimethylamino-, Difluorethyl-, Trifluorethyl-, Benzyl-, -NR⁶C(=O)-R⁹, -C(=O)-NR⁶R⁷, -C(=O)-R⁸, und/oder -S(=O)₂-R⁹.

In der allgemeinen Formel (I) steht R^{1a} ferner überaus bevorzugt für Isoxazolyl- oder Pyrazolyl-, die gegebenenfalls ein- oder mehrfach, gleich oder verschieden mit C₁-C₂-Alkyl-substituiert sein können.

In der allgemeinen Formel (I) steht R^{1a} ferner überaus bevorzugt für Piperazinyl-, Pyrrolidinyl-, Piperidinyl-, Morpholinyl-, Thiomorpholinyl- oder Azetidinyl-, die gegebenenfalls ein- oder mehrfach, gleich oder verschieden substituiert sein können mit Hydroxy, Oxo, C₁-C₃-Alkyl-, Methoxy-, Dimethylamino-, Difluorethyl-, Trifluorethyl-, -NR⁶C(=O)-R⁹, -C(=O)-NR⁶R⁷, und/oder -C(=O)-R⁸.

In der allgemeinen Formel (I) steht R^{1a} ferner überaus bevorzugt für Piperazinyl-, Pyrrolidinyl-, Piperidinyl-, Morpholinyl-, Thiomorpholinyl- oder Azetidinyl-, die gegebenenfalls einfach substituiert sein können mit C₁-C₃-Alkyl.

In der allgemeinen Formel (I) steht R^{1a} ferner überaus bevorzugt für Piperazinyl-, Pyrrolidinyl-, Piperidinyl-, Morpholinyl-, Thiomorpholinyl- oder Azetidinyl-, die gegebenenfalls einfach substituiert sein können mit Methyl.

In der allgemeinen Formel (I) steht R^{1a} ferner überaus bevorzugt für Piperazinyl-, das gegebenenfalls ein- oder mehrfach, gleich oder verschieden substituiert sein kann mit Hydroxy, Oxo, C₁-C₃-Alkyl-, Methoxy-, Dimethylamino-, Difluorethyl-, Trifluorethyl-, -NR⁶C(=O)-R⁹, - C(=O)-NR⁶R⁷, und/oder -C(=O)-R⁸.

In der allgemeinen Formel (I) stehen R^{1b} und R^{1c} bevorzugt und unabhängig voneinander für Wasserstoff, Halogen, Hydroxy, Cyano, Nitro oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl-Rest, und/oder einen monocyclischen Heterocyclylrest mit 4 bis 7 Ringatomen.

In der allgemeinen Formel (I) steht R^{1b} bevorzugt für Wasserstoff, Halogen, Hydroxy, Cyano, Nitro oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl-Rest, oder einen monocyclischen Heterocyclylrest mit 4 bis 7 Ringatomen.

In der allgemeinen Formel (I) steht R^{1b} besonders bevorzugt für Wasserstoff, Halogen, Hydroxy, Cyano, Nitro oder für einen C₁-C₃-Alkyl-, C₁-C₃-Alkoxy-, Fluor-C₁-C₃-Alkyl- oder Fluor-C₁-C₃-Alkoxy-Rest.

In der allgemeinen Formel (I) steht R^{1c} besonders bevorzugt für Wasserstoff, Fluor, Chlor, Brom oder Cyano.

In der allgemeinen Formel (I) steht R^{1b} ganz besonders bevorzugt für Wasserstoff, Fluor, Brom oder Cyano.

In der allgemeinen Formel (I) steht R^{1c} ganz besonders bevorzugt für Wasserstoff oder Brom.

In der allgemeinen Formel (I) steht R^{1c} ganz besonders bevorzugt für Wasserstoff.

In der allgemeinen Formel (I) steht R^{1b} ganz besonders bevorzugt für Wasserstoff, Fluor, Brom oder Cyano und R^{1c} steht für Wasserstoff.

In der allgemeinen Formel (I) kann R² für einen C₁-C₃-Alkyl- oder Trifluormethyl- oder einen C₃-oder C₄-Cycloalkylrest stehen.

In der allgemeinen Formel (I) steht R² bevorzugt für Methyl-, Ethyl- oder Isopropyl-.

In der allgemeinen Formel (I) steht R² besonders bevorzugt für Methyl- oder Ethyl-.

In der allgemeinen Formel (I) steht R² überaus bevorzugt für Methyl-.

In der allgemeinen Formel (I) steht R³ bevorzugt für Cyclopropyl-, C₁-C₃-Alkyl-, C₁-C₃-Alkoxy-, Amino-, Cyclopropylamino- oder C₁-C₃-Alkylamino-.

In der allgemeinen Formel (I) steht R³ besonders bevorzugt für Cyclopropyl-, C₁-C₃-Alkyl-, C₁-C₃-Alkoxy-, Cyclopropylamino- oder C₁-C₃-Alkylamino-.

In der allgemeinen Formel (I) steht R³ ganz besonders bevorzugt für Cyclopropyl-, Methyl-, Ethyl-, Methoxy-, Ethoxy-, Cyclopropylamino-, Methylamino- oder Ethylamino-.

In der allgemeinen Formel (I) steht R³ überaus bevorzugt für Methylamino-.

In der allgemeinen Formel (I) können R⁴ und R⁵ unabhängig voneinander stehen für für Wasserstoff, Hydroxy, Cyano, Nitro, Amino, Aminocarbonyl-, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die gegebenenfalls ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, monocyclischem Heterocyclyl- mit 3 bis 8 Ringatomen und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl, oder
für C₃-C₁₀-Cycloalkyl-, das gegebenenfalls ein- oder mehrfach, gleich oder verschieden substituiert sein kann mit Halogen, Amino, Hydroxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, und/oder einem monocyclischen Heterocyclylrest mit 3 bis 8 Ringatomen,
oder
für monozyklisches Heteroaryl- mit 5 oder 6 Ringatomen, das gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷, und/oder einem monocyclischen Heterocyclylrest mit 3 bis 8 Ringatomen,
oder
für monozyklisches Heterocyclyl- mit 3 bis 8 Ringatomen, das gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷, und/oder einem monocyclischen Heterocyclylrest mit 3 bis 8 Ringatomen,
oder
für Phenyl-, das gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl-, C₁-C₆-Alkylaminosulfonyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl- und/oder einem monocyclischen Heterocyclylrest mit 3 bis 8 Ringatomen.

In der allgemeinen Formel (I) stehen R⁴ und R⁵ bevorzugt und unabhängig voneinander für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbo-nyl- oder C₁-C₆-Alkylaminosulfonyl-,
oder
für C₁-C₆-Alkyl-, C₁-C₆-Alkoxy-, C₁-C₆-Alkylamino-, C₁-C₆-Alkylcarbonyl-amino-, C₁-C₆-Alkylaminocarbonyl- oder C₁-C₆-Alkylaminosulfonyl-, die ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, einem monocyclischen Heterocyclylrest mit 4 bis 7 Ringatomen, und/oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl-, oder
für einen C₃-C₁₀-Cycloalkylrest, der gegebenenfalls ein- oder mehrfach, gleich oder verschieden substituiert sein kann mit Halogen, Amino, Hydroxy, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, und/oder einem monocyclischen Heterocyclylrest mit 4 bis 7 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen monozyklischen Heterocyclylrest mit 4 bis 7 Ringatomen, der gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷, und/oder einem monocyclischen Heterocyclylrest mit 4 bis 7 Ringatomen.

In der allgemeinen Formel (I) steht R⁴ bevorzugt für einen C₃-C₁₀-Cycloalkylrest, der gegebenenfalls ein- oder mehrfach, gleich oder verschieden substituiert sein kann mit Halogen, Amino, Hydroxy, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, und/oder einem monocyclischen Heterocyclylrest mit 4 bis 7 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen monozyklischen Heterocyclylrest mit 4 bis 7 Ringatomen, der gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷, und/oder einem monocyclischen Heterocyclylrest mit 4 bis 7 Ringatomen,
und R⁵ steht bevorzugt für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbo-nyl- oder C₁-C₆-Alkylaminosulfonyl-,
oder
für C₁-C₆-Alkyl-, C₁-C₆-Alkoxy-, C₁-C₆-Alkylamino-, C₁-C₆-Alkylcarbonyl-amino-, C₁-C₆-Alkylaminocarbonyl- oder C₁-C₆-Alkylaminosulfonyl-, die ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, einem monocyclischen Heterocyclylrest mit 4 bis 7 Ringatomen, und/oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl-.

In der allgemeinen Formel (I) steht R⁴ bevorzugt für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbo-nyl- oder C₁-C₆-Alkylaminosulfonyl-,
oder
für C₁-C₆-Alkyl-, C₁-C₆-Alkoxy-, C₁-C₆-Alkylamino-, C₁-C₆-Alkylcarbonyl-amino-, C₁-C₆-Alkylaminocarbonyl- oder C₁-C₆-Alkylaminosulfonyl-, die ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, einem monocyclischen Heterocyclylrest mit 4 bis 7 Ringatomen, und/oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl-, und R⁵ steht bevorzugt für einen C₃-C₁₀-Cycloalkylrest, der gegebenenfalls ein- oder mehrfach, gleich oder verschieden substituiert sein kann mit Halogen, Amino, Hydroxy, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, und/oder einem monocyclischen Heterocyclylrest mit 4 bis 7 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen monozyklischen Heterocyclylrest mit 4 bis 7 Ringatomen, der gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷, und/oder einem monocyclischen Heterocyclylrest mit 4 bis 7 Ringatomen.

In der allgemeinen Formel (I) stehen R⁴ und R⁵ besonders bevorzugt und unabhängig voneinander für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₃-Alkyl-, C₁-C₃-Alkoxy-, C₁-C₃-Alkylamino-, die ein- oder mehrfach, gleich oder verschieden substituiert sein können mit Halogen, Amino, Hydroxy, Carboxy, Hydroxy-C₁-C₃-Alkyl-, C₁-C₃-Alkoxy-, C₁-C₃-Alkylamino-, Amino-C₁-C₃-Alkyl-, einem monocyclischen Heterocyclylrest mit 4 bis 7 Ringatomen, und/oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl,
oder
für einen C₃-C₇-Cycloalkylrest, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen monozyklischen Heterocyclylrest mit 4 bis 7 Ringatomen, der gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylaminocarbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen.

In der allgemeinen Formel (I) steht R⁴ besonders bevorzugt für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₃-Alkyl-, C₁-C₃-Alkoxy-, C₁-C₃-Alkylamino-, die ein- oder mehrfach, gleich oder verschieden substituiert sein können mit Halogen, Amino, Hydroxy, Carboxy, Hydroxy-C₁-C₃-Alkyl-, C₁-C₃-Alkoxy-, C₁-C₃-Alkylamino-, Amino-C₁-C₃-Alkyl-, einem monocyclischen Heterocyclylrest mit 4 bis 7 Ringatomen, und/oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl,
und R⁵ steht besonders bevorzugt für einen C₃-C₇-Cycloalkylrest, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen monozyklischen Heterocyclylrest mit 4 bis 7 Ringatomen, der gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylaminocarbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen.

In der allgemeinen Formel (I) steht R⁴ besonders bevorzugt für einen C₃-C₇-Cycloalkylrest, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen monozyklischen Heterocyclylrest mit 4 bis 7 Ringatomen, der gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylaminocarbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen, und R⁵ steht besonders bevorzugt für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₃-Alkyl-, C₁-C₃-Alkoxy-, C₁-C₃-Alkylamino-, die ein- oder mehrfach, gleich oder verschieden substituiert sein können mit Halogen, Amino, Hydroxy, Carboxy, Hydroxy-C₁-C₃-Alkyl-, C₁-C₃-Alkoxy-, C₁-C₃-Alkylamino-, Amino-C₁-C₃-Alkyl-, einem monocyclischen Heterocyclylrest mit 4 bis 7 Ringatomen, und/oder einem monocyclischen Heteroarylrest mit 5 oder 6 Ringatomen, worin die genannten monocyclischen Heterocyclyl- und Heteroarylreste ihrerseits gegebenenfalls einfach substituiert sein können durch C₁-C₃-Alkyl.

In der allgemeinen Formel (I) steht R⁴ besonders bevorzugt für einen C₃-C₇-Cycloalkylrest, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen monozyklischen Heterocyclylrest mit 4 bis 7 Ringatomen, der gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylaminocarbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen.

In der allgemeinen Formel (I) steht R⁴ besonders bevorzugt für einen C₃-C₇-Cycloalkylrest, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen.

In der allgemeinen Formel (I) steht R⁴ besonders bevorzugt für einen monozyklischen Heteroarylrest mit 5 oder 6 Ringatomen, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen.

In der allgemeinen Formel (I) steht R⁴ besonders bevorzugt für einen monozyklischen Heterocyclylrest mit 4 bis 7 Ringatomen, der gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen.

In der allgemeinen Formel (I) steht R⁴ besonders bevorzugt für einen Phenylrest, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylaminocarbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen.

In der allgemeinen Formel (I) steht R⁵ besonders bevorzugt für einen C₃-C₇-Cycloalkylrest, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen,
oder
für einen monozyklischen Heteroarylrest mit 5 oder 6 Ringatomen, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen monozyklischen Heterocyclylrest mit 4 bis 7 Ringatomen, der gegebenenfalls ein- oder mehrfach, gleich oder verschieden substituiert sein kann mit Halogen, Amino, Hydroxy, Cyano, Oxo, Carboxy, C₁-C₅-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylaminocarbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen.

In der allgemeinen Formel (I) steht R⁵ besonders bevorzugt für einen C₃-C₇-Cycloalkylrest, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen.

In der allgemeinen Formel (I) steht R⁵ besonders bevorzugt für einen monozyklischen Heteroarylrest mit 5 oder 6 Ringatomen, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸,-S(=O)₂R⁹, -NR⁶R⁷, und/oder einem monocyclischen Heterocyclylrest mit 4 bis 7 Ringatomen.

In der allgemeinen Formel (I) steht R⁵ besonders bevorzugt für einen monozyklischen Heterocyclylrest mit 4 bis 7 Ringatomen, der gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸,-S(=O)₂R⁹, -NR⁶R⁷, und/oder einem monocyclischen Heterocyclylrest mit 4 bis 7 Ringatomen.

In der allgemeinen Formel (I) steht R⁵ besonders bevorzugt für einen Phenylrest, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylaminocarbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen.

In der allgemeinen Formel (I) stehen R⁴ und R⁵ ganz besonders bevorzugt und unabhängig voneinander für Wasserstoff, Hydroxy, Cyano, Amino, Chlor, C₁-C₆-Alkyl-, Methoxy-, Ethoxy- oder C₁-C₃-Alkylcarbonylamino-,
oder
für Difluormethoxy- oder Trifluormethoxy-,
oder
für C₁-C₃-Alkoxy-, welches mit Pyridinyl-, Morpholinyl-, Pyrrolidinyl- oder Piperazinyl-substituiert sein kann,
worin das Pyridinyl- und Piperazinyl- seinerseits gegebenenfalls mit C₁-C₃-Alkyl- substituiert sein kann,
oder
für Cyclopropyl-,
oder
für Pyridinyl-, Pyrazolyl-, Triazolyl- oder Isoxazolyl-, die gegebenenfalls ein- oder mehrfach, gleich oder verschieden substituiert sein können mit Hydroxy, Methyl-,
oder
für Pyrrolidinyl-, Morpholinyl-, Piperidinyl-, Piperazinyl-, Oxazolidinyl-, Thiomorpholinyl-, die gegebenenfalls ein- oder mehrfach substituiert sein können mit Methyl-, Oxo, -S(=O)₂R⁹,
oder
für gegebenenfalls mit C₁-C₃-Alkylaminosulfonyl- oder Fluor substituiertes Phenyl.

In der allgemeinen Formel (I) steht R⁴ ganz besonders bevorzugt für Wasserstoff, Hydroxy, Cyano, Amino, Chlor, C₁-C₆-Alkyl-, Methoxy-, Ethoxy- oder C₁-C₃-Alkylcarbonylamino-,
oder
für Difluormethoxy- oder Trifluormethoxy-,
oder
für C₁-C₃-Alkoxy-, welches mit Pyridinyl-, Morpholinyl-, Pyrrolidinyl- oder Piperazinyl-substituiert sein kann,
worin das Pyridinyl- und Piperazinyl- seinerseits gegebenenfalls mit C₁-C₃-Alkyl- substituiert sein kann,
oder
für Cyclopropyl-,
oder
für Pyridinyl-, Pyrazolyl-, Triazolyl- oder Isoxazolyl-, die gegebenenfalls ein- oder mehrfach, gleich oder verschieden substituiert sein können mit Hydroxy, Methyl-,
oder
für Pyrrolidinyl-, Morpholinyl-, Piperidinyl-, Piperazinyl-, Oxazolidinyl-, Thiomorpholinyl-, die gegebenenfalls ein- oder mehrfach substituiert sein können mit Methyl-, Oxo, -S(=O)₂R⁹,
oder
für gegebenenfalls mit C₁-C₃-Alkylaminosulfonyl- oder Fluor substituiertes Phenyl.

In der allgemeinen Formel (I) steht R⁵ ganz besonders bevorzugt für Wasserstoff, Hydroxy, Cyano, Amino, Chlor, C₁-C₆-Alkyl-, Methoxy-, Ethoxy- oder C₁-C₃-Alkylcarbonylamino-,
oder
für Difluormethoxy- oder Trifluormethoxy-,
oder
für C₁-C₃-Alkoxy-, welches mit Pyridinyl-, Morpholinyl-, Pyrrolidinyl- oder Piperazinyl-substituiert sein kann,
worin das Pyridinyl- und Piperazinyl- seinerseits gegebenenfalls mit C₁-C₃-Alkyl- substituiert sein kann,
oder
für Cyclopropyl-,
oder
für Pyridinyl-, Pyrazolyl-, Triazolyl- oder Isoxazolyl-, die gegebenenfalls ein- oder mehrfach, gleich oder verschieden substituiert sein können mit Hydroxy, Methyl-,
oder
für Pyrrolidinyl-, Morpholinyl-, Piperidinyl-, Piperazinyl-, Oxazolidinyl-, Thiomorpholinyl-, die gegebenenfalls ein- oder mehrfach substituiert sein können mit Methyl-, Oxo, -S(=O)₂R⁹,
oder
für gegebenenfalls mit C₁-C₃-Alkylaminosulfonyl- oder Fluor substituiertes Phenyl.

In der allgemeinen Formel (I) steht R⁴ ganz besonders bevorzugt für Cyclopropyl-,
oder
für Pyridinyl-, Pyrazolyl-, Triazolyl- oder Isoxazolyl-, die gegebenenfalls ein- oder mehrfach, gleich oder verschieden substituiert sein können mit Hydroxy, Methyl-,
oder
für Pyrrolidinyl-, Morpholinyl-, Piperidinyl-, Piperazinyl-, Oxazolidinyl-, Thiomorpholinyl-, die gegebenenfalls ein- oder mehrfach substituiert sein können mit Methyl-, Oxo, -S(=O)₂R⁹,
oder
für gegebenenfalls mit C₁-C₃-Alkylaminosulfonyl- oder Fluor substituiertes Phenyl.

In der allgemeinen Formel (I) steht R⁵ ganz besonders bevorzugt für Cyclopropyl-,
oder
für Pyridinyl-, Pyrazolyl-, Triazolyl- oder Isoxazolyl-, die gegebenenfalls ein- oder mehrfach, gleich oder verschieden substituiert sein können mit Hydroxy, Methyl-,
oder
für Pyrrolidinyl-, Morpholinyl-, Piperidinyl-, Piperazinyl-, Oxazolidinyl-, Thiomorpholinyl-, die gegebenenfalls ein- oder mehrfach substituiert sein können mit Methyl-, Oxo, -S(=O)₂R⁹,
oder
für gegebenenfalls mit C₁-C₃-Alkylaminosulfonyl- oder Fluor substituiertes Phenyl.

In der allgemeinen Formel (I) steht R⁴ ganz besonders bevorzugt für C₁-C₃-Alkoxy-, welches mit Pyridinyl-, Morpholinyl-, Pyrrolidinyl- oder Piperazinyl- substituiert sein kann,
worin das Pyridinyl- und Piperazinyl- seinerseits gegebenenfalls mit C₁-C₃-Alkyl- substituiert sein kann.

In der allgemeinen Formel (I) steht R⁵ ganz besonders bevorzugt für C₁-C₃-Alkoxy-, welches mit Pyridinyl-, Morpholinyl-, Pyrrolidinyl- oder Piperazinyl- substituiert sein kann,
worin das Pyridinyl- und Piperazinyl- seinerseits gegebenenfalls mit C₁-C₃-Alkyl- substituiert sein kann.

In der allgemeinen Formel (I) steht R⁴ ganz besonders bevorzugt für Difluormethoxy- oder Trifluormethoxy-.

In der allgemeinen Formel (I) steht R⁵ ganz besonders bevorzugt für Difluormethoxy- oder Trifluormethoxy-.

In der allgemeinen Formel (I) steht R⁵ überaus bevorzugt für Trifluormethoxy-.

In der allgemeinen Formel (I) steht R⁴ überaus bevorzugtfür Cyclopropyl-,
oder
für Pyridinyl-, Pyrazolyl-, Triazolyl- oder Isoxazolyl-, die gegebenenfalls ein- oder mehrfach, gleich oder verschieden substituiert sein können mit Hydroxy, Methyl-,
oder
für Pyrrolidinyl-, Morpholinyl-, Piperidinyl-, Piperazinyl-, Oxazolidinyl- oder Thiomorpholinyl-, die gegebenenfalls ein- oder mehrfach substituiert sein können mit Oxo, Methyl-, -S(=O)₂R⁹, oder
für gegebenenfalls mit C₁-C₃-Alkylaminosulfonyl- oder Fluor substituiertes Phenyl-, und R⁵ steht überaus bevorzugt für Wasserstoff, Hydroxy, Cyano, Chlor, C₁-C₆-Alkyl-, Methoxy-, Ethoxy-, C₁-C₃-Alkylcarbonylamino-, Difluormethoxy- oder Trifluormethoxy-.

In der allgemeinen Formel (I) steht R⁴ überaus bevorzugtfür Wasserstoff, Chlor, Methoxy-, Ethoxy-, Difluormethoxy- oder Trifluormethoxy- steht, und
R⁵ steht überaus bevorzugt für Cyclopropyl-,
oder
für Pyridinyl- oder Pyrazolyl- , die gegebenenfalls ein- oder mehrfach, mit Methyl- substituiert sein können,
oder
für Morpholinyl-, Piperidinyl-, Piperazinyl-, oder Thiomorpholinyl-, die gegebenenfalls ein- oder mehrfach substituiert sein können mit Oxo, Methyl-, -S(=O)₂R⁹,
oder
für mit C₁-C₃-Alkylaminosulfonyl- substituiertes Phenyl.

In der allgemeinen Formel (I) stehen R⁴ und R⁵ überaus bevorzugt und unabhängig voneinander für Wasserstoff, Hydroxy, Cyano, Chlor, C₁-C₆-Alkyl-, Methoxy-, Ethoxy- , C₁-C₃-Alkylcarbonylamino-, Difluormethoxy- oder Trifluormethoxy-.

In der allgemeinen Formel (I) stehen R⁴ und R⁵ überaus bevorzugt und unabhängig voneinander für unabhängig voneinander für Wasserstoff, Chlor, Methoxy-, Ethoxy- , Difluormethoxy- oder Trifluormethoxy-.

In der allgemeinen Formel (I) stehen R⁶ und R⁷ bevorzugt und unabhängig voneinander für Wasserstoff, C₁-C₃-Alkyl-, Cyclopropyl-, Di-C₁-C₃-Alkyl-Amino-C₁-C₃-Alkyl- oder Fluorpyridyl-.

In der allgemeinen Formel (I) stehen R⁶ und R⁷ besonders bevorzugt und unabhängig voneinander für Wasserstoff, C₁-C₃-Alkyl-, Cyclopropyl-, Di-C₁-C₃-Alkyl-Amino-C₁-C₃-Alkyl-.

In der allgemeinen Formel (I) stehen R⁶ und R⁷ ganz besonders bevorzugt und unabhängig voneinander für Wasserstoff oder C₁-C₃-Alkyl-.

In der allgemeinen Formel (I) kann R⁸ stehen für Hydroxy, C₁-C₆-Alkyl-, Halogen-C₁-C₃-Alkyl-, Hydroxy-C₁-C₃-Alkyl-, C₁-C₃-Alkoxy-C₁-C₃-Alkyl-, C₃-C₈-Cycloalkyl-, Phenyl-, monocyclisches Heterocyclyl- mit 3 bis 8 Ringatomen oder monocyclisches Heteroaryl- mit 5 oder 6 Ringatomen steht, worin Phenyl-, Heteroaryl- und Heterocyclyl- gegebenenfalls ein- oder zweifach substituiert sein können durch Halogen, C₁-C₃-Alkoxy- oder C₁-C₃-Alkyl-.

In der allgemeinen Formel (I) steht R⁸ bevorzugt für Hydroxy, C₁-C₆-Alkyl-, Halogen-C₁-C₃-Alkyl-, Hydroxy-C₁-C₃-Alkyl-, C₁-C₃-Alkoxy-C₁-C₃-Alkyl-, C₃-C₈-Cycloalkyl-, Phenyl-, monocyclisches Heterocyclyl- mit 5 oder 6 Ringatomen.

In der allgemeinen Formel (I) steht R⁸ ganz besonders bevorzugt für Hydroxy, C₁-C₃-Alkyl-, Hydroxy-C₁-C₃-Alkyl-, Trifluormethyl-, Pyrrolidinyl-, Morpholinyl- oder Piperidinyl-.

In der allgemeinen Formel (I) steht R⁸ ganz besonders bevorzugt für C₁-C₃-Alkyl-.

In der allgemeinen Formel (I) steht R⁸ ganz besonders bevorzugt für Methyl-.

In der allgemeinen Formel (I) steht R⁹ bevorzugt für Wasserstoff, C₁-C₆-Alkyl- oder C₁-C₄-Alkoxy-.

In der allgemeinen Formel (I) steht R⁹ besonders bevorzugt für C₁-C₄-Alkyl- oder C₁-C₄-Alkoxy-.

In der allgemeinen Formel (I) steht R⁹ besonders bevorzugt für Methyl-.

In der allgemeinen Formel (I) steht R⁹ besonders bevorzugt für *tert*-Butoxy-.

In der allgemeinen Formel (I) liegt das durch das an R² gebundene Kohlenstoffatom des Benzodiazepin-Gerüsts repräsentierte Stereozentrum bevorzugt entweder racemisch oder überwiegend oder vollständig in der (S)-Konfiguration vor.

In der allgemeinen Formel (I) liegt das durch das an R² gebundene Kohlenstoffatom des Benzodiazepin-Gerüsts repräsentierte Stereozentrum bevorzugt racemisch vor.

In der allgemeinen Formel (I) liegt das durch das an R² gebundene Kohlenstoffatom des Benzodiazepin-Gerüsts repräsentierte Stereozentrum besonders bevorzugt überwiegend oder vollständig in der (*S*)-Konfiguration vor.

In der allgemeinen Formel (I) liegt das durch das an R² gebundene Kohlenstoffatom des Benzodiazepin-Gerüsts repräsentierte Stereozentrum besonders bevorzugt überwiegend in der (S)-Konfiguration vor.

In der allgemeinen Formel (I) liegt das durch das an R² gebundene Kohlenstoffatom des Benzodiazepin-Gerüsts repräsentierte Stereozentrum besonders bevorzugt vollständig in der (*S*)-Konfiguration vor.

Gegenstand der Erfindung sind weiterhin Verbindungen der allgemeinen Formel (I), in der A für Phenyl steht und R⁴ für Wasserstoff, Fluor, Chlor oder Brom steht und R⁵ für C₁-C₆-Alkoxy-, das ein- oder mehrfach, gleich oder verschieden mit Halogen substituiert ist, steht, und R^{1a} für Halogen steht.

Gegenstand der Erfindung sind weiterhin Verbindungen der allgemeinen Formel (I), in der A für Phenyl steht und R⁴ für Wasserstoff steht und R⁵ für C₁-C₆-Alkoxy steht, welches ein- oder mehrfach, gleich oder verschieden mit einem monocyclischen Heterocyclylrest mit 3 bis 8 Ringatomen, und/oder einem monocyclischen Heteroarylrest mit 5 oder 6 Ringatomen substituiert ist, worin die genannten monocyclischen Heterocyclyl- und Heteroarylreste ihrerseits gegebenenfalls einfach substituiert sein können durch C₁-C₃-Alkyl, und R^{1a} für Halogen steht.

Gegenstand der Erfindung sind weiterhin Verbindungen der allgemeinen Formel (I), in der A für Phenyl steht und R⁴ für Wasserstoff steht und R⁵ für C₁-C₆-Alkoxy steht, welches ein- oder mehrfach, gleich oder verschieden mit einem monocyclischen Heterocyclylrest mit 4 bis 7 Ringatomen, und/oder einem monocyclischen Heteroarylrest mit 5 oder 6 Ringatomen substituiert ist, worin die genannten monocyclischen Heterocyclyl- und Heteroarylreste ihrerseits gegebenenfalls einfach substituiert sein können durch C₁-C₃-Alkyl, und R^{1a} für Halogen steht.

Bevorzugt sind Verbindungen der allgemeinen Formel (I), in der A für Phenyl steht und R⁴ für Wasserstoff, Fluor, Chlor oder Brom steht und R⁵ für C₁-C₃-Alkoxy-, das ein- oder mehrfach, gleich oder verschieden mit Halogen substituiert ist, steht, und R^{1a} für Halogen steht.

Bevorzugt sind ferner Verbindungen der allgemeinen Formel (I), in der A für Phenyl steht und R⁴ für Wasserstoff steht und R⁵ für C₁-C₃-Alkoxy steht, welches ein- oder mehrfach, gleich oder verschieden mit einem monocyclischen Heterocyclylrest mit 4 bis 7 Ringatomen, und/oder einem monocyclischen Heteroarylrest mit 5 oder 6 Ringatomen substituiert ist, worin die genannten monocyclischen Heterocyclyl- und Heteroarylreste ihrerseits gegebenenfalls einfach substituiert sein können durch C₁-C₃-Alkyl, und R^{1a} für Halogen steht.

Ganz besonders interessant sind Verbindungen der allgemeinen Formel (I), in der A für einen Phenylring steht und R⁴ für Wasserstoff oder Chlor steht und R⁵ für Trifluormethoxy steht, und R^{1a} für Chlor steht.
Ganz besonders interessant sind ferner Verbindungen der allgemeinen Formel (I), in der A für einen Phenylring steht und R⁴ für Wasserstoff steht und R⁵ für C₁-C₃-Alkoxy steht, welches mit Morpholinyl-, Pyrrolidinyl-, Piperazinyl- oder Pyridyl- substituiert ist, wobei das Piperazinyl- und Pyridinyl- selbst mit C₁-C₃-Alkyl- substituiert sein kann, und R^{1a} für Chlor steht.

Ganz besonders interessant sind außerdem die Verbindungen der hergestellten Ausführungsbeispiele Nr. 1; 15; 32; 33; 164; 164.2; 165; 166 und 167.

Interessant sind auch solche Verbindungen der allgemeinen Formel (I), in der A für Phenyl steht und R^{1a} einen Phenylrest steht, der gegebenenfalls ein- oder mehrfach, gleich oder verschieden substituiert sein kann mit Fluor, Chlor, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Dimethylamino-, -C(=O)NR⁶R⁷, -C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Trifluormethyl-, Difluorethyl-, Trifluorethyl-, Trifluormethoxy-, Hydroxy-C₁-C₃-Alkyl-, Cyclopropyl-, Chlorthienyl-, Morpholino- und/oder Pyridinyl-.

Die in den jeweiligen Kombinationen bzw. bevorzugten Kombinationen von Resten im Einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Ebenfalls als von der vorliegenden Erfindung als erfasst anzusehen ist die Verwendung der Salze der erfindungsgemäßen Verbindungen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen SäureAdditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen weiterhin Basen-Additionssalze beispielsweise von Alkalimetallen wie Natrium oder Kalium, von Erdalkalimetallen wie Calcium oder Magnesium, oder von Ammoniumsalzen, die abgeleitet sind von Ammoniak oder organischen Aminen, die 1 bis 16 Kohlenstoff-Atome enthalten, wie zum Beispiel Methylamin, Ethylamin, Diethylamin, Triethylamin, Ethyldiisopropylamin, Monoethanolamin, Diethanolamin, Triethanolamin, Dicyclohexylamin, Dimethylaminoethanol, Procain, Dibenzylamin, N-Methylmorpholin, Arginin, Lysin, Ethylenediamin, N-Methylpiperidin, N-Methylglucamin, Dimethylglucamin, Ethylglucamin, 1,6-Hexadiamin, Glucosamin, Sarcosin, Serinol, Tris(hydroxymethyl)aminomethan, Aminopropanediol, Sovak-Base, und/oder 1-Amino-2,3,4-butantriol. Ferner können die erfindungsgemäßen Verbindungen Basen-Additionssalze bilden mit quarternären Ammonium-Ionen, welche beispielsweise durch Qarternisierung entsprechender Amine mit Agentien wie niederen Alkylhalogeniden, zum Beispiel Methyl-, Ethyl-, Propyl- und Butylchloriden, -bromiden und -iodiden, Dialkylsulfaten wie Dimethyl-, Diethyl- Dibutyl- und Diamylsulfat, langkettigen Halogeniden wie Decyl-, Lauryl-, Myristyl- und Stearylchloriden, - bromiden und -iodiden, oder Arylalkylhalogeniden wie Benzylbromid oder Phenethylbromid erhalten werden können. Beispiele derartige quarternärer Ammoniumionen sind Tetramethylammonium, Tetraethylammonium, Tetra(*n*-propyl)ammonium, Tetra(*n-*butyl)ammonium, sowie Benzyltrimethylammonium.

Ein weiterer Gegenstand der vorliegenden Erfindung sind alle möglichen kristallinen und polymorphen Formen der erfindungsgemäßen Verbindungen, wobei die Polymorphe entweder als einzelne Polymorphe oder als Gemisch mehrerer Polymorphe in allen Konzentrationsbereichen vorliegen können.

Weiterer Gegenstand der vorliegenden Erfindung sind Arzneimittel, die die erfindungsgemäßen Verbindungen und mindestens einen oder mehrere weitere Wirkstoffe, insbesondere zur Prophylaxe und/oder Therapie von Tumorerkrankungen, enthalten.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere. Die erfindungsgemäßen Verbindungen weisen an der Position 4 ein Asymmetriezentrum auf. Sie können daher als reine Enantiomere, Racemate aber auch als Diastereomere oder deren Gemische vorliegen, wenn einer oder mehrere der in der Formel (I) beschriebenen Substituenten ein weiteres Asymmetrieelement enthält, beispielsweise ein chirales Kohlenstoffatom. Die vorliegende Erfindung umfasst deshalb auch Enantiomere und Diastereomere und ihre jeweiligen Mischungen. Aus solchen Mischungen lassen sich die reinen Enantiomere und Diastereomere in bekannter Weise isolieren; vorzugsweise werden hierfür chromatographische Verfahren verwendet, insbesondere die HPLC-Chromatographie an chiraler bzw. achiraler Phase.

In der Regel inhibieren die erfindungsgemäßen Enantiomere unterschiedlich stark das Target und sind unterschiedlich aktiv in den untersuchten Krebszelllinien. Das aktivere Enantiomer ist bevorzugt, welches oft das 4S-Enantiomere ist.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Die erfindungsgemäßen Verbindungen können systemisch und/oder lokal wirken. Zu diesem Zweck kann sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.
Weiterer Gegenstand der vorliegenden Erfindung sind Arzneimittel, die die erfindungsgemäßen Verbindungen, üblicherweise zusammen mit einem oder mehreren inerten, nichttoxischen, pharmazeutisch geeigneten Hilfsstoffen enthalten, sowie die erfindungsgemäßen Verbindungen zur Verwendung zu den zuvor genannten Zwecken.
Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.
Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen. Dabei ist auf Remington's Pharmaceutical Science, 15th ed. Mack Publishing Company, East Pennsylvania (1980) hinzuweisen.

Die pharmazeutischen Formulierungen können
in fester Form, zum Beispiel als Tabletten, Dragees, Pillen, Suppositorien, Kapseln, transdermale Systeme oder
in halbfester Form, zum Beispiel als Salben, Cremes, Gele, Suppositorien, Emulsionen oder in flüssiger Form, zum Beispiel als Lösungen, Tinkturen, Suspensionen oder Emulsionen vorliegen.
Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

Die vorliegende Erfindung betrifft die erfindungsgemäßen Verbindungen.
Sie können für die Prophylaxe und Therapie von menschlichen Erkrankungen eingesetzt werden, insbesondere von Tumorerkrankungen.

Die erfindungsgemäßen Verbindungen können insbesondere verwendet werden, um die Zellproliferation und/oder die Zellteilung zu inhibieren oder zu reduzieren und/oder Apoptose zu induzieren.

Die erfindungsgemäßen Verbindungen eignen sich insbesondere zur Prophylaxe und/oder Therapie von hyper-proliferativen Erkrankungen wie beispielsweise
- Psoriasis,
- Keloide und andere Hyperplasien, die die Haut betreffen,
- gutartige Prostathyperplasien (BPH),
- solide Tumore und
- hämatologische Tumore.

Als solide Tumore sind erfindungsgemäß beispielsweise Tumore behandelbar der Brust, des Respirationstraktes, des Gehirns , der Fortpflanzungsorgane, des Magen-Darmtraktes, des Urogenitaltraktes, des Auges, der Leber, der Haut, des Kopfes und des Halses, der Schilddrüse, der Nebenschilddrüse, der Knochen sowie des Bindegewebes und Metastasen dieser Tumore. Als hämatologische Tumore sind beispielsweise behandelbar
- multiple Myelome,
- Lymphome oder
- Leukämien.

Als Brusttumore sind beispielsweise behandelbar:
- Mammakarzinome mit positivem Hormonrezeptorstatus
- Mammakarzinome mit negativem Hormonrezeptorstatus
- Her-2 positive Mammakarzinome
- Hormonrezeptor- und Her-2 negative Mammakarzinome
- BRCA -assoziierte Mammakarzinome
- entzündliche Mammakarzinome.

Als Tumore des Respirationstraktes sind beispielsweise behandelbar
- nicht-kleinzellige Bronchialkarzinome, wie zum Beispiel Plattenepithelkarzinom, Adenokarzinom, großzelliges Karzinom und
- kleinzellige Bronchialkarzinome.

Als Tumore des Gehirns sind beispielsweise behandelbar
- Gliome,
- Glioblastome,
- Astrozytome,
- Meningiome und
- Medulloblastome.

Als Tumore der männlichen Fortpflanzungsorgane sind beispielsweise behandelbar:
- Prostatakarzinome,
- Maligne Nebenhodentumore,
- Maligne Hodentumore und
- Peniskarzinome.

Als Tumore der weiblichen Fortpflanzungsorgane sind beispielsweise behandelbar:
- Endometriumkarzinome
- Zervixkarzinome
- Ovarialkarzinome
- Vaginalkarzimome
- Vulvarkarzinome

Als Tumore des Magen-Darm-Traktes sind beispielsweise behandelbar:
- Kolorektale Karzinome
- Analkarzinome
- Magenkarzinome
- Pankreaskarzinome
- Ösophagukarzinome
- Gallenblasenkarzinome
- Dünndarmkarzinome
- Speicheldrüsenkarzinome
- Neuroendokrine Tumore
- Gastrointestinale Stromatumore

Als Tumore des Urogenital-Traktes sind beispielsweise behandelbar:
- Harnblasenkarzinome
- Nierenzellkarzinome
- Karzinome des Nierenbeckens und der ableitenden Harnwege

Als Tumore des Auges sind beispielsweise behandelbar:
- Retinoblastome
- Intraokulare Melanome

Als Tumore der Leber sind beispielsweise behandelbar:
- Hepatozelluläre Karzinome
- Cholangiozelluläre Karzinome

Als Tumore der Haut sind beispielsweise behandelbar:
- Maligne Melanome
- Basaliome
- Spinaliome
- Kaposi-Sarkome
- Merkelzellkarzinome

Als Tumore des Kopfes und Halses sind beispielsweise behandelbar:
- Larynxkarzinome
- Karzinome des Pharynx und der Mundhöhle
- Karzinome der Mittellinienstrukturen (wie z.B. NMC, C.A. French, Annu. Rev. Pathol. 2012, 7:247-265)

Als Sarkome sind beispielsweise behandelbar:
- Weichteilsarkome
- Osteosarkome

Als Lymphome sind beispielsweise behandelbar:
- Non-Hodgkin-Lymphome
- Hodgkin-Lymphome
- Kutane Lymphome
- Lymphome des zentralen Nervensystems
- AIDS-assoziierte Lymphome

Als Leukämien sind beispielsweise behandelbar:
- Akute myeloische Leukämien
- Chronische myeloische Leukämien
- Akute lymphatische Leukämien
- Chronische lymphatische Leukämien
- Haarzellleukämien

Vorteilhaft können die erfindungsgemäßen Verbindungen verwendet werden zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Besonders vorteilhaft können die erfindungsgemäßen Verbindungen verwendet werden zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Die erfindungsgemäßen Verbindungen eignen sich auch zur Prophylaxe und/oder Therapie von benignen hyperproliferativen Krankheiten wie zum Beispiel Endometriose, Leiomyom und benigne Prostatahyperplasie.

Die erfindungsgemäßen Verbindungen eignen sich auch zur Fertilitätskontrolle des Mannes.

Die erfindungsgemäßen Verbindungen eignen sich auch zur Prophylaxe und/oder Therapie von systemischen inflammatorischen Krankheiten, insbesondere LPS-induzierter endotoxischer Schock und/oder Bakterien-induzierte Sepsis.

Die erfindungsgemäßen Verbindungen eignen sich auch zur Prophylaxe und/oder Therapie von inflammatorischen oder Autoimmunerkrankungen wie zum Beispiel:
- Lungenerkrankungen, die mit entzündlichen, allergischen und/oder proliferativen Prozessen einhergehen: chronisch obstruktive Lungenerkrankungen jeglicher Genese, vor allem Asthma bronchiale; Bronchitis unterschiedlicher Genese; alle Formen der restriktiven Lungenerkrankungen, vor allem allergische Alveolitis; alle Formen des Lungenödems, vor allem toxisches Lungenödem; Sarkoidosen und Granulomatosen, insbesondere Morbus Boeck
- Rheumatische Erkrankungen/Autoimmunerkrankungen/Gelenkerkrankungen, die mit entzündlichen, allergischen und/oder proliferativen Prozessen einhergehen: alle Formen rheumatischer Erkrankungen, insbesondere rheumatoide Arthritis, akutes rheumatisches Fieber, Polymyalgia rheumatica; reaktive Arthritis; entzündliche Weichteilerkrankungen sonstiger Genese; arthritische Symptome bei degenerativen Gelenkerkankungen (Arthrosen); traumatische Arthritiden; Kollagenosen jeglicher Genese, z.B. systemischer Lupus erythematodes, Sklerodermie, Polymyositis, Dermatomyositis, Sjögren-Syndrom, Still-Syndrom, Felty-Syndrom
- Allergien, die mit entzündlichen und/oder proliferativen Prozessen einhergehen: alle Formen allergischer Reaktionen, z.B. Quincke Ödem, Heuschnupfen, Insektenstich, allergische Reaktionen auf Arzneimittel, Blutderivate, Kontrastmittel etc., anaphylaktischer Schock, Urtikaria, Kontaktdermatitis
- Gefäßentzündugen (Vaskulitiden): Panarterilitis nodosa, Arterilitis temporalis, Erythema nodosum
- Dermatologische Erkrankungen, die mit entzündlichen, allergischen und/oder proliferativen Prozessen einhergehen: atopische Dermatitis; Psoriasis; Pityriasis rubra pilaris; erythematöse Erkrankungen, ausgelöst durch unterschiedliche Noxen, z.B. Strahlen, Chemikalien, Verbrennungen etc.; bullöse Dermatosen; Erkrankungen des lichenoiden Formenkreises; Pruritus; Seborrhoisches Ekzem; Rosacea; Pemphigus vulgaris; Erythema exsudativum multiforme; Balanitis; Vulvitis; Haarausfall wie Alopecia areata; kutane T-Zell Lymphome
- Nierenerkrankungen, die mit entzündlichen, allergischen und/oder proliferativen Prozessen einhergehen: nephrotisches Syndrom; alle Nephritiden
- Lebererkrankungen, die mit entzündlichen, allergischen und/oder proliferativen Prozessen einhergehen: akuter Leberzellzerfall; akute Hepatitis unterschiedlicher Genese, z.B. viral, toxisch, arneimittelinduziert; chronisch aggressive und/oder chronisch intermittierende Hepatitis
- Gastrointestinale Erkrankungen, die mit entzündlichen, allergischen und/oder proliferativen Prozessen einhergehen: regionale Enteritis (Morbus Crohn); Colitis ulcerosa; Gastritis; Refluxoesophagitis; Gastroenteritiden anderer Genese, z.B. einheimische Sprue
- Proktologische Erkrankungen, die mit entzündlichen, allergischen und/oder proliferativen Prozessen einhergehen: Analekzem; Fissuren; Hämorrhoiden; idiopatische Proktitis
- Augenerkrankungen, die mit entzündlichen, allergischen und/oder proliferativen Prozessen einhergehen: allergische Keratitis, Uveitis, Iritis; Konjuktivitis; Blepharitis; Neuritis nervi optici; Chlorioditis; Opthalmia sympathica
- Erkrankungen des Hals-Nasen-Ohren-Bereiches, die mit entzündlichen, allergischen und/oder proliferativen Prozessen einhergehen: allergische Rhinitis, Heuschnupfen; Otitis externa, z.B. bedingt durch Kontaktexem, Infektion etc.; Otitis media
- Neurologische Erkrankungen, die mit entzündlichen, allergischen und/oder proliferativen Prozessen einhergehen: Hirnödem, vor allem Tumor-bedingtes Hirnödem; Multiple Sklerose; akute Encephalomyelitis; Meningitis; verschiedene Formen von Krampfanfällen, z.B. BNS-Krämpfe
- Bluterkrankungen, die mit entzündlichen, allergischen und/oder proliferativen Prozessen einhergehen: erworbene hämolytische Anämie; idiopathische Thrombozytopenie
- Tumorerkrankungen, die mit entzündlichen, allergischen und/oder proliferativen Prozessen einhergehen: akute lymphatische Leukämie; maligne Lymphome; Lymphogranulomatosen; Lymphosarkome; ausgedehnte Metastasierungen, vor allem bei Mamma-, Bronchial- und Prostatakarzinom
- Endokrine Erkrankungen, die mit entzündlichen, allergischen und/oder proliferativen Prozessen einhergehen: endokrine Orbitopathie; thyreotoxische Krise; Thyreoditis de Quervain; Hashimoto Thyreoditis; Morbus Basedow
- Organ- und Gewebstransplantationen, Graft-versus-Host disease
- Schwere Schockzuständen, z.B. anaphylaktischer Schock, systemic inflammatory response syndrome (SIRS)
- Substitutionstherapie bei: angeborene primäre Nebenniereninsuffizienz, z.B. kongenitales adrenogenitales Syndrom; erworbene primäre Nebenniereninsuffizienz, z.B. Morbus Addison, autoimmune Adrenalitis, postinfektiös, Tumoren, Metastasen, etc; angeborne sekundäre Nebenniereninsuffizienz, z.B. kongenitaler Hypopituitarismus; erworbene sekundäre Nebenniereninsuffizenz, z.B. postinfektiös, Tumoren, etc
- Emesis, die mit entzündlichen, allergischen und/oder proliferativen Prozessen einhergehen, z.B. in Kombination mit einem 5-HT3-Antagonisten bei Zytostatikabedingten Erbrechen
- Schmerzen bei entzündlicher Genese, z.B. Lumbago

Die erfindungsgemäßen Verbindungen eignen sich auch für die Behandlung von viralen Erkrankungen, wie zum Beispiel Infektionen die verursacht sind durch Papilloma-Viren, Herpes-Viren, Epstein-Barr-Viren, Hepatitis B- oder C-Viren, und humane Immunschwäche-Viren. Die erfindungsgemäßen Verbindungen eignen sich auch für die Behandlung von Atherosklerose, Dyslipidemie, Hypercholesterolemie, Hypertriglyceridämie, perifere Gefäßerkrankungen, kardiovaskuläre Erkrankungen, Angina, pectoris, Ischemie, Schlaganfall, Myokardinfarkt, angioplastische Restenose, Bluthochdruck, Thrombose, Adipositas, Endotoxemie.

Die erfindungsgemäßen Verbindungen eignen sich auch für die Behandlung von neurodegenerativen Krankheiten wie zum Beispiel multiple Sklerose, Alzheimer's Krankheit und Parkinson's Krankheit.

Diese Erkrankungen sind gut charakterisiert im Menschen, existieren aber auch bei anderen Säugetieren.

Ein weiterer Gegenstand der vorliegenden Anmeldung sind die erfindungsgemäßen Verbindungen zur Verwendung als Arzneimittel, insbesondere zur Prophylaxe und/oder Therapie von Tumorerkrankungen.

Ein weiterer Gegenstand der vorliegenden Anmeldung sind die erfindungsgemäßen Verbindungen zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.

Ein weiterer Gegenstand der vorliegenden Anmeldung sind die erfindungsgemäßen Verbindungen zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Ein weiterer Gegenstand der Erfindung ist die Verwendung der erfindungsgemäßen Verbindungen zur Herstellung eines Arzneimittels.

Ein weiterer Gegenstand der vorliegenden Anmeldung ist die Verwendung der erfindungsgemäßen Verbindungen zur Herstellung eines Arzneimittels zur Prophylaxe und/oder Therapie von Tumorerkrankungen.
Ein weiterer Gegenstand der vorliegenden Anmeldung ist die Verwendung der erfindungsgemäßen Verbindungen zur Herstellung eines Arzneimittels zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.
Ein weiterer Gegenstand der vorliegenden Anmeldung ist die Verwendung der erfindungsgemäßen Verbindungen zur Herstellung eines Arzneimittels zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Auch beschrieben ist die Verwendung der erfindungsgemäßen Verbindungen zur Prophylaxe und/oder Therapie von Tumorerkrankungen.

Auch beschrieben ist die Verwendung der erfindungsgemäßen Verbindungen zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptornegativen, Hormonrezeptor-positiven oder BRCA-assozüerten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.
auch beschrieben ist die Verwendung der erfindungsgemäßen Verbindungen zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Ein weiterer Gegenstand der vorliegenden Anmeldung sind pharmazeutische Formulierungen in Form von Tabletten enthaltend eine der erfindungsgemäßen Verbindungen zur Prophylaxe und/oder Therapie von Leukämien, insbesondere akuten myeolischen Leukämien, Prostatakarzinomen, insbesondere Androgenrezeptor-positiven Prostatakarzinomen, Zervixkarzinomen, Mammakarzinomen, insbesondere von Hormonrezeptor-negativen, Hormonrezeptor-positiven oder BRCA-assoziierten Mammakarzinomen, Pankreaskarzinomen, Nierenzellkarzinomen, Hepatozellulären Karzinomen, Melanomen und anderen Hauttumoren, Nicht-Kleinzelligen Bronchialkarzinomen, Endometriumkarzinomen und Kolorektalen Karzinomen.
Ein weiterer Gegenstand der vorliegenden Anmeldung sind pharmazeutische Formulierungen in Form von Tabletten enthaltend eine der erfindungsgemäßen Verbindungen zur Prophylaxe und/oder Therapie von Leukämien, insbesondere akuten myeloischen Leukämien, Prostatakarzinomen, insbesondere Androgenrezeptor-positiven Prostatakarzinomen, Mammakarzinomen, insbesondere Estrogenrezeptor-alpha negativen Mammakarzinomen, Melanomen oder Multiplen Myelomen.

Auch beschrieben ist die Verwendung der erfindungsgemäßen Verbindungen zur Behandlung von Erkrankungen, die mit proliferativen Prozessen einhergehen.

Auch beschrieben ist die Verwendung der erfindungsgemäßen Verbindungen zur Behandlung von benignen Hyperplasien, inflammatorischen Erkrankungen, autoimmunen Erkrankungen, Sepsis, viralen Infektionen, Gefäßerkrankungen und neurodegenerativen Erkrankungen.
Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend eine erfindungsgemäße Verbindung und einen oder mehrere weitere Wirkstoffe, insbesondere zur Prophylaxe und/oder Therapie der zuvor genannten Erkrankungen.
Beispielsweise können die erfindungsgemäßen Verbindungen mit bekannten anti-hyperproliferativen, zytostatischen oder zytotoxischen Substanzen zur Behandlung von Krebserkrankungen kombiniert werden. Die Kombination der erfindungsgemäßen Verbindungen mit anderen für die Krebstherapie gebräuchlichen Substanzen oder auch mit der Strahlentherapie ist besonders angezeigt.

Als geeignete Kombinationswirkstoffe seien beispielhaft genannt, ohne dass diese Aufzählung abschliessend wäre:
Abiraterone acetate, Abraxane, Acolbifen, Actimmun, Actinomycin D (Dactinomycin), Afatinib, Affinitak,Afinitor, Aldesleukin, Alendronsäure, Alfaferon, Alitretinoin, Allopurinol, Aloprim, Aloxi, Alpharadin, Altretamin, Aminoglutethimid, Aminopterin, Amifostin, Amrubicin, Amsacrin, Anastrozol, Anzmet, Apatinib, Aranesp, Arglabin, Arsen-trioxid, Aromasin, Arzoxifen, Asoprisnil, L-Asparaginase, Atamestan, Atrasentan, Avastin, Axitinib, 5-Azacytidin, Azathioprin, BCG oder tice-BCG, Bendamustin, Bestatin, Beta-methason-Acetat, Betamethason-Natriumphosphat, Bexaroten, Bicalutamid, Bleomycin-Sulfat, Broxuridin, Bortezomib, Bosutinib, Busulfan, Cabazitaxel, Calcitonin, Campath, Camptothecin, Capecitabin, Carboplatin, Carfilzomib, Carmustin, Casodex,CCI-779, CDC-501, Cediranib, Cefeson, Celebrex, Celmoleukin, Cerubidin, Cediranib, Chlorambucil, Cisplatin, Cladribin, Clodronsäure, Clofarabin, Colaspase, Corixa, Crisnatol, Crizotinib, Cyclophosphamid, Cyproterone-Acetat, Cytarabin, Dacarbazin, Dactinomycin, Dasatinib, Daunorubicin, DaunoXome, Decadron, Decadron-Phosphat, Decitabin, Degarelix, Delestrogen, Denileukin Diftitox, Depomedrol, Deslorelin, Dexrazoxan, Diethylstilbestrol, Diflucan, 2',2'-Difluordeoxycytidin, DN-101, Docetaxel, Doxifluridin, Doxorubicin (Adriamycin), Dronabinol, dSLIM, Dutasterid, DW-166HC, Edotecarin, Eflornithin, Eligard, Elitek, Ellence, Emend, Enzalutamide, Epirubicin, Epoetin-alfa, Epogen, Epothilon und seine Derivate, Eptaplatin, Ergamisol, Erlotinib, Erythro-Hydroxynonyladenin, Estrace, Estradiol, Estramustin-Natriumphosphat, Ethinylestradiol, Ethyol, Etidronsäure, Etopophos, Etoposid, Everolimus, Exatecan, Exemestan, Fadrozol, Farston, Fenretinid, Filgrastim, Finasterid, Fligrastim, Floxuridin, Fluconazol, Fludarabin, 5-Fluordeoxyuridin-Monophosphat, 5-Fluoruracil (5-FU), Fluoxymesteron, Flutamid, Folotin, Formestan, Fosteabin, Fotemustin, Fulvestrant, Gammagard, Gefitinib, Gemcitabin, Gemtuzumab, Gleevec, Gliadel, Goserelin, Gossypol, Granisetron-Hydrochlorid, Hexamethylmelamin, Histamin-Dihydrochlorid, Histrelin, Holmium-166-DOTPM, Hycamtin, Hydrocorton, erythro-Hydroxynonyladenin, Hydroxyharnstoff, Hydroxyprogesteronecaproat, Ibandronsäure, Ibritumomab Tiuxetan, Idarubicin, Ifosfamid, Imatinib, Iniparib, Interferon-alpha, Interferon-alpha-2, Interferon-alpha-2α, Interferon-alpha-2β, Interferon-alpha-n1, Interferon-alpha-n3, Interferon-beta, Interferon-gamma-1α, Interleukin-2, Intron A, Iressa, Irinotecan, Ixabepilon, Keyhole limpet Hemocyanin, Kytril, Lanreotid, Lapatinib, Lasofoxifen, Lentinan-Sulfat, Lestaurtinib, Letrozol, Leucovorin, Leuprolid, Leuprolid-Acetat, Levamisol, Levofolinsäure-Calciumsalz, Levothroid, Levoxyl, Libra, liposomales MTP-PE, Lomustin, Lonafarnib, Lonidamin, Marinol, Mechlorethamin, Mecobalamin, Medroxyprogesteron-Acetat, Megestrol-Acetat, Melphalan, Menest, 6-Mercaptopurin, Mesna, Methotrexat, Metvix, Miltefosin, Minocyclin, Minodronat, Miproxifen, Mitomycin C, Mitotan, Mitoxantron, Modrenal, MS-209, MX-6, Myocet, Nafarelin, Nedaplatin, Nelarabine, Nemorubicin, Neovastat, Neratinib, Neulasta, Neumega, Neupogen, Nilotimib, Nilutamid, Nimustin, Nolatrexed, Nolvadex, NSC-631570, Obatoclax, Oblimersen, OCT-43, Octreotid, Olaparib, Ondansetron-Hydrochlorid, Onko-TCS, Orapred, Osidem, Oxaliplatin, Paclitaxel, Pamidronat-Dinatrium, Pazopanib, Pediapred, Pegaspargase, Pegasys, Pemetrexed, Pentostatin, N-Phosphono-acetyl-L-Aspartat, Picibanil, Pilocarpin-Hydrochlorid, Pirarubicin, Plerixafor, Plicamycin, PN-401, Porfimer-Natrium, Prednimustin, Prednisolon, Prednison, Premarin, Procarbazin, Procrit, QS-21, Quazepam, R-1589, Raloxifene, Raltitrexed, Ranpirnas, RDEA119, Rebif, Regorafenib, 13-cis-Retinsäure, Rhenium-186-Etidronat, Rituximab, Roferon-A, Romidepsin, Romurtid, Ruxolitinib, Salagen, Salinomycin, Sandostatin, Sargramostim, Satraplatin, Semaxatinib, Semustin, Seocalcitol, Sipuleucel-T, Sizofiran, Sobuzoxan, Solu-Medrol, Sorafenib, Streptozocin, Strontium-89-chlorid, Sunitinib, Synthroid, T-138067, Tamoxifen, Tamsulosin, Tarceva, Tasonermin, Tastolacton, Taxoprexin, Taxoter, Teceleukin, Temozolomid, Temsirolimus, Teniposid, Testosteron-Propionat, Testred, Thalidomid, Thymosin-alpha-1, Thioguanin, Thiotepa, Thyrotropin, Tiazorufin, Tiludronsäure, Tipifarnib, Tirapazamin, TLK-286, Toceranib, Topotecan, Toremifen, Tositumomab, Tastuzumab, Teosulfan, TransMID-107R, Tretinoin, Trexall, Trimethylmelamin, Trimetrexat, Triptorelin-Acetat, Triptorelin-Pamoat, Trofosfamid, UFT, Uridin, Valrubicin, Valspodar, Vandetanib, Vapreotid, Vatalanib, Vemurafinib, Verte-porfin, Vesnarinon, Vinblastin, Vincristin, Vindesin, Vinflumin, Vinorelbin, Virulizin, Vismodegib, Xeloda, Z-100, Zinecard, Zinostatin-Stimalamer, Zofran, Zoledronsäure

Auch die Kombination der erfindungsgemässen Verbindung mit einem P-TEFb oder CDK9 Inhibitor ist besonders angezeigt.

In viel versprechender Weise lassen sich die erfindungsgemäßen Verbindungen auch mit biologischen Therapeutika wie Antikörpern (z.B. Aflibercept, Alemtuzumab, Bevacizumab, Brentuximumab, Catumaxomab, Cetuximab, Denosumab, Edrecolomab, Gemtuzumab, Ibritumomab, Ipilimumab, Ofatumumab, Panitumumab, Pertuzumab, Rituximab, Tositumumab, Trastuzumab) und rekombinanten Proteinen kombinieren.

Die erfindungsgemäßen Verbindungen können auch in Kombination mit anderen, gegen die Angiogenese gerichteten Therapien positive Effekte erzielen, wie zum Beispiel mit Bevacizumab, Axitinib, Regorafenib, Cediranib, Sorafenib, Sunitinib oder Thalidomid. Kombinationen mit Antihormonen und steroidalen metabolischen Enzyminhibitoren sind wegen ihres günstigen Nebenwirkungsprofils besonders geeignet.

Generell können mit der Kombination der erfindungsgemäßen Verbindungen mit anderen, zytostatisch oder zytotoxisch wirksamen Agentien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Verbindungen auch in Verbindung mit einer Strahlentherapie und/oder einer chirurgischen Intervention eingesetzt werden.

### Syntheserouten zur Herstellung der Verbindungen der allgemeinen Formel (I)

Die folgenden Schemata und allgemeinen Arbeitsvorschriften veranschaulichen den allgemeinen synthetischen Zugang zu den erfindungsgemäßen Verbindungen der Formel (I), ohne dass die Synthese der erfindungsgemäßen Verbindungen als auf diese beschränkt angesehen sein soll. 4,5-Dihydro-*3H*-2,3-benzodiazepine der allgemeinen Formel (I) lassen sich analog zu in der Literatur beschriebenen Verfahren herstellen. In Abhängigkeit von den vorhandenen Substituenten können gegebenenfalls Schutzgruppenstrategien erforderlich sein, die jedoch dem Fachmann allgemein bekannt sind.
In Schema 1 ist die Synthese von 4,5-Dihydro-3*H*-2,3-benzodiazepinen unter Verwendung einer 3,4-Dihydro-1*H*-2-benzopyran-Zwischenstufe (III) beschrieben, wobei A, n und die Reste R^{1a}, R^{1b}, R², R⁴ und R⁵ die in der allgemeinen Formel (I) angegebenen Bedeutungen aufweisen, wobei (R^{1b})ₙ auch gleichbedeutend stehen kann für R^{1b} und R^{1c}. Entsprechende Herangehensweisen sind beispielsweise bei F. Gatta et al. Il Farmaco - Ed. Sc. 1985, 40, 942 bzw. in WO2008124075 oder WO200198280 beschrieben.

Die verwendeten Aldehyde sind kommerziell erhältlich bzw. ihre Herstellung ist dem Fachmann bekannt. R^{1a} und R^{1b} können auch auf einer späteren Stufe der Synthese eingeführt werden, beispielsweise wie in den Schemata 5a, 6, 7, 8, 9 und 11 beschrieben.
Die verwendeten 1-Aryl-2-propanole (II) sind entweder kommerziell erhältlich oder werden in dem Fachmann allgemein bekannter Weise durch Reduktion der entsprechenden Ketone (IIa), z.B. durch Reduktion mit Lithiumaluminiumhydrid in THF, hergestellt.
Dieser Syntheseweg wird bevorzugt bei elektronenreich substituierten Arylpropanolen (II) (z.B. mit Alkoxy) beschritten.

3,4-Dihydro-1*H*-2-benzopyrane (III) werden durch Kondensation der 1-Aryl-2-propanole (II) mit aromatischen oder heteroaromatischen Aldeyden unter sauren Bedingungen erhalten. Die Umsetzung erfolgt bevorzugt bei erhöhter Temperatur (ca. 100°C) in salzsaurem Dioxan in Anwesenheit von wasserfreiem Zinkchlorid. Die weitere Umsetzung der 3,4-Dihydro-1*H-*2-benzopyrane (III) kann auf verschiedenen Wegen erfolgen:
Oxidative Ringöffnung mittels Chrom(VI)oxid/Schwefelsäure liefert das Diketon (IV), welches mit Hydrazin zum 4-Methyl-1-aryl-5*H*-2,3-benzodiazepin oder 4-Methyl-1-heteroaryl-5*H*-2,3-benzodiazepin (V) zyklisiert werden kann (vgl. US5288863). Reduktion mit z.B. Natriumcyanoborhydrid (Synthetic Communications, 2002, 32, 527) liefert dann das gewünschte 4,5-Dihydro-3*H*-2,3-benzodiazepinderivat (VI).
Oxidation mit Luftsauerstoff liefert das 1-Aryl-3,4-dihydro-1*H*-2-benzopyran-1-ol oder 1-Heteroaryl-3,4-dihydro-1*H*-2-benzopyran-1-ol (VII), das unter Wasserabspaltung mit H₂NNHBoc zum entsprechenden Hydrazonderivat (VIII) umgesetzt werden kann. Dieses kann durch Mesylierung und nachfolgende Behandlung mit Base zum Boc-geschützen 4,5-Dihydro-3*H*-2,3-benzodiazepinderivat (IX) zyklisiert werden, das wiederum durch saure Entschützung in allgemein bekannter Weise in das entsprechende 4,5-Dihydro-3*H*-2,3-benzodiazepinderivat (VI) überführt werden kann. 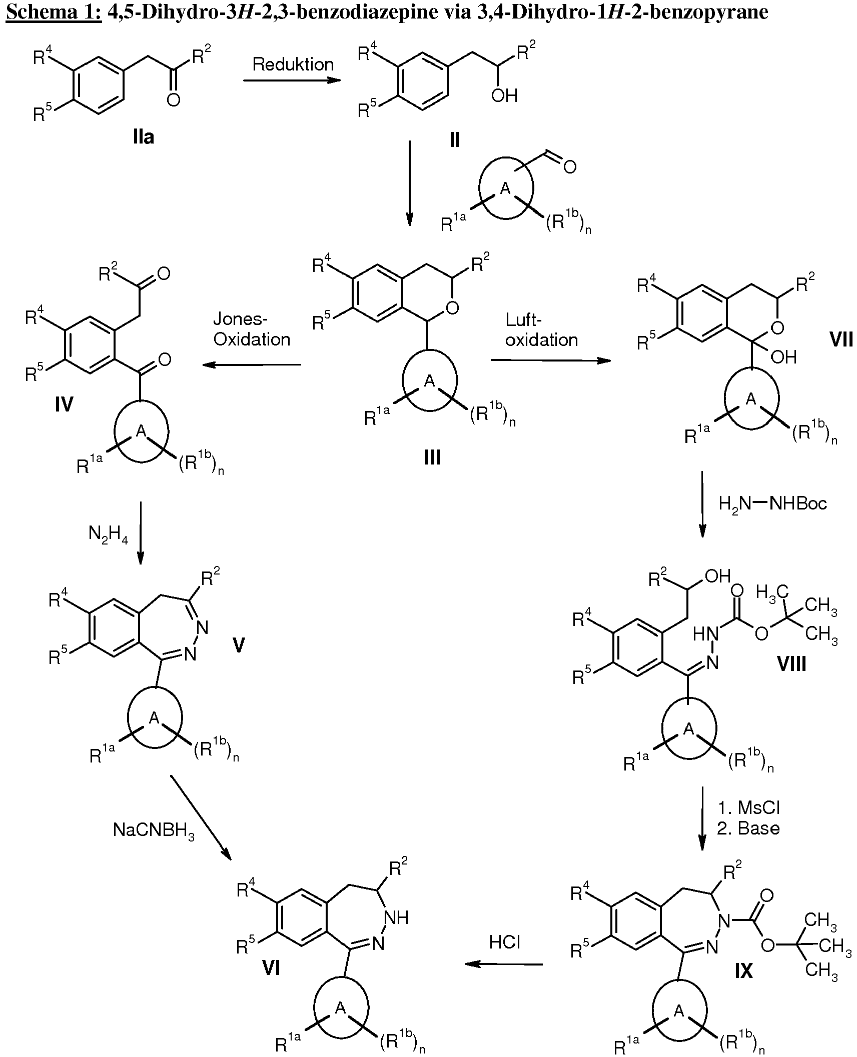
In Schema 2 ist die Synthese von 4,5-Dihydro-3*H*-2,3-benzodiazepinen aus Indanonen (X) beschrieben.

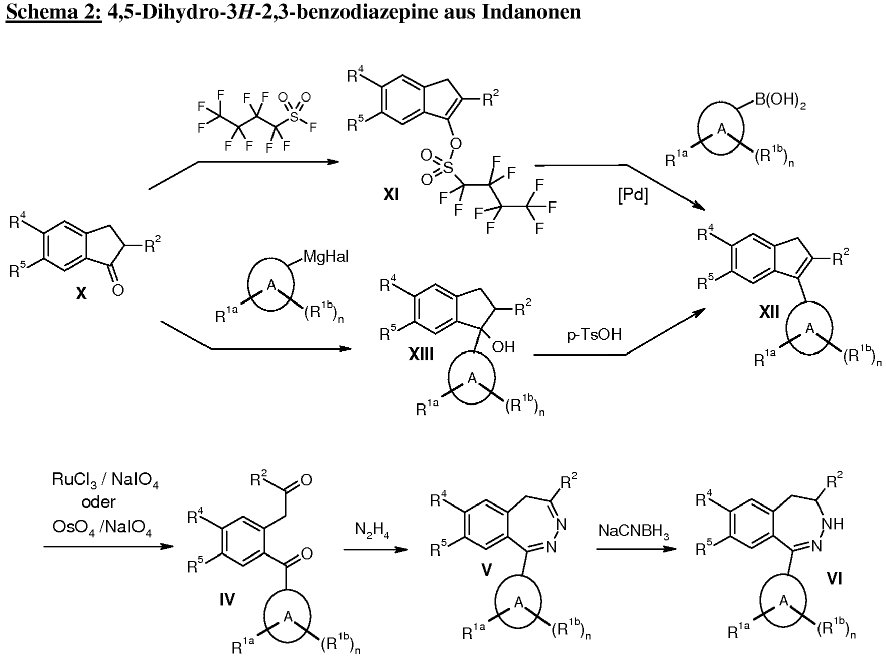

A, n, sowie die Reste R^{1a}, R^{1b}, R², R⁴ und R⁵ in Schema 2 weisen die in der allgemeinen Formel (I) angegebenen Bedeutungen auf, wobei (R^{1b})ₙ auch gleichbedeutend stehen kann für R^{1b} und R^{1c}. Das Indanon (X) kann in das entsprechende 3-Aryl-1*H*-inden oder 3-Heteroaryl-1*H*-inden (XII) überführt werden. Dazu können folgende Verfahren Verwendung finden:
- Das Indanonderivat (X) kann z.B. in allgemein bekannter Weise in das entsprechende Enol-Nonaflat (XI) überführt, und anschließend durch eine palladiumkatalysierte Suzuki-Kupplung mit den entsprechenden Boronsäurederivaten zum Inden (XII) umgesetzt werden.
- Das Indanonderivat (X) kann durch Addition von magnesiumorganischen Reagenzien in allgemein bekannter Weise in die entsprechenden Indanole (XIII) überführt werden, welche leicht durch säurekatalysierte Eliminierung die entsprechenden Indene (XII) bilden.
Die 3-Aryl-1*H*-indene bzw. 3-Heteroaryl-1*H*-indene (XII) lassen sich durch oxidative Methoden, z.B. mit Ruthenium(III)chlorid/Natriumperiodat (Bioorganic and Medicinal Chemistry Letters, 2011, 21, 2554) in die entsprechenden Diketone (IV) überführen. Diese können analog Schema 1 zu den entsprechenden 4,5-Dihydro-3*H*-2,3-benzodiazepinderivaten (VI) umgesetzt werden.

Die für die Herstellung der Ausführungsbeispiele verwendeten Indanone (X) sind entweder kommerziell erhältlich oder können z.B. wie in Schema 3 gezeigt hergestellt werden, wobei die Reste R², R⁴ und R⁵ die in der allgemeinen Formel (I) angegebenen Bedeutungen aufweisen.

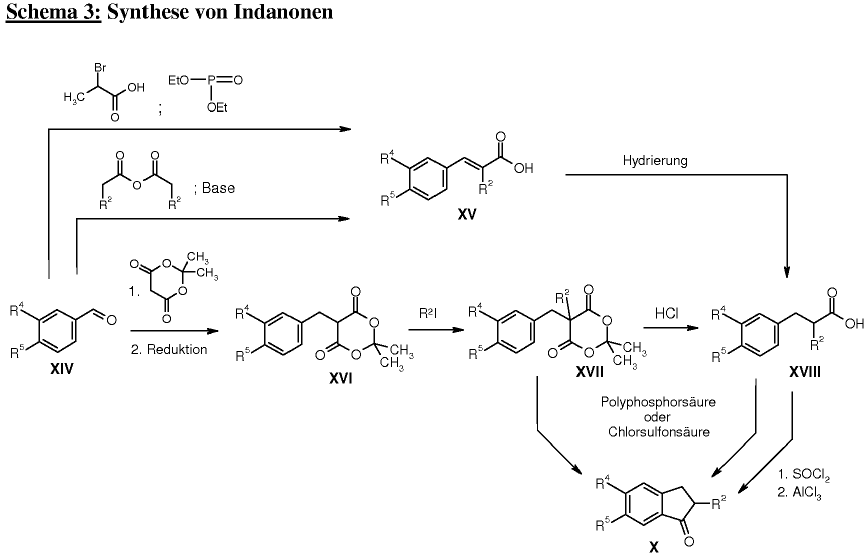

Aus den entsprechenden aromatischen Aldehyden (XIV) können durch literaturbekannte Verfahren z.B. via Perkin-Reaktion (Medicinal Chemistry Research, 2004, Vol. 13, 660) oder Wittig-Reaktion (Journal of Organic Chemistry, 2001, Vol. 66, 3682) die 2-Methyl-3-arylpropansäuren (XVIII) hergestellt werden. Diese können z.B. mit Chlorsulfonsäure oder Polyphosphorsäure zu den entsprechenden Indanonen (X) zyklisiert werden (vgl. Synthesis 2009, 627 und Org. Process Res. Dev. 2011, 15, 570-580, J. Org. Chem. 2005, 70, 1316 und Bioorg. Med. Chem. Lett. 2011, 21, 2554-2558).
Schema 4 veranschaulicht die Herstellung der erfindungsgemäßen Beispielverbindungen, ausgehend von 4,5-Dihydro-3*H*-2,3-benzodiazepinen (VI) mittels allgemein bekannter Umsetzungen z.B. mit Säurechloriden, Anhydriden, Chloroformiaten oder Isocyanaten bzw, Isothiocyanaten, wobei A, n und die Reste R^{1a}, R^{1b}, R², R³, R⁴ und R⁵ die in der allgemeinen Formel (I) angegebenen Bedeutungen aufweisen, wobei (R^{1b})ₙ auch gleichbedeutend stehen kann für R^{1b} und R^{1c}. Die entsprechenden Alkylharnstoffe (XIX) können auch durch Umsetzung einer reaktiven Zwischenstufe, wie z.B. dem 4-Nitrophenylcarbamat, mit Alkylaminen erhalten werden. 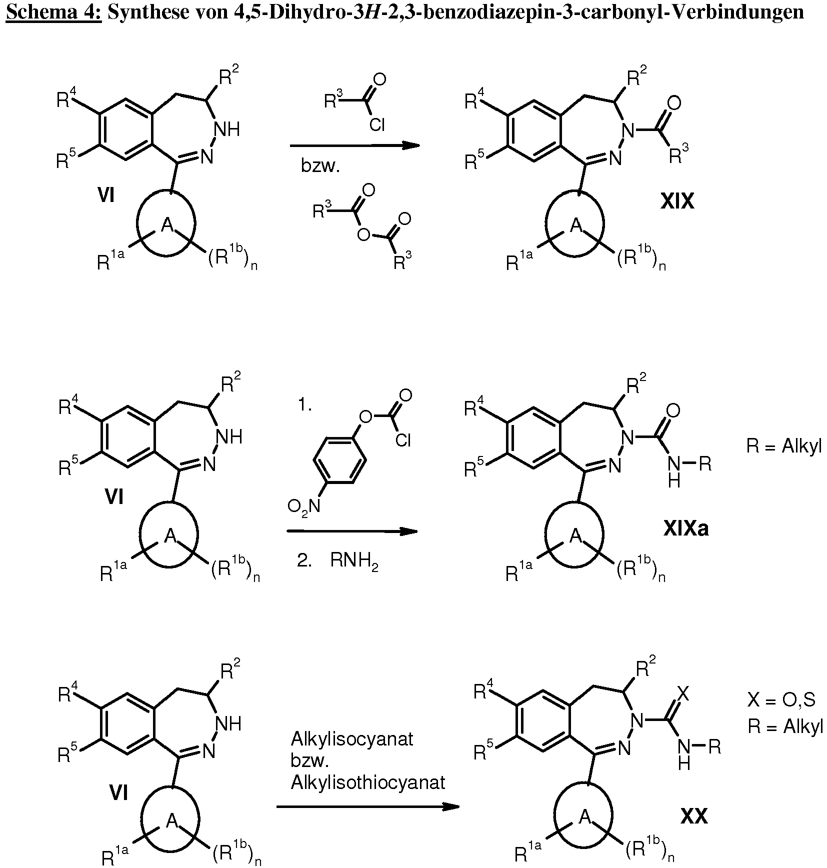 R^{1a}, R⁴ und R⁵ können auch auf einer späteren Stufe der Synthese eingeführt werden, beispielsweise wie in den Schemata 5a, 5b, 5c, 6, 8, 9 und 11 beschrieben.

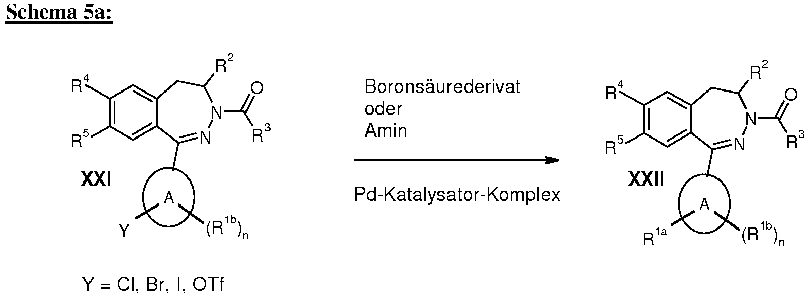

Die Reste R^{1b}, R², R³, R⁴, R⁵, sowie A und n in Schema 5a weisen die in der allgemeinen Formel (I) angegebenen Bedeutungen auf, wobei (R^{1b})ₙ auch gleichbedeutend stehen kann für R^{1b} und R^{1c}. In Schema 5a steht R^{1a} beispielsweise für einen Phenylrest oder einen monocyclischen Heteroarylrest mit 5 oder 6 Ringatomen wie in der allgemeinen Formel (I) für R^{1a} definiert, oder für einen Aminrest, insbesondere für einen monozyklischen Heterocyclylrest mit 3 bis 8 Ringatomen, wie in der allgemeinen Formel (I) für R^{1a} definiert, mit der Maßgabe, dass dieser über ein im Heterocyclus befindliches Stickstoffatom an den Rest des Moleküls gebunden ist.

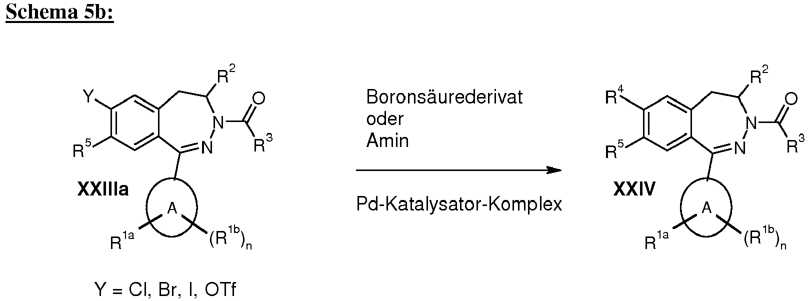

Die Reste R^{1a}, R^{1b}, R², R³, R⁵, sowie A und n in Schema 5b weisen die in der allgemeinen Formel (I) angegebenen Bedeutungen auf, wobei (R^{1b})ₙ auch gleichbedeutend stehen kann für R^{1b} und R^{1c}. In Schema 5b steht R⁴ beispielsweise für einen Phenylrest oder einen monocyclischen Heteroarylrest mit 5 oder 6 Ringatomen wie in der allgemeinen Formel (I) für R⁴ definiert, oder für einen Aminrest, insbesondere für einen monozyklischen Heterocyclylrest mit 3 bis 8 Ringatomen, wie in der allgemeinen Formel (I) für R⁴ definiert, mit der Maßgabe, dass dieser über ein im Heterocyclus befindliches Stickstoffatom an den Rest des Moleküls gebunden ist.

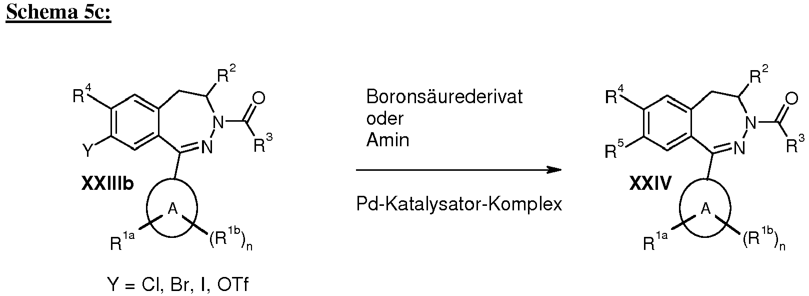

Die Reste R^{1a}, R^{1b}, R², R³, R⁴, sowie A und n in Schema 5c weisen die in der allgemeinen Formel (I) angegebenen Bedeutungen auf, wobei (R^{1b})ₙ auch gleichbedeutend stehen kann für R^{1b} und R^{1c}. In Schema 5c steht R⁵ beispielsweise für einen Phenylrest oder einen monocyclischen Heteroarylrest mit 5 oder 6 Ringatomen wie in der allgemeinen Formel (I) für R⁵ definiert, oder für einen Aminrest, insbesondere für einen monozyklischen Heterocyclylrest mit 3 bis 8 Ringatomen, wie in der allgemeinen Formel (I) für R⁵ definiert, mit der Maßgabe, dass dieser über ein im Heterocyclus befindliches Stickstoffatom an den Rest des Moleküls gebunden ist.

Schemata 5a, 5b und 5c verdeutlichen die Herstellung von Ausführungsbeispielen, die sich durch dem Fachmann allgemein bekannte Palladium-katalysierte Kupplungsreaktionen, ausgehend z.B. von Brom-substituierten Aryl- oder Heteroarylderivaten (XXI, XXIIIa und XXIIIb) durch Umsetzung mit den entsprechenden Boronsäurederivaten (Chem. Rev. 1995, 95, 2457-2483; Angewandte Chemie, International Edition (2002), 41(22), 4176-4211) oder Aminen herstellen lassen. Die Intermediate XXI, XXIIIa und XXIIIb können analog der gezeigten Syntheserouten hergestellt werden.

Boronsäurederivate sind kommerziell verfügbar oder in allgemein bekannter Weise herstellbar. Die Herstellung der erfindungsgemäßen Beispielverbindungen durch Umsetzung mit Aminen erfolgt z.B. unter Buchwald-Hartwig Bedingungen (Journal of Organometallic Chemistry (1999), 576(1-2), 125-146).

Entsprechende Pyrazolderivate (XXVI) (vgl. US200419045) bzw. Morpholinderivate (XXVII) (vgl. US2006199804) lassen sich alternativ und beispielsweise für A = Phenyl auch ausgehend von Anilinderivaten nach literaturbekannten Methoden, wie in Schema 6 gezeigt, herstellen.

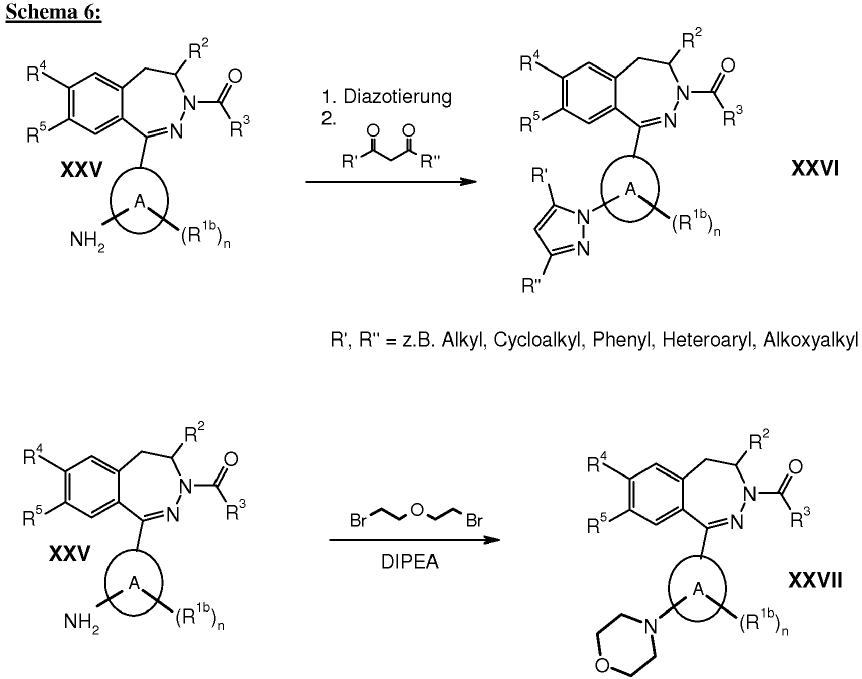

Die Reste R^{1b}, R², R³, R⁴, R⁵, sowie A und n in Schema 6 weisen die in der allgemeinen Formel (I) angegebenen Bedeutungen auf wobei (R^{1b})ₙ auch gleichbedeutend stehen kann für R^{1b} und R^{1c}. Schema 7 verdeutlicht die Synthese von Ausführungsbeispielen, die sich durch dem Fachmann allgemein bekannte Bromierungen von Verbindungen der Formel XXVIII herstellen lassen (Synth. Commun. 1993, 23, 855). In den genannten Verbindungen der Formel XXVIII weisen R², R³, R⁴, R⁵, n sowie A die in der allgemeinen Formel (I) angegebenen Bedeutungen auf, und Hetcyc steht für einen monozyklischen Heterocyclylrest mit 3 bis 8 Ringatomen, wie in der allgemeinen Formel (I) für R^{1a} definiert, mit der Maßgabe, dass dieser über ein im Heterocyclus befindliches Stickstoffatom an den Rest des Moleküls gebunden ist. Die resultierenden bromierten Ausführungsbeispiele der Formel XXIX lassen sich nach literaturbekannten Verfahren (Advanced Synthesis and Catalysis 2011, 777; Journal of the American Chemical Society 2003, 2890; US 2009/62541) in die entsprechenden Cyanoverbindungen der Formel XXX überführen. Die hier eingesetzten Intermediate der Formel XXVIII können mit den voranstehend beschriebenen Synthesemethoden hergestellt werden.

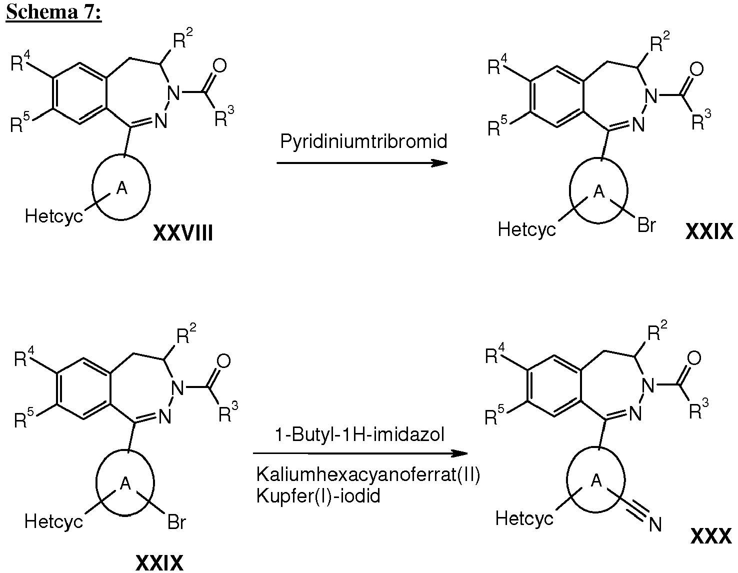

Schema 8 veranschaulicht die Herstellung von Ausführungsbeispielen, die sich, ausgehend von bromierten Zwischenprodukten der Formel XXIa, in denen R^{1b}, R², R³, R⁴, R⁵, A und n die in der allgemeinen Formel (I) angegebenen Bedeutungen aufweisen, wobei (R^{1b})ₙ auch gleichbedeutend stehen kann für R^{1b} und R^{1c}, mit dem Fachmann bekannten Methoden, beispielsweise durch Palladium-katalysierte Umsetzung mit Kohlendioxid (European Journal of Organic Chemistry 2000, 2253), zu Carbonsäurederivaten der Formel XXXI umsetzen lassen. Diese wiederum lassen sich anschließend in Analogie zu literaturbekannten Verfahren in die entsprechenden Oxadiazolderivate der Formeln XXXII und XXXIII überführen (Tetrahedron 2009, 65, 9989; Tetrahedron Letters 2006, 4827; Tetrahedron 2009, 9989). Die hier eingesetzten Intermediate der Formel XXIa können mit den voranstehend beschriebenen Synthesemethoden hergestellt werden.

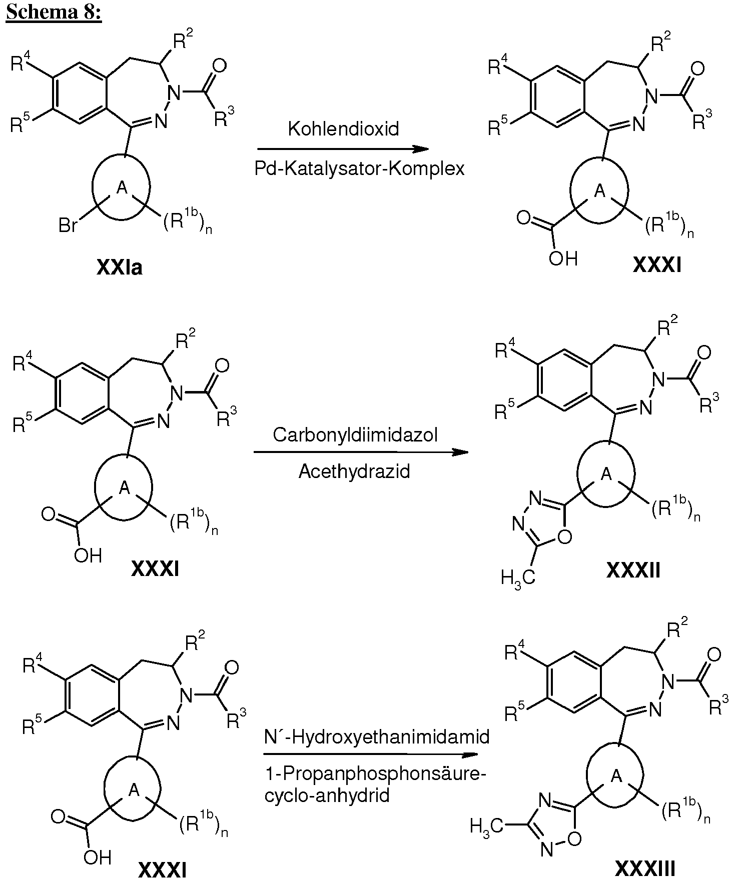

Schema 9 verdeutlicht die Herstellung von Ausführungsbeispielen, die sich durch dem Fachmann allgemein bekannte Kupfer-katalysierte Kupplungsreaktionen (siehe J. Am. Chem. Soc. 2001, 123, 7727; J. Am. Chem. Soc. 2002, 124, 7421), ausgehend z.B. von bromierten Zwischenprodukten der Formel XXIIIc, in der R^{1a}, R^{1b}, R², R³, R⁵, A sowie n die in der allgemeinen Formel (I) angegebenen Bedeutungen aufweisen, wobei (R^{1b})ₙ auch gleichbedeutend stehen kann für R^{1b} und R^{1c}, sowie Verbindungen der Formel XXIa, in der R^{1b}, R², R³, R⁴, R⁵, A sowie n die in der allgemeinen Formel (I) angegebenen Bedeutungen aufweisen, wobei (R^{1b})ₙ auch gleichbedeutend stehen kann für R^{1b} und R^{1c}, durch Umsetzung mit Amiden oder Carbamaten in die entsprechenden gekuppelten Derivate der Formeln XXXIV, in denen R⁴ für ein über ein Stickstoff-Atom gebundenes, gegebenenfalls cyclisches Carboxamid oder Carbamat steht, und XXXV, in denen wiederum R^{1a} für für ein über ein Stickstoff-Atom gebundenes, gegebenenfalls cyclisches Carboxamid oder Carbamat steht, überführen lassen. Die hier eingesetzten Intermediate der Formeln XXIIIc und XXIa können mit den voranstehend beschriebenen Synthesemethoden hergestellt werden.

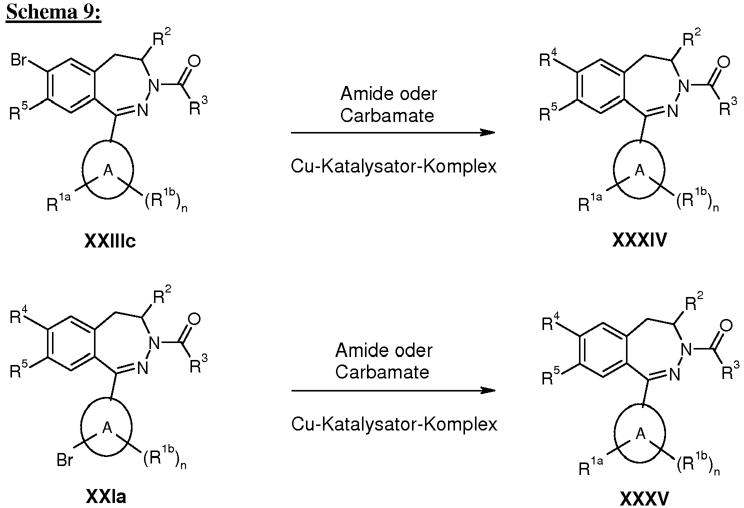

Schema 10 veranschaulicht die Herstellung von erfindungsgemäßen Beispielverbindungen, ausgehend von Dimethoxy-substituierten 4,5-Dihydro-3H-2,3-benzodiazepinen der Formel XXXVI, in denen R^{1a}, R^{1b}, R², R³, A und n die in der allgemeinen Formel (I) angegebenen Bedeutungen aufweisen, wobei (R^{1b})ₙ auch gleichbedeutend stehen kann für R^{1b} und R^{1c}, mittels allgemein bekannter Umsetzungen beispielsweise mit Bortribromid (Bioorganic and Medicinal Chemistry Letters 2012, 2827) zu den entsprechenden Dihydroxy-derivaten der Formel XXXVII, welche sich ihrerseits mit geeigneten Alkylierungsmitteln in die entsprechenden Etherderivate XXXVIII überführen lassen, in denen R⁴ und R⁵ stehen für gegebenenfalls substituierte AlkoxyReste (Journal of Medicinal Chemistry 2000, 3244). Die hier eingesetzten Intermediate der Formel XXXVI können mit den voranstehend beschriebenen Synthesemethoden hergestellt werden.

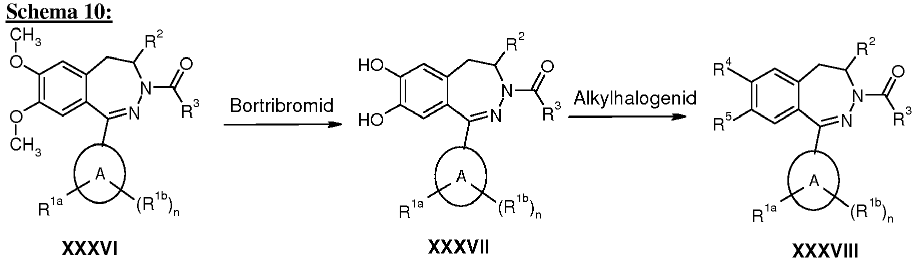

Wie in Schema 11 gezeigt, lassen sich ausgehend von bromierten Zwischenprodukten der Formel XXIa, in denen R^{1b}, R², R³, R⁴, R⁵, A und n die in der allgemeinen Formel (I) angegebenen Bedeutungen aufweisen, wobei (R^{1b})ₙ auch gleichbedeutend stehen kann für R^{1b} und R^{1c}, erfindungsgemäße Verbindungen der Formel XL durch Palladium-katalysierte Umsetzung von XXIa mit cyclischen Sulfonamiden mit freier NH-Gruppe der Formel XXXIX, in der p für eine Zahl 1, 2, 3 oder 4 steht, so dass der Cyclus 5 bis 8 Ringglieder hat, herstellen (Org. Lett. 2011, 2564).

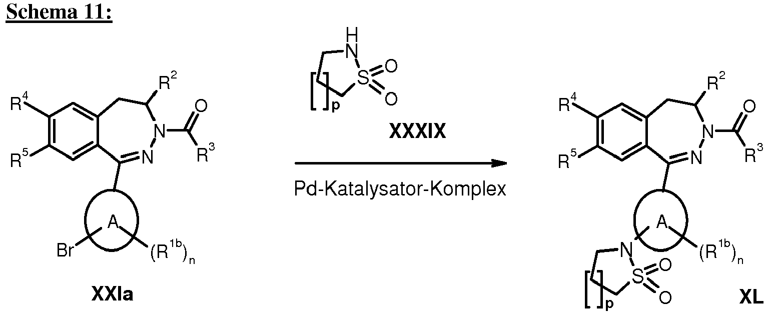

### Abkürzungen:

- abs.: absolut
- ACN: Acetonitril
- AS: Ameisensäure
- Boc: *tert-*Butoxycarbonyl
- CDCl₃: Deuterochloroform
- CO₂: Kohlendioxid
- d: Tag
- DEA: Diethylamin
- DIPEA: *N*,*N*-Diisopropylethylamin
- DMAP: 4-*N*,*N*-Dimethylaminopyridin
- DMF: Dimethylformamid
- DMSO: Dimethylsulfoxid
- d. Th.: der Theorie
- eq.: Äquivalent
- ESI: Elektrospray-Ionisation (bei MS)
- ges.: gesättigt
- h: Stunde
- HOBt: 1-Hydroxy-1*H*-benzotriazol x H₂O
- HPLC: Hochdruck-, Hochleistungsflüssigchromatographie
- IPA: 2-Propanol
- konz.: konzentriert
- LC-MS: Flüssigchromatographie-gekoppelte Massenspektrometrie
- min: Minuten
- MS: Massenspektrometrie
- Ms: Methansulfonyl
- MW: Molekulargewicht [g/mol]
- NMP: N-Methylpyrrolidon
- NMR: Kernresonanzspektroskopie
- R_{f}: Retentionsindex (bei DC)
- RP-HPLC: Reverse Phase HPLC
- RT: Raumtemperatur
- Rₜ: Retentionszeit (bei HPLC)
- SFC: supercritical fluid chromatography (überkritische Flüssigkeitschromatographie)
- TFA: Trifluoressigsäure
- THF: Tetrahydrofuran

Prozentuale Ausbeuteangaben (in % d. Th) sind gegebenenfalls reinheits-adjustiert.

### LC-MS Methoden:

Methode 1: Instrument: Waters Acquity LCT; Säule: Phenomenex Kinetex C18, 50 mm x 2.1 mm, 2,6µ; Eluent A: Wasser/0,05% AS, Eluent B: ACN/0,05% AS; Gradient: 0,0 min 98% A → 0,2 min: 98% A → 1,7 min: 10% A → 1,9 min: 10% A → 2 min: 98% A → 2,5 min: 98% A; Fluss: 1,3 ml/min; Säulentemperatur: 60°C; UV-Detektion: 200-400 nm.
Methode 2: Instrument: Waters Acquity Platform ZQ4000; Säule: Waters BEHC 18, 50 mm x 2,1 mm, 1,7µ; Eluent A: Wasser/0,05% AS, Eluent B: ACN/0,05% AS; Gradient: 0,0 min 98% A → 0,2 min: 98% A → 1,7 min: 10% A → 1,9 min: 10% A → 2 min: 98% A → 2,5 min: 98% A; Fluss: 1,3 ml/min; Säulentemperatur: 60°C; UV-Detektion: 200-400 nm.
Methode 3: UPLC-SQD-HCOOH; Instrument: Waters Acquity UPLC-MS SQD; Säule: Acquity UPLC BEH C18 1,7 50x2,1mm; Eluent A: Wasser + 0,1% Vol. Ameisensäure (99%), Eluent B: Acetonitril; Gradient: 0-1,6 min 1-99% B, 1,6-2,0 min 99% B; Fluss 0,8 ml/min; Temperatur: 60 °C; Injektion: 2 µl; DAD scan: 210-400 nm.
Methode 4: Instrument MS: Waters ZQ; Instrument HPLC: Waters Acquity; Säule: Acquity BEH C18 (Waters), 50 mm x 2,1mm, 1,7 µm; Eluent A: Wasser + 0,1% Ameisensäure, Eluent B: Acetonitril (Lichrosolv Merck); Gradient: 0,0 min 99% A - 1,6 min 1% A - 1,8 min 1% A - 1,81 min 99% A - 2,0 min 99% A; Ofen: 60 °C; Fluss 0,800 ml/min; UV-Detektion PDA 210-400 nm. (AMC Methode)
Methode 5: Instrument: Agilent 1290-Platform, ESI-TOF 6224; Säule: Waters BEH C18, 50 mm x 2,1 mm, 1,7µ; Eluent A: Wasser/0,05% AS, Eluent B: ACN/0,05% AS; Gradient: 0,0 min 98% A, bis 1,7 min linear auf 10% A, bis 2,0 min: 10% A, Fluss: 1,2 ml/min; Säulentemperatur: 60°C; UV-Detektion: 200-400 nm.

### Analytische HPLC Methoden:

Methode A: System: Waters Alliance 2695, DAD 996, ESA Corona; Säule: Chiralpak IC 3µm 100x4,6 mm; Eluent: Hexan / 2-Propanol 80:20 (v/v); Fluss: 1,0 ml/min; Säulentemperatur: 25°C; Detektion: DAD 254 nm.
Methode B: System: Waters Alliance 2695, DAD 996; Säule: Chiralpak ID 3µm 100x4,6 mm; Eluent Hexan / 2-Propanol 70:30 (v/v); Fluss: 1,0 ml/min; Säulentemperatur: 25°C; Detektion: DAD 280 nm.
Methode C: System: Waters Alliance 2695, DAD 996; Säule: Chiralpak IC 3µm 100x4,6 mm; Eluent: Hexan / Ethanol 70:30 (v/v); Fluss: 1,0 ml/min; Säulentemperatur: 25°C; Detektion: DAD 254 nm.
Methode Ca: System: Waters Alliance 2695, DAD 996, ESA: Corona; Säule: Chiralpak IC 3µm 100x4,6 mm; Eluent: Hexan / Ethanol 70:30 (v/v); Fluss: 1,0 ml/min; Säulentemperatur: 25°C; Detektion: DAD 280 nm.
Methode D: System: Agilent 1260 AS, MWD, Aurora SFC-Modul; Säule: Chiralpak IA 3µm 100x4,6 mm; Eluent: CO₂ / Ethanol + 0,2% Vol. Diethylamin 90/10; Fluss: 4,0 ml/min; Druck (outlet): 120 bar; Säulentemperatur: 37,5°C; Detektion: DAD 254 nm.
Methode Da: System: Agilent 1260 AS, MWD, Aurora SFC-Modul; Säule: Chiralpak IA 3µm 100x4,6 mm; Eluent: CO₂ / Methanol 97/3; Fluss: 4,0 ml/min; Druck (outlet): 100 bar; Säulentemperatur: 37,5°C; Detektion: DAD 254 nm.
Methode E: System: Waters Alliance 2695, DAD 996, ESA Corona; Säule: Chiralpak IC 3µm 100x4,6 mm; Eluent: Ethanol / Methanol 50:50 (v/v); Fluss: 1,0 ml/min; Säulentemperatur: 25°C; Detektion: DAD 254 nm.
Methode Ea: System: Waters Alliance 2695, DAD 996, ESA Corona; Säule: Chiralpak IC 3µm 100x4,6 mm; Eluent: Ethanol / Methanol/DEA 50:50:0.1 (v/v/v); Fluss: 1,0 ml/min; Säulentemperatur: 25°C; Detektion: DAD 254 nm.
Methode F: System: Agilent 1260 AS, MWD, Aurora SFC-Modul; Säule: Chiralpak IC 3µm 100x4,6 mm; Eluent: CO₂ / 2-Propanol + 0,2% Vol. Diethylamin 90/10; Fluss: 4,0 ml/min; Druck (outlet): 100 bar; Säulentemperatur: 37,5°C; Detektion: DAD 254 nm.
Methode Fa: System: Agilent 1260 AS, MWD, Aurora SFC-Modul; Säule: Chiralpak IC 5µm 100x4,6 mm; Eluent: CO₂ / Ethanol 6/4; Fluss: 4,0 ml/min; Druck (outlet): 100 bar; Säulentemperatur: 37,5°C; Detektion: DAD 254 nm.
Methode G: System: Waters Alliance 2695, DAD 996, ESA: Corona; Säule: Chiralpak ID 3µm 100x4,6 mm; Eluent: Hexan/2-Propanol 70:30 (v/v) +0,1% DEA; Fluss: 1,0 ml/min; Säulentemperatur: 25°C; Detektion: DAD 254 nm.
Methode H: System: Agilent: 1260 AS, MWD, Aurora SFC-Modul; Säule: Chiralpak IA 5µm 100x4.6 mm; Eluent: CO₂ / Methanol 9:1; Fluss: 4.0 mL/min; Druck (outlet): 100 bar; Säulentemperatur: 37,5°C; Detektion: DAD 254 nm.
Methode J: System: Waters: Alliance 2695, DAD 996, ESA: Corona; Säule: Chiralpak IC 3µm 100x4.6 mm; Eluent: Ethanol/Methanol/Diethylamin 50:50:0.1 (v/v/v); Fluss: 1,0 ml/min; Säulentemperatur: 25°C; Detektion: DAD 254 nm.
Methode L: System: Waters: Alliance 2695, DAD 996; Säule: Chiralpak ID-3 3µm 100x4.6 mm; Eluent: Hexan/IPA 70:30 (v/v) +0,1% DEA; Fluss: 1,0 ml/min; Säulentemperatur: 25°C; Detektion: DAD 280 nm.
Methode M: System: Waters: Alliance 2695, DAD 996, ESA: Corona; Säule: Chiralpak IC 3µm 100x4.6 mm; Eluent: Hexan/Ethanol/Diethylamin 80:20:0.1 (v/v/v); Fluss: 1,0 ml/min; Säulentemperatur: 25°C; Detektion: DAD 254 nm.
Methode N: System: Agilent: 1260 AS, MWD, Aurora SFC-Modul; Säule: Chiralpak IA 5µm 100x4.6 mm; Eluent: CO₂ / 2-Propanol 75:25; Fluss: 4.0 ml/min; Druck (outlet): 100 bar; Säulentemperatur: 37,5°C; Detektion: DAD 254 nm.
Methode O: System: Waters: Alliance 2695, DAD 996; Säule: Chiralpak ID 5µm 150x4.6 mm; Eluent: Hexan/2-Propanol/Diethylamin 70:30:0.1 (v/v/v); Fluss: 1,0 ml/min; Säulentemperatur: 25°C; Detektion: DAD 280 nm.
Methode P: System: Waters: Alliance 2695, DAD 996, ESA: Corona; Säule: Chiralpak IC 3µm 100x4.6 mm; Eluent: Methanol 100 (v); Fluss: 1.0 ml/min; Säulentemperatur: 25°C; Detektion: DAD 280 nm.
Methode Q: System: Waters: Alliance 2695, DAD 996, ESA: Corona; Säule: Chiralpak IC 3µm 100x4.6 mm; Eluent: Hexan / Ethanol / Diethylamin 70:30:0.1 (v/v/v); Fluss: 1.0 ml/min; Säulentemperatur: 25°C; Detektion: DAD 254 nm.
Methode R: System: Waters: Alliance 2695, DAD 996, ESA: Corona; Säule: Chiralpak IA 3µm 100x4.6 mm; Eluent: Hexan / 2-Propanol 70:30 (v/v); Fluss: 1.0 ml/min; Säulentemperatur: 25°C; Detektion: DAD 254 nm.
Methode Ra: System: Waters: Alliance 2695, DAD 996, ESA: Corona; Säule: Chiralpak IA 3µm 100x4.6 mm; Eluent: Hexan / 2-Propanol / DEA 70:30:0.1 (v/v); Fluss: 1.0 ml/min; Säulentemperatur: 25°C; Detektion: DAD 254 nm.
Methode S: System: Waters: Alliance 2695, DAD 996; Säule: Chiralpak ID 5µm 150x4.6 mm; Eluent: Hexan/Ethanol/Diethylamin 70:30:0.1 (v/v/v); Fluss: 1,0 mL/min; Säulentemperatur: 25°C; Detektion: DAD 254 nm.
Methode T: System: SFC01; Säule: Chiralpak AZ-H 5µm 250x4.6 mm; Eluent: CO₂ / 2-Propanol 70:30; Fluss: 3,0 ml/min; Detektion: DAD 254 nm.
Methode U: System: Waters: Alliance 2695, DAD 996; ESA: Corona; Säule: Chiralpak ID 5µm 150x4.6 mm; Eluent: Hexan / 2-Propanol 70:30 (v/v); Fluss: 1,0 ml/min; Säulentemperatur: 25°C; Detektion: DAD 280 nm.
Methode Ua: System: Waters: Alliance 2695, DAD 996; ESA: Corona; Säule: Chiralpak ID 3µm 100x4.6 mm; Eluent: Hexan / 2-Propanol 70:30 (v/v) + 0.1 DEA; Fluss: 1,0 ml/min; Säulentemperatur: 25°C; Detektion: DAD 254 nm.
Methode K: System: Waters: Alliance 2695, DAD 996, ESA: Corona; Säule: Chiralpak IA 5µm 150x4.6 mm; Eluent: Hexan / Ethanol / Diethylamin 70:30:0.1 (v/v/v); Fluss: 1,0 ml/min; Säulentemperatur: 25°C; Detektion: DAD 254 nm.
Methode K1: System: Waters: Alliance 2695, DAD 996, ESA: Corona; Säule: Chiralpak IC 5µm 150x4.6 mm; Eluent: Hexan / Ethanol / Diethylamin 70:30:0.1 (v/v/v); Fluss: 1,0 ml/min; Säulentemperatur: 25°C; Detektion: DAD 280 nm.
Methode W: System: Agilent: 1260 AS, MWD, Aurora SFC-Modul; Säule: Chiralpak IC 5µm 100x4.6 mm; Eluent: CO₂/Ethanol 75/25; Fluss: 4.0 mL/min; Druck (outlet): 150 bar; Säulentemperatur: 40°C; Detektion: DAD 254 nm.
Methode V: System: Waters: Alliance 2695, DAD 996; ESA: Corona; Säule: Chiralpak ID 3µm 100x4.6 mm; Eluent: Hexan / Ethanol/ Diethylamin 70:30:0.1 (v/v/v); Fluss: 1,0 ml/min; Säulentemperatur: 25°C; Detektion: DAD 280 nm.
Methode Y: System: Agilent: 1260 AS, MWD, Aurora SFC-Modul; Säule: Chiralpak ID 5µm 100x4.6 mm; Eluent: CO₂/Ethanol 70/30; Fluss: 4.0 mL/min; Druck (outlet): 100 bar;
Methode Y1: System: Agilent: 1260 AS, MWD, Aurora SFC-Modul; Säule: Chiralpak ID 3µm 100x4.6 mm; Eluent: CO₂/2-Propanol 6/4; Fluss: 4.0 mL/min; Druck (outlet): 100 bar; Detektion: DAD 254 nm.
Methode L1: System: Waters: Alliance 2695, DAD 996, ESA: Corona; Säule: Chiralpak IA 5µm 150x4.6 mm; Eluent: Acetonitril / MTBE 70:30 (v/v) +0,1% DEA; Fluss: 1.0 ml/min; Säulentemperatur: 25°C; Detektion: DAD 254 nm.

### Präparative HPLC Methoden:

Methode I: System: Agilent Prep 1200, 2xPrep Pump, DLA, MWD, Prep FC; Säule: Chiralpak IC 5µm 250x30 mm; Eluent: Hexan / 2-Propanol 80:20 (v/v); Fluss: 60 ml/min; Säulentemperatur: 25°C; Detektion: UV 254 nm.
Methode II: System: Sepiatec Prep SFC100; Säule: Chiralpak IA 5µm 250x20 mm; Eluent: CO₂ / Ethanol + 0.5% Vol. Diethylamin 80/20; Fluss: 80 ml/min; Druck(outlet): 150 bar; Säulentemperatur: 40°C; Detektion: UV 254 nm.
Methode III: System: Dionex Pump P 580, Gilson Liquid Handler 215, Knauer UV-Detektor K-2501; Säule: Chiralpak IC 5µm 250x30 mm; Eluent: Hexan / Ethanol 70:30 (v/v); Fluss: 50 ml/min; Säulentemperatur: 25°C; Detektion: UV 254 nm.
Methode IIIa: System: Dionex Pump P 580, Gilson Liquid Handler 215, Knauer UV-Detektor K-2501; Säule: Chiralpak IC 5µm 250x30 mm; Eluent: Hexan / Ethanol /Diethylamin 70:30:0.1 (v/v/v); Fluss: 50 ml/min; Säulentemperatur: 25°C; Detektion: UV 280 nm.
Methode IV: System: Sepiatec Prep SFC100; Säule: Chiralpak IC 5µm 250x20 mm; Eluent: CO₂ / 2-Propanol + 0.2% Vol. Diethylamin 90/10; Fluss: 80 mL/min; Druck(outlet): 150 bar; Säulentemperatur: 40°C; Detektion: UV 254 nm.
Methode IVa: System: Sepiatec Prep SFC100; Säule: Chiralpak IC 5µm 250x20 mm; Eluent: CO₂ / Ethanol 6/4; Fluss: 60 mL/min; Druck(outlet): 100 bar; Säulentemperatur: 37°C; Detektion: UV 254 nm.
Methode V: System: Agilent: Prep 1200, 2xPrep Pump, DLA, MWD, Prep FC; Säule: Chiralpak ID 5µm 250x20 mm; Eluent: Hexan / 2-Propanol 70:30 (v/v) +0,1% DEA; Fluss: 23,5 ml/min; Temperatur: RT; Detektion: UV 254 nm.
Methode Va: System: Agilent: Prep 1200, 2xPrep Pump, DLA, MWD, Prep FC; Säule: Chiralpak ID 5µm 250x30 mm; Eluent: Hexan / 2-Propanol 70:30 (v/v) +0,1% DEA; Fluss: 50 ml/min; Temperatur: RT; Detektion: UV 254 nm.
Methode VI:: System: Agilent: Prep 1200, 2xPrep Pump, DLA, MWD, Prep FC; Säule: Chiralpak IC 5µm 250x20 mm; Eluent: Ethanol / Methanol / Diethylamin 50:50:0.1 (v/v/v); Fluss: 20 ml/min; Temperatur: RT; Detektion: UV 254 nm.
Methode VIa:: System: Agilent: Prep 1200, 2xPrep Pump, DLA, MWD, Prep FC; Säule: Chiralpak IC 5µm 250x30 mm; Eluent: Ethanol / Methanol / Diethylamin 50:50:0.1 (v/v/v); Fluss: 35 ml/min; Temperatur: RT; Detektion: UV 254 nm.
Methode VII: System: Dionex: Pump P 580, Gilson: Liquid Handler 215, Knauer: UV-Detektor K-2501; Säule: Chiralpak IC 5µm 250x30 mm; Eluent: Ethanol / Methanol / Diethylamin 50:50:0.1 (v/v/v); Fluss: 40 ml/min; Temperatur: RT; Detektion: UV 254 nm.
Methode VIII: System: Sepiatec: Prep SFC 100; Säule: Chiralpak IA 5µm 250x20 mm; Eluent: CO₂ / Methanol 8:2; Fluss: 80 ml/min; Druck (outlet): 150 bar; Temperatur: 40°C; Detektion: UV 254 nm.
Methode VIIIa: System: Sepiatec: Prep SFC 100; Säule: Chiralpak IA 5µm 250x20 mm; Eluent: CO₂ / Methanol 97:3; Fluss: 80 ml/min; Druck (outlet): 100 bar; Temperatur: 40°C; Detektion: UV 254 nm.
Methode XI: System: Agilent: Prep 1200, 2xPrep Pump, DLA, MWD, Prep FC; Säule: Chiralpak IC 5µm 250x20 mm; Eluent: Methanol 100 (v); Fluss: 50 ml/min; Temperatur: RT; Detektion: UV 280 nm.
Methode XII: System: Agilent: Prep 1200, 2xPrep Pump, DLA, MWD, Prep FC; Säule: Chiralpak IC 5µm 250x20 mm Nr.: 007; Eluent: Hexan / Ethanol / Diethylamin 70:30:0.1 (v/v/v); Fluss: 25 ml/min; Temperatur: RT; Detektion: UV 254 nm.
Methode XIIa: System: Agilent: Prep 1200, 2xPrep Pump, DLA, MWD, Prep FC; Säule: Chiralpak IC 5µm 250x30 mm Nr.: 009; Eluent: Hexan / Ethanol / Diethylamin 70:30:0.1 (v/v/v); Fluss: 50 ml/min; Temperatur: RT; Detektion: UV 254 nm.
Methode XIII: System: Agilent: Prep 1200, 2xPrep Pump, DLA, MWD, Prep FC; Säule: Chiralpak IA 5µm 250x30 mm Nr.: 010; Eluent: Hexan / 2-Propanol 70/30 (v/v); Fluss: 40 ml/min; Temperatur: RT; Detektion: UV 254 nm.
Methode XIV: System: Agilent: Prep 1200, 2xPrep Pump, DLA, MWD, Prep FC; Säule: Chiralpak IA 5µm 250x20 mm Nr.: 008; Eluent: Hexan / Ethanol / Diethylamin 70:30:0.1 (v/v/v); Fluss: 30 ml/min; Temperatur: RT; Detektion: UV 254 nm.
Methode XV: System: Sepiatec: Prep SFC 100; Säule: Chiralpak IA 5µm 250x20 mm; Eluent: CO₂ / Methanol 9:1; Fluss: 60 mL/min; Druck (outlet): 150 bar; Temperatur: 40°C; Detektion: UV 254 nm.
Methode XVI: System: Agilent: Prep 1200, 2xPrep Pump, DLA, MWD, Prep FC; Säule: Chiralpak IA 5µm 250x20 mm Nr.: 006; Eluent: Hexan / Ethanol 70/30 (v/v); Fluss: 30 ml/min; Temperatur: RT; Detektion: UV 280 nm.
Methode XVIa: System: Agilent: Prep 1200, 2xPrep Pump, DLA, MWD, Prep FC; Säule: Chiralpak IC 5µm 250x20 mm Nr.: 021; Eluent: Hexan / Ethanol 70/30 (v/v); Fluss: 50 ml/min; Temperatur: RT; Detektion: UV 280 nm.
Methode XVII: System: Agilent: Prep 1200, 2xPrep Pump, DLA, MWD, Prep FC; Säule: Chiralpak IA 5µm 250x20 mm Nr.: 001; Eluent: Hexan / Ethanol 70/30 (v/v) +0,1% Diethylamin; Fluss: 20 ml/min; Temperatur: RT; Detektion: UV 254 nm.
Methode XVIII: System: Sepiatec Prep SFC100; Säule: Chiralpak IC 5µm 250x20 mm; Eluent: CO2 / Ethanol 75/25; Fluss: 80 mL/min; Druck (outlet): 150 bar; Säulentemperatur: 40°C; Detektion: UV 254 nm.
Methode XIX: System: Agilent: Prep 1200, 2xPrep Pump, DLA, MWD, Prep FC; Säule: Chiralpak ID 5µm 250x30 mm Nr.: 018; Eluent: Hexan / Ethanol 70/30 (v/v) +0,1% Diethylamin; Fluss: 50 ml/min; Temperatur: RT; Detektion: UV 280 nm.
Methode XX: System: Agilent: Prep 1200, 2xPrep Pump, DLA, MWD, Prep FC; Säule: Chiralpak IA 5µm 250x20 mm Nr.: 003; Eluent: Hexan / 2-Propanol 70/30 (v/v) +0,1% Diethylamin; Fluss: 25 ml/min; Temperatur: RT; Detektion: UV 280 nm.
Methode XXa: System: Agilent: Prep 1200, 2xPrep Pump, DLA, MWD, Prep FC; Säule: Chiralpak IA 5µm 250x20 mm Nr.: 014; Eluent: Acetonitril / MTBE 90/10 (v/v) +0,1% Diethylamin; Fluss:30 ml/min; Temperatur: RT; Detektion: UV 254 nm.
Methode XXb: System: Agilent: Prep 1200, 2xPrep Pump, DLA, MWD, Prep FC; Säule: Chiralpak IA 5µm 250x20 mm Nr.: 007; Eluent: Hexan / 2-Propanol 70/30 (v/v) +0,1% Diethylamin; Fluss: 20 ml/min; Temperatur: RT; Detektion: UV 254 nm.
Methode XXI: System: Sepiatec Prep SFC100; Säule: Chiralpak ID 5µm 250x20 mm; Eluent: CO₂ / Ethanol 78/22; Fluss: 60 mL/min; Druck (outlet): 150 bar; Säulentemperatur: 40°C; Detektion: UV 254 nm
Methode XXIa: System: Sepiatec Prep SFC100; Säule: Chiralpak ID 5µm 250x20 mm; Eluent: CO₂ / 2-Propanol 6/4; Fluss: 80 mL/min; Druck (outlet): 150 bar; Säulentemperatur: 40°C; Detektion: UV 254 nm
Methode XXII: System: Agilent: Prep 1200, 2xPrep Pump, DLA, MWD, Prep FC; Säule: Chiralpak ID 5µm 250x20 mm Nr.: 003; Eluent: Hexan / 2-Propanol 70/30 (v/v); Fluss: 30 ml/min; Temperatur: RT; Detektion: UV 280 nm.

Die nachfolgenden Beispiele beschreiben die Herstellung der Zwischenverbindungen, die vorzugsweise zur Herstellung der erfindungsgemäßen Verbindungen verwendet werden. Gegenstand der vorliegenden Erfindung sind somit auch Zwischenverbindungen der allgemeinen Formel (I-A1), 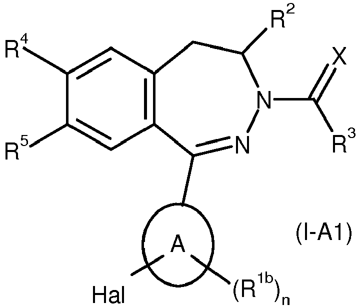 in der X, A, R^{1b}, R², R³, R⁴, R⁵ und n die in der allgemeinen Formel (I) angegebenen Bedeutungen haben, und wobei Hal für Fluor, Chlor oder Brom steht, und wobei (R^{1b})ₙ auch gleichbedeutend stehen kann für R^{1b} und R^{1c}, die bevorzugt zur Herstellung der Verbindungen der allgemeinen Formel I verwendet werden können.
Vorzugsweise steht Hal für Chlor oder Brom.

Gegenstand der vorliegenden Erfindung sind somit weiterhin Zwischenverbindungen der allgemeinen Formel (Ia) 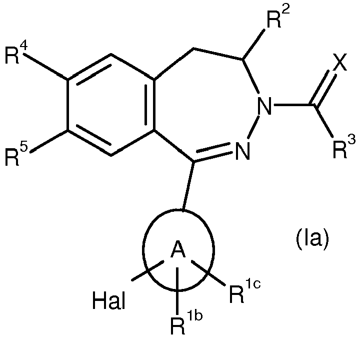 in der X, A, R^{1b}, R^{1c}, R², R³, R⁴ und R⁵ die in der allgemeinen Formel (I) angegebenen Bedeutungen haben, und Hal für Fluor, Chlor oder Brom steht, und die bevorzugt zur Herstellung der Verbindungen der allgemeinen Formel (I) verwendet werden können.
Vorzugsweise steht Hal für Chlor oder Brom.

### Herstellung der Zwischenverbindungen

### Beispiel 1A

### 2,2-Dimethyl-5-[4-(trifluormethoxy)benzyl]-1,3-dioxan-4,6-dion

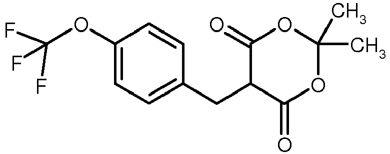

25,4 g (134 mmol) 4-(Trifluormethoxy)benzaldehyd (CAS [659-28-9]), 19,3 g (134 mmol) Meldrumsäure (2,2-Dimethyl-1,3-dioxan-4,6-dion, CAS [2033-24-1]) und 1,93 g (13,4 mmol) Piperidiniumacetat (CAS [4540-33-4]) wurden in 500 ml Ethanol gelöst und 30 min bei RT gerührt. Die Reaktionslösung wurde mittels eines Eisbads auf 0°C gekühlt und weitere 10 min gerührt. 12,6 g (200 mmol) Natriumcyanoborhydrid wurden portionsweise eingetragen, dann ließ man auf RT kommen und weitere 1,5 h rühren. Dann wurden vorsichtig 250 ml 2M Salzsäure zugegeben und man ließ weiterrühren, bis die Gasentwicklung vollständig beendet war (ca. 30 min). Das Ethanol wurde am Rotationsverdampfer entfernt, der Rückstand in 2M Salzsäure aufgenommen und mit Dichlormethan mehrfach extrahiert. Die vereinigten organischen Phasen wurden mit Natriumsulfat getrocknet und das Lösemittel am Rotationsverdampfer entfernt. Man erhielt 32,7 g (41% d. Th.) Rohprodukt als weißen Feststoff, der ohne weitere Reinigung weiter umgesetzt wurde.
LCMS (Methode 1): Rₜ = 1,33 min; m/z = 319 (M+H)⁺

### Beispiel 2A

### 5-(4-Brombenzyl)-2,2-dimethyl-1,3-dioxan-4,6-dion

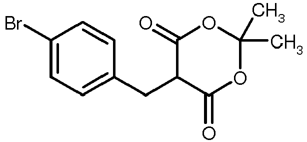

Die Verbindung wurde analog Beispiel **1A** aus 25,0 g (135 mmol) 4-Brombenzaldehyd, 19,4 g (135 mmol) Meldrumsäure, 1,95 g (13,5 mmol) Piperidiniumacetat und 12,7 g (202 mmol) Natriumcyanoborhydrid hergestellt. Man erhielt 27,1 g (64% d. Th.) des gewünschten Produkts, das roh weiter umgesetzt wurde.
LCMS (Methode 2): Rₜ = 1,23 min; m/z = 313, 315 (Br-Isotopenmuster, M+H)⁺

### Beispiel 3A

### 5-(4-Methoxybenzyl)-2,2-dimethyl-1,3-dioxan-4,6-dion

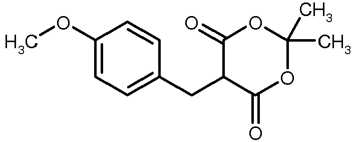

30,0 g (220 mmol) 4-Methoxybenzaldehyd wurden mit 500 ml Wasser vorgelegt und 33,3 g (231 mmol) Meldrumsäure darin suspendiert. Die Mischung wurde 2 h bei einer Innentemperatur von 75°C mechanisch gerührt. Aus der gelblichen Emulsion entstand dabei eine gelbe Suspension. Nach dem Abkühlen wurde die Reaktionsmischung mit Dichlormethan extrahiert und mit Magnesiumsulfat getrocknet. Diese Dichlormethanlösung des Zwischenprodukts 5-(4-Methoxybenzyliden)-2,2-dimethyl-1,3-dioxan-4,6-dion (¹H-NMR (300MHz, CDCl₃): δ = 1,78 (s, 6H), 3,90 (s, 3H), 6,98 (d, 2H), 8,23 (d, 2H), 8,37 (s, 1H)) wurde direkt weiter bearbeitet. Die Lösung (800 ml) wurde auf -3°C gekühlt und 110 ml Essigsäure zugegeben. Dann wurden 20,8 g (155 mmol) Natriumborhydrid innerhalb von 60 min portionsweise eingetragen (Temperatur max. 2°C). Die entstehende Suspension wurde 15 min bei RT nachgerührt. Die Reaktionsmischung wurde durch vorsichtiges Zutropfen von 300 ml Wasser gequencht und ca. 30 min bei RT nachgerührt. Die Phasen wurden getrennt und die organische Phase mit Natriumhydrogencarbonatlösung und Wasser gewaschen und mit Magnesiumsulfat getrocknet. Die Lösemittel wurden am Rotationsverdampfer entfernt. Man erhielt 46,60 g (80% d. Th.) des gewünschten Produkts als gelbliches Öl, das zu einem schwach gelben Feststoff kristallisierte und keiner weiteren Reinigung bedurfte.
¹H-NMR (300MHz, CDCl₃): δ = 1,48 (s, 3H), 1,72 (s, 3H), 3,44 (d, 2H), 3,72 (t, 1H), 3,77 (s, 3H), 6,82 (d, 2H), 7,24 (d, 2H).

### Beispiel 4A

### 2,2,5-Trimethyl-5-[4-(trifluormethoxy)benzyl]-1,3-dioxan-4,6-dion

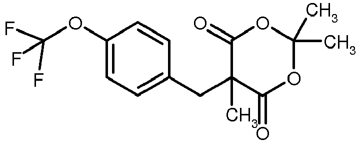

32,7 g (103 mmol) 2,2-Dimethyl-5-[4-(trifluormethoxy)benzyl]-1,3-dioxan-4,6-dion (Beispiel **1A**) und 21,3 g (154 mmol) Kaliumcarbonat wurden bei RT in 400 ml DMF vorgelegt und langsam 72,9 g (514 mmol, 32,0 ml) Iodmethan zugetropft. Die Mischung wurde 1,5 h kräftig bei RT gerührt und dann auf Wasser gegeben. Es wurde 3x mit Ethylacetat extrahiert, die vereinigten organischen Phasen mit ges. Natriumchloridlösung gewaschen und mit Natriumsulfat getrocknet. Die Lösemittel wurden am Rotationsverdampfer entfernt und das Rohprodukt (32,5 g farbloses Öl) mittels Flashchromatographie (SiO₂, Hexan/Ethylacetat) gereinigt. Man erhielt 20,0 g (55% d. Th.) des gewünschten Produkts als farbloses Öl.
¹H-NMR (300MHz, DMSO-*d*₆): δ = 0,99 (s, 3H), 1,57 (s, 3H), 1,63 (s, 3H), 3,22 (s, 2H), 7,12 (d, 2H), 7,31 (d, 2H).

### Beispiel 5A

### 5-(4-Brombenzyl)-2,2,5-trimethyl-1,3-dioxan-4,6-dion

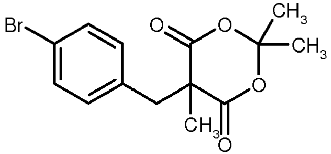

Die Verbindung wurde analog Beispiel **4A** aus 7,37 g (23,5 mmol) 5-(4-Brombenzyl)-2,2-dimethyl-1,3-dioxan-4,6-dion (Beispiel **2A**), 16,7 g (118 mmol) Iodmethan und 4,88 g (35,3 mmol) Kaliumcarbonat hergestellt. Man erhielt 8 g Rohprodukt, das direkt weiter umgesetzt wurde. ¹H-NMR (300MHz, CDCl₃): δ = 1,06 (s, 3H), 1,62 (s, 3H), 1,74 (s, 3H), 3,28 (s, 2H), 7,05 (d, 2H), 7,40 (s, 2H).

### Beispiel 6A

### 5-(4-Methoxybenzyl)-2,2,5-trimethyl-1,3-dioxan-4,6-dion

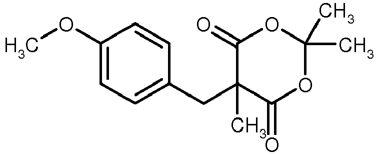

548 g (2,08 mol) 5-(4-Methoxybenzyl)-2,2-dimethyl-1,3-dioxan-4,6-dion (Beispiel **3A**) wurden in 3000 ml Dimethylsulfoxid vorgelegt und unter Rühren bei RT 295 g (2,08 mol) Iodmethan zugetropft. Mit Hilfe eines Eisbades wurde die Lösung auf ca. 15°C Innentemperatur gekühlt und 231 g (2,28 mol) Triethylamin innerhalb von 30 min zugetropft (Innentemperatur 15-22°C). Es wurde 30 min im Eisbad und 3 h bei RT nachgerührt. Die Mischung wurde auf verdünnte Natriumchloridlösung gegeben (2 Portionen mit je 12 1 Wasser und je 500 g Natriumchlorid) und mit Methyl-*tert*-butylether extrahiert. Die organischen Phasen wurden vereinigt, mit halbgesättigter Natriumchloridlösung gewaschen und mit Magnesiumsulfat getrocknet. Die Lösemittel wurden entfernt, man erhielt 497 g (86% d. Th.) des gewünschten Produkts als gelblichen wachsartigen Feststoff. Eine weitere Reinigung war durch Kristallisation (Hexan/Isopropanol) oder chromatographisch möglich.
¹H-NMR (400MHz, CDCl₃): δ = 0,98 (s, 3H), 1,60 (s, 3H), 1,72 (s, 3H), 3,27 (s, 2H), 3,76 (s, 3H), 6,79 (d, 2H), 7, 09 (s, 2H).

### Beispiel 7A

### 2-Methyl-3-[4-(trifluormethoxy)phenyl]propansäure

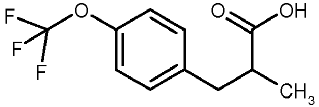

19,0 g (57,2 mmol) 2,2,5-Trimethyl-5-[4-(trifluormethoxy)benzyl]-1,3-dioxan-4,6-dion (Beispiel **4A**) wurden in 90 ml Dioxan und 35 ml konz. wässrige Salzsäure aufgenommen und 2 h bei 125 °C unter Rückfluss erhitzt. Man ließ die Mischung abkühlen und entfernte die Lösemittel am Rotationsverdampfer. Der Rückstand (19,5 g farbloses Harz) wurde 1 h auf 200°C erhitzt. Das erhaltene Rohprodukt wurde ohne weitere Reinigung weiter umgesetzt.
LCMS (Methode 2): Rₜ = 1,21 min; m/z [ES-] = 247 (M-H)⁻
¹H-NMR (300MHz, DMSO-*d*₆): δ = 1,12 (s, 3H), 3,06 (s, 2H), 7,21 - 7,27 (m, 4H).

### Beispiel 8A

### 3-(4-Fluor-3-methoxyphenyl)-2-methylprop-2-ensäure

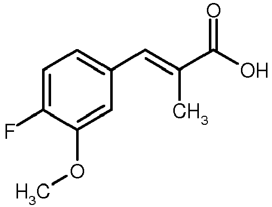

2,18 g (14,1 mmol) 4-Fluor-3-methoxybenzaldehyd (CAS [128495-46-5]), 2,45 g (14,1 mmol) Natriumpropionat (CAS [137-40-6]) und 1,84 g (14,1 mmol) Propionsäureanhydrid (CAS [123-62-6]) wurden zusammen 3 h bei 150°C gerührt. Die zunächst weiße Suspension wurde dabei zu einer klaren Lösung. Die Mischung wurde abgekühlt, mit 2M Natronlauge verdünnt und 2x mit Diethylether ausgeschüttelt. Die wäßrige Phase wurde mit 6M Salzsäure sauer gestellt (pH ca. 5) und 3x mit Ethylacetat extrahiert. Die vereinigten organischen Phasen wurden mit ges. Natriumchloridlösung gewaschen, mit Natriumsulfat getrocknet und die Lösemittel am Rotationsverdampfer entfernt. Man erhielt 1,70 g (51% d. Th.) des Produkts als gelben Feststoff, der ohne weitere Reinigung weiter umgesetzt wurde.
LCMS (Methode 2): Rₜ = 1,06 min; m/z [ES-] = 209 (M-H)⁻
¹H-NMR (300MHz, DMSO-*d*₆): δ = 2,01 (d, 3H), 3,85 (s, 3H), 7,00 - 7,03 (m, 1H), 7,19 - 7,25 (m, 2H), 7,51 (s, 1H).

Analog Beispiel **8A** wurden aus dem entsprechenden Aldehyd folgende Verbindung hergestellt:

| Nr | Struktur | Name | Analytische Daten |
|---|---|---|---|
| **9A** | 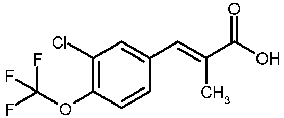 | 3-[3-Chlor-4-(trifluormethoxy)ph enyl]-2-methylprop-2-ensäure | ¹H-NMR (300MHz, DMSO-*d*₆): δ = 1,98 (d, 3H), 7,51 - 7,59 (m, 3H), 7,75 (d, 1H). |
| | | | LCMS (Methode 2): Rₜ = 1,32 min; m/z [ES-]= 279/281 (M-H, Cl-Isotope)⁻ |

### Beispiel 10A

### 3-(4-Fluor-3-methoxyphenyl)-2-methylpropansäure

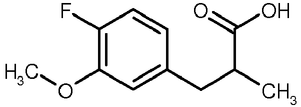

1,55 g (7,34 mmol) 3-(4-Fluor-3-methoxyphenyl)-2-methylprop-2-ensäure (Beispiel **8A**) wurden in Anwesenheit von 780 mg Palladium-Katalysator (10% Pd auf Aktivkohle, 0,73 mmol) in 70 ml Ethylacetat 1 h bei RT unter intensivem Schütteln hydriert (1 atm Wasserstoffatmosphäre). Anschließend wurde der Katalysator abfiltriert, mit Dichlormethan nachgewaschen und das Filtrat am Rotationsverdampfer eingeengt. Man erhielt 1,34 g (60%) 3-(4-Fluor-3-methoxyphenyl)-2-methylpropansäure als öliges Produkt.
LCMS (Methode 2): Rₜ = 1,01 min; m/z [ES-] = 211 (M-H)⁻
¹H-NMR (300MHz, DMSO-*d*₆): δ = 1,00 (d, 3H), 2,49 - 2,66 (m, 2H), 2,84 (dd, 1H), 3,77 (s, 3H), 6,67 - 6,72 (m, 1H), 6,96 (dd, 1H), 7,04 (dd, 1H).

Analog Beispiel **10A** wurden aus Beispiel **9A** folgende Verbindung hergestellt:

| Nr | Struktur | Name | Analytische Daten |
|---|---|---|---|
| **11A** | 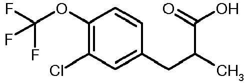 | 3-[3-Chlor-4-(trifluormethoxy)pheny 1]-2-methylpropansäure | ¹H-NMR (300MHz, DMSO-*d*₆): δ = 1,02 (d, 3H), 2,61- 2,71 (m, 2H), 2,82 - 2,91 (m, 1H), 7,27 (dd, 1H), 7,44 (dd, 1H), 7,50 (d, 1H). |
| | | | LCMS (Methode 2): Rₜ = 1,28 min; m/z [ES⁻] = 281/283 (M-H, Cl-Isotope)⁻ |

### Beispiel 12A

### 2-Methyl-6-(trifluormethoxy)indan-1-on

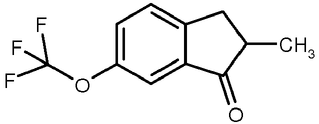

17,2 g (69,3 mmol) rohe 2-Methyl-3-[4-(trifluormethoxy)phenyl]propansäure (Beispiel **7A**) wurden in 100 ml Dichlormethan gelöst und bei RT 12,1 ml (16,6 g, 166 mmol) Thionylchlorid und 0,16 ml DMF zugetropft. Anschließend wurde ca. 30 min unter Rückfluss erhitzt, bis die Gasentwicklung beendet war. Man ließ die Lösung abkühlen und entfernte die Lösemittel am Rotationsverdampfer. Der Rückstand (gelber Feststoff) wurde in 35 ml Dichlormethan aufgenommen und bei RT zu einer Suspension von 10,2 g (76,2 mmol) wasserfreiem Aluminiumchlorid in 200 ml Dichlormethan getropft. Die dunkelrote Lösung wurde 30 min gerührt, dann auf Wasser gegeben und die Phasen getrennt. Die wässrige Phase wurde 3x mit Dichlormethan extrahiert, mit Wasser, ges. Natriumhydrogencarbonatlösung und ges. Natriumchloridlösung gewaschen und mit Natriumsulfat getrocknet. Die Lösemittel wurden entfernt und der Rückstand (10,0 g) flashchromatographisch gereinigt (SiO₂, Hexan/Dioxan). Man erhielt 5,84 g (14% d. Th.) des Produkts als gelbes Öl.
LCMS (Methode 2): Rₜ = 1,27 min; m/z = 231;272 (M+H)⁺ / (M+ACN+H)⁺

### Beispiel 13A

### 6-Brom-2-methylindan-1-on

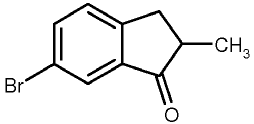

3,64 g (11,1 mmol) 5-(4-Brombenzyl)-2,2,5-trimethyl-1,3-dioxan-4,6-dion (Beispiel **5A**) wurden mit 36 ml Polyphosphorsäure versetzt und die Mischung 10 h bei 100°C gerührt. Nach dem Abkühlen wurde die Mischung auf Eiswasser gegeben und mit Ethylacetat extrahiert. Die vereinigten organischen Phasen wurden mit ges. Natriumhydrogencarbonatlösung gewaschen und mit Natriumsulfat getrocknet. Die Lösemittel wurden am Rotationsverdampfer entfernt und der Rückstand flashchromatographisch gereinigt (SiO₂, Hexan/Ethylacetat). Man erhielt 812 mg (32% d. Th.) des Produkts als braunes Öl.
LCMS (Methode 1): Rₜ = 1,18 min; m/z = 225;227 (Br-Isotopenmuster, M+H)⁺ und 266;268 (Br-Isotopenmuster, M+ACN+H)⁺
¹H-NMR (400MHz, CDCl₃): δ = 1,31 (d, 3H), 2,68 (dd, 1H), 2,72 - 2,79 (m, 1H), 3,35 (dd, 1H), 7,34 (d, 1H), 7,68 (dd, 1H), 7,88 (d, 1H).

### Beispiel 14A

### 6-Methoxy-2-methylindan-1-on

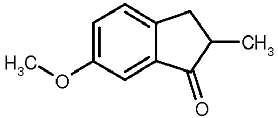

628 g (5,39 mol) Polyphosphorsäure (CAS [8017-16-1]) wurden vorgelegt und auf ca. 100°C erhitzt. Unter Rühren wurden dann 100 g (359 mmol) 5-(4-Methoxybenzyl)-2,2,5-trimethyl-1,3-dioxan-4,6-dion (Beispiel **6A**), gelöst in 400 ml Toluol, innerhalb von 15 min zugetropft. Es wurde ca. 15 min nachgerührt, dann wurde die Reaktionsmischung auf Wasser (ca. 3 l) gegeben, Reste wurden mit Dichlormethan überführt. Es wurde mit weiterem Dichlormethan und Wasser verdünnt und die Phasen getrennt. Die wässrige Phase wurde mit Dichlormethan nachextrahiert und die vereinigten organischen Phasen mehrfach vorsichtig mit halbgesättigter Natriumhydrogencarbonatlösung sowie mit Wasser gewaschen und mit Magnesiumsulfat getrocknet. Die Lösemittel wurden am Rotationsverdampfer entfernt. Man erhielt 58,2 g (92% d. Th.) des Rohprodukts als braunes Öl, das ohne Reinigung weiter umgesetzt wurde. Eine Reinigung ist durch Kugelrohrdestillation bei 140°C möglich, man erhielt dabei ein fast farbloses Öl.
¹H-NMR (300MHz, CDCl₃): δ = 1,30 (d, 3H), 2,64 (dd, 1H), 2,70 - 2,78 (m, 1H), 3,32 (dd, 1H), 3,82 (s, 3H), 7,15 - 7,21 (m, 2H), 7,33 (d, 1H).

### Beispiel 15A

### 6-Fluor-5-methoxy-2-methylindan-1-on

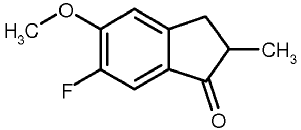

4,10 g (19,3 mmol) 3-(4-Fluor-3-methoxyphenyl)-2-methylpropansäure (Beispiel **10A)** wurden unter Eisbadkühlung vorsichtig mit 13,5 g (116 mmol, 7,71 ml) Chlorsulfonsäure (CAS [7790-94-5]) versetzt und 2 h im Eisbad nachgerührt. Dann wurde die Reaktion durch vorsichtige, portionsweise Zugabe von zerstoßenem Eis beendet. Die Mischung wurde mit Dichlormethan extrahiert und die vereinigten organischen Phasen mit ges. Natriumhydrogencarbonatlösung und ges. Natriumchloridlösung gewaschen und mit Natriumsulfat getrocknet. Die Lösemittel wurden am Rotationsverdampfer entfernt und der braune Rückstand flashchromatographisch gereinigt (SiO₂, Dichlormethan/Methanol 0-3%). Man erhielt 2,15 g (57% d. Th.) des Produkts als blassgelben Feststoff.
LCMS (Methode 2): Rₜ = 1,01 min; m/z = 195 (M+H)⁺
¹H-NMR (300MHz, DMSO-*d*₆): δ = 1,13 (d, 3H), 2,57 - 2,71 (m, 2H), 3,30 (dd, 1H), 3,90 (s, 3H), 7,29 (d, 1H), 7,37 (d, 1H).

Analog Beispiel **15A** wurde aus der entsprechenden Carbonsäure (Beispiel **11A)** folgendes Indanon hergestellt:

| Nr | Struktur | Name | Analytische Daten |
|---|---|---|---|
| **16A** | 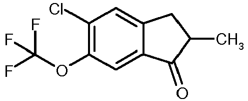 | 5-Chlor-2-methyl-6-(trifluormethoxy)indan -1-on | ¹H-NMR (300MHz, DMSO-*d*₆): δ = 1,17 (d, 3H), 2,66-2,83 (m, 2H), 3,37 (dd, 1H), 7,66 (s, 1H), 7,94 (d, 1H). |
| | | | LCMS (Methode 2): Rₜ = 1,38 min; m/z = 265/267 (M+H, Cl-Isotope)⁺, 306/308 (M+H+ACN, Cl-Isotope)⁺ |

### Beispiel 17A

### 3-(4-Chlorphenyl)-2-methyl-5-(trifluormethoxy)-1H-inden

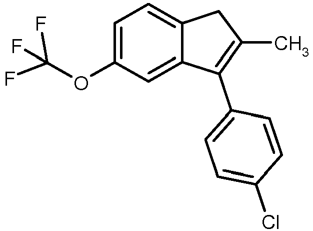

38,1 ml 4-Chlorphenylmagnesiumbromid (1M in Diethylether, 38,1 mmol) wurde unter Argon in 80 ml THF vorgelegt und bei RT 5,84 g (25,4 mmol) 2-Methyl-6-trifluormethoxy-indan-1-on (Beispiel **12A),** gelöst in 20 ml THF, zugetropft. Es wurde 1 h bei RT nachgerührt, dann die Mischung auf ges. Ammoniumchloridlösung gegeben, 3x mit Ethylacetat extrahiert und die vereinigten organischen Phasen mit ges. Natriumchloridlösung gewaschen, mit Natriumsulfat getrocknet und die Lösemittel am Rotationsverdampfer entfernt.
Der Rückstand wurde in 375 ml Dichlormethan aufgenommen, 55 mg 4-Toluolsulfonsäuremonohydrat zugegeben und 16 h bei RT gerührt. Die Reaktionsmischung wurde auf ges. Natriumhydrogencarbonatlösung gegeben, 3x mit Dichlormethan extrahiert und die vereinigten organischen Phasen mit ges. Natriumchloridlösung gewaschen, mit Natriumsulfat getrocknet und das Lösemittel am Rotationsverdampfer entfernt. Der Rückstand wurde flashchromatographisch gereinigt (SiO₂, Hexan/Ethylacetat). Man erhielt 2,42 g (21% d. Th.) des Produkts als farbloses Harz.
LCMS (Methode 1): Rₜ = 1,76 min; m/z = 325 (M+H)^{+ 1}H-NMR (400MHz, DMSO-*d*₆): δ = 2,09 (s, 3H), 3,53 (s, 2H), 6,93 (s, br, 1H), 7,07 - 7,12 (m, 1H), 7,38 (d, 2H), 7,50 - 7,56 (m, 1H), 7,54 (d, 2H).

### Beispiel 18A

### 5-Brom-3-(4-chlorphenyl)-2-methyl-1H-inden

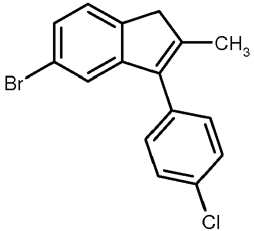

Die Herstellung erfolgte analog Beispiel **17A** aus 800 mg (3,55 mmol) 6-Brom-2-methylindan-1-on (Beispiel **13A**), 5,33 ml 1M 4-Chlorphenylmagnesiumbromid-Lösung in 8 ml THF, die Eliminierung des dabei gebildeten tertiären Alkohols erfolgte mit Hilfe von 7 mg (0,04 mmol) 4-Toluolsulfonsäure-monohydrat. Das Rohprodukt wurde direkt weiter umgesetzt.

Analog Beispiel **17A** wurde aus den entsprechenden Indanonen durch Reaktion mit 4-Chlorphenylmagnesiumbromid und anschließender Wasser-Eliminierung mit Hilfe von 4-Toluolsulfonsäure folgende Verbindungen hergestellt:

| Nr | Struktur | Name | Analytische Daten |
|---|---|---|---|
| **21A** | 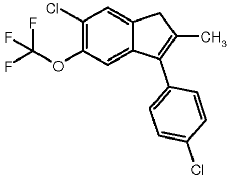 | 6-Chlor-3-(4-chlorphenyl)-2-methyl-5-(trifluormethoxy)-1*H*-inden | ¹H-NMR (300MHz, DMSO-*d*₆): δ = 2,08 (s, 3H), 3,57 (s, 2H), 7,07 (d, 1H), 7,38 (d, 2H), 7,55 (d, 2H), 7,73 (s, 1H). |
| | | | LCMS (Methode 2): Rₜ = 1,81 min; m/z [ES⁻] = 357/359 (M-H, Cl-Isoptope)⁻ |
| **22A** | 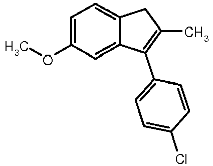 | 3-(4-Chlorphenyl)-5-methoxy-2-methyl-1*H-*inden | ¹H-NMR (300MHz, CDCl₃): δ = 2,11 (s, 3H), 3,40 (s, 2H), 3,78 (s, 3H), 6,72 (dd, 1H), 6,73 (sbr, 1H), 7,32 (d, 1H), 7,33 (d, 2H), 7,44 (d, 2H). |

### Beispiel 23A

### 3-(5-Methoxy-2-methyl-1H-inden-3-yl)pyridin

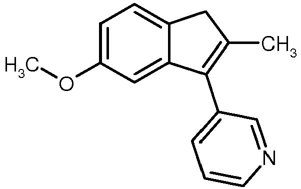

6,28 g (30,6 mmol) 3-Iodpyridin (CAS [1120-90-7]) wurden in 30 ml THF vorgelegt und bei 0°C 24 ml Isopropylmagnesiumchlorid-Lithiumchlorid-Komplex-Lösung (1,3 M in THF, CAS[807329-97-1]) zugetropft und 30 min bei 0°C nachgerührt. Dann wurden 2,0 g (11,4 mmol) 6-Methoxy-2-methylindan-1-on (Beispiel **14A),** gelöst in 30 ml THF bei 0°C zugetropft und 3 h bei 0°C nachgerührt. Die Reaktion wurde durch Zugabe von 100 ml ges. Ammoniumchloridlösung abgebrochen und 10 min nachgerührt. Dann wurde die Mischung mit Ethylacetat extrahiert, die vereinigten organischen Phasen mit Wasser und ges. Natriumchloridlösung gewaschen und mit Natriumsulfat getrocknet. Die Lösemittel wurden am Rotationsverdampfer entfernt und der Rückstand in 60 ml Dichlormethan wieder aufgenommen. Es wurden 4,8 g (25 mmol) 4-Toluolsulfonsäure in 2 Portionen zugegeben und die Mischung insgesamt 36 h auf 35-45°C erhitzt. Dann wurde das Gemisch unter Eisbadkühlung mit 100 ml ges. Natriumhydrogencarbonatlösung versetzt, 10 min nachgerührt und mit Dichlormethan extrahiert. Die vereinigten organischen Phasen wurden mit Wasser gewaschen und mit Natriumsulfat getrocknet. Die Lösemittel wurden am Rotationsverdampfer entfernt und der Rückstand flashchromatographisch gereinigt (SiO₂, Hexan/Ethylacetat). Man erhielt 851 mg (30% d. Th.) des gewünschten Produkts als gelbes Öl.
LCMS (Methode 2): Rₜ = 0,98 min; m/z = 238 (M+H)⁺; 279 (M+ACN+H)⁺
¹H-NMR (600MHz, CDCl₃): δ = 2,15 (s, 3H), 3,45 (s, 2H), 3,78 (s, 3H), 6,72 (d, 1H), 6,75 (dd, 1H), 7,34 (d, 1H), 7,41 (dd, 1H), 7,73 (dt, 1H), 8,62 (dd, 1H), 8,67 (d, 1H).

### Beispiel 24A

### 5-Methoxy-2-methyl-1H-inden-3-yl-1,1,2,2,3,3,4,4,4-nonafluorbutan-1-sulfonat

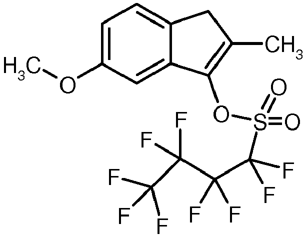

12,6 g (71,5 mmol) frisch kugelrohrdestilliertes 6-Methoxy-2-methylindan-1-on (Beispiel **14A)** wurde in 400 ml THF vorgelegt und 10 min im Eisbad gerührt. Dann wurden 17,1 g (85,8 mmol) Kaliumhexamethyldisilazid (CAS [40949-94-8]) portionsweise zugegeben (Innentemperatur maximal 5°C). Nach weiteren 10 min im Eisbad wurde auf -72°C gekühlt und dann zügig 25,9 g (85,8 mmol) 1,1,2,2,3,3,4,4,4-Nonafluorbutan-1-sulfonylfluorid (CAS [375-72-4]) zugegeben. Die Innentemperatur stieg dabei vorübergehend auf 10°C an, die Lösung färbte sich grünbraun. Es wurde 30 min bei -72°C und weitere 30 min bei 0°C nachgerührt, dann wurde die Reaktionsmischung auf Wasser gegeben und mit Dichlormethan extrahiert. Die organischen Phasen wurden mit Wasser gewaschen, mit Natriumsulfat getrocknet und die Lösemittel entfernt. Das Rohprodukt (25 g orangefarbenes Öl) wurde flashchromatographisch gereinigt (SiO₂, Hexan/Ethylacetat). Man erhielt 16,5 g (45% d. Th.) des Produkts als orangefarbenes Harz.
LCMS (Methode 2): Rₜ = 1,69 min; m/z = 459 (M+H)⁺
¹H-NMR (300MHz, DMSO-*d*₆): δ = 2,09 (s, 3H), 3,47 (s, 2H), 3,73 (s, 3H), 6,68 (d, 1H), 6,82 (dd, 1H), 7,34 (d, 1H).

### Beispiel 25A

### 1-[2-(4-Chlorbenzoyl)-4-(trifluormethoxy)phenyl]propan-2-on

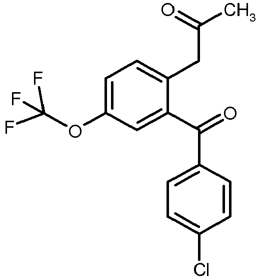

2,42 g (7,45 mmol) 3-(4-Chlorphenyl)-2-methyl-5-trifluormethoxy-1*H*-inden (Beispiel **17A**) wurden in 14 ml Hexan und 14 ml Acetonitril vorgelegt und 34 mg (0,15 mmol) Ruthenium(III)chlorid-Hydrat (CAS [14898-67-0]) zugegeben. Es wurde 10 min bei 0°C gerührt und dann 3,19 g (14,9 mmol) Natriumperiodat portionsweise eingetragen. Die braune Suspension wurde 30 min nachgerührt, dann auf 4M Salzsäure gegeben. Es wurde 3x mit Ethylacetat extrahiert, die vereinigten organischen Phasen mit ges. Natriumchloridlösung gewaschen, mit Natriumsulfat getrocknet und das Lösemittel am Rotationsverdampfer entfernt. Der Rückstand wurde flashchromatographisch gereinigt (SiO₂, Hexan/Ethylacetat). Man erhielt 2,18 g (52% d. Th.) des Produkts als farbloses Harz.
LCMS (Methode 1): Rₜ = 1, 45 min; m/z = 357 (M+H)⁺
¹H-NMR (400MHz, DMSO-*d*₆): δ = 2,05 (s, 3H), 3,98 (s, 2H), 7,31 - 7,33 (m, 1H), 7,46 (d, 1H), 7,50 - 7,56 (m, 1H), 7,60 (d, 2H), 7,68 (d, 2H).

### Beispiel 26A

### 1-[4-Brom-2-(4-chlorbenzoyl)phenyl]propan-2-on

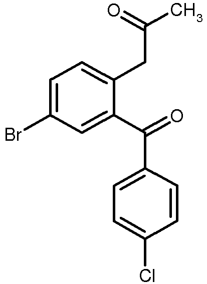

Die Herstellung erfolgte analog Beispiel **25A** aus 1,14 g (3,57 mmol) 5-Brom-3-(4-chlorphenyl)-2-methyl-1*H*-inden (Beispiel **18A)**, 16 mg (0,07 mmol) Ruthenium(III)-chlorid-Hydrat und 1,53 g (7,13 mmol) Natriumperiodat. Man erhielt 347 mg (28% d. Th.) des gewünschten Produkts als orangefarbenes Öl.
LCMS (Methode 1): Rₜ = 1, 41 min; m/z = 351;353;355 (Br-Cl-Isotopenmuster, M+H)^{+ 1}H-NMR (400MHz, CDCl₃): δ = 2,18 (s, 3H), 3,96 (s, 2H), 7,14 (d, 1H), 7,45 - 7,50 (m, 3H), 7,61 (dd, 1H), 7,76 (d, 2H).

### Beispiel 27A

### 1-[4-Methoxy-2-(pyridin-3-ylcarbonyl)phenyl]propan-2-on

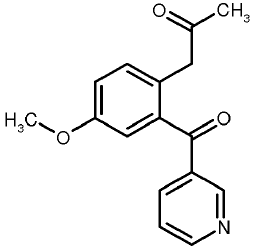

844 mg (3,56 mmol) 3-(5-Methoxy-2-methyl-1*H*-inden-3-yl)pyridin (Beispiel **23A)** wurden in 15 ml Dimethoxyethan und 5 ml Wasser vorgelegt und mit 4,5 ml (0,36 mmol) Osmiumtetroxidlösung (CAS [20816-12-0], 2,5% in *tert*-Butanol) versetzt. Anschließend wurden 1,52 g (7,11 mmol) Natriumperiodat (CAS [7790-28-5]) portionsweise zugegeben und 3 h bei RT gerührt. Die Reaktionsmischung wurde über einige Zentimeter Kieselgel 60 filtriert, mit Ethylacetat nachgewaschen und das Filtrat mit Wasser und ges. Natriumchloridlösung gewaschen und mit Natriumsulfat getrocknet. Die Lösemittel wurden am Rotationsverdampfer entfernt. Man erhielt 439 mg braunen Rückstand als Rohprodukt (ca. 45% d. Th.), das direkt weiter umgesetzt wurde.
LCMS (Methode 2): Rₜ = 0, 90 min; m/z = 270 (M+H)⁺

Analog Beispiel **25A** wurden folgende Verbindungen aus den entsprechenden 1*H*-Indenen hergestellt:

| Nr | Struktur | Name | Analytische Daten |
|---|---|---|---|
| **28A** | 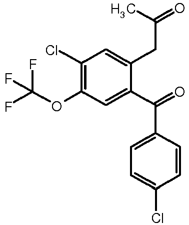 | 1-[5-Chlor-2-(4-chlorbenzoyl)-4-(trifluormethoxy)pheny l]propan-2-on | ¹H-NMR (400MHz, CDCl₃): δ = 2,06 (s, 3H), 3,99 (s, 2H), 7,54 (d, 1H), 7,60 (d, 2H), 7,69 (d, 2H), 7,72 (s, 1H). |
| | | | LCMS (Methode 2): Rₜ = 1,52 min; m/z = 391/393 (M+H, Cl-Isotope)⁺ |
| **29A** | 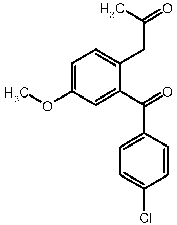 | 1-[2-(4-Chlorbenzoyl)-4-methoxyphenyl] propan -2-on | ¹H-NMR (300MHz, CDCl₃): δ = 2,15 (s, 3H), 3,89 (s, 2H), 3,78 (s, 3H), 6,89 (d, 1H), 7,02 (dd, 1H), 7,18 (d, 1H), 7,44 (d, 2H), 7,77 (d, 2H). |

### Beispiel 30A

### 1-(4-Chlorphenyl)-4-methyl-8-(trifluormethoxy)-5H-2,3-benzodiazepin

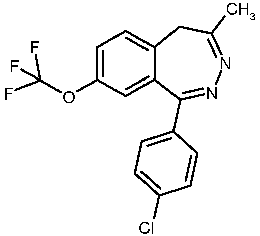

2,18 g (6,11 mmol) 1-[2-(4-Chlorbenzyl)-4-trifluormethoxyphenyl]propan-2-on (Beispiel **25A**) wurden in 25 ml Ethanol vorgelegt und 920 mg (18,3 mmol) Hydrazinhydrat zugegeben. Man ließ 30 min bei RT rühren und gab die Reaktionsmischung dann auf Wasser und extrahierte 3x mit Ethylacetat. Die vereinigten organischen Phasen wurden mit ges. Natriumchloridlösung gewaschen, mit Natriumsulfat getrocknet und das Lösemittel am Rotationsverdampfer entfernt. Der Rückstand wurde flashchromatographisch gereinigt (SiO₂, Hexan/Ethylacetat). Man erhielt 1,53 g (65% d. Th.) des Produkts als blassgelben Feststoff.
LCMS (Methode 1): Rₜ = 1,44 min; m/z = 353 (M+H)⁺
¹H-NMR (400MHz, DMSO-*d*₆): δ = 2,05 (s, 3H), 2,89 (d, 1H), 3,61 (d, 1H), 7,20 (s, br, 1H), 7,49 - 7,54 (m, 4H), 7,59 - 7,66 (m, 2H).

### Beispiel 31A

### 8-Brom-1-(4-chlorphenyl)-4-methyl-5H-2,3-benzodiazepin

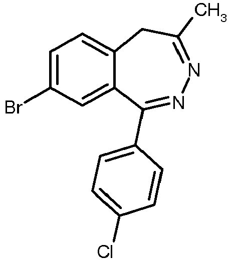

Die Herstellung erfolgte analog Beispiel **30A** aus 340 mg (967 µmol) 1-[4-Brom-2-(4-chlorbenzoyl)phenyl]propan-2-on (Beispiel **26A**) und 702 mg (14,0 mmol) Hydrazinhydrat in 34 ml Ethanol. Man erhielt 376 mg des Rohprodukts als orangefarbenes Öl, das ohne Reinigung direkt weiter umgesetzt wurde.
LCMS (Methode 1): Rₜ = 1,41 min; m/z = 347;349;351 (Br-Cl-Isotopenmuster, M+H)⁺
¹H-NMR (300MHz, CDCl₃): δ = 2,15 (s, 3H), 3,01 (d, 1H), 3,35 (d, 1H), 7,19 (d, 1H), 7,41 (d, 2H), 7,45 (d, 1H), 7,60 (d, 2H), 7,66 (dd, 1H).

### Beispiel 32A

### 8-Methoxy-4-methyl-1-(pyridin-3-yl)-5H-2,3-benzodiazepin

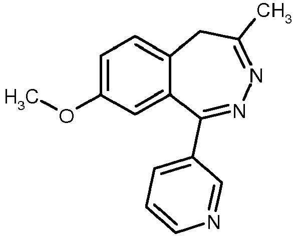

Die Herstellung erfolgte analog Beispiel **30A** aus 430 mg (1,60 mmol) rohem 1-[4-Methoxy-2-(pyridin-3-ylcarbonyl)phenyl]propan-2-on (Beispiel **27A**) und 888 mg (17,7 mmol) Hydrazinhydrat in 43 ml Ethanol. Man erhielt 408 mg des Rohprodukts als braunes Harz, das ohne Reinigung direkt weiter umgesetzt wurde.
LCMS (Methode 2): Rₜ = 0,79 min; m/z = 266 (M+H)⁺

Analog Beispiel **30A** wurden aus den entsprechenden Diketonen folgende Verbindungen hergestellt:

| Nr | Struktur | Name | Analytische Daten |
|---|---|---|---|
| **33A** | 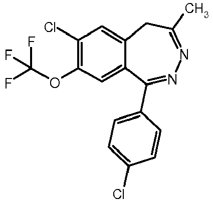 | 7-Chlor-1-(4-chlorphenyl)-4-methyl-8-(trifluormethoxy)-5*H*-2,3-benzodiazepin | ¹H-NMR (300MHz, DMSO-*d*₆): δ = 2,07 (s, 3H), 2,89 (d, 1H), 3,62 (d, 1H), 7,39 (d, 1H), 7,49 - 7,55 (m, 4H), 7,94 (s, 1H). |
| | | | LCMS (Methode 1): Rₜ = 1,51 min; m/z = 387/389/391 (M+H, Cl-Isotope)⁺ |
| **34A** | 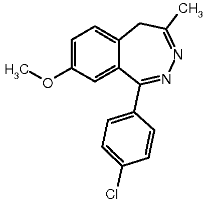 | 1-(4-Chlorphenyl)-8-methoxy-4-methyl-5*H-*2,3-benzodiazepin | ¹H-NMR (400MHz, CDCl₃): δ = 2,12 (s, 3H), 3,00 (dd, 1H), 3,27 (dd, 1H), 3,72 (s, 3H), 6,78 (d, 1H), 7,08 (dd, 1H), 7,18 (d, 1H), 7,37 (d, 2H), 7,63 (d, 2H). |
| | | | LCMS (Methode 3): Rₜ = 1,03 min; m/z = 284;286 (Cl-Isotopenmuster, M+H)⁺ |

### Beispiel 35A

### (±)-1-(4-Chlorphenyl)-4-methyl-8-(trifluormethoxy)-4,5-dihydro-3H-2,3-benzodiazepin

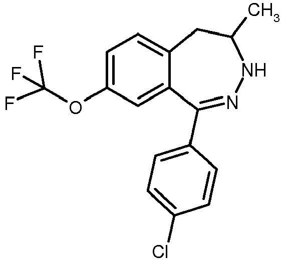

1,53 g (4,34 mmol) 1-(4-Chlorphenyl)-4-methyl-8-(trifluormethoxy)-5*H*-2,3-benzodiazepin (Beispiel **30A)** wurden bei RT in 160 ml Methanol vorgelegt, 2,5 ml 2M Salzsäure zugegeben und 1,36 g (21,7 mmol) Natriumcyanoborhydrid eingetragen. Man ließ 1 h bei RT rühren, dann wurde die Mischung mit 2M Natronlauge alkalisch gestellt (pH ca. 8). Das Methanol wurde am Rotationsverdampfer weitgehend entfernt und der Rückstand zwischen Wasser und Dichlormethan verteilt. Die Phasen wurden getrennt und die wässrige Phase mit Dichlormethan extrahiert. Die vereinigten organischen Phasen wurden mit ges. Natriumchloridlösung gewaschen, mit Natriumsulfat getrocknet und das Lösemittel am Rotationsverdampfer entfernt. Der Rückstand wurde flashchromatographisch gereinigt (SiO₂, Hexan/Ethylacetat). Man erhielt 1,00 g (61% d. Th.) des Produkts als gelbes kristallisierendes Harz.
LCMS (Methode 2): Rₜ = 1,50 min; m/z = 355 (M+H)⁺
¹H-NMR (400MHz, DMSO-*d*₆): δ = 1,10 (d, 3H), 2,75 (dd, 1H), 2,99 (dd, 1H), 3,76 - 3,83 (m, 1H), 6,84 (s, br, 1H), 7,21 - 7,24 (m, 1H), 7,32 - 7,38 (m, 5H), 7,64 (s, br, 1H).

### Beispiel 36A

### (±)-8-Brom-1-(4-chlorphenyl)-4-methyl-4,5-dihydro-3H-2,3-benzodiazepin

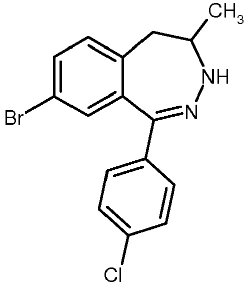

Die Herstellung erfolgte analog Beispiel **35A** aus 336 mg (966 µmol) 8-Brom-1-(4-chlorphenyl)-4-methyl-5*H*-2,3-benzodiazepin (Beispiel **31A**) und 304 mg (4,83 mmol) Natriumcyanoborhydrid in 33 ml Methanol und 0,5 ml 2M Salzsäure. Das Rohprodukt (282 mg) wurde flashchromatographisch gereinigt (SiO₂, Hexan/Ethylacetat). Man erhielt 141 mg (42% d. Th.) des Produkts als orangefarbenen Schaum.
LCMS (Methode 1): Rₜ = 1,39 min; m/z = 349;351;353 (Br-Cl-Isotopenmuster, M+H)⁺
¹H-NMR (300MHz, CDCl₃): δ = 1,26 (d, 3H), 2,65 (dd, 1H), 2,92 (dd, 1H), 4,02 - 4,13 (m, 1H), 5,46 (s, br, 1H), 7,12 (d, 1H), 7,22 (d, 1H), 7,34 (d, 2H), 7,42 - 7,48 (m, 3H).

### Beispiel 37A

### (±)-8-Methoxy-4-methyl-1-(pyridin-3-yl)-4,5-dihydro-3H-2,3-benzodiazepin

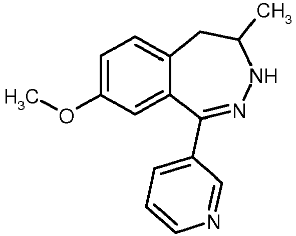

Die Herstellung erfolgte analog Beispiel **35A** aus 400 mg (1,51 mmol) rohem 8-Methoxy-4-methyl-1-(pyridin-3-yl)-5*H*-2,3-benzodiazepin (Beispiel **32A**) und 473 mg (7,54 mmol) Natriumcyanoborhydrid in 20 ml Methanol und 0,6 ml 2M Salzsäure. Man erhielt 155 mg (ca. 38% d. Th.) des Rohprodukts als braunes Harz. Es wurde direkt weiter umgesetzt.
LCMS (Methode 2): Rₜ = 0,77 min; m/z = 268 (M+H)⁺

Analog Beispiel **35A** wurden folgende Verbindungen aus den entsprechenden 5*H*-2,3-Benzodiazepinen hergestellt:

| Nr | Struktur | Name | Analytische Daten |
|---|---|---|---|
| **38A** | 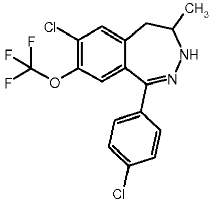 | (±)-7-Chlor-1-(4-chlorphenyl)-4-methyl-8-(trifluormethoxy)-4,5-dihydro-3*H*-2,3-benzodiazepin | ¹H-NMR (300MHz, DMSO-*d*₆): δ = 1,11 (d, 3H), 2,78 (dd, 1H), 3,02 (dd, 1H), 3,73 - 3,83 (m, 1H), 6,99 (d, 1H), 7,31 - 7,38 (m, 4H), 7,58 (s, 1H), 7,84 (d, 1H). |
| | | | LCMS (Methode 2): Rₜ = 1,26 min; m/z = 389/391 (M+H, Cl-Isotopenmuster)⁺ |
| **39A** | 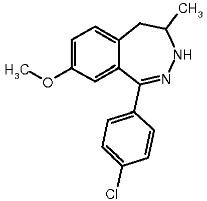 | (±)-1-(4-Chlorphenyl)-4,5-dihydro-8-methoxy-4-methyl-3*H-*2,3-benzodiazepin | ¹H-NMR (400MHz, CDCl₃): δ = 1,24 (d, 3H), 2,60 (dd, 1H), 2,90 (dd, 1H), 3,70 (s, 3H), 4,07 (qdd, 1H), 6,61 (d, 1H), 6,89 (dd, 1H), 7,16 (d, 1H), 7,32 (d, 2H), 7,51 (d, 2H). |
| | | | LCMS (Methode 3): Rₜ = 1,09 min; m/z = 301;303 (Cl-Isotopenmuster, M+H)⁺ |

### Beispiel 40A

### 1-(3,4-Dimethoxyphenyl)propan-2-ol

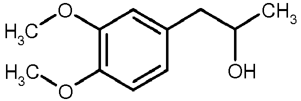

147 mg (3,86 mmol) Lithiumaluminiumhydrid wurden bei 0°C in 30 ml THF vorgelegt und 1,00 g (5,15 mmol) 1-(3,4-Dimethoxyphenyl)propan-2-on, gelöst in 10 ml THF, zugetropft. Man ließ 2 h bei 0°C nachrühren und gab dann vorsichtig nacheinander 0,1 ml Wasser, 0,1 ml 2M Natronlauge und weitere 0,3 ml Wasser zu. Nach weiteren 30 min Rühren bei RT filtrierte man über Kieselgel/Natriumsulfat ab, wusch mit Ethylacetat nach und engte das Filtrat am Rotationsverdampfer ein. Man erhielt 950 mg Produkt (82% d. Th.), das direkt weiter umgesetzt wurde.
LCMS (Methode 2): Rₜ = 0,82 min; m/z = 197 (M+H)⁺; 179 (M-H₂O+H)⁺
¹H-NMR (300MHz, DMSO-*d*₆): δ = 0,98 (d, 3H), 2,43 (dd, 1H), 2,59 (dd, 1H), 3,67 (s, 3H), 3,69 (s, 3H), 3,70 - 3,79 (m, 1H), 4,43 (d, 1H), 6,65 (dd, 1H), 6,75 (d, 1H), 6,79 (d, 1H).

### Beispiel 41A

### 1-(4-Bromphenyl)-3,4-dihydro-6,7-dimethoxy-3-methyl-1H-2-benzopyran

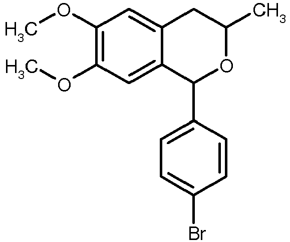

950 mg (4,84 mmol) 1-(3,4-Dimethoxyphenyl)propan-2-ol (Beispiel **40A)** und 950 mg (5,14 mmol) 4-Brombenzaldehyd (CAS [1122-91-4]) wurden bei RT in 4 ml Dioxan vorgelegt und 7,70 ml Zinkchloridlösung (0,7 M in THF, CAS [7646-85-7]) und 2,45 ml Salzsäure (4 M in Dioxan, CAS [7647-01-0]) zugegeben. Dann wurde die Mischung 3 h unter Rückfluss erhitzt und weitere 14 h bei RT nachgerührt. Die Mischung wurde auf Wasser gegeben, mit Ethylacetat extrahiert und die vereinigten organischen Phasen mit ges. Natriumhydrogencarbonatlösung und ges. Natriumchloridlösung gewaschen und mit Natriumsulfat getrocknet. Die Lösemittel wurden am Rotationsverdampfer entfernt. Man erhielt 3,0 g Rohprodukt als hellbraunes Öl, das ohne Reinigung direkt weiter umgesetzt wurde.
LCMS (Methode 2): Rₜ = 1,44 min; m/z = 363; 365 (Br-Isotopenmuster, M+H)⁺

### Alternative Herstellungsvorsschrift von Beispiel 41A

349.2 g (1.779 mol) (±)-1-(3,4-Dimethoxyphenyl)propan-2-ol (Beispiel **40A**) und 329.2 g (1.779 mol) 4-Brombenzaldehyd (CAS [1122-91-4]) wurden bei RT in 3 1 Toluol vorgelegt und mit 140 ml Salzsäure (36% wässrige Lösung) versetzt und für 2 Tage bei RT gerührt. Dann wurde die Mischung auf 2 1 Wasser gegossen und 2x mit je 2 1 Ethylacetat extrahiert und die vereinigten organischen Phasen 1x mit ges. wässriger Natriumhydrogencarbonatlösung und 1x mit 2 1 Wasser gewaschen und mit Natriumsulfat getrocknet. Das Lösemittel wurde am Rotationsverdampfer reduziert. Dabei fiel das Produkt als farbloser Feststoff aus. Kurz vor der Trockene wurde 1 1 Hexan zugegeben und im Eisbad abgekühlt. Der Feststoff wurde abgesaugt, mit Hexan gewaschen und dann im Vakuum bei 50°C getrocknet. Man erhielt 598.9 g (93% d. Th) des Produktes (Isomerengemisch), das ohne weitere Reinigung direkt weiter umgesetzt wurde.

Analog Beispiel **41A** wurde folgende Verbindung aus Beispiel **40A** und 3-Brombenzaldehyd hergestellt:

| Nr | Struktur | Name | Analytische Daten |
|---|---|---|---|
| **42A** | 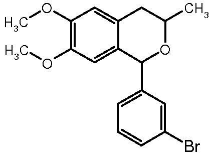 | 1-(3-Bromphenyl)-3,4-dihydro-6,7-dimethoxy-3-methyl-1*H*-2-benzopyran | LCMS (Methode 3): Rₜ = 1,40 min; m/z = 363; 365 (M+H, Br-Isotopenmuster)⁺ |

### Beispiel 43A

### 1-[2-(4-Brombenzoyl)-4,5-dimethoxyphenyl]propan-2-on

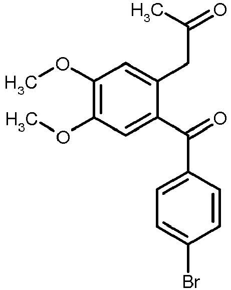

3,00 g (8,26 mmol) 1-(4-Bromphenyl)-3,4-dihydro-6,7-dimethoxy-3-methyl-1*H*-2-benzopyran (Beispiel **41A**) wurden zusammen mit 3 g Kieselgel bei 0°C in 30 ml Aceton vorgelegt. Dann wurde eine Lösung von 3,01 g (30,1 mmol) Chrom(VI)oxid (CAS [1333-82-0]) in 10 ml konz. Schwefelsäure und 20 ml Wasser langsam zugetropft und 1 h bei RT nachgerührt. Die dann rotbraune Mischung wurde auf Waser gegeben und mit Ethylacetat extrahiert. Die organischen Phasen wurden mit ges. Natriumchloridlösung neutral gewaschen, mit Natriumsulfat getrocknet und die Lösemittel am Rotationsverdampfer entfernt. Der Rückstand (3 g) wurde flashchromatographisch gereinigt (SiO₂, Hexan/Ethylacetat). Man erhielt 1,03 g (33% d. Th., 2 Stufen) des Produkts als gelben Feststoff.
LCMS (Methode 2): Rₜ = 1,26 min; m/z = 377; 379 (Br-Isotopenmuster, M+H)⁺

### Alternative Herstellungsvorsschrift von Beispiel 43A

Herstellung von Jones Reagenz:
267g Chrom-VI-oxid (CrO₃) wurde vorsichtig in 230 ml Schwefelsäure (95-97%) eingetragen. Dabei wurde mit Wassereis und Wasser gekühlt, so dass die Innentemperatur bei 35-40°C lag. Anfangs fiel ein orangenes Kristallisat an, das sich unter der Wasserzugabe langsam auflöste. Nach Zugabe von 500ml Wasser hatte sich bis auf geringen Bodensatz alles gelöst. Es wurde für 30 min bei RT gerührt, dann in eine Flasche überführt und auf 1000 ml mit Wasser aufgefüllt. Es resultierte eine ca. 2.6 M Lösung.

496.5 g (1.367 mol) (±)-1-(4-Bromphenyl)-3,4-dihydro-6,7-dimethoxy-3-methyl-1*H*-2-benzopyran (Beispiel **13A)** wurden in 5 1 Aceton vorgelegt, auf 0°C abgekühlt und mit 50g Kieselgel versetzt. Dann wurden 1.9 1 Chromschwefelsäure (Jones Reagenz) innerhalb von 4 h zugetropft und 1 h bei RT nachgerührt. Nach erfolgtem Umsatz wurde zu dem Reaktionsgemisch langsam 4 1 Wasser zugegeben. Es wurde 3x mit je 4 1 Ethylacetat extrahiert. Die vereinigten organischen Phasen wurden mit 4 1 ges. wässriger Natriumhydrogencarbonatlösung und 3x mit je 4 1 ges. wässriger Natriumchloridlösung gewaschen und mit Natriumsulfat getrocknet. Das Lösemittel wurde am Rotationsverdampfer reduziert. Dabei fiel das Produkt als farbloser Feststoff aus. Kurz vor der Trockene wurde mit 500 ml Hexan versetzt und im Eisbad abgekühlt. Der Feststoff wurde abgesaugt, mit Hexan gewaschen und dann im Vakuum bei 50°C getrocknet. Man erhielt 334.1 g (65 % d. Th) des Produktes, das ohne weitere Reinigung direkt weiter umgesetzt wurde.

Analog Beispiel **43A** wurde folgende Verbindung aus Beispiel **42A** hergestellt:

| Nr | Struktur | Name | Analytische Daten |
|---|---|---|---|
| **44A** | 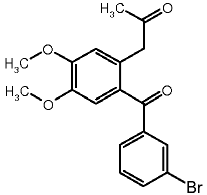 | 1-[2-(3-Brombenzoyl)-4,5-dimethoxyphenyl]prop an-2-on | LCMS (Methode 3): Rₜ = 1,21 min; m/z = 377; 379 (M+H, Br-Isotopenmuster)⁺ |

### Beispiel 45A

### 1-(4-Bromphenyl)-7,8-dimethoxy-4-methyl-5H-2,3-benzodiazepin

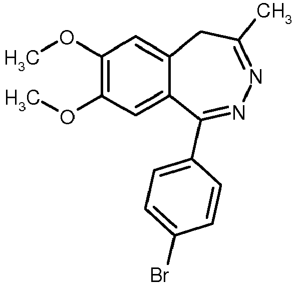

730 mg (1,94 mmol) 1-[2-(4-Brombenzoyl)-4,5-dimethoxyphenyl]propan-2-on (Beispiel **43A**) wurden mit 513 mg (10,3 mmol) Hydrazinhydrat in 7 ml Ethanol 1 h bei 100°C Badtemperatur gerührt. Nach dem Abkühlen wurde die Mischung mit Chlorwasserstoffgas gesättigt (ca. 5 min einleiten). Die Reaktionslösung wurde auf Wasser gegeben, mit 1M Natronlauge alkalisiert und mit Dichlormethan extrahiert. Die vereinigten organischen Phasen wurden mit Natriumsulfat getrocknet und das Lösemittel am Rotationsverdampfer entfernt. Der Rückstand (1 g gelber Feststoff) wurde flashchromatographisch gereinigt (SiO₂, Dichlormethan/Methanol 0-3%). Man erhielt 390 mg (50% d. Th.) des Produkts als gelben Feststoff.
LCMS (Methode 2): Rₜ = 1,20 min; m/z = 373;375 (Br-Isotopenmuster, M+H)⁺
¹H-NMR (300MHz, DMSO-*d*₆): δ = 2,02 (s, 3H), 2,71 (d, 1H), 3,42 (d, 1H), 3,59 (s, 3H), 3,83 (s, 3H), 6,70 (s, 1H), 7,06 (s, 1H), 7,50 (d, 2H), 7,61 (d, 2H).

### Alternative Herstellungsvorsschrift für Beispiel 45A

471 g (1.249 mol) 1-[2-(4-Brombenzoyl)-4,5-dimethoxyphenyl]propan-2-on (Beispiel **43A**) wurden in 4.5 1 Ethanol bei 0°C vorgelegt und tropfenweise mit 402 ml Hydrazinhydrat (6.62 mol) versetzt. Man ließ auf RT kommen und rührte für 2 Tage bei dieser Temperatur. Vom Feststoff wurde abdekantiert und der klare Überstand am Rotationsverdampfer aufkonzentriert. Das Konzentrat wurde mit dem Feststoff vereint. Nach Zugabe von 8 l Eiswasser wurde für 2 Tage nachgerührt. Der so erhatlene Niederschlag wurde abgesaugt, mit Wasser gewaschen und dann im Vakuum bei 50°C getrocknet. Man erhielt 409.8 g (88 % d. Th) des Produktes, das ohne weitere Reinigung direkt weiter umgesetzt wurde.

Analog Beispiel **45A** wurde folgende Verbindung aus Beispiel **44A** hergestellt:

| Nr | Struktur | Name | Analytische Daten |
|---|---|---|---|
| **46A** | 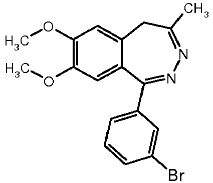 | 1-(3-Bromphenyl)-7,8-dimethoxy-4-methyl-5*H*-2,3-benzodiazepin | ¹H-NMR (300MHz, CDCl₃): δ = 2,15 (s, 3H), 2,98 (d, 1H), 3,27 (d, 1H), 3,75 (s, 3H), 3,97 (s, 3H), 6,73 (s, 1H), 6,75 (s, 1H), 7,27 (dd, 1H), 7,55 (dbr, 1H), 7,61 (dbr, 1H), 7,86 (m, 1H). |
| | | | LCMS (Methode 3): Rₜ = 1,15 min; m/z = 373; 375 (M+H, Br-Isotopenmuster)⁺ |

### Beispiel 47A

### (±)-1-(4-Bromphenyl)-7,8-dimethoxy-4-methyl-4,5-dihydro-3H-2,3-benzodiazepin

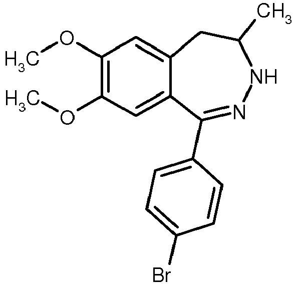

1,99 g (5,33 mmol) 1-(4-Bromphenyl)-7,8-dimethoxy-4-methyl-5*H*-2,3-benzodiazepin (hergestellt nach der Vorschrift in Beispiel **45A)** wurden bei RT in 200 ml Methanol vorgelegt, 3,0 ml 2M Salzsäure zugegeben und 1,68 g (26,6 mmol) Natriumcyanoborhydrid eingetragen. Man ließ 1 h bei RT rühren, dann wurde die Mischung mit 2M Natronlauge alkalisch gestellt (pH ca. 8). Das Methanol wurde am Rotationsverdampfer weitgehend entfernt und der Rückstand zwischen Wasser und Dichlormethan verteilt. Die Phasen wurden getrennt und die wässrige Phase mit Dichlormethan extrahiert. Die vereinigten organischen Phasen wurden mit ges. Natriumchloridlösung gewaschen, mit Natriumsulfat getrocknet und das Lösemittel am Rotationsverdampfer entfernt. Der Rückstand wurde flashchromatographisch gereinigt (SiO₂, Hexan/Ethylacetat). Man erhielt 1,56 g (78% d. Th.) des Produkts als gelbes kristallisierendes Harz.
LCMS (Methode 2): Rₜ = 0,96 min; m/z = 375;377 (Br-Isotopenmuster, M+H)⁺
¹H-NMR (400MHz, DMSO-*d*₆): δ = 1,09 (d, 3H), 2,58 (dd, 1H), 2,83 (dd, 1H), 3,27 (s, 3H), 3,51 (s, 3H), 3,77 - 3,82 (m, 1H), 6,47 (s, 1H), 6,85 (s, 1H), 7,01 (d, 1H), 7,33 (d, 2H), 7,47 (d, 2H).

Analog Beispiel **47A** wurde folgende Verbindung aus Beispiel **46A** hergestellt:

| Nr | Struktur | Name | Analytische Daten |
|---|---|---|---|
| **48A** | 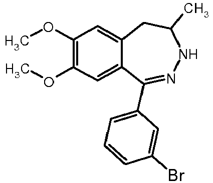 | (±)-1-(3-Bromphenyl)-7,8-dimethoxy-4-methyl-4,5-dihydro-3*H*-2,3-benzodiazepin | ¹H-NMR (300MHz, CDCl₃): δ = 1,28 (d, 3H), 2,62 (dd, 1H), 2,89 (dd, 1H), 3,71 (s, 3H), 3,94 (s, 3H), 4,11 (m, 1H), 6,59 (s, 1H), 6,76 (s, 1H), 7,22 (dd, 1H), 7,45 (dbr, 1H), 7,48 (dbr, 1H), 7,75 (m, 1H). |
| | | | LCMS (Methode 3): Rₜ = 0,99 min; m/z = 375; 377 (M+H, Br-Isotopenmuster)⁺ |

### Beispiel 49A

### (±)-1-(4-Bromphenyl)-7,8-dimethoxy-N,4-dimethyl-4,5-dihydro-3H-2,3-benzodiazepin-3-carboxamid

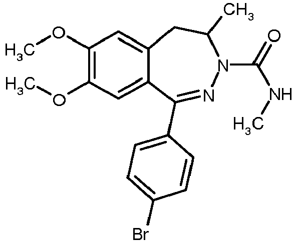

1.56 g (4.16 mmol) (±)-1-(4-Bromphenyl)-7,8-dimethoxy-4-methyl-4,5-dihydro-3*H*-2,3-benzodiazepin (Beispiel **47A**) wurden bei RT in 50 ml THF gelöst, 1.68 g (8.31 mmol) 4-Nitrophenylchloroformat (CAS [7693-46-1]) zugetropft und 1 h bei RT gerührt. Die klare gelbe Lösung wurde dabei langsam trüb. Es wurden 20.8 ml (41.6 mmol) 2M Lösung von Methylamin in THF zugetropft und die Mischung 5 h bei 60°C gerührt. Man ließ langsam auf RT kommen, engte am Rotationsverdampfer ein, verteilte die Mischung zwischen Wasser und Ethylacetat und trennte die Phasen. Die wässrige Phase wurde mit Ethylacetat extrahiert. Die vereinigten organischen Phasen wurden mit ges. Natriumchloridlösung gewaschen, mit Natriumsulfat getrocknet und das Lösemittel am Rotationsverdampfer entfernt.

Da die Umsetzung nicht vollständig war (UPLC/MS-Kontrolle), wurde die Reaktion mit dem erhaltenen Rohprodukt/Zwischenprodukt/Ausgangsmaterial-Gemisch erneut analog durchgeführt, um vollständigen Umsatz zu erhalten. Das dann erhaltene Rohprodukt wurde flashchromatographisch gereinigt (SiO₂, Hexan/Ethylacetat). Man erhielt 1.90 g (100% d. Th.) des gewünschten Produkts als gelben Schaum.
LCMS (Methode 2): Rₜ = 1,33 min; m/z = 432;434 (Br-Isotopenmuster, M+H)⁺
¹H-NMR (400MHz, DMSO-*d*₆): δ = 0,92 (d, 3H), 2,64 (d, 3H), 2,67 (dd, 1H), 2,91 (dd, 1H), 3,53 (s, 3H), 3,80 (s, 3H), 5,03 - 5,11 (m, 1H), 6,47 (s, 1H), 6,60 (q, 1H), 6,98 (s, 1H), 7,56 (s, 4H).

### Enantiomerentrennung von Beispiel 49A

### (±)-1-(4-Bromphenyl)-7,8-dimethoxy-N,4-dimethyl-4,5-dihydro-3H-2,3-benzodiazepin-3-carboxamid

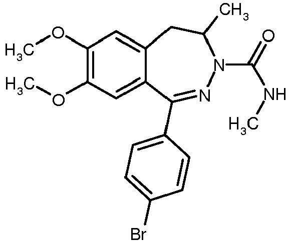

19,9 g der nach der Vorschrift in Beispiel **49A** hergestellten Verbindung wurden mittels chiraler präparativer HPLC unter folgenden Bedingungen in die Enantiomere getrennt:
System: SFC Prep 400; Säule: Chiralpak AZ-H 5µm 250x50 mm; Eluent: CO₂ / Isopropanol 75:25 (v/v); Fluss: 300 ml/min; Temperatur: 38°C; Druck 80 bar; Lösung: 5 g / 100 ml Methanol / Acetonitril 50:50 (v/v); Detektion: UV 220 nm.

### Beispiel 49.1A: (4R)-1-(4-Bromphenyl)-7,8-dimethoxy-N,4-dimethyl-4,5-dihydro-3H-2,3-benzodiazepin-3-carboxamid

9,29 g, hellgelber Feststoff, HPLC (Methode T): Rₜ = 3,29 min, Reinheit > 99%
Drehwert: [α]_{D}²⁰ = -89,3° (c = 1,00; Methanol)

### Beispiel 49.2A: (4S)-1-(4-Bromphenyl)-7,8-dimethoxy-N,4-dimethyl-4,5-dihydro-3H-2,3-benzodiazepin-3-carboxamid

9,9 g, hellgelber Feststoff, HPLC (Methode T): Rₜ = 4,55 min, Reinheit 96%
Drehwert: [α]_{D}²⁰ = +81,3° (c = 1,00; Methanol)

### Alternative Herstellungsvorschrift für Beispiel 49A

260 g (415.70 mmol) roh eingesetztes (±)-1-(4-Bromphenyl)-7,8-dimethoxy-4-methyl-4,5-dihydro-3*H*-2,3-benzodiazepin (vgl. Beispiel **47A)** wurden bei RT in 2000 ml THF gelöst, 167.6 g (831.4 mmol) 4-Nitrophenylchloroformat (CAS [7693-46-1]) zugegeben und danach 64.47 g (498.8 mmol) Diisopropylethylamin zugetropft. Wegen einer auftretenden Wäremetönung wurde mit einem Eisbad gegengekühlt. Der Ansatz rührte 6 h bei RT.Danach wurden 2078 ml (4157 mmol) einer 2 molaren Lösung von Methylamin in THF unter Eisbadkühlung zugetropft und danach der Ansatz für 16 h bei RT gerührt. Die gelbe Suspension wurde mit 2 molarer Natronlauge versetzt und danach dreimal mit je 700 ml Ethylacetat extrahiert. Die vereinigten organischen Phasen wurden viermal mit je 200 ml 2 molarer Natronlauge gewaschen, mit Natriumsulfat getrocknet und das Lösemittel am Rotationsverdampfer entfernt. Das erhaltene Rohprodukt wurde chromatographisch gereinigt. Man erhielt 147.5 g (51% d. Th. über 2 Stufen) des gewünschten Produkts.

Analog Beispiel **49A** wurden folgende Verbindungen aus den entsprechenden 4,5-Dihydro-3*H*-2,3-benzodiazepinen hergestellt:

| Nr | Struktur | Name | Analytische Daten |
|---|---|---|---|
| **50A** | 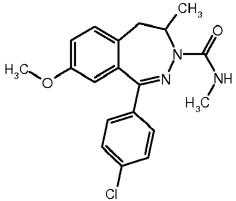 | (±)-1-(4-Chlorphenyl)-8-methoxy-*N*,4-dimethyl-4,5-dihydro-3*H*-2,3-benzodiazepin-3-carboxamid | ¹H-NMR (300MHz, CDCl₃): δ = 0,92 (d, 3H), 2,86 (dd, 1H), 2,88 (d, 3H), 3,09 (dd, 1H), 3,69 (s, 3H), 5,43 (m, 1H), 6,59 (d, 1H), 6,88 (dd, 1H), 7,13 (d, 1H), 7,36 (d, 2H), 7,42 (d, 2H). |
| | | | LCMS (Methode 3): Rₜ = 1,35 min; m/z = 358;360 (Cl-Isotopenmuster, M+H)⁺ |
| **51A** | 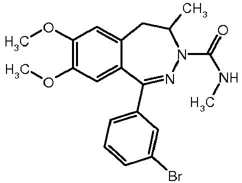 | (±)-1-(3-Bromphenyl)-7,8-dimethoxy-*N*,4-dimethyl-4,5-dihydro-3*H*-2,3-benzodiazepin-3-carboxamid | ¹H-NMR (500MHz, CDCl₃): δ = 0,95 (d, 3H), 2,86 (dd, 1H), 2,90 (d, 3H), 3,12 (dd, 1H), 3,66 (s, 3H), 3,93 (s, 3H), 5,48 (m, 1H), 6,50 (m, 1H), 6,54 (s, 1H), 6,71 (s, 1H), 7,26 (dd, 1H), 7,39 (dbr, 1H), 7,52 (dbr, 1H), 7,64 (m, 1H). |
| | | | LCMS (Methode 3): Rₜ = 1,27 min; m/z = 432; 434 (M+H, Br-Isotopenmuster)⁺ |

### Beispiel 52A

### (±)-8-Brom-1-(4-chlorphenyl)-N,4-dimethyl-4,5-dihydro-3H-2,3-benzodiazepin-3-carboxamid

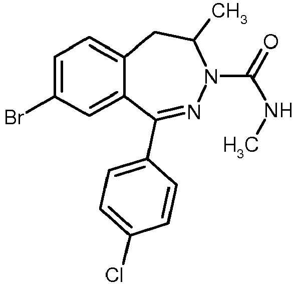

100 mg (286 µmol) 8-Brom-1-(4-chlorphenyl)-4-methyl-4,5-dihydro-3*H*-2,3-benzodiazepin (Beispiel **36A)** wurden bei RT in 5 ml THF gelöst, 58 mg (286 µmol) 4-Nitrophenylchloroformat (CAS [7693-46-1]) zugegeben und 2 h bei RT gerührt. Zu der klaren orangefarbenen Lösung wurden 1,43 ml (2,86 mmol) 2M Lösung von Methylamin in THF zugetropft und die Mischung 16 h bei 60°C gerührt. Man ließ auf RT kommen, verteilte die Mischung zwischen Wasser und Ethylacetat und trennte die Phasen. Die wässrige Phase wurde mit Ethylacetat extrahiert. Die vereinigten organischen Phasen wurden mit Wasser und ges. Natriumchloridlösung gewaschen, mit Natriumsulfat getrocknet und das Lösemittel am Rotationsverdampfer entfernt. Das Rohprodukt wurde flashchromatographisch gereinigt (SiO₂, Hexan/Ethylacetat). Man erhielt 92 mg (79% d. Th.) des gewünschten Produkts als orangefarbenen Schaum.
LCMS (Methode 1): Rₜ = 1,49 min; m/z = 406;408;410 (Br-Cl-Isotopenmuster, M+H)⁺
¹H-NMR (300MHz, CDCl₃): δ = 0,91 (d, 3H), 2,89 (d, 3H), 2,90 (dd, 1H), 3,09 (dd, 1H), 5,43 - 5,53 (m, 1H), 6,43 - 6,51 (m, 1H), 7,11 (d, 1H), 7,21 (d, 1H), 7,39 (s, 4H), 7,45 (dd, 1H).

### Enantiomerentrennung

320 mg (±)-8-Brom-1-(4-chlorphenyl)-*N*,4-dimethyl-4,5-dihydro-3*H*-2,3-benzodiazepin-3-carboxamid wurden mittels chiraler präparativer HPLC unter folgenden Bedingungen in die Enantiomere getrennt:
System: Agilent: Prep 1200, 2xPrep Pump G1361A, DLA G2258A, MWD G1365D, Prep FC G1364B; Säule: Chiralpak IC 5µm 250x20 mm; Eluent: Hexan / Ethanol 95:05 (v/v); Fluss: 30 ml/min; Temperatur: RT; Lösung: 320 mg / 3,4 ml MeOH; Injektion: 2 x 200µl, 5 x 400µl; Detektion: UV 254 nm.

### Beispiel 52.1A:

116 mg, farbloser Feststoff, HPLC (Methode C): Rₜ = 2,6 min, Reinheit > 99%
Drehwert: [α]_{D}²⁰ = -111,2° ± 0,08° (c = 1,00; Methanol)

### Beispiel 52.2A:

123 mg, farbloser Feststoff, HPLC (Methode C): Rₜ = 3,1 min, Reinheit 99,5%
Drehwert: [α]_{D}²⁰ = +110,2° ± 0,07° (c = 1,00; Methanol)

### Beispiel 53A

### (±)-1-(4-Aminophenyl)-8-methoxy-N,4-dimethyl-4,5-dihydro-3H-2,3-benzodiazepin-3-carboxamid

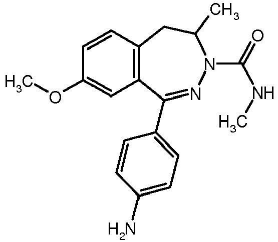

Die Herstellung der Titelverbindung ist in WO97/28135 A1 (Schering AG) als Beispiel 5 beschrieben.
UPLC/MS (Methode 3): Rₜ = 0,92 min; m/z = 339 (M+H)⁺
¹H-NMR (300MHz, DMSO-*d*₆): δ = 1,07 (d, 3H), 2,37 (dd, 1H), 2,60 (d, 3H), 2,81 (dd 1H), 3,69 (s, 3H), 4,74 (m, 1H), 5,70 (sbr, 2H), 6,19 (qbr, 1H), 6,53 (d, 1H), 6,57 (d, 2H), 6,98 (dd, 1H), 7,28 (d, 1H), 7,45 (d, 2H).

### Enantiomerentrennung (präparative Methode III)

### Beispiel 53.1A: (4R)-1-(4-Aminophenyl)-8-methoxy-N,4-dimethyl-4,5-dihydro-3H-2,3-benzodiazepin-3-carboxamid

1,64 g, gelber Feststoff, HPLC (Methode C): Rₜ = 5,05 min, Reinheit 99%, [α]_{D}²⁰ = -637,8° ± 0,12° (c = 1,040; MeOH)

### Beispiel 53.2A: (4S)-1-(4-Aminophenyl)-8-methoxy-N,4-dimethyl-4,5-dihydro-3H-2,3-benzodiazepin-3-carboxamid

1,71 g, gelber Feststoff, HPLC (Methode C): Rₜ = 6,75 min, Reinheit 95%, [α]_{D}²⁰ = +604,9° ± 0,10° (c = 1,030; MeOH)

### Beispiel 54A

### (4S)-1-(4-Bromphenyl)-8-methoxy-N,4-dimethyl-4,5-dihydro-3H-2,3-benzodiazepin-3-carboxamid

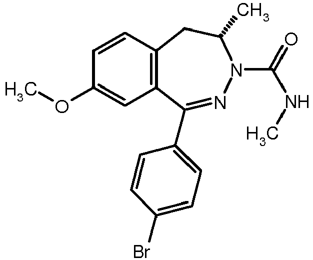

1000 mg (2,96 mmol) (4*S*)-1-(4-Aminophenyl)-8-methoxy-*N*,4-dimethyl-4,5-dihydro-3*H*-2,3-benzodiazepin-3-carboxamid (Beispiel **53.2A)** wurden in 20 ml Acetonitril gelöst, mit 528 mg (2,36 mmol) Kupfer(II)bromid und 351 µl (2,96 mmol) *tert*-Butylnitrit versetzt, und 2 Stunden bei Raumtemperatur gerührt. Der Ansatz wurde auf 1 M wässrige Salzsäure gegossen und mit Ethylacetat extrahiert. Die organische Phase wurde mit gesättigter wässriger Natriumchloridlösung gewaschen, über Natriumsulfat getrocknet, filtriert, und zur Trockne eingeengt. Der Rückstand wurde flashchromatographisch gereinigt. Man erhielt 505 mg (42% d. Th.) der Titelverbindung.
UPLC/MS (Methode 3): Rₜ = 1,37 min; m/z = 402;404 (Br-Isotopenmuster, M+H)⁺
¹H-NMR (300MHz, CDCl₃): δ = 0,91 (d, 3H), 2,85 (dd, 1H), 2,88 (d, 3H), 3,09 (dd, 1H), 3,69 (s, 3H), 5,44 (m, 1H), 6,48 (m, 1H), 6,59 (d, 1H), 6,87 (dd, 1H), 7,13 (d, 1H), 7,35 (d, 2H), 7,52 (d, 2H).

### Beispiel 55A

### (±)-1-(4-Chlorphenyl)-8-hydroxy-N,4-dimethyl-4,5-dihydro-3H-2,3-benzodiazepin-3-carboxamid

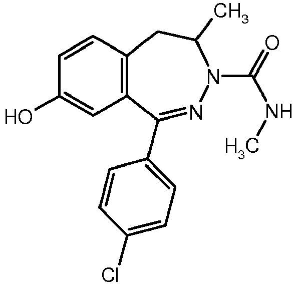

Zu einer Lösung von 2,27 g (6,34 mmol) (±)-1-(4-Chlorphenyl)-8-methoxy-*N*,4-dimethyl-4,5-dihydro-3*H*-2,3-benzodiazepin-3-carboxamid (Beispiel **50A)** in 100 ml Dichlormethan wurden unter Eisbadkühlung langsam 38 ml (38,1 mmol) Bortribromid getropft. Das Gemisch wurde 16 Stunden bei Raumtemperatur gerührt. Der Ansatz wurde auf Eiswasser gegossen und mit Dichlormethan extrahiert. Die organische Phase wurde über Natriumsulfat getrocknet, filtriert, und zur Trockne eingeengt. Man erhielt 2,16 g (99% d. Th.) der Titelverbindung.
UPLC/MS (Methode 3): Rₜ = 1,12 min; m/z = 344; 346 (Cl-Isotopenmuster, M+H)⁺
¹H-NMR (300MHz, DMSO-*d*₆): δ = 0,90 (d, 3H), 2,63 (dd, 1H), 2,65 (d, 3H), 2,89 (dd, 1H), 5,02 (m, 1H), 6,63 (m, 1H), 6,37 (d, 1H), 6,79 (dd, 1H), 7,16 (d, 1H), 7,47 (d, 2H), 7,61 (d, 2H), 9,53 (sbr, 1H).

### Beispiel 56A

### 1-(3-Brom-4-fluorphenyl)-6,7-dimethoxy-3-methyl-3,4-dihydro-1H-2-benzopyran

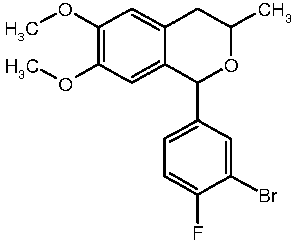

Die Herstellung erfolgte analog Beispiel **41A** ausgehend von kommerziell erhältlichem 3-Brom-4-fluorbenzaldehyd (CAS [77771-02-9]).
LCMS (Methode 3): Rₜ = 1,44 min; m/z = 381; 383 (Br-Isotopenmuster, M+H)⁺

### Beispiel 57A

### 1-[2-(3-Brom-4-fluorbenzoyl)-4,5-dimethoxyphenyl]propan-2-on

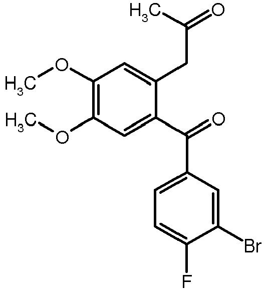

Die Herstellung erfolgte analog Beispiel **43A.**
LCMS (Methode 3): Rₜ = 1,25 min; m/z = 395; 397 (Br-Isotopenmuster, M+H)⁺

### Beispiel 58A

### 1-(3-Brom-4-fluorphenyl)-7,8-dimethoxy-4-methyl-5H-2,3-benzodiazepin

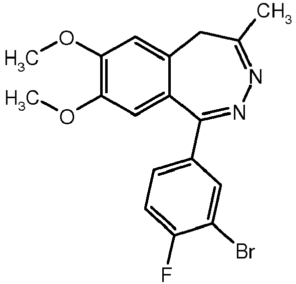

Die Herstellung erfolgte analog Beispiel **45A.**
LCMS (Methode 3): Rₜ = 1,21 min; m/z = 381; 383 (Br-Isotopenmuster, M+H)⁺
¹H-NMR (400MHz, CDCl₃): δ = 2,16 (s, 3H), 2,99 (d, 1H), 3,29 (d, 1H), 3,77 (s, 3H), 3,98 (s, 3H), 6,74 (s, 2H), 7,16 (dd, 1H), 7,62 (ddd, 1H), 7,94 (dd, 1H).

### Beispiel 59A

### (±)-1-(3-Brom-4-fluorphenyl)-7,8-dimethoxy-4-methyl-4,5-dihydro-3H-2,3-benzodiazepin

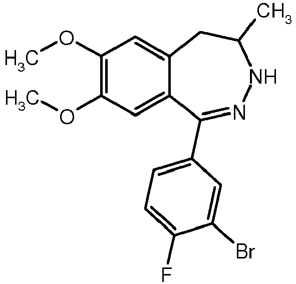

Die Herstellung erfolgte analog Beispiel **47A.**
LCMS (Methode 3): Rₜ = 1,03 min; m/z = 393; 395 (Br-Isotopenmuster, M+H)⁺
¹H-NMR (400MHz, CDCl₃): δ = 1,29 (d, 3H), 2,61 (dd, 1H), 2,89 (dd, 1H), 3,72 (s, 3H), 3,95 (s, 3H), 4,12 (m, 1H), 6,57 (s, 1H), 6,77 (s, 1H), 7,10 (dd, 1H), 7,45 (ddd, 1H), 7,81 (dd, 1H).

### Beispiel 60A

### (±)-1-(3-Brom-4-fluorphenyl)-7,8-dimethoxy-N,4-dimethyl-4,5-dihydro-3H-2,3-benzodiazepin-3-carboxamid

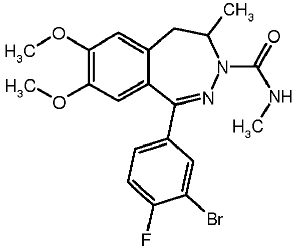

Die Herstellung erfolgte analog Beispiel **49A.**
LCMS (Methode 3): Rₜ = 1,31 min; m/z = 450; 452 (Br-Isotopenmuster, M+H)⁺
¹H-NMR (400MHz, CDCl₃): δ = 0,95 (d, 3H), 2,86 (dd, 1H), 2,90 (d, 3H), 3,10 (dd, 1H), 3,67 (s, 3H), 3,93 (s, 3H), 5,48 (m, 1H), 6,44 (m, 1H), 6,52 (s, 1H), 6,71 (s, 1H), 7,14 (dd, 1H), 7,39 (ddd, 1H), 7,69 (dd, 1H).

Analog Beispiel **8A** wurde aus 4-Nitrobenzaldehyd folgende Verbindung hergestellt:

| Nr | Struktur | Name | Analytische Daten |
|---|---|---|---|
| **61A** | 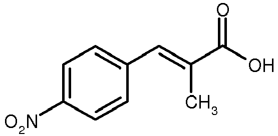 | 2-Methyl-3-(4-nitrophenyl)acryl-säure | ¹H-NMR (400MHz, DMSO-*d*₆): δ = 2,01 (s, 3H), 7,62 (s, 1H), 7,70 (d, 2H), 8,23 (d, 2H), 12,8 (s, br, 1H). |
| | | | LCMS (Methode 2): Rₜ = 0,99 min; m/z [ES-]= 206 (M-H)⁻ |

### Beispiel 62A

### (±)-3-(4-Aminophenyl)-2-methylpropansäure

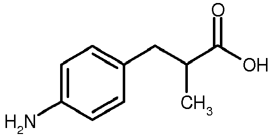

41 g (0,2 mol) 2-Methyl-3-(4-nitrophenyl)acrylsäure wurden analog Beispiel 10A reduziert. Man erhielt 21 g (90%) der gewünschten Verbindung als gelbes kristallisierendes Öl.
LCMS (Methode 2): Rₜ = 0.48 min; m/z = 180 (M+H)⁺
¹H-NMR (400MHz, CDCl₃): δ = 0, 95 (d, 3H), 2,36 (dd, 1H), 2,42 - 2,45 (m, 1H), 2,70 (dd, 1H), 6,43 (d, 2H), 6.78 (d, 2H).

### Beispiel 63A

### (±)-6-Amino-2-methylindan-1-on

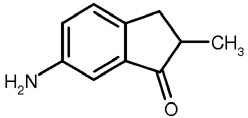

38 g (11,1 mmol) (±)-3-(4-Aminophenyl)-2-methylpropansäure (Beispiel **62A)** wurden mit 310 g Polyphosphorsäure versetzt und die Mischung 7 h bei 150°C mit einem Druckluftrührers gerührt. Nach dem Abkühlen wurde die Mischung vorsichtig portionsweise mit Wasser verdünnt und anschließend unter Eiskühlung mit 32%iger Natronlauge alkalisiert (pH = 10). Die Mischung wurde mit Dichlormethan extrahiert und die vereinigten organischen Phasen mit Natriumsulfat getrocknet. Die Lösemittel wurden am Rotationsverdampfer entfernt und das Rohprodukt (26 g) direkt weiter umgesetzt.
LCMS (Methode 2): Rₜ = 0,69 min; m/z = 162; 203 (M+H; M+ACN+H)⁺
¹H-NMR (300MHz, DMSO-*d*₆): δ = 1,11 (d, 3H), 2,53 - 2,60 (m, 1H), 2,68 (dd, 1H), 3,15 (dd, 1H), 5.25 (s, br, 2H), 6,71 (d, 1H), 6,88 (dd, 1H), 7,16 (d, 1H).

### Beispiel 64A

### (±)-tert-Butyl-(2-methyl-3-oxo-2,3-dihydro-1H-inden-5-yl)carbamat

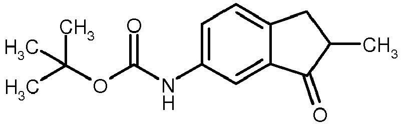

15,0 g (93,0 mmol) (±)-6-Amino-2-methylindan-1-on wurden in 450 ml Dichlormethan gelöst, 10 min im Eisbad gerührt, dann 21,3 g (97,7 mmol) Di-*tert*-butyldicarbonat zugegeben und weitere 16 h bei RT gerührt. Die Mischung wurde auf Wasser gegeben, mit Dichlormethan extrahiert, die vereinigten organischen Phasen mit ges. Natriumchloridlösung gewaschen und die Lösemittel am Rotationsverdampfer entfernt. Das Rohprodukt wurde chromatographisch gereinigt (SieO₂, Hexan/Ethylacetat 0-30%). Man erhielt 13,3 g (50% d. Th.) als gelben Schaum.
LCMS (Methode 2): Rₜ = 1,21 min; m/z = 262; 303 (M+H)⁺; (M+ACN+H)⁺
¹H-NMR (400MHz, DMSO-*d*₆): δ = 1,14 (d, 3H), 1,45 (s, 9H), 2,58 (dd, 1H), 2,61 - 2,70 (m, 1H), 3,25 (dd, 1H), 7,40 (d, 1H), 7,63 (dd, 1H), 7,77 (d, 1H), 9.51 (s, 1H).

Analog Beispiel **15A** wurden aus den entsprechenden Carbonsäuren folgende Indanone hergestellt:

| Nr | Struktur | Name | Analytische Daten |
|---|---|---|---|
| **65A** | 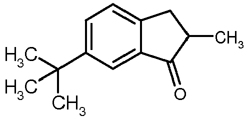 | (±)-6-*tert*-Butyl-2-methylindan-1-on | ¹H-NMR (400 MHz, CDCl₃): δ = 1,31 (d, 3H), 1,34 (s, 9H), 2,65-2,77 (m, 2H), 3,31-3,40 (m, 1H), 7,39 (d, 1H), 7,66 (dd, 1H), 7,78 (d, 1H). |
| | | | LCMS (Methode 2): Rₜ = 1,35 min; m/z = 244 (M+CH₃CN)⁺. |
| **66A** | 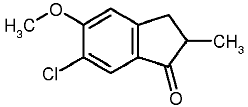 | (±)-6-Chlor-5-methoxy-2-methylindan-1-on | ¹H-NMR (300 MHz, CDCl₃): δ = 1,29 (d, 3H), 2,61-2,78 (m, 2H), 3,28-3,40 (m, 1H), 3,98 (s, 3H), 6,92 (s, 1H), 7,75 (s, 1H). |
| | | | LCMS (Methode 2): Rₜ = 1,10 min; m/z = 211 (M+H)⁺. |

Analog Beispiel **17A** wurden aus den entsprechenden Indanonen durch Reaktion mit 4-Chlorphenylmagnesiumbromid und anschließender Wasser-Eliminierung mit Hilfe von 4-Toluolsulfonsäure folgende Verbindungen hergestellt:

| Nr | Struktur | Name | Analytische Daten |
|---|---|---|---|
| **67A** | 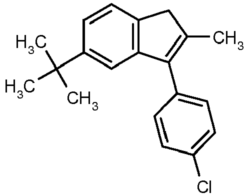 | 5-*tert*-Butyl-3-(4-chlorphenyl)-2-methyl-1*H*-inden | ¹H-NMR (300 MHz, CDCl₃): δ = 1,31 (s, 9H), 2,11 (s, 3H), 3,41 (s, 2H), 7,18-7,25 (m, 2H), 7,31-7,41 (m, 3H), 7,46 (d, 2H). |
| | | | LCMS (Methode 3): Rₜ = 1,0 min; m/z = 499 (M+H)⁺. |
| **68A** | 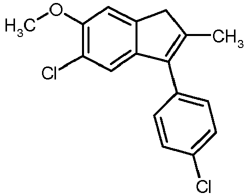 | 5-Chlor-3-(4-chlorphenyl)-6-methoxy-2-methyl-1*H-*inden | ¹H-NMR (400 MHz, CDCl₃): δ = 2,10 (s, 3H), 3,42 (s, 2H), 3,93 (s, 3H), 7,09 (s, 1H), 7,14 (s, 1H), 7,31 (d, 2H), 7,44 (d, 2H). |
| | | | LCMS (Methode 2): Rₜ = 1,68 min; m/z = 305 (M+H)⁺. |
| **69A** | 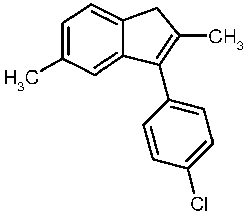 | 3-(4-Chlorphenyl)-2,5-dimethyl-1*H*-inden | ¹H-NMR (300 MHz, CDCl₃): δ = 2,11 (s, 3H), 2,35 (s, 3H), 3,42 (s, 2H), 6,96-7,02 (m, 2H), 7,31 (d, 1H), 7,33 (d, 2H), 7,45 (d, 2H). |
| | | | LCMS (Methode 2): Rₜ = 1,70 min; m/z = 255 (M+H)⁺. |
| **70A** | 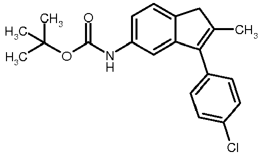 | *tert*-Butyl-[3-(4-chlorphenyl)-2-methyl-1*H*-inden-5-yl]carbamat | LCMS (Methode 2): Rₜ = 1,64 min; m/z = 256 (M+H)⁺ |

Analog Beispiel **25A** wurden folgende Verbindungen aus den entsprechenden 1*H*-Indenen hergestellt:

| Nr | Struktur | Name | Analytische Daten |
|---|---|---|---|
| **71A** | 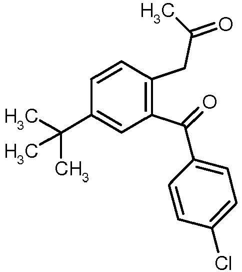 | 1-[4-*tert*-Butyl-2-(4-chlorbenzoyl)phenyl]-propan-2-on | ¹H-NMR (400 MHz, CDCl₃): δ = 1,28 (s, 9H), 2,18 (s, 3H), 3,96 (s, 2H), 7,19 (d, 1H), 7,38 (d, 1H), 7,45 (d, 2H), 7,50 (dd, 1H), 7,77 (d, 2H). |
| | | | LCMS (Methode 2): Rₜ = 1,54 min; m/z = 329 (M+H)⁺. |
| **72A** | 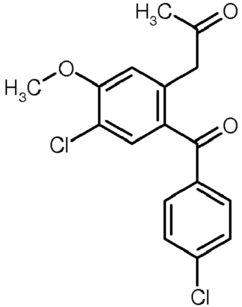 | 1-[4-Chlor-2-(4-chlorbenzoyl)-5-methoxyphenyl]propan -2-on | ¹H-NMR (400 MHz, CDCl₃): δ = 2,24 (s, 3H), 3,98 (s, 3H), 4,01 (s, 2H), 6,80 (s, 1H), 7,43 (s, 1H), 7,45 (d, 2H), 7,70 (d, 2H). |
| | | | LCMS (Methode 2): Rₜ = 1,39 min; m/z = 337; 339 (Cl-Isotopenmuster, M+H)⁺. |
| **73A** | 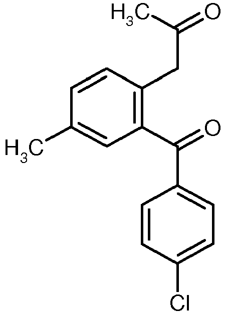 | 1-[2-(4-Chlorbenzoyl)-4-methylphenyl]propan-2-on | ¹H-NMR (400 MHz, CDCl₃): δ = 2,17 (s, 3H), 2,34 (s, 3H), 3,93 (s, 2H), 7,15 (d, 1H), 7,17 (s, br, 1H), 7,29 (dd, 1H), 7,44 (d, 2H), 7,76 (d, 2H). |
| | | | LCMS (Methode 2): Rₜ = 1,37 min; m/z = 287 (M+H)⁺. |
| **74A** | 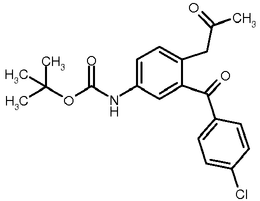 | *tert*-Butyl-[3-(4-chlorbenzoyl)-4-(2-oxopropyl)phenyl] carb amat | ¹H-NMR (300MHz, DMSO-*d*₆): δ = 1,39 (s, 9H), 2,01 (s, 3H), 3,83 (s, 2H), 7,20 (d, 1H), 7,44 (d, 1H), 7,51 - 7,57 (m, 1H), 7,59 (d, 2H), 7,69 (d, 2H), 9,42 (s, 1H). |
| | | | LCMS (Methode 2): Rₜ = 1,39 min; m/z = 388 (M+H)⁺ |

### Beispiel 75A

### 8-tert-Butyl-1-(4-chlorphenyl)-4-methyl-5H-2,3-benzodiazepin

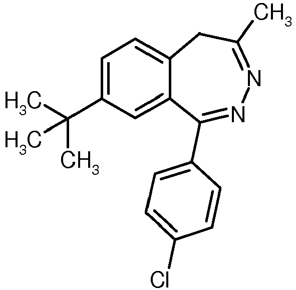

7,0 g (21,29 mmol) 1-[4-*tert*-Butyl-2-(4-chlorbenzoyl)phenyl]propan-2-on (Beispiel **71A**) wurden mit 7,46 g (149 mmol) Hydrazinhydrat in 80,6 ml Ethanol 72 h bei RT gerührt. Die Reaktionslösung wurde auf Wasser gegeben, mit 1M Natronlauge alkalisiert und mit Dichlormethan extrahiert. Die vereinigten organischen Phasen wurden mit Natriumsulfat getrocknet und das Lösemittel am Rotationsverdampfer entfernt. Man erhielt 6,5 g (94% d. Th.) des Rohproduktes, das ohne weitere Aufreinigung im nächsten Reaktionsschritt umgesetzt wurde. Ein Teil des Rückstandes wurde mittels präparativer HPLC gereinigt.
LCMS (Methode 2): Rₜ = 1,54 min; m/z = 325 (M+H)⁺.
¹H-NMR (300 MHz, CDCl₃): δ = 1,26 (s, 9H), 2,15 (s, 3H), 3,04 (d, 1H), 3,33 (d, 1H), 7,21 (d, 1H), 7,31 (d, 1H), 7,38 (d, 2H), 7,55 (dd, 1H), 7,64 (d, 2H).

### Beispiel 76A

### tert-Butyl-[1-(4-chlorphenyl)-4-methyl-5H-2,3-benodiazepin-8-yl]carbamat

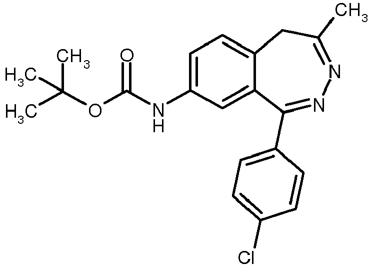

5,10 g (13,1 mmol) *tert*-Butyl-[3-(4-chlorbenzoyl)-4-(2-oxopropyl)phenyl]carbamat (Beispiel 74A) und 3,49 g (69,7 mmol) Hydrazinhydrat wurden zusammen in 100 ml Ethanol 1,5 h bei 100°C erhitzt. Danach wurde die Mischung abgekühlt, auf Wasser gegeben und mit Ethylacetat extrahiert. Die vereinigten organischen Phasen wurden mit Wasser und ges. Natriumchloridlösung gewaschen und mit Natriumsulfat getrocknet und eingeengt. Man erhielt 5 g Rohprodukt, das ohne weitere Reinigung direkt weiter verwendet wurde.
LCMS (Methode 1): Rₜ = 1,37 min; m/z = 384 (M+H)⁺
¹H-NMR (400MHz, CDCl₃): δ = 1,48 (s, 9H), 2,13 (s, 3H), 3,03 (d, 1H), 3,30 (d, 1H), 6,48 (s, br, 1H), 7,11 (d, 1H), 7,22 (d, 1H), 7,37 (d, 2H), 7,62 (d, 2H), 7,73 (d, 1H).

Analog Beispiel **75A** wurden folgende Verbindungen aus den entsprechenden Diketoverbindungen hergestellt:

| Nr | Struktur | Name | Analytische Daten |
|---|---|---|---|
| **77A** | 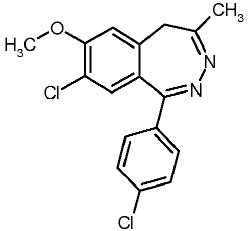 | 8-Chlor-1-(4-chlorphenyl)-7-methoxy-4-methyl-5*H-*2,3-benzodiazepin | ¹H-NMR (400 MHz, CDCl₃): δ = 2,25 (s, 3H), 3,12 (d, 1H), 3,41 (d, 1H), 4,01 (s, 3H), 6,82 (s, 1H), 7,32 (s, 1H), 7,42 (d, 2H), 7,62 (d, 2H). |
| | | | LCMS (Methode 2): Rₜ = 1,37 min; m/z = 333; 335 (Cl-Isotopenmuster, M+H)⁺. |
| **78A** | 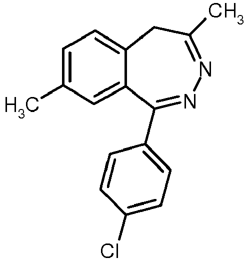 | 1-(4-Chlorphenyl)-4,8-dimethyl-5*H*-2,3-benzodiazepin | ¹H-NMR (300 MHz, CDCl₃): δ = 2,13 (s, 3H), 2,33 (s, 3H), 3,03 (d, 1H), 3,31 (d, 1H), 7,10 (s, br, 1H), 7,17 (d, 1H), 7,34 (dd, 1H), 7,38 (d, 2H), 7,62 (d, 2H). |
| | | | LCMS (Methode 2): Rₜ = 1,36 min; m/z = 283 (M+H)⁺. |

Analog Beispiel **47A** wurden folgende Verbindungen hergestellt:

| Nr | Struktur | Name | Analytische Daten |
|---|---|---|---|
| **79A** | 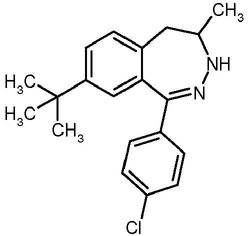 | (±)-8-*tert*-Butyl-1-(4-chlorphenyl)-4-methyl-4,5-dihydro-3*H*-2,3-benzodiazepin | LCMS (Methode 2): Rₜ = 1,42 min; m/z = 327 (M+H)⁺. |
| **80A** | 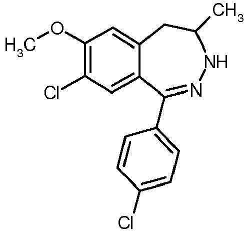 | (±)-8-Chlor-1-(4-chlorphenyl)-7-methoxy-4-methyl-4,5-dihydro-3*H*-2,3-benzodiazepin | LCMS (Methode 2): Rₜ = 1,25 min; m/z = 335; 337 (Cl-Isotopenmuster, M+H)⁺. |
| **81A** | 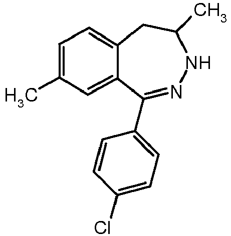 | (±)-1-(4-Chlorphenyl)-4,8-dimethyl-4,5-dihydro-3*H*-2,3-benzodiazepin | ¹H-NMR (300 MHz, CDCl₃): δ = 1,27 (d, 3H), 2,28 (s, 3H), 2,59 (dd, 1H), 2,92 (dd, 1H), 4,06-4,20 (m, 1H), 6,89 (s, 1H), 7,16 (s, br, 2H), 7,34 (d, 2H), 7,51 (d, 2H). |
| | | | LCMS (Methode 2): Rₜ= 1,17 min; m/z = 285 (M+H)⁺. |
| **82A** | 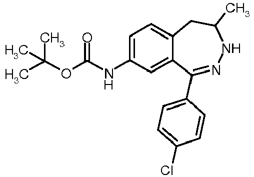 | (±)-tert-Butyl-[1-(4-chlorphenyl)-4-methyl-4,5-dihydro-3*H*-2,3-benzodiazepin-8-yl]carbamat | ¹H-NMR (400MHz, DMSO-*d*₆): δ = 1,60 (d, 3H), 1,37 (s, 9H), 2,58 (dd, 1H), 2,82 (dd, 1H), 3,75 - 3,81 (m, 1H), 7,07 (d, 1H), 7,09 (d, 1H), 7,14 (d, 1H), 7,33 - 7,38 (m, 4H), 9,16 (s, br, 1H). |
| | | | LCMS (Methode 2): Rₜ = 1,23 min; m/z = 386 (M+H)⁺ |

Analog Beispiel **49A** wurden folgende Verbindungen aus den entsprechenden 4,5-Dihydro-3*H*-2,3-benzodiazepinen hergestellt:

| Nr | Struktur | Name | Analytische Daten |
|---|---|---|---|
| **83A** | 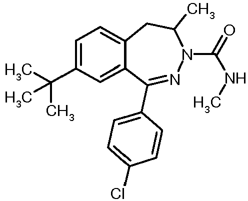 | (±)-8-*tert*-Butyl-1-(4-chlorphenyl)-*N*,4-dimethyl-4,5-dihydro-3*H*-2,3-benzodiazepin-3-carboxamid | ¹H-NMR (300 MHz, CDCl₃): δ = 0,95 (d, 3H), 1,21 (s, 9H), 2,86 (dd, 1H), 2,89 (d, 3H), 3,09 (dd, 1H), 5,36-5,49 (m, 1H), 6,43 (q, 1H), 7,08 (d, 1H), 7,15 (d, 1H), 7,32-7,40 (m, 3H), 7,41-7,47 (m, 2H). |
| | | | LCMS (Methode 2): Rₜ = 1,61 min; m/z = 384 (M+H)⁺. |
| **83.1A** | 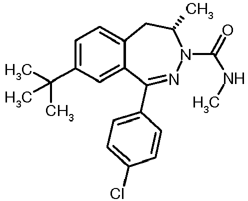 | (4*S*)-8-*tert*-Butyl-1-(4-chlorphenyl)-N,4-dimethyl-4,5-dihydro-3*H*-2,3-benzodiazepin-3-carboxamid | LCMS (Methode 2): Rₜ = 1,61 min; m/z = 384 (M+H)⁺ |
| | | | HPLC (Methode M): Rₜ = 2,48 min |
| | | | [α]_{D}²⁰ = -133,8° (c = 1,00; Chloroform) |
| **83.2A** | 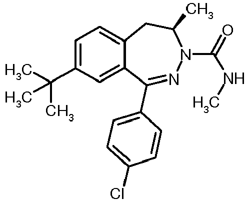 | (4*R*)-8-*tert*-Butyl-1-(4-chlorphenyl)-N,4-dimethyl-4,5-dihydro-3*H*-2,3-benzodiazepin-3-carboxamid | LCMS (Methode 2): Rt = 1,61 min; m/z = 384 (M+H)⁺ |
| | | | HPLC (Methode M): Rₜ = 2,84 min |
| | | | [α]_{D}²⁰ = 167,7° (c = 1,00; Chloroform) |
| **84A** | 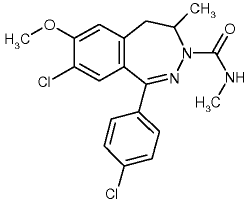 | (±)-8-Chlor-1-(4-chlorphenyl)-7-methoxy-*N*,4-dimethyl-4,5-dihydro-3*H*-2,3-benzodiazepin-3-carboxamid | ¹H-NMR (400 MHz, CDCl₃): δ = 0,97 (d, 3H), 2,88 (s, br, 3H), 2,88-2,94 (m, 1H), 3,14 (dd, 1H), 3,96 (s, 3H), 5,45-5,54 (m, 1H), 6,46 (s, br, 1H), 6,77 (s, 1H), 7,08 (s, 1H), 7,38 (s, br, 4H). |
| | | | LCMS (Methode 2): Rₜ = 1,46 min; m/z = 392; 394 (Cl-Isotopenmuster, M+H)⁺. |
| **84.1A** | 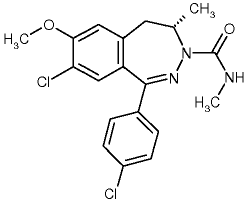 | (4*S*)-8-Chlor-1-(4-chlorphenyl)-7-methoxy-*N*,4-dimethyl-4,5-dihydro-3*H*-2,3-benzodiazepin-3-carboxamid | LCMS (Methode 2): Rₜ = 1,46 min; m/z = 392; 394 (Cl-Isotopenmuster, M+H)⁺. |
| | | | HPLC (Methode O): Rₜ = 3,67 min |
| | | | [α]_{D}²⁰ = 70,5° (c = 1,00; Methanol) |
| **84.2A** | 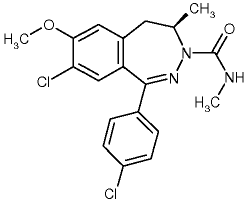 | (*4R*)-8-Chlor-1-(4-chlorphenyl)-7-methoxy-*N*,4-dimethyl-4,5-dihydro-3*H*-2,3-benzodiazepin-3-carboxamid | LCMS (Methode 2): Rₜ = 1,46 min; m/z = 392; 394 (Cl-Isotopenmuster, M+H)⁺. |
| | | | HPLC (Methode O): Rₜ = 2,47 min |
| **85A** | 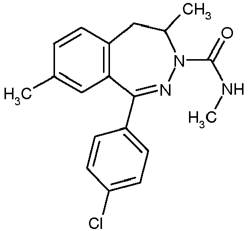 | (±)-1-(4-Chlorphenyl)-*N*,4,8-trimethyl-4,5-dihydro-3*H*-2,3-benzodiazepin-3-carboxamid | ¹H-NMR (300 MHz, CDCl₃): δ = 0,93 (d, 3H), 2,25 (s, 3H), 2,86 (dd, 1H), 2,88 (s, br, 3H), 3,09 (dd, 1H), 5,36-5,48 (m, 1H), 6,44 (s, br, 1H), 6,86 (s, br, 1H), 7,08-7,18 (m, 2H), 7,36 (d, 2H), 7,42 (d, 2H). |
| | | | LCMS (Methode 2): Rₜ = 1,47 min; m/z = 342 (M+H)⁺. |
| **85.1A** | 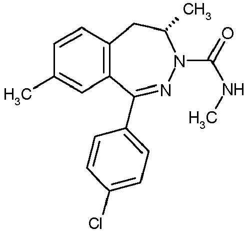 | (*4S*)*-*1-(4-Chlorphenyl)-*N*,4,8-trimethyl-4,5-dihydro-3*H*-2,3-benzodiazepin-3-carboxamid | Präp. HPLC: Methode XV Analyt. HPLC (Methode H): Rₜ = 1,82 min |
| | | | LCMS (Methode 2): Rₜ = 1,46 min; m/z = 342 (M+H)⁺. |
| | | | [α]_{D}²⁰ = 213,9° (c = 1,00; Methanol) |
| **85.2A** | 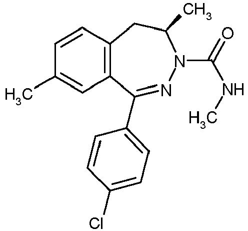 | (*4R*)*-*1-(4-Chlorphenyl)-*N*,4,8-trimethyl-4,5-dihydro-3*H*-2,3-benzodiazepin-3-carboxamid | Präp. HPLC: Methode XV Analyt. HPLC (Methode H): Rₜ = 1,32 min |
| | | | LCMS (Methode 2): Rₜ = 1,46 min; m/z = 342 (M+H)⁺. |
| | | | [α]_{D}²⁰ = -181,9° (c = 1,00; Methanol) |
| **86A** | 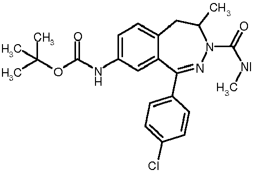 | (±)*-tert-*Butyl-[1-(4-chlorphenyl)-4-methyl-3-(methylcarbamoyl)-4,5-dihydro-3*H*-2,3-benzodiazepin-8-yl]carbamat | ¹H-NMR (400MHz, DMSO-*d*₆): δ = 0,89 (d, 3H), 1,37 (s, 9H), 2,60 - 2,66 (m, 1H), 2,63 (d, 3H), 2,90 (dd, 1H), 4,97 - 5,05 (m, 1H), 6,61 (q, 1H), 7,14 (d, 1H), 7,21 (d, 1H), 7,43 - 7,47 (m, 1H), 7,45 (d, 2H), 7,62 (d, 2H), 9,30 (s, br, 1H). |
| | | | LCMS (Methode 2): Rₜ = 1,45 min; m/z = 443 (M+H)⁺ |

### Beispiel 87A

### (±)-8-Amino-1-(4-chlorphenyl)-N,4-dimethyl-4,5-dihydro-3H-2,3-benzodiazepin-3-carboxamid

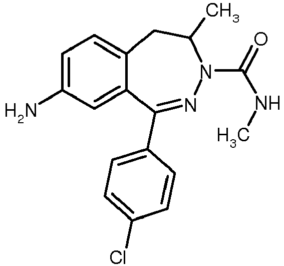

4,50 g (10,2 mmol) (±)-*tert*-Butyl-[1-(4-chlorphenyl)-4-methyl-3-(methylcarbamoyl)-4,5-dihydro-3*H*-2,3-benzodiazepin-8-yl]carbamat (Beispiel 86A) wurden in 100 ml Dichlormethan vorgelegt und bei 0°C mit 15 ml (20,3 mmol) Trifluoressigsaure versetzt und dann bei RT 4 h weiter gerührt. Die Mischung wurde vorsichtig auf 20%ige Kaliumcarbonatlösung gegeben und mit Dichlormethan extrahiert. Die vereinigten organischen Phasen wurden mit Natriumsulfat getrocknet und die Lösemittel am Rotationsverdampfer entfernt. Man erhielt 3,40 g (97% d. Th.) des gewünschten Produkts als bräunlichen Feststoff.
LCMS (Methode 2): Rₜ = 1,12 min; m/z = 343 (M+H)⁺
¹H-NMR (300MHz, DMSO-*d*₆): δ = 0,88 (d, 3H), 2,52 (dd, 1H), 2,63 (d, 3H), 2,80 (dd, 1H), 4,89 - 5,05 (m, 1H), 5,01 (s, br, 2H), 6,19 (d, 1H), 6,52 - 6,59 (m, 2H), 6,96 (d, 1H), 7,44 (d, 1H), 7,61 (d, 2H).

### Beispiel 88A

### (±)-1-[8-tert-Butyl-1-(4-chlorphenyl)-4-methyl-4,5-dihydro-3H-2,3-benzodiazepin-3-yl]ethanon

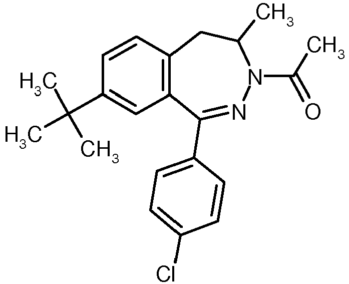

300 mg (0,918 mmol) (±)-8-*tert*-Butyl-1-(4-chlorphenyl)-4-methyl-4,5-dihydro-3*H*-2,3-benzodiazepin (Beispiel **79A)** wurden in 3,5 ml Dichlormethan suspendiert und bei RT 281 mg (2,75 mmol) Essigsäureanhydrid zugegeben. Es entstand eine klare, hellgelbe Lösung. Man rührte für 16 h, dann wurde durch Zugabe von gesättigter wässriger Natriumhydrogencarbonat Lösung pH 6 eingestellt und mit Dichlormethan zweimal extrahiert. Die vereinigten organischen Phasen wurden mit Natriumsulfat getrocknet und das Lösemittel am Rotationsverdampfer entfernt. Der Rückstand wurde mittels präparativer HPLC gereinigt. Man erhielt 210 mg (62% d. Th.) des Produktes als Feststoff.
LCMS (Methode 2): Rt = 1,61 min; m/z = 369 (M+H)+.
¹H-NMR (300 MHz, CDCl₃): δ = 1,18 (d, 3H), 1,24 (s, 9H), 2,17 (s, 3H), 2,72 (dd, 1H), 2,93 (dd, 1H), 5,24-5,37 (m, 1H), 7,06 (d, 1H), 7,21 (d, 1H), 7,36-7,45 (m, 3H), 7,59 (d, 2H).

### Beispiel 89A

### (±)-8-Acetamido-1-(4-chlorphenyl)-N,4-dimethyl-4,5-dihydro-3H-2,3-benzodiazepin-3-carboxamid

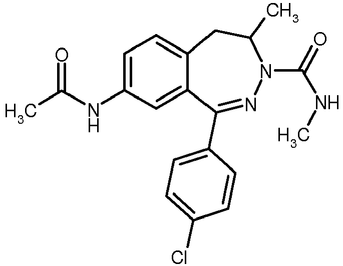

840 mg (2,45 mmol) (±)-8-Amino-1-(4-chlorphenyl)-*N*,4-dimethyl-4,5-dihydro-3*H*-2,3-benzodiazepin-3-carboxamid (Beispiel 87A) wurden in Dichlormethan gelöst und bei 0°C 0,41 ml (2,94 mmol) Triethylamin und 0,21 ml (2,94 mmol) Acetylchlorid zugegeben und 1 h gerührt. Dann wurde die Mischung auf Wasser gegeben, mit Dichlormethan extrahiert, die vereinigten organischen Phasen mit Natriumsulfat getrocknet und die Lösemittel am Rotationsverdampfer entfernt. Man erhielt 940 mg (99%) des gewünschten Produkts als bräunlichen festen Schaum.
LCMS (Methode 2): Rₜ = 1,15 min; m/z = 385 (M+H)⁺
¹H-NMR (400MHz, DMSO-*d*₆): δ = 0,87 (d, 3H), 1,93 (s, 3H), 2,64 (d, 3H), 2,69 (dd, 1H), 2,93 (dd, 1H), 5,02 - 5,10 (m, 1H), 6,66 (q, 1H), 7,22 (d, 1H), 7,25 (d, 1H), 7,45 (d, 2H), 7,60 (d, 2H), 7,60 - 7,63 (m, 1H), 9,88 (s, 1H).

### Beispiel 90A

### (±)-1-(4-Bromphenyl)-7-hydroxy-8-methoxy-N,4-dimethyl-4,5-dihydro-3H-2,3-benzodiazepin-3-carboxamid

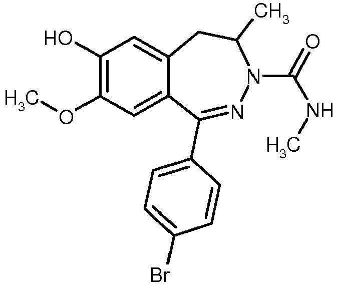

2,5 g (18,5 mmol) Aluminiumtrichlorid wurden in 40 ml Dichlormethan vorgelegt und langsam bei RT eine Lösung von 2,0 g (4,6 mmol) (±)-1-(4-Bromphenyl)-7,8-dimethoxy-*N*,4-dimethyl-4,5-dihydro-3*H*-2,3-benzodiazepin-3-carboxamid (Beispiel **49A)** in 20 ml Dichlormethan zugetropft. Die erst gelbe, dann orange Suspension wurde noch weitere 2 h bei RT gerührt. Dann wurde die Mischung auf Wasser gegeben, mit Dichlormethan extrahiert, die vereinigten organischen Phasen mit ges. Natriumchloridlösung gewaschen und mit Natriumsulfat getrocknet. Die Lösemittel wurden am Rotationsverdampfer entfernt und das Rohprodukt chromatographisch gereinigt (SiO2, Hexan/Ethylacetat 0-70%). Man erhielt 1,14 g (59% d. Th.) der gewünschten 7-Hydroxy-Verbindung als gelben Feststoff. Außerdem erhielt man 0,63 g (32% d. Th.) des regioisomeren (±)-1-(4-Bromphenyl)-8-hydroxy-7-methoxy-*N*,4-dimethyl-4,5-dihydro-3*H*-2,3-benzodiazepin-3-carboxamid.
LCMS (Methode 2): Rₜ = 1,23 min; m/z = 418/420 (M+H)⁺, Br-Isotopenmuster
¹H-NMR (300MHz, CDCl₃): δ = 0,90 (d, 3H), 2,56 (dd, 1H), 2,63 (d, 3H), 2,82 (dd, 1H), 3,54 (s, 3H), 4,96 - 5,07 (m, 1H), 6,44 (s, 1H), 6,58 (q, 1H), 6,74 (s, 1H), 7,56 (s, 4H), 9,59 (s, 1H).

### Beispiel 91A

### (±)-1-(4-Bromphenyl)-7,8-dihydroxy-N,4-dimethyl-4,5-dihydro-3H-2,3-benzodiazepin-3-carboxamid

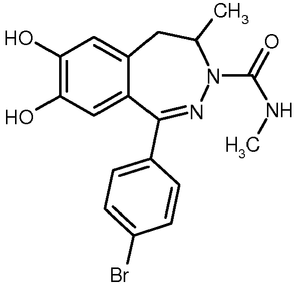

1,5 g (3,47 mmol) (±)-1-(4-Bromphenyl)-7,8-dimethoxy-*N*,4-dimethyl-4,5-dihydro-3*H*-2,3-benzodiazepin-3-carboxamid (Beispiel **49A)** wurden in 15 ml Dichlormethan vorgelegt und bei 0°C langsam 6,94 ml einer 1M Lösung von Bortribromid in Dichlormethan zugetropft und bei RT 16 h gerührt. Es wurden weitere 3,5 ml Bortribromidlösung nachgegeben und weitere 20 h gerührt. Die orangebraune Lösung wurde mit 25 ml 4M Salzsäure versetzt, 10 min gerührt und dann mit Ethylacetat extrahiert. Die vereinigten organischen Phasen wurden mit Wasser und ges. Natriumchloridlösung gewaschen und mit Natriumsulfat getrocknet. Die Lösemittel wurden am Rotationsverdampfer entfernt und das Rohprodukt chromatographisch gereinigt. Man erhielt 1,17 g (83% d. Th.) des gewünschten Produkts.
LCMS (Methode 2): Rₜ = 1,12 min; m/z = 404/406 (M+H)⁺, Br-Isotopenmuster
¹H-NMR (400MHz, DMSO-*d*₆): δ = 0,87 (d, 3H), 2,58 (dd, 1H), 2,63 (d, 3H), 2,81 (dd, 1H), 5,01 - 5,09 (m, 1H), 6,34 (s, 1H), 6,58 (q, 1H), 6,66 (s, 1H), 7,49 (d, 2H), 7,56 (d, 2H).

### Beispiel 92A

### (±)-1-(4-Bromphenyl)-7,8-bis(difluormethoxy)-N,4-dimethyl-4,5-dihydro-3H-2,3-benzodiazepin-3-carboxamid

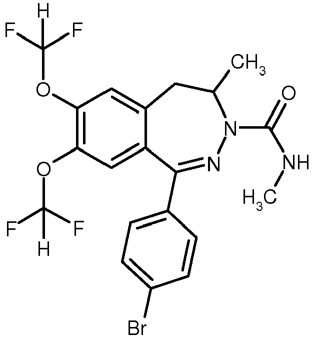

115 mg (2,28 mmol) Natriumhydroxid wurden in 7,5 ml DMF suspendiert, bei RT 200 mg (495 µmol) (±)-1-(4-Bromphenyl)-7,8-dihydroxy-*N*,4-dimethyl-4,5-dihydro-3*H*-2,3-benzodiazepin-3-carboxamid zugegeben und 15 min gerührt. Die Mischung wurde auf 0°C gekühlt und langsam 0,63 ml (4,95 mmol) Chlordifluoracetat zugegeben. Man ließ auf RT kommen und rührte dann noch 5 h bei 70°C. Die Mischung wurde nach dem Abkühlen auf Wasser gegeben. Man extrahierte mit Ethylacetat, die vereinigten organischen Phasen wurden mit ges. Natriumchloridlösung gewaschen und mit Natriumsulfat getrocknet. Das Rohprodukt wurde chromatographisch gereinigt (SiO2, Hexan/Ethylacetat 0-50-75%). Man erhielt 113 mg (17% d. Th.) des Produkts.
LCMS (Methode 2): Rₜ = 1,44 min; m/z = 504/506 (M+H)⁺, Br-Isotopenmuster

### Beispiel 93A

### (±)-1-(4-Bromphenyl)-7,8-diethoxy-N,4-dimethyl-4,5-dihydro-3H-2,3-benzodiazepin-3-carboxamid

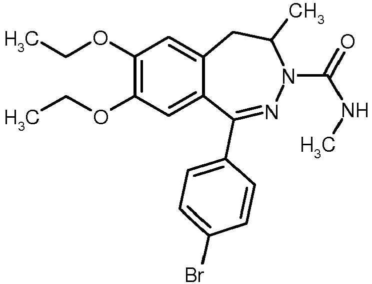

133 mg (329 µmol) (±)-1-(4-Bromphenyl)-7,8-dihydroxy-*N*,4-dimethyl-4,5-dihydro-3*H*-2,3-benzodiazepin-3-carboxamid (Beispiel **91A**) wurden in 5 ml DMF vorgelegt und bei RT 236 mg (724 mol) Cäsiumcarbonat und 54 µl (724 µmol) Bromethan zugegeben und 16 h bei RT gerührt. Die Mischung wurde auf Wasser gegeben und mit Ethylacetat extrahiert. Die vereinigten organischen Phasen wurden mit Wasser und ges. Natriumchloridlösung gewaschen und mit Natriumsulfat getrocknet. Die Lösemittel wurden am Rotationsverdampfer entfernt. Man erhielt 152 mg (100% d. Th.) des gewünschten Produkts.
LCMS (Methode 2): Rₜ = 1,47 min; m/z = 460/462 (M+H)⁺, Br-Isotopenmuster
¹H-NMR (400MHz, DMSO-*d*₆): δ = 0,90 (d, 3H), 1,18 (t, 3H), 1,32 (t, 3H), 2,64 (d, 3H), 2,66 (dd, 1H), 2,90 (dd, 1H), 3,72 - 3,80 (m, 2H), 4,08 (q, 2H), 5,03 - 5,11 (m, 1H), 6,45 (s, 1H), 6,62 (q, 1H), 6,96 (s, 1H), 7,52 - 7,58 (m, 4H).

### Beispiel 94A

### (4S)-1-(4-{[(2-Chlorethoxy)acetyl]amino}phenyl)-8-methoxy-N,4-dimethyl-4,5-dihydro-3H-2,3-benzodiazepin-3-carboxamid

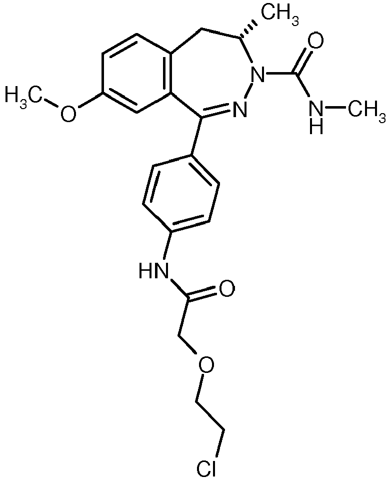

60 mg (0,177 mmol) (4*S*)-1-(4-Aminophenyl)-8-methoxy-*N*,4-dimethyl-4,5-dihydro-3*H*-2,3-benzodiazepin-3-carboxamid (Beispiel **53.2A**) wurden unter Argon in 3 ml Toluol vorgelegt und mit 33 mg (0,213 mmol) (2-Chlorethoxy)acetylchlorid versetzt. Man erhitzte für 5h auf 100°C. Der Ansatz wurde nach dem Abkühlen mit Wasser versetzt, etwas gesättigte wässrige Natriumhydrogencarbonat Lösung zugegeben und dreimal mit Ethylacetat extrahiert. Die vereinigten organischen Phasen wurden getrocknet und am Rotationsverdampfer vom Lösungsmittel befreit. Das Rohprodukt (112 mg) wurde ohne weitere Aufreinigung im nächsten Reaktionsschritt eingesetzt.
UPLC/MS (Methode 1): Rₜ = 1,2 min; m/z = 459 (M+H)⁺

### Beispiel 95A

### (±)-7-Brom-1-(4-chlorphenyl)-8-methoxy-N,4-dimethyl-4,5-dihydro-3H-2,3-benzodiazepin-3-carboxamid

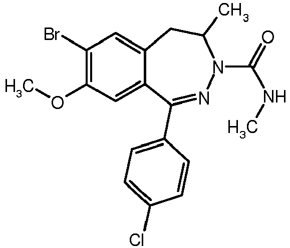

638 mg (1,69 mmol) (±)-1-(4-Chlorphenyl)-8-methoxy-*N*,4-dimethyl-4,5-dihydro-3*H*-2,3-benzodiazepin-3-carboxamid (Beispiel **50A**) wurden bei Raumtemperatur in 50 ml Dichlormethan gelöst und mit 2,17 g (6,78 mmol) Pyridinium-tribromid (CAS [39416-48-3]) versetzt. Die orangefarbene Lösung wurde 16,5 Stunden zum Rückfluss erhitzt. Es wurden weitere 1,08 g (3,49 mmol) Pyridinium-tribromid hinzugegeben und die Mischung 2 Tage zum Rückfluss erhitzt. Das Reaktionsgemisch wurde auf gesättigte wässrige Natriumthiosulfatlösung gegeben und dreimal mit Dichlormethan extrahiert. Die vereinigten organischen Phasen wurden mit gesättigter wässriger Natriumthiosulfatlösung gewaschen, mit Natriumsulfat getrocknet und das Lösemittel am Rotationsverdampfer entfernt. Das Rohprodukt wurde flashchromatographisch gereinigt (SiO₂, Hexan/Ethylacetat). Man erhielt 642 mg (82% d. Th.) des gewünschten Produkts als gelben Schaum.
LCMS (Methode 3): Rₜ = 1,44 min; m/z = 436; 438; 440 (Br-Cl-Isotopenmuster; M+H)^{+ 1}H-NMR (300MHz, CDCl₃): δ = 0,93 (d, 3H), 2,83 (dd, 1H), 2,89 (d, 3H), 3,07 (dd, 1H), 3,67 (s, 3H), 5,46 (m, 1H), 6,49 (m, 1H), 6,57 (s, 1H), 7,38 (d, 2H), 7,40 (s, 1H), 7,41 (d, 2H).

### Beispiel 96A

### (±)-1-(4-Chlorphenyl)-7-cyan-8-methoxy-N,4-dimethyl-4,5-dihydro-3H-2,3-benzodiazepin-3-carboxamid

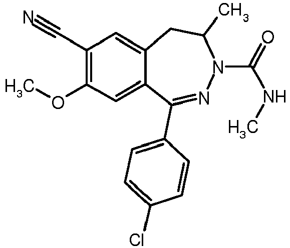

640 mg (1,39 mmol) (±)-7-Brom-1-(4-chlorphenyl)-8-methoxy-*N*,4-dimethyl-4,5-dihydro-3*H*-2,3-benzodiazepin-3-carboxamid (Beispiel **95A)** wurden 12 ml Toluol gelöst und mit 173 mg (1,39 mmol) 1-Butyl-1*H*-imidazol, 118 mg (0,28 mmol) im Mörser verriebenem und getrocknetem Kaliumhexacyanoferrat(II) sowie 26,5 mg (0,14 mmol) Kupfer(I)iodid versetzt. Das Reaktionsgemisch wurde 6 Stunden bei 160°C in der Mikrowelle bestrahlt. Zur Aufarbeitung wurde das Reaktionsgemisch über Celite^{®} filtriert, mit Ethylacetat nachgewaschen, und das Filtrat im Vakuum eingeengt. Das Rohprodukt wurde flashchromatographisch gereinigt (SieO₂, Hexan/Ethylacetat). Man erhielt 430 mg (79% d. Th.) des gewünschten Produkts als grauen Schaum.
LCMS (Methode 3): Rₜ = 1,30 min; m/z = 383; 385 (Cl-Isotopenmuster; M+H)⁺
¹H-NMR (400MHz, CDCl₃): δ = 0,89 (d, 3H), 2,90 (d, 3H), 2,91 (dd, 1H), 3,09 (dd, 1H), 3,71 (s, 3H), 5,50 (m, 1H), 6,52 (qbr, 1H), 6,66 (s, 1H), 7,38 (d, 2H), 7,39 (s, 1H), 7,41 (d, 2H).

### Beispiel 97A

### (±)-1-(4-Bromphenyl)-7-(difluormethoxy)-8-methoxy-N,4-dimethyl-4,5-dihydro-3H-2,3-benzodiazepin-3-carboxamid

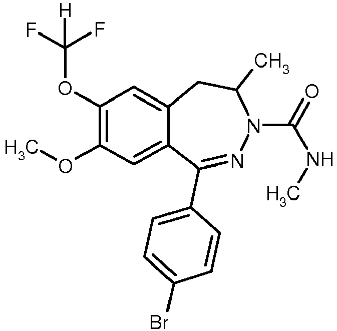

1,58 g (39,5 mmol) Natriumhydroxid wurden in 42 ml DMF suspendiert. Bei RT wurden 1,14 g (2,73 mmol) (±)-1-(4-Bromphenyl)-7-hydroxy-8-methoxy-*N*,4-dimethyl-4,5-dihydro-3*H*-2,3-benzodiazepin-3-carboxamid (Beispiel **90A**) zugegeben und 15 min bei RT und dann 10 min bei 0°C gerührt. Bei 0°C wurden 4,32 g (27,3 mmol) Ethyl-chlor(difluor)acetat (CAS [383-62-0]) zugegeben und 5 h bei 70°C nachgerührt. Die Mischung wurde nach dem Abkühlen auf Wasser gegeben, mit Ethylacetat extrahiert, die vereinigten organischen Phasen mit ges. Natriumchloridlösung gewaschen und mit Natriumsulfat getrocknet. Die Lösemittel wurden am Rotationsverdampfer entfernt und das Rohprodukt chromatographisch gereinigt (SiO₂, Hexan/Ethylacetat 0-60%). Man erhielt 370 mg (29% d. Th.) des gewünschten Produkts als blassgelben Schaum.
LCMS (Methode 2): Rₜ = 1,42 min; m/z = 468/470 (M+H)⁺, Br-Isotopenmuster
¹H-NMR (400MHz, CDCl₃): δ = 0,91 (d, 3H), 2,64 (d, 3H), 2,65 (dd, 1H), 2,92 (dd, 1H), 3,61 (s, 3H), 4,99 - 5,08 (m, 1H), 6,65 (q, 1H), 6,67 (s, 1H), 7,13 (t, J=75 Hz, 1H), 7,20 (s, 1H), 7,59 (s, 4H).

Analog Beispiel **8A** wurden aus dem entsprechenden kommerziell erhältlichen Aldehyden folgende Verbindungen hergestellt:

| Nr | Struktur | Name | Analytische Daten |
|---|---|---|---|
| **98A** | 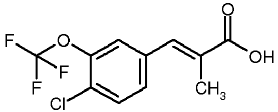 | (2E)-3-[4-Chlor-3-(trifluormethoxy)ph enyl]-2-methylacrylsäure | ¹H-NMR (300MHz, CDCl₃): δ = 2.13 (s, 3H), 7.30 (d, 1H), 7.38 (s, br, 1H), 7.52 (d, 1H), 7.73 (s, br, 1H). |
| | | | LCMS (Methode 2): Rₜ = 1.31 min; m/z [ES-]= 279/281 (M-H, Cl-Isotope)⁻ |
| **99A** | 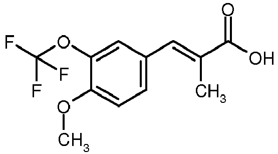 | (2E)-3-[4-Methoxy-3-(trifluormethoxy)ph enyl]-2-methylacrylsäure | ¹H-NMR (300MHz, CDCl₃): δ = 2.15 (d, 3H), 3.93 (s, 3H), 7.03 (d, 1H), 7.33-7.40 (m, 2H), 7.72 (s, br, 1H). |
| | | | LCMS (Methode 1): Rₜ = 1.16 min; m/z [ES-]= 277 (M+H)⁺ |

Analog Beispiel **10A** wurden aus Beispiel **98A** bzw. Beispiel **99A** folgende Verbindungen hergestellt:

| Nr | Struktur | Name | Analytische Daten |
|---|---|---|---|
| **100 A** | 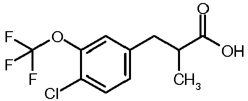 | 3-[4-Chlor-3-(trifluormethoxy)pheny 1]-2-methylpropansäure | ¹H-NMR (400MHz, CDCl₃): δ = 1.21 (d, 3H), 2.67-2.81 (m, 2H), 2.98-3.08 (m, 1H), 7.08 (dd, 1H), 7.16 (t, 1H), 7.38 (d, 1H). |
| | | | LCMS (Methode 2): Rₜ = 1.27 min; m/z [ES⁻] = 281/283 (M-H, Cl-Isotope)⁻ |
| **101 A** | 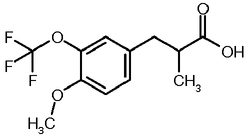 | 3-[4-Methoxy-3-(trifluormethoxy)pheny 1]-2-methylpropansäure | ¹H-NMR (300MHz, CDCl₃): δ = 1.19 (d, 3H), 2.60-2.79 (m, 2H), 2.99 (dd, 1H), 3.85 (s, 3H), 6.91 (d, 1H), 7.03-7.10 (m, 2H). |
| | | | LCMS (Methode 2): Rₜ = 1.16 min; m/z [ES-] = 278 (M-H)⁻ |

Analog Beispiel **15A** wurde aus der entsprechenden Carbonsäure (Beispiel **100A** bzw. Beispiel **101A)** folgende Indanone hergestellt:

| Nr | Struktur | Name | Analytische Daten |
|---|---|---|---|
| **102 A** | 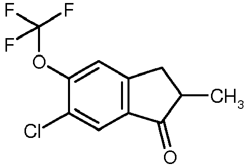 | 6-Chlor-2-methyl-5-(trifluormethoxy)indan -1-on | ¹H-NMR (300MHz, CDCl₃): δ = 1.32 (d, 3H), 2.68-2.84 (m, 2H), 3.40 (dd, 1H), 7.40 (s, br, 1H), 7.85 (s, 1H). |
| | | | LCMS (Methode 2): Rₜ = 1.36 min; m/z = 266 (M+H)⁺ |
| **103 A** | 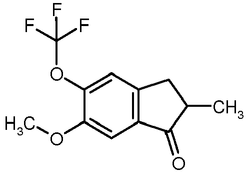 | 6-Methoxy-2-methyl-5-(trifluormethoxy)indan -1-on | ¹H-NMR (300MHz, CDCl₃): δ = 1.31 (d, 3H), 2.63-2.81 (m, 2H), 3.35 (dd, 1H), 3.91 (s, 3H), 7.30 (s, br, 2H). |
| | | | LCMS (Methode 2): Rₜ = 1.27 min; m/z = 261 (M+H)⁺ |

Analog Beispiel **17A** wurden aus den entsprechenden Indanonen, die ggf. kommerziell erhältlichen sind, durch Reaktion mit 4-Chlorphenylmagnesiumbromid und anschließender Wasser-Eliminierung mit Hilfe von 4-Toluolsulfonsäure folgende Verbindungen hergestellt:

| Nr | Struktur | Name | Analytische Daten |
|---|---|---|---|
| **104 A** | 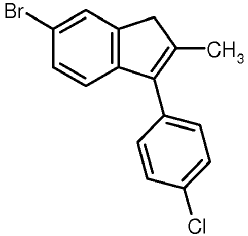 | 6-Brom-3-(4-chlorphenyl)-2-methyl-1H-inden | ¹H-NMR (400 MHz, CDCl₃): δ = 2.11 (s, 3H), 3.44 (s, 2H), 7.02 (d, 1H), 7.30 (d, 2H), 7.35 (dd, 1H), 7.44 (d, 2H), 7.56 (d, 1H). |
| | | | LCMS (Methode 2): Rₜ = 1.75 min; m/z = 319; 321 (Br-Isotopenmuster, M+H)⁺ |
| **105 A** | 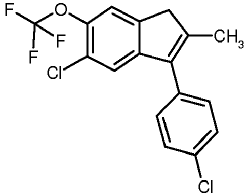 | 5-Chlor-3-(4-chlorphenyl)-2-methyl-1H-inden-6-yl-trifluormethylether | ¹H-NMR (400 MHz, CDCl₃): δ = 2.13 (s, 3H), 3.46 (s, 2H), 7.20 (s, 1H), 7.29 (d, 2H), 7.38 (s, br, 1H), 7.46 (d, 2H). |
| | | | LCMS (Methode 2): Rₜ = 1.75 min; m/z = 357; 359 (Cl-Isotopenmuster, M+H)⁺ |
| **106 A** | 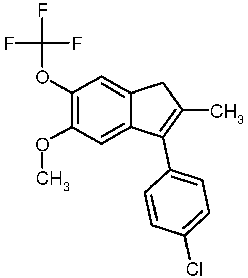 | 3-(4-Chlorphenyl)-5-methoxy-2-methyl-6-(trifluormethoxy)-1H-inden | ¹H-NMR (400 MHz, CDCl₃): δ = 2.11 (s, 3H), 3.41 (s, 2H), 3.81 (s, 3H), 6.76 (s, 1H), 7.30 (s, 1H), 7.31 (d, 2H), 7.47 (d, 2H). |
| | | | LCMS (Methode 1): Rₜ = 1.72 min; m/z = 355 (M+H)⁺ |

### Beispiel 107A

### (3,4-Dimethoxyphenyl)acetaldehyd

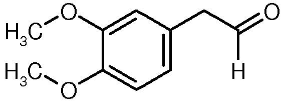

In 395 ml Dichlormethan wurden 18 g (96.81 mmol) 2-(3,4-Dimethoxyphenyl)ethanol vorgelegt und portionsweise 50.8 g (116.17 mmol) Dess-Martin-Reagenz zugegeben. Dabei wurde die Temperatur bei 20°C gehalten. Das Reaktionsgemisch wurde bei RT für 3h gerührt. Zu der Suspension wurden 200 ml ges. wässrige Natriumhydrogencarbonatlösung und 200 ml ges. wässrige Natriumdithionitlösung zugegeben. Man rührte für 30 min und trennte dann die organische Phase ab. Die wäsrige Phase wurde 3x mit Dichlormethan extrahiert und die vereinigten organischen Phasen einmal mit Natriumhydrogencarbonatlösung und einmal mit Wasser gewaschen. Nach Trocknen mit Natriumsulfat und Entfernen des Lösungsmittels erhielt man 16.6 g Produkt (95% d. Th.), das direkt weiter umgesetzt wurde.
LCMS (Methode 1): Rₜ = 0.74 min; m/z = 181 (M+H)⁺
¹H-NMR (300MHz, CDCl₃): δ = 3.66 (d, 2H), 3.91 (s, 6H), 6.73 (d, 1H), 6.81 (t, 1H), 6.90 (d, 1H), 9.75 (t, 1H).

### Beispiel 108A

### (±)-1-(3,4-Dimethoxyphenyl)butan-2-ol

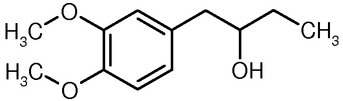

Unter Argon wurden 22.19 g (166.48 mmol) Ethylmagnesiumbromid vorgelegt und auf -40°C abgekühlt. 25 g (138.7 mmol) (3,4-Dimethoxyphenyl)acetaldehyd (Beispiel **107A**) wurden in 60 ml THF gelöst und langsam innerhalb von 30 min zu der Grignardlösung getropft. Dabei wurde eine Innenbadtemperatur vom max. -35°C eingehalten. Das Reaktionsgemisch wurde nach erfolgter Zugabe für 2h bei RT gerührt. Zur Aufarbeitung wurden 300 ml gekühlte ges. wässrige Ammoniumchloridlösung zugegeben und die organische Phase abgetrennt. Die wäsrige Phase wurde 3x mit Ethylacetat extrahiert und die vereinigten organischen Phasen einmal mit Wasser und einmal mit ges. wässriger Natriumchloridlösung gewaschen. Nach Trocknen mit Natriumsulfat und Entfernen des Lösungsmittels erhielt man 21.53 g Produkt (64% d. Th.), das direkt weiter umgesetzt wurde.
LCMS (Methode 1): Rₜ = 0.92 min; m/z = 211 (M+H)⁺; 193 (M-H₂O+H)⁺
¹H-NMR (400MHz, CDCl₃): δ = 1.03 (t, 3H), 1.52-1.66 (m, 2H), 2.59 (dd, 1H), 2.82 (dd, 1H), 3.69-3.80 (m, 1H), 3.84-3.96 (m, 1H), 3.89 (s, 3H), 3.90 (s, 3H), 6.77 (s, 1H), 6.78 (d, 1H), 6.85 (d, 1H).

### Beispiel 109A

### (±)-1-(3,4-Dimethoxyphenyl)-3-methylbutan-2-ol

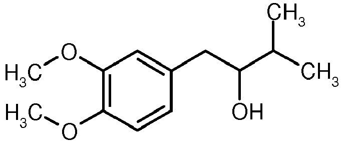

Unter Argon wurden 6.16 g (59.93 mmol) 2-Propylmagnesiumchlorid vorgelegt und auf -40°C abgekühlt. 9 g (49.9 mmol) (3,4-Dimethoxyphenyl)acetaldehyd (Beispiel **107A**) wurden in 30 ml THF gelöst und langsam innerhalb von 30 min zu der Grignardlösung getropft. Das Reaktionsgemisch wurde nach erfolgter Zugabe für 2h bei RT gerührt. Zur Aufarbeitung wurden 150 ml gekühlte ges. wässrige Ammoniumchloridlösung zugegeben und die organische Phase abgetrennt. Die wäsrige Phase wurde 3x mit Ethylacetat extrahiert und die vereinigten organischen Phasen einmal mit Wasser und einmal mit ges. wässriger Natriumchloridlösung gewaschen. Nach Trocknen mit Natriumsulfat und Entfernen des Lösungsmittels erhielt man 9.76 g Produkt (58% d. Th.), das direkt weiter umgesetzt wurde.
LCMS (Methode 1): Rₜ = 1.03 min; m/z = 225 (M+H)⁺; 207 (M-H₂O+H)⁺
¹H-NMR (300MHz, CDCl₃): δ = 1.03 (d, 6H), 1.51 (s, br, 1H), 1.72-1.84 (m, 1H), 2.56 (dd, 1H), 2.84 (dd, 1H), 3.58 (m, 1H), 3.89 (s, 3H), 3.91 (s, 3H), 6.74-6.88 (m, 3H).

Analog Beispiel **41A** wurden folgende Verbindungen aus Beispiel **108A** bzw. Beispiel **109A** und 4-Brombenzaldehyd hergestellt:

| Nr | Struktur | Name | Analytische Daten |
|---|---|---|---|
| **110 A** | 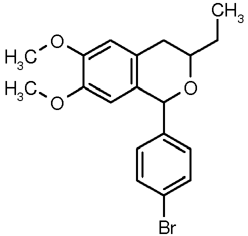 | 1-(4-Bromphenyl)-3-ethyl-6,7-dimethoxy-3,4-dihydro-1H-isochromen | LCMS (Methode 1): Rₜ = 1.53 min; m/z = 377; 379 (Br-Isotopenmuster, M+H)⁺ |
| **111 A** | 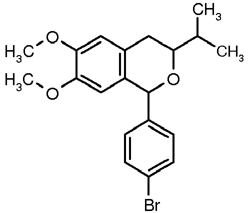 | 1-(4-Bromphenyl)-3-isopropyl-6,7-dimethoxy-3,4-dihydro-1H-isochromen | LCMS (Methode 5): Rₜ = 1.58 min; m/z = 391; 393 (Br-Isotopenmuster, M+H)⁺ |

Analog Beispiel **43A** wurden folgende Diketone hergestellt:

| Nr | Struktur | Name | Analytische Daten |
|---|---|---|---|
| **112 A** | 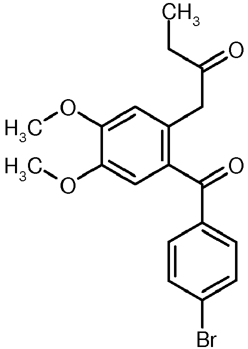 | 1-[2-(4-Brombenzoyl)-4,5-dimethoxyphenyl]buta n-2-on | ¹H-NMR (300MHz, CDCl₃): δ = 1.06 (t, 3H), 2.54 (q, 2H), 3.80 (s, 3H), 3.92 (s, 2H), 3.97 (s, 3H), 6.78 (s, 1H), 6.92 (s, 1H), 7.63 (d, 2H), 7.68 (d, 2H). |
| | | | LCMS (Methode 1): Rₜ = 1.33 min; m/z = 391; 393 (M+H, Br-Isotopenmuster)⁺ |
| **113 A** | 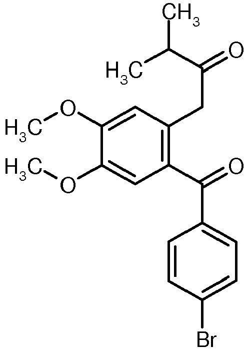 | 1-[2-(4-Brombenzoyl)-4,5-dimethoxyphenyl]-3-methylbutan-2-on | ¹H-NMR (400MHz, CDCl₃): δ = 1.12 (d, 6H), 2.69-2.80 (m, 1H), 3.80 (s, 3H), 3.97 (s, 3H), 4.02 (s, 2H), 6.77 (s, 1H), 6.91 (s, 1H), 7.62 (d, 2H), 7.68 (d, 2H). |
| | | | LCMS (Methode 1): Rₜ = 1.59 min; m/z = 407, 409 (M+H, Br-Isotopenmuster)⁺ |
| **114 A** | 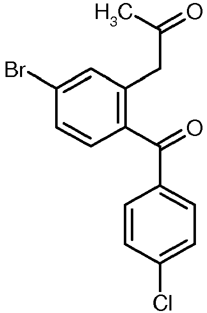 | 1-[5-Brom-2-(4-chlorbenzoyl)phenyl] ac eton | ¹H-NMR (300MHz, CDCl₃): δ = 2.19 (s, 3H), 4.00 (s, 2H), 7.24 (d, 1H), 7.40-7.51 (m, 4H), 7.73 (d, 2H). |
| | | | LCMS (Methode 2): Rₜ = 1.40 min; m/z = 351, 353 (M+H, Br-Isotopenmuster)⁺ |
| **115 A** | 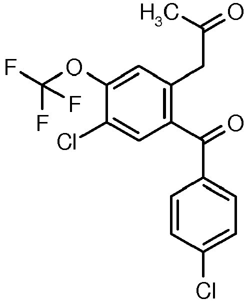 | 1-[4-Chlor-2-(4-chlorbenzoyl)-5-(trifluormethoxy)phenyl ]aceton | ¹H-NMR (300MHz, CDCl₃): δ = 2.20 (s, 3H), 4.04 (s, 2H), 7.21 (s, br, 1H), 7.45-7.52 (m, 3H), 7.77 (d, 2H). |
| | | | LCMS (Methode 2): Rₜ = 1.40 min; m/z = 391, 393 (M+H, Cl-Isotopenmuster)⁺ |
| **116 A** | 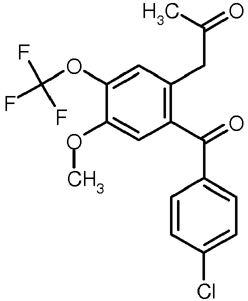 | 1-[2-(4-Chlorbenzoyl)-4-methoxy-5-(trifluormethoxy)pheny l]aceton | ¹H-NMR (300MHz, CDCl₃): δ = 2.19 (s, 3H), 3.83 (s, 3H), 3.95 (s, 2H), 6.99 (s, 1H), 7.15 (s, 1H), 7.49 (d, 2H), 7.81 (d, 2H). |
| | | | LCMS (Methode 1): Rₜ= 1.44 min; m/z = 387 (M+H)⁺ |

Analog Beispiel **45A,** allerdings bei RT und ohne Einleiten von Chlorwasserstoffgas, wurden folgende Benzodiazepine hergestellt:

| Nr | Struktur | Name | Analytische Daten |
|---|---|---|---|
| **117 A** | 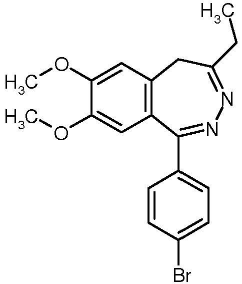 | 1-(4-Bromphenyl)-4-ethyl-7,8-dimethoxy-5H-2,3-benzodiazepin | ¹H-NMR (400MHz, CDCl₃): δ = 1.20 (t, 3H), 2.40-2.58 (m, 2H), 2.99 (d, 1H), 3.32 (d, 1H), 3.77 (s, 3H), 3.99 (s, 3H), 6.74 (s, 1H), 6.77 (s, 1H), 7.56 (d, 2H), 7.62 (d, 2H). |
| | | | LCMS (Methode 2): Rₜ = 1.19 min; m/z = 387, 389 (Br-Isotopenmuster, M+H)⁺ |
| **118 A** | 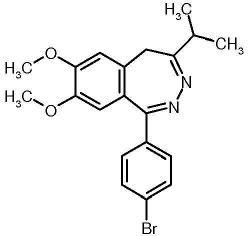 | 1-(4-Bromphenyl)-4-isopropyl-7,8-dimethoxy-5H-2,3-benzodiazepin | ¹H-NMR (300MHz, CDCl₃): δ = 1.19 (d, 3H), 1.24 (d, 3H), 2.71 (m, 1H), 2.93 (d, 1H), 3.43 (d, 1H), 3.78 (s, 3H), 3.98 (s, 3H), 6.75 (s, 1H), 6.77 (s, 1H), 7.56 (d, 2H), 7.61 (d, 2H). |
| | | | LCMS (Methode 5): Rₜ = 1.28 min; m/z = 401, 403 (Br-Isotopenmuster, M+H)⁺ |
| **119 A** | 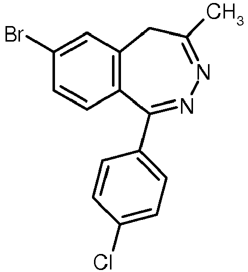 | 7-Brom-1-(4-chlorphenyl)-4-methyl-5H-2,3-benzodiazepin | ¹H-NMR (300MHz, CDCl₃): δ = 2.27 (s, 3H), 3.13 (d, 1H), 3.40 (d, 1H), 7.20 (d, 1H), 7.40 (d, 2H), 7.51 (s, 1H), 7.55 (d, 1H), 7.61 (d, 2H). |
| | | | LCMS (Methode 2): Rₜ = 1.41 min; m/z = 347, 349 (Br-Isotopenmuster, M+H)⁺ |
| **120 A** | 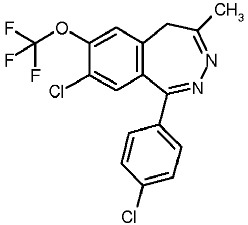 | 8-Chlor-1-(4-chlorphenyl)-4-methyl-7-(trifluormethoxy)-5H-2,3-benzodiazepin | ¹H-NMR (300MHz, CDCl₃): δ = 2.20 (s, 3H), 3.11 (d, 1H), 3.41 (d, 1H), 7.30 (s, 1H), 7.45 (d, 2H), 7.47 (s, 1H), 7.62 (d, 2H). |
| | | | LCMS (Methode 1): Rₜ = 1.55 min; m/z = 387, 389 (Cl-Isotopenmuster, M+H)⁺ |
| **121 A** | 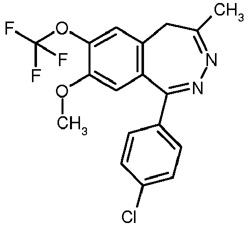 | 1-(4-Chlorphenyl)-8-methoxy-4-methyl-7-(trifluormethoxy)-5H-2,3-benzodiazepin | LCMS (Methode 1): Rₜ = 1.44 min; m/z = 383 (M+H)⁺ |

Analog Beispiel **47A** wurde folgende Amine aus den entsprechenden 5H-2,3-Benzodiazepinen hergestellt:

| Nr | Struktur | Name | Analytische Daten |
|---|---|---|---|
| **122 A** | 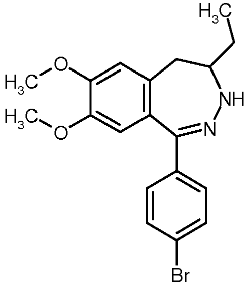 | (±)-1-(4-Bromphenyl)-4-ethyl-7,8-dimethoxy-4,5-dihydro-3H-2,3-benzodiazepin | ¹H-NMR (300MHz, CDCl₃): δ = 1.04 (t, 3H), 1.56-1.71 (m, 2H), 2.66 (dd, 1H), 2.91 (dd, 1H), 3.73 (s, 3H), 3.87 (quin, 1H), 3.97 (s, 3H), 5.48 (s, br, 1H), 6.60 (s, 1H), 6.78 (s, 1H), 7.46 (d, 2H), 7.52 (d, 2H). |
| | | | LCMS (Methode 2): Rₜ = 0.96 min; m/z = 389, 391 (Br-Isotopenmuster, M+H)⁺ |
| **123 A** | 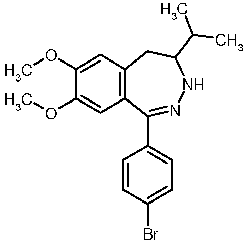 | (±)-1-(4-Bromphenyl)-4-isopropyl-7,8-dimethoxy-4,5-dihydro-3H-2,3-benzodiazepin | LCMS (Methode 5): Rₜ = 1.17 min; m/z = 403, 405 (Br-Isotopenmuster, M+H)⁺ |
| **124 A** | 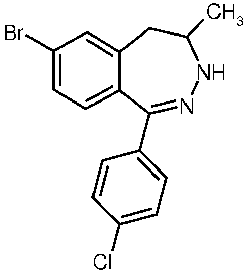 | (±)-7-Brom-1-(4-chlorphenyl)-4-methyl-4,5-dihydro-3H-2,3-benzodiazepin | ¹H-NMR (300MHz, CDCl₃): δ = 1.29 (d, 3H), 2.65 (dd, 1H), 2.93 (dd, 1H), 4.04-4.17 (m, 1H), 6.97 (d, 1H), 7.30-7.51 (m, 6H). |
| | | | LCMS (Methode 2): Rₜ = 1.43 min; m/z = 349, 351 (Br-Isotopenmuster, M+H)⁺ |
| **125 A** | 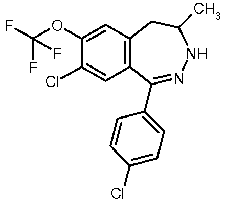 | (±)-8-Chlor-1-(4-chlorphenyl)-4-methyl-7-(trifluormethoxy)-4,5-dihydro-3H-2,3-benzodiazepin | ¹H-NMR (300MHz, CDCl₃): δ = 1.34 (d, 3H), 2.72 (dd, 1H), 2.99 (dd, 1H), 4.07-4.21 (m, 1H), 7.24 (s, 1H), 7.29 (s, 1H), 7.39 (d, 2H), 7.49 (d, 2H). |
| | | | LCMS (Methode 2): Rₜ = 1.63 min; m/z = 389, 391 (Cl-Isotopenmuster, M+H)⁺ |
| **126 A** | 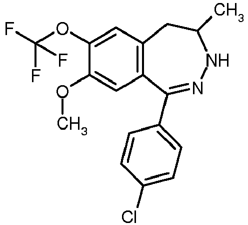 | (±)-1-(4-Chlorphenyl)-8-methoxy-4-methyl-7-(trifluormethoxy)-4,5-dihydro-3H-2,3-benzodiazepin | LCMS (Methode 3): Rₜ = 1.63 min; m/z = 385 (M+H)⁺ |

Analog Beispiel **49A** wurden folgende Verbindungen aus den entsprechenden 4,5-Dihydro-3*H*-2,3-benzodiazepinen hergestellt:

| Nr | Struktur | Name | Analytische Daten |
|---|---|---|---|
| **127 A** | 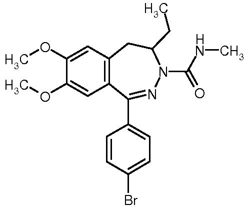 | (±)-1-(4-Bromphenyl)-4-ethyl-7,8-dimethoxy-N-methyl-4,5-dihydro-3H-2,3-benzodiazepin-3-carboxamid | ¹H-NMR (400MHz, CDCl₃): δ = 0.86 (t, 3H), 1.04-1.17 (m, 1H), 1.32-1.44 (m, 1H), 2.91 (d, 3H), 3.01 (dd, 1H), 3.08 (dd, 1H), 3.68 (s, 3H), 3.95 (s, 3H), 5.27-5.36 (m, 1H), 6.58 (s, 1H), 6.68 (q, 1H), 6.73 (s, 1H), 7.37 (d, 2H), 7.54 (d, 2H). |
| | | | LCMS (Methode 2): Rₜ = 1.53 min; m/z = 446; 448 (Br-Isotopenmuster, M+H)⁺ |
| **128 A** | 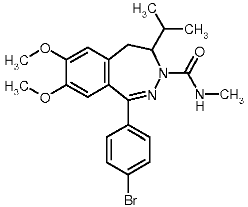 | (±)-1-(4-Bromphenyl)-4-isopropyl-7,8-dimethoxy-N-methyl-4,5-dihydro-3H-2,3-benzodiazepin-3-carboxamid | ¹H-NMR (400MHz, CDCl₃): δ = 0.84 (d, 3H), 0.86 (d, 3H), 0.97 (t, 1H), 2.90 (d, 3H), 3.01 (dd, 1H), 3.15 (dd, 1H), 3.67 (s, 3H), 3.95 (s, 3H), 5.16-5.32 (m, 1H), 6.61 (s, 1H), 6.66 (q, 1H), 6.75 (s, 1H), 7.36 (d, 2H), 7.55 (d, 2H). |
| | | | LCMS (Methode 5): Rₜ = 1.40 min; m/z = 460; 462 (Br-Isotopenmuster, M+H)⁺ |
| **129 A** | 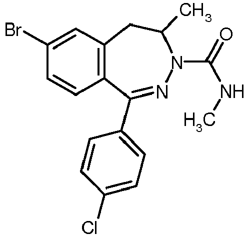 | (±)-7-Brom-1-(4-chlorphenyl)-N,4-dimethyl-4,5-dihydro-3H-2,3-benzodiazepin-3-carboxamid | ¹H-NMR (300MHz, CDCl₃): δ = 0.93 (d, 3H), 2.84-2.94 (m, 4H), 3.11 (d, 1H), 5.44-5.56 (m, 1H), 6.44-6.55 (m, 1H), 6.95 (d, 1H), 7.33 (dd, 1H), 7.36-7.42 (m, 5H). |
| | | | LCMS (Methode 1): Rₜ = 1.51 min; m/z = 406; 408 (Br-Isotopenmuster, M+H)⁺ |
| **130 A** | 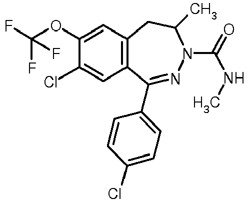 | (±)-8-Chlor-1-(4-chlorphenyl)-N,4-dimethyl-7-(trifluormethoxy)-4,5-dihydro-3H-2,3-benzodiazepin-3-carboxamid | ¹H-NMR (400MHz, CDCl₃): δ = 0.95 (d, 3H), 2.92 (d, 3H), 2.96 (dd, 1H), 3.16 (dd, 1H), 5.51-5.60 (m, 1H), 6.50 (q, 1H), 7.22 (dd, 1H), 7.23 (s, 1H), 7.40 (d, 2H), 7.43 (d, 2H). |
| | | | LCMS (Methode 1): Rₜ = 1.61 min; m/z = 446; 448 (Cl-Isotopenmuster, M+H)⁺ |
| **131 A** | 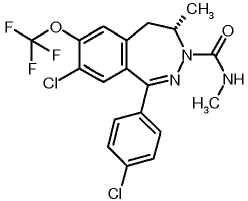 | (4S)-8-Chlor-1-(4-chlorphenyl)-N,4-dimethyl-7-(trifluormethoxy)-4,5-dihydro-3H-2,3-benzodiazepin-3-carboxamid | Präp. HPLC: Methode VIIIa Analyt. HPLC (Methode Da): Rₜ = 2.37 min |
| | | | [α]_{D}²⁰ = +73.0° (c = 1,00; MeOH) LCMS (Methode 2): Rₜ = 1.64 min; m/z = 446; 448 (Cl-Isotopenmuster, M+H)⁺ |
| **132 A** | 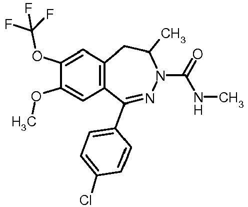 | (±)-1-(4-Chlorphenyl)-8-methoxy-N,4-dimethyl-7-(trifluormethoxy)-4,5-dihydro-3H-2,3-benzodiazepin-3-carboxamid | ¹H-NMR (400MHz, CDCl₃): δ = 0.96 (d, 3H), 2.88 (dd, 1H), 2.92 (d, 3H), 3.12 (dd, 1H), 3.69 (s, 3H), 5.44-5.54 (m, 1H), 6.46-6.54 (m, 1H), 6.71 (s, 1H), 7.12 (s, 1H), 7.41 (d, 2H), 7.46 (d, 2H). |
| | | | LCMS (Methode 1): Rₜ = 1.50 min; m/z = 442 (M+H)⁺ |

### Beispiel 133A

### (±)-1-[1-(4-Bromphenyl)-7,8-dimethoxy-4-methyl-4,5-dihydro-3H-2,3-benzodiazepin-3-yl]ethanon

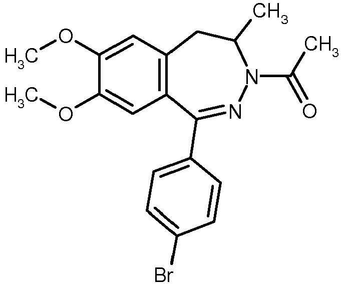

274 mg (0,73 mmol) (±)-1-(4-Bromphenyl)-7,8-dimethoxy-4-methyl-4,5-dihydro-3H-2,3-benzodiazepin (Beispiel **47A**) wurden mit 7 ml (73 mmol) Essigsäureanhydrid versetzt und 1 h bei 140°C Manteltemperatur gerührt. Nach dem Abkühlen wurden 25 ml ges. Natriumhydrogencarbonatlösung zugegeben. Das Gemisch wurde mit Ethylacetat extrahiert. Die vereinigten organischen Phasen wurden mit Wasser und ges. Natriumchloridlösung gewaschen, mit Natriumsulfat getrocknet und das Lösemittel am Rotationsverdampfer entfernt. Der Rückstand wurde chromatographisch gereinigt. Man erhielt 195 mg (64% d. Th.) des Produktes als braunes Öl.
LCMS (Methode 1): Rt = 1,29 min; m/z = 417/419 (M+H)⁺, Br-Isotopenmuster.
¹H-NMR (300 MHz, CDCl₃): δ = 1,19 (d, 3H), 2,22 (s, 3H), 2,76 (dd, 1H), 2,95 (dd, 1H), 3,74 (s, 3H), 3,97 (s, 3H), 5,33-5,42 (m, 1H), 6,57 (s, 1H), 6,80 (s, 1H), 7,53 (d, 2H), 7,59 (d, 2H).

### Beispiel 134A

### (±)-4-[7,8-Dimethoxy-4-methyl-3-[(methylamino)carbonyl]-4,5-dihydro-3H-2,3-benzodiazepin-1-yl]benzoesäure

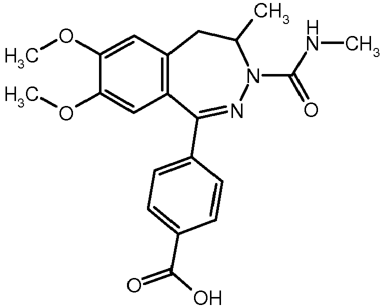

5,03 g (11,64 mmol) (±)-1-(4-Bromphenyl)-7,8-dimethoxy-*N*,4-dimethyl-4,5-dihydro-3*H*-2,3-benzodiazepin-3-carboxamid (Beispiel **49A**) wurden in 65 ml Dimethylsulfoxid suspendiert und mit 1,33 g (2,33 mmol) 1,1'-Bis(diphenylphosphino)ferrocen (CAS [12150-46-8]), 130 mg (0,58 mmol) Palladium(II)acetat und 4,5 g (46,5 mmol) Kaliumacetat versetzt. Das Gemisch wurde eine halbe Stunde bei Raumtemperatur einem Kohlenmonoxid-Druck von 19 bar unter Rühren ausgesetzt. Dann wurde entspannt, evakuiert, und bei 100°C erneut unter einem Kohlenmonoxid-Druck von 20 bar für 20 Stunden gerührt. Zur Aufarbeitung wurde filtriert, mit Ethylacetat nachgewaschen, die organische Phase dreimal mit gesättigter wässriger Natriumchloridlösung gewaschen, mit Natriumsulfat getrocknet und das Lösemittel am Rotationsverdampfer entfernt. Der Rückstand wurde chromatographisch gereinigt. Man erhielt 466 mg (10% d. Th.) des Produktes als dunklen Feststoff.
LCMS (Methode 3): Rₜ= 0,96 min; m/z = 398 (M+H)⁺.
¹H-NMR (400 MHz, CDCl₃): δ = 0,96 (d, 3H), 2,89 (dd, 1H), 2,91 (d, 3H), 3,17 (dd, 1H), 3,64 (s, 3H), 3,94 (s, 3H), 5,52 (m, 1H), 6,53 (s, 1H), 6,58 (m, 1H), 6,72 (s, 1H), 7,58 (d, 2H), 8,13 (d, 2H).

### Beispiel 135A

### 7,8-Dimethoxy-4-methyl-1-[4-(4-methylpiperazin-1-yl)phenyl]-5H-2,3-benzodiazepin

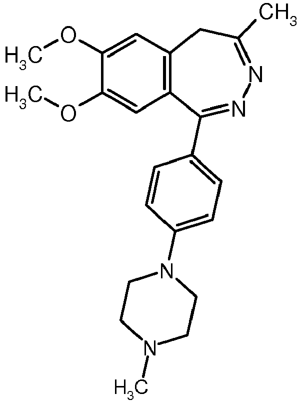

Die Verbindung wurde analog Beispiel 7 aus 1-(4-Bromphenyl)-7,8-dimethoxy-4-methyl-5*H*-2,3-benzodiazepin (Beispiel **45A**) hergestellt.
LCMS (Methode 3): Rₜ = 0,71 min; m/z = 393 (M+H)
¹H-NMR (300 MHz, DMSO-*d*₆): δ = 2,02 (s, 3H), 2,22 (s, 3H), 2,45 (m, 4H), 2,73 (d, 1H), 3,23 (m, 4H), 3,38 (d, 1H), 3,62 (s, 3H), 3,86 (s, 3H), 6,74 (s, 1H), 6,96 (d, 2H), 7,05 (s, 1H), 7,45 (d, 2H).

### Beispiel 136A

### (±)-7,8-Dimethoxy-4-methyl-1-[4-(4-methylpiperazin-1-yl)phenyl]-4,5-dihydro-3H-2,3-benzodiazepin

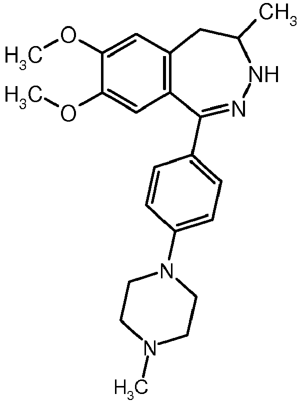

Die Verbindung wurde analog Beispiel 7 aus 7,8-Dimethoxy-4-methyl-1-[4-(4-methylpiperazin-1-yl)phenyl]-5*H*-2,3-benzodiazepin (Beispiel **135A**) hergestellt.
LCMS (Methode 3): Rₜ = 0,57 min; m/z = 395 (M+H)
¹H-NMR (300 MHz, DMSO-*d*₆): δ = 1,09 (s, 3H), 2,21 (s, 3H), 2,41 (m, 1H), 2,43 (m, 4H), 2,74 (dd, 1H), 3,17 (m, 4H), 3,58 (s, 3H), 3,81 (s, 3H), 3,88 (m, 1H), 6,18 (m, 1H), 6,53 (s, 1H), 6,89 (d, 2H), 6,90 (s, 1H), 7,34 (d, 2H).

### Beispiel 137A

### (±)-1-(4-Bromphenyl)-N-ethyl-7,8-dimethoxy-4-methyl-4,5-dihydro-3H-2,3-benzodiazepin-3-carboxamid

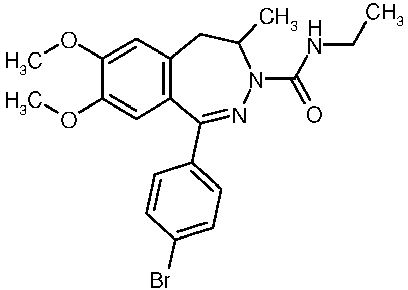

Eine Lösung von 150 mg (0.4 mmol) (±)-1-(4-Bromphenyl)-7,8-dimethoxy-4-methyl-4,5-dihydro-3H-2,3-benzodiazepin (Beispiel **47A**) in 6 ml Dichlormethan wurden bei RT mit 56.8 mg (0.8 mmol) Ethylisocyanat und für 16 h bei dieser Temperatur gerührt. Der Ansatz wurde vom Lösemittel am Rotationsverdampfer befreit und der Rückstand mittels präp. HPLC gereinigt. Man erhielt 120 mg (67% d.Th.) der Titelverbindung als Feststoff.
LCMS (Methode 5): Rₜ = 1.35 min; m/z = 446; 448 (Br-Isotopenmuster, M+H)⁺
¹H-NMR (400MHz, CDCl₃): δ = 0.96 (d, 3H), 1.18 (t, 3H), 2.84 (dd, 1H), 3.12 (dd, 1H), 3.29-3.39 (m, 2H), 3.66 (s, 3H), 3.93 (s, 3H), 5.40-5.50 (m, 1H), 6.53 (t, 1H), 6.55 (s, 1H), 6.71 (s, 1H), 7.36 (d, 2H), 7.53 (d, 2H).

Die in den vorangestellten Beispielen genannten Verbindungen 49.1A; 49.2A; 50A; 51A; 52A; 52.1A; 52.2A; 54A; 55A; 60A; 83A; 83.1A; 83.2A; 84A; 84.1A; 84.2A; 85A; 85.1A; 85.2A; 87A bis 93A und 95A bis 97A; 127A bis 133A sowie 137A stellen somit wertvolle ausgewählte Zwischenverbindungen der allgemeinen Formel (Ia) dar, die bevorzugt bei der Herstellung der erfindungsgemäßen Verbindungen zur Anwendung kommen.

Die folgenden erfindungsgemäßen Zwischenprodukte der allgemeinen Formel Ia sind somit ebenfalls Gegenstand der vorliegenden Erfindung:
(4R)-1-(4-Bromphenyl)-7,8-dimethoxy-N,4-dimethyl-4,5-dihydro-3H-2,3-benzodiazepin-3-carboxamid
(4S)-1-(4-Bromphenyl)-7,8-dimethoxy-N,4-dimethyl-4,5-dihydro-3H-2,3-benzodiazepin-3-carboxamid
(±)-1-(4-Bromphenyl)-7,8-dimethoxy-N,4-dimethyl-4,5-dihydro-3H-2,3-benzodiazepin-3-carboxamid
(±)-1-(4-Chlorphenyl)-8-methoxy-N,4-dimethyl-4,5-dihydro-3H-2,3-benzodiazepin-3-carboxamid
(±)-1-(3-Bromphenyl)-7,8-dimethoxy-N,4-dimethyl-4,5-dihydro-3H-2,3-benzodiazepin-3-carboxamid
(±)-8-Brom-1-(4-chlorphenyl)-N,4-dimethyl-4,5-dihydro-3H-2,3-benzodiazepin-3-carboxamid
(4R)-8-Brom-1-(4-chlorphenyl)-N,4-dimethyl-4,5-dihydro-3H-2,3-benzodiazepin-3-carboxamid
(4S)-8-Brom-1-(4-chlorphenyl)-N,4-dimethyl-4,5-dihydro-3H-2,3-benzodiazepin-3-carboxamid
(4S)-1-(4-Bromphenyl)-8-methoxy-N,4-dimethyl-4,5-dihydro-3H-2,3-benzodiazepin-3-carboxamid
(±)-1-(4-Chlorphenyl)-8-hydroxy-N,4-dimethyl-4,5-dihydro-3H-2,3-benzodiazepin-3-carboxamid
(±)-1-(3-Brom-4-fluorphenyl)-7,8-dimethoxy-N,4-dimethyl-4,5-dihydro-3H-2,3-benzodiazepin-3-carboxamid
(±)-8-tert-Butyl-1-(4-chlorphenyl)-N,4-dimethyl-4,5-dihydro-3H-2,3-benzodiazepin-3-carboxamid
(4S)-8-tert-Butyl-1-(4-chlorphenyl)-N,4-dimethyl-4,5-dihydro-3H-2,3-benzodiazepin-3-carboxamid
(4R)-8-tert-Butyl-1-(4-chlorphenyl)-N,4-dimethyl-4,5-dihydro-3H-2,3-benzodiazepin-3-carboxamid
(±)-8-Chlor-1-(4-chlorphenyl)-7-methoxy-N,4-dimethyl-4,5-dihydro-3H-2,3-benzodiazepin-3-carboxamid
(4S)-8-Chlor-1-(4-chlorphenyl)-7-methoxy-N,4-dimethyl-4,5-dihydro-3H-2,3-benzodiazepin-3-carboxamid
(4R)-8-Chlor-1-(4-chlorphenyl)-7-methoxy-N,4-dimethyl-4,5-dihydro-3H-2,3-benzodiazepin-3-carboxamid
(±)-1-(4-Chlorphenyl)-N,4,8-trimethyl-4,5-dihydro-3H-2,3-benzodiazepin-3-carboxamid
(4S)-1-(4-Chlorphenyl)-N,4,8-trimethyl-4,5-dihydro-3H-2,3-benzodiazepin-3-carboxamid
(4R)-1-(4-Chlorphenyl)-N,4,8-trimethyl-4,5-dihydro-3H-2,3-benzodiazepin-3-carboxamid
(±)-8-Amino-1-(4-chlorphenyl)-N,4-dimethyl-4,5-dihydro-3H-2,3-benzodiazepin-3-carboxamid
(±)-1-[8-tert-Butyl-1-(4-chlorphenyl)-4-methyl-4,5-dihydro-3H-2,3-benzodiazepin-3-yl]ethanon
(±)-8-Acetamido-1-(4-chlorphenyl)-N,4-dimethyl-4,5-dihydro-3H-2,3-benzodiazepin-3-carboxamid
(±)-1-(4-Bromphenyl)-7-hydroxy-8-methoxy-N,4-dimethyl-4,5-dihydro-3H-2,3-benzodiazepin-3-carboxamid
(±)-1-(4-Bromphenyl)-7,8-dihydroxy-N,4-dimethyl-4,5-dihydro-3H-2,3-benzodiazepin-3-carboxamid
(±)-1-(4-Bromphenyl)-7,8-bis(difluormethoxy)-N,4-dimethyl-4,5-dihydro-3H-2,3-benzodiazepin-3-carboxamid
(±)-1-(4-Bromphenyl)-7,8-diethoxy-N,4-dimethyl-4,5-dihydro-3H-2,3-benzodiazepin-3-carboxamid
(±)-7-Brom-1-(4-chlorphenyl)-8-methoxy-N,4-dimethyl-4,5-dihydro-3H-2,3-benzodiazepin-3-carboxamid
(±)-1-(4-Chlorphenyl)-7-cyan-8-methoxy-N,4-dimethyl-4,5-dihydro-3H-2,3-benzodiazepin-3-carboxamid
(±)-1-(4-Bromphenyl)-7-(difluormethoxy)-8-methoxy-N,4-dimethyl-4,5-dihydro-3H-2,3-benzodiazepin-3-carboxamid
(±)-1-(4-Bromphenyl)-4-ethyl-7,8-dimethoxy-N-methyl-4,5-dihydro-3H-2,3-benzodiazepin-3-carboxamid
(±)-1-(4-Bromphenyl)-4-isopropyl-7,8-dimethoxy-N-methyl-4,5-dihydro-3H-2,3-benzodiazepin-3-carboxamid
(±)-7-Brom-1-(4-chlorphenyl)-N,4-dimethyl-4,5-dihydro-3H-2,3-benzodiazepin-3-carboxamid
(±)-8-Chlor-1-(4-chlorphenyl)-N,4-dimethyl-7-(trifluormethoxy)-4,5-dihydro-3H-2,3-benzodiazepin-3-carboxamid
(4S)-8-Chlor-1-(4-chlorphenyl)-N,4-dimethyl-7-(trifluormethoxy)-4,5-dihydro-3H-2,3-benzodiazepin-3-carboxamid
(±)-1-(4-Chlorphenyl)-8-methoxy-N,4-dimethyl-7-(trifluormethoxy)-4,5-dihydro-3H-2,3-benzodiazepin-3-carboxamid
(±)-1-[1-(4-Bromphenyl)-7,8-dimethoxy-4-methyl-4,5-dihydro-3H-2,3-benzodiazepin-3-yl]ethanon
(±)-1-(4-Bromphenyl)-N-ethyl-7,8-dimethoxy-4-methyl-4,5-dihydro-3H-2,3-benzodiazepin-3-carboxamid

### Herstellung der erfindungsgemäßen Verbindungen der allgemeinen Formel I

### Beispiel 1

### (±)-1-(4-Chlorphenyl)-N,4-dimethyl-8-(trifluormethoxy)-4,5-dihydro-3H-2,3-benzodiazepin-3-carboxamid

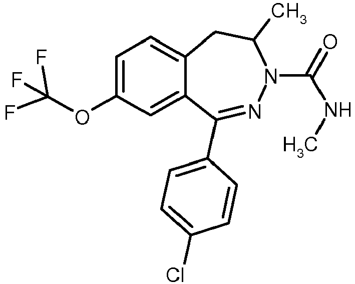

145 mg (3,64 mmol) Natriumhydroxid wurden in 8,7 ml Wasser gelöst und bei 0°C eine Lösung von 860 mg (2,42 mmol) (±)-1-(4-Chlorphenyl)-4-methyl-8-(trifluormethoxy)-4,5-dihydro-3H-2,3-benzodiazepin (Beispiel **35A**) in 35 ml Ethylacetat zugegeben. Nach 20 min Rühren bei 0°C wurden 877 mg (7,27 mmol, 0,80 ml) Isopropenylchlorformiat (CAS [57933-83-2]) zugesetzt und die Mischung 2,5 h bei 0°C intensiv gerührt. Anschließend wurden die Phasen getrennt, die organische Phase mit Natriumsulfat getrocknet und zur Trockne eingeengt. Der Rückstand wurde in 35 ml THF gelöst, 2 mg (0,02 mmol) 1-Methylpyrrolidin und 6,1 ml (12,1 mmol) 2M Methylaminlösung in THF zugegeben. Die Reaktionsmischung wurde im verschlossenen Druckrohr insgesamt 10 h bei 70 °C gerührt. Dabei wurde nach 5 h unterbrochen, die Mischung abgekühlt und weitere 6,1 ml Methylaminlösung zugegeben und erneut für 5 h auf 70°C erhitzt. Die Mischung wurde dann bei RT zwischen Wasser und Ethylacetat verteilt und die Phasen getrennt. Die wässrige Phase wurde mit Ethylacetat extrahiert. Die vereinigten organischen Phasen wurden mit ges. Natriumchloridlösung gewaschen, mit Natriumsulfat getrocknet und das Lösemittel am Rotationsverdampfer entfernt. Der Rückstand wurde flashchromatographisch gereinigt (SiO₂, Hexan/Ethylacetat). Man erhielt 540 mg (54% d. Th.) des gewünschten Produkts als gelbes Öl, das nach Einengen aus Dichlormethan als fester Schaum anfiel. Außerdem wurden 428 mg (36% d. Th.) des Zwischenprodukts Isopropenyl 1-(4-chlorphenyl)-4-methyl-8-(trifluormethoxy)-4,5-dihydro-3*H*-2,3benzodiazepin-3-carboxylat isoliert. (LCMS Nebenprodukt (Methode 2): Rₜ = 1,60 min; m/z = 439 (M+H)⁺).
Analytik der Titelverbindung: LCMS (Methode 2): Rₜ = 1,53 min; m/z = 412;414 (Cl-Isotopenmuster, M+H)⁺
¹H-NMR (300MHz, CDCl₃): δ = 0,91 (d, 3H), 2,90 (d, 3H), 2,96 (dd, 1H), 3,14 (dd, 1H), 5,46 - 5,55 (m, 1H), 6,47 - 6,52 (m, 1H), 6,94 (s, br, 1H), 7,17 - 7,29 (m, 2H), 7,39 (s, 4H).

### Enantiomerentrennung

488 mg (±)-1-(4-Chlorphenyl)-*N*,4-dimethyl-8-(trifluormethoxy)-4,5-dihydro-3*H*-2,3-benzodiazepin-3-carboxamid (Beispiel **1**) wurden mittels chiraler präparativer HPLC unter folgenden Bedingungen in die Enantiomere getrennt:
System: Dionex: Pump P 580, Gilson: Liquid Handler 215, Knauer: UV-Detektor K-2501; Säule:
   Chiralpak IC 5µm 250x30 mm; Eluent: Hexan / 2-Propanol 80:20 (v/v); Fluss: 50 ml/min;
   Temperatur: RT; Lösung: 488 mg / 8 ml DCM/EtOH; Injektion: 16 x 0,5 ml; Detektion: UV 254 nm.

### Beispiel 1.1: (4S)-1-(4-Chlorphenyl)-N,4-dimethyl-8-(trifluormethoxy)-4,5-dihydro-3H-2,3-benzodiazepin-3-carboxamid

224 mg, HPLC (Methode A): Rₜ = 3,31 min, Reinheit > 99,9%
Drehwert: [α]_{D}²⁰ = -265,9° ± 0,21° (c = 1,00; Chloroform)

### Beispiel 1.2: (4R)-1-(4-Chlorphenyl)-N,4-dimethyl-8-(trifluormethoxy)-4,5-dihydro-3H-2,3-benzo-diazepin-3-carboxamid

231 mg, HPLC (Methode A): Rₜ = 4,18 min, Reinheit 99,5%
Drehwert: [α]_{D}²⁰ = +254,7° ± 0,16° (c = 1,00; Chloroform)

### Beispiel 2

### (±)-1-[4-(3,5-Dimethyl-4-isoxazolyl)phenyl]-7,8-dimethoxy-N,4-dimethyl-4,5-dihydro-3H-2,3-benzodiazepin-3-carboxamid

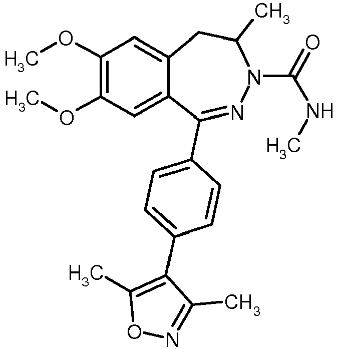

1,13 g (2,61 mmol) (±)-1-(4-Bromphenyl)-7,8-dimethoxy-*N*,4-dimethyl-4,5-dihydro-3*H*-2,3-benzodiazepin-3-carboxamid (Beispiel **49A**) wurden in 18 ml 1,4-Dioxan gelöst und 932 mg (6,61 mmol) 3,5-Dimethylisoxazol-4-boronsäure (CAS [16114-47-9]), 2,90 ml 1,5 M wässrige Kaliumcarbonatlösung und 363 mg (0,44 mmol) Dichlor[1,1'-bis(diphenylphosphino)ferrocen]palladium(II) (Komplex mit CH₂Cl₂, CAS [95464-05-4]) zugegeben. Die Mischung wurde in 3 Portionen aufgeteilt und jede jeweils 15 min bei 130°C in der Mikrowelle bestrahlt. Die Teilansätze wurden wieder vereinigt und am Rotationsverdampfer zur Trockne eingeengt. Der Rückstand wurde flashchromatographisch gereinigt (SiO₂, Hexan/Ethylacetat). Man erhielt 900 mg (73% d. Th.) des gewünschten Produkts als braunen Schaum.
LCMS (Methode 2): Rₜ = 1,22 min; m/z = 449 (M+H)⁺
¹H-NMR (400MHz, DMSO-*d*₆): δ = 0,97 (d, 3H), 2,24 (s, 3H), 2,42 (s, 3H), 2,65 (d, 3H), 2,61 - 2,67 (m, 1H), 2,91 (dd, 1H), 3,56 (s, 3H), 3,81 (s, 3H), 4,99 - 5,08 (m, 1H), 6,54 (s, 1H), 6,56 (q, 1H), 7,01 (s, 1H), 7,41 (d, 2H), 7,74 (d, 2H).

### Enantiomerentrennung

900 mg (±)-1-[4-(3,5-Dimethyl-4-isoxazolyl)phenyl]-7,8-dimethoxy-*N*,4-dimethyl-4,5-dihydro-3*H-*2,3-benzodiazepin-3-carboxamid (Beispiel **2**) wurden mittels chiraler präparativer HPLC unter folgenden Bedingungen in die Enantiomere getrennt:
System: Agilent: Prep 1200, 2xPrep Pump G1361A, DLA G2258A, MWD G1365D, Prep FC G1364B; Säule: Chiralpak ID 5µm 250x20 mm; Eluent: Hexan / Isopropanol 70:30 (v/v); Fluss: 40 ml/min; Temperatur: RT; Lösung: 900mg / 6 ml MeOH/MeCl; Injektion: 12 x 0,1 ml, 13 x 0,2 ml; Detektion: UV 280 nm.

### Beispiel 2.1: (4R)-1-[4-(3,5-Dimethyl-4-isoxazolyl)phenyl]-7,8-dimethoxy-N,4-dimethyl-4,5-dihydro-3H-2,3-benzodiazepin-3-carboxamid

324 mg, farbloser Feststoff, HPLC (Methode B): Rₜ = 7,6 min, Reinheit > 99%
Drehwert: [α]_{D}²⁰ = -150,9° ± 0,08° (c = 1,06; Methanol)

### Beispiel 2.2: (4S)-1-[4-(3,5-Dimethyl-4-isoxazolyl)phenyl]-7,8-dimethoxy-N,4-dimethyl-4,5-dihydro-3H-2,3-benzodiazepin-3-carboxamid

321 mg, farbloser Feststoff, HPLC (Methode B): Rₜ = 9,0 min, Reinheit 98,6%
Drehwert: [α]_{D}²⁰ = +148,0° ± 0,08° (c = 1,03; Methanol)

Die absolute Stereochemie von Beispiel 2.2 wurde durch eine Röntgenstrukturanalyse des Komplexes aus Beispiel 2.2 und der Bromodomäne 1 von BRD4 bestimmt.

### Beispiel 3

### (±)-7,8-Dimethoxy-N,4-dimethyl-1-[4-(1H-pyrazol-3-yl)phenyl]-4,5-dihydro-3H-2,3-benzodiazepin-3-carboxamid

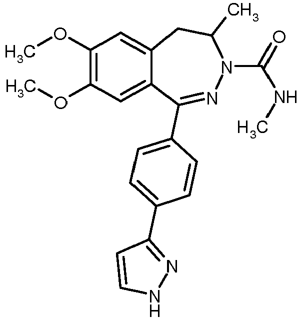

Die Reaktion wurde analog Beispiel **2** mit 100 mg (231 µmol) (±)-1-(4-Bromphenyl)-7,8-dimethoxy-*N*,4-dimethyl-4,5-dihydro-3*H*-2,3-benzodiazepin-3-carboxamid (Beispiel **49A**), 2 ml 1,4-Dioxan, 65 mg (585 µmol) 1*H*-Pyrazol-5-boronsäure (CAS [376584-63-3]), 0,25 ml 1,5 M wässriger Kaliumcarbonatlösung und 32 mg (0,44 mmol) Dichlor[1,1'-bis(diphenylphosphino)ferrocen]palladium(II) (Komplex mit CH₂Cl₂, CAS [95464-05-4]) durchgeführt. Man erhielt 55 mg (56% d. Th.) des Produkts.
LCMS (Methode 2): Rₜ = 1,04 min; m/z = 420 (M+H)⁺
1H-NMR (300MHz, DMSO-*d*6): δ = 0,97 (d, 3H), 2,61 - 2,67 (m, 1H), 2,65 (d, 3H), 2,91 (dd, H), 3,54 (s, 3H), 3,81 (s, 3H), 4,99 - 5,08 (m, 1H), 6,50 - 6,53 (m, 2H), 7,76 - 7,78 (m, 1H), 7,00 (s, 1H), 7,66 (d, 2H), 7,73 - 7,75 (m, 1H), 7,84 (s, 2H), 12,92 (br, 1H).

Analog Beispiel **2** wurden aus Beispiel **49A** und den entsprechenden kommerziell erhältlichen Boronsäurederivaten folgende Beispielverbindungen hergestellt:

| Nr | Struktur | Name | Analytische Daten |
|---|---|---|---|
| 4 | 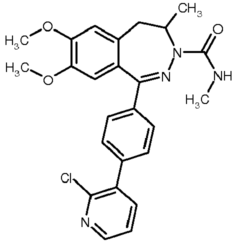 | (±)-1-[4-(2-Chlorpyridin-3-yl)phenyl]-7,8-dimethoxy-*N*,4-dimethyl-4,5-dihydro-3*H*-2,3-benzodiazepin-3-carboxamid | ¹H-NMR (300MHz, DMSO-*d*6): δ = 0,96 (d, 3H), 2,65 (d, 3H), 2,66 - 2,71 (m, 1H), 2,93 (dd, H), 3,55 (s, 3H), 3,81 (s, 3H), 5,00 - 5,11 (m, 1H), 6,54 (s, 1H), 6,60 (q, 1H), 7,01 (s, 1H), 7,52 (d, 2H), 7,51 - 7,55 (m, 1H), 7,76 (d, 2H), 7,92 (dd, 1H), 8,43 (dd, 1H). |
| | | | LCMS (Methode 2): Rₜ = 1,24 min; m/z = 465 (M+H)⁺ |
| 4.1 | 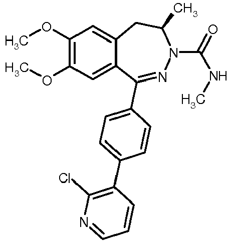 | (*R*)-1-[4-(2-Chlorpyridin-3-yl)phenyl]-7,8-dimethoxy-*N*,4-dimethyl-4,5-dihydro-3*H*-2,3-benzodiazepin-3-carboxamid | Präp. HPLC: Methode XXII |
| | | | Analyt. HPLC (Methode B): Rₜ = 7,75 min |
| 4.2 | 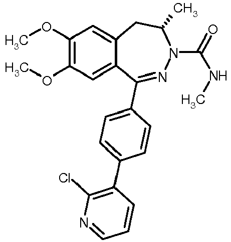 | (*S*)*-*1*-*[4-(2-Chlorpyridin-3-yl)phenyl]-7,8-dimethoxy-*N*,4-dimethyl-4,5-dihydro-3*H*-2,3-benzodiazepin-3-carboxamid | Präp. HPLC: Methode XXII |
| | | | Analyt. HPLC (Methode B): Rₜ = 8,89 min |
| 5 | 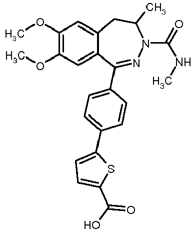 | (±)-5-(4-{7,8-Dimethoxy-4-methyl-3-[(methylamino)carbonyl]-4,5-dihydro-3*H*-2,3-benzodiazepin-1-yl}phenyl)thiophen-2-carbonsäure | LCMS (Methode 2): Rₜ= 1,15 min; m/z = 480 (M+H)⁺ |
| 6 | 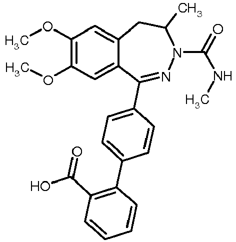 | (±)-4'-{7,8-Dimethoxy-4-methyl-3-[(methylamino)carbonyl]-4,5-dihydro-3*H*-2,3-benzodiazepin-1-yl}biphenyl-2-carbonsäure | LCMS (Methode 2): Rₜ= 1,19 min; m/z = 474 (M+H)⁺ |

### Beispiel 7

(±)-7,8-Dimethoxy-*N*,4-dimethyl-1-[4-(morpholin-4-yl)phenyl]-4,5-dihydro-3*H*-2,3-benzodiazepin-3-carboxamid

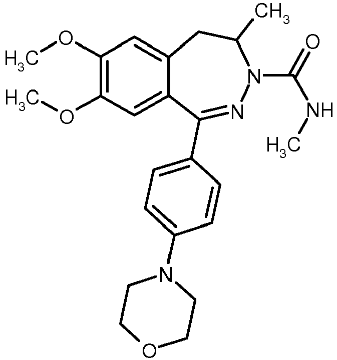

100 mg (231 µmol) (±)-1-(4-Bromphenyl)-7,8-dimethoxy-*N*,4-dimethyl-4,5-dihydro-3*H*-2,3-benzodiazepin-3-carboxamid (Beispiel **49A**) wurden in 2 ml Toluol gelöst und 24 mg (278 µmol) Morpholin, 56 mg (578 µmol) Natrium-*tert*-butanolat, 2 mg (2 µmol) tris(Dibenzylidenaceton)dipalladium(0) (CAS [51364-51-3]) und 5,5 mg (12 µmol) 2-(Dicyclohexylphosphino)-2',4',6'-triisopropylbiphenyl (CAS [564483-18-7]) zugegeben. Die Mischung wurde 15 min bei 130°C in der Mikrowelle bestrahlt. Der Katalysator wurde abfiltriert, das Filtrat am Rotationsverdampfer zur Trockne eingeengt und der Rückstand mittels präparativer HPLC gereinigt. Man erhielt 7 mg (9% d. Th.) des gewünschten Produkts als farblosen Feststoff.
LCMS (Methode 2): Rₜ = 1,09 min; m/z = 439 (M+H)⁺
¹H-NMR (300MHz, DMSO-*d*6): δ = 1,02 (d, 3H), 2,39 - 2,44 (m, 1H), 2,60 (d, 3H), 2,81 (dd, H), 3,14 - 3,22 (m, 4H), 3,58 (s, 3H), 3,68 - 3,74 (m, 4H), 3,80 (s, 3H), 4,82 - 4,94 (m, 1H), 6,27 (q, 1H), 6,51 (s, 1H), 6,93 (d, 2H), 6,99 (s, 1H), 7,57 (s, 2H).

### Beispiel 8

### (±)-1-(4-Chlorphenyl)-N,4-dimethyl-8-(pyridin-4-yl)-4,5-dihydro-3H-2,3-benzodiazepin-3-carboxamid

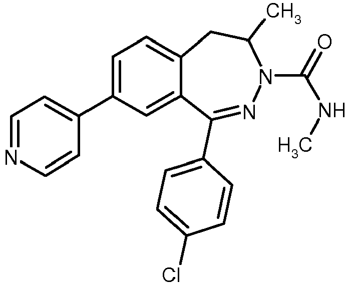

100 mg (246 µmol) 8-Brom-1-(4-chlorphenyl)-*N*,4-dimethyl-4,5-dihydro-3*H*-2,3-benzodiazepin-3-carboxamid (Beispiel **52A**) wurden in 1,3 ml Toluol und 0,2 ml Wasser gelöst und 30 mg (246 µmol) Pyridin-4-boronsäure (CAS [1692-15-5]), 51 mg (369 µmol) Kaliumcarbonat und 18 mg (25 µmol) Dichlor[1,1'-bis(diphenylphosphino)ferrocen]palladium(II) (CAS [95464-05-4]) zugegeben. Die Mischung wurde 16 h bei 70°C gerührt. Das Gemisch wurde mit ges. Natriumhydrogencarbonatlösung versetzt und mit Ethylacetat extrahiert. Die vereinigten organischen Phasen wurden mit Wasser und ges. Natriumchloridlösung gewaschen und mit Natriumsulfat getrocknet. Die Lösemittel wurden am Rotationsverdampfer entfernt. Der braune Rückstand (88 mg) wurde mittels präparativer HPLC gereinigt. Man erhielt 6,8 mg (7% d. Th.) des gewünschten Produkts als leicht gelblichen Feststoff.
UPLC/MS (Methode 3): Rₜ = 1,03 min; m/z = 405 (M+H)⁺
¹H-NMR (300MHz, CDCl₃): δ = 0,97 (d, 3H), 2,91 (d, 3H), 2,99 (dd, 1H), 3,19 (dd, 1H), 5,47 - 5,57 (m, 1H), 6,48 (q, 1H), 7,33 - 7,49 (m, 8H), 7,61 (dd, 1H), 8,62 (d, 2H).

Analog Beispiel 8 wurden aus Beispiel **52A** und den entsprechenden kommerziell erhältlichen Boronsäurederivaten folgende Beispielverbindungen hergestellt:

| Nr | Struktur | Name | Analytische Daten |
|---|---|---|---|
| 9 | 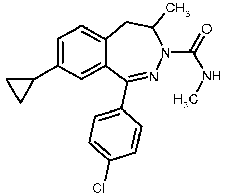 | (±)-1-(4-Chlorphenyl)-8-cyclopropyl-N,4-dimethyl-4,5-dihydro-3*H-*2,3-benzodiazepin-3-carboxamid | LCMS (Methode 3): Rₜ = 1,54 min; m/z = 368 (M+H)⁺ |
| | | | ¹H-NMR (300MHz, CDCl₃): δ = 0,53 - 0,58 (m, 2H), 0,86 - 0,94 (m, 2H), 0,93 (d, 3H), 1,73 - 1,82 (m, 1H), 2,85 (dd, 1H), 2,88 (d, 3H), 3,09 (dd, 1H), 5,47 - 5,48 (m, 1H), 6,40 - 6,46 (m, br, 1H), 6,78 (d, 1H), 7,00 (dd, 1H), 7,11 (d, 1H), 7,35 - 7,44 (m, 4H). |
| 10 | 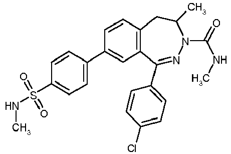 | (±)-1-(4-Chlorphenyl)-*N*,4-dimethyl-8-{4-[(methylamino)sulfonyl]p henyl}-4,5-dihydro-3*H-*2,3-benzodiazepin-3-carboxamid | LCMS (Methode 3): Rₜ = 1,32 min; m/z = 497 (M+H)⁺ |
| | | | ¹H-NMR (300MHz, CDCl₃): δ = 0,98 (d, 3H), 2,69 (d, 3H), 2,91 (d, 3H), 2,99 (dd, 1H), 3,19 (dd, 1H), 3,09 (dd, 1H), 4,26 (q, 1H), 5,47 - 5,56 (m, 1H), 6,48 (q, 1H), 7,30 - 7,48 (m, 6H), 7,55 - 7,61 (m, 3H), 7,88 (d, 2H). |

### Beispiel 11

### (±)-1-(4-Chlorphenyl)-N,4-dimethyl-8-(morpholin-4-yl)-4,5-dihydro-3H-2,3-benzodiazepin-3-carboxamid

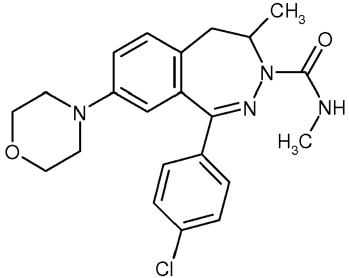

200 mg (492 µmol) 8-Brom-1-(4-chlorphenyl)-*N*,4-dimethyl-4,5-dihydro-3*H*-2,3-benzodiazepin-3-carboxamid (Beispiel **52A**) wurden in 4 ml Toluol gelöst und 52 mg (590 µmol) Morpholin, 118 mg (1,23 mmol) Natrium-*tert*-butanolat, 4,5 mg (5 µmol) tris(Dibenzylidenaceton)dipalladium(0) (CAS [51364-51-3]) und 15 mg (25 µmol) (*R*)-(+)-2,2'-bis(Diphenylphosphino)-1,1'-binaphthyl (CAS [98327-87-8]) zugegeben. Das Gemisch wurde mit ges. Natriumhydrogencarbonat versetzt und mit Ethylacetat extrahiert. Die vereinigten organischen Phasen wurden mit Wasser und ges. Natriumchloridlösung gewaschen und mit Natriumsulfat getrocknet. Die Lösemittel wurden am Rotationsverdampfer entfernt und der Rückstand flashchromatographisch gereinigt. Man erhielt 44 mg (22% d. Th.) des gewünschten Produkts als orangefarbenen Schaum.
LCMS (Methode 2): Rₜ = 1,33 min; m/z = 413 (M+H)⁺
¹H-NMR (300MHz, CDCl₃): δ = 0,93 (d, 3H), 2,82 (dd, 1H), 2,88 (d, 3H), 2,81 (dd, H), 2,97 - 3,00 (m, 4H), 3,07 (dd, 1H), 3,78 - 3,81 (m, 4H), 5,35 - 5,45 (m, 1H), 6,44 (q, br, 1H), 6,58 (d, 1H), 6,89 (dd, 1H), 7,12 (d, 1H), 7,36 (d, 2H), 7,44 (d, 2H).

### Enantiomerentrennung

14 mg (±)-1-(4-Chlorphenyl)-*N*,4-dimethyl-8-(morpholin-4-yl)-4,5-dihydro-3*H*-2,3-benzodiazepin-3-carboxamid (Beispiel **11**) wurden mittels chiraler präparativer HPLC unter folgenden Bedingungen in die Enantiomere getrennt:
System: Dionex: Pump P 580, Gilson: Liquid Handler 215, Knauer: UV-Detektor K-2501; Säule: Chiralpak IC 5µm 250x20 mm; Eluent: Ethanol / Methanol 50:50 (v/v); Fluss: 15 ml/min; Temperatur: RT; Lösung: 14 mg / 1.2 ml EtOH/MeOH; Injektion: 1 x 1.2 ml; Detektion: UV 254 nm.

### Beispiel 11.1: (4S)-1-(4-Chlorphenyl)-N,4-dimethyl-8-(morpholin-4-yl)-4,5-dihydro-3H-2,3-benzodiazepin-3-carboxamid

6 mg, farbloser Feststoff, HPLC (Methode E): Rₜ = 2,88 min, Reinheit 99,9%

### Beispiel 11.2: (4R)-1-(4-Chlorphenyl)-N,4-dimethyl-8-(morpholin-4-yl)-4,5-dihydro-3H-2,3-benzodiazepin-3-carboxamid

6 mg, farbloser Feststoff, HPLC (Methode E): Rₜ = 3,22 min, Reinheit 97,1 %

### Beispiel 12

### (±)-1-(4-Chlorphenyl)-N,4-dimethyl-8-(4-methylpiperazin-1-yl)-4,5-dihydro-3H-2,3-benzodiazepin-3-carboxamid

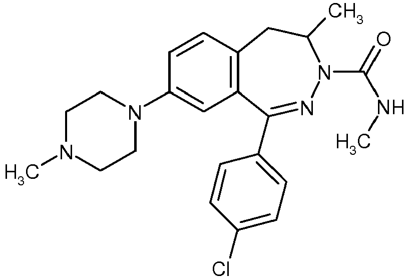

100 mg (246 µmol) 8-Brom-1-(4-chlorphenyl)-*N*,4-dimethyl-4,5-dihydro-3*H*-2,3-benzodiazepin-3-carboxamid (Beispiel **52A**) wurden in 2 ml 1,4-Dioxan gelöst und 30 mg (295 µmol) 1-Methylpiperazin (CAS [109-01-3]), 33 mg (34 µmol) Natrium-*tert*-butanolat, 11 mg (12 µmol) tris(Dibenzylidenaceton)dipalladium(0) (CAS [51364-51-3]) und 6 mg (12 µmol) Xphos (= 2-(Dicyclohexylphosphino)-2',4',6'-triisopropylbiphenyl, CAS [564483-18-7]) zugegeben. Die Mischung wurde 15 min bei 130°C in der Mikrowelle (150 W) bestrahlt. Das Gemisch wurde mit ges. Natriumhydrogencarbonatlösung versetzt und mit Ethylacetat extrahiert. Die vereinigten organischen Phasen wurden mit Wasser und ges. Natriumchloridlösung gewaschen und mit Natriumsulfat getrocknet. Die Lösemittel wurden am Rotationsverdampfer entfernt und der Rückstand mittels präparativer HPLC gereinigt. Man erhielt 7 mg (7% d. Th.) des gewünschten Produkts als schwach gelben Feststoff.
LCMS (Methode 3): Rₜ = 0,91 min; m/z = 426 (M+H)⁺
¹H-NMR (300MHz, CDCl₃): δ = 0,94 (d, 3H), 2,32 (s, 3H), 2,50 - 2,53 (m, 4H), 2,81 (dd, 1H), 2,88 (d, 3H), 3,00 - 3,09 (m, 5H), 5,33 - 5,45 (m, 1H), 6,38 - 6,46 (m, 1H), 6,60 (d, 1H), 6,90 (dd, 1H), 7,11 (d, 1H), 7,36 (d, 2H), 7,45 (d, 2H).

Analog Beispiel **12** wurde aus Beispiel **52A** und dem entsprechenden kommerziell erhältlichen Amin folgende Beispielverbindung hergestellt:

| Nr | Struktur | Name | Analytische Daten |
|---|---|---|---|
| 13 | 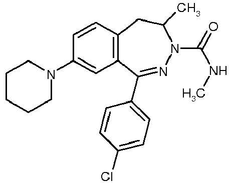 | (±)-1-(4-Chlorphenyl)-*N*,4-dimethyl-8-(piperidin-1-yl)-4,5-dihydro-3*H*-2,3-benzodiazepin-3-carboxamid | LCMS (Methode 2): Rₜ = 1,35 min; m/z = 411 (M+H)⁺ |
| | | | ¹H-NMR (300MHz, CDCl₃): δ = 0,94 (d, 3H), 1,55 - 1,69 (m, 5H), 2,79 (dd, 1H), 2,88 (d, 3H), 2,96 - 3,00 (m, 4H), 3,05 (dd, 1H), 5,33 - 5,43 (m, 1H), 6,38 - 6,45 (m, 1H), 6,60 (d, 1H), 6,92 (dd, 1H), 7,09 (d, 1H), 7,36 (d, 2H), 7,45 (d, 2H). |

### Beispiel 14

### (±)-8-Methoxy-N,4-dimethyl-1-(pyridin-3-yl)-4,5-dihydro-3H-2,3-benzodiazepin-3-carboxamid

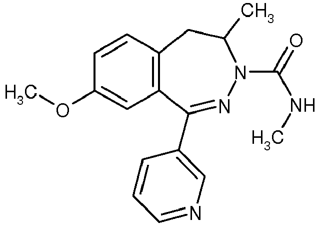

Die Verbindung wurde analog Beispiel **49A** aus 155 mg (0,48 mmol) rohem 8-Methoxy-4-methyl-1-(pyridin-3-yl)-4,5-dihydro-3*H*-2,3-benzodiazepin (Beispiel **37A**), 230 mg (1,16 mmol) 4-Nitrophenylchloroformat und 3,0 ml 2M Methylamin-Lösung in 8 ml THF hergestellt. Man erhielt 26 mg (ca. 13% d.Th.) Produkt als orangefarbenen Schaum.
UPLC/MS (Methode 3): Rₜ = 0,97 min; m/z = 325 (M+H)⁺
¹H-NMR (300MHz, CDCl₃): δ = 0,90 (d, 3H), 2,90 (d, 3H), 2,91 (dd, 1H), 3,14 (dd, 1H), 3,69 (s, 3H), 5,47 - 5,53 (m, 1H), 6,51 - 6,60 (m, 1H), 6,60 (d, 1H), 6,89 (dd, 1H), 7,15 (d, 1H), 7,29 - 7,33 (m, 1H), 7,71 - 7,75 (m, 1H), 8,62 (dd, 2H), 8,77 (d, 1H).

Analog Beispiel **49A** wurde folgende Beispielverbindung aus dem entsprechenden 4,5-Dihydro-3*H*-2,3-benzodiazepin hergestellt:

| Nr | Struktur | Name | Analytische Daten |
|---|---|---|---|
| 15 | 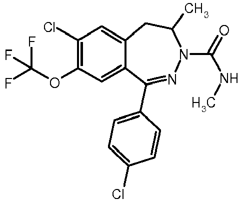 | (±)-7-Chlor-1-(4-chlorphenyl)-*N*,4-dimethyl-8-(trifluormethoxy)-4,5-dihydro-3*H*-2,3-benzodiazepin-3-carboxamid | ¹H-NMR (300MHz, DMSO-*d*₆): δ = 0,86 (d, 3H), 2,65 (d, 3H), 2,87 (dd, 1H), 3,04 (dd, 1H), 5,11 - 5,21 (m, 1H), 6,81 (q, 1H), 7,06 (s, 1H), 7,45 (d, 2H), 7,60 (d, 2H), 7,75 (s, 1H). |
| | | | LCMS (Methode 1): Rₜ = 1,57 min; m/z = 446;448 (Cl-Isotopenmuster, M+H)⁺ |

### Enantiomerentrennung

Präparative HPLC nach Methode IV

### Beispiel 15.1: 7-Chlor-1-(4-chlorphenyl)-N,4-dimethyl-8-(trifluormethoxy)-4,5-dihydro-3H-2,3-benzodiazepin-3-carboxamid, Enantiomer 1

HPLC (Methode F): Rₜ = 2,86 min, Reinheit >99%

### Enantiomer 2: 7-Chlor-1-(4-chlorphenyl)-N,4-dimethyl-8-(trifluormethoxy)-4,5-dihydro-3H-2,3-benzodiazepin-3-carboxamid, Enantiomer 2

HPLC (Methode F): Rₜ = 3,70 min, Reinheit >99%

### Beispiel 16

### (4S)-1-[4-(3,5-Dimethyl-1H-pyrazol-1-yl)phenyl]-8-methoxy-N,4-dimethyl-4,5-dihydro-3H-2,3-benzodiazepin-3-carboxamid

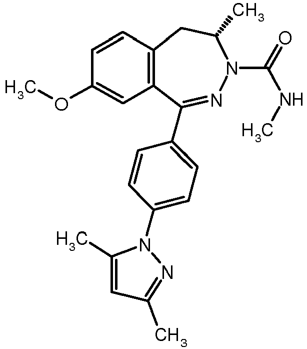

Unter Argon wurden 100 mg (0,30 mmol) (4*S*)-1-(4-Aminophenyl)-8-methoxy-*N*,4-dimethyl-4,5-dihydro-3*H*-2,3-benzodiazepin-3-carboxamid (Beispiel **53.2A**) in 5ml konzentrierter Salzsäure gelöst und auf 0°C abgekühlt. Innerhalb von 25 min wurde eine Lösung von 24,5 mg Natriumnitrit in 1 ml Wasser zudosiert und 30 min bei dieser Temperatur gerührt. Dann wurde eine Lösung aus 140 mg Zinn(II)chlorid in 1 ml konzentrierter Salzsäure binnen 30 min langsam zugetropft. Das Eisbad wurde entfernt und 45 min bei RT nachgerührt. Dann wurden 60,7 µl (0,59 mmol) 2,4-Pentandion zugegeben und 30 min nachgerührt. Zuletzt wurden 2 ml Acetonitril zugegeben und 1 h bei RT nachgerührt. Der Ansatz wurde auf Eiswasser gegeben und mit Natronlauge ein pH 10 eingestellt und dreimal mit Essigsäureethylester extrahiert. Das Lösemittel wurde am Rotationsverdampfer entfernt und der Rückstand flashchromatographisch gereinigt. Man erhielt 79 mg (63% d. Th.) des gewünschten Produkts.
UPLC/MS (Methode 2): Rₜ = 1,32 min; m/z = 418 (M+H)⁺
¹H-NMR (300MHz, CDCl₃): δ = 0,95 (d, 3H), 2,32 (s, 3H), 2,37 (s, 3H), 2,86 (dd, 1H), 2,90 (d, 3H), 3,11 (dd 1H), 3,69 (s, 3H), 5,39-5,48 (m, 1H), 6,03 (s, 1H), 6,46-6,54 (m, 1H), 6,66 (d, 1H), 6,89 (dd, 1H), 7,14 (d, 1H), 7,48 (d, 2H), 7,57 (d, 2H).

### Beispiel 17

### (4S)-8-Methoxy-N,4-dimethyl-1-[4-(morpholin-4-yl)phenyl]-4,5-dihydro-3H-2,3-benzodiazepin-3-carboxamid

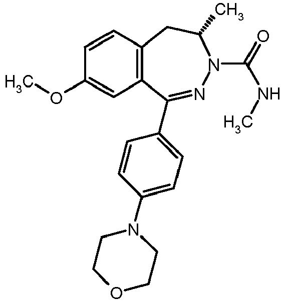

Unter Argon wurden 100 mg (0,30 mmol) (4*S*)-1-(4-Aminophenyl)-8-methoxy-*N*,4-dimethyl-4,5-dihydro-3*H*-2,3-benzodiazepin-3-carboxamid (Beispiel **53.2A**) in 4 ml *N*,*N*-Dimethylacetamid gelöst, mit 103 mg (0,44 mmol) 1-Brom-2-(2-bromethoxy)ethan und 0,1 ml (0,59 mmol) Diisopropylethylamin versetzt. Man rührte bei 120°C für drei Tage. Der Ansatz wurde auf Wasser gegeben und dreimal mit Ethylacetat extrahiert. Das Lösemittel wurde am Rotationsverdampfer entfernt und der Rückstand flashchromatographisch gereinigt. Man erhielt 95 mg (79% d. Th.) des gewünschten Produkts.
UPLC/MS (Methode 2): Rₜ = 1.18 min; m/z = 409 (M+H)⁺
¹H-NMR (300MHz, CDCl₃): δ = 1,07 (d, 3H), 2,72 (dd, 1H), 2,86 (d, 3H), 2,95 (dd, 1H), 3,25 (m 4H), 3,71 (s, 3H), 3,89 (m, 4H), 5,19-5,30 (m, 1H), 6,12 (m, 1H), 6,67 (d, 1H), 6,89 (dd, 1H), 6,92 (d, 2H), 7,15 (d, 1H), 7,51 (d, 2H).

### Beispiel 18

### (4S)-1-[4-(4-Isoxazolyl)phenyl]-8-methoxy-N,4-dimethyl-4,5-dihydro-3H-2,3-benzodiazepin-3-carboxamid

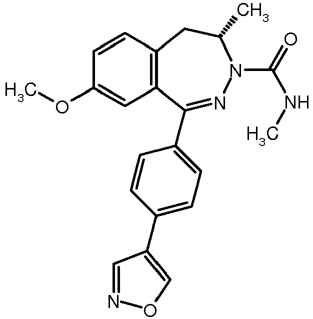

Die Reaktion wurde analog Beispiel 3 mit 100 mg (249 µmol) (4*S*)-1-(4-Bromphenyl)-8-methoxy-*N*,4-dimethyl-4,5-dihydro-3*H*-2,3-benzodiazepin-3-carboxamid (Beispiel **54A**), 3,6 ml 1,4-Dioxan, 56 mg (497 µmol) Isoxazol-4-boronsäure (CAS [1008139-25-0]), 0,25 ml 2 M wässriger Kaliumcarbonatlösung und 41 mg (50 µmol) Dichlor[1,1'-bis(diphenylphosphino)ferrocen]palladium(II) (Komplex mit CH₂Cl₂, CAS [95464-05-4]) durchgeführt. Man erhielt 6 mg (6% d. Th.) der Titelverbindung.
UPLC/MS (Methode 3): Rₜ = 1,17 min; m/z = 391 (M+H)⁺
¹H-NMR (300MHz, CDCl₃): δ = 0,94 (d, 3H), 2,87 (dd, 1H), 2,89 (d, 3H), 3,11 (dd, 1H), 3,69 (s, 3H), 5,43 (m, 1H), 6,51 (m, 1H), 6,65 (d, 1H), 6,89 (dd, 1H), 7,14 (d, 1H), 7,51 (d, 2H), 7,55 (d, 2H), 8,60 (s, 1H), 8,73 (s, 1H).

Analog zu Beispiel **3** wurden folgende Beispielverbindungen mit den entsprechenden, kommerziell erhältlichen Boronsäuren aus der in Beispiel **54A** erhaltenen Verbindung hergestellt:

| Nr | Struktur | Name | Analytische Daten |
|---|---|---|---|
| 19 | 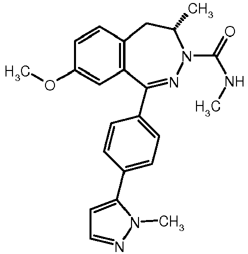 | (4*S*)-8-Methoxy-*N*,4-dimethyl-1-[4-(1-methyl-1*H*-pyrazol-5-yl)phenyl]-4,5-dihydro-3*H*-2,3-benzodiazepin-3-carboxamid | ¹H-NMR (300MHz, CDCl₃): δ = 0,95 (d, 3H), 2,87 (dd, 1H), 2,90 (d, 3H), 3,11 (dd, 1H), 3,71 (s, 3H), 3,95 (s, 3H), 5,44 (m, 1H), 6,36 (d, 1H), 6,52 (m, 1H), 6,68 (d, 1H), 6,90 (dd, 1H), 7,15 (d, 1H), 7,45 (d, 2H), 7,54 (d, 1H), 7,58 (d, 2H). |
| | | | UPLC/MS (Methode 3): Rₜ= 1,16 min; m/z = 404 (M+H)⁺ |
| 20 | 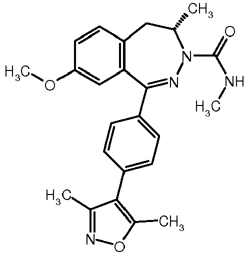 | (4*S*)-1-[4-(3,5-Dimethyl-4-isoxazolyl)phenyl]-8-methoxy-*N*,4-dimethyl-4,5-dihydro-3*H*-2,3-benzodiazepin-3-carboxamid | ¹H-NMR (300MHz, CDCl₃): δ = 0,97 (d, 3H), 2,33 (s, 3H), 2,46 (s, 3H), 2,86 (dd, 1H), 2,90 (d, 3H), 3,11 (dd, 1H), 3,72 (s, 3H), 5,42 (m, 1H), 6,48 (m, 1H), 6,70 (d, 1H), 6,90 (dd, 1H), 7,16 (d, 1H), 7,28 (d, 2H), 7,57 (d, 2H). |
| | | | UPLC/MS (Methode 3): Rₜ = 1,25 min; m/z = 419 (M+H)⁺ |
| 21 | 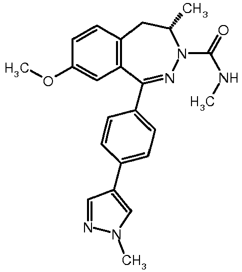 | (4*S*)-8-Methoxy-*N*,4-dimethyl-1-[4-(1-methyl-1*H-*pyrazol-4-yl)phenyl]-4,5-dihydro-3*H*-2,3-benzodiazepin-3-carboxamid | ¹H-NMR (300MHz, CDCl₃): δ = 0,99 (d, 3H), 2,82 (dd, 1H), 2,89 (d, 3H), 3,06 (dd, 1H), 3,69 (s, 3H), 3,97 (s, 3H), 5,38 (m, 1H), 6,42 (m, 1H), 6,68 (d, 1H), 6,89 (dd, 1H), 7,14 (d, 1H), 7,48 (d, 2H), 7,51 (d, 2H), 7,66 (s, 1H), 7,80 (s, 1H). |
| | | | UPLC/MS (Methode 3): Rₜ = 1,08 min; m/z = 404 (M+H)⁺ |
| 22 | 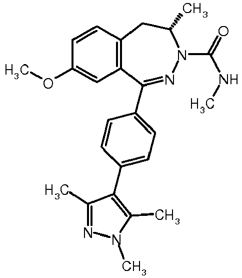 | (4*S*)-8-Methoxy-*N*,4-dimethyl-1-[4-(1,3,5-trimethyl-1*H*-pyrazol-4-yl)phenyl]-4,5-dihydro-3*H*-2,3-benzodiazepin-3-carboxamid | ¹H-NMR (300MHz, CDCl₃): δ = 1,00 (d, 3H), 2,29 (s, 6H), 2,82 (dd, 1H), 2,90 (d, 3H), 3,07 (dd, 1H), 3,72 (s, 3H), 3,80 (s, 3H), 5,38 (m, 1H), 6,41 (m, 1H), 6,73 (d, 1H), 6,90 (dd, 1H), 7,16 (d, 1H), 7,27 (d, 2H), 7,56 (d, 2H). |
| | | | UPLC/MS (Methode 3): Rₜ = 1,16 min; m/z = 432 (M+H)⁺ |
| 23 | 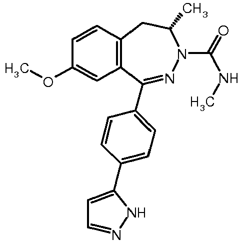 | (4*S*)-8-Methoxy-*N*,4-dimethyl-1-[4-(1*H-*pyrazol-5-yl)phenyl]-4,5-dihydro-3*H*-2,3-benzodiazepin-3-carboxamid | ¹H-NMR (300MHz, CDCl₃): δ = 0,97 (d, 3H), 2,85 (dd, 1H), 2,90 (d, 3H), 3,09 (dd, 1H), 3,68 (s, 3H), 5,41 (m, 1H), 6,49 (m, 1H), 6,68 (d, 1H), 6,68 (sbr, 1H), 6,89 (dd, 1H), 7,14 (d, 1H), 7,57 (d, 2H), 7,81 (d, 2H), 7,66 (sbr, 1H). |
| | | | UPLC/MS (Methode 3): Rₜ = 1,04 min; m/z = 390 (M+H)⁺ |

Analog zu Beispiel **16** wurden ausgehend vom Anilinderivat **53**.**2A** durch Umsetzung mit den entsprechenden, kommerziell erhältlichen Diketonen folgende Beispielverbindung hergestellt. Durch die Verwendung unsymmetrischer Diketone entstehen jeweils Regioisomere, die durch präparative HPLC getrennt werden können.

| Nr | Struktur | Name | Analytische Daten |
|---|---|---|---|
| 24 | 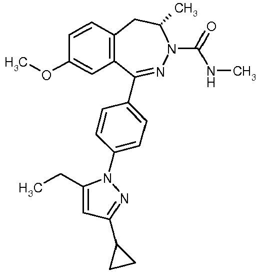 | (4*S*)-8-Methoxy-*N*,4-dimethyl-1-[4-(1-methyl-1*H*-pyrazol-5-yl)phenyl]-4,5-dihydro-3*H*-2,3-benzodiazepin-3-carboxamid | UPLC/MS (Methode 2): Rₜ = 1.46 min; m/z = 458 (M+H)⁺. |
| | | | ¹H-NMR (500 MHz, CDCl₃): δ = 0.79 -0.84 (m, 2H), 0.95 (d, 3H), 0.97 - 1.01 (m, 2H), 1.24 (t, 3H), 2.02 - 2.07 (m, 1H), 2.69 (q, 2H), 2.83 - 2.93 (m, 4H), 3.11(dd, 1H), 3.69 (s, 3H), 5.39 - 5.47 (m, 1H), 5.92 (s, 1H), 6.50 (m, 1H), 6.66 (d, 1H), 6.89 (dd, 1H), 7.14 (d, 1H), 7.46 (d, 2H), 7.58 (d, 2H). |
| 25 | 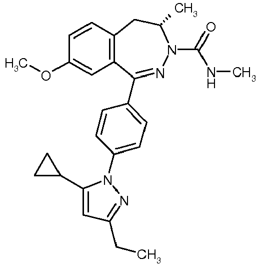 | (4*S*)-1-[4-(5-Cyclopropyl-3-ethyl-1*H*-pyrazol-1-yl)phenyl]-8-methoxy-*N*,4-dimethyl-4,5-dihydro-3*H*-2,3-benzodiazepin-3-carboxamid | UPLC/MS (Methode 2): Rₜ = 1.47 min; m/z = 458 (M+H)⁺. |
| | | | ¹H-NMR (300 MHz, CDCl₃): δ = 0.79 -0.84 (m, 2H), 0.95 (d, 3H), 1.0 - 1.06 (m, 2H), 1.28 (t, 3H), 1.8 - 1.9 (m, 1H), 2.71 (q, 2H), 2.84 - 2.95 (m, 4H), 3.07 - 3.14 (m, 1H), 3.69 (s, 3H), 5.37 - 5.47 (m, 1H), 5.84 (s, 1H), 6.45 - 6.54 (m, 1H), 6.66 (d, 1H), 6.89 (dd, 1H), 7.14 (d, 1H), 7.59 (d, 2H), 7.67 (d, 2H). |
| 26 | 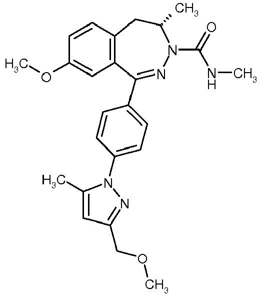 | (4*S*)-8-Methoxy-1-{4-[3-(methoxymethyl)-5-methyl-1*H*-pyrazol-1-yl]phenyl}-*N*,4-dimethyl-4,5-dihydro-3*H*-2,3-benzodiazepin-3-carboxamid | UPLC/MS (Methode 2): Rₜ = 1.25 min; m/z = 448 (M+H)⁺. |
| | | | ¹H-NMR (500 MHz, CDCl₃): δ = 0.94 (d, 3H), 2.40 (s, 3H), 2.84 - 2.93 (m, 4H), 3.12 (dd, 1H), 3.45 (s, 3H), 3.69 (s, 3H), 4.52 (s, 2H), 5.44 (m, 1H), 6.28 (s, 1H), 6.51 (m, 1H), 6.66 (d, 1H), 6.89 (dd, 1H), 7.14 (d, 1H), 7.48 (d, 2H), 7.59 (d, 2H). |
| 27 | 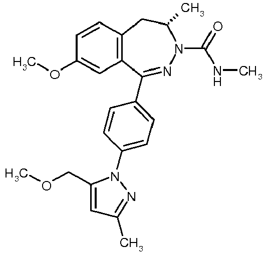 | (4*S*)-8-Methoxy-1-{4-[5-(methoxymethyl)-3-methyl-1*H*-pyrazol-1-yl]phenyl}-*N*,4-dimethyl-4,5-dihydro-3*H*-2,3-benzodiazepin-3-carboxamid | UPLC/MS (Methode 2): Rₜ = 1.28 min; m/z = 448 (M+H)⁺. |
| | | | ¹H-NMR (300 MHz, CDCl₃): δ = 0.95 (d, 3H), 2.36 (s, 3H), 2.82 - 2.93 (m, 4H), 3.10 (dd, 1H), 3.41 (s, 3H), 3.68 (s, 3H), 4.42 (dd, 2H), 5.37 - 5.48 (m, 1H), 6.29 (s, 1H), 6.49 (m, 1H), 6.67 (d, 1H), 6.89 (dd, 1H), 7.14 (d, 1H), 7.58 (d, 2H), 7.64 (d, 2H). |
| 28 | 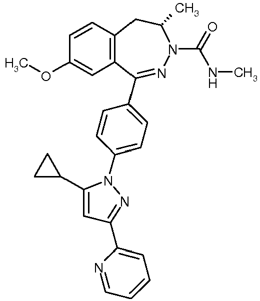 | (4*S*)-1-{4-[5-Cyclopropyl-3-(pyridin-2-yl)-1*H-*pyrazol-1-yl]phenyl}-8-methoxy-*N*,4-dimethyl-4,5-dihydro-3*H*-2,3-benzodiazepin-3-carboxamid | UPLC/MS (Methode 2): Rₜ = 1.24 min; m/z = 507 (M+H)⁺. |
| | | | ¹H-NMR (500MHz, CDCl₃): δ = 0.88 - 0.92 (m, 2H), 0.96 (d, 3H), 1.08 - 1.13 (m, 2H), 1.90 - 1.96 (m, 1H), 2.85 - 2.94 (m, 4H), 3.13 (dd, 1H), 3.71 (s, 3H), 5.45 (m, 1H), 6.38 (s, 1H), 6.53 (m, 1H), 6.70 (d, 1H), 6.91 (dd, 1H), 7.16 (d, 1H), 7.47 (br. s., 1H), 7.64 (d, 2H), 7.74 (d, 2H), 8.32 (d, 1H), 8.59 (br s, 1H), 9.10 (br s, 1H). |
| 29 | 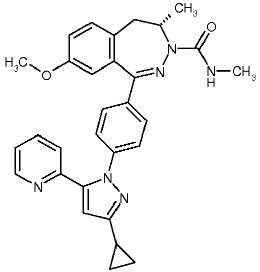 | (4*S*)-1-{4-[3-Cyclopropyl-5-(pyridin-2-yl)-1*H-*pyrazol-1-yl]phenyl}-8-methoxy-*N*,4-dimethyl-4,5-dihydro-3*H*-2,3-benzodiazepin-3-carboxamid | UPLC/MS (Methode 2): Rₜ = 1.32 min; m/z = 507 (M+H)⁺. |
| | | | ¹H-NMR (500MHz, CDCl₃): δ = 0.85 - 0.89 (m, 2H), 0.93 (d, 3H), 1.00 - 1.06 (m, 2H), 2.07 (tt, 2H), 2.82 - 2.91 (m, 4H), 3.08 (dd, 1H), 3.68 (s, 3H), 5.38 - 5.45 (m, 1H), 6.30 (s, 1H), 6.45 (m, 1H), 6.59 (d, 1H), 6.87 (dd, 1H), 7.12 (d, 1H), 7.28 (d, 2H), 7.32 - 7.44 (m, 1H), 7.49 (d, 2H), 7.66 (d, 1H), 8.59 (br s, 1H). |

### Beispiel 30

### (4S)-8-Methoxy-N,4-dimethyl-1-[4-(1H-tetrazol-1-yl)phenyl]-4,5-dihydro-3H-2,3-benzodiazepin-3-carboxamid

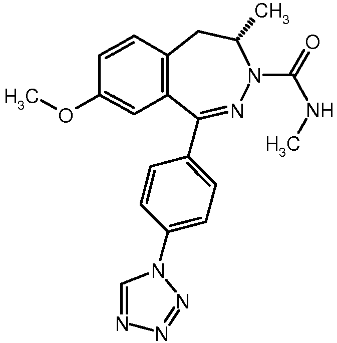

Unter Argon wurden 100 mg (0,30 mmol) (4*S*)-1-(4-Aminophenyl)-8-methoxy-*N*,4-dimethyl-4,5-dihydro-3*H*-2,3-benzodiazepin-3-carboxamid (Beispiel **53.2A**) mit 157 µl (0,95 mmol) Orthoameisensäuretriethylester, 271 µl (4,73 mmol) Essigsäure und 24 mg (0,37 mmol) Natriumazid für 3 Stunden bei 80°C gerührt. Der Ansatz wurde auf gesättigte wässrige Natriumhydrogencarbonatlösung gegeben und dreimal mit Essigsäureethylester extrahiert. Das Lösemittel wurde am Rotationsverdampfer entfernt und der Rückstand flashchromatographisch gereinigt. Man erhielt 74 mg (63% d. Th.) des gewünschten Produkts.
UPLC/MS (Methode 3): Rₜ = 1,04 min; m/z = 392 (M+H)⁺
¹H-NMR (300MHz, CDCl₃): δ = 0,91 (d, 3H), 2,92 (dd, 1H), 2,90 (d, 3H), 3,15 (dd, 1H), 3,70 (s, 3H), 5,50 (m, 1H), 6,55 (m, 1H), 6,61 (d, 1H), 6,90 (dd, 1H), 7,16 (d, 1H), 7,69 (d, 2H), 7,74 (d, 2H), 9,05 (s, 1H).

### Beispiel 31

### (±)-1-[3-(3,5-Dimethylisoxazol-4-yl)phenyl]-7,8-dimethoxy-N,4-dimethyl-4,5-dihydro-3H-2,3-benzodiazepin-3-carboxamid

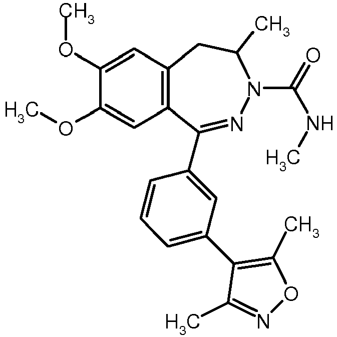

Die Reaktion wurde analog Beispiel **2** mit 170 mg (393 µmol) (±)-1-(3-Bromphenyl)-7,8-dimethoxy-*N*,4-dimethyl-4,5-dihydro-3*H*-2,3-benzodiazepin-3-carboxamid (Beispiel **51A**), 4 ml 1,4-Dioxan, 111 mg (768 µmol) 3,5-Dimethylisoxazol-4-boronsäure (CAS [16114-47-9]), 0,39 ml 2 M wässriger Kaliumcarbonatlösung und 64 mg (79 µmol) Dichlor[1,1'-bis(diphenylphosphino)ferrocen]palladium(II) (Komplex mit CH₂Cl₂, CAS [95464-05-4]) durchgeführt. Man erhielt 97 mg (54% d. Th.) der Titelverbindung.
UPLC/MS (Methode 3): Rₜ = 1,16 min; m/z = 449 (M+H)⁺
¹H-NMR (300MHz, CDCl₃): δ = 0,98 (d, 3H), 2,27 (s, 3H), 2,41 (s, 3H), 2,86 (dd, 1H), 2,89 (d, 3H), 3,13 (dd, 1H), 3,66 (s, 3H), 3,94 (s, 3H), 5,46 (m, 1H), 6,52 (m, 1H), 6,61 (s, 1H), 6,73 (s, 1H), 7,29 (m, 1H), 7,33 (sbr, 1H), 7,47 (m, 1H), 7,51 (m, 1H).

### Beispiel 32

### (±)-1-(4-Chlorphenyl)-N,4-dimethyl-8-[2-(morpholin-4-yl)ethoxy]-4,5-dihydro-3H-2,3-benzodiazepin-3-carboxamid

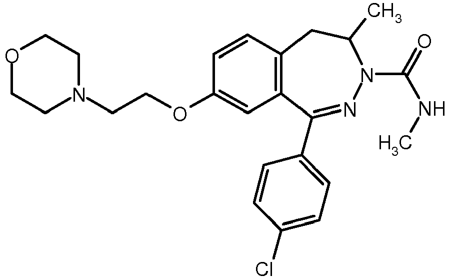

Eine Lösung von 57,4 mg (167 µmol) (±)-1-(4-Chlorphenyl)-*N*,4-dimethyl-8-hydroxy-4,5-dihydro-3*H*-2,3-benzodiazepin-3-carboxamid (Beispiel **55A**) in 5 ml *N,N*-Dimethylformamid wurde mit 272 mg Caesiumcarbonat versetzt und 1 Stunde bei Raumtemperatur gerührt. Anschließend wurden 77,7 mg (417 µmol) 4-(2-Chlorethyl)morpholin hinzugegeben. Die Mischung wurde 16 Stunden bei 60°C gerührt. Zur Aufarbeitung wurde das Reaktionsgemisch eingeengt, mit Wasser versetzt und dreimal mit Ethylacetat extrahiert. Die organische Phase wurde über Natriumsulfat getrocknet, filtriert und eingeengt. Nach Flashchromatographie erhielt man 26 mg (33% d. Th.) der Titelverbindung.
UPLC/MS (Methode 3): Rₜ = 0,88 min; m/z = 457; 459 (M+H, Cl-Isotopenmuster)⁺
¹H-NMR (300MHz, CDCl₃): δ = 0,92 (d, 3H), 2,53 (m, 4H), 2,73 (m, 2H), 2,85 (dd, 1H), 2,88 (d, 3H), 3,08 (dd, 1H), 3,72 (m, 4H), 3,97 (m, 2H), 5,42 (m, 1H), 6,60 (d, 1H), 6,88 (dd, 1H), 7,12 (d, 1H), 7,36 (d, 2H), 7,41 (d, 2H).

Analog zu Beispiel **32** wurde folgende Beispielverbindung mit dem entsprechenden, kommerziell erhältlichen Heterocyclylchloralkan hergestellt:

| Nr | Struktur | Name | Analytische Daten |
|---|---|---|---|
| 33 | 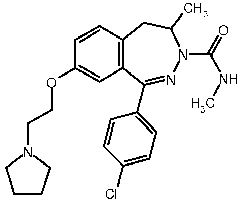 | (±)-1-(4-Chlorphenyl)-*N*,4-dimethyl-8-[2-(pyrrolidin-1-yl)ethoxy]-4,5-dihydro-3*H*-2,3-benzodiazepin-3-carboxamid | ¹H-NMR (300MHz, CDCl₃): δ = 0,92 (d, 3H), 1,81 (m, 4H), 2,63 (m, 4H), 2,85 (dd, 1H), 2,87 (m, 2H), 2,88 (d, 3H), 3,08 (dd, 1H), 4,00 (m, 2H), 5,42 (m, 1H), 6,45 (m, 1H), 6,61 (d, 1H), 6,90 (dd, 1H), 7,11 (d, 1H), 7,36 (d, 2H), 7,41 (d, 2H). |
| | | | UPLC/MS (Methode 3): Rₜ = 0,88 min; m/z = 457; 459 (M+H, Cl-Isotopenmuster)⁺ |

### Beispiel 34

### (±)-7,8-Dimethoxy-N,4-dimethyl-1-[4-(2-oxooxazolidin-3-yl)phenyl]-4,5-dihydro-3H-2,3-benzodiazepin-3-carboxamid

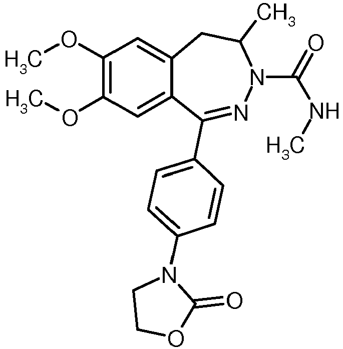

100 mg (0,231 mmol) (±)-1-(4-Bromphenyl)-7,8-dimethoxy-*N*,4-dimethyl-4,5-dihydro-3*H*-2,3-benzodiazepin-3-carboxamid (Beispiel **49A**), 22 mg (0,254 mmol) Oxazolidin-2-on, 98 mg (0,46 mmol) Kaliumphosphat und 88 mg (0,46 mmol) Kupfer(I)iodid wurden unter Argon in 4 ml entgastem Dioxan vorgelegt. Anschließend wurden unter Argon 82 mg (0,93 mmol) *N*,*N-*Dimethylethylendiamin zugegeben, erneut entgast und die Mischung für 3 Stunden bei 130°C erhitzt. Nach dem Abkühlen wurde die Mischung mit Ethylacetat und gesättigter wässriger Ammoniumchloridlösung versetzt. Die wässrige Phase wurde noch dreimal mit Ethylacetat extrahiert, die vereinigten organischen Phasen mit Natriumsulfat getrocknet. Das Lösemittel wurden am Rotationsverdampfer entfernt und der Rückstand mittels präparativer HPLC gereinigt. Man erhielt 50 mg (49% d. Th.) des gewünschten Produkts als Feststoff.
LCMS (Methode 2): Rₜ = 1,0 min; m/z = 439 (M+H)⁺
¹H-NMR (400MHz, CDCl₃): δ = 1,03 (d, 3H), 2,82 (dd, 1H), 2,88 (s, br, 3H), 3,08 (dd, 1H), 3,67 (s, 3H), 3,94 (s, 3H), 4,09-4,16 (m, 2H), 4,53 (t, 2H), 5,37-5,47 (m, 1H), 6,41 (m, 1H), 6,59 (s, 1H), 6,74 (s, 1H), 7,55 (d, 2H), 7,60 (d, 2H).

### Beispiel 34.1

(4*S*)-7,8-Dimethoxy-*N*,4-dimethyl-1-[4-(2-oxooxazolidin-3-yl)phenyl]-4,5-dihydro-3*H*-2,3-benzodiazepin-3-carboxamid

Analog der Herstellung von Beispiel **34** wurden ausgehend von 100 mg (0,231 mmol) (4*S*)-1-(4-Bromphenyl)-7,8-dimethoxy-*N*,4-dimethyl-4,5-dihydro-3*H*-2,3-benzodiazepin-3-carboxamid (Beispiel **49.2A**) durch Umsetzung mit 22 mg (0,254 mmol) Oxazolidin-2-on nach Aufreinigung mittels präparativer HPLC 84 mg (82% d. Th.) des gewünschten Produkts als Feststoff erhalten.
LCMS (Methode 2): Rₜ = 1,0 min; m/z = 439 (M+H)⁺
[α]_{D}²⁰ = 237,1° (c = 1,00; Methanol)

Analog Beispiel **34** wurden aus Beispiel **49A** bzw. Beispiel **49.2A** und den entsprechenden kommerziell erhältlichen Lactamen bzw. cyclischen Carbamaten folgende Beispielverbindungen hergestellt:

| Nr | Struktur | Name | Analytische Daten |
|---|---|---|---|
| **35** | 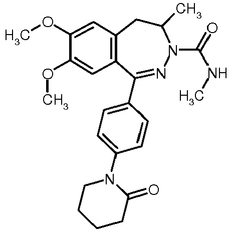 | (±)-7,8-Dimethoxy-*N*,4-dimethyl-1-[4-(2-oxopiperidin-1-yl)phenyl]-4,5-dihydro-3*H*-2,3-benzodiazepin-3-carboxamid | ¹H-NMR (300MHz, CDCl₃): δ = 1,00 (d, 3H), 1,92-2,02 (m, 2H), 2,54-2,65 (m, 2H), 2,80 (dd, 1H), 2,88 (s, br, 3H), 3,06 (d, 1H), 3,67 (s, 3H), 3,68-3,75 (m, 2H), 3,93 (s, 3H), 5,41 (q, 1H), 6,44 (m, 1H), 6,63 (s, 3H), 6,72 (s, 1H), 7,30 (d, 2H), 7,53 (d, 2H). |
| | | | LCMS (Methode 2): Rₜ = 1,04 min; m/z = 451 (M+H)⁺ |
| **36** | 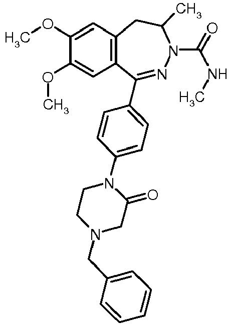 | (±)-1-[4-(4-Benzyl-2-oxopiperazin-1-yl)phenyl]-7,8-dimethoxy-*N*,4-dimethyl-4,5-dihydro-3*H*-2,3-benzodiazepin-3-carboxamid | ¹H-NMR (400MHz, CDCl₃): δ = 0,99 (d, 3H), 2,78-2,87 (m, 3H), 2,89 (d, 3H), 3,08 (dd, 1H), 3,37 (s, 2H), 3,65 (s, 2H), 3,67 (s, 3H), 3,69-3,77 (m, 2H), 3,93 (s, 3H), 5,38-5,47 (m, 1H), 6,45 (m, 1H), 6,62 (s, 1H), 6,72 (s, 1H), 7,30-7,39 (m, 7H) 7,53 (d, 2H). |
| | | | LCMS (Methode 2): Rₜ = 1,06 min; m/z = 542 (M+H)⁺ |
| **37** | 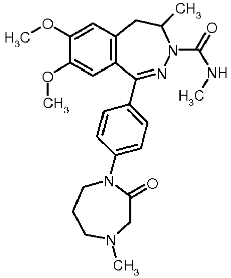 | (±)-7,8-Dimethoxy-*N*,4-dimethyl-1-[4-(4-methyl-2-oxo-1,4-diazepan-1-yl)phenyl]-4,5-dihydro-3*H*-2,3-benzodiazepin-3-carboxamid | ¹H-NMR (300MHz, CDCl₃): δ = 0,98 (d, 3H), 2,06-2,20 (m, 2H), 2,70 (s, 3H), 2,82 (dd, 1H), 2,88 (d, 3H), 3,08 (dd, 1H), 3,18 (m, 2H), 3,68 (s, 3H), 3,82 (s, br, 2H), 3,90-4,01 (m, 2H), 3,93 (s, 3H), 5,43 (m, 1H), 6,46 (q, 1H), 6,62 (s, 1H), 6,72 (s, 1H), 7,28 (d, 2H) 7,53 (d, 2H). |
| | | | LCMS (Methode 2): Rₜ = 0,70 min; m/z = 480 (M+H)⁺ |
| **38** | 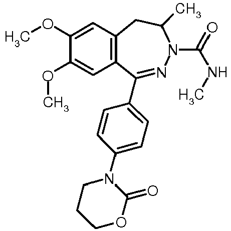 | (±)-7,8-Dimethoxy-*N*,4-dimethyl-1-[4-(2-oxo-1,3-oxazinan-3-yl)phenyl]-4,5-dihydro-3*H*-2,3-benzodiazepin-3-carboxamid | ¹H-NMR (400MHz, CDCl₃): δ = 1,00 (d, 3H), 2,20-2,30 (m, 2H), 2,82 (dd, 1H), 2,88 (d, 3H), 3,08 (dd, 1H), 3,67 (s, 3H), 3,78 (t, 2H), 3,93 (s, 3H), 4,45 (t, 2H), 5,37-5,48 (m, 1H), 6,45 (m, 1H), 6,62 (s, 1H), 6,72 (s, 1H), 7,38 (d, 2H), 7,54 (d, 2H). |
| | | | LCMS (Methode 2): Rₜ = 0,96 min; m/z = 453 (M+H)⁺ |
| **39** | 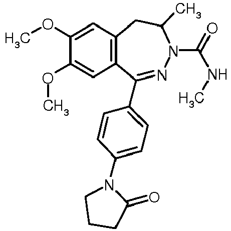 | (±)-7,8-Dimethoxy-*N*,4-dimethyl-1-[4-(2-oxopyrrolidin-1-yl)phenyl]-4,5-dihydro-3*H*-2,3-benzodiazepin-3-carboxamid | ¹H-NMR (400MHz, CDCl₃): δ = 1,03 (d, 3H), 2,16-2,25 (m, 2H), 2,65 (t, 2H), 2,80 (dd, 1H), 2,88 (d, 3H), 3,07 (dd, 1H), 3,66 (s, 3H), 3,88-3,93 (m, 2H), 3,93 (s, 3H), 5,35-5,45 (m, 1H), 6,40 (s, br, 1H), 6,60 (s, 1H), 6,73 (s, 1H), 7,53 (d, 2H), 7,68 (d, 2H). |
| | | | LCMS (Methode 2): Rₜ = 1,04 min; m/z = 437 (M+H)⁺ |
| **40** | 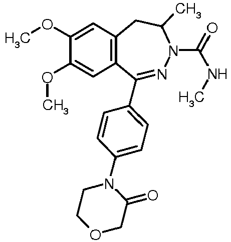 | (±)-7,8-Dimethoxy-*N*,4-dimethyl-1-[4-(3-oxomorpholin-4-yl)phenyl]-4,5-dihydro-3*H*-2,3-benzodiazepin-3-carboxamid | ¹H-NMR (400MHz, CDCl₃): δ = 1,01 (d, 3H), 2,82 (dd, 1H), 2,89 (d, 3H), 3,08 (dd, 1H), 3,68 (s, 3H), 3,80-3,86 (m, 2H), 3,94 (s, 3H), 4,07 (t, 2H), 4,37 (s, 2H), 5,39-5,49 (m, 1H), 6,46 (s, br, 1H), 6,62 (s, 1H), 6,73 (s, 1H), 7,40 (d, 2H), 7,57 (d, 2H). |
| | | | LCMS (Methode 2): Rₜ = 0,95 min; m/z = 453 (M+H)⁺ |
| **40**.**1** | 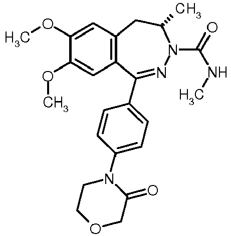 | (4*S*)-7,8-Dimethoxy-*N*,4-dimethyl-1-[4-(3-oxomorpholin-4-yl)phenyl]-4,5-dihydro-3*H*-2,3-benzodiazepin-3-carboxamid | ¹H-NMR (400MHz, CDCl₃): δ = 0,99 (d, 3H), 2,83 (dd, 1H), 2,89 (s, br, 3H), 3,09 (dd, 1H), 3,67 (s, 3H), 3,80-3,86 (m, 2H), 3,94 (s, 3H), 4,06 (t, 2H), 4,37 (s, 2H), 5,39-5,49 (m, 1H), 6,47 (s, br, 1H), 6,62 (s, 1H), 6,72 (s, 1H), 7,40 (d, 2H), 7,56 (d, 2H). |
| | | | LCMS (Methode 2): Rₜ = 0,95 min; m/z = 453 (M+H)⁺. |
| | | | [α]_{D}²⁰ = 135,4° (c = 1,00; Methanol) |
| **41** | 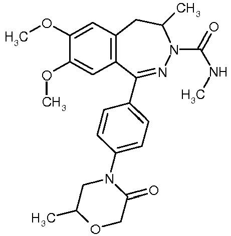 | (±)-7,8-Dimethoxy-*N*,4-dimethyl-1-[4-(2-methyl-5-oxomorpholin-4-yl)phenyl]-4,5-dihydro-3*H*-2,3-benzodiazepin-3-carboxamid (Stereoisomerengemisch) | ¹H-NMR (400MHz, CDCl₃): δ = 1,01 (d, 3H), 1,36 (d, 3H), 2,82 (dd, 1H), 2,89 (s, br, 3H), 3,09 (d, 1H), 3,54-3,62 (s, 1H), 3,68 (s, 3H), 3,69-3,77 (m, 1H), 3,94 (s, 3H), 4,05-4,16 (m, 1H), 4,35 (d, 1H), 4,43 (d, 1H), 5,44 (m, 1H), 6,45 (s, br, 1H), 6,62 (s, 1H), 6,73 (s, 1H), 7,39 (d, 2H), 7,56 (d, 2H). |
| | | | LCMS (Methode 2): Rₜ = 1,03 min; m/z = 467 (M+H)⁺ |
| **42** | 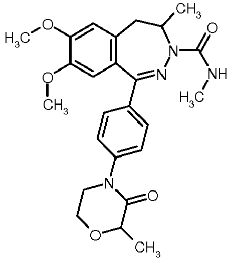 | (±)-7,8-Dimethoxy-*N*,4-dimethyl-1-[4-(2-methyl-3-oxomorpholin-4-yl)phenyl]-4,5-dihydro-3*H*-2,3-benzodiazepin-3-carboxamid (Stereoisomerengemisch) | ¹H-NMR (400MHz, CDCl₃): δ = 1,01 (d, 3H), 1,57 (dd, 3H), 2,82 (dd, 1H), 2,88 (s, br, 3H), 3,09 (d, 1H), 3,61-3,66 (m, 1H), 3,67/3,68 (s, 3H), 3,94 (s, 3H), 3,98-4,08 (m, 2H), 4,11-4,18 (m, 1H), 4,38-4,46 (m, 1H), 5,39-5,49 (m, 1H), 6,45 (s, br, 1H), 6,63 (s, 1H), 6,73 (s, 1H), 7,38 (d, 2H), 7,56 (d, 2H). |
| | | | LCMS (Methode 2): Rₜ = 1,02 min; m/z = 467 (M+H)⁺ |
| **43** | 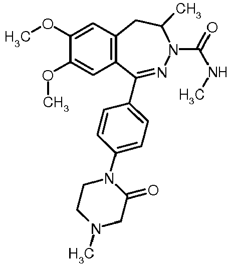 | (±)-7,8-Dimethoxy-*N*,4-dimethyl-1-[4-(4-methyl-2-oxopiperazin-1-yl)phenyl]-4,5-dihydro-3*H*-2,3-benzodiazepin-3-carboxamid | ¹H-NMR (500MHz, CDCl₃): δ = 0,97 (d, 3H), 2,84 (dd, 1H), 2,89 (s, 3H), 2,99 (s, 3H), 3,11 (dd, 1H), 3,37 (br. s., 1H), 3,64 (br. s., 1H), 3,67 (s, 3H), 3,77 (br. s., 2H), 3,94 (s, 3H), 4,27 (br. s., 1H), 4,83 (br. s., 1H), 5,46 (td, 1H), 6,49 (br. s., 1H), 6,59 (s, 1H), 6,72 (s, 1H), 7,38 (d, 2H), 7,56 (d, 2H). |
| | | | LCMS (Methode 2): Rₜ = 0,70 min; m/z = 466 (M+H)⁺. |
| **43**.**1** | 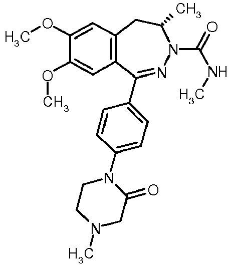 | (4*S*)-7,8-Dimethoxy-*N*,4-dimethyl-1-[4-(4-methyl-2-oxopiperazin-1-yl)phenyl]-4,5-dihydro-3*H*-2,3-benzodiazepin-3-carboxamid | Präp. HPLC: Methode XIV |
| | | | Analyt. HPLC (Methode S): Rₜ = 8,73 min |
| | | | LCMS (Methode 2): Rₜ = 0,70 min; m/z = 466 (M+H)⁺. |
| | | | [α]_{D}²⁰ = 107,0° (c = 1,00; Methanol) |
| **43**.**2** | 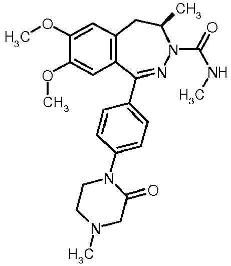 | (4R)-7,8-Dimethoxy-N,4-dimethyl-1-[4-(4-methyl-2-oxopiperazin-1-yl)phenyl]-4,5-dihydro-3*H*-2,3-benzodiazepin-3-carboxamid | Präp. HPLC: Methode XIV |
| | | | Analyt. HPLC (Methode S): Rₜ = 5,90 min |
| | | | LCMS (Methode 2): Rₜ = 0,70 min; m/z = 466 (M+H)⁺. |
| | | | [α]_{D}²⁰ = -119,4° (c = 1,00; Methanol) |

### Beispiel 44

### (±)-7,8-Dimethoxy-N,4-dimethyl-1-[4-(2-oxopiperazin-1-yl)phenyl]-4,5-dihydro-3H-2,3-benzodiazepin-3-carboxamid

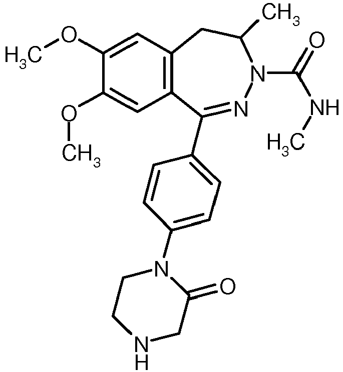

87 mg (0,161 mmol) (±)-1-[4-(4-Benzyl-2-oxopiperazin-1-yl)phenyl]-7,8-dimethoxy-*N*,4-dimethyl-4,5-dihydro-3*H*-2,3-benzodiazepin-3-carboxamid (Beispiel **36**) wurden unter Argon in 1 ml Ethanol vorgelegt und mit 17 mg Palladium auf Kohle (10%) versetzt. Man hydrierte mit Wasserstoff mit aufgesetztem Ballon für 16h. Da der Umsatz noch nicht vollständig war, wurde eine Spatelspitze Katalysator erneut zugefügt und weitere 16h mit Wasserstoff hydriert. Der Rohansatz wurde mittels PTFE-Filter vom Katalysator befreit, mit Ethylacetat nachgewaschen und anschließend das Lösemittel am Rotationsverdampfer entfernt. Der Rückstand wurde mittels präparativer HPLC gereinigt. Man erhielt 20 mg (20% d. Th.) des gewünschten Produkts als Feststoff.
LCMS (Methode 2): Rₜ = 0,67 min; m/z = 452 (M+H)⁺
¹H-NMR (300MHz, CDCl₃): δ = 0,98 (d, 3H), 2,78-2,87 (m, 1H), 2,88 (d, 3H), 3,09 (dd, 1H), 3,49 (m, 2H), 3,67 (s, 3H), 3,74-3,79 (m, 1H), 3,86-3,91 (m, 1H), 3,93 (s, 3H), 3,95-4,01 (m, 2H), 4,22 (s, br 1H), 5,39-5,49 (m, 1H), 6,44-6,51 (m, 1H), 6,60 (s, 1H), 6,72 (s, 1H), 7,35 (d, 2H), 7,55 (d, 2H).

Analog zu Beispiel **2** wurden aus Beispiel **49A** bzw. Beispiel **49.2A** und den entsprechenden kommerziell erhältlichen Boronsäurederivaten folgende Beispielverbindungen hergestellt:

| Nr | Struktur | Name | Analytische Daten |
|---|---|---|---|
| **45** | 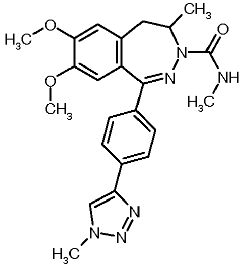 | (±)-7,8-Dimethoxy-*N*,4-dimethyl-1-[4-(1-methyl-1*H*-1,2,3-triazol-4-yl)phenyl]-4,5-dihydro-3*H*-2,3-benzodiazepin-3-carboxamid | ¹H-NMR (400MHz, CDCl₃): δ = 1,01 (d, 3H), 2,84 (dd, 1H), 2,90 (d, 3H), 3,12 (dd, 1H), 3,66 (s, 3H), 3,94 (s, 3H), 4,18 (s, 3H), 5,40-5,50 (m, 1H), 6,46-6,53 (m, 1H), 6,63 (s, 1H), 6,73 (s, 1H), 7,57 (d, 2H), 7,80 (s, 1H), 7,86 (d, 2H). |
| | | | LCMS (Methode 2): Rₜ = 1,01 min; m/z = 435 (M+H)⁺ |
| **45.1** | 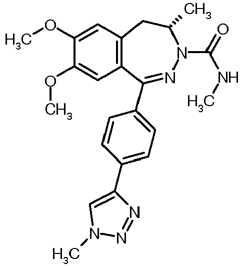 | (4*S*)-7,8-Dimethoxy-*N*,4-dimethyl-1-[4-(1-methyl-1*H*-1,2,3-triazol-4-yl)phenyl]-4,5-dihydro-3*H*-2,3-benzodiazepin-3-carboxamid | LCMS (Methode 2): Rₜ = 1,00 min; m/z = 435 (M+H)⁺ |
| | | | [α]_{D}²⁰ = -71,3° (c = 1,00; Chloroform) |
| **46** | 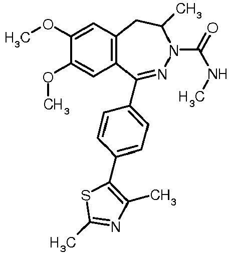 | (±)-1-[4-(2,4-Dimethylthiazol-5-yl)phenyl]-7,8-dimethoxy-*N*,4-dimethyl-4,5-dihydro-3*H*-2,3-benzodiazepin-3-carboxamid | ¹H-NMR (300MHz, CDCl₃): δ = 1,01 (d, 3H), 2,51 (s, 3H), 2,71 (s, 3H), 2,84 (dd, 1H), 2,90 (d, 3H), 3,12 (dd, 1H), 3,68 (s, 3H), 3,94 (s, 3H), 5,44 (m, 1H), 6,48 (q, 1H), 6,63 (s, 1H), 6,73 (s, 1H), 7,44 (d, 2H), 7,55 (d, 2H). |
| | | | LCMS (Methode 2): Rₜ = 1,24 min; m/z = 465 (M+H)⁺ |
| **46.1** | 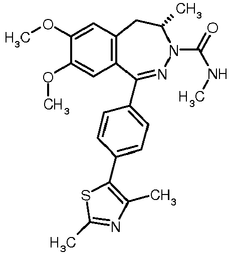 | (4*S*)-1-[4-(2,4-Dimethylthiazol-5-yl)phenyl]-7,8-dimethoxy-*N*,4-dimethyl-4,5-dihydro-3*H*-2,3-benzodiazepin-3-carboxamid Enantiomer 1 von Bsp. | HPLC (Methode L): Rₜ = 5,9 min |
| | | **46** | |
| | | Trennung HPLC-Methode VIII | |
| **46.2** | 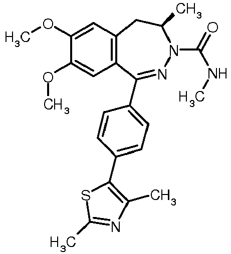 | (4*R*)-1-[4-(2,4-Dimethylthiazol-5-yl)phenyl]-7,8-dimethoxy-*N*,4-dimethyl-4,5-dihydro-3*H*-2,3-benzodiazepin-3-carboxamid Enantiomer 2 von Bsp. | HPLC (Methode L): Rₜ = 8,9 min |
| | | | [α]_{D}²⁰ = -127,2° (c = 1,00; Methanol) |
| | | **46** | |
| | | Trennung HPLC-Methode VIII | |
| **47** | 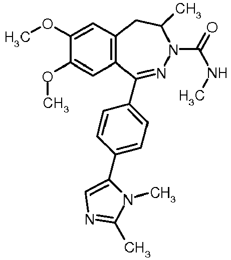 | (±)-1-[4-(1,2-Dimethyl-1*H*-imidazol-5-yl)phenyl]-7,8-dimethoxy-*N*,4-dimethyl-4,5-dihydro-3*H*-2,3-benzodiazepin-3-carboxamid | ¹H-NMR (300MHz, CDCl₃): δ = 1,01 (d, 3H), 2,46 (s, 3H), 2,83 (dd, 1H), 2,89 (d, 3H), 3,10 (dd, 1H), 3,58 (s, 3H), 3,68 (s, 3H), 3,94 (s, 3H), 5,43 (m, 1H), 6,47 (q, 1H), 6,63 (s, 1H), 6,73 (s, 1H), 7,03 (s, 1H), 7,39 (d, 2H), 7,57 (d, 2H). |
| | | | LCMS (Methode 2): Rₜ = 0,75 min; m/z = 448 (M+H)⁺ |
| **48** | 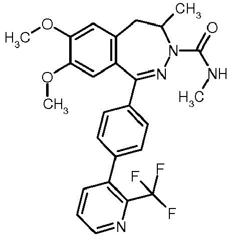 | (±)-7,8-Dimethoxy-*N*,4-dimethyl-1-{4-[2-(trifluormethyl)pyridin-3-yl]phenyl}-4,5-dihydro-3*H*-2,3-benzodiazepin-3-carboxamid | ¹H-NMR (400MHz, CDCl₃): δ = 1,01 (d, 3H), 2,87 (dd, 1H), 2,92 (d, 3H), 3,17 (dd, 1H), 3,67 (s, 3H), 3,94 (s, 3H), 5,50 (m, 1H), 6,56 (q, 1H), 6,59 (s, 1H), 6,73 (s, 1H), 7,37 (d, 2H), 7,54-7,61 (m, 3H), 7,79 (dd, 1H), 8,76 (dd, 1H). |
| | | | LCMS (Methode 2): Rₜ = 1,28 min; m/z = 499 (M+H)⁺ |
| **49** | 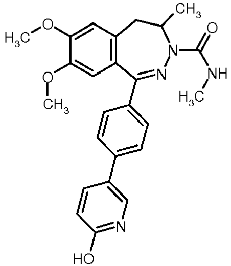 | (±)-1-[4-(6-Hydroxypyridin-3-yl)phenyl]-7,8-dimethoxy-*N*,4-dimethyl-4,5-dihydro-3*H*-2,3-benzodiazepin-3-carboxamid | ¹H-NMR (400MHz, CDCl₃): δ = 1,01 (d, 3H), 2,84 (dd, 1H), 2,91 (d, 3H), 3,12 (dd, 1H), 3,68 (s, 3H), 3,95 (s, 3H), 5,45 (m, 1H), 6,48 (q, 1H), 6,62 (s, 1H), 6,69-6,80 (s, br 1H), 6,74 (s, 1H), 7,46 (s, br, 2H), 7,58 (d, 2H), 7,73 (s, br, 1H), 7,86 (s, br, 1H). |
| | | | LCMS (Methode 2): Rₜ = 0,94 min; m/z = 447 (M+H)⁺ |
| **49**.**1** | 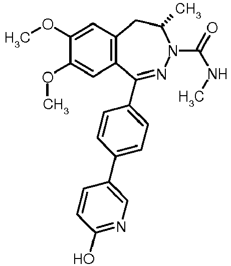 | (4*S*)-1-[4-(6-Hydroxypyridin-3-yl)phenyl]-7,8-dimethoxy-*N*,4-dimethyl-4,5-dihydro-3*H*-2,3-benzodiazepin-3-carboxamid | LCMS (Methode 2): Rₜ = 0,97 min; m/z = 447 (M+H)⁺ |
| | | | [α]_{D}²⁰ = -102,3° (c = 1,00; CHCl₃) |
| **50** | 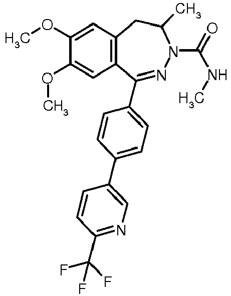 | (±)-7,8-Dimethoxy-*N*,4-dimethyl-1-{4-[6-(trifluormethyl)pyridin-3-yl]phenyl}-4,5-dihydro-3*H*-2,3-benzodiazepin-3-carboxamid | ¹H-NMR (300MHz, CDCl₃): δ = 0,99 (d, 3H), 2,86 (dd, 1H), 2,91 (d, 3H), 3,15 (dd, 1H), 3,68 (s, 3H), 3,95 (s, 3H), 5,49 (m, 1H), 6,54 (m, 1H), 6,62 (s, 1H), 6,74 (s, 1H), 7,65 (s, br, 4H), 7,79 (d, 1H), 8,10 (dd, 1H), 9,0 (s, br, 1H). |
| | | | LCMS (Methode 2): Rₜ = 1,35 min; m/z = 499 (M+H)⁺ |
| **50.1** | 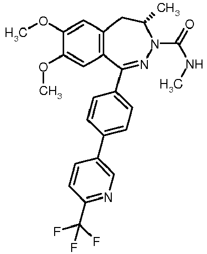 | (4S)-7,8-Dimethoxy-N,4-dimethyl-1-{4-[6-(trifluormethyl)pyridin-3-yl]phenyl}-4,5-dihydro-3H-2,3-benzodiazepin-3-carboxamid | Präp. HPLC: Methode IIIa |
| | | | Analyt. HPLC (Methode K1): Rₜ = 3.24 min |
| | | | [α]_{D}²⁰ = 109.2° (c = 1.00; MeOH) |
| | | | LCMS (Methode 1): Rₜ = 1.36 min; m/z = 499 (M+H)⁺ |
| **50**.**2** | 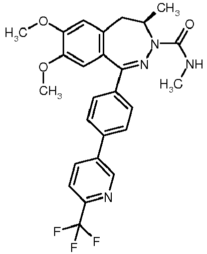 | (4R)-7,8-Dimethoxy-N,4-dimethyl-1-{4-[6-(trifluormethyl)pyridin-3-yl]phenyl}-4,5-dihydro-3H-2,3-benzodiazepin-3-carboxamid | Präp. HPLC: Methode IIIa |
| | | | Analyt. HPLC (Methode K1): Rₜ = 2.54 min |
| | | | [α]_{D}²⁰ = -113.2° (c = 1.00; MeOH) |
| | | | LCMS (Methode 1): Rₜ = 1.36 min; m/z = 499 (M+H)⁺ |
| **51** | 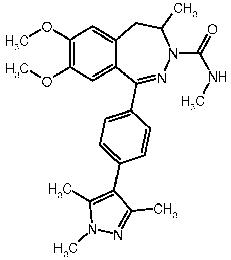 | (±)-7,8-Dimethoxy-*N*,4-dimethyl-1-[4-(1,3,5-trimethyl-1*H*-pyrazol-4-yl)phenyl]-4,5-dihydro-3*H*-2,3-benzodiazepin-3-carboxamid | ¹H-NMR (400MHz, CDCl₃): δ = 1,05 (d, 3H), 2,29 (s, 6H), 2,82 (dd, 1H), 2,90 (d, 3H), 3,08 (dd, 1H), 3,69 (s, 3H), 3,81 (s, 3H), 3,95 (s, 3H), 5,41 (m, 1H), 6,42 (qbr, 1H), 6,67 (s, 1H), 6,75 (s, 1H), 7,28 (d, 2H), 7,56 (d, 2H). |
| | | | LCMS (Methode 3): Rₜ = 1,11 min; m/z = 462 (M+H)⁺ |
| **51**.**1** | 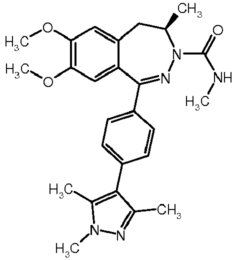 | (4*R*)-7,8-Dimethoxy-*N*,4-dimethyl-1-[4-(1,3,5-trimethyl-1*H*-pyrazol-4-yl)phenyl]-4,5-dihydro-3*H*-2,3-benzodiazepin-3-carboxamid | Präp. HPLC: Methode XVI |
| | | | Analyt. HPLC (Methode U): Rₜ = 7,78 min |
| | | | [α]_{D}²⁰ = -199,0° (c = 1,00; MeOH) |
| **51**.**2** | 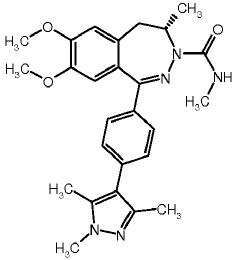 | (4*S*)-7,8-Dimethoxy-*N*,4-dimethyl-1-[4-(1,3,5-trimethyl-1*H*-pyrazol-4-yl)phenyl]-4,5-dihydro-3*H*-2,3-benzodiazepin-3-carboxamid | Präp. HPLC: Methode XVI |
| | | | Analyt. HPLC (Methode U): Rₜ = 10,16 min |
| | | | [α]_{D}²⁰ = +183,3° (c = 1,00; MeOH) |
| **52** | 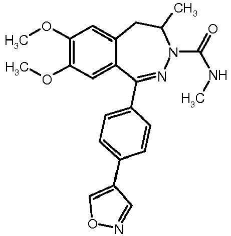 | (±)-1-[4-(Isoxazol-4-yl)phenyl]-7,8-dimethoxy-*N*,4-dimethyl-4,5-dihydro-3*H*-2,3-benzodiazepin-3-carboxamid | ¹H-NMR (400MHz, CDCl₃): δ = 0,99 (d, 3H), 2,85 (dd, 1H), 2,90 (d, 3H), 3,12 (dd, 1H), 3,66 (s, 3H), 3,94 (s, 3H), 5,46 (m, 1H), 6,50 (m, 1H), 6,60 (s, 1H), 6,73 (s, 1H), 7,51 (d, 2H), 7,56 (d, 2H), 8,61 (s, 1H), 8,74 (s, 1H). |
| | | | LCMS (Methode 3): Rₜ = 1,12 min; m/z = 421 (M+H)⁺ |

Analog zu Beispiel **2** wurden aus Beispiel **51A** und den entsprechenden kommerziell erhältlichen Boronsäurederivaten folgende Beispielverbindungen hergestellt:

| Nr | Struktur | Name | Analytische Daten |
|---|---|---|---|
| **53** | 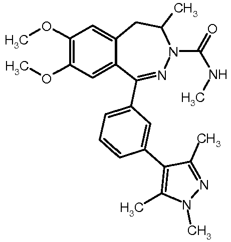 | (±)-7,8-Dimethoxy-*N*,4-dimethyl-1-[3-(1,3,5-trimethyl-1*H*-pyrazol-4-yl)phenyl]-4,5-dihydro-3*H*-2,3-benzodiazepin-3-carboxamid | ¹H-NMR (300MHz, CDCl₃): δ = 1,01 (d, 3H), 2,24 (s, 3H), 2,25 (s, 3H), 2,83 (dd, 1H), 2,88 (d, 3H), 3,11 (dd, 1H), 3,67 (s, 3H), 3,78 (s, 3H), 3,93 (s, 3H), 5,44 (m, 1H), 6,51 (qbr, 1H), 6,65 (s, 1H), 6,72 (s, 1H), 7,26 (m, 1H), 7,31 (m, 1H), 7,44 (m, 2H). |
| | | | LCMS (Methode 3): Rₜ = 1,12 min; m/z = 462 (M+H)⁺ |
| **54** | 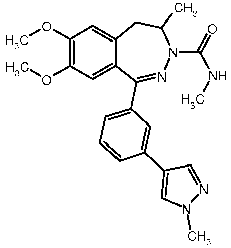 | (±)-7,8-Dimethoxy-*N*,4-dimethyl-1-[3-(1-methyl-1*H*-pyrazol-4-yl)phenyl]-4,5-dihydro-3*H*-2,3-benzodiazepin-3-carboxamid | ¹H-NMR (300MHz, CDCl₃): δ = 1,00 (d, 3H), 2,86 (dd, 1H), 2,88 (d, 3H), 3,13 (dd, 1H), 3,63 (s, 3H), 3,94 (s, 3H), 3,95 (s, 3H), 5,48 (m, 1H), 6,51 (m, 1H), 6,61 (s, 1H), 6,73 (s, 1H), 7,36 (m, 1H), 7,39 (dd, 1H), 7,50 (m, 1H), 7,55 (m, 1H), 7,61 (s, 1H), 7,76 (s, 1H). |
| | | | LCMS (Methode 3): Rₜ = 1,04 min; m/z = 434 (M+H)⁺ |
| **55** | 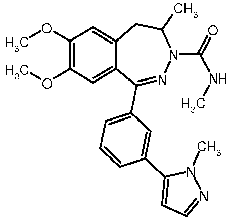 | (±)-7,8-Dimethoxy-*N*,4-dimethyl-1-[3-(1-methyl-1*H*-pyrazol-5-yl)phenyl]-4,5-dihydro-3*H*-2,3-benzodiazepin-3-carboxamid | ¹H-NMR (300MHz, CDCl₃): δ = 0,98 (d, 3H), 2,87 (dd, 1H), 2,89 (d, 3H), 3,14 (dd, 1H), 3,65 (s, 3H), 3,93 (s, 3H), 3,94 (s, 3H), 5,48 (m, 1H), 6,33 (d, 1H), 6,50 (qbr, 1H), 6,59 (s, 1H), 6,73 (s, 1H), 7,42-7,58 (m, 4H), 7,54 (d, 1H). |
| | | | LCMS (Methode 3): Rₜ = 1,07 min; m/z = 434 (M+H)⁺ |

Analog zu Beispiel **2** wurden aus Beispiel **60A** und den entsprechenden kommerziell erhältlichen Boronsäurederivaten folgende Beispielverbindungen hergestellt:

| Nr | Struktur | Name | Analytische Daten |
|---|---|---|---|
| **56** | 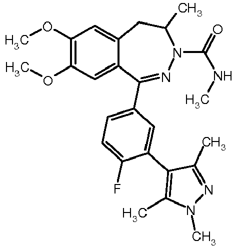 | (±)-1-[4-Fluoro-3-(1,3,5-trimethyl-1*H*-pyrazol-4-yl)phenyl]-7,8-dimethoxy-*N*,4-dimethyl-4,5-dihydro-3*H*-2,3-benzodiazepin-3-carboxamid | ¹H-NMR (300MHz, CDCl₃): δ = 0,99 (d, 3H), 2,18 (s, 6H), 2,83 (dd, 1H), 2,88 (d, 3H), 3,09 (dd, 1H), 3,68 (s, 3H), 3,78 (s, 3H), 3,93 (s, 3H), 5,43 (m, 1H), 6,44 (qbr, 1H), 6,62 (s, 1H), 6,72 (s, 1H), 7,17 (dd, 1H), 7,28 (dd, 1H), 7,46 (ddd, 1H). |
| | | | LCMS (Methode 3): Rₜ = 1,15 min; m/z = 480 (M+H)⁺ |
| **57** | 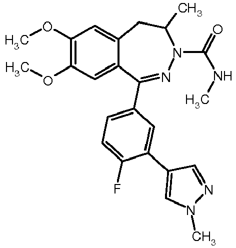 | (±)-1-[4-Fluoro-3-(1-methyl-1*H*-pyrazol-4-yl)phenyl]-7,8-dimethoxy-*N*,4-dimethyl-4,5-dihydro-3*H*-2,3-benzodiazepin-3-carboxamid | ¹H-NMR (300MHz, CDCl₃): δ = 0,99 (d, 3H), 2,85 (m, 1H), 2,89 (d, 3H), 3,12 (dd, 1H), 3,64 (s, 3H), 3,95 (s, 3H), 3,97 (s, 3H), 5,47 (m, 1H), 6,46 (m, 1H), 6,59 (s, 1H), 6,73 (s, 1H), 7,14 (dd, 1H), 7,31 (m, 1H), 7,64 (dd, 1H), 7,79 (s, 1H), 7,83 (s, 1H). |
| | | | LCMS (Methode 3): Rₜ = 1,14 min; m/z = 452 (M+H)⁺ |
| **58** | 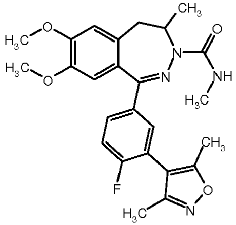 | (±)-1-[3-(3,5-Dimethylisoxazol-4-yl)-4-fluorophenyl]-7,8-dimethoxy-*N*,4-dimethyl-4,5-dihydro-3*H*-2,3-benzodiazepin-3-carboxamid | ¹H-NMR (300MHz, CDCl₃): δ = 0,98 (d, 3H), 2,23 (s, 3H), 2,36 (s, 3H), 2,86 (dd, 1H), 2,89 (d, 3H), 3,11 (dd, 1H), 3,68 (s, 3H), 3,94 (s, 3H), 5,46 (m, 1H), 6,45 (qbr, 1H), 6,58 (s, 1H), 6,72 (s, 1H), 7,21 (dd, 1H), 7,30 (dd, 1H), 7,53 (ddd, 1H). |
| | | | LCMS (Methode 3): Rₜ = 1,25 min; m/z = 467 (M+H)⁺ |

Analog zu Beispiel **2** wurden aus Beispiel **49A** und den entsprechenden kommerziell erhältlichen Boronsäurederivaten folgende Beispielverbindungen hergestellt:

| Nr | Struktur | Name | LCMS-Daten (Methode 4) | |
|---|---|---|---|---|
| | | | Rₜ [min] | [M+H]⁺ |
| **59** | 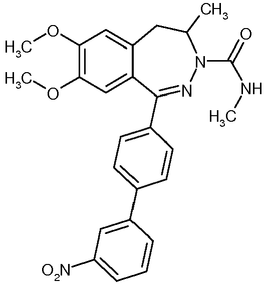 | (±)-7,8-Dimethoxy-*N*,4-dimethyl-1-(3'-nitrobiphenyl-4-yl)-4,5-dihydro-3*H*-2,3-benzodiazepin-3-carboxamid | 1,34 | 475 |
| **60** | 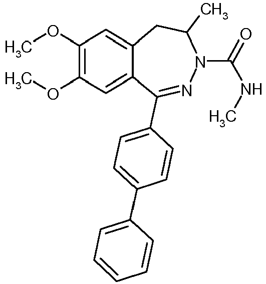 | (±)-1-(Biphenyl-4-yl)-7,8-dimethoxy-*N*,4-dimethyl-4,5-dihydro-3*H*-2,3-benzodiazepin-3-carboxamid | 1,39 | 430 |
| **61** | 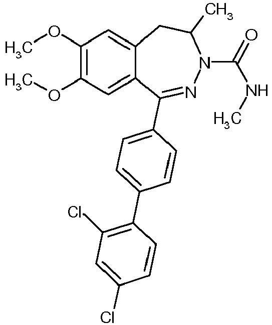 | (±)-1-(2',4'-Dichlorbiphenyl-4-yl)-7,8-dimethoxy-*N*,4-dimethyl-4,5-dihydro-3*H*-2,3-benzodiazepin-3-carboxamid | 1,57 | 498 |
| **62** | 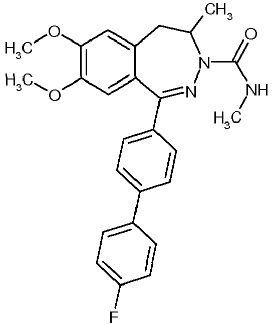 | (±)-1-(4'-Fluorbiphenyl-4-yl)-7,8-dimethoxy-*N*,4-dimethyl-4,5-dihydro-3*H*-2,3-benzodiazepin-3-carboxamid | 1,39 | 448 |
| **63** | 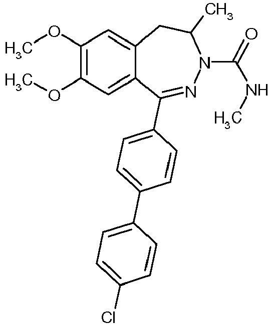 | (±)-1-(4'-Chlorbiphenyl-4-yl)-7,8-dimethoxy-*N*,4-dimethyl-4,5-dihydro-3*H*-2,3-benzodiazepin-3-carboxamid | 1,49 | 464 |
| **64** | 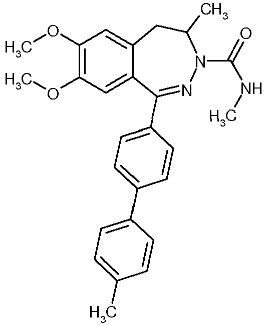 | (±)-7,8-Dimethoxy-*N*,4-dimethyl-1-(4'-methylbiphenyl-4-yl)-4,5-dihydro-3*H*-2,3-benzodiazepin-3-carboxamid | 1,47 | 444 |
| **65** | 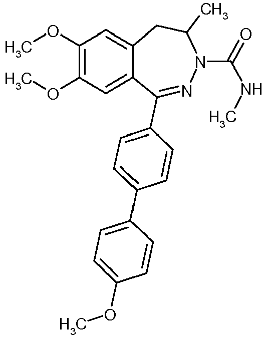 | (±)-7,8-Dimethoxy-1-(4'-methoxybiphenyl-4-yl)-*N*,4-dimethyl-4,5-dihydro-3*H*-2,3-benzodiazepin-3-carboxamid | 1,35 | 460 |
| **66** | 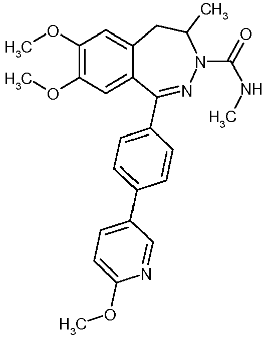 | (±)-7,8-Dimethoxy-1-[4-(6-methoxypyridin-3-yl)phenyl]-*N*,4-dimethyl-4,5-dihydro-3*H-*2,3-benzodiazepin-3-carboxamid | 1,27 | 461 |
| **67** | 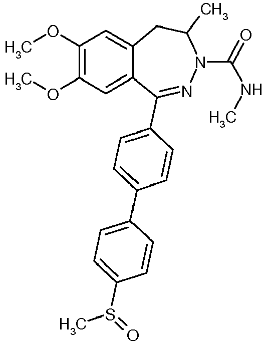 | (±)-7,8-Dimethoxy-*N*,4-dimethyl-1-[4'-(methylsulfinyl)biphenyl-4-yl]-4,5-dihydro-3*H*-2,3-benzodiazepin-3-carboxamid | 1,05 | 492 |
| **68** | 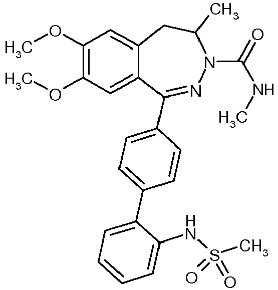 | (±)-7,8-Dimethoxy-*N*,4-dimethyl-1-{2'-[(methylsulfonyl)amino]biphe nyl-4-yl}-4,5-dihydro-3*H*-2,3-benzodiazepin-3-carboxamid | 1,16 | 523 |
| **69** | 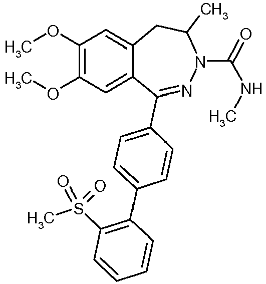 | (±)-7,8-Dimethoxy-*N*,4-dimethyl-1-[2'-(methylsulfonyl)biphenyl-4-yl]-4,5-dihydro-3*H*-2,3-benzodiazepin-3-carboxamid | 1,16 | 508 |
| **70** | 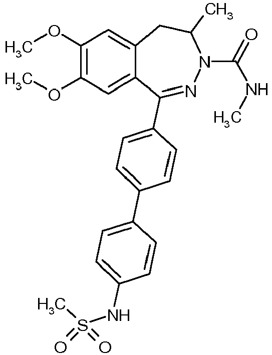 | (±)-7,8-Dimethoxy-*N*,4-dimethyl-1-{4'-[(methylsulfonyl)amino]biphe nyl-4-yl}-4,5-dihydro-3*H*-2,3-benzodiazepin-3-carboxamid | 1,10 | 523 |
| **71** | 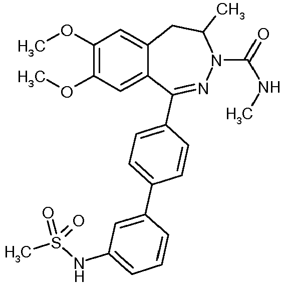 | (±)-7,8-Dimethoxy-*N*,4-dimethyl-1-{3'-[(methylsulfonyl)amino]biphe nyl-4-yl}-4,5-dihydro-3*H*-2,3-benzodiazepin-3-carboxamid | 1,13 | 523 |
| **72** | 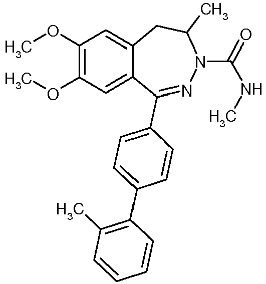 | (±)-7,8-Dimethoxy-*N*,4-dimethyl-1-(2'-methylbiphenyl-4-yl)-4,5-dihydro-3*H*-2,3-benzodiazepin-3-carboxamid | 1,45 | 444 |
| **73** | 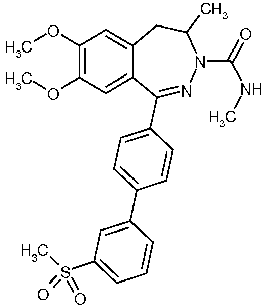 | (±)-7,8-Dimethoxy-*N*,4-dimethyl-1-[3'-(methylsulfonyl)biphenyl-4-yl]-4,5-dihydro-3*H*-2,3-benzodiazepin-3-carboxamid | 1,14 | 508 |
| **74** | 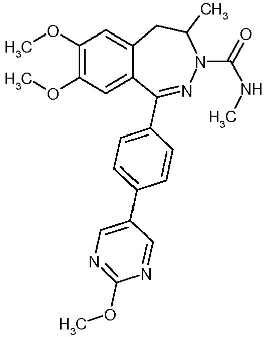 | (±)-7,8-Dimethoxy-1-[4-(2-methoxypyrimidin-5-yl)phenyl]-*N*,4-dimethyl-4,5-dihydro-3*H*-2,3-benzodiazepin-3-carboxamid | 1,12 | 462 |
| **75** | 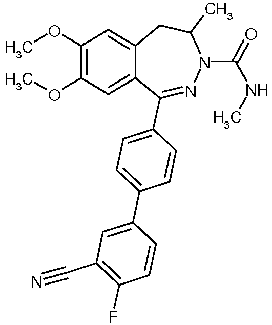 | (±)-1-(3'-Cyan-4'-fluorbiphenyl-4-yl)-7,8-dimethoxy-*N*,4-dimethyl-4,5-dihydro-3*H*-2,3-benzodiazepin-3-carboxamid | 1,31 | 473 |
| **76** | 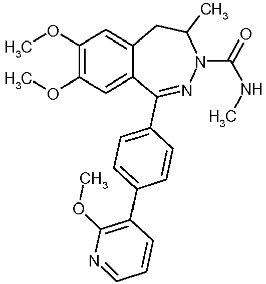 | (±)-7,8-Dimethoxy-1-[4-(2-methoxypyridin-3-yl)phenyl]-*N*,4-dimethyl-4,5-dihydro-3*H-*2,3-benzodiazepin-3-carboxamid | 1,27 | 461 |
| **77** | 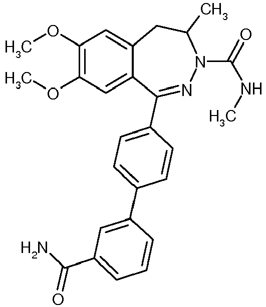 | (±)-1-(3'-Carbamoylbiphenyl-4-yl)-7,8-dimethoxy-*N*,4-dimethyl-4,5-dihydro-3*H*-2,3-benzodiazepin-3-carboxamid | 1,03 | 473 |
| **78** | 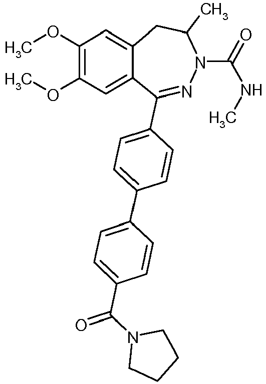 | (±)-7,8-Dimethoxy-*N*,4-dimethyl-1-[4'-(pyrrolidin-1-ylcarbonyl)biphenyl-4-yl]-4,5-dihydro-3*H*-2,3-benzodiazepine-3-carboxamide | 1,19 | 527 |
| **79** | 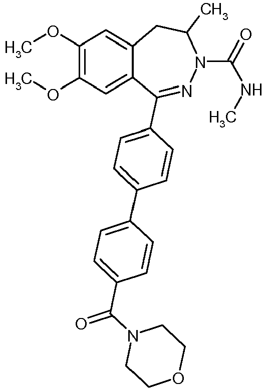 | (±)-7,8-Dimethoxy-*N*,4-dimethyl-1-[4'-(morpholin-4-ylcarbonyl)biphenyl-4-yl]-4,5-dihydro-3*H*-2,3-benzodiazepin-3-carboxamid | 1,10 | 543 |
| **80** | 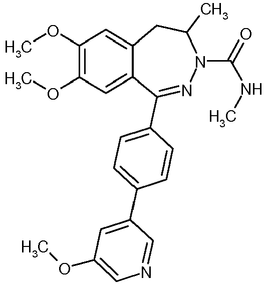 | (±)-7,8-Dimethoxy-1-[4-(5-methoxypyridin-3-yl)phenyl]-*N*,4-dimethyl-4,5-dihydro-3*H-*2,3-benzodiazepin-3-carboxamid | 1,06 | 461 |
| **81** | 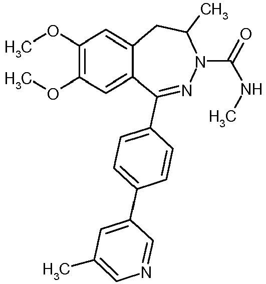 | (±)-7,8-Dimethoxy-*N*,4-dimethyl-1-[4-(5-methylpyridin-3-yl)phenyl]-4,5-dihydro-3*H*-2,3-benzodiazepin-3-carboxamid | 0,94 | 445 |
| **82** | 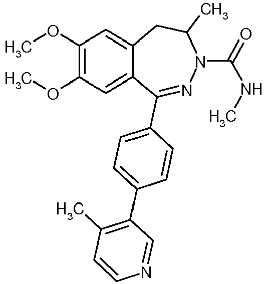 | (±)-7,8-Dimethoxy-*N*,4-dimethyl-1-[4-(4-methylpyridin-3-yl)phenyl]-4,5-dihydro-3*H*-2,3-benzodiazepin-3-carboxamid | 0,85 | 445 |
| **83** | 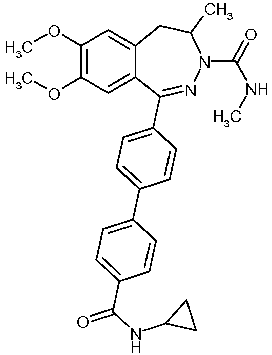 | (±)-1-[4'-(Cyclopropylcarbamoyl)biphe nyl-4-yl]-7,8-dimethoxy-*N*,4-dimethyl-4,5-dihydro-3*H*-2,3-benzodiazepin-3-carboxamid | 1,13 | 513 |
| **84** | 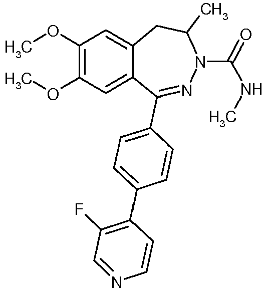 | (±)-1-[4-(3-Fluorpyridin-4-yl)phenyl]-7,8-dimethoxy-*N*,4-dimethyl-4,5-dihydro-3*H*-2,3-benzodiazepin-3-carboxamid | 1,15 | 449 |
| **85** | 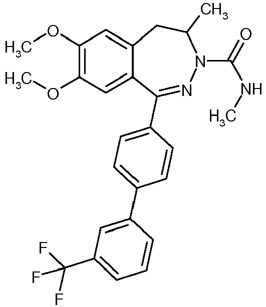 | (±)-7,8-Dimethoxy-*N*,4-dimethyl-1-[3'-(trifluormethyl)biphenyl-4-yl]-4,5-dihydro-3*H*-2,3-benzodiazepin-3-carboxamid | 1,48 | 498 |
| **86** | 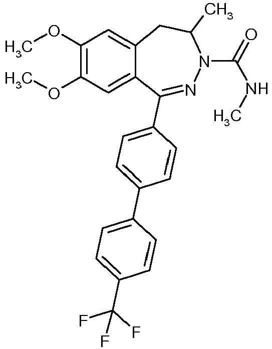 | (±)-7,8-Dimethoxy-*N*,4-dimethyl-1-[4'-(trifluormethyl)biphenyl-4-yl]-4,5-dihydro-3*H*-2,3-benzodiazepin-3-carboxamid | 1,49 | 498 |
| **87** | 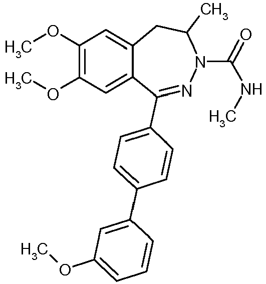 | (±)-7,8-Dimethoxy-1-(3'-methoxybiphenyl-4-yl)-*N*,4-dimethyl-4,5-dihydro-3*H*-2,3-benzodiazepin-3-carboxamid | 1,36 | 460 |
| **88** | 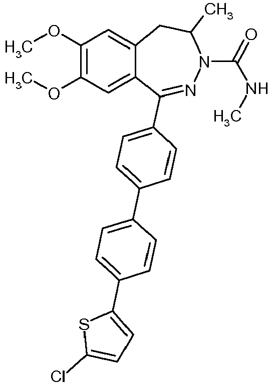 | (±)-1-[4'-(5-Chlorthien-2-yl)biphenyl-4-yl]-7,8-dimethoxy-*N*,4-dimethyl-4,5-dihydro-3*H*-2,3-benzodiazepin-3-carboxamid | 1,52 | 470 |
| **89** | 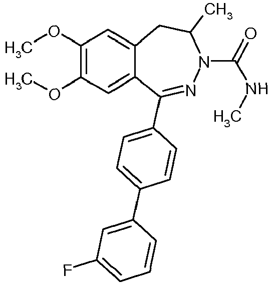 | (±)-1-(3'-Fluorbiphenyl-4-yl)-7,8-dimethoxy-*N*,4-dimethyl-4,5-dihydro-3*H*-2,3-benzodiazepin-3-carboxamid | 1,40 | 448 |
| **90** | 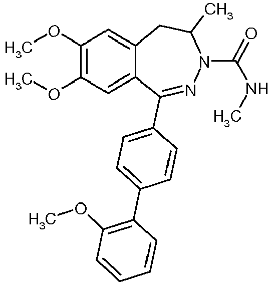 | (±)-7,8-Dimethoxy-1-(2'-methoxybiphenyl-4-yl)-*N*,4-40dimethyl-4,5-dihydro-3*H-*2,3-benzodiazepin-3-carboxamid | 1,37 | 460 |
| **91** | 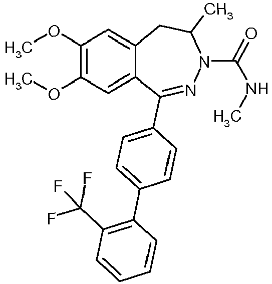 | (±)-7,8-Dimethoxy-*N*,4-dimethyl-1-[2'-(trifluormethyl)biphenyl-4-yl]-4,5-dihydro-3*H*-2,3-benzodiazepin-3-carboxamid | 1,45 | 498 |
| **92** | 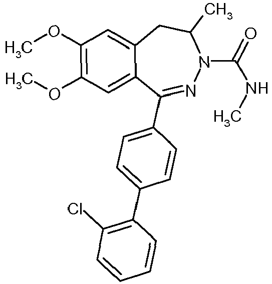 | (±)-1-(2'-Chlorbiphenyl-4-yl)-7,8-dimethoxy-*N*,4-dimethyl-4,5-dihydro-3*H*-2,3-benzodiazepin-3-carboxamid | 1,44 | 464 |
| **93** | 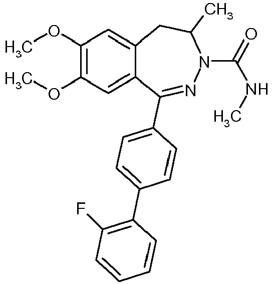 | (±)-1-(2'-Fluorbiphenyl-4-yl)-7,8-dimethoxy-*N*,4-dimethyl-4,5-dihydro-3*H*-2,3-benzodiazepin-3-carboxamid | 1,38 | 448 |
| **94** | 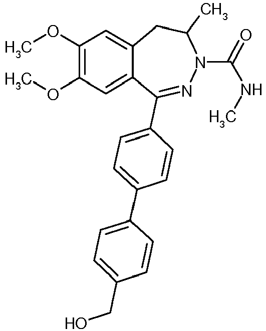 | (±)-1-[4'-(Hydroxymethyl)biphenyl-4-yl]-7,8-dimethoxy-*N*,4-dimethyl-4,5-dihydro-3*H*-2,3-benzodiazepin-3-carboxamid | 1,10 | 460 |
| **95** | 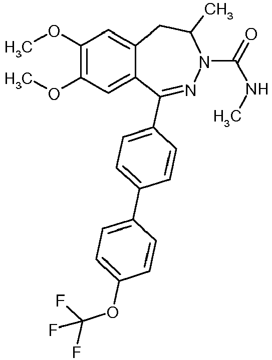 | (±)-7,8-Dimethoxy-*N*,4-dimethyl-1-[4'-(trifluormethoxy)biphenyl-4-yl]-4,5-dihydro-3*H*-2,3-benzodiazepin-3-carboxamid | 1,51 | 514 |
| **96** | 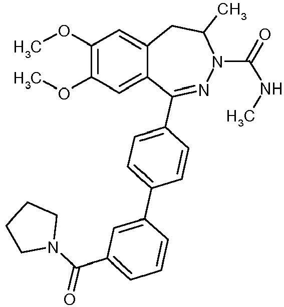 | (±)-7,8-Dimethoxy-*N*,4-dimethyl-1-[3'-(pyrrolidin-1-ylcarbonyl)biphenyl-4-yl]-4,5-dihydro-3*H*-2,3-benzodiazepin-3-carboxamid | 1,20 | 527 |
| **97** | 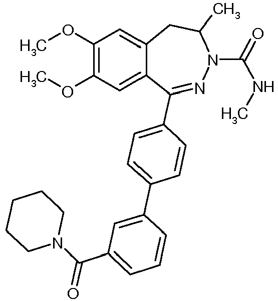 | (±)-7,8-Dimethoxy-*N*,4-dimethyl-1-[3'-(piperidin-1-ylcarbonyl)biphenyl-4-yl]-4,5-dihydro-3*H*-2,3-benzodiazepin-3-carboxamid | 1,29 | 541 |
| **98** | 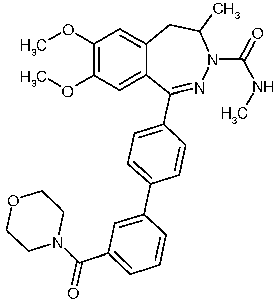 | (±)-7,8-Dimethoxy-*N*,4-dimethyl-1-[3'-(morpholin-4-ylcarbonyl)biphenyl-4-yl]-4,5-dihydro-3*H*-2,3-benzodiazepin-3-carboxamid | 1,12 | 543 |
| **99** | 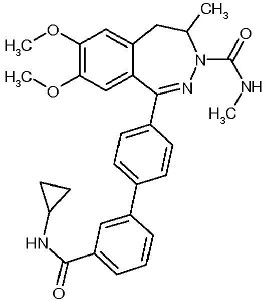 | (±)-1-[3'-(Cyclopropylcarbamoyl)biphe nyl-4-yl]-7,8-dimethoxy-*N*,4-dimethyl-4,5-dihydro-3*H*-2,3-benzodiazepin-3-carboxamid | 1,15 | 513 |
| **100** | 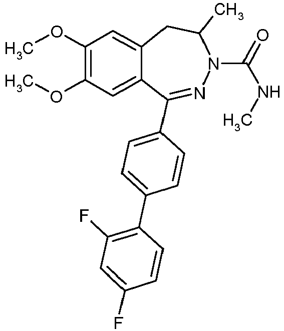 | (±)-1-(2',4'-Difluorbiphenyl-4-yl)-7,8-dimethoxy-*N*,4-dimethyl-4,5-dihydro-3*H*-2,3-benzodiazepin-3-carboxamid | 1,41 | 466 |
| **101** | 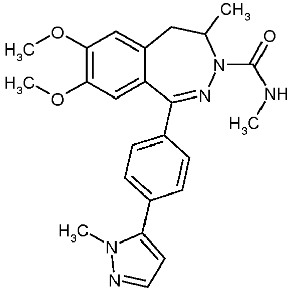 | (±)-7,8-Dimethoxy-*N*,4-dimethyl-1-[4-(1-methyl-1*H-*pyrazol-5-yl)phenyl]-4,5-dihydro-3*H*-2,3-benzodiazepin-3-carboxamid | 1,09 | 434 |
| **102** | 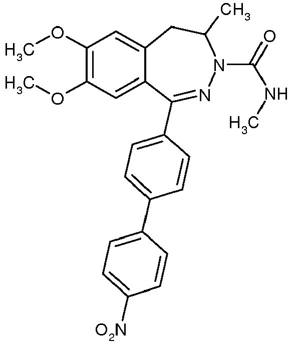 | (±)-7,8-Dimethoxy-*N*,4-dimethyl-1-(4'-nitrobiphenyl-4-yl)-4,5-dihydro-3*H*-2,3-benzodiazepin-3-carboxamid | 1,34 | 475 |
| **103** | 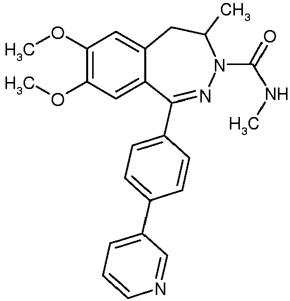 | (±)-7,8-Dimethoxy-*N*,4-dimethyl-1-[4-(pyridin-3-yl)phenyl]-4,5-dihydro-3*H-*2,3-benzodiazepin-3-carboxamid | 0,93 | 431 |
| **104** | 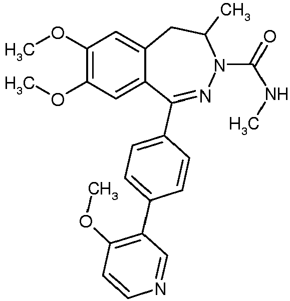 | (±)-7,8-Dimethoxy-1-[4-(4-methoxypyridin-3-yl)phenyl]-*N*,4-dimethyl-4,5-dihydro-3*H-*2,3-benzodiazepin-3-carboxamid | 0,77 | 461 |
| **105** | 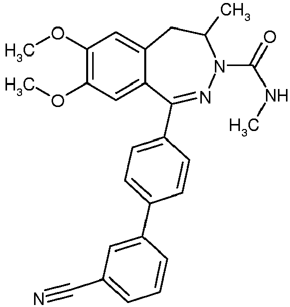 | (±)-1-(3'-Cyanbiphenyl-4-yl)-7,8-dimethoxy-*N*,4-dimethyl-4,5-dihydro-3*H*-2,3-benzodiazepin-3-carboxamid | 1,28 | 455 |
| **106** | 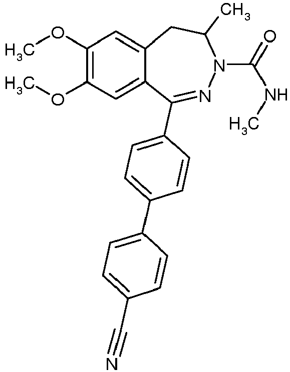 | (±)-1-(4'-Cyanbiphenyl-4-yl)-7,8-dimethoxy-*N*,4-dimethyl-4,5-dihydro-3*H*-2,3-benzodiazepin-3-carboxamid | 1,27 | 455 |
| **107** | 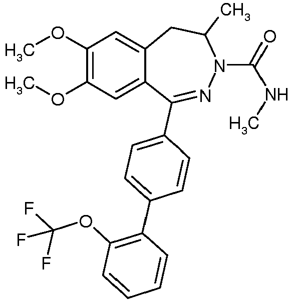 | (±)-7,8-Dimethoxy-*N*,4-dimethyl-1-[2'-(trifluormethoxy)biphenyl-4-yl]-4,5-dihydro-3*H*-2,3-benzodiazepin-3-carboxamid | 1,48 | 514 |
| **108** | 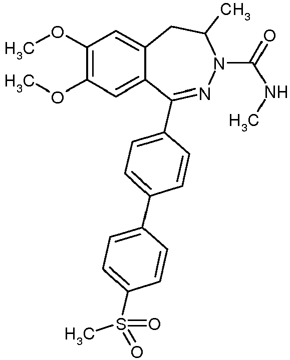 | (±)-7,8-Dimethoxy-*N*,4-dimethyl-1-[4'-(methylsulfonyl)biphenyl-4-yl]-4,5-dihydro-3*H*-2,3-benzodiazepin-3-carboxamid | 1,12 | 508 |
| **109** | 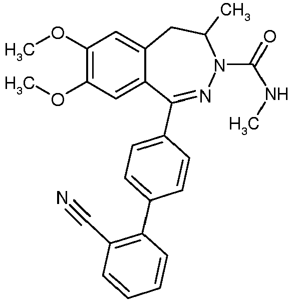 | (±)-1-(2'-Cyanbiphenyl-4-yl)-7,8-dimethoxy-*N*,4-dimethyl-4,5-dihydro-3*H*-2,3-benzodiazepin-3-carboxamid | 1,27 | 455 |
| **110** | 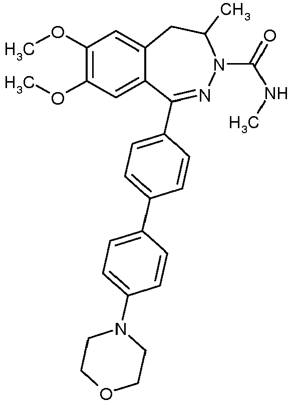 | (±)-7,8-Dimethoxy-*N*,4-dimethyl-1-[4'-(morpholin-4-yl)biphenyl-4-yl]-4,5-dihydro-3*H*-2,3-benzodiazepin-3-carboxamid | 1,29 | 515 |
| **111** | 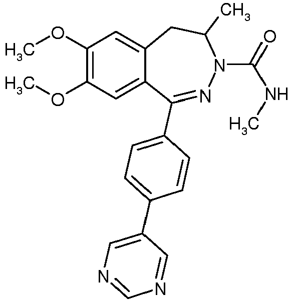 | (±)-7,8-Dimethoxy-*N*,4-dimethyl-1-[4-(pyrimidin-5-yl)phenyl]-4,5-dihydro-3*H-*2,3-benzodiazepin-3-carboxamid | 1,01 | 432 |
| **112** | 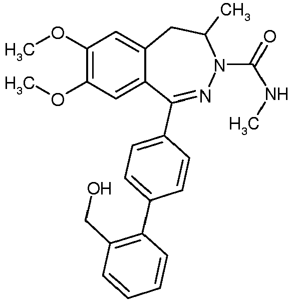 | (±)-1-[2'-(Hydroxymethyl)biphenyl-4-yl]-7,8-dimethoxy-*N*,4-dimethyl-4,5-dihydro-3*H*-2,3-benzodiazepie-3-carboxamid | 1,17 | 460 |
| **113** | 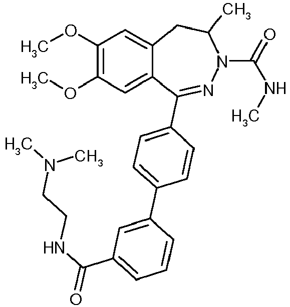 | (+)-1-(3'-{[2-(Dimethylamino)ethyl]carbamoyl}biphenyl-4-yl)-7,8-dimethoxy-*N*,4-dimethyl-4,5-dihydro-3*H*-2,3-benzodiazepin-3-carboxamid | 0,84 | 544 |
| **114** | 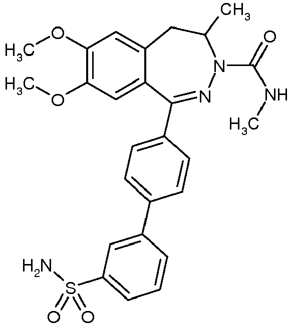 | (±)-7,8-Dimethoxy-*N*,4-dimethyl-1-(3'-sulfamoylbiphenyl-4-yl)-4,5-dihydro-3*H*-2,3-benzodiazepin-3-carboxamid | 1,06 | 509 |
| **115** | 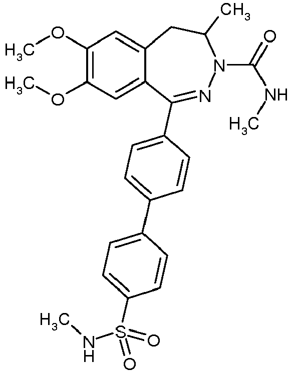 | (±)-7,8-Dimethoxy-*N*,4-dimethyl-1-[4'-(methylsulfamoyl)biphenyl-4-yl]-4,5-dihydro-3*H*-2,3-benzodiazepin-3-carboxamid | 1,13 | 523 |
| **116** | 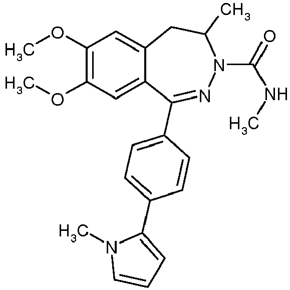 | (±)-7,8-Dimethoxy-*N*,4-dimethyl-1-[4-(1-methyl-1*H-*pyrrol-2-yl)phenyl]-4,5-dihydro-3*H*-2,3-benzodiazepin-3-carboxamid | 1,28 | 433 |
| **117** | 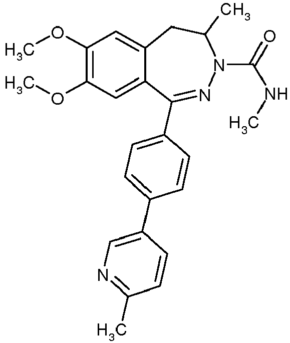 | (±)-7,8-Dimethoxy-*N*,4-dimethyl-1-[4-(6-methylpyridin-3-yl)phenyl]-4,5-dihydro-3*H*-2,3-benzodiazepin-3-carboxamid | 0,87 | 445 |
| **118** | 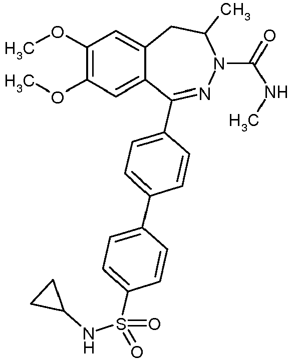 | (±)-1-[4'-(Cyclopropylsulfamoyl)biphen yl-4-yl]-7,8-dimethoxy-*N*,4-dimethyl-4,5-dihydro-3*H*-2,3-benzodiazepin-3-carboxamid | 1,20 | 549 |
| **119** | 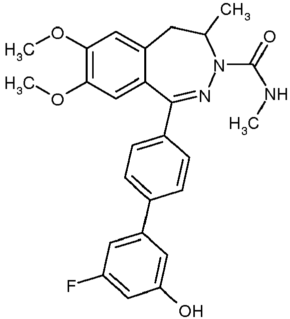 | (±)-1-(3'-Fluor-5'-hydroxybiphenyl-4-yl)-7,8-dimethoxy-*N*,4-dimethyl-4,5-dihydro-3*H*-2,3-benzodiazepin-3-carboxamid | 1,20 | 464 |
| **120** | 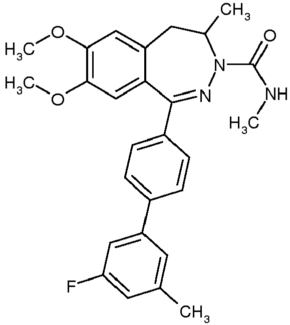 | (±)-1-(3'-Fluor-5'-methylbiphenyl-4-yl)-7,8-dimethoxy-*N*,4-dimethyl-4,5-dihydro-3*H*-2,3-benzodiazepin-3-carboxamid | 1,48 | 462 |
| **121** | 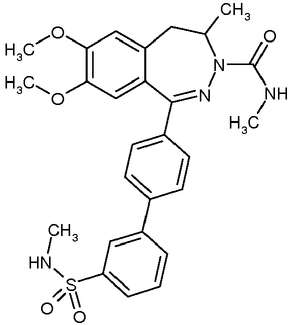 | (±)-7,8-Dimethoxy-*N*,4-dimethyl-1-[3'-(methylsulfamoyl)biphenyl-4-yl]-4,5-dihydro-3*H*-2,3-benzodiazepin-3-carboxamid | 1,15 | 523 |
| **122** | 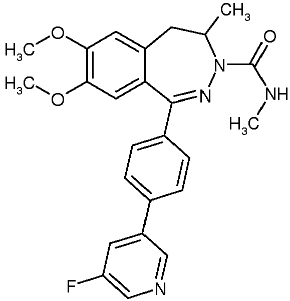 | (±)-1-[4-(5-Fluorpyridin-3-yl)phenyl]-7,8-dimethoxy-*N*,4-dimethyl-4,5-dihydro-3*H*-2,3-benzodiazepin-3-carboxamid | 1,18 | 449 |
| **123** | 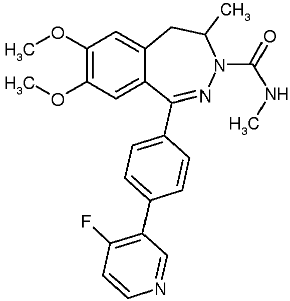 | (±)-1-[4-(4-Fluorpyridin-3-yl)phenyl]-7,8-dimethoxy-*N*,4-dimethyl-4,5-dihydro-3*H*-2,3-benzodiazepin-3-carboxamid | 1,11 | 449 |
| **124** | 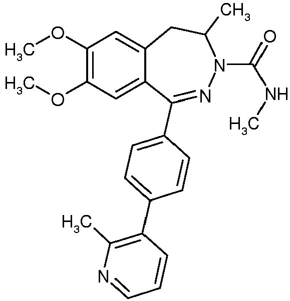 | (±)-7,8-Dimethoxy-*N*,4-dimethyl-1-[4-(2-methylpyridin-3-yl)phenyl]-4,5-dihydro-3*H*-2,3-benzodiazepin-3-carboxamid | 0,82 | 445 |
| **125** | 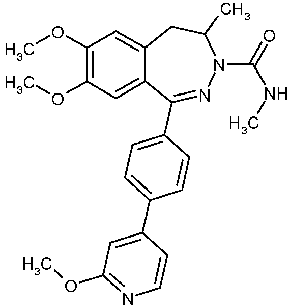 | (±)-7,8-Dimethoxy-1-[4-(2-methoxypyridin-4-yl)phenyl]-*N*,4-dimethyl-4,5-dihydro-3*H-*2,3-benzodiazepin-3-carboxamid | 1,26 | 461 |
| **126** | 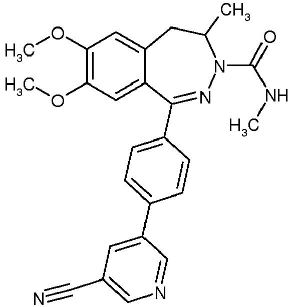 | (±)-1-[4-(5-Cyanpyridin-3-yl)phenyl]-7,8-dimethoxy-*N*,4-dimethyl-4,5-dihydro-3*H*-2,3-benzodiazepin-3-carboxamid | 1,14 | 456 |

### Alternatives Verfahren zur Herstellung von Beispiel 7

### (±)-7,8-Dimethoxy-N,4-dimethyl-1-[4-(morpholin-4-yl)phenyl]-4,5-dihydro-3H-2,3-benzodiazepin-3-carboxamid

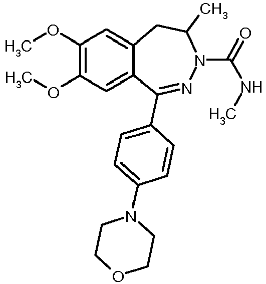

100 mg (231 µmol) (±)-1-(4-Bromphenyl)-7,8-dimethoxy-*N*,4-dimethyl-4,5-dihydro-3*H*-2,3-benzodiazepin-3-carboxamid (Beispiel **49A**) wurden unter Argon in 5 ml entgastem Toluol vorgelegt. Es wurden 22 µl (22 mg, 254 µmol) Morpholin, 31 mg (324 µmol) Natrium-*tert-*butanolat und 9 mg (12 µmol) Chlor-(2-dicyclohexylphosphino-2,4,6-triisopropyl-1,1-biphenyl)[2-(2-amino-1,1-biphenyl)]palladium(II) (CAS [1310584-14-5]) zugegeben. Die Mischung wurde erneut entgast, mit Argon gesättigt und dann 6 Stunden bei 110°C gerührt. Nach dem Abkühlen wurde die Mischung zwischen 15 ml ges- Natriumhydrogencarbonatlösung und 15 ml Ethylacetat verteilt und die Phasen getrennt. Die wässrige Phase wurde mit Ethylacetat extrahiert, die vereinigten organischen Phasen mit Wasser und ges. Natriumchloridlösung gewaschen und mit Natriumsulfat getrocknet. Die Lösemittel wurden am Rotationsverdampfer entfernt und der Rückstand (156 mg gelbes Öl) flashchromatographisch gereinigt (SiO₂, Hexan/Ethylacetat). Man erhielt 87 mg (86% d. Th.) des gewünschten Produkts als gelben Feststoff.
LCMS (Methode 2): Rₜ = 1,08 min; m/z = 439 (M+H)⁺
¹H-NMR (300MHz, CDCl₃): δ = 1, 10 (d, 3H), 2,73 (dd, 1H), 2,86 (d, 3H), 2,95 (dd, 1H), 3,25-3,28 (m, 4H), 3,70 (s, 3H), 3,88 - 3,91 (m, 4H), 3,93 (s, 3H), 5,25 - 5,35 (m, 1H), 6,14 (q, 1H), 6,63 (s, 1H), 6,75 (s, 1H), 6,93 (d, 2H), 7,51 (d, 2H).

### Enantiomerentrennung

78 mg (±)-7,8-Dimethoxy-*N*,4-dimethyl-1-[4-(morpholin-4-yl)phenyl]-4,5-dihydro-3*H*-2,3-benzodiazepin-3-carboxamid (Beispiel **7**) wurden mittels chiraler präparativer HPLC mit der Methode V in die Enantiomere getrennt:

### Beispiel 7.1: (4R)-7,8-Dimethoxy-N,4-dimethyl-1-[4-(morpholin-4-yl)phenyl]-4,5-dihydro-3H-2,3-benzodiazepin-3-carboxamid

20 mg gelblicher Feststoff, HPLC (Methode G): Rₜ = 6,08 min, Reinheit 100%

### Beispiel 7.2: (4S)-7,8-Dimethoxy-N,4-dimethyl-1-[4-(morpholin-4-yl)phenyl]-4,5-dihydro-3H-2,3-benzodiazepin-3-carboxamid

20 mg gelblicher Feststoff, HPLC (Methode G): Rₜ = 7,42 min, Reinheit 99,3%

### Beispiel 127.1

### (4S)-7,8-Dimethoxy-N,4-dimethyl-1-[4-(4-methylpiperazin-1-yl)phenyl]-4,5-dihydro-3H-2,3-benzodiazepin-3-carboxamid

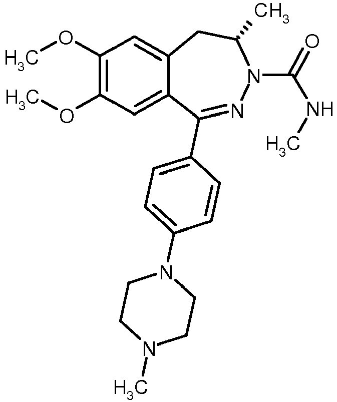

1,30 g (3,01 mmol) (4*S*)-1-(4-Bromphenyl)-7,8-dimethoxy-*N*,4-dimethyl-4,5-dihydro-3*H*-2,3-benzodiazepin-3-carboxamid (Beispiel **49.2A**) wurden unter Argon in 65 ml entgastem Toluol vorgelegt. Es wurden 0,37 ml (331 mg, 3,31 mmol) 1-Methylpiperazin, 405 mg (4,21 mmol) Natrium-*tert*-butanolat und 118 mg (0,15 mmol) Chlor-(2-dicyclohexylphosphino-2,4,6-triisopropyl-1,1-biphenyl)[2-(2-amino-1,1-biphenyl)]palladium(II) (CAS [1310584-14-5]) zugegeben. Die Mischung wurde erneut entgast, mit Argon gesättigt und dann 12 Stunden bei 80°C gerührt. Nach dem Abkühlen wurde die Mischung auf ges. Natriumhydrogencarbonatlösung gegeben und mit Ethylacetat extrahiert. Die vereinigten organischen Phasen wurden mit ges. Natriumchloridlösung gewaschen und mit Natriumsulfat getrocknet. Die Lösemittel wurden am Rotationsverdampfer entfernt und der Rückstand (1,5 g oranger Schaum) flashchromatographisch gereinigt (SiO₂, Dichlormethan/Methanol 0-3-10%). Man erhielt 850 mg (63% d. Th.) des gewünschten Produkts als gelben Feststoff.
LCMS (Methode 2): Rₜ = 0,69 min; m/z = 452 (M+H)⁺
¹H-NMR (400MHz, DMSO-*d₆*): δ = 1,02 (d, 3H), 2,19 (s, 3H), 2,38 - 2,44 (m, 4H), 2,46 - 2,51 (m, 1H), 2,60 (d, 3H), 2,80 (dd, 1H), 3,18 - 3,24 (m, 4H), 3,57 (s, 3H), 3,80 (s, 3H), 4,82 - 4,90 (m, 1H), 6,25 (q, 1H), 6,51 (s, 1H), 6,91 (d, 2H), 6,99 (s, 1H), 7,55 (d, 2H).
Spezifische optische Drehung: [α]_{D}²⁰ = 374,4° +/-0,17° (c = 1,00; Methanol)

Analog Beispiel **127.1** wurden aus Beispiel **49A** bzw. Beispiel **49.2A** und den entsprechenden kommerziell erhältlichen Aminen, gegebenenfalls mit nachfolgender Enantiomerentrennung per chiraler präparativer HPLC, folgende Beispielverbindungen hergestellt:

| Nr | Struktur | Name | Analytische Daten |
|---|---|---|---|
| **128** | 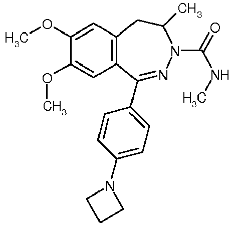 | (±)-1-[4-(Azetidin-1-yl)phenyl]-7,8-dimethoxy-*N*,4-dimethyl-4,5-dihydro-3*H*-2,3-benzodiazepin-3-carboxamid | ¹H-NMR (400MHz, CDCl₃): δ = 1,17 (d, 3H), 2,39 - 2,46 (m, 2H), 2,70 (dd, 1H), 2,85 (d, 3H), 2,89 (dd, 1H), 3,71 (s, 3H), 3,93 (s, 3H), 3,95 - 3,99 (m, 4H), 5,19 - 5,27 (m, 1H), 5,96 (q, 1H), 6,42 (d, 2H), 6,64 (s, 1H), 6,76 (s, 1H), 7,49 (d, 2H). |
| | | | LCMS (Methode 2): Rₜ = 1,18 min; m/z = 409 (M+H)⁺ |
| **128**.**1** | 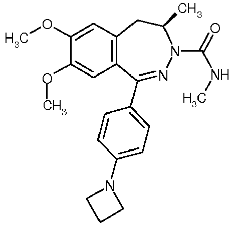 | (4*R*)-1-[4-(Azetidin-1-yl)phenyl]-7,8-dimethoxy-*N*,4-dimethyl-4,5-dihydro-3*H*-2,3-benzodiazepin-3-carboxamid | Präp. HPLC: Methode VII |
| | | | Analyt. HPLC (Methode J): Rₜ = 2,93 min |
| | | | [α]_{D}²⁰ = -415° (c = 1,00; Methanol) |
| **128**.**2** | 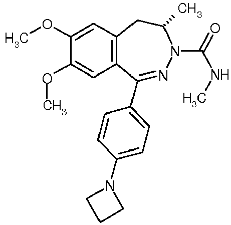 | (4*S*)-1-[4-(Azetidin-1-yl)phenyl]-7,8-dimethoxy-*N*,4-dimethyl-4,5-dihydro-3*H*-2,3-benzodiazepin-3-carboxamid | Präp. HPLC: Methode VII |
| | | | Analyt. HPLC (Methode J): Rₜ = 3,68 min |
| | | | [α]_{D}²⁰ = 442° (c = 1,00; Methanol) |
| **129** | 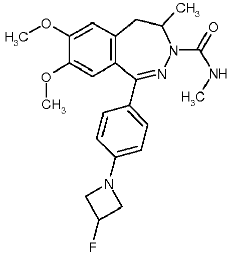 | (±)-1-[4-(3-Fluorazetidin-1-yl)phenyl]-7,8-dimethoxy-*N*,4-dimethyl-4,5-dihydro-3*H*-2,3-benzodiazepin-3-carboxamid | ¹H-NMR (300MHz, CDCl₃): δ = 1,12 (d, 3H), 2,72 (dd, 1H), 2,86 (d, 3H), 2,93 (dd, 1H), 3,70 (s, 3H), 3,93 (s, 3H), 3,98 - 4,10 (m, 2H), 4,20 - 4,31 (m, 2H), 5,21 - 5,31 (m, 1H), 5,32-5,39 und 5,52 - 5,58 (m, 1H), 6,05 (q, 1H), 6,47 (d, 2H), 6,62 (s, 1H), 6,75 (s, 1H), 7,48 (d, 2H). |
| | | | LCMS (Methode 2): Rₜ = 1,15 min; m/z = 427 (M+H)⁺ |
| **129**.**1** | 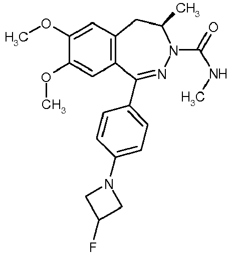 | (4*R*)-1-[4-(3-Fluorazetidin-1-yl)phenyl]-7,8-dimethoxy-*N*,4-dimethyl-4,5-dihydro-3*H*-2,3-benzodiazepin-3-carboxamid | Präp. HPLC: Methode VII |
| | | | Analyt. HPLC (Methode J): Rₜ = 2,93 min |
| | | | [α]_{D}²⁰ = -340° (c = 1,00; Methanol) |
| **129**.**2** | 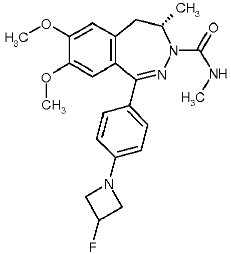 | (4*S*)-1-[4-(3-Fluorazetidin-1-yl)phenyl]-7,8-dimethoxy-*N*,4-dimethyl-4,5-dihydro-3*H*-2,3-benzodiazepin-3-carboxamid | Präp. HPLC: Methode VII |
| | | | Analyt. HPLC (Methode J): Rₜ = 3,68 min |
| | | | [α]_{D}²⁰ = 401° (c = 1,00; Methanol) |
| **130** | 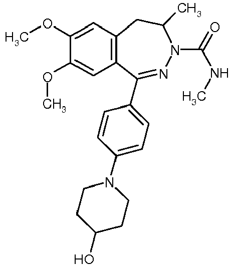 | (±)-1-[4-(4-Hydroxypiperidin-1-yl)phenyl]-7,8-dimethoxy-*N*,4-dimethyl-4,5-dihydro-3*H*-2,3-benzodiazepin-3-carboxamid | ¹H-NMR (300MHz, DMSO-*d*₆): δ = 1,02 (d, 3H), 1,35 - 1,47 (m, 2H), 1,72 - 1,83 (m, 2H), 2,36 - 2,50 (m, 1H), 2,59 (d, 3H), 2, 79 (dd, 1H), 2,88 - 3,00 (m, 2H), 3,57 (s, 3H), 3,57 - 3,67 (m, 3H), 3,80 (s, 3H), 4,65 (d, 1H), 4,78 - 4,90 (m, 1H), 6,22 (q, 1H), 6,52 (s, 1H), 6,90 (d, 2H), 6,99 (s, 1H), 7,54 (d, 2H). |
| | | | LCMS (Methode 2): Rₜ = 0,93 min; m/z = 453 (M+H)⁺ |
| **130**.**1** | 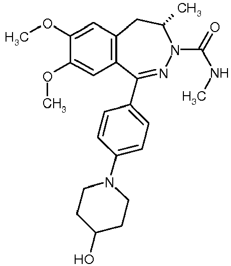 | (4*S*)-1-[4-(4-Hydroxypiperidin-1-yl)phenyl]-7,8-dimethoxy-*N*,4-dimethyl-4,5-dihydro-3*H*-2,3-benzodiazepin-3-carboxamid | [α]_{D}²⁰ = 336° (c = 1,00; Methanol) |
| | | | LCMS (Methode 2): Rₜ = 0,93 min; m/z = 453 (M+H)⁺ |
| **131** | 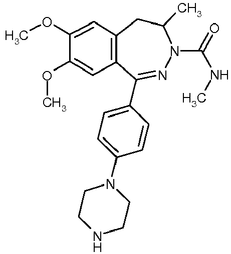 | (±)-7,8-Dimethoxy-*N*,4-dimethyl-1-[4-(piperazin-1-yl)phenyl]-4,5-dihydro-3*H*-2,3-benzodiazepin-3-carboxamid | ¹H-NMR (300MHz, DMSO-*d*₆): δ = 1,02 (d, 3H), 2,38 - 2,49 (m, 1H), 2,59 (d, 3H), 2, 80 (dd, 1H), 2,84-2,87 (m, 4H), 3,16 - 3,19 (m, 4H), 3,56 (s, 3H), 3,80 (s, 3H), 4,80 - 4,91 (m, 1H), 6,26 (q, 1H), 6,50 (s, 1H), 6,91 (d, 2H), 6,99 (s, 1H), 7,56 (d, 2H). |
| | | | LCMS (Methode 2): Rₜ = 0,69 min; m/z = 438 (M+H)⁺ |
| **131**.**1** | 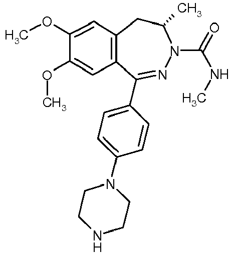 | (4*S*)-7,8-Dimethoxy-*N*,4-dimethyl-1-[4-(piperazin-1-yl)phenyl]-4,5-dihydro-3*H*-2,3-benzodiazepin-3-carboxamid | [α]_{D}²⁰ = +323,7° (c = 1,00; Methanol) |
| | | | LCMS (Methode 1): Rₜ = 0,70 min; m/z = 438 (M+H)⁺ |
| **127** | 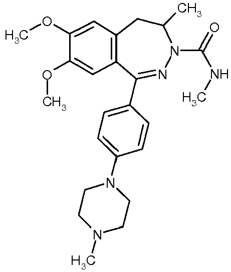 | (±)-7,8-Dimethoxy-*N*,4-dimethyl-1-[4-(4-methylpiperazin-1-yl)phenyl]-4,5-dihydro-3*H*-2,3-benzodiazepin-3-carboxamid | ¹H-NMR (300MHz, CDCl₃): δ = 1,10 (d, 3H), 2,39 (s, 3H), 2,58 - 2,66 (m, 4H), 2,72 (dd, 1H), 2,86 (d, 3H), 2, 94 (dd, 1H), 3,29 - 3,37 (m, 4H), 3,70 (s, 3H), 3,93 (s, 3H), 5,24 - 5,34 (m, 1H), 6,10 (q, 1H), 6,64 (s, 1H), 6,74 (s, 1H), 6,92 (d, 2H), 7,49 (d, 2H). |
| | | | LCMS (Methode 1): Rₜ = 0,73 min; m/z = 452 (M+H)⁺ |
| **132** | 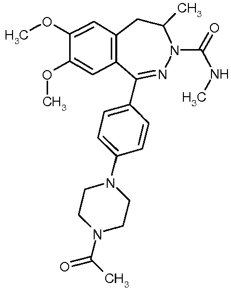 | (±)-1-[4-(4-Acetylpiperazin-1-yl)phenyl]-7,8-dimethoxy-*N*,4-dimethyl-4,5-dihydro-3*H*-2,3-benzodiazepin-3-carboxamid | ¹H-NMR (300MHz, DMSO-*d*₆): δ = 1,01 (d, 3H), 2,01 (s, 3H), 2,39 - 2,50 (m, 1H), 2,59 (d, 3H), 2, 81 (dd, 1H), 3,16 - 3,31 (m, 4H), 3,51 - 3,59 (m, 4H), 3,56 (s, 3H), 3,80 (s, 3H), 4,80-4,92 (m, 1H), 6,28 (q, 1H), 6,50 (s, 1H), 6,94 (d, 2H), 6,99 (s, 1H), 7,58 (d, 2H). |
| | | | LCMS (Methode 2): Rₜ = 0,97 min; m/z = 480 (M+H)⁺ |
| **132**.**1** | 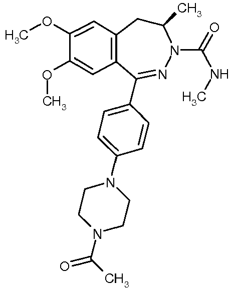 | (4*R*)-1-[4-(4-Acetylpiperazin-1-yl)phenyl]-7,8-dimethoxy-*N*,4-dimethyl-4,5-dihydro-3*H*-2,3-benzodiazepin-3-carboxamid | Präp. HPLC: Methode XI |
| | | | Analyt. HPLC (Methode L): Rₜ = 7,27 min |
| **132**.**2** | 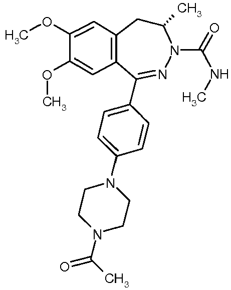 | (4*S*)-1-[4-(4-Acetylpiperazin-1-yl)phenyl]-7,8-dimethoxy-*N*,4-dimethyl-4,5-dihydro-3*H*-2,3-benzodiazepin-3-carboxamid | Präp. HPLC: Methode XI |
| | | | Analyt. HPLC (Methode L): Rₜ = 8,98 min |
| **133** | 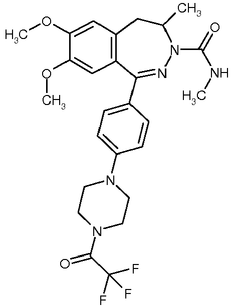 | (±)-7,8-Dimethoxy-*N*,4-dimethyl-1-{4-[4-(trifluoracetyl)piperazin-1-yl]phenyl}-4,5-dihydro-3*H*-2,3-benzodiazepin-3-carboxamid | ¹H-NMR (300MHz, DMSO-*d*₆): δ = 1,01 (d, 3H), 2,40 - 2,49 (m, 1H), 2,60 (d, 3H), 2, 81 (dd, 1H), 3,31-3,40 (m, 4H), 3,56 (s, 3H), 3,65-3,75 (m, 4H), 3,80 (s, 3H), 4,82-4,94 (m, 1H), 6,29 (q, 1H), 6,50 (s, 1H), 6,95 (d, 2H), 6,99 (s, 1H), 7,59 (d, 2H). |
| | | | LCMS (Methode 2): Rₜ = 1,22 min; m/z = 534 (M+H)⁺ |
| **134** | 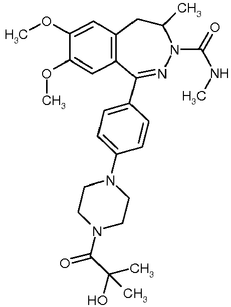 | (±)-1-{4-[4-(2-Hydroxy-2-methylpropanoyl)piperazin-1-yl]phenyl}-7,8-dimethoxy-*N*,4-dimethyl-4,5-dihydro-3*H*-2,3-benzodiazepin-3-carboxamid | ¹H-NMR (300MHz, CDCl₃): δ = 1,08 (d, 3H), 1,54 (s, 6H), 2,74 (dd, 1H), 2,87 (d, 3H), 2,97 (dd, 1H), 3,27-3,34 (m, 4H), 3,69 (s, 3H), 3,65-3,92 (m, 4H), 3,93 (s, 3H), 4.07 (s, 1H), 6,25 - 6,37 (m, 1H), 6,17 (q, 1H), 6,62 (s, 1H), 6,74 (s, 1H), 6,92 (d, 2H), 7,50 (d, 2H). |
| | | | LCMS (Methode 1): Rₜ = 1,02 min; m/z = 524 (M+H)⁺ |
| **134**.**1** | 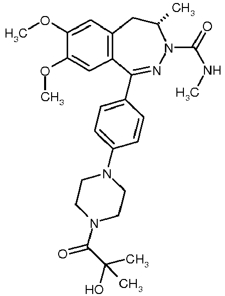 | (4*S*)-1-{4-[4-(2-Hydroxy-2-methylpropanoyl)piperazin-1-yl]phenyl}-7,8-dimethoxy-*N*,4-dimethyl-4,5-dihydro-3*H*-2,3-benzodiazepin-3-carboxamid | [α]_{D}²⁰ = +291.8° (c = 1,00; Methanol) |
| | | | LCMS (Methode 1): Rₜ = 1,01 min; m/z = 524 (M+H)⁺ |
| **135** | 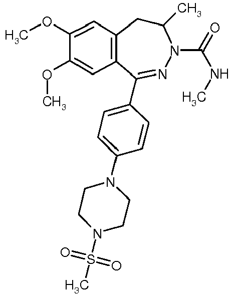 | (±)-7,8-Dimethoxy-*N*,4-dimethyl-1-{4-[4-(methylsulfonyl)piperazin-1-yl]phenyl}-4,5-dihydro-3*H*-2,3-benzodiazepin-3-carboxamid | ¹H-NMR (300MHz, DMSO-*d*₆): δ = 1,01 (d, 3H), 2,38 - 2,49 (m, 1H), 2,60 (d, 3H), 2, 81 (dd, 1H), 2,89 (s, 3H), 3,17 - 3,26 (m, 4H), 3,30 - 3,38 (m, 4H), 3,56 (s, 3H), 3,80 (s, 3H), 4,82 - 4,94 (m, 1H), 6,29 (q, 1H), 6,51 (s, 1H), 6,97 (d, 2H), 6,99 (s, 1H), 7,58 (d, 2H). |
| | | | LCMS (Methode 2): Rₜ = 1,04 min; m/z = 516 (M+H)⁺ |
| **135.1** | 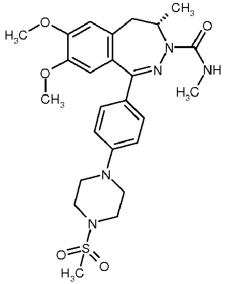 | (4*S*)-7,8-Dimethoxy-*N*,4-dimethyl-1-{4-[4-(methylsulfonyl)piperazin-1-yl]phenyl}-4,5-dihydro-3*H*-2,3-benzodiazepin-3-carboxamid | [α]_{D}²⁰ = +314.3° (c = 1,00; Methanol) |
| | | | LCMS (Methode 1): Rₜ = 1,08 min; m/z = 516 (M+H)⁺ |
| **136** | 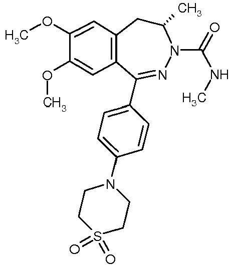 | (4*S*)-1-[4-(1,1-Dioxidothiomorpholin-4-yl)phenyl]-7,8-dimethoxy-*N*,4-dimethyl-4,5-dihydro-3*H*-2,3-benzodiazepin-3-carboxamid | ¹H-NMR (300MHz, DMSO-*d*₆): δ = 1,01 (d, 3H), 2,40 - 2,50 (m, 1H), 2,60 (d, 3H), 2, 81 (dd, 1H), 3,06-3,15 (m, 4H), 3,57 (s, 3H), 3,80 (s, 3H), 3,82 - 3,98 (m, 4H), 4,82 - 4,94 (m, 1H), 6,29 (q, 1H), 6,52 (s, 1H), 7,00 (s, 1H), 7,01 (d, 2H), 7,59 (d, 2H). |
| | | | LCMS (Methode 2): Rₜ = 0,96 min; m/z = 487 (M+H)⁺ |
| | | | [α]_{D}²⁰ = +309,1° (c = 1,00; Methanol) |
| **137** | 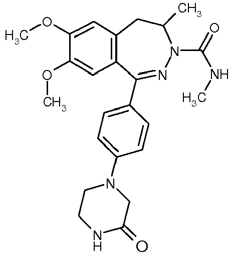 | (±)-7,8-Dimethoxy-*N*,4-dimethyl-1-[4-(3-oxopiperazin-1-yl)phenyl]-4,5-dihydro-3*H*-2,3-benzodiazepin-3-carboxamid | ¹H-NMR (300MHz, DMSO-*d*₆): δ = 1,02 (d, 3H), 2,38 - 2,51 (m, 1H), 2,60 (d, 3H), 2, 81 (dd, 1H), 3,26-3,33 (m, 2H), 3,44 - 3,50 (m, 2H), 3,56 (s, 3H), 3,76 - 3,79 (m, 2H), 3,80 (s, 3H), 4,80 - 4,93 (m, 1H), 6,27 (q, 1H), 6,50 (s, 1H), 6,89 (d, 2H), 6,99 (s, 1H), 7,58 (d, 2H), 8,08 (s, 1H). |
| | | | LCMS (Methode 2): Rₜ = 0,88 min; m/z = 452 (M+H)⁺ |
| **138** | 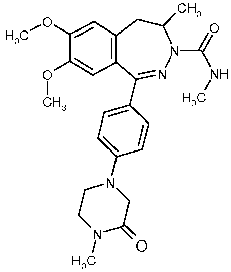 | (±)-7,8-Dimethoxy-*N*,4-dimethyl-1-[4-(4-methyl-3-oxopiperazin-1-yl)phenyl]-4,5-dihydro-3*H*-2,3-benzodiazepin-3-carboxamid | ¹H-NMR (300MHz, CDCl₃): δ = 1,09 (d, 3H), 2,73 (dd, 1H), 2, 87 (d, 3H), 2,96 (dd, 1H), 3,06 (s, 3H), 3,49-3,54 (m, 2H), 3,55 - 3,61 (m, 2H), 3,69 (s, 3H), 3,93 (s, 3H), 3,96 (s, 2H), 5,26 - 5,35 (m, 1H), 6,15 (q, 1H), 6,62 (s, 1H), 6,74 (s, 1H), 6,86 (d, 2H), 7,51 (d, 2H). |
| | | | LCMS (Methode 2): Rₜ = 0,96 min; m/z = 466 (M+H)⁺ |
| **138**.**1** | 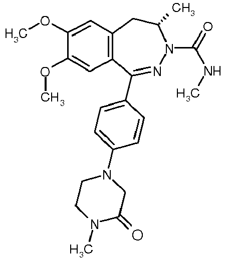 | (4*S*)-7,8-Dimethoxy-*N*,4-dimethyl-1-[4-(4-methyl-3-oxopiperazin-1-yl)phenyl]-4,5-dihydro-3*H*-2,3-benzodiazepin-3-carboxamid | [α]_{D}²⁰ = +327.3° (c = 1,00; Methanol) |
| | | | LCMS (Methode 1): Rₜ = 0,95 min; m/z = 466 (M+H)⁺ |
| **139** | 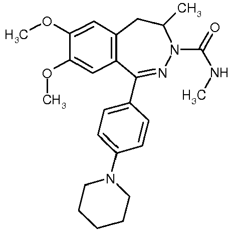 | (±)-7,8-Dimethoxy-*N*,4-dimethyl-1-[4-(piperidin-1-yl)phenyl]-4,5-dihydro-3*H*-2,3-benzodiazepin-3-carboxamid | ¹H-NMR (300MHz, CDCl₃): δ = 1,13 (d, 3H), 1,56 -1,76 (m, 6H), 2,71 (dd, 1H), 2, 86 (d, 3H), 2,91 (dd, 1H), 3,24 - 3,32 (m, 4H), 3,70 (s, 3H), 3,93 (s, 3H), 5,20 - 5,30 (m, 1H), 6,03 (q, 1H), 6,65 (s, 1H), 6,75 (s, 1H), 6,91 (d, 2H), 7,49 (d, 2H). |
| | | | LCMS (Methode 2): Rₜ = 1,24 min; m/z = 437 (M+H)⁺ |

Analog zur Herstellung von Beispiel **7** (alternative Methode) wurden aus Beispiel **51A** und den entsprechenden kommerziell erhältlichen Aminen folgende Beispielverbindungen hergestellt:

| Nr | Struktur | Name | Analytische Daten |
|---|---|---|---|
| **140** | 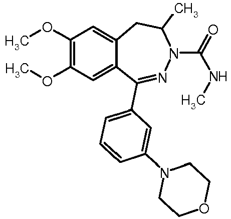 | (±)-7,8-Dimethoxy-*N*,4-dimethyl-1-[3-(morpholin-4-yl)phenyl]-4,5-dihydro-3*H*-2,3-benzodiazepin-3-carboxamid | ¹H-NMR (300MHz, CDCl₃): δ = 1,00 (d, 3H), 2,83 (dd, 1H), 2,87 (d, 3H), 3,10 (dd, 1H), 3,16 (m, 4H), 3,65 (s, 3H), 3,86 (m, 4H), 3,93 (s, 3H), 5,45 (m, 1H), 6,47 (qbr, 1H), 6,61 (s, 1H), 6,71 (s, 1H), 6,96 (dbr, 1H), 7,00 (sbr, 1H), 7,03 (dbr, 1H), 7,31 (dd, 1H). |
| | | | LCMS (Methode 3): Rₜ = 1,11 min; m/z = 439 (M+H)⁺ |
| **141** | 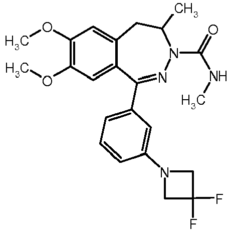 | (±)-1-[3-(3,3-Difluorazetidin-1-yl)phenyl]-7,8-dimethoxy-*N*,4-dimethyl-4,5-dihydro-3*H*-2,3-benzodiazepin-3-carboxamid | ¹H-NMR (400MHz, CDCl₃): δ = 0,99 (d, 3H), 2,84 (dd, 1H), 2,87 (d, 3H), 3,11 (dd, 1H), 3,66 (s, 3H), 3,93 (s, 3H), 4,23 (m, 4H), 5,46 (m, 1H), 6,48 (m, 1H), 6,55 (dbr, 1H), 6,59 (m, 2H), 6,71 (s, 1H), 6,96 (dbr, 1H), 7,28 (dd, 1H). |
| | | | LCMS (Methode 3): Rₜ = 1,22 min; m/z = 445 (M+H)⁺ |
| **142** | 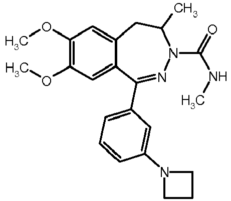 | (±)-1-[3-(Azetidin-1-yl)phenyl]-7,8-dimethoxy-*N*,4-dimethyl-4,5-dihydro-3*H*-2,3-benzodiazepin-3-carboxamid | ¹H-NMR (300MHz, CDCl₃): δ = 1,00 (d, 3H), 2,40 (m, 2H), 2,82 (dd, 1H), 2,87 (d, 3H), 3,09 (dd, 1H), 3,67 (s, 3H), 3,91 (m, 4H), 3,93 (s, 3H), 5,43 (m, 1H), 6,48 (m, 1H), 6,57 (m, 2H), 6,62 (s, 1H), 6,71 (s, 1H), 6,89 (m, 1H), 7,24 (dd, 1H). |
| | | | LCMS (Methode 3): Rₜ = 1,22 min; m/z = 409 (M+H)⁺ |
| **143** | 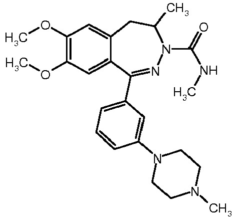 | (±)-7,8-Dimethoxy-*N*,4-dimethyl-1-[3-(4-methylpiperazin-1-yl)phenyl]-4,5-dihydro-3*H*-2,3-benzodiazepin-3-carboxamid | ¹H-NMR (400MHz, CDCl₃): δ = 0,99 (d, 3H), 2,36 (s, 3H), 2,59 (m, 4H), 2,83 (dd, 1H), 2,86 (d, 3H), 3,09 (dd, 1H), 3,22 (m, 4H), 3,64 (s, 3H), 3,93 (s, 3H), 5,43 (m, 1H), 6,47 (qbr, 1H), 6,61 (s, 1H), 6,71 (s, 1H), 6,99 (m, 2H), 7,01 (sbr, 1H), 7,28 (dd, 1H). |
| | | | LCMS (Methode 3): Rₜ = 0,76 min; m/z = 452 (M+H)⁺ |

Analog zur Herstellung von Beispiel 7 (alternative Methode) wurden aus Beispiel **60A** und den entsprechenden kommerziell erhältlichen Aminen folgende Beispielverbindungen hergestellt:

| Nr | Struktur | Name | Analytische Daten |
|---|---|---|---|
| **144** | 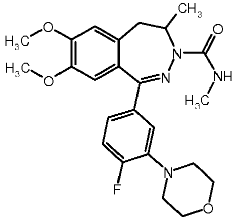 | (±)-1-[4-Fluor-3-(morpholin-4-yl)phenyl]-7,8-dimethoxy-*N*,4-dimethyl-4,5-dihydro-3*H-*2,3-benzodiazepin-3-carboxamid | ¹H-NMR (300MHz, CDCl₃): δ = 0,99 (d, 3H), 2,85 (dd, 1H), 2,88 (d, 3H), 3,08 (m, 1H), 3,09 (m, 4H), 3,66 (s, 3H), 3,88 (m, 4H), 3,94 (s, 3H), 5,45 (m, 1H), 6,43 (qbr, 1H), 6,56 (s, 1H), 6,72 (s, 1H), 7,02-7,18 (m, 3H). |
| | | | LCMS (Methode 3): Rₜ = 0,96 min; m/z = 457 (M+H)⁺ |
| **145** | 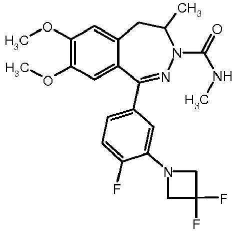 | (±)-1-[3-(3,3-Difluorazetidin-1-yl)-4-fluorphenyl]-7,8-dimethoxy-*N*,4-dimethyl-4,5-dihydro-3*H*-2,3-benzodiazepin-3-carboxamid | ¹H-NMR (400MHz, CDCl₃): δ = 0,99 (d, 3H), 2,83 (dd, 1H), 2,88 (d, 3H), 3,09 (dd, 1H), 3,68 (s, 3H), 3,94 (s, 3H), 4,32 (m, 4H), 5,45 (m, 1H), 6,41 (m, 1H), 6,56 (d, 1H), 6,63 (dd, 1H), 6,72 (s, 1H), 6,91 (ddd, 1H), 7,02 (dd, 1H). |
| | | | LCMS (Methode 3): Rₜ = 1,31 min; m/z = 463 (M+H)⁺ |
| **146** | 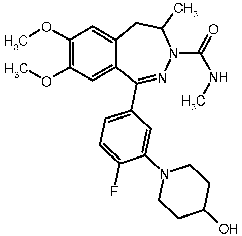 | (±)-1-[4-Fluor-3-(4-hydroxypiperidin-1-yl)phenyl]-7,8-dimethoxy-*N*,4-dimethyl-4,5-dihydro-3*H*-2,3-benzodiazepin-3-carboxamid | ¹H-NMR (400MHz, CDCl₃): δ = 1,00 (d, 3H), 1,76 (m, 2H), 2,03 (m, 2H), 2,82 (dd, 1H), 2,85 (m, 2H), 2,88 (d, 3H), 3,07 (m, 1H), 3,34 (m, 2H), 3,66 (s, 3H), 3,85 (m, 1H), 3,94 (s, 3H), 5,42 (m, 1H), 6,40 (m, 1H), 6,56 (s, 1H), 6,72 (s, 1H), 7,00-7,14 (m, 3H). |
| | | | LCMS (Methode 3): Rₜ = 1,03 min; m/z = 471 (M+H)⁺ |

### Beispiel 147

### (±)-7,8-Dimethoxy-N,4-dimethyl-1-[4-(5-methyl-3-phenyl-1H-pyrazol-1-yl)phenyl]-4,5-dihydro-3H-2,3-benzodiazepin-3-carboxamid

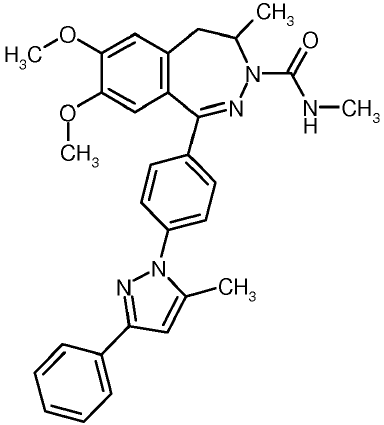

100 mg (0,231 mmol) (±)-1-(4-Bromphenyl)-7,8-dimethoxy-*N*,4-dimethyl-4,5-dihydro-3*H*-2,3-benzodiazepin-3-carboxamid (Beispiel **49A**), 44 mg (0,28 mmol) 5-Methyl-3-phenyl-1*H*-pyrazol, 67 mg (0,49 mmol) Kaliumcarbonat, 4,4 mg (0,023 mmol) Kupfer(I)iodid unter Argon in 2 ml entgastem Toluol vorgelegt. Anschließend wurden unter Argon 13,2 mg (0,093 mmol) (±)-(*trans*)-*N*,*N*'-Dimethylcyclohexan-1,2-diamin zugegeben, erneut entgast und die Mischung für 16 Stunden bei 140°C erhitzt. Nach dem Abkühlen wurde die Mischung abfiltriert, mit Ethylacetat gewaschen und im Vakuum getrocknet. Der Rückstand wurde mittels präparativer HPLC gereinigt. Man erhielt 17 mg (14% d. Th.) des gewünschten Produkts als Feststoff.
LCMS (Methode 2): Rₜ = 1,44 min; m/z = 510 (M+H)⁺
¹H-NMR (300MHz, CDCl₃): δ = 1,01 (d, 3H), 2,45 (s, 3H), 2,87 (dd, 1H), 2,92 (d, 3H), 3,14 (dd, 1H), 3,70 (s, 3H), 3,95 (s, 3H), 5,43-5,52 (m, 1H), 6,48-6,56 (m, 1H), 6,58 (s, 1H), 6,65 (s, 1H), 6,74 (s, 1H), 7,34 (t, 1H), 7,39-7,47 (m, 2H), 7,58 (d, 2H), 7,63 (d, 2H), 7,89 (d, 2H).

Analog zu Beispiel **147** wurden ausgehend vom Bromderivat **49A** durch Umsetzung mit den entsprechenden, kommerziell erhältlichen Pyrazolen folgende Beispielverbindung hergestellt. Durch die Verwendung des enantiomerenreinen Bromides **49**.**2A** konnten direkt die entsprechenden enantiomerenreinen Produkte erhalten werden.

| Nr | Struktur | Name | Analytische Daten |
|---|---|---|---|
| **147**.**1** | 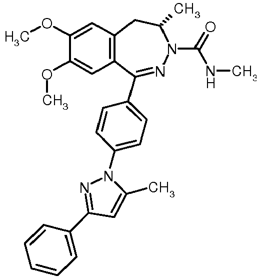 | (4*S*)-7,8-Dimethoxy-*N*,4-dimethyl-1-[4-(5-methyl-3-phenyl-1*H-*pyrazol-1-yl)phenyl]-4,5-dihydro-3*H*-2,3-benzodiazepin-3-carboxamid | LCMS (Methode 2): Rₜ = 1,44 min; m/z = 510 (M+H)⁺. |
| | | | [a]_{D}²⁰ = 68,8° (c = 1,00; Methanol) |
| **148** | 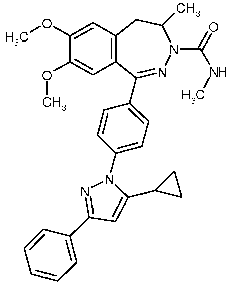 | (±)-1-[4-(5-Cyclopropyl-3-phenyl-1*H*-pyrazol-1-yl)phenyl]-7,8-dimethoxy-*N*,4-dimethyl-4,5-dihydro-3*H*-2,3-benzodiazepin-3-carboxamid | ¹H-NMR (300 MHz, CDCl₃): δ = 0,89 (d, 2H), 1,01 (d, 3H), 1,07 (d, 2H), 1,84-1,98 (m, 1H), 2,86 (dd, 1H), 2,92 (d, 3H), 3,14 (d, 1H), 3,70 (s, 3H), 3,95 (s, 3H), 5,40-5,53 (m, 1H), 6,35 (s, 1H), 6,52 (s, br, 1H), 6,65 (s, 1H), 6,74 (s, 1H), 7,34 (d, 1H), 7,42 (t, 2H), 7,64 (d, 2H), 7,77 (d, 2H), 7,88 (d, 2H). |
| | | | LCMS (Methode 2): Rₜ = 1,52 min; m/z = 536 (M+H)⁺. |
| **148**.**1** | 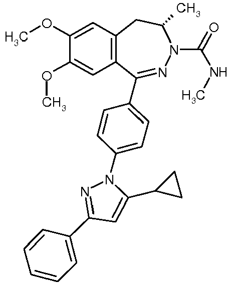 | (4*S*)-1-[4-(5-Cyclopropyl-3-phenyl-1*H*-pyrazol-1-yl)phenyl]-7,8-dimethoxy-*N*,4-dimethyl-4,5-dihydro-3*H*-2,3-benzodiazepin-3-carboxamid | LCMS (Methode 2): Rₜ = 1,52 min; m/z = 536 (M+H)⁺. |
| | | | [a]_{D}²⁰ = 103,9° (c = 1,00; Methanol) |
| **149** | 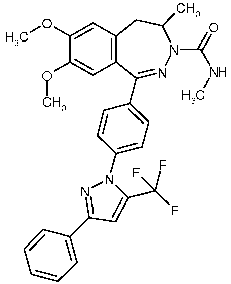 | (±)-7,8-Dimethoxy-*N*,4-dimethyl-1-{4-[3-phenyl-5-(trifluormethyl)-1*H-*pyrazol-1-yl]phenyl}-4,5-dihydro-3*H*-2,3-benzodiazepin-3-carboxamid | ¹H-NMR (400 MHz, CDCl₃): δ = 0,98 (d, 3H), 2,79-2,88 (m, 1H), 2,90 (s, br., 3H), 3,12 (d, 1H), 3,65 (s, 3H), 3,93 (s, 3H), 5,41-5,53 (m, 1H), 6,42-6,52 (m, 1H), 6,53 (s, 1H), 6,70 (s, 1H), 6,78 (s, 1H), 7,26-7,31 (m, 2H), 7,31-7,41 (m, 5H), 7,49 (d, 2H). |
| | | | LCMS (Methode 2): Rₜ = 1,52 min; m/z = 564 (M+H)⁺. |
| **150** | 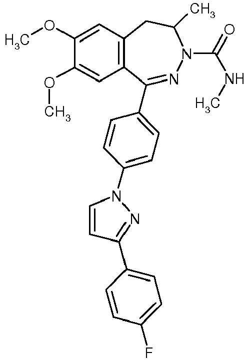 | (±)-1-{4-[3-(4-Fluorphenyl)-1*H-*pyrazol-1-yl]phenyl}-7,8-dimethoxy-*N*,4-dimethyl-4,5-dihydro-3*H*-2,3-benzodiazepin-3-carboxamid | ¹H-NMR (400MHz, CDCl₃): δ = 1,02 (d, 3H), 2,86 (dd, 1H), 2,91 (s, 3H), 3,13 (d, 1H), 3,67 (s, 3H), 3,95 (s, 3H), 5,46 (m, 1H), 6,49 (m, 1H), 6,63 (s, 1H), 6,72-6,81 (m, 2H), 7,09-7,19 (m, 2H), 7,62 (d, 2H), 7,81 (d, 2H), 7,87-7,95 (m, 2H), 7,99-8,04 (m, 1H). |
| | | | LCMS (Methode 2): Rₜ = 1,46 min; m/z = 514 (M+H)⁺. |
| **150**.**1** | 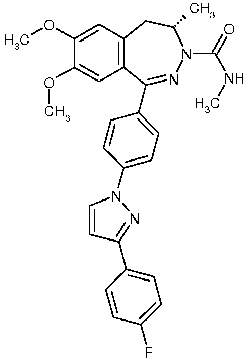 | (4*S*)-1-{4-[3-(4-Fluorphenyl)-1*H-*pyrazol-1-yl]phenyl}-7,8-dimethoxy-*N*,4-dimethyl-4,5-dihydro-3*H*-2,3-benzodiazepin-3-carboxamid | Präp. HPLC: Methode V |
| | | | Analyt. HPLC (Methode F): Rₜ = 12,1 min |
| | | | LCMS (Methode 2): Rₜ = 1,46 min; m/z = 514 (M+H)⁺. |
| | | | [a]_{D}²⁰ = 142,0° (c = 1,00; Methanol) |
| **150.2** | 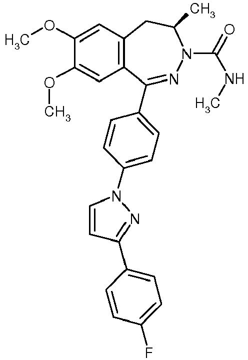 | (4*R*)-1-{4-[3-(4-Fluorphenyl)-1*H-*pyrazol-1-yl]phenyl}-7,8-dimethoxy-*N*,4-dimethyl-4,5-dihydro-3*H*-2,3-benzodiazepin-3-carboxamid | Präp. HPLC: Methode V |
| | | | Analyt. HPLC (Methode F): Rₜ = 7,6 min |
| | | | LCMS (Methode 2): Rₜ = 1,46 min; m/z = 514 (M+H)⁺. |
| | | | [a]_{D}²⁰ = -137.3° (c = 1,00; Methanol) |
| **151** | 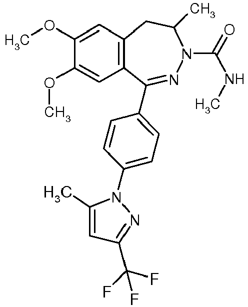 | (±)-7,8-Dimethoxy-*N*,4-dimethyl-1-{4-[5-methyl-3-(trifluormethyl)-1*H-*pyrazol-1-yl]phenyl}-4,5-dihydro-3*H*-2,3-benzodiazepin-3-carboxamid | ¹H-NMR (500MHz, CDCl₃): δ = 0,98 (d, 3H), 2,41 (s, 3H), 2,84 - 2,95 (m, 4H), 3,15 (dd, 1H), 3,67 (s, 3H), 3,94 (s, 3H), 5,46 - 5,53 (m, 1H), 6,50 (s, 1H), 6,51 - 6,55 (m, 1H), 6,60 (s, 1H), 6,73 (s, 1H), 7,49 (d, 2H), 7,60 - 7,65 (m, 2H). |
| | | | LCMS (Methode 2): Rₜ = 1,38 min; m/z = 502 (M+H)⁺. |

### Beispiel 152

### (±)-7,8-Dimethoxy-N,4-dimethyl-1-[4-(1H-1,2,4-triazol-1-yl)phenyl]-4,5-dihydro-3H-2,3-benzodiazepin-3-carboxamid

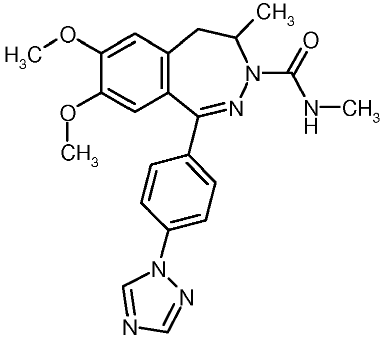

100 mg (0,231 mmol) (±)-1-(4-Bromphenyl)-7,8-dimethoxy-*N*,4-dimethyl-4,5-dihydro-3*H*-2,3-benzodiazepin-3-carboxamid (Beispiel **49A**), 19 mg (0,28 mmol) 1*H*-1,2,4-Triazol, 98 mg (0,46 mmol) Kaliumphosphat, 2,2 mg (0,012 mmol) Kupfer(I)iodid und 3,3 mg (0,023 mmol) (±)-(*trans*)-*N,N*-Dimethylcyclohexan-1,2-diamin werden unter Argon in 2 ml DMF vorgelegt, die Lösung entgast und die Mischung für 16 Stunden bei 110°C erhitzt. Da der Umsatz gering war, wurden weitere 2 mg Kupfer(I)iodid zugesetzt, erneut entgast und für weitere 10h bei 140°C erhitzt. Nach dem Abkühlen wurde die Mischung mit ges. wässriger Ammoniumchloridlösung versetzt und dreimal mit Ethylacetat extrahiert und die vereinigten organischen Phasen im Vakuum getrocknet. Der Rückstand wurde mittels präparativer HPLC gereinigt. Man erhielt 15 mg (2% d. Th.) des gewünschten Produkts als Feststoff.
LCMS (Methode 2): Rₜ = 1,01 min; m/z = 421 (M+H)⁺
¹H-NMR (500MHz, DMSO-*d*₆): δ = 0,98 (d, 3H), 2,70 (d, 3H), 2,73 (dd, 1H), 2,97 (dd, 1H), 3,57 (s, 3H), 3,85 (s, 3H), 5,09-5,17 (m, 1H), 6,55 (s, 1H), 6,67 (q, 1H), 7,03 (s, 1H), 7,80-7,85 (m, 2H), 7,89 - 7,94 (m, 2H), 8,26 (s, 1H), 9,39 (s, 1H).

Analog zu Beispiel **152** wurden ausgehend vom Bromderivat **49A** durch Umsetzung mit den entsprechenden, kommerziell erhältlichen Triazolen folgende Beispielverbindung hergestellt.

| Nr | Struktur | Name | Analytische Daten |
|---|---|---|---|
| **153** | 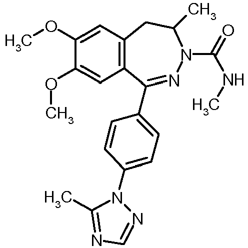 | (±)-7,8-Dimethoxy-*N*,4-dimethyl-1-[4-(5-methyl-1*H*-1,2,4-triazol-1-yl)phenyl]-4,5-dihydro-3*H*-2,3-benzodiazepin-3-carboxamid | ¹H-NMR (400MHz, CDCl₃): δ = 0,98 (d, 3H), 2,51 (s, 3H), 2,87-2,95 (dd, 1H), 2,90 (d, 3H), 3,14 (dd, 1H), 3,65 (s, 3H), 3,94 (s, 3H), 5,43 - 5,53 (m, 1H), 6,51 (q, 1H), 6,58 (s, 1H), 6,73 (s, 1H), 7,61 (d, 2H), 7,68 (d, 2H), 8,51 (s, br, 1H). |
| | | | LCMS (Methode 2): Rₜ = 1,04 min; m/z = 435 (M+H)+. |
| **154** | 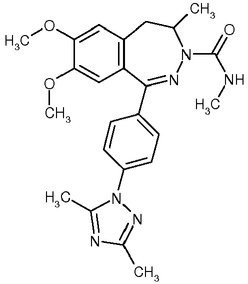 | (±)-1-[4-(3,5-Dimethyl-1*H*-1,2,4-triazol-1-yl)phenyl]-7,8-dimethoxy-*N*,4-dimethyl-4,5-dihydro-3*H*-2,3-benzodiazepin-3-carboxamid | ¹H-NMR (300MHz, CDCl₃): δ = 0,97 (d, 3H), 2,50 (s, 3H), 2,64 (s, br, 3H), 2,88 (dd, 1H), 2,91 (d, 3H), 3,15 (d, 1H), 3,66 (s, 3H), 3,94 (s, 3H), 5,43-5,55 (m, 1H), 6,53 (q, 1H), 6,57 (s, 1H), 6,73 (s, 1H), 7,48 (d, 2H), 7,61-7,70 (m, 2H). |
| | | | LCMS (Methode 2): Rₜ = 1,01 min; m/z = 449 (M+H)⁺. |

### Beispiel 155

### (±)-8-tert-Butyl-1-[4-(3,5-dimethylisoxazol-4-yl)phenyl]-N,4-dimethyl-4,5-dihydro-3H-2,3-benzodiazepin-3-carboxamid

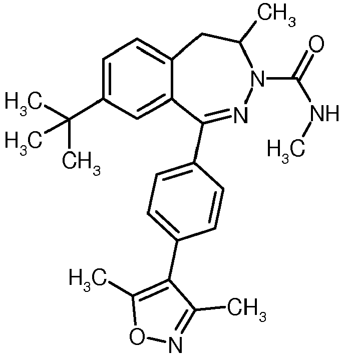

150 mg (0,391 mmol) (±)-8-*tert*-Butyl-1-(4-chlorphenyl)-*N*,4-dimethyl-4,5-dihydro-3*H*-2,3-benzodiazepin-3-carboxamid (Beispiel **83A**) wurden in 7,3 ml eines 1:0,1 Gemisches aus THF und Wasser gelöst und mit Argon entgast. Danach wurden 113,5 mg (1,95 mmol) Kaliumfluorid, 57,3 mg (0,41 mmol) (3,5-Dimethylisoxazol-4-yl)boronsäure und 15,4 mg (0,02 mmol) Chloro(2-dicyclohexylphosphino-2',4',6'-triisopropyl-1,1'-biphenyl)[2-(2'-amino-1,1'-biphenyl)]palladium(II) (CAS [1310584-14-5]) zugegeben und die Reaktionsmischung nochmals sorgfältig mit Argon entgast. Es wurde für 5h auf 80°C erhitzt. Nach Abkühlen wurde mit gesättigter wässriger Natriumhydrogencarbonat-Lösung versetzt und dreimal mit Ethylacetat ausgeschüttelt. Die vereinigten organischen Phasen wurden getrocknet und am Rotationsverdampfer zur Trockne eingeengt. Der Rückstand wurde mittels präparativer HPLC gereinigt. Man erhielt 43 mg (25% d. Th.) des gewünschten Produkts als Feststoff.
LCMS (Methode 2): Rₜ = 1,50 min; m/z = 445 (M+H)+
¹H-NMR (300 MHz, CDCl₃): δ = 0,99 (d, 3H), 1,22 (s, 9H), 2,33 (s, 3H), 2,46 (s, 3H), 2,87 (dd, 1H), 2,90 (d, 3H), 3,10 (dd, 1H), 5,36-5,49 (m, 1H), 6,45 (q, 1H), 7,14 (d, 1H), 7,18 (d, 1H), 7,30 (d, 2H), 7,37 (dd, 1H), 7,59 (d, 2H).

### Beispiel 156

### (±)-7-Chlor-1-[4-(3,5-dimethylisoxazol-4-yl)phenyl]-N,4-dimethyl-8-(trifluormethoxy)-4,5-dihydro-3H-2,3-benzodiazepin-3-carboxamid

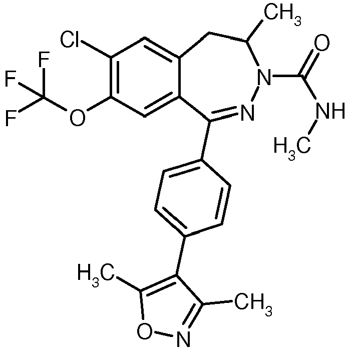

Die Herstellung erfolgte analog dem Verfahren zur Herstellung von Beispiel 155 unter Verwendung von Beispiel 15 als Ausgangsmaterial. Man erhielt ein Gemisch der gewünschten Verbindung mit dem regioisomeren (±)-1-(4-Chlorphenyl)-7-(3,5-dimethylisoxazol-4-yl)-*N*,4-dimethyl-8-(trifluormethoxy)-4,5-dihydro-3*H*-2,3-benzodiazepin-3-carboxamid. Die Regioisomere wurden mittels präparativer HPLC getrennt (Methode: System: Waters Autopurificationsystem: Pump 254, Sample Manager 2767, CFO, DAD 2996, SQD 3100, Prep FC; Säule: XBrigde C18 5µm 100x30 mm; Eluent: A = H2O + 0,1% HCOOH (99%), B = Acetonitril; Gradient: 0-8 min 60-80% B Fluss: 50 ml/min; Temperatur: RT; Detektion: DAD scan range 210-400 nm). Aus 100 mg Beispiel **15** erhielt man neben 14,7 mg des Regioisomeren (LCMS (Methode 2): Rₜ = 1,54 min; m/z = 507 (M+H)⁺; ¹H-NMR (400MHz, CDCl₃): δ = 0,97 (d, 3H), 2,20 (s, 3H), 2,34 (s, 3H), 2,92 (d, 3H), 2,98 (dd, 1H), 3,18 (dd, 1H), 5,53 - 5,59 (m, 1H), 6,52 (q, 1H), 7,10 (s, 1H), 7,13 (s, 1H), 7,40 - 7,46 (m, 4H).) 5 mg der gewünschten Verbindung.
LCMS (Methode 2): Rₜ = 1,51 min; m/z = 507 (M+H)⁺
¹H-NMR (400MHz, CDCl₃): δ = 0,90 (d, 3H), 2,26 (s, 3H), 2,40 (s, 3H), 2,85 (d, 3H), 2,88 (dd, 1H), 3,08 (dd, 1H), 5,44 - 5,52 (m, 1H), 6,48 (q, 1H), 7,05 (d, 1H), 7,24 (s, 1H), 7,28 (d, 2H), 7,45 (d, 2H)

### Enantiomerentrennung:

35 mg (±)-7-Chlor-1-[4-(3,5-dimethylisoxazol-4-yl)phenyl]-*N*,4-dimethyl-8-(trifluormethoxy)-4,5-dihydro-3*H*-2,3-benzodiazepin-3-carboxamid wurden (Beispiel 165) wurden mittels chiraler präparativer HPLC mit der Methode XVIII in die Enantiomere getrennt:

### Beispiel 156.1: (4S)-7-Chlor-1-[4-(3,5-dimethylisoxazol-4-yl)phenyl]-N,4-dimethyl-8-(trifluormethoxy)-4,5-dihydro-3H-2,3-benzodiazepin-3-carboxamid

15 mg gelblicher Feststoff, HPLC (Methode W): Rₜ = 1,91 min, Reinheit 98%

### Beispiel 156.2: (4R)-7-Chlor-1-[4-(3,5-dimethylisoxazol-4-yl)phenyl]-N,4-dimethyl-8-(trifluormethoxy)-4,5-dihydro-3H-2,3-benzodiazepin-3-carboxamid

10 mg gelblicher Feststoff, HPLC (Methode W): Rₜ = 2,86 min, Reinheit 96%

Unter Verwendung des Verfahrens zur Herstellung von Beispiel 155 wurden folgende Verbindungen aus den entsprechenden Chlorphenyl-Vorläuferverbindungen hergestellt:

| Nr | Struktur | Name | Analytische Daten |
|---|---|---|---|
| **157** | 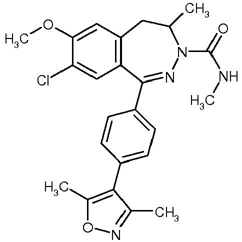 | (±)-8-Chlor-1-[4-(3,5-dimethylisoxazol-4-yl)phenyl]-7-methoxy-*N*,4-dimethyl-4,5-dihydro-3*H*-2,3-benzodiazepin-3-carboxamid | ¹H-NMR (300 MHz, CDCl₃): δ = 1,00 (d, 3H), 2,34 (s, 3H), 2,47 (s, 3H), 2,84-2,97 (m, 4H), 3,14 (dd, 1H), 3,97 (s, 3H), 5,43-5,55 (m, 1H), 6,43-6,55 (m, 1H), 6,80 (s, 1H), 7,17 (s, 1H), 7,30 (d, 2H), 7,53 (d, 2H). |
| | | | LCMS (Methode 2): Rₜ = 1,35 min; m/z = 453 (M+H)⁺. |
| **157.1** | 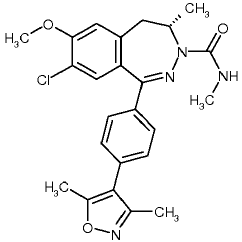 | (4*S*)-8-Chlor-1-[4-(3,5-dimethylisoxazol-4-yl)phenyl]-7-methoxy-*N*,4-dimethyl-4,5-dihydro-3*H*-2,3-benzodiazepin-3-carboxamid | LCMS (Methode 2): Rₜ = 1,35 min; m/z = 453 (M+H)⁺ |
| | | | HPLC (Methode N): Rₜ = 2,82 min |
| | | | [α]D²⁰ = 131,8° (c = 1,00; Chloroform) |
| **157.2** | 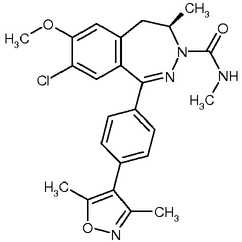 | (4*R*)-8-Chlor-1-[4-(3,5-dimethylisoxazol-4-yl)phenyl]-7-methoxy-*N*,4-dimethyl-4,5-dihydro-3*H*-2,3-benzodiazepin-3-carboxamid | LCMS (Methode 2): Rₜ = 1,35 min; m/z = 453 (M+H)⁺ |
| | | | HPLC (Methode N): Rₜ = 4,27 min |
| **158** | 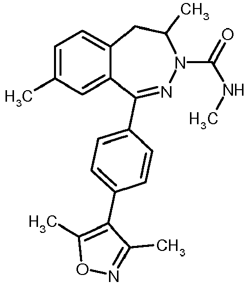 | (±)-1-[4-(3,5-Dimethylisoxazol-4-yl)phenyl]-*N*,4,8-trimethyl-4,5-dihydro-3*H*-2,3-benzodiazepin-3-carboxamid | ¹H-NMR (600 MHz, CDCl₃): δ = 0,98 (d, 3H), 2,28 (s, 3H), 2,34 (s, 3H), 2,47 (s, 3H), 2,87 (dd, 1H), 2,90 (d, 3H), 3,10 (dd, 1H), 5,39 - 5,44 (m, 1H), 6,45 (q, 1H), 6,96 (s, 1H), 7,12-7,17 (m, 2H), 7,30 (d, 2H), 7,57 (d, 2H). |
| | | | LCMS (Methode 2): Rₜ = 1,35 min; m/z = 403 (M+H)⁺. |

### Beispiel 160

### (±)-7,8-Bis(difluormethoxy)-1-[4-(3,5-dimethylisoxazol-4-yl)phenyl]-N,4-dimethyl-4,5-dihydro-3H-2,3-benzodiazepin-3-carboxamid

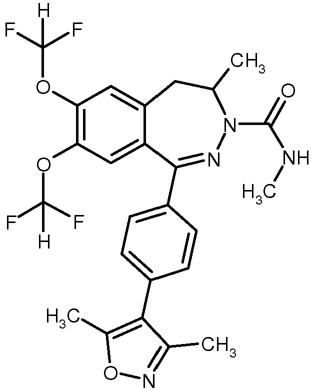

286 mg (7,17 mmol) (±)-1-(4-Bromphenyl)-7,8-bis(difluormethoxy)-*N*,4-dimethyl-4,5-dihydro-3*H-*2,3-benzodiazepin-3-carboxamid (Beispiel **92A)** wurden analog Beispiel 155 umgesetzt und das Rohprodukt mittels präparativer HPLC gereinigt. Man erhielt 22 mg (19% d. Th.) des gewünschten Produkts.
LCMS (Methode 2): Rₜ = 1,35 min; m/z = 521 (M+H)⁺
¹H-NMR (300MHz, DMSO-*d*₆): δ = 0,96 (d, 3H), 2,24 (s, 3H), 2,42 (s, 3H), 2,65 (d, 3H), 2,74 (dd, 1H), 3,02 (dd, 1H), 5,02 - 5,12 (m, 1H), 6,67 (q, 1H), 6,97 (s, 1H), 7,08 (t, J = 74 Hz, 1H), 7,27 (t, J = 74 Hz, 1H), 7,41 (s, 1H), 7,43 (d, 2H), 7,74 (d, 2H).

### Beispiel 161

### (±)-1-[4-(3,5-Dimethylisoxazol-4-yl)phenyl]-7,8-diethoxy-N,4-dimethyl-4,5-dihydro-3H-2,3-benzodiazepin-3-carboxamid

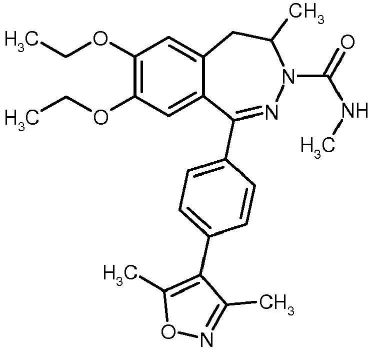

Die Herstellung erfolgte analog Beispiel **160** unter Verwendung von Beispiel **93A** als Ausgangsmaterial.
LCMS (Methode 1): Rₜ = 1,35 min; m/z = 477 (M+H)⁺
¹H-NMR (300MHz, DMSO-*d*₆): δ = 0,95 (d, 3H), 1,20 (t, 3H), 1,32 (t, 3H), 2,24 (s, 3H), 2,42 (s, 3H), 2,59 - 2,69 (m, 1H), 2,64 (d, 3H), 2,90 (dd, 1H), 3,79 (q, 2H), 4,08 (q, 2H), 4,97 - 5,09 (m, 1H), 6,51 (s, 1H), 6,59 (q, 1H), 6,99 (s, 1H), 7,41 (d, 2H), 7,72 (d, 2H).

Aus Beispiel **97A** und den entsprechenden kommerziell erhältlichen Boronsäuren wurden unter Verwendung des Verfahrens zur Herstellung von Beispiel **155** folgende Beispielverbindungen hergestellt:

| Nr | Struktur | Name | Analytische Daten |
|---|---|---|---|
| **162** | 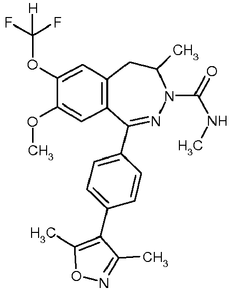 | (±)-7-(Difluormethoxy)-1-[4-(3,5-dimethylisoxazol-4-yl)phenyl]-8-methoxy-*N*,4-dimethyl-4,5-dihydro-3*H*-2,3-benzodiazepin-3-carboxamid | ¹H-NMR (300MHz, DMSO-*d*₆): δ = 0,96 (d, 3H), 2,24 (s, 3H), 2,42 (s, 3H), 2,58 -2,65 (m, 1H), 2,65 (d, 3H), 2,93 (dd, 1H), 3,64 (s, 3H), 4,94-5,05 (m, 1H), 6,60 (q, 1H), 6,73 (s, 1H), 7,14 (t, J=75 Hz, 1H), 7,23 (s, 1H), 7,42 (d, 2H), 7,76 (d, 2H). |
| | | | LCMS (Methode 2): Rₜ = 1,32 min; m/z = 485 (M+H)⁺ |
| **162.1** | 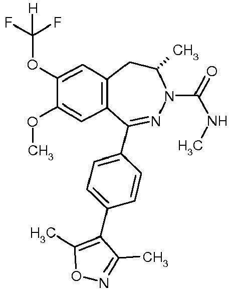 | (4*S*)-7-(Difluormethoxy)-1-[4-(3,5-dimethylisoxazol-4-yl)phenyl]-8-methoxy-*N*,4-dimethyl-4,5-dihydro-3*H*-2,3-benzodiazepin-3-carboxamid | Präp. HPLC: Methode XII |
| | | | Analyt. HPLC (Methode Q): Rₜ = 8,39 min |
| **162.2** | 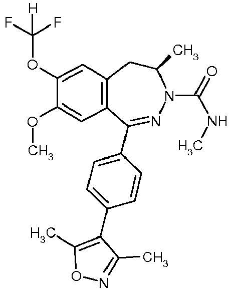 | (4*R*)-7-(Difluormethoxy)-1-[4-(3,5-dimethylisoxazol-4-yl)phenyl]-8-methoxy-*N*,4-dimethyl-4,5-dihydro-3*H*-2,3-benzodiazepin-3-carboxamid | Präp. HPLC: Methode XII |
| | | | Analyt. HPLC (Methode Q): Rₜ = 10,53 min |

Analog Beispiel **127.1** wurden aus Beispiel **97A** und den entsprechenden kommerziell erhältlichen Aminen folgende Beispielverbindungen hergestellt:

| Nr | Struktur | Name | Analytische Daten |
|---|---|---|---|
| **163** | 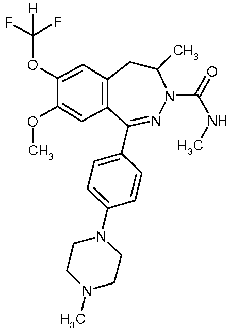 | (±)-7-(Difluormethoxy)-8-methoxy-*N*,4-dimethyl-1-[4-(4-methylpiperazin-1-yl)phenyl]-4,5-dihydro-3*H*-2,3-benzodiazepin-3-carboxamid | ¹H-NMR (400MHz, DMSO-*d*₆): δ = 1,02 (d, 3H), 2,20 (s, 3H), 2,37 -2,44 (m, 5H), 2,60 (d, 3H), 2,84 (dd, 1H), 3,19 - 3,25 (m, 4H), 3,65 (s, 3H), 4,77 - 4,86 (m, 1H), 6,30 (q, 1H), 6,70 (s, 1H), 6,93 (d, 2H), 7,13 (t, 1H), 7,21 (s, 1H), 7,58 (d, 2H). |
| | | | LCMS (Methode 2): Rₜ = 0,80 min; m/z = 488 (M+H)⁺ |
| **163.1** | 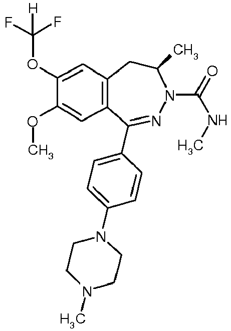 | (4*R*)-7-(Difluormethoxy)-8-methoxy-*N*,4-dimethyl-1-[4-(4-methylpiperazin-1-yl)phenyl]-4,5-dihydro-3*H*-2,3-benzodiazepin-3-carboxamid | Präp. HPLC: Methode XII |
| | | | Analyt. HPLC (Methode Q): Rₜ = 5,01 min |
| **163.2** | 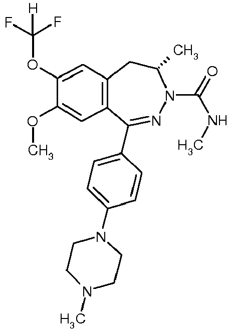 | (4*S*)-7-(Difluormethoxy)-8-methoxy-*N*,4-dimethyl-1-[4-(4-methylpiperazin-1-yl)phenyl]-4,5-dihydro-3*H*-2,3-benzodiazepin-3-carboxamid | Präp. HPLC: Methode XII |
| | | | Analyt. HPLC (Methode Q): Rₜ = 7,00 min |

Analog zu Beispiel **32** wurden folgende Beispielverbindungen mit den entsprechenden, kommerziell erhältlichen Heterocyclylchloralkanen bzw. Heteroarylchloralkanen hergestellt:

| Nr | Struktur | Name | Analytische Daten |
|---|---|---|---|
| **164** | 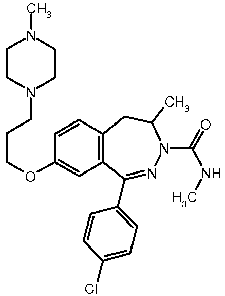 | (±)-1-(4-Chlorphenyl)-*N*,4-dimethyl-8-[3-(4-methylpiperazin-1-yl)propoxy]-4,5-dihydro-3*H*-2,3-benzodiazepin-3-carboxamid | ¹H-NMR (300MHz, CDCl₃): δ = 0,92 (d, 3H), 1,89 (m, 2H), 2,28 (s, 3H), 2,46 (m, 10H), 2,84 (dd, 1H), 2,88 (d, 3H), 3,08 (dd, 1H), 3,87 (m, 2H), 5,42 (m, 1H), 6,45 (qbr, 1H), 6,58 (d, 1H), 6,87 (dd, 1H), 7,11 (d, 1H), 7,36 (d, 2H), 7,42 (d, 2H). |
| | | | LC/MS (Methode 3): Rₜ = 0,89 min; m/z = 484; 486 (M+H, Cl-Isotopenmuster)⁺ |
| **164.1** | 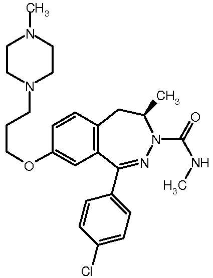 | (4*R*)-1-(4-Chlorphenyl)-*N*,4-dimethyl-8-[3-(4-methylpiperazin-1-yl)propoxy]-4,5-dihydro-3*H*-2,3-benzodiazepin-3-carboxamid (Vergleichsbeispiel) | Präp. HPLC: Methode XVII |
| | | | Analyt. HPLC (Methode K): Rₜ= 1,88 min |
| | | | [α]_{D}²⁰ = -124,2° (c = 1,00; MeOH) |
| **164.2** | 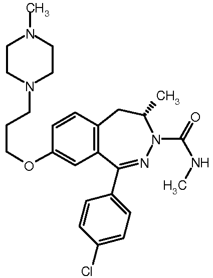 | (4*S*)-1-(4-Chlorphenyl)-*N*,4-dimethyl-8-[3-(4-methylpiperazin-1-yl)propoxy]-4,5-dihydro-3*H*-2,3-benzodiazepin-3-carboxamid | Präp. HPLC: Methode XVII |
| | | | Analyt. HPLC (Methode K): Rₜ = 2,41 min |
| | | | [α]_{D}²⁰ = +114,2° (c = 1,00; MeOH) |
| **165** | 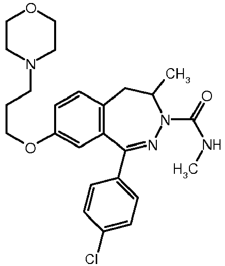 | (±)-1-(4-Chlorphenyl)-*N*,4-dimethyl-8-[3-(morpholin-4-yl)propoxy]-4,5-dihydro-3*H*-2,3-benzodiazepin-3-carboxamid | ¹H-NMR (300MHz, CDCl₃): δ = 0,92 (d, 3H), 1,93 (m, 2H), 2,49 (m, 4H), 2,51 (m, 2H), 2,84 (dd, 1H), 2,89 (d, 3H), 3,08 (dd, 1H), 3,73 (m, 4H), 3,88 (m, 2H), 5,42 (m, 1H), 6,45 (qbr, 1H), 6,58 (d, 1H), 6,87 (dd, 1H), 7,11 (d, 1H), 7,36 (d, 2H), 7,42 (d, 2H). |
| | | | LC/MS (Methode 3): Rₜ = 0,89 min; m/z = 471; 473 (M+H, Cl-Isotopenmuster)⁺ |
| **166** | 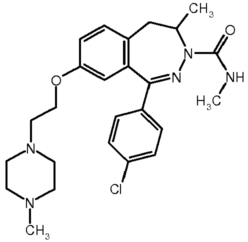 | (±)-1-(4-Chlorphenyl)-*N*,4-dimethyl-8-[2-(4-methylpiperazin-1-yl)ethoxy]-4,5-dihydro-3*H*-2,3-benzodiazepin-3-carboxamid | ¹H-NMR (300MHz, CDCl₃): δ = 0,92 (d, 3H), 2,42 (s, 3H), 2,69 (m, 8H), 2,78 (tbr, 2H), 2,85 (m, 1H), 2,88 (d, 3H), 3,08 (dd, 1H), 3,95 (tbr, 2H), 5,42 (m, 1H), 6,45 (qbr, 1H), 6,58 (d, 1H), 6,87 (dd, 1H), 7,12 (d, 1H), 7,36 (d, 2H), 7,41 (d, 2H). |
| | | | LC/MS (Methode 3): Rₜ = 0,94 min; m/z = 470; 472 (M+H, Cl-Isotopenmuster)⁺ |
| **167** | 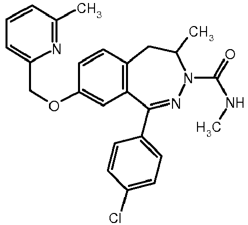 | (±)-1-(4-Chlorphenyl)-*N*,4-dimethyl-8-[(6-methylpyridin-2-yl)methoxy]-4,5-dihydro-3*H*-2,3-benzodiazepin-3-carboxamid | ¹H-NMR (300MHz, CDCl₃): δ = 0,92 (d, 3H), 2,53 (s, 3H), 2,85 (dd, 1H), 2,88 (d, 3H), 3,07 (dd, 1H), 4,99 (d, 1H), 5,04 (d, 1H), 5,43 (m, 1H), 6,47 (qbr, 1H), 6,64 (d, 1H), 6,98 (dd, 1H), 7,08 (d, 1H), 7,13 (d, 1H), 7,23 (d, 1H), 7,29 (d, 2H), 7,33 (d, 2H), 7,58 (dd, 1H). |
| | | | LC/MS (Methode 3): Rₜ = 1,31 min; m/z = 449; 451 (M+H, Cl- Isotopenmuster)⁺ |

### Beispiel 168

### (±)-1-[4-(3,5-Dimethylisoxazol-4-yl)phenyl]-8-hydroxy-N,4-dimethyl-4,5-dihydro-3H-2,3-benzodiazepin-3-carboxamid

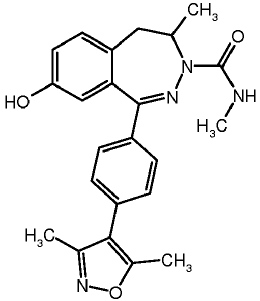

Ausgehend vom Racemat der in Beispiel **20** beschriebenen Verbindung wurde die Titelverbindung analog zu Beispiel **55A** hergestellt.
LCMS (Methode 3): Rₜ = 1,06 min; m/z = 405 (M+H)⁺
¹H-NMR (400MHz, DMSO-*d*₆): δ = 0,95 (d, 3H), 2,24 (s, 3H), 2,42 (s, 3H), 2,57 (dd, 1H), 2,65 (d, 3H), 2,88 (dd, 1H), 4,95 (m, 1H), 6,44 (d, 1H), 6,58 (qbr, 1H), 6,81 (dd, 1H), 7,18 (d, 1H), 7,43 (d, 2H), 7,73 (d, 2H), 9,63 (sbr, 1H).

### Beispiel 169

### (±)-1-[4-(3,5-Dimethylisoxazol-4-yl)phenyl]-N,4-dimethyl-8-[3-(morpholin-4-yl)propoxy]-4,5-dihydro-3H-2,3-benzodiazepin-3-carboxamid

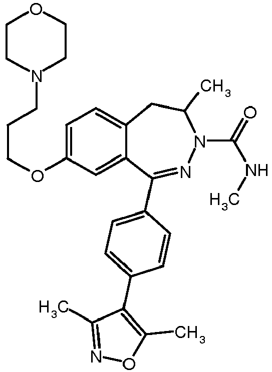

Die Herstellung erfolgte analog zu Beispiel **32.**
LCMS (Methode 3): Rₜ = 0,88 min; m/z = 532 (M+H)⁺
¹H-NMR (400MHz, CDCl₃): δ = 0,98 (d, 3H), 1,96 (m, 2H), 2,33 (s, 3H), 2,47 (s, 3H), 2,51 (m, 4H), 2,54 (m, 2H), 2,84 (dd, 1H), 2,90 (d, 3H), 3,08 (dd, 1H), 3,73 (m, 4H), 3,93 (m, 2H), 5,40 (m, 1H), 6,44 (qbr, 1H), 6,69 (d, 1H), 6,90 (dd, 1H), 7,14 (d, 1H), 7,29 (d, 2H), 7,58 (d, 2H).

### Beispiel 170

### (±)-7-Cyan-1-[4-(3,5-dimethylisoxazol-4-yl)phenyl]-8-methoxy-N,4-dimethyl-4,5-dihydro-3H-2,3-benzodiazepin-3-carboxamid

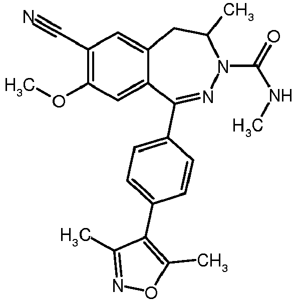

Die Herstellung erfolgte analog zu Beispiel **155.**
LCMS (Methode 3): Rₜ = 1,19 min; m/z = 444 (M+H)⁺
¹H-NMR (400MHz, CDCl₃): δ = 0,94 (d, 3H), 2,33 (s, 3H), 2,47 (s, 3H), 2,90 (dd, 1H), 2,92 (d, 3H), 3,11 (dd, 1H), 3,73 (s, 3H), 5,50 (m, 1H), 6,54 (qbr, 1H), 6,74 (s, 1H), 7,33 (d, 2H), 7,42 (s, 1H), 7,54 (d, 2H).

### Beispiel 171

### (±)-8-Acetamido-N,4-dimethyl-1-[4-(morpholin-4-yl)phenyl]-4,5-dihydro-3H-2,3-benzodiazepin-3-carboxamid

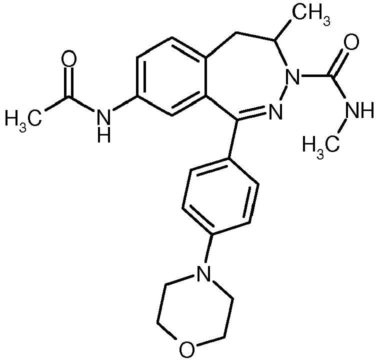

200 mg (520 µmol) (±)-8-Acetamido-1-(4-chlorphenyl)-*N*,4-dimethyl-4,5-dihydro-3*H*-2,3-benzodiazepin-3-carboxamid (Beispiel 89A) wurden unter Argon in 8 ml entgastem Toluol vorgelegt. Es wurden 47 µl (47 mg, 540 µmol) Morpholin, 70 mg (728 µmol) Natrium-*tert-*butanolat und 20 mg (26 µmol) Chlor-(2-dicyclohexylphosphino-2,4,6-triisopropyl-1,1-biphenyl)[2-(2-amino-1,1-biphenyl)] palladium(II) [CAS 1310584-14-5] zugegeben. Die Mischung wurde erneut entgast, mit Argon gesättigt und dann 5 Stunden bei 110°C gerührt. Es wurden weitere 25 mg Morpholin, 35 mg Natrium-*tert*.-butanolat und 10 mg Katalysator nachgegeben und weitere 7 h bei 110°C gerührt. Nach dem Abkühlen wurde die Mischung zwischen ges. Natriumhydrogencarbonatlösung und Ethylacetat verteilt und die Phasen getrennt. Die wässrige Phase wurde mit Ethylacetat extrahiert, die vereinigten organischen Phasen mit Wasser und ges. Natriumchloridlösung gewaschen und mit Natriumsulfat getrocknet. Die Lösemittel wurden am Rotationsverdampfer entfernt und der Rückstand mittels präparativer HPLC gereinigt. Man erhielt 9,8 mg (4,2% d. Th.) des gewünschten Produkts.
LCMS (Methode 2): Rₜ = 0,98 min; m/z = 436 (M+H)⁺
¹H-NMR (300MHz, DMSO-*d*₆): δ = 1,00 (d, 3H), 1,94 (s, 3H), 2,34 - 2,40 (m, 1H), 2,59 (d, 3H), 2,82 (dd, 1H), 3,14 - 3,22 (m, 4H), 3,66 - 3,77 (m, 4H), 4,76 - 4,89 (m, 1H), 6,31 (q, 1H), 6,94 (d, 2H), 7,25 (d, 1H), 7,31 (d, 1H), 7,52 - 7,60 (m, 3H), 9,90 (s, 1H).

### Beispiel 172

### (±)-8-Acetamido-N,4-dimethyl-1-[4-(4-methylpiperazin-1-yl)phenyl]-4,5-dihydro-3H-2,3-benzodiazepin-3-carboxamid

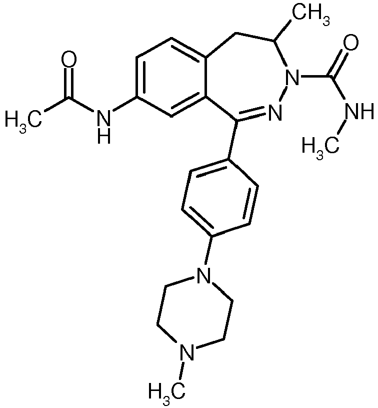

Die Verbindung wurde analog Beispiel **127.1** hergestellt.
LCMS (Methode 2): Rₜ = 0,65 min; m/z = 449 (M+H)⁺
¹H-NMR (300MHz, DMSO-*d*₆): δ = 1,01 (d, 3H), 1,94 (s, 3H), 2,19 (s, 3H), 2,34 - 2,53 (m, 5H), 2,59 (d, 3H), 2,82 (dd, 1H), 3,20 - 3,24 (m, 4H), 4,75 - 4,86 (m, 1H), 6,28 (q, 1H), 6,92 (d, 2H), 7,25 (d, 1H), 7,31 (d, 1H), 7,54 (d, 2H), 7,52 - 7,57 (m, 1H), 9,90 (s, 1H).

### Beispiel 173

### (±)-8-Acetamido-1-[4-(3,5-dimethylisoxazol-4-yl)phenyl]-N,4-dimethyl-4,5-dihydro-3H-2,3-benzodiazepin-3-carboxamid

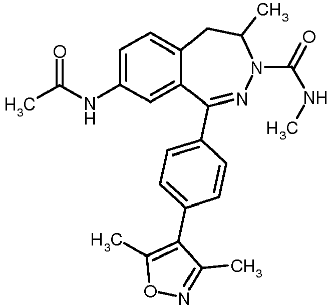

200 mg (520 µmol) (±)-8-Acetamido-1-(4-chlorphenyl)-*N*,4-dimethyl-4,5-dihydro-3*H*-2,3-benzodiazepin-3-carboxamid wurden in 8 ml entgastem THF/Wasser 10:1 gelöst und 76 mg (540 µmol) 3,5-Dimethylisoxazol-4-boronsäure, 150 mg (260 µmol) Kaliumfluorid und 20 mg (26 µmol) Chlor-(2-dicyclohexylphosphino-2,4,6-triisopropyl-1,1-biphenyl)[2-(2-amino-1,1-biphenyl)]palladium(II) zugegeben. Die Reaktionsmischung wurde erneut entgast und unter Argonatmosphäre bei 80°C 7 Stunden gerührt. Dann wurde die Mischung zwischen ges. Natriumhydrogencarbonatlösung und Ethylacetat verteilt und die Phasen getrennt. Die wässrige Phase wurde mit Ethylacetat extrahiert, die vereinigten organischen Phasen mit Wasser und ges. Natriumchloridlösung gewaschen und mit Natriumsulfat getrocknet. Die Lösemittel wurden am Rotationsverdampfer entfernt und der Rückstand mittels präparativer HPLC gereinigt. Man erhielt 24,4 mg (11% d. Th.) des gewünschten Produkts.
LCMS (Methode 2): Rₜ = 1,09 min; m/z = 446 (M+H)⁺
¹H-NMR (300MHz, DMSO-*d*₆): δ = 0,94 (d, 3H), 1,93 (s, 3H), 2,24 (s, 3H), 2,42 (s, 3H), 2,59-2,68 (m, 1H), 2,64 (d, 3H), 2,92 (dd, 1H), 4,93 - 5,05 (m, 1H), 6,58 (q, 1H), 7,28 (d, 1H), 7,29 (s, 1H), 7,43 (d, 1H), 7,64 (dd, 1H), 7,74 (d, 2H), 9,92 (s, 1H).

### Beispiel 174

### (±)-1-(4-Chlorphenyl)-8-(3,5-dimethyl-1H-pyrazol-1-yl)-N,4-dimethyl-4,5-dihydro-3H-2,3-benzodiazepin-3-carboxamid

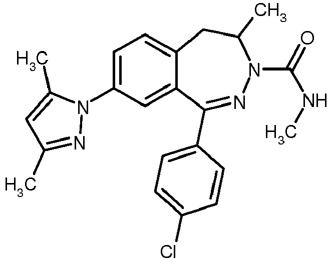

Die Verbindung wurde analog Beispiel 16 mit einer Ausbeute von 87% hergestellt.
LCMS (Methode 1): Rₜ = 1,38 min; m/z = 422 (M+H)⁺
¹H-NMR (300MHz, DMSO-*d*₆): δ = 0,91 (d, 3H), 2,07 (s, 3H), 2,17 (s, 3H), 2,65 (d, 3H), 2,83 (dd, 1H), 3,05 (dd, 1H), 5,10 - 5,18 (m, 1H), 5,98 (s, 1H), 6,71 (q, 1H), 7,02 (d, 1H), 7,45 (d, 2H), 7,43 - 7,53 (m, 2H), 7,64 (d, 2H).

### Beispiel 175

### (±)-1-[4-(3,5-Dimethylisoxazol-4-yl)phenyl]-8-(3,5-dimethyl-1H-pyrazol-1-yl)-N,4-dimethyl-4,5-dihydro-3H-2,3-benzodiazepin-3-carboxamid

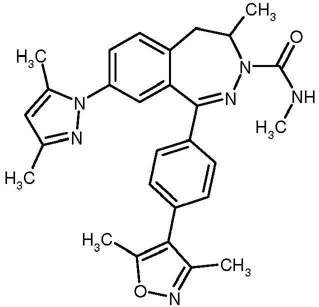

Die Verbindung wurde analog Beispiel 155 mit einer Ausbeute von 24% hergestellt.
LCMS (Methode 1): Rₜ = 1,31 min; m/z = 483 (M+H)⁺
¹H-NMR (300MHz, DMSO-*d*₆): δ = 0,95 (d, 3H), 2,07 (s, 3H), 2,19 (s, 3H), 2,23 (s, 3H), 2,41 (s, 3H), 2,65 (d, 3H), 2,80 (dd, 1H), 3,05 (dd, 1H), 5,05 - 5,15 (m, 1H), 5,99 (s, 1H), 6,69 (q, 1H), 7,08 (d, 1H), 7,43 (d, 2H), 7,44 - 7,55 (m, 2H), 7,75 (d, 2H).

### Beispiel 176

### (±)-1-[4-(3,5-Dimethylisoxazol-4-yl)phenyl]-N,4-dimethyl-8-(morpholin-4-yl)-4,5-dihydro-3H-2,3-benzodiazepin-3-carboxamid

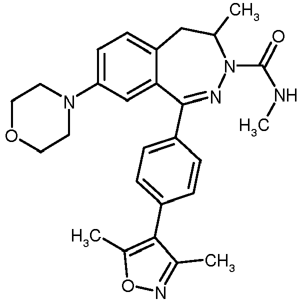

Die Verbindung wurde analog Beispiel 155 aus (±)-1-(4-Chlorphenyl)-*N*,4-dimethyl-8-(morpholin-4-yl)-4,5-dihydro-3*H*-2,3-benzodiazepin-3-carboxamid (Beispiel 11) hergestellt.
LCMS (Methode 2): Rₜ = 1,24 min; m/z = 474 (M+H)⁺
¹H-NMR (400MHz, DMSO-*d*₆): δ = 0,96 (d, 3H), 2,24 (s, 3H), 2,42 (s, 3H), 2,56 (dd, 1H), 2,64 (d, 3H), 2,87 (dd, 1H), 2,92 - 2,98 (m, 4H), 3,61 - 3,67 (m, 4H), 4,89 - 4,97 (m, 1H), 6,52 - 6,56 (m, 2H), 7,02 (dd, 1H), 7,23 (d, 1H), 7,42 (d, 2H), 7,76 (d, 2H).

### Enantiomerentrennung

90 mg (±)-1-[4-(3,5-Dimethylisoxazol-4-yl)phenyl]-*N*,4-dimethyl-8-(morpholin-4-yl)-4,5-dihydro-3*H*-2,3-benzodiazepin-3-carboxamid (Beispiel **185)** wurden mittels chiraler präparativer HPLC mit der Methode XIII in die Enantiomere getrennt:

### Beispiel 176.1: (4R)-1-[4-(3,5-Dimethylisoxazol-4-yl)phenyl]-N,4-dimethyl-8-(morpholin-4-yl)-4,5-dihydro-3H-2,3-benzodiazepin-3-carboxamid

27 mg Feststoff, HPLC (Methode R): Rₜ = 2,58 min, Reinheit 98.2% / 100% ee

### Beispiel 176.2: (4S)-1-[4-(3,5-Dimethylisoxazol-4-yl)phenyl]-N,4-dimethyl-8-(morpholin-4-yl)-4,5-dihydro-3H-2,3-benzodiazepin-3-carboxamid

23 mg Feststoff, HPLC (Methode R): Rₜ = 3,06 min, Reinheit 96,3% / 92,7% ee

### Beispiel 177

### (4S)-8-Methoxy-N,4-dimethyl-1-[4-(3-oxomorpholin-4-yl)phenyl]-4,5-dihydro-3H-2,3-benzodiazepin-3-carboxamid

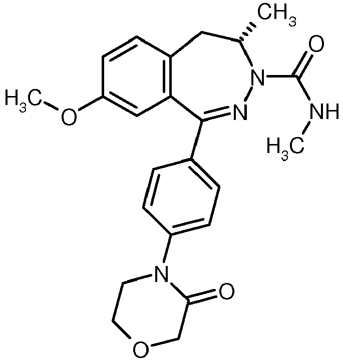

112 mg (0,244 mmol) (4*S*)-1-(4-{[(2-Chlorethoxy)acetyl]amino}phenyl)-8-methoxy-*N*,4-dimethyl-4,5-dihydro-3*H*-2,3-benzodiazepin-3-carboxamid (Beispiel **94A**) wurden in 1.1 ml Acetonitril gelöst und mit 71 mg (0,512 mmol) Kaliumcarbonat versetzt. Nach Zugabe einer katalytischen Menge Natriumiodids erhitzte man für 5h am Rückfluss. Der Ansatz wurde filtriert, mit Acetonitril nachgewaschen und das Lösemittel am Rotationsverdampfer entfernt. Der Rückstand wurde mittels präparativer HPLC gereinigt. Man erhielt 16 mg (15% d. Th.) des gewünschten Produkts als Feststoff.
UPLC/MS (Methode 1): Rt = 1.0 min; m/z = 423 (M+H)⁺

Analog Beispiel **127.1** wurden aus Beispiel **49A** bzw. Beispiel **49.2A** und den entsprechenden kommerziell erhältlichen Aminen folgende Beispielverbindungen hergestellt:

| Nr | Struktur | Name | Analytische Daten |
|---|---|---|---|
| **178** | 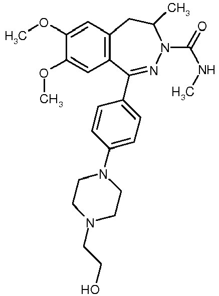 | (±)-1-{4-[4-(2-Hydroxyethyl)piperazin-1-yl]phenyl}-7,8-dimethoxy-N,4-dimethyl-4,5-dihydro-3*H*-2,3-benzodiazepin-3-carboxamid | ¹H-NMR (500MHz, CDCl₃): δ = 1,11 (d, 3H), 2,61 - 2,64 (m, 2H), 2,69-2,71 (m, 4H), 2,73 (dd, 1H), 2,87 (d, 3H), 2, 94 (dd, 1H), 3,30 - 3,32 (m, 4H), 3,65 - 3,69 (m, 2H), 3,70 (s, 3H), 3,93 (s, 3H), 5,24 - 5,33 (m, 1H), 6,12 (q, 1H), 6,64 (s, 1H), 6,75 (s, 1H), 6,91 (d, 2H), 7,49 (d, 2H). LCMS (Methode 1): Rₜ = 0,72 min; m/z = 482 (M+H)⁺ |
| **179** | 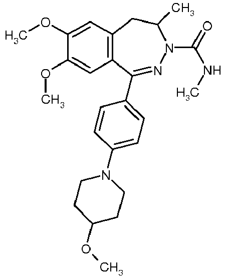 | (±)-7,8-Dimethoxy-1-[4-(4-methoxypiperidin-1-yl)phenyl]-N,4-dimethyl-4,5-dihydro-3*H*-2,3-benzodiazepin-3-carboxamid | ¹H-NMR (500MHz, CDCl₃): δ = 1,12 (d, 3H), 1,68 - 1,75 (m, 2H), 1,99-2,05 (m, 2H), 2, 72 (dd, 1H), 2,86 (d, 3H), 2,92 (dd, 1H), 3,04 - 3,09 (m, 2H), 3,39 (s, 3H), 3,60 - 3,64 (m, 2H), 3,70 (s, 3H), 3,93 (s, 3H), 5,24-5,30 (m, 1H), 6,07 (q, 1H), 6,64 (s, 1H), 6,75 (s, 1H), 6,91 (d, 2H), 7,48 (d, 2H). |
| | | | LCMS (Methode 1): Rₜ = 1,13 min; m/z = 467 (M+H)⁺ |
| **179.1** | 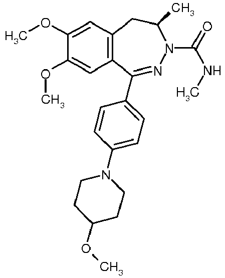 | (4*R*)-7,8-Dimethoxy-1-[4-(4-methoxypiperidin-1-yl)phenyl]-N,4-dimethyl-4,5-dihydro-3*H*-2,3-benzodiazepin-3-carboxamid | Präp. HPLC: Methode XX |
| | | | Analyt. HPLC (Methode L): Rₜ = 7,77 min |
| **179.2** | 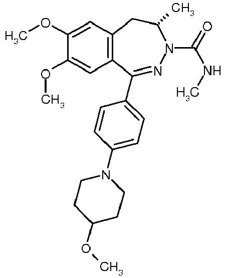 | (4*S*)-7,8-Dimethoxy-1-[4-(4-methoxypiperidin-1-yl)phenyl]-N,4-dimethyl-4,5-dihydro-3*H*-2,3-benzodiazepin-3-carboxamid | Präp. HPLC: Methode XX |
| | | | Analyt. HPLC (Methode L): Rₜ = 12,0 min |
| **180** | 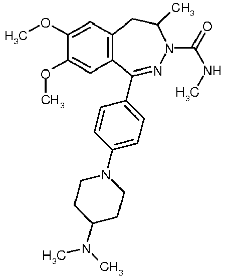 | (±)-1-{4-[4-(Dimethylamino)piperidi n-1-yl]phenyl}-7,8-dimethoxy-N,4-dimethyl-4,5-dihydro-3*H*-2,3-benzodiazepin-3-carboxamid | ¹H-NMR (500MHz, CDCl₃): δ = 1,05 (d, 3H), 1,52 - 1,62 (m, 3H), 1,85-1,91 (m, 2H), 2,25 (s, 6H), 2, 65 (dd, 1H), 2,72 - 2,78 (m, 2H), 2,79 (d, 3H), 2,85 (dd, 1H), 3,63 (s, 3H), 3,76 - 3,82 (m, 2H), 3,86 (s, 3H), 5,17 - 5,23 (m, 1H), 6,00 (q, 1H), 6,58 (s, 1H), 6,68 (s, 1H), 6,84 (d, 2H), 7,41 (d, 2H). |
| | | | LCMS (Methode 1): Rₜ = 0,76 min; m/z = 480 (M+H)⁺ |
| **180.1** | 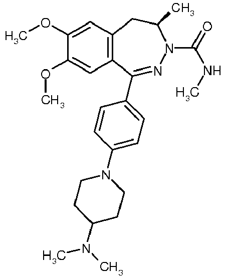 | (4*R*)-1-{4-[4-(Dimethylamino)piperidi n-1-yl]phenyl}-7,8-dimethoxy-N,4-dimethyl-4,5-dihydro-3*H*-2,3-benzodiazepin-3-carboxamid | Präp. HPLC: Methode XX |
| | | | Analyt. HPLC (Methode L): Rₜ = 9,20 min |
| **180.2** | 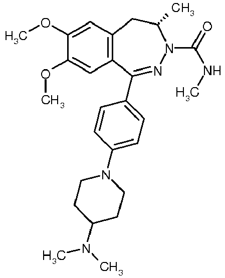 | (4*S*)-1-{4-[4-(Dimethylamino)piperidi n-1-yl]phenyl}-7,8-dimethoxy-N,4-dimethyl-4,5-dihydro-3*H*-2,3-benzodiazepin-3-carboxamid | Präp. HPLC: Methode XX |
| | | | Analyt. HPLC (Methode L): Rₜ = 14,3 min |
| **181** | 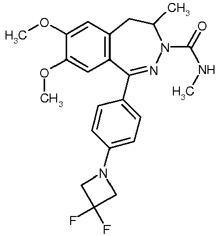 | (±)-1-[4-(3,3-Difluorazetidin-1-yl)phenyl]-7,8-dimethoxy-N,4-dimethyl-4,5-dihydro-3*H*-2,3-benzodiazepin-3-carboxamid | ¹H-NMR (500MHz, CDCl₃): δ = 1,10 (d, 3H), 2,74 (dd, 1H), 2,87 (d, 3H), 2, 96 (dd, 1H), 3,70 (s, 3H), 3,94 (s, 3H), 4,29 (t, 4H), 5,27 - 5,34 (m, 1H), 6,14 (q, 1H), 6,50 (d, 2H), 6,61 (s, 1H), 6,75 (s, 1H), 7,49 (d, 2H). |
| | | | LCMS (Methode 1): Rₜ = 1,21 min; m/z = 445 (M+H)⁺ |
| **182** | 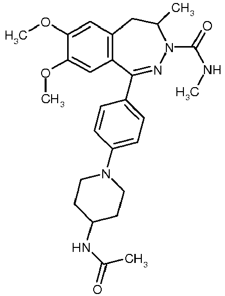 | (±)-1-[4-(4-Acetamidopiperidin-1-yl)phenyl]-7,8-dimethoxy-N,4-dimethyl-4,5-dihydro-3*H*-2,3-benzodiazepin-3-carboxamid | ¹H-NMR (500MHz, CDCl₃): δ = 1,11 (d, 3H), 1,50 - 1,58 (m, 2H), 1,99 (s, 3H), 2,04 -2,10 (m, 2H), 2,72 (dd, 1H), 2,86 (d, 3H), 2,92 - 2,98 (m, 3H), 3,70 (s, 3H), 3,73 - 3,80 (m, 2H), 3,93 (s, 3H), 3,95 - 4,03 (m, 1H), 5,25 - 5,32 (m, 1H), 5,34 (d, 1H), 6,10 (q, 1H), 6,64 (s, 1H), 6,75 (s, 1H), 6,91 (d, 2H), 7,48 (d, 2H). |
| | | | LCMS (Methode 1): Rₜ = 0,94 min; m/z = 494 (M+H)⁺ |
| **183** | 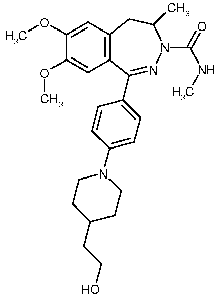 | (±)-1-{4-[4-(2-Hydroxyethyl)piperidin-1-yl]phenyl}-7,8-dimethoxy-N,4-dimethyl-4,5-dihydro-3H-2,3-benzodiazepin-3-carboxamid | ¹H-NMR (500MHz, CDCl₃): δ = 1,13 (d, 3H), 1,35 - 1,43 (m, 2H), 1,56-1,60 (m, 2H), 1,63 - 1,71 (m, 1H), 1,81 - 1,87 (m, 2H), 2,71 (dd, 1H), 2,78 - 2,84 (m, 2H), 2,86 (d, 3H), 2, 91 (dd, 1H), 3,70 (s, 3H), 3,74 - 3,77 (m, 2H), 3,78 - 3,83 (m, 2H), 3,93 (s, 3H), 5,23 - 5,29 (m, 1H), 6,05 (q, 1H), 6,65 (s, 1H), 6,75 (s, 1H), 6,92 (d, 2H), 7,48 (d, 2H). |
| | | | LCMS (Methode 1): Rₜ = 0,96 min; m/z = 481 (M+H)⁺ |
| **184** | 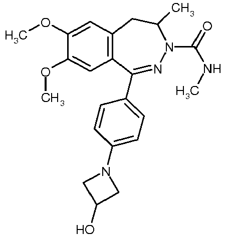 | (±)-1-[4-(3-Hydroxyazetidin-1-yl)phenyl]-7,8-dimethoxy-N,4-dimethyl-4,5-dihydro-3H-2,3-benzodiazepin-3-carboxamid | ¹H-NMR (400MHz, CDCl₃): δ = 1,16 (d, 3H), 2.21 (d, 1H), 2,73 (dd, 1H), 2,88 (d, 3H), 2, 92 (dd, 1H), 3,73 (s, 3H), 3,80 (dd, 2H), 3,96 (s, 3H), 4,27 (t, 2H), 4,80 - 4,87 (m, 1H), 5,22-5,31 (m, 1H), 6,02 (q, 1H), 6,47 (d, 2H), 6,65 (s, 1H), 6,76 (s, 1H), 7,50 (d, 2H). |
| | | | LCMS (Methode 1): Rₜ = 0,91 min; m/z = 425 (M+H)⁺ |
| **185** | 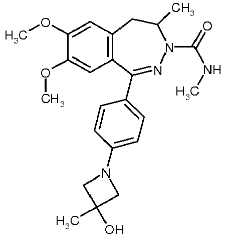 | (±)-1-[4-(3-Hydroxy-3-methylazetidin-1-yl)phenyl]-7,8-dimethoxy-N,4-dimethyl-4,5-dihydro-3H-2,3-benzodiazepin-3-carboxamid | ¹H-NMR (400MHz, CDCl₃): δ = 1,16 (d, 3H), 1,66 (s, 3H), 2.10 (s,br, 1H), 2,73 (dd, 1H), 2,88 (d, 3H), 2, 92 (dd, 1H), 3,73 (s, 3H), 3,86 (d, 2H), 3,95 (d, 2H), 3,96 (s, 3H), 5,22 - 5,30 (m, 1H), 6,02 (q, 1H), 6,48 (d, 2H), 6,65 (s, 1H), 6,76 (s, 1H), 7,50 (d, 2H). |
| | | | LCMS (Methode 1): Rₜ = 0,97 min; m/z = 439 (M+H)⁺ |
| **185.1** | 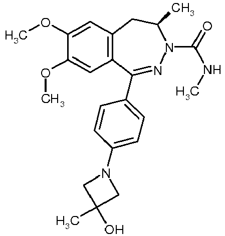 | (4*R*)-1-[4-(3-Hydroxy-3-methylazetidin-1-yl)phenyl]-7,8-dimethoxy-N,4-dimethyl-4,5-dihydro-3H-2,3-benzodiazepin-3-carboxamid | Präp. HPLC: Methode XXI |
| | | | Analyt. HPLC (Methode Y): Rₜ = 2,35 min |
| **185.2** | 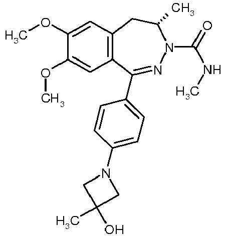 | (4*S*)-1-[4-(3-Hydroxy-3-methylazetidin-1-yl)phenyl]-7,8-dimethoxy-N,4-dimethyl-4,5-dihydro-3H-2,3-benzodiazepin-3-carboxamid | Präp. HPLC: Methode XXI |
| | | | Analyt. HPLC (Methode Y): Rₜ = 3,13 min |
| **186** | 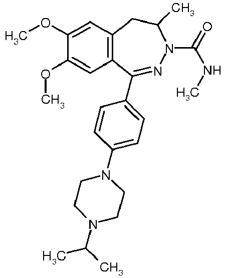 | (±)-1-[4-(4-Isopropylpiperazin-1-yl)phenyl]-7,8-dimethoxy-N,4-dimethyl-4,5-dihydro-3H-2,3-benzodiazepin-3-carboxamid | ¹H-NMR (400MHz, CDCl₃): δ = 1,12 - 1,14 (m, 9H), 2,71 - 2,79 (m, 6H), 2,89 (d, 3H), 2,96 (dd, 1H), 3,32-3,35 (m, 4H), 3,72 (s, 3H), 3,96 (s, 3H), 5,26 - 5,34 (m, 1H), 6,11 (q, 1H), 6,67 (s, 1H), 6,77 (s, 1H), 6,94 (d, 2H), 7,52 (d, 2H). |
| | | | LCMS (Methode 1): Rₜ = 0,76 min; m/z = 480 (M+H)⁺ |
| **187** | 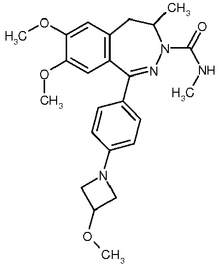 | (±)-7,8-Dimethoxy-1-[4-(3-methoxyazetidin-1-yl)phenyl]-N,4-dimethyl-4,5-dihydro-3H-2,3-benzodiazepin-3-carboxamid | ¹H-NMR (300MHz, CDCl₃): δ = 1,16 (d, 3H), 2,73 (dd, 1H), 2,88 (d, 3H), 2, 93 (dd, 1H), 3,38 (s, 3H), 3,73 (s, 3H), 3,80 - 3,84 (m, 2H), 3,96 (s, 3H), 4.17 - 4,22 (m, 2H), 4.37 - 4,43, 5,22 - 5,32 (m, 1H), 6,02 (q, 1H), 6,47 (d, 2H), 6,65 (s, 1H), 6,78 (s, 1H), 7,50 (d, 2H). |
| | | | LCMS (Methode 1): Rₜ = 1,11 min; m/z = 439 (M+H)⁺ |
| **188** | 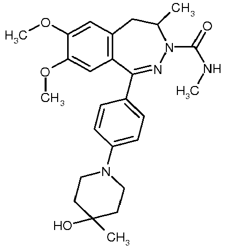 | (±)-1-[4-(4-Hydroxy-4-methylpiperidin-1-yl)phenyl]-7,8-dimethoxy-N,4-dimethyl-4,5-dihydro-3H-2,3-benzodiazepin-3-carboxamid | ¹H-NMR (400MHz, CDCl₃): δ = 1,15 (d, 3H), 1,34 (s, 3H), 1,71 - 1,84 (m, 4H), 2,74 (dd, 1H), 2,89 (d, 3H), 2, 95 (dd, 1H), 3,29 - 3,35 (m, 2H), 3,49 - 3,54 (m, 2H), 3,73 (s, 3H), 3,96 (s, 3H), 5,24 - 5,33 (m, 1H), 6,08 (q, 1H), 6,68 (s, 1H), 6,78 (s, 1H), 6,95 (d, 2H), 7,51 (d, 2H). |
| | | | LCMS (Methode 1): Rₜ = 0,96 min; m/z = 467 (M+H)⁺ |
| **188.1** | 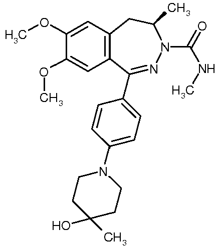 | (4*R*)-1-[4-(4-Hydroxy-4-methylpiperidin-1-yl)phenyl]-7,8-dimethoxy-N,4-dimethyl-4,5-dihydro-3H-2,3-benzodiazepin-3-carboxamid | Präp. HPLC: Methode XX |
| | | | Analyt. HPLC (Methode L): Rₜ = 5,45 min |
| **188.2** | 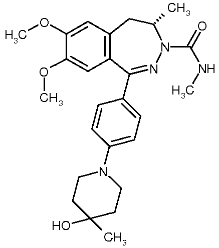 | (4*S*)-1-[4-(4-Hydroxy-4-methylpiperidin-1-yl)phenyl]-7,8-dimethoxy-N,4-dimethyl-4,5-dihydro-3H-2,3-benzodiazepin-3-carboxamid | Präp. HPLC: Methode XX |
| | | | Analyt. HPLC (Methode L): Rₜ = 8,05 min |
| **189** | 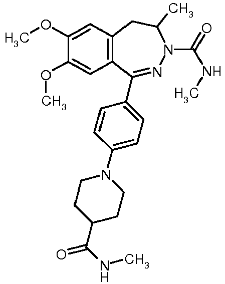 | (±)-7,8-Dimethoxy-N,4-dimethyl-1-{4-[4-(methylcarbamoyl)piperi din-1-yl]phenyl}-4,5-dihydro-3H-2,3-benzodiazepin-3-carboxamid | ¹H-NMR (300MHz, CDCl₃): δ = 1.14 (d, 3H), 1.82-2.06 (m, 4H), 2.24-2.38 (m, 1H), 2.72 (dd, 1H), 2.81-3.02 (m, 9H), 3.73 (s, 3H), 3.89 (d, 2H), 3.96 (s, 3H), 5.29 (m, 1H), 5.55 (m, 1H), 6.10 (m, 1H), 6.67 (s, 1H), 6.77 (s, 1H), 6.93 (d, 2H), 7.51 (d, 2H). |
| | | | LCMS (Methode 1): Rₜ = 0.93 min; m/z = 494 (M+H)⁺ |
| **189.1** | 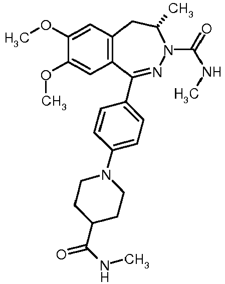 | (4*S*)-7,8-Dimethoxy-N,4-dimethyl-1-{4-[4-(methylcarbamoyl)piperi din-1-yl]phenyl}-4,5-dihydro-3H-2,3-benzodiazepin-3-carboxamid | Präp. HPLC: Methode XIX |
| | | | Analyt. HPLC (Methode V): Rₜ = 7.35 min |
| | | | [α]_{D}²⁰ = 292.7° (c = 1.00; MeOH) |
| **189.2** | 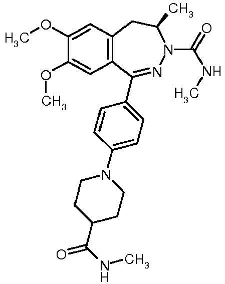 | (*4R*)-7,8-Dimethoxy-N,4-dimethyl-1-{4-[4-(methylcarbamoyl)piperi din-1-yl]phenyl}-4,5-dihydro-3H-2,3-benzodiazepin-3-carboxamid | Präp. HPLC: Methode XIX |
| | | | Analyt. HPLC (Methode V): Rₜ = 6.23 min |
| | | | [α]_{D}²⁰ = -311.5° (c = 1.00; MeOH) |
| **190** | 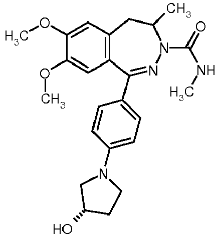 | (±)-1-{4-[(3S)-3-Hydroxypyrrolidin-1-yl]phenyl}-7,8-dimethoxy-N,4-dimethyl-4,5-dihydro-3H-2,3-benzodiazepin-3-carboxamid | LCMS (Methode 2): Rₜ = 0.80 min; m/z = 439 (M+H)⁺ |
| **190.1** | 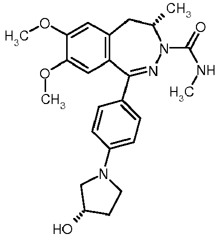 | (4S)-1-{4-[(3S)-3-Hydroxypyrrolidin-1-yl]phenyl}-7,8-dimethoxy-N,4-dimethyl-4,5-dihydro-3H-2,3-benzodiazepin-3-carboxamid | ¹H-NMR (300MHz, CDCl₃): δ = 1.20 (d, 3H), 1.74 (s, br, 1H), 2.07-2.30 (m, 2H), 2.71 (dd, 1H), 2.88 (d, 3H), 2.90 (dd, 1H), 3.37 (d, 1H), 3.47 (dt, 1H), 3.57-3.65 (m, 2H), 3.74 (s, 3H), 3.96 (s, 3H), 4.68 (m, 1H), 5.17-5.30 (m, 1H), 5.94 (q, 1H), 6.59 (d, 2H), 6.68 (s, 1H), 6.79 (s, 1H), 7.54 (d, 2H). |
| | | | LCMS (Methode 1): Rₜ = 0.92 min; m/z = 439 (M+H)⁺ |
| **191** | 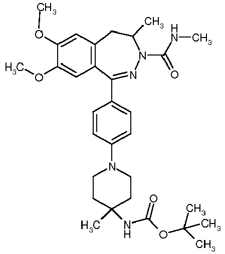 | (±)-tert-Butyl-(1-{4-[7,8-dimethoxy-4-methyl-3-(methylcarbamoyl)-4,5-dihydro-3H-2,3-benzodiazepin-1-yl]phenyl}-4-methylpiperidin-4-yl)carbamat | ¹H-NMR (300MHz, CDCl₃): δ = 1.14 (d, 3H), 1.43 (s, 3H), 1.47 (s, 9H), 1.77 (dt, 2H), 2.16 (d, br, 2H), 2.74 (dd, 1H), 2.88 (d, 3H), 2.95 (dd, 1H), 3.16 (dt, 2H), 3.43-3.54 (m, 2H), 3.73 (s, 3H), 3.96 (s, 3H), 4.41 (s, br, 1H), 5.23-5.37 (m, 1H), 6.10 (q, 1H), 6.67 (s, 1H), 6.77 (s, 1H), 6.94 (d, 2H), 7.51 (d, 2H). |
| | | | LCMS (Methode 2): Rₜ = 1.29 min; m/z = 566 (M+H)⁺ |
| **192** | 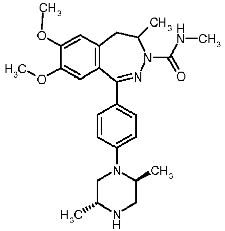 | (±)-1-{4-[(2S,SR)-2,5-Dimethylpiperazin-1-yl]phenyl}-7,8-dimethoxy-N,4-dimethyl-4,5-dihydro-3H-2,3-benzodiazepin-3-carboxamid | ¹H-NMR (300MHz, CDCl₃): δ = 1.05 (d, 3H), 1.10 (dd, 3H), 1.15 (d, 3H), 2.65 (dd, 1H), 2.73-2.84 (m, 2H), 2.90 (d, 3H), 3.02 (dt, 1H), 3.10-3.29 (m, 4H), 3.71 (s, 3H), 3.96 (s, 3H), 5.30-5.42 (m, 1H), 6.21-6.31 (m, 1H), 6.65 (s, 1H), 6.76 (s, 1H), 7.09 (d, 2H), 7.51 (d, 2H). |
| | | | LCMS (Methode 2): Rₜ = 0.64 min; m/z = 466 (M+H)⁺ |
| **193** | 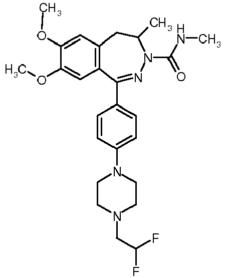 | (±)-1-{4-[4-(2,2-Difluorethyl)piperazin-1-yl]phenyl}-7,8-dimethoxy-N,4-dimethyl-4,5-dihydro-3H-2,3-benzodiazepin-3-carboxamid | ¹H-NMR (600MHz, DMSO-*d*₆): δ = 1.05 (d, 3H), 2.44-2.49 (m, 1H), 2.63 (d, 3H), 2.66-2.69 (m, 4H), 2.76-2.87 (m, 3H), 3.23-3.28 (m, 4H), 3.60 (s, 3H), 3.84 (s, 3H), 4.90 (dquin, 1H), 6.07-6.33 (m, 2H), 6.55 (s, 1H), 6.95 (d, 2H), 7.02 (s, 1H), 7.59 (d, 2H). |
| | | | LCMS (Methode 2): Rₜ = 0.90 min; m/z = 502 (M+H)⁺ |
| **194** | 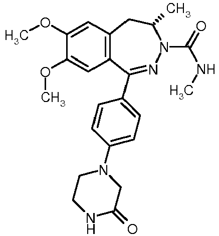 | (4S)-7,8-Dimethoxy-N,4-dimethyl-1-[4-(3-oxopiperazin-1-yl)phenyl]-4,5-dihydro-3H-2,3-benzodiazepin-3-carboxamid | ¹H-NMR (300MHz, CDCl₃): δ = 1.00 (d, 3H), 2.59-2.69 (m, 5H), 2.92 (dd, 1H), 3.03 (t, 2H), 3.41 (s, 2H), 3.59 (s, 3H), 3.63-3.68 (m, 2H), 3.84 (s, 3H), 4.97-5.10 (m, 1H), 6.50-6.56 (m, 2H), 7.03 (s, 1H), 7.40 (d, 2H), 7.70 (d, 2H). |
| | | | LCMS (Methode 1): Rₜ = 0.69 min; m/z = 452 (M+H)⁺ |
| **195** | 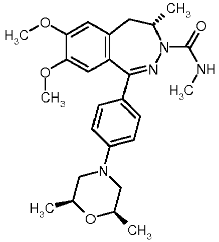 | (4S)-1-{4-[(2R,6S)-2,6-Dimethylmorpholin-4-yl]phenyl}-7,8-dimethoxy-N,4-dimethyl-4,5-dihydro-3H-2,3-benzodiazepin-3-carboxamid | ¹H-NMR (300MHz, CDCl₃): δ = 1.13 (d, 3H), 1.31 (d, 6H), 2.52 (dd, 2H), 2.75 (dd, 1H), 2.89 (d, 3H), 2.98 (dd, 1H), 3.58 (d, 2H), 3.72 (s, 3H), 3.78-3.90 (m, 2H), 3.96 (s, 3H), 5.25-5.39 (m, 1H), 6.15 (q, 1H), 6.66 (s, 1H), 6.77 (s, 1H), 6.92 (d, 2H), 7.52 (d, 2H). |
| | | | LCMS (Methode 1): Rₜ = 1.23 min; m/z = 467 (M+H)⁺ |
| **196** | 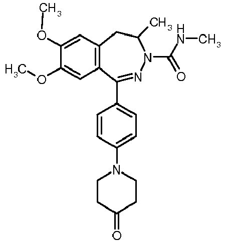 | (±)-7,8-Dimethoxy-N,4-dimethyl-1-[4-(4-oxopiperidin-1-yl)phenyl]-4,5-dihydro-3H-2,3-benzodiazepin-3-carboxamid | ¹H-NMR (300MHz, CDCl₃): δ = 1.13 (d, 3H), 2.62 (t, 4H), 2.76 (dd, 1H), 2.89 (d, 3H), 2.98 (dd, 1H), 3.73 (s, 3H), 3.71-3.79 (m, 4H), 3.97 (s, 3H), 5.27-5.39 (m, 1H), 6.12-6.20 (m, 1H), 6.68 (s, 1H), 6.78 (s, 1H), 6.99 (d, 2H), 7.56 (d, 2H). |
| | | | LCMS (Methode 5): Rₜ = 0.92 min; m/z = 451 (M+H)⁺ |
| **197** | 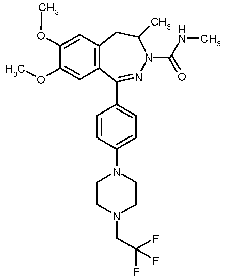 | (±)-7,8-Dimethoxy-N,4-dimethyl-1-{4-[4-(2,2,2-trifluorethyl)piperazin-1-yl]phenyl}-4,5-dihydro-3H-2,3-benzodiazepin-3-carboxamid | ¹H-NMR (400MHz, CDCl₃): δ = 1.12 (d, 3H), 2.75 (dd, 1H), 2.85-2.92 (m, 4H), 2.88 (d, 3H), 2.97 (dd, 1H), 3.08 (q, 2H), 3.31-3.39 (m, 4H), 3.72 (s, 3H), 3.96 (s, 3H), 5.27-5.37 (m, 1H), 6.12-6.19 (m, 1H), 6.66 (s, 1H), 6.77 (s, 1H), 6.94 (d, 2H), 7.52 (d, 2H). |
| | | | LCMS (Methode 5): Rₜ = 1.22 min; m/z = 520 (M+H)⁺ |
| **198** | 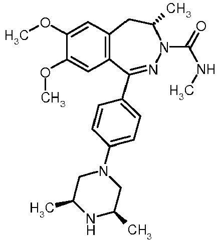 | (4S)-1-{4-[(3R,5S)-3,5-Dimethylpiperazin-1-yl]phenyl}-7,8-dimethoxy-N,4-dimethyl-4,5-dihydro-3H-2,3-benzodiazepin-3-carboxamid | ¹H-NMR (300MHz, CDCl₃): δ = 1.14 (d, 3H), 1.19 (d, 6H), 2.40 (t, 2H), 2.74 (dd, 1H), 2.88 (d, 3H), 2.96 (dd, 1H), 3.01-3.13 (m, 2H), 3.66 (dd, 2H), 3.73 (s, 3H), 3.96 (s, 3H), 5.24-5.37 (m, 1H), 6.11 (q, 1H), 6.67 (s, 1H), 6.77 (s, 1H), 6.93 (d, 2H), 7.51 (d, 2H). |
| | | | LCMS (Methode 1): Rₜ = 0.77 min; m/z = 466 (M+H)⁺ |

### Beispiel 199

### (±)-7,8-Dihydroxy-N,4-dimethyl-1-[4-(4-methylpiperazin-1-yl)phenyl]-4,5-dihydro-3H-2,3-benzodiazepin-3-carboxamid

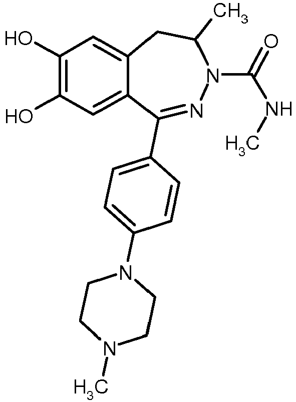

Die Verbindung wurde analog Beispiel **91A** aus Beispiel **127** hergestellt.
LCMS (Methode 1): Rₜ = 1,01 min; m/z = 424 (M+H)⁺
¹H-NMR (300MHz, DMSO-*d*₆): δ = 0,97 (d, 3H), 2,50 (m, 1H), 2,60 (d, 3H), 3,11 (s, 3H), 3,22 - 3,53 (m, 5H), 3,77 - 3,94 (m, 4H), 4,80 - 4,90 (m, 1H), 6,26 - 6,32 (m, 1H), 6,36 (s, 1H), 6,69 (s, 1H), 6,99 (d, 2H), 7,57 (d, 2H), 8,91 (s, br, 1H), 9,36 (s, br, 1H).

### Beispiel 200

### (±)-7,8-Diethoxy-N,4-dimethyl-1-[4-(4-methylpiperazin-1-yl)phenyl]-4,5-dihydro-3H-2,3-benzodiazepin-3-carboxamid

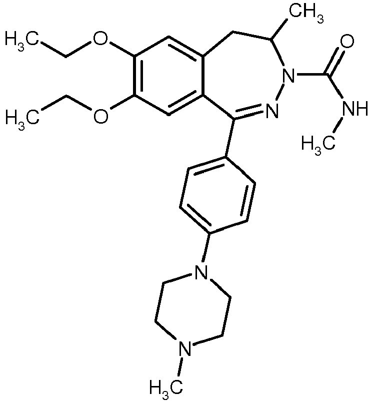

Die Verbindung wurde analog Beispiel **93A** aus Beispiel **199** hergestellt.
LCMS (Methode 1): Rₜ = 1,48 min; m/z = 480 (M+H)⁺
¹H-NMR (600MHz, DMSO-*d*₆): δ = 1,03 (d, 3H), 1,22 (t, 3H), 1,35 (t, 3H), 2,51 (m, 1H), 2,64 (d, 3H), 2,84 (dd, 1H), 3,14 (s, 3H), 3,30 - 3,37 (m, 2H), 3,51 (d, 2H), 3,77 - 3,88 (m, 4H), 3,92 (d, 2H), 4,11 (q, 2H), 4,90 - 4,96 (m, 1H), 6,37 (q, 1H), 6,50 (s, 1H), 7,00 (s, 1H), 7,02 (d, 2H), 7,62 (d, 2H).

Analog Beispiel **127.1** wurden aus Beispiel **127A** den entsprechenden kommerziell erhältlichen Aminen folgende Beispielverbindungen hergestellt:

| Nr | Struktur | Name | Analytische Daten |
|---|---|---|---|
| **201** | 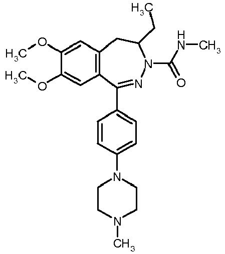 | (±)-4-Ethyl-7,8-dimethoxy-N-methyl-1-[4-(4-methylpiperazin-1-yl)phenyl]-4,5-dihydro-3H-2,3-benzodiazepin-3-carboxamid | ¹H-NMR (300MHz, CDCl₃): δ = 0.90 (t, 3H), 1.21-1.37 (m, 1H), 1.50-1.61 (m, 1H), 2.40 (s, 3H), 2.56-2.69 (m, 4H), 2.84 (dd, 1H), 2.88 (d, 3H), 2.99 (dd, 1H), 3.29-3.39 (m, 4H), 3.71 (s, 3H), 3.96 (s, 3H), 5.12-5.24 (m, 1H), 6.22-6.32 (m, 1H), 6.68 (s, 1H), 6.76 |
| | | | (s, 1H), 6.94 (d, 2H), 7.49 (d, 2H). |
| | | | LCMS (Methode 1): Rₜ = 0.65 min; m/z = 466 (M+H)⁺ |
| **201.1** | 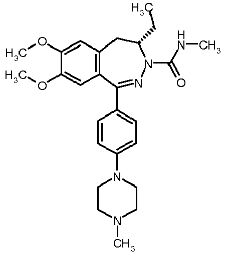 | (4S)-4-Ethyl-7,8-dimethoxy-N-methyl-1-[4-(4-methylpiperazin-1-yl)phenyl]-4,5-dihydro-3H-2,3-benzodiazepin-3-carboxamid | Präp. HPLC: Methode XIIa |
| | | | Analyt. HPLC (Methode Q): Rₜ = 10.90 min |
| | | | [α]_{D}²⁰ = 100.8° (c = 1.00; CHCl₃) |
| | | | LCMS (Methode 5): Rₜ = 0.64 min; m/z = 466 (M+H)⁺ |
| **202** | 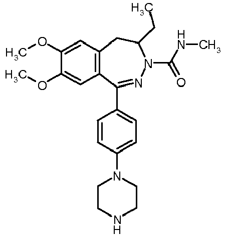 | (±)-4-Ethyl-7,8-dimethoxy-N-methyl-1-[4-(piperazin-1-yl)phenyl]-4,5-dihydro-3H-2,3-benzodiazepin-3-carboxamid | ¹H-NMR (300MHz, CDCl₃): δ = 0.90 (t, 3H), 1.23-1.38 (m, 1H), 1.52-1.61 (m, 1H), 2.85 (dd, 1H), 2.88 (d, 3H), 2.99 (dd, 1H), 3.04-3.11 (m, 4H), 3.23-3.31 (m, 4H), 3.71 (s, 3H), 3.96 (s, 3H), 5.13-5.22 (m, 1H), 6.26 (q, 1H), 6.69 (s, 1H), 6.77 (s, 1H), 6.93 (d, 2H), 7.49 (d, 2H). |
| | | | LCMS (Methode 1): Rₜ = 0.65 min; m/z = 452 (M+H)⁺ |
| **202.1** | 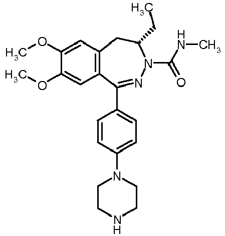 | (4S)-4-Ethyl-7,8-dimethoxy-N-methyl-1-[4-(piperazin-1-yl)phenyl]-4,5-dihydro-3H-2,3-benzodiazepin-3-carboxamid | Präp. HPLC: Methode XXa |
| | | | Analyt. HPLC (Methode L1): Rₜ = 4.10 min |
| | | | [α]_{D}²⁰ = 86.2° (c = 1.00; CHCl₃) |
| | | | LCMS (Methode 5): Rₜ = 0.63 min; m/z = 452 (M+H)⁺ |
| **202.2** | 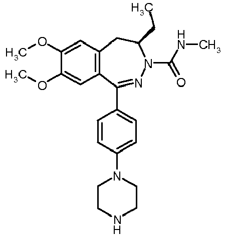 | (4R)-4-Ethyl-7,8-dimethoxy-N-methyl-1-[4-(piperazin-1-yl)phenyl]-4,5-dihydro-3H-2,3-benzodiazepin-3-carboxamid | Präp. HPLC: Methode XXa |
| | | | Analyt. HPLC (Methode L1): Rₜ = 5.50 min |
| | | | [α]_{D}²⁰ = -103.4° (c = 1.00; CHCl₃) |
| | | | LCMS (Methode 5): Rₜ = 0.63 min; m/z = 452 (M+H)⁺ |
| **203** | 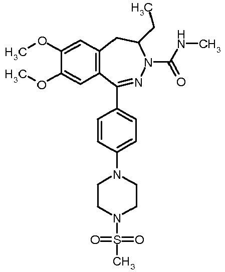 | (±)-4-Ethyl-7,8-dimethoxy-N-methyl-1-{4-[4-(methylsulfonyl)piperazi n-1-yl]phenyl}-4,5-dihydro-3H-2,3-benzodiazepin-3-carboxamid | ¹H-NMR (300MHz, CDCl₃): δ = 0.89 (t, 3H), 1.18-1.33 (m, 1H), 1.46-1.59 (m, 1H), 2.87 (s, 3H), 2.81-2.88 (m, 1H), 2.89 (d, 3H), 3.01 (dd, 1H), 3.35-3.47 (m, 8H), 3.70 (s, 3H), 3.96 (s, 3H), 5.15-5.25 (m, 1H), 6.30-6.38 (m, 1H), 6.66 (s, 1H), 6.76 (s, 1H), 6.95 (d, 2H), 7.49 (d, 2H). |
| | | | LCMS (Methode 1): Rₜ = 1.01 min; m/z = 530 (M+H)⁺ |
| **203.1** | 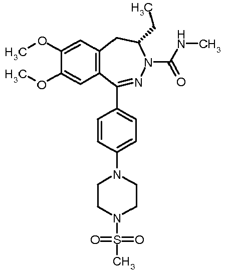 | (4S)-4-Ethyl-7,8-dimethoxy-N-methyl-1-{4-[4-(methylsulfonyl)piperazi n-1-yl]phenyl}-4,5-dihydro-3H-2,3-benzodiazepin-3-carboxamid | Präp. HPLC: Methode IVa |
| | | | Analyt. HPLC (Methode Fa): Rₜ = 6.22 min |
| | | | [α]_{D}²⁰ = 252.5° (c = 1.00; MeOH) |
| | | | LCMS (Methode 5): Rₜ = 1.0 min; m/z = 530 (M+H)⁺ |
| **203.2** | 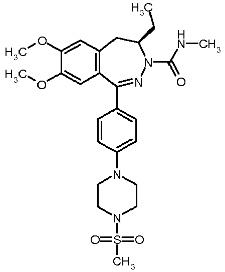 | (4R)-4-Ethyl-7,8-dimethoxy-N-methyl-1-{4-[4-(methylsulfonyl)piperazi n-1-yl]phenyl}-4,5-dihydro-3H-2,3-benzodiazepin-3-carboxamid | Präp. HPLC: Methode IVa |
| | | | Analyt. HPLC (Methode Fa): Rₜ = 4.37 min |
| | | | [α]_{D}²⁰ = -257.4° (c = 1.00; MeOH) |
| | | | LCMS (Methode 5): Rₜ = 1.0 min; m/z = 530 (M+H)⁺ |
| **204** | 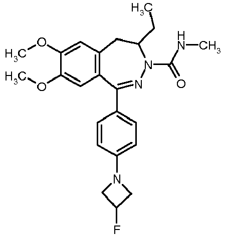 | (±)-4-Ethyl-1-[4-(3-fluorazetidin-1-yl)phenyl]-7,8-dimethoxy-N-methyl-4,5-dihydro-3H-2,3-benzodiazepin-3-carboxamid | ¹H-NMR (300MHz, CDCl₃): δ = 0.92 (t, 3H), 1.26-1.46 (m, 1H), 1.53-1.71 (m, 1H), 2.83 (m, 1H), 2.87 (d, 3H), 2.98 (dd, 1H), 3.72 (s, 3H), 3.96 (s, 3H), 4.0-4.15 (m, 2H), 4.23-4.37 (m, 2H), 5.10-5.23 (m, 1H), 5.49 (d, 1H), 6.20 (q, 1H), 6.49 (d, 2H), 6.67 (s, 1H), 6.78 (s, 1H), 7.52 (s, br, 2H). |
| | | | LCMS (Methode 5): Rₜ = 1.13 min; m/z = 441 (M+H)⁺ |
| **205** | 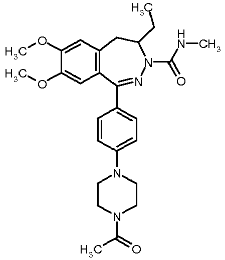 | (±)-1-[4-(4-Acetylpiperazin-1-yl)phenyl]-4-ethyl-7,8-dimethoxy-N-methyl-4,5-dihydro-3H-2,3-benzodiazepine-3-carboxamid | ¹H-NMR (300MHz, CDCl₃): δ = 0.90 (t, 3H), 1.19-1.36 (m, 1H), 1.47-1.62 (m, 1H), 2.18 (s, 3H), 2.83-2.93 (m, 1H), 2.88 (d, 3H), 3.01 (dd, 1H), 3.26-3.38 (m, 4H), 3.66-3.73 (m, 2H), 3.70 (s, 3H), 3.80-3.88 (m, 2H), 3.96 (s, 3H), 5.14-5.26 (m, 1H), 6.29-6.38 (m, 1H), 6.66 (s, 1H), 7.77 (s, 1H), 6.96 (d, 2H), 7.51 (d, 2H). |
| | | | LCMS (Methode 5): Rₜ = 0.92 min; m/z = 494 (M+H)⁺ |
| **206** | 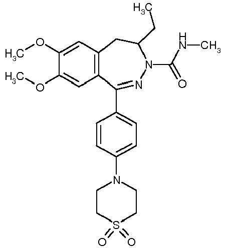 | (±)-1-[4-(1,1-Dioxidothiomorpholin-4-yl)phenyl]-4-ethyl-7,8-dimethoxy-N-methyl-4,5-dihydro-3H-2,3-benzodiazepin-3-carboxamid | ¹H-NMR (300MHz, CDCl₃): δ = 0.90 (t, 3H), 1.17-1.34 (m, 1H), 1.45-1.59 (m, 1H), 2.85-2.95 (m, 1H), 2.89 (d, 3H), 3.02 (dd, 1H), 3.12-3.20 (m, 4H), 3.72 (s, 3H), 3.97 (s, 3H), 3.96-4.03 (m, 4H), 5.17-5.28 (m, 1H), 6.34-6.43 (m, 1H), 6.66 (s, 1H), 6.77 (s, 1H), 6.94 (d, 2H), 7.53 (d, 2H). |
| | | | LCMS (Methode 5): Rₜ = 0.92 min; m/z = 501 (M+H)⁺ |
| **207** | 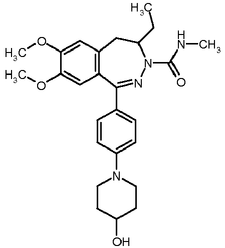 | (±)-4-Ethyl-1-[4-(4-hydroxypiperidin-1-yl)phenyl]-7,8-dimethoxy-N-methyl-4,5-dihydro-3H-2,3-benzodiazepin-3-carboxamid | ¹H-NMR (400MHz, CDCl₃): δ = 0.90 (t, 3H), 1.25-1.38 (m, 1H), 1.51-1.64 (m, 2H), 1.67-1.78 (m, 2H), 2.01-2.10 (m, 2H), 2.84 (dd, 1H), 2.88 (d, 3H), 2.98 (dd, 1H), 3.07 (dt, 2H), 3.66-3.71 (m, 2H), 3.71 (s, 3H), 3.89-3.95 (m 1H), 3.96 (s, 3H), 5.12-5.21 (m, 1H), 6.20-6.26 (m, 1H), 6.69 (s, 1H), 6.77 (s, 1H), 6.94 (d, 2H), 7.48 (d, 2H). |
| | | | LCMS (Methode 5): Rₜ = 0.87 min; |
| | | | m/z = 467 (M+H)⁺ |
| **208** | 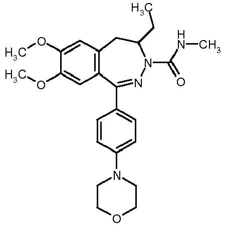 | (±)-4-Ethyl-7,8-dimethoxy-N-methyl-1-[4-(morpholin-4-yl)phenyl]-4,5-dihydro-3H-2,3-benzodiazepin-3-carboxamid | ¹H-NMR (300MHz, CDCl₃): δ = 0.90 (t, 3H), 1.20-1.36 (m, 1H), 1.50-1.65 (m, 1H), 2.86 (dd, 1H), 2.88 (d, 3H), 3.00 (dd, 1H), 3.24-3.31 (m, 4H), 3.71 (s, 3H), 3.87-3.94 (m, 4H), 3.96 (s, 3H), 5.13-5.24 (m, 1H), 6.30 (q, 1H), 6.68 (s, 1H), 6.76 (s, 1H), 6.93 (d, 2H), 7.50 (d, 2H). |
| | | | LCMS (Methode 5): Rₜ = 1.06 min; m/z = 453 (M+H)⁺ |
| **209** | 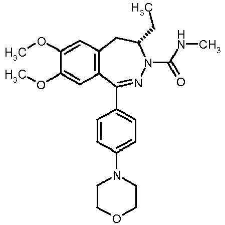 | (4S)-4-Ethyl-7,8-dimethoxy-N-methyl-1-[4-(morpholin-4-yl)phenyl]-4,5-dihydro-3H-2,3-benzodiazepin-3-carboxamid | Präp. HPLC: Methode XIIa Analyt. HPLC (Methode V): Rₜ = 12.46 min |
| | | | [α]_{D}²⁰ = 75.1° (c = 1.00; CHCl₃) |
| | | | LCMS (Methode 5): Rₜ = 1.06 min; m/z = 453 (M+H)⁺ |

Analog Beispiel **127.1** wurden aus Beispiel **130A** den entsprechenden kommerziell erhältlichen Aminen folgende Beispielverbindungen hergestellt. Unter den Reaktionsbedingungen wurden vielfach Gemische aus mono- und di-aminierten Produkten, sowie mono-dehalogenierte Kupplungsprodukte erhalten, die chromatographisch getrennt wurden.

| Nr | Struktur | Name | Analytische Daten |
|---|---|---|---|
| **210** | 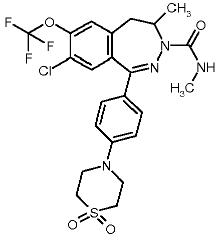 | (±)-8-Chlor-1-[4-(1,1-dioxidothiomorpholin-4-yl)phenyl]-N,4-dimethyl-7-(trifluormethoxy)-4,5-dihydro-3H-2,3-benzodiazepin-3-carboxamid | ¹H-NMR (400MHz, CDCl₃): δ = 1.02 (d, 3H), 2.79 - 2.92 (m, 4H), 3.06 (dd, 1H), 3.11 - 3.18 (m, 4H), 3.92 - 4.01 (m, 4H), 5.35 - 5.48 (m, 1H), 6.27 (br. s., 1H), 6.95 (d, 2H), 7.21 (d, 1H), 7.26 (s, 1H), 7.46 (d, 2H). |
| | | | LCMS (Methode 1): Rₜ = 1.28 min; m/z = 545 (M+H)⁺ |
| **211** | 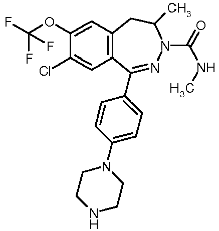 | (±)-8-Chlor-N,4-dimethyl-1-[4-(piperazin-1-yl)phenyl]-7-(trifluormethoxy)-4,5-dihydro-3H-2,3-benzodiazepin-3-carboxamid | ¹H-NMR (400MHz, CDCl₃): δ = 1.07 (d, 3H), 2.79 (dd, 1H), 2.86 (d, 3H), 3.00 (dd, 1H), 3.05-3.11 (m, 4H), 3.26-3.35 (m, 4H), 5.27-5.37 (m, 1H), 6.12 (q, 1H), 6.92 (d, 2H), 7.21 (s, br, 1H), 7.28 (s, 1H), 7.44 (d, 2H). |
| | | | LCMS (Methode 1): Rₜ = 0.93 min; m/z = 496 (M+H)⁺ |
| **212** | 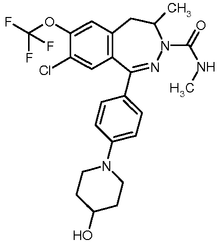 | (±)-8-Chlor-1-[4-(4-hydroxypiperidin-1-yl)phenyl]-N,4-dimethyl-7-(trifluormethoxy)-4,5-dihydro-3H-2,3-benzodiazepin-3-carboxamid | ¹H-NMR (300MHz, CDCl₃): δ = 1.11 (d, 3H), 1.72-1.86 (m, 2H), 1.99-2.11 (m, 2H), 2.78 (dd, 1H), 2.88 (d, 3H), 3.00 (dd, 1H), 3.04-3.16 (m, 2H), 3.66-3.78 (m, 2H), 3.88-4.01 (m, 1H), 5.25-5.38 (m, 1H), 6.05-6.15 (m, 1H), 6.95 (d, 2H), 7.23 (s, 1H), 7.29 (s, 1H), 7.46 (d, 2H). |
| | | | LCMS (Methode 1): Rₜ = 1.26 min; m/z = 511 (M+H)⁺ |
| **213** | 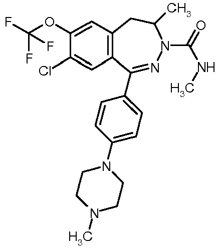 | (±)-8-Chlor-N,4-dimethyl-1-[4-(4-methylpiperazin-1-yl)phenyl]-7-(trifluormethoxy)-4,5-dihydro-3H-2,3-benzodiazepin-3-carboxamid | ¹H-NMR (400MHz, CDCl₃): δ = 1.04 (d, 3H), 2.61 (br. s., 3H), 2.73 - 2.93 (m, 7H), 3.03 (dd, 2H), 3.51 (d, 4H), 5.37 (br. s., 1H), 6.20 (br. s., 1H), 6.93 (d, 2H), 7.20 (s, 1H), 7.25 (s, 1H), 7.43 (d, 2H). |
| | | | LCMS (Methode 1): Rₜ = 0.95 min; m/z = 510 (M+H)⁺ |
| **214** | 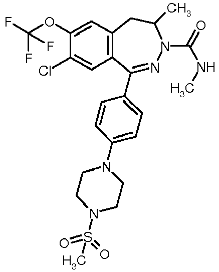 | (±)-8-Chlor-N,4-dimethyl-1-{4-[4-(methylsulfonyl)piperazi n-1-yl]phenyl}-7-(trifluormethoxy)-4,5-dihydro-3H-2,3-benzodiazepin-3-carboxamid | ¹H-NMR (300MHz, CDCl₃): δ = 1.06 (d, 3H), 2.85 (dd, 1H), 2.87 (s, 3H), 2.90 (d, 3H), 3.06 (d, 1H), 3.39-3.51 (br. s., 8H), 5.34-5.46 (m, 1H), 6.25 (q, 1H), 6.97 (d, 2H), 7.23 (s, 1H), 7.28 (s, 1H), 7.47 (d, 2H). |
| | | | LCMS (Methode 1): Rₜ = 1.35 min; |
| | | | m/z = 574 (M+H)⁺ |
| **215** | 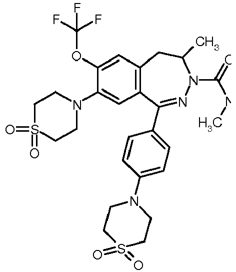 | (±)-8-(1,1-Dioxidothiomorpholin-4-yl)-1-[4-(1,1-dioxidothiomorpholin-4-yl)phenyl]-N,4-dimethyl-7-(trifluormethoxy)-4,5-dihydro-3H-2,3-benzodiazepin-3-carboxamid | ¹H-NMR (400MHz, CDCl₃): δ = 1.08 (d, 3H), 2.80 (dd, 1H), 2.90 (s, 3H), 3.01 (dd, 1H), 3.15-3.20 (m, 8H), 3.42-3.48 (m, 4H), 3.98-4.03 (m, 4H), 5.31-5.41 (m, 1H), 6.12-6.26 (m, 1H), 6.86 (s, 1H), 6.97 (d, 2H), 7.16 (d, 1H), 7.51 (d, 2H). |
| | | | LCMS (Methode 1): Rₜ = 1.11 min; m/z = 644 (M+H)⁺ |
| **216** | 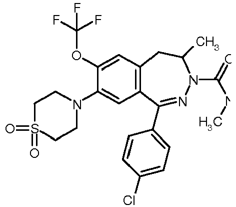 | (±)-1-(4-Chlorphenyl)-8-(1,1-dioxidothiomorpholin-4-yl)-N,4-dimethyl-7-(trifluormethoxy)-4,5-dihydro-3H-2,3-benzodiazepin-3-carboxamid | ¹H-NMR (400MHz, DMSO-*d₆*): δ = 0.95 (d, 3H), 2.68 (d, 3H), 2.75 (dd, 1H), 2.91 (s, 3H), 2.92 - 3.05 (m, 5H), 3.16 - 3.23 (m, 4H), 5.02 - 5.13 (m, 1H), 6.70 (q, 1H), 6.76 (s, 1H), 7.39 (d, 1H), 7.49 (d, 2H), 7.69 (d, 2H). |
| | | | LCMS (Methode 5): Rₜ = 1.33 min; m/z = 574 (M+H)⁺ |
| **217** | 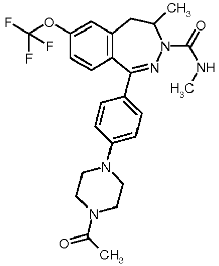 | (±)-1-[4-(4-Acetylpiperazin-1-yl)phenyl]-N,4-dimethyl-7-(trifluormethoxy)-4,5-dihydro-3H-2,3-benzodiazepin-3-carboxamid | ¹H-NMR (400MHz, CDCl₃): δ = 1.09 (d, 3H), 2.18 (s, 3H), 2.84 (dd, 1H), 2.89 (d, 3H), 3.06 (dd, 1H), 3.26-3.36 (m, 4H), 3.68 (t, 2H), 3.82 (t, 2H), 5.33-5.42 (m, 1H), 6.19 (q, 1H), 6.94 (d, 2H), 7.08 (d, 1H), 7.13 (s, br, 1H), 7.20 (d, 1H), 7.48 (d, 2H). |
| | | | LCMS (Methode 1): Rₜ = 1.21 min; m/z = 504 (M+H)⁺ |
| **218** | 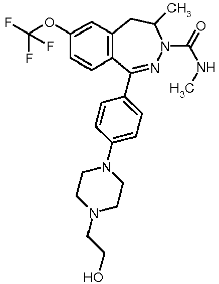 | (±)-1-{4-[4-(2-Hydroxyethyl)piperazin-1-yl]phenyl}-N,4-dimethyl-7-(trifluormethoxy)-4,5-dihydro-3H-2,3-benzodiazepin-3-carboxamid | ¹H-NMR (400MHz, CDCl₃): δ = 1.07 (d, 3H), 2.83 (d, 1H), 2.90 (d, 3H), 2.93 (d, 1H), 2.94-3.01 (m, 2H), 3.04-3.17 (m, 5H), 3.52-3.61 (m, 4H), 3.89-3.96 (m, 2H), 5.32-5.44 (m, 1H), 6.18-6.25 (m, 1H), 6.95 (d, 2H), 7.08 (d, 1H), 7.13 (s, br, 1H), 7.19 (d, 1H), 7.48 (d, 2H). |
| | | | LCMS (Methode 1): Rₜ = 1.03 min; m/z = 506 (M+H)⁺ |
| **219** | 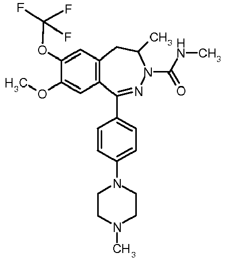 | (±)-8-Methoxy-N,4-dimethyl-1-[4-(4-methylpiperazin-1-yl)phenyl]-7-(trifluormethoxy)-4,5-dihydro-3H-2,3-benzodiazepin-3-carboxamid | ¹H-NMR (300MHz, CDCl₃): δ = 1.11 (d, 3H), 2.40 (s, 3H), 2.58-2.65 (m, 4H), 2.73 (dd, 1H), 2.89 (d, 3H), 2.96 (dd, 1H), 3.32-3.39 (m, 4H), 3.73 (s, 3H), 5.24-5.34 (m, 1H), 6.12 (q, 1H), 6.79 (s, 1H), 6.95 (d, 2H), 7.14 (s, br, 1H), 7.51 (d, 2H). |
| | | | LCMS (Methode 5): Rₜ = 0.80 min; m/z = 506 (M+H)⁺ |

Analog zu Beispiel 2 wurden aus Beispiel **127A** bzw. Beispiel **128A** und den entsprechenden kommerziell erhältlichen Boronsäurederivaten folgende Beispielverbindungen hergestellt:

| Nr | Struktur | Name | Analytische Daten |
|---|---|---|---|
| **220** | 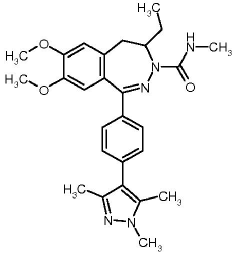 | (±)-4-Ethyl-7,8-dimethoxy-N-methyl-1-[4-(1,3,5-trimethyl-1H-pyrazol-4-yl)phenyl]-4,5-dihydro-3H-2,3-benzodiazepin-3-carboxamid | ¹H-NMR (400MHz, CDCl₃): δ = 0.90 (t, 3H), 1.13-1.26 (m, 1H), 1.42-1.56 (m, 1H), 2.32 (s, 6H), 2.92 (d, 3H), 2.98 (dd, 1H), 3.08 (dd, 1H), 3.70 (s, 3H), 3.83 (s, 3H), 3.97 (s, 3H), 5.24-5.32 (m, 1H), 6.57 (q, 1H), 6.71 (s, 1H), 6.76 (s, 1H), 7.30 (d, 2H), 7.56 (d, 2H). |
| | | | LCMS (Methode 5): Rₜ = 1.10 min; |
| | | | m/z = 476 (M+H)⁺ |
| **221** | 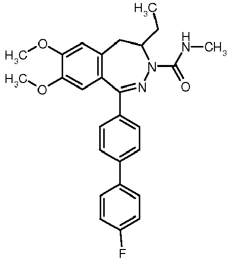 | (±)-4-Ethyl-1-(4'-fluorbiphenyl-4-yl)-7,8-dimethoxy-N-methyl-4,5-dihydro-3H-2,3benzodiazepin-3-carboxamid | ¹H-NMR (400MHz, CDCl₃): δ = 0.89 (t, 3H), 1.11-1.23 (m, 1H), 1.39-1.51 (m, 1H), 2.92 (d, 3H), 3.01 (dd, 1H), 3.10 (dd, 1H), 3.69 (s, 3H), 3.97 (s, 3H), 5.27-5.35 (m, 1H), 6.63 (q, 1H), 6.69 (s, 1H), 6.76 (s, 1H), 7.18 (dd, 2H), 7.55-7.66 (m, 6H). |
| | | | LCMS (Methode 5): Rₜ = 1.42 min; m/z = 462 (M+H)⁺ |
| **222** | 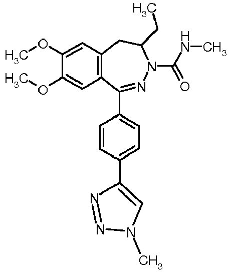 | (±)-4-Ethyl-7,8-dimethoxy-N-methyl-1-[4-(1-methyl-1H-1,2,3-triazol-4-yl)phenyl] -4,5-dihydro-3H-2,3-benzodiazepin-3-carboxamid | ¹H-NMR (400MHz, CDCl₃): δ = 0.88 (t, 3H), 1.08-1.22 (m, 1H), 1.37-1.49 (m, 1H), 2.92 (d, 3H), 3.01 (dd, 1H), 3.10 (dd, 1H), 3.67 (s, 3H), 3.96 (s, 3H), 4.20 (s, 3H), 5.27-5.37 (m, 1H), 6.63 (q, 1H), 6.67 (s, 1H), 6.75 (s, 1H), 7.57 (d, 2H), 7.82 (s, 1H), 7.88 (d, 2H). |
| | | | LCMS (Methode 5): Rₜ = 0.97 min; m/z = 449 (M+H)⁺ |
| **223** | 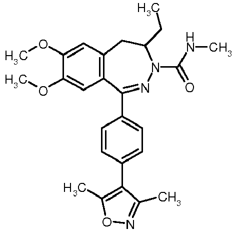 | (±)-1-[4-(3,5-Dimethyl-1,2-oxazol-4-yl)phenyl]-4-ethyl-7,8-dimethoxy-N-methyl-4,5-dihydro-3H-2,3-benzodiazepin-3-carboxamid | ¹H-NMR (300MHz, CDCl₃): δ = 0.89 (t, 3H), 1.07-1.23 (m, 1H), 1.36-1.52 (m, 1H), 2.35 (s, 3H), 2.49 (s, 3H), 2.92 (d, 3H), 3.01 (dd, 1H), 3.10 (dd, 1H), 3.70 (s, 3H), 3.97 (s, 3H), 5.26-5.37 (m, 1H), 6.62 (q, 1H), 6.67 (s, 1H), 6.76 (s, 1H), 7.32 (d, 2H), 7.59 (d, 2H). |
| | | | LCMS (Methode 5): Rₜ = 1.21 min; m/z = 463 (M+H)⁺ |
| **224** | 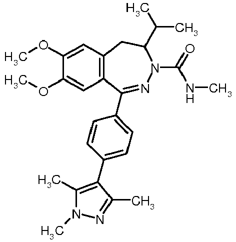 | (±)-4-Isopropyl-7,8-dimethoxy-N-methyl-1-[4-(1,3,5-trimethyl-1H-pyrazol-4-yl)phenyl]-4,5-dihydro-3H-2,3-benzodiazepin-3-carboxamid | ¹H-NMR (300MHz, CDCl₃): δ = 0.87 (d, 3H), 0.89 (d, 3H), 1.32-1.46 (m, 1H), 2.32 (s, 6H), 2.90 (d, 3H), 3.0 (dd, 1H), 3.13 (dd, 1H), 3.69 (s, 3H), 3.83 (s, 3H), 3.96 (s, 3H), 5.14-5.24 (m, 1H), 6.64 (q, 1H), 6.73 (s, 1H), 6.77 (s, 1H), 7.30 (d, 2H), 7.55 (d, 2H). |
| | | | LCMS (Methode 5): Rₜ = 1.17 min; m/z = 490 (M+H)⁺ |

Analog Beispiel 34 wurden aus Beispiel **127A** und den entsprechenden kommerziell erhältlichen Lactamen bzw. cyclischen Carbamaten folgende Beispielverbindungen hergestellt:

| Nr | Struktur | Name | Analytische Daten |
|---|---|---|---|
| **225** | 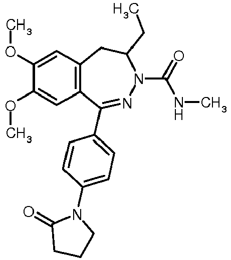 | (±)-4-Ethyl-7,8-dimethoxy-N-methyl-1-[4-(2-oxopyrrolidin-1-yl)phenyl]-4,5-dihydro-3H-2,3-benzodiazepin-3-carboxamid | ¹H-NMR (400MHz, CDCl₃): δ = 0.88 (t, 3H), 1.10-1.22 (m, 1H), 1.38-1.50 (m, 1H), 2.22 (pent, 2H), 2.67 (dd, 2H), 2.91 (d, 3H), 2.97 (dd, 1H), 3.07 (dd, 1H), 3.68 (s, 3H), 3.93 (dd, 2H), 3.95 (s, 3H), 5.23-5.31 (m, 1H), 6.56 (q, 1H), 6.64 (s, 1H), 6.75 (s, 1H), 7.52 (d, 2H), 7.68 (d, 2H). |
| | | | LCMS (Methode 1): Rₜ = 1.11 min; m/z = 452 (M+H)⁺ |
| **226** | 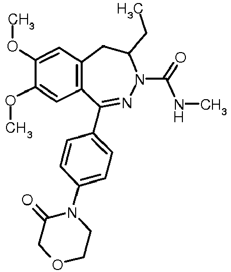 | (±)-4-Ethyl-7,8-dimethoxy-N-methyl-1-[4-(3-oxomorpholin-4-yl)phenyl]-4,5-dihydro-3H-2,3-benzodiazepin-3-carboxamid | ¹H-NMR (400MHz, CDCl₃): δ = 0.87 (t, 3H), 1.07-1.19 (m, 1H), 1.34-1.47 (m, 1H), 2.91 (d, 3H), 2.99 (dd, 1H), 3.07 (dd, 1H), 3.69 (s, 3H), 3.81-3.90 (m, 2H), 3.96 (s, 3H), 4.09 (t, 2H), 4.39 (s, 2H), 5.26-5.34 (m, 1H), 6.61 (q, 1H), 6.66 (s, 1H), 6.74 (s, 1H), 7.41 (d, 2H), 7.55 (d, 2H). |
| | | | LCMS (Methode 5): Rₜ = 0.91 min; m/z = 467 (M+H)⁺ |
| **227** | 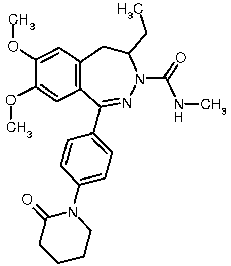 | (±)-4-Ethyl-7,8-dimethoxy-N-methyl-1-[4-(2-oxopiperidin-1-yl)phenyl]-4,5-dihydro-3H-2,3-benzodiazepin-3-carboxamid | ¹H-NMR (400MHz, CDCl₃): δ = 0.88 (t, 3H), 1.08-1.21 (m, 1H), 1.36-1.48 (m, 1H), 1.94-2.06 (m, 4H), 2.62 (t, 2H), 2.90 (d, 3H), 2.98 (dd, 1H), 3.06 (dd, 1H), 3.69 (s, 3H), 3.73 (t, 2H), 3.95 (s, 3H), 5.24-5.33 (m, 1H), 6.59 (q, 1H), 6.68 (s, 1H), 6.74 (s, 1H), 7.32 (d, 2H), 7.53 (d, 2H). |
| | | | LCMS (Methode 5): Rₜ = 1.01 min; m/z = 465 (M+H)⁺ |
| **228** | 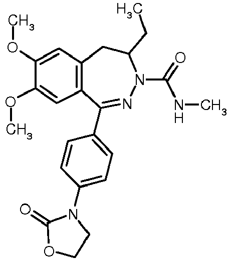 | (±)-4-Ethyl-7,8-dimethoxy-N-methyl-1-[4-(2-oxo-1,3-oxazolidin-3-yl)phenyl]-4,5-dihydro-3H-2,3-benzodiazepin-3-carboxamid | ¹H-NMR (300MHz, CDCl₃): δ = 0.88 (t, 3H), 1.07-1.24 (m, 1H), 1.35-1.51 (m, 1H), 2.91 (d, 3H), 2.98 (dd, 1H), 3.08 (dd, 1H), 3.68 (s, 3H), 3.96 (s, 3H), 4.15 (dd, 2H), 4.56 (t, 2H), 5.23-5.34 (m, 1H), 6.56 (q, 1H), 6.63 (s, 1H), 6.75 (s, 1H), 7.53 (d, 2H), 7.61 (d, 2H). |
| | | | LCMS (Methode 5): Rₜ = 0.97 min; m/z = 453 (M+H)⁺ |

Analog Beispiel 34 wurden aus Beispiel **49.2A** und den entsprechenden kommerziell erhältlichen Lactamen bzw. cyclischen Carbamaten folgende Beispielverbindungen hergestellt:

| Nr | Struktur | Name | Analytische Daten |
|---|---|---|---|
| **229** | 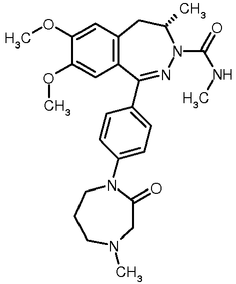 | (4S)-7,8-Dimethoxy-N,4-dimethyl-1-[4-(4-methyl-2-oxo-1,4-diazepan-1-yl)phenyl]-4,5-dihydro-3H-2,3-benzodiazepin-3-carboxamid | ¹H-NMR (300MHz, CDCl₃): δ = 0.98 (d, 3H), 2.08 (m, 2H), 2.64 (s, 3H), 2.81 (dd, 1H), 2.88 (d, 3H), 3.03-3.16 (m, 3H), 3.68 (s, 3H), 3.75 (s, br, 2H), 3.87-3.92 (m, 2H), 3.93 (s, 3H), 5.36-5.49 (m, 1H), 6.45 (q, 1H), 6.62 (s, 1H), 6.72 (s, 1H), 7.27 (d, 2H), 7.52 (d, 2H). |
| | | | LCMS (Methode 1): Rₜ = 0.72 min; m/z = 480 (M+H)⁺ |

Analog Beispiel 34 wurden aus Beispiel **129A** und den entsprechenden kommerziell erhältlichen Lactamen bzw. cyclischen Carbamaten folgende Beispielverbindungen hergestellt.

| Nr | Struktur | Name | Analytische Daten |
|---|---|---|---|
| **230** | 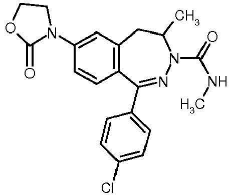 | (±)-1-(4-Chlorphenyl)-N,4-dimethyl-7-(2-oxo-1,3-oxazolidin-3-yl)-4,5-dihydro-3H-2,3-benzodiazepin-3-carboxamid | ¹H-NMR (400MHz, CDCl₃): δ = 0.97 (d, 3H), 2.92 (d, 3H), 2.99 (dd, 1H), 3.20 (d, 1H), 4.07-4.20 (m, 2H), 4.54 (t, 2H), 5.48-5.59 (m, 1H), 6.47-6.57 (m, 1H), 7.12 (d, 1H), 7.34-7.46 (m, 5H), 7.53 (d, 1H). |
| | | | LCMS (Methode 1): Rₜ = 1.23 min; m/z = 413 (M+H)⁺ |
| **230. 1** | 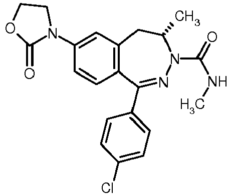 | (4S)-1-(4-Chlorphenyl)-N,4-dimethyl-7-(2-oxo-1,3-oxazolidin-3-yl)-4,5-dihydro-3H-2,3-benzodiazepin-3-carboxamid | Präp. HPLC: Methode XXIa |
| | | | Analyt. HPLC (Methode Y1): Rₜ = 3.93 min |
| | | | [α]_{D}²⁰ = 108.1° (c = 1.00; MeOH) |
| | | | LCMS (Methode 1): Rₜ = 1.36 min; m/z = 499 (M+H)⁺ |
| **231** | 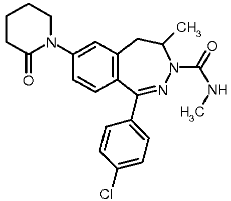 | (±)-1-(4-Chlorphenyl)-N,4-dimethyl-7-(2-oxopiperidin-1-yl)-4,5-dihydro-3H-2,3-benzodiazepin-3-carboxamid | ¹H-NMR (400MHz, Cdl₃): δ = 0.99 (d, 3H), 1.93-2.05 (m, 4H), 2.61 (t, 2H), 2.88-2.95 (m, 1H), 2.91 (d, 3H), 3.15 (dd, 1H), 3.65-3.76 (m, 2H), 5.43-5.53 (m, 1H), 6.42-6.50 (m, 1H), 7.09-7.17 (m, 2H), 7.21 (d, 1H), 7.39 (d, 2H), 7.46 (d, 2H). |
| | | | LCMS (Methode 1): Rₜ = 1.22 min; m/z = 425 (M+H)⁺ |
| **232** | 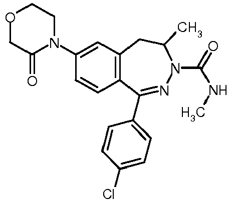 | (±)-1-(4-Chlorphenyl)-N,4-dimethyl-7-(3-oxomorpholin-4-yl)-4,5-dihydro-3H-2,3-benzodiazepin-3-carboxamid | ¹H-NMR (400MHz, CDCl₃): δ = 0.99 (d, 3H), 2.91 (d, 3H), 2.94 (dd, 1H), 3.17 (dd, 1H), 3.77-3.89 (m, 2H), 4.08 (t, 2H), 4.38 (s, 2H), 5.46-5.56 (m, 1H), 6.42-6.50 (m, 1H), 7.15 (d, 1H), 7.24 (dd, 1H), 7.31 (d, 1H), 7.39 (d, 2H), 7.45 (d, 2H). |
| | | | LCMS (Methode 1): Rₜ = 1.15 min; m/z = 427 (M+H)⁺ |

Analog Beispiel **127.1** wurden aus Beispiel **129A** den entsprechenden kommerziell erhältlichen Aminen folgende Beispielverbindungen hergestellt. Unter den Reaktionsbedingungen wurden vielfach Gemische aus mono- und di-aminierten Produkten erhalten, die chromatographisch getrennt wurden.

| Nr | Struktur | Name | Analytische Daten |
|---|---|---|---|
| **233** | 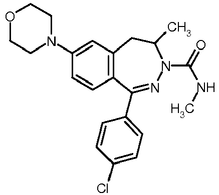 | (±)-1-(4-Chlorphenyl)-N,4-dimethyl-7-(morpholin-4-yl)-4,5-dihydro-3H-2,3-benzodiazepin-3-carboxamid | ¹H-NMR (300MHz, CDCl₃): δ = 1.00 (d, 3H), 2.88 (dd, 1H), 2.90 (d, 3H), 3.16 (dd, 1H), 3.24-3.33 (m, 4H), 3.86-3.95 (m, 4H), 5.44-5.57 (m, 1H), 6.49 (q, 1H), 6.67-6.79 (m, 2H), 6.99 (d, 1H), 7.37 (d, 2H), 7.43 (d, 2H). |
| | | | LCMS (Methode 1): Rₜ = 1.34 min; m/z = 413 (M+H)⁺ |
| **234** | 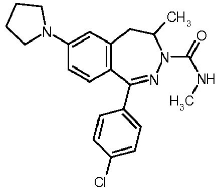 | (±)-1-(4-Chlorphenyl)-N,4-dimethyl-7-(pyrrolidin-1-yl)-4,5-dihydro-3H-2,3-benzodiazepin-3-carboxamid | ¹H-NMR (400MHz, CDCl₃): δ = 1.04 (d, 3H), 2.00-2.11 (m, 4H), 2.88 (dd, 1H), 2.89 (d, 3H), 3.15 (dd, 1H), 3.36 (m, 4H), 5.44-5.55 (m, 1H), 6.35 (dd, 1H), 6.40 (d, 1H), 6.48 (q, 1H), 6.91 (d, 1H), 7.36 (d, 2H), 7.44 (d, 2H). |
| | | | LCMS (Methode 1): Rₜ = 1.56 min; m/z = 397 (M+H)⁺ |
| **235** | 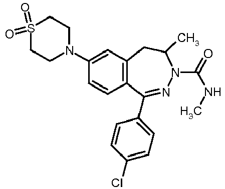 | (±)-1-(4-Chlorphenyl)-7-(1,1-dioxidothiomorpholin-4-yl)-N,4-dimethyl-4,5-dihydro-3H-2,3-benzodiazepin-3-carboxamid | ¹H-NMR (300MHz, CDCl₃): δ = 0.99 (d, 3H), 2.80-3.00 (m, 1H), 2.89 (d, 3H), 3.13 (s, br, 5H), 3.97 (s, br, 4H), 5.43-5.60 (m, 1H), 6.42-6.54 (m, 1H), 6.64-6.79 (m, 2H), 7.02 (d, 1H), 7.33-7.50 (m, 4H). |
| | | | LCMS (Methode 1): Rₜ = 1.21 min; m/z = 461 (M+H)⁺ |
| **236** | 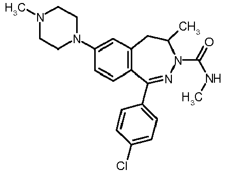 | (±)-1-(4-Chlorphenyl)-N,4-dimethyl-7-(4-methylpiperazin-1-yl)-4,5-dihydro-3H-2,3-benzodiazepin-3-carboxamid | ¹H-NMR (400MHz, CDCl₃): δ = 1.00 (d, 3H), 2.43 (s, 3H), 2.66 (t, 4H), 2.87 (dd, 1H), 2.90 (d, 3H), 3.15 (dd, 1H), 3.37 (t, 4H), 5.44-5.54 (m, 1H), 6.48 (q, 1H), 6.69 (dd, 1H), 6.73 (d, 1H), 6.96 (d, 1H), 7.37 (d, 2H), 7.43 (d, 2H). |
| | | | LCMS (Methode 1): Rₜ = 0.91 min; m/z = 426 (M+H)⁺ |
| **237** | 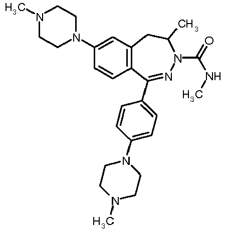 | (±)-N,4-Dimethyl-7-(4-methylpiperazin-1-yl)-1-[4-(4-methylpiperazin-1-yl)phenyl] -4,5-dihydro-3H-2,3-benzodiazepin-3-carboxamid | ¹H-NMR (400MHz, CDCl₃): δ = 1.12 (d, 3H), 2.42 (s, 3H), 2.43 (s, 3H), 2.63-2.71 (m, 8H), 2.77 (dd, 1H), 2.87 (d, 3H), 2.98 (dd, 1H), 3.36 (t, 8H), 5.27-5.37 (m, 1H), 6.13 (q, 1H), 6.71 (dd, 1H), 6.76 (d, 1H), 6.92 (d, 2H), 7.03 (d, 1H), 7.49 (d, 2H). |
| | | | LCMS (Methode 1): Rₜ = 0.55 min; m/z = 490 (M+H)⁺ |
| **238** | 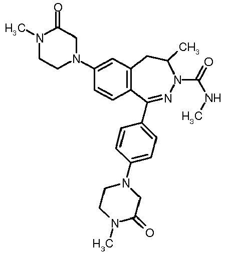 | (±)-N,4-Dimethyl-7-(4-methyl-3-oxopiperazin-1-yl)-1-[4-(4-methyl-3-oxopiperazin-1-yl)phenyl] -4,5-dihydro-3H-2,3-benzodiazepin-3-carboxamid | ¹H-NMR (400MHz, CDCl₃): δ = 1.17 (d, 3H), 2.79 (dd, 1H), 2.86 (d, 3H), 2.98 (dd, 1H), 3.08 (s, 6H), 3.50-3.57 (m, 4H), 3.59-3.67 (m, 4H), 4.00 (s, br, 4H), 5.29-5.39 (m, 1H), 6.15 (q, 1H), 6.67 (dd, 1H), 6.72 (d, 1H), 6.89 (d, 2H), 7.07 (d, 1H), 7.59 (d, 2H). |
| | | | LCMS (Methode 1): Rₜ = 0.87 min; m/z = 518 (M+H)⁺ |

Analog Beispiel 2 wurden aus Beispiel **129A** und den entsprechenden kommerziell erhältlichen Boronosäurederivaten folgende Beispielverbindungen hergestellt:

| Nr | Struktur | Name | Analytische Daten |
|---|---|---|---|
| **239** | 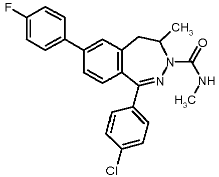 | (±)-1-(4-Chlorphenyl)-7-(4-fluorphenyl)-N,4-dimethyl-4,5-dihydro-3H-2,3-benzodiazepin-3-carboxamid | ¹H-NMR (300MHz, CDCl₃): δ = 1.00 (d, 3H), 2.93 (s, br, 3H), 3.02 (d, 1H), 3.24 (d, 1H), 5.49-5.65 (m, 1H), 6.46-6.62 (m, 1H), 7.13-7.23 (m, 3H), 7.36-7.51 (m, 6H), 7.59 (d, 1H), 7.62 (d, 1H). |
| | | | LCMS (Methode 1): Rₜ = 1.60 min; m/z = 422 (M+H)⁺ |
| **240** | 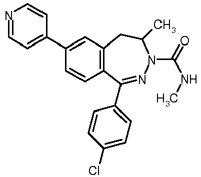 | (±)-1-(4-Chlorphenyl)-N,4-dimethyl-7-(pyridin-4-yl)-4,5-dihydro-3H-2,3-benzodiazepin-3-carboxamid | ¹H-NMR (400MHz, MeOD): δ = 1.06 (d, 3H), 2.82 (s, br, 3H), 3.03 (dd, 1H), 3.22 (dd, 1H), 5.27-5.38 (m, 1H), 7.29 (d, 1H), 7.46 (d, 2H), 7.60-7.68 (m, 3H), 7.81 (d, 1H), 7.91 (s, 1H), 8.17 (s, br, 2H), 8.78 (s, br, 2H). |
| | | | LCMS (Methode 1): Rₜ = 1.07 min; m/z = 405 (M+H)⁺ |
| **241** | 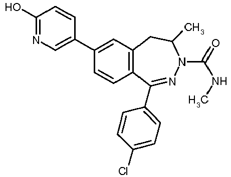 | (±)-1-(4-Chlorphenyl)-7-(6-hydroxypyridin-3-yl)-N,4-dimethyl-4,5-dihydro-3H-2,3-benzodiazepin-3-carboxamid | ¹H-NMR (400MHz, MeOD): δ = 1.06 (d, 3H), 2.81 (s, br, 3H), 2.93 (dd, 1H), 3.13 (d, 1H), 5.21-5.33 (m, 1H), 6.63-6.74 (m, 1H), 7.13 (d, 1H), 7.40-7.49 (m, 3H), 7.53-7.67 (m, 4H), 7.84 (s, br, 1H), 8.05 (s, br, 1H). |
| | | | LCMS (Methode 1): Rₜ = 1.15 min; m/z = 421 (M+H)⁺ |
| **242** | 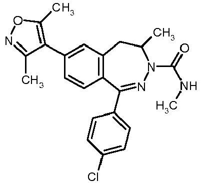 | (±)-1-(4-Chlorphenyl)-7-(3,5-dimethyl-1,2-oxazol-4-yl)-N,4-dimethyl-4,5-dihydro-3H-2,3-benzodiazepin-3-carboxamid | ¹H-NMR (300MHz, CDCl₃): δ = 0.99 (d, 3H), 2.33 (s, 3H), 2.47 (s, 3H), 2.94 (s, br, 3H), 3.01 (d, 1H), 3.22 (d, 1H), 5.49-5.63 (m, 1H), 6.46-6.58 (m, 1H), 7.09-7.22 (m, 3H), 7.41 (d, 2H), 7.48 (d, 2H). |
| | | | LCMS (Methode 1): Rₜ = 1.42 min; m/z = 423 (M+H)⁺ |
| **243** | 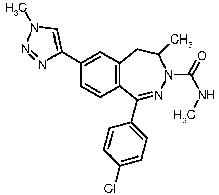 | (±)-1-(4-Chlorphenyl)-N,4-dimethyl-7-(1-methyl-1H-1,2,3-triazol-4-yl)-4,5-dihydro-3H-2,3-benzodiazepin-3-carboxamid | ¹H-NMR (300MHz, CDCl₃): δ = 0.98 (d, 3H), 2.92 (d, 3H), 3.03 (dd, 1H), 3.22 (dd, 1H), 4.20 (s, 3H), 5.48-5.63 (m, 1H), 6.50-6.60 (m, 1H), 7.17 (d, 1H), 7.40 (d, 2H), 7.45 (d, 2H), 7.64 (d, 1H), 7.77 (s, 1H), 7.82 (s, 1H). |
| | | | LCMS (Methode 1): Rₜ = 1.22 min; m/z = 409 (M+H)⁺ |

### Beispiel 130.2

### (4R)-1-[4-(4-Hydroxypiperidin-1-yl)phenyl]-7,8-dimethoxy-N,4-dimethyl-4,5-dihydro-3H-2,3-benzodiazepin-3-carboxamid

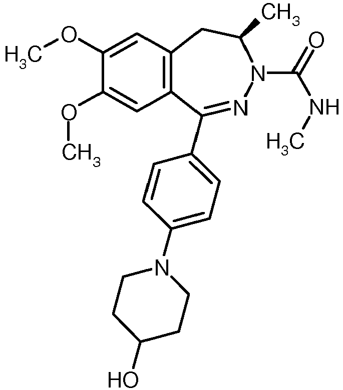

1.0 g (2.3 mmol) (4R)-1-(4-Bromphenyl)-7,8-dimethoxy-N,4-dimethyl-4,5-dihydro-3H-2,3-benzodiazepin-3-carboxamid (Beispiel 49.1A) wurden unter Argon in 25 ml THF vorgelegt. Es wurden 63.5 mg (0.069 mmol) Tris(dibenzylidenaceton)dipalladium (CAS [51364-51-3]) und 91 mg (0.231 mmol) 2'-(Dicyclohexylphosphino)-N,N-dimethylbiphenyl-2-amin (CAS [213697-53-1]) zugegeben und kurz mit Argon entgast. Dann wurden 311 mg (3.24 mmol) Natrium-tert-butanolat und danach 936 mg (9.25 mmol) Piperidin-4-ol zugegeben, erneut entgast und 1 h bei 70°C Ölbadtemperatur gerührt. Der Ansatz wurde nach Abkühlen mit Diatomenerde versetzt, das Lösemittel am Rotationsverdampfer entfernt und der Rückstand flashchromatographisch (Aminophase) gereinigt. Man erhielt 590 mg (55% d. Th.) des gewünschten Produkts als Feststoff.
LCMS (Methode 5): Rₜ = 0.80 min; m/z = 453 (M+H)⁺
¹H-NMR (300MHz, DMSO-*d₆*): δ = 1.05 (d, 3H), 1.36-1.51 (m, 2H), 1.74-1.87 (m, 2H), 2.42 (d, 1H), 2.61 (d, 3H), 2.82 (dd, 1H), 2.96 (t, 2H), 3.59 (s, 3H), 3.61-3.72 (m, 3H), 3.82 (s, 3H), 4.70 (d, 1H), 4.80-4.94 (m, 1H), 6.25 (q, 1H), 6.54 (s, 1H), 6.93 (d, 2H), 7.02 (s, 1H), 7.57 (d, 2H).
Spezifische optische Drehung: [α]_{D}²⁰ = -385.5° +/-0,18° (c = 1,00; Methanol)

Analog Beispiel **130.2** wurden aus Beispiel **49**.**1A** und Methylpiperazin folgende Beispielverbindungen hergestellt:

| Nr | Struktur | Name | Analytische Daten |
|---|---|---|---|
| **127**.**2** | 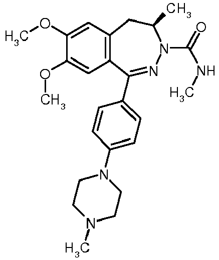 | (4R)-7,8-Dimethoxy-N,4-dimethyl-1-[4-(4-methylpiperazin-1-yl)phenyl]-4,5-dihydro-3H-2,3-benzodiazepin-3-carboxamid | [α]_{D}²⁰ = -361.8° (c = 1.00; MeOH) |
| | | | LCMS (Methode 5): Rₜ = 0.56 min; m/z = 452 (M+H)⁺ |

### Beispiel 244

### (±)-1-[4-(1,1-Dioxido-1,2-thiazolidin-2-yl)phenyl]-7,8-dimethoxy-N,4-dimethyl-4,5-dihydro-3H-2,3-benzodiazepin-3-carboxamid

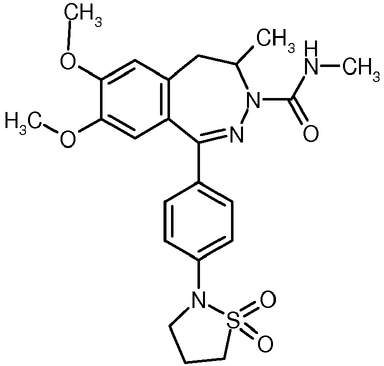

100 mg (0.231 mmol) (±)-1-(4-Bromphenyl)-7,8-dimethoxy-*N*,4-dimethyl-4,5-dihydro-3*H*-2,3-benzodiazepin-3-carboxamid (Beispiel **49A**), 33.6 mg (0.278 mmol) 1,2-Thiazolidin-1,1-dioxid (CAS [5908-62-3]), 64 mg (0.46 mmol) Kaliumcarbonat und 13 mg (0.023 mmol) Allylchloropalladium Dimer (CAS [12012-95-2]) werden in 3 ml 2-Methyltetrahydrofuran vorgelegt und die Suspension 10 min mit Argon entgast. Dann werden 39 mg (0.093 mmol) Ditert-butyl(2',4',6'-triisopropylbiphenyl-2-yl)phosphan (CAS [564483-19-8]) zugegeben, erneut mit Argon entgast und für 16h auf 80°C erhitzt. Das Rohgemisch wird filtriert, dann wird das Lösungsmittel entfernt und der erhaltene Rückstand über präp. HPLC gereinigt. Man erhielt 32 mg (29 % d. Th.) des gewünschten Produkts als Feststoff.
LCMS (Methode 5): Rₜ = 0.90 min; m/z = 473 (M+H)⁺
¹H-NMR (300MHz, CDCl₃): δ = 1.04 (d, 3H), 2.61 (pent, 2H), 2.83 (dd, 1H), 2.91 (d, 3H), 3.09 (dd, 1H), 3.46 (t, 2H), 3.70 (s, 3H), 3.87 (t, 2H), 3.96 (s, 3H), 5.37-5.50 (m, 1H), 6.42 (q, 1H), 6.62 (s, 1H), 6.75 (s, 1H), 7.30 (d, 2H), 7.55 (d, 2H).

### Beispiel 245

### (±)-1-{7,8-Dimethoxy-4-methyl-1-[4-(4-methylpiperazin-1-yl)phenyl] -4,5-dihydro-3H-2,3-benzodiazepin-3-yl}ethanon

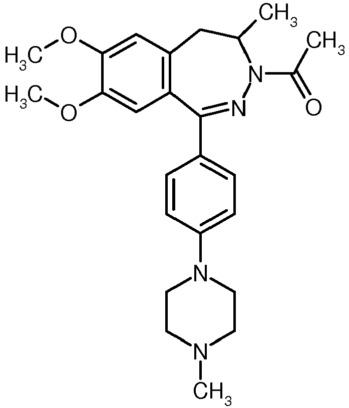

Unter analoger Verwendung des Verfahrens zur Herstellung von Beispiel **127.1** wurde aus (±)-1-[1-(4-Bromphenyl)-7,8-dimethoxy-4-methyl-4,5-dihydro-3H-2,3-benzodiazepin-3-yl]ethanon **(**Beispiel **133A**) die gewünschte Verbindung erhalten.
LCMS (Methode 1): Rt = 0,70 min; m/z = 437 (M+H)⁺
¹H-NMR (300 MHz, CDCl₃): δ = 1,33 (d, 3H), 2,07 (s, 3H), 2,39 (s, 3H), 2,59 - 2,62 (m, 4H), 2,65 - 2,82 (m, 2H), 3,35 - 3,38 (m, 4H), 3,77 (s, 3H), 3,96 (s, 3H), 5,23-5,36 (m, 1H), 6,65 (s, 1H), 6,82 (s, 1H), 6,96 (d, 2H), 7,64 (d, 2H).

### Enantiomerentrennung

Präparative HPLC nach Methode VIa

### Beispiel 245.1: 1-{(4S)-7,8-Dimethoxy-4-methyl-1-[4-(4-methylpiperazin-1-yl)phenyl]-4,5-dihydro-3H-2,3-benzodiazepin-3-yl}ethanon (Enantiomer 1)

HPLC (Methode Ea): Rₜ = 5.38 min, Reinheit >99%
Spezifische optische Drehung: [α]_{D}²⁰ = 225.3° +/-0.33° (c = 1,00; Methanol)

### Enantiomer 2: 1-{(4R)-7,8-Dimethoxy-4-methyl-1-[4-(4-methylpiperazin-1-yl)phenyl]-4,5-dihydro-3H-2,3-benzodiazepin-3-yl}ethanon

HPLC (Methode Ea): Rₜ = 4.13 min, Reinheit >99%
Spezifische optische Drehung: [α]_{D}²⁰ = -201.4° +/-0.27° (c = 1,00; Methanol)

Analog Beispiel 2 wurden aus Beispiel **133A** und dem entsprechend kommerziell erhältlichen Boronsäurederivat folgende Beispielverbindung hergestellt:

| | | | |
|---|---|---|---|
| **246** | 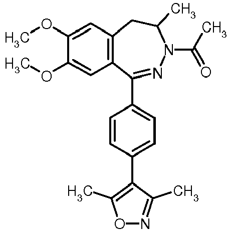 | (±)-1-{1-[4-(3,5-Dimethyl-1,2-oxazol-4-yl)phenyl]-7,8-dimethoxy-4-methyl-4,5-dihydro-3H-2,3-benzodiazepin-3-yl}ethanon | ¹H-NMR (300MHz, CDCl₃): δ = 1.24 (d, 3H), 2.23 (s, 3H), 2.35 (s, 3H), 2.49 (s, 3H), 2.78 (dd, 1H), 2.94 (dd, 1H), 3.77 (s, 3H), 3.98 (s, 2H), 5.32 - 5.44 (m, 1H), 6.66 (s, 1H), 6.83 (s, 1H), 7.36 (d, 2H), 7.77 (d, 2H). |
| | | | LCMS (Methode 1): Rₜ = 1.19 min; m/z = 434 (M+H)⁺ |

### Beispiel 247

### (±)-7,8-Dimethoxy-N,4-dimethyl-1-[4-(5-methyl-1,3,4-oxadiazol-2-yl)phenyl]-4,5-dihydro-3H-2,3-benzodiazepin-3-carboxamid

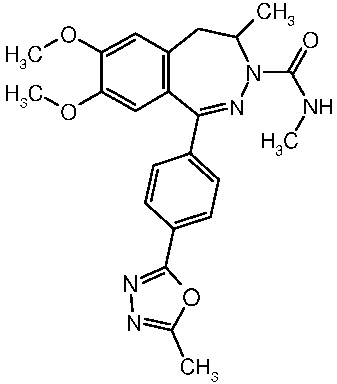

Zu einer Lösung von 163 mg (410 µmol) (±)-4-[7,8-Dimethoxy-4-methyl-3-[(methylamino)car-bonyl]-4,5-dihydro-3*H*-2,3-benzodiazepin-1-yl]benzoesäure (Beispiel **134A**) in 10 ml *N,N-*Dimethylformamid wurden bei 0°C 69,9 mg (431 µmol) *N*,*N'*-Carbonyldiimidazol gegeben. Nach 4,5 Stunden wurden 46 mg (616 µmol) Acethydrazid hinzugegeben, und die Mischung über Nacht bei 80°C gerührt. Zur Aufarbeitung wurde mit Wasser versetzt und dreimal mit Ethylacetat extrahiert. Die organische Phase wurde mit gesättigter wässriger Natriumchloridlösung und mit Wasser gewaschen, mit Natriumsulfat getrocknet und das Lösemittel am Rotationsverdampfer entfernt. Das Rohprodukt wurde sodann in 10 ml Dichlormethan gelöst und bei 0°C mit 23 mg (331 µmol) 1*H*-Imidazol, 76 mg (291 µmol) Triphenylphosphin sowie 97 mg (291 µmol) Tetrabromkohlenstoff versetzt. Dieses Gemisch wurde über Nacht bei Raumtemperatur gerührt. Zur Aufarbeitung wurde mit Wasser versetzt und dreimal mit Ethylacetat extrahiert. Die organische Phase wurde mit gesättigter wässriger Natriumchloridlösung gewaschen, mit Natriumsulfat getrocknet und das Lösemittel am Rotationsverdampfer entfernt. Nach Chromatographie wurden 10 mg der Titelverbindung als gelber Film erhalten.
LCMS (Methode 3): Rₜ = 1,01 min; m/z = 436 (M+H)⁺
¹H-NMR (400 MHz, CDCl₃): δ = 0,96 (d, 3H), 2,64 (s, 3H), 2,88 (dd, 1H), 2,91 (d, 3H), 3,16 (dd, 1H), 3,64 (s, 3H), 3,94 (s, 3H), 5,50 (m, 1H), 6,55 (s, 1H), 6,58 (sbr, 1H), 6,72 (s, 1H), 7,62 (d, 2H), 8,05 (d, 2H).

### Beispiel 248

### (±)-7,8-Dimethoxy-N,4-dimethyl-1-[4-(3-methyl-1,2,4-oxadiazol-5-yl)phenyl]-4,5-dihydro-3H-2,3-benzodiazepin-3-carboxamid

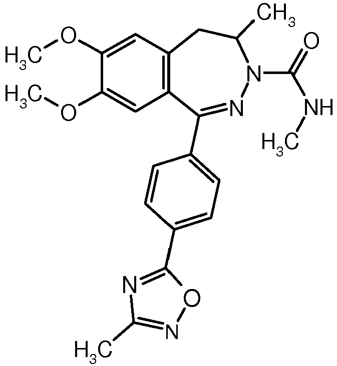

Eine Lösung von 175 mg (440 µmol) (±)-4-[7,8-Dimethoxy-4-methyl-3-[(methylamino)carbonyl]-4,5-dihydro-3*H*-2,3-benzodiazepin-1-yl]benzoesäure (Beispiel **134A**), 49 mg (661 µmol) *N*'-Hydroxyethanimidamid (CAS [22059-22-9]), 184 µl (1,32 mmol) Triethylamin und 643 µl (1,10 mmol) 1-Propanphosphonsaeure-*cyclo*-anhydrid (CAS [68957-94-8]) in 20 ml Ethylacetat wurde über 48 Stunden auf 80°C erwärmt. Zur Aufarbeitung wurde mit gesättigter wässriger Natriumhydrogencarbonatlösung versetzt und dreimal mit Ethylacetat extrahiert. Die organische Phase wurde mit gesättigter wässriger Natriumhydrogencarbonatlösung gewaschen, mit Natriumsulfat getrocknet und das Lösemittel am Rotationsverdampfer entfernt. Nach Chromatographie wurden 26 mg (13% d.Th.) der Titelverbindung als gelber Schaum erhalten.
LCMS (Methode 3): Rₜ = 1,21 min; m/z = 436 (M+H)⁺
¹H-NMR (400 MHz, CDCl₃): δ = 0,96 (d, 3H), 2,50 (s, 3H), 2,89 (dd, 1H), 2,91 (d, 3H), 3,17 (dd, 1H), 3,64 (s, 3H), 3,94 (s, 3H), 5,52 (m, 1H), 6,55 (s, 1H), 6,57 (q, 1H), 6,72 (s, 1H), 7,63 (d, 2H), 8,14 (d, 2H).

Unter Verwendung des Verfahrens zur Herstellung von Beispiel 155 wurde Beispiel 249 aus Beispiel 1 hergestellt. Nachfolgend wurde per präparativer chiraler HPLC in die Enantiomere getrennt.

| Nr | Struktur | Name | Analytische Daten |
|---|---|---|---|
| **249** | 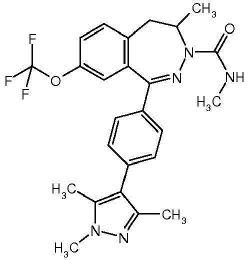 | (±)-*N*,4-Dimethyl-8-(trifluormethoxy)-1-[4-(1,3,5-trimethyl-1*H*-pyrazol-4-yl)phenyl]-4,5-dihydro-3*H*-2,3-benzodiazepin-3-carboxamid | ¹H-NMR (400 MHz, CDCl₃): δ = 0,99 (d, 3H), 2,30 (s, 6H), 2,91 (d, 3H), 2,92 (dd, 1H), 3,13 (dd, 1H), 3,80 (s, 3H), 5,46 (m, 1H), 6,47 (q, 1H), 7,06 (d, 1H), 7,21 (dd, 1H), 7,29 (d, 1H), 7,29 (d, 2H), 7,51 (d, 2H). |
| | | | LCMS (Methode 3): Rₜ = 1,34 min; m/z = 486 (M+H)⁺. |
| **249.1** | 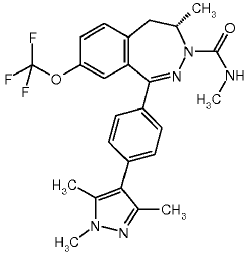 | (4*S*)-*N*,4-Dimethyl-8-(trifluormethoxy)-1-[4-(1,3,5-trimethyl-1*H*-pyrazol-4-yl)phenyl]-4,5-dihydro-3*H*-2,3-benzodiazepin-3-carboxamid | Präp. HPLC: Methode XVIa |
| | | | Analyt. HPLC (Methode Ca): Rₜ = 4.77 min |
| | | | [α]_{D}²⁰ = 198.1° (c = 1.00; MeOH) |
| **249.2** | 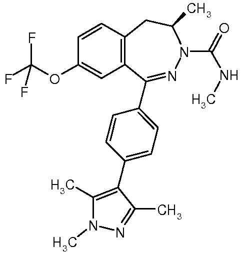 | (4*R*)-*N*,4-Dimethyl-8-(trifluormethoxy)-1-[4-(1,3,5-trimethyl-1*H*-pyrazol-4-yl)phenyl]-4,5-dihydro-3*H*-2,3-benzodiazepin-3-carboxamid | Präp. HPLC: Methode XVIa |
| | | | Analyt. HPLC (Methode Ca): Rₜ = 7.21 min |
| | | | [α]_{D}²⁰ = -189.7° (c = 1.00; MeOH) |

Unter Verwendung des Verfahrens zur Herstellung von Beispiel 171, gegebenenfalls mit nachfolgender Enantiomerentrennung durch chirale präparative HPLC, wurden folgende Verbindungen aus Beispiel 1 hergestellt:

| Nr | Struktur | Name | Analytische Daten |
|---|---|---|---|
| 250 | 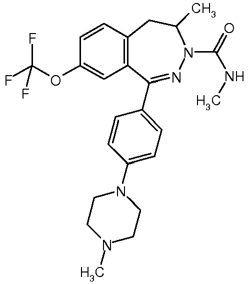 | (±)-*N*,4-Dimethyl-1-[4-(4-methyl,-1-piperazinyl)phenyl]-8-(trifluormethoxy)-4,5-dihydro-3*H*-2,3-benzodiazepin-3-carboxamid | ¹H-NMR (300 MHz, CDCl₃): δ = 1,07 (d, 3H), 2,37 (s, 3H), 2,58 (m, 4H), 2,79 (dd, 1H), 2,87 (d, 3H), 3,00 (dd, 1H), 3,32 (m, 4H), 5,30 (m, 1H), 6,12 (q, 1H), 6,91 (d, 2H), 7,02 (d, 1H), 7,19 (dd, 1H), 7,26 (d, 1H), 7,45 (d, 2H). |
| | | | LCMS (Methode 3): Rₜ = 0,88 min; m/z = 476 (M+H)⁺. |
| **250.1** | 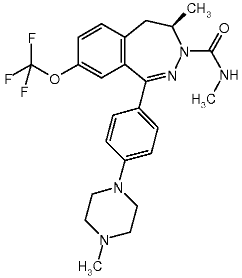 | (4*R*)-*N*,4-Dimethyl-1-[4-(4-methyl,-1-piperazinyl)phenyl] -8-(trifluormethoxy)-4,5-dihydro-3*H*-2,3-benzodiazepin-3-carboxamid | Präp. HPLC: Methode XXb |
| | | | Analyt. HPLC (Methode Ra): Rₜ = 1.88 min |
| | | | [α]_{D}²⁰ = -442.8 (c = 1.00; MeOH) |
| **250.2** | 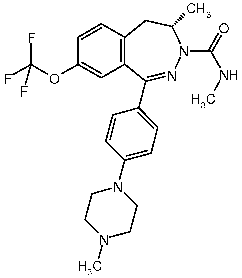 | *(4S)*-*N*,4-Dimethyl-1-[4-(4-methyl-1-piperazinyl)phenyl] -8-(trifluormethoxy)-4,5-dihydro-3*H*-2,3-benzodiazepin-3-carboxamid | Präp. HPLC: Methode XXb |
| | | | Analyt. HPLC (Methode Ra): Rₜ = 2.32 min |
| | | | [α]_{D}²⁰ = 428.6° (c = 1.00; MeOH) |
| 251 | 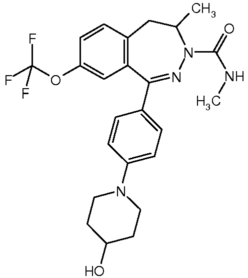 | (±)-1-[4-(4-Hydroxy-1-piperidinyl)phenyl]-*N*,4-dimethyl-8-(trifluormethoxy)-4,5-dihydro-3*H*-2,3-benzodiazepin-3-carboxamid | ¹H-NMR (300 MHz, CDCl₃): δ = 1,08 (d, 3H), 1,69 (m, 2H), 2,02 (m, 2H), 2,78 (dd, 1H), 2,86 (d, 3H), 2,99 (dd, 1H), 3,06 (m, 2H), 3,69 (m, 2H), 3,92 (m, 1H), 5,28 (m, 1H), 6,08 (q, 1H), 6,92 (d, 2H), 7,02 (d, 1H), 7,19 (dd, 1H), 7,27 (d, 1H), 7,45 (d, 2H). |
| | | | LCMS (Methode 3): Rₜ = 0,88 min; m/z = 476 (M+H)⁺. |

### Beispiel 252

### (±)-1-[2,4-Dibrom-5-(4-methylpiperazin-1-yl)phenyl] -7,8-dimethoxy-N,4-dimethyl-4,5-dihydro-3H-2,3-benzodiazepin-3-carboxamid

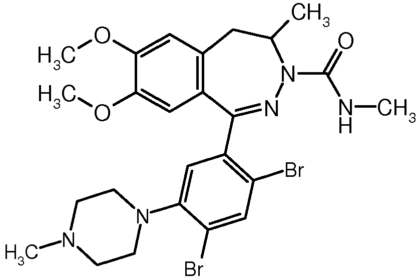

Die Verbindung wurde analog Beispiel **95A** aus (±)-7,8-Dimethoxy-*N*,4-dimethyl-1-[3-(4-methylpiperazin-1-yl)phenyl]-4,5-dihydro-3*H*-2,3-benzodiazepin-3-carboxamid (Beispiel 143) hergestellt.
LCMS (Methode 3): Rₜ = 0,94 min; m/z = 608, 610, 612 (Br₂-Isotopenmuster, M+H)
¹H-NMR (300 MHz, CDCl₃): δ = 0,96 (d, 3H), 2,85 (d, 3H), 2,96 (s, 3H), 2,97 (dd, 1H), 3,22 (dd, 1H), 3,45 (mbr, 8H), 3,61 (s, 3H), 3,92 (s, 3H), 5,63 (m, 1H), 6,25 (s, 1H), 6,52 (q, 1H), 6,71 (s, 1H), 7,08 (s, 1H), 7,85 (s, 1H).

### Beispiel 253

### (4S)-1-[3-Brom-4-(4-methylpiperazin-1-yl)phenyl]-7,8-dimethoxy-N,4-dimethyl-4,5-dihydro-3H-2,3-benzodiazepin-3-carboxamid

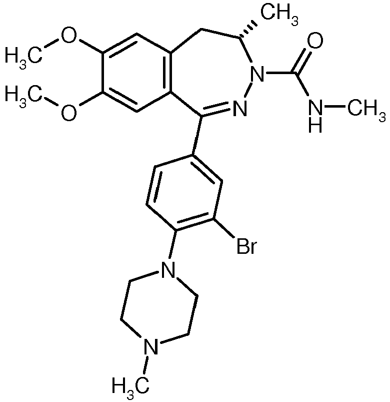

Die Verbindung wurde analog Beispiel **95A** aus (4*S*)-7,8-Dimethoxy-N,4-dimethyl-1-[4-(4-methylpiperazin-1-yl)phenyl]-4,5-dihydro-3H-2,3-benzodiazepin-3-carboxamid (Beispiel **127.1**) hergestellt.
LCMS (Methode 3): Rₜ = 0.78 min; m/z = 529, 532 (Br-Isotopenmuster, M+H)
¹H-NMR (300 MHz, CDCl₃): δ = 1.02 (d, 3H), 2.42 (s, 3H), 2.60-2.76 (m, 4H), 2.82 (dd, 1H), 2.91 (d, 3H), 3.07 (dd, 1H), 3.12-3.26 (m, 4H), 3.71 (s, 3H), 3.96 (s, 3H), 5.36-5.49 (m, 1H), 6.38 (q, 1H), 6.62 (s, 1H), 6.75 (s, 1H), 7.08 (d, 1H), 7.43 (dd, 1H), 7.76 (d, 1H).

### Beispiel 254

### (4S)-1-[3-Cyan-4-(4-methylpiperazin-1-yl)phenyl]-7,8-dimethoxy-N,4-dimethyl-4,5-dihydro-3H-2,3-benzodiazepin-3-carboxamid

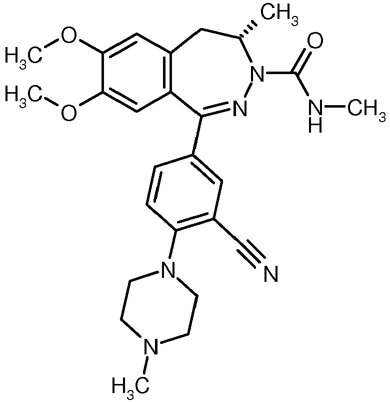

Die Verbindung wurde analog Beispiel **96A** aus (4S)-1-[3-Brom-4-(4-methylpiperazin-1-yl)phenyl]-7,8-dimethoxy-N,4-dimethyl-4,5-dihydro-3H-2,3-benzodiazepin-3-carboxamid (Beispiel 253) hergestellt.
LCMS (Methode 3): Rt = 0.72 min; m/z = 477 (M+H)⁺.
¹H-NMR (400 MHz, CDCl₃): δ = 1.01 (d, 3H), 2.41 (s, 3H), 2.68 (t, 4H), 2.83 (dd, 1H), 2.92 (d, 3H), 3.09 (dd, 1H), 3.32-3.42 (m, 4H), 3.72 (s, 3H), 3.96 (s, 3H), 5.40-5.50 (m, 1H), 6.37 (q, 1H), 6.56 (s, 1H), 6.75 (s, 1H), 7.02 (d, 1H), 7.61 (dd, 1H), 7.76 (d, 1H).
Spezifische optische Drehung: [α]_{D}²⁰ = 154.6° +/-0.28° (c = 1,00; Methanol)

Unter Verwendung des Verfahrens zur Herstellung von Beispiel **88A** wurden folgende Verbindungen aus Beispiel **136A** hergestellt:

| Nr | Struktur | Name | Analytische Daten |
|---|---|---|---|
| **255** | 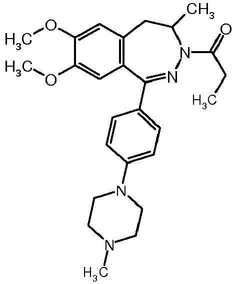 | (±)-7,8-Dimethoxy-4-methyl-1-[4-(4-methylpiperazin-1-yl)phenyl]-3-(1-oxopropyl)-4,5-dihydro-3*H*-2,3-benzodiazepin | ¹H-NMR (400MHz, DMSO-*d*₆): δ = 0,89 (t, 3H), 1,15 (d, 3H), 2,13 (m, 1H), 2,22 (s, 3H), 2,34 (m, 1H), 2,44 (m, 4H), 2,49 (m, 1H), 2,84 (dd, 1H), 3,27 (m, 4H), 3,62 (s, 3H), 3,84 (s, 3H), 5,00 (m, 1H), 6,60 (s, 1H), 6,99 (d, 2H), 7,05 (s, 1H), 7,51 (d, 2H). |
| | | | LCMS (Methode 3): Rₜ = 0,75 min; m/z = 451 (M+H)⁺. |
| **256** | 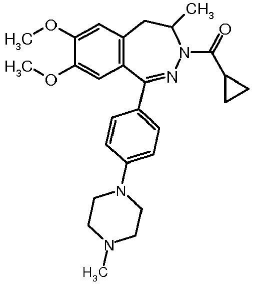 | (±)-3-(Cyclopropylcarbonyl )-7,8-dimethoxy-4-methyl-1-[4-(4-methylpiperazin-1-yl)phenyl]-4,5-dihydro-3*H*-2,3-benzodiazepin | ¹H-NMR (400MHz, DMSO-*d*₆): δ = 0,57 (m, 1H), 0,65 (m, 1H), 0,74 (m, 2H), 1,15 (d, 3H), 2,22 (s, 3H), 2,44 (m, 4H), 2,50 (m, 1H), 2,84 (dd, 1H), 3,27 (m, 4H), 3,63 (s, 3H), 3,84 (s, 3H), 5,01 (m, 1H), 6,62 (s, 1H), 6,99 (d, 2H), 7,05 (s, 1H), 7,54 (d, 2H). |
| | | | LCMS (Methode 3): Rₜ = 0,74 min; m/z = 463 (M+H)⁺. |

Unter Verwendung des Verfahrens zur Herstellung von Beispiel **49A** wurde mit Cyclopropylamin folgende Verbindung aus Beispiel **136A** hergestellt:

| Nr | Struktur | Name | Analytische Daten |
|---|---|---|---|
| **257** | 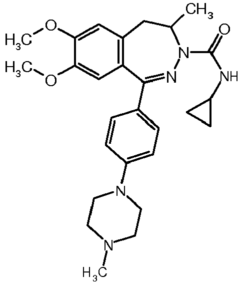 | (±)-*N*-Cyclopropyl-7,8-dimethoxy-4-methyl-1-[4-(4-methylpiperazin-1-yl)phenyl]-4,5-dihydro-3*H*-2,3-benzodiazepin | ¹H-NMR (300MHz, DMSO-*d*₆): δ = 0,45 (m, 2H), 0,56 (m, 2H), 1,05 (d, 3H), 2,22 (s, 3H), 2,39 (m, 1H), 2,44 (m, 4H), 2,47 (m, 1H), 2,85 (dd, 1H), 3,24 (m, 4H), 3,69 (s, 3H), 3,83 (s, 3H), 4,90 (m, 1H), 6,29 (d, 1H), 6,56 (s, 1H), 6,95 (d, 2H), 7,02 (s, 1H), 7,51 (d, 2H). |
| | | | LCMS (Methode 3): Rₜ = 1,12 min; m/z = 478 (M+H)⁺. |

### Beispiel 258

### (±)-7,8-Dimethoxy-N,4-dimethyl-1-[4-(4-methylpiperazin-1-yl)phenyl]-4,5-dihydro-3H-2,3-benzodiazepin-3-carbothioamid

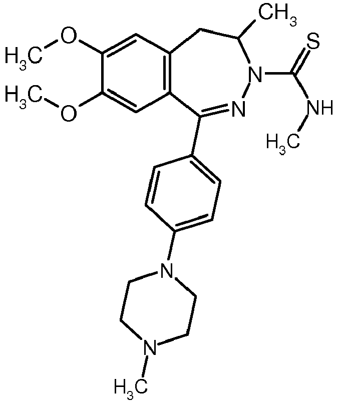

Eine Lösung von 100 mg (215 µmol) (±)-7,8-Dimethoxy-4-methyl-1-[4-(4-methylpiperazin-1-yl)phenyl]-4,5-dihydro-3*H*-2,3-benzodiazepin (Beispiel **136A**), 79 mg (1077 µmol) Methylisothiocyanat und 37,5 µl (215 µmol) Triethylamin in 2,5 ml Tetrahydrofuran wurde über Nacht zum Rückfluß erhitzt. Zur Aufarbeitung wurde mit gesättigter wässriger Ammoniumcarbonatlösung versetzt und und dreimal mit Ethylacetat extrahiert. Die organische Phase wurde mit gesättigter wässriger Ammoniumcarbonatlösung und mit Wasser gewaschen, mit Natriumsulfat getrocknet und das Lösemittel am Rotationsverdampfer entfernt. Nach Chromatographie wurden 77 mg (69% d.Th.) der Titelverbindung als gelber Schaum erhalten.
LCMS (Methode 3): Rₜ = 0,85 min; m/z = 468 (M+H)⁺
¹H-NMR (400MHz, DMSO-*d*₆): δ = 1,24 (d, 3H), 2,22 (s, 3H), 2,44 (m, 4H), 2,47 (m, 1H), 2,86 (m, 1H), 2,89 (d, 3H), 3,29 (m, 4H), 3,63 (s, 3H), 3,84 (s, 3H), 5,73 (m, 1H), 6,58 (s, 1H), 6,97 (d, 2H), 7,09 (s, 1H), 7,18 (q, 1H), 7,68 (d, 2H).

### Beispiel 259

### Methyl-(±)-7,8-dimethoxy-4-methyl-1-[4-(4-methylpiperazin-1-yl)phenyl]-4,5-dihydro-3H-2,3-benzodiazepin-3-carboxylat

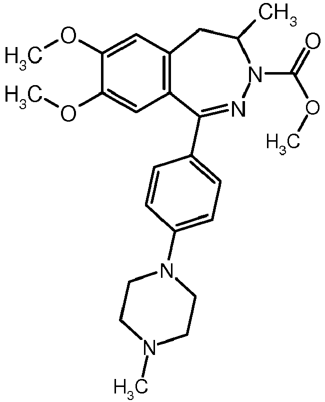

Eine Lösung von 100 mg (215 µmol) (±)-7,8-Dimethoxy-4-methyl-1-[4-(4-methylpiperazin-1-yl)phenyl]-4,5-dihydro-3*H*-2,3-benzodiazepin (Beispiel **136A**) und 50 µl (646 µmol) Chlorameisensäuremethylester in 4 ml Dichlormethan wurde eine Stunde auf 40°C erwärmt. Zur Aufarbeitung wurde mit gesättigter wässriger Natriumhydrogencarbonatlösung versetzt und dreimal mit Dichlormethan extrahiert. Die organische Phase wurde mit gesättigter wässriger Natriumhydrogencarbonatlösung und mit Wasser gewaschen, mit Natriumsulfat getrocknet und das Lösemittel am Rotationsverdampfer entfernt. Nach Chromatographie wurden 53 mg (52% d.Th.) der Titelverbindung als gelber Schaum erhalten.
LCMS (Methode 3): Rₜ = 0,78 min; m/z = 453 (M+H)⁺
¹H-NMR (300MHz, DMSO-*d*₆): δ = 1,21 (d, 3H), 2,22 (s, 3H), 2,44 (m, 4H), 2,39 (m, 1H), 2,82 (dd, 1H), 3,55 (s, 3H), 3,26 (m, 4H), 3,63 (s, 3H), 3,84 (s, 3H), 4,69 (m, 1H), 6,58 (s, 1H), 6,98 (d, 2H), 7,06 (s, 1H), 7,50 (d, 2H).

Analog Beispiel 259 wurde folgende Verbindung aus Beispiel **136A** mit Chlorameisensäureethylester hergestellt:

| Nr | Struktur | Name | Analytische Daten |
|---|---|---|---|
| **260** | 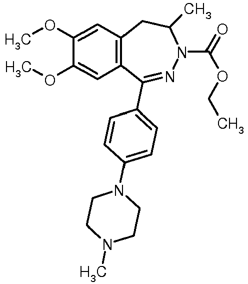 | Ethyl-(±)-7,8-dimethoxy-4-methyl-1-[4-(4-methylpiperazin-1-yl)phenyl]-4,5-dihydro-3*H*-2,3-benzodiazepin-3-carboxylat | ¹H-NMR (300MHz, DMSO-*d*₆): δ = 1,15 (t, 3H), 1,21 (d, 3H), 2,22 (s, 3H), 2,44 (m, 4H), 2,40 (m, 1H), 2,82 (dd, 1H), 3,26 (m, 4H), 3,63 (s, 3H), 3,84 (s, 3H), 3,99 (q, 2H), 4,69 (m, 1H), 6,58 (s, 1H), 6,98 (d, 2H), 7,06 (s, 1H), 7,50 (d, 2H). |
| | | | LCMS (Methode 3): Rₜ = 0,85 min; m/z = 467 (M+H)⁺. |

### Beispiel 261

### (±)-N-Ethyl-7,8-dimethoxy-4-methyl-1-[4-(4-methylpiperazin-1-yl)phenyl]-4,5-dihydro-3H-2,3-benzodiazepin-3-carboxamid

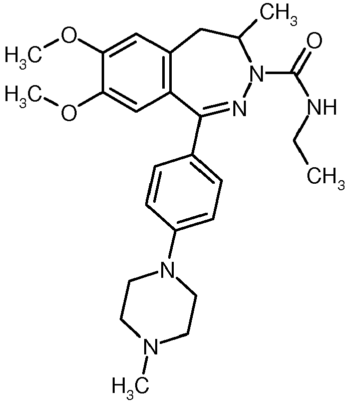

Unter Verwendung des Verfahrens zur Herstellung von Beispiel **127.1** wurde Beispiel 261 aus dem Intermediat **137A** hergestellt.
LCMS (Methode 5): Rₜ = 0.62 min; m/z = 466 (M+H)⁺
¹H-NMR (400MHz, CDCl₃): δ = 1.13 (d, 3H), 1.20 (t, 3H), 2.39 (s, 3H), 2.58-2.64 (m, 4H), 2.74 (dd, 1H), 2.97 (dd, 1H), 3.27-3.40 (m, 6H), 3.73 (s, 3H), 3.96 (s, 3H), 5.25-5.35 (m, 1H), 6.20 (t, 1H), 6.68 (s, 1H), 6.77 (s, 1H), 6.94 (d, 2H), 7.51 (d, 2H).

### Enantiomerentrennung

56 mg (±)-N-Ethyl-7,8-dimethoxy-4-methyl-1-[4-(4-methylpiperazin-1-yl)phenyl]-4,5-dihydro-3H-2,3-benzodiazepin-3-carboxamid (Beispiel X) wurden mittels chiraler präparativer HPLC mit der Methode Va in die Enantiomere getrennt. Dabei erhielt man folgendes Ausführungsbeispiel:

### Beispiel 261.1: (4S)-N-Ethyl-7,8-dimethoxy-4-methyl-1-[4-(4-methylpiperazin-1-yl)phenyl]-4,5-dihydro-3H-2,3-benzodiazepin-3-carboxamid

22 mg Feststoff, Analyt. HPLC (Methode Ua): Rₜ = 7.53 min, Reinheit 99%
[α]_{D}²⁰ = 384.1° (c = 1.00; MeOH)

### Biologische Wirksamkeit der erfindungsgemäßen Verbindungen

### 1. Assays

### 1.1 Protein-Protein Wechsetwirkunssassay

### Bindungsassay BRD4 / acetyliertes Peptid H4 ("PRQ")

Zur Beurteilung der BRD4-Bindungsstärke der in dieser Anmeldung beschriebenen Substanzen wurde deren Fähigkeit quantifiziert, die Wechselwirkung zwischen BRD4 (BD1) und acetyliertem Histon H4 dosisabhängig zu hemmen (Filippakopoulos et al., Cell, 2012, 149:214-231).

Zu diesem Zweck wurde ein zeitaufgelöster Fluoreszenz-Resonanz-Energie-Transfer (TR-FRET) Assay verwendet, der die Bindung zwischen N-terminal His₆-getaggtem BRD4 (BD1) (Aminosäuren 67-152, wobei auch längere Konstrukte möglich sind, vorzugsweise Aminosäuren 44-168) und einem synthetischen acetylierten Histon H4 (Ac-H4) Peptid mit Sequenz GRGK(Ac)GGK(Ac)GLGK(Ac)GGAK(Ac)RHGSGSK-Biotin misst. Das nach Filippakopoulos et al., Cell, 2012, 149:214-231 produzierte rekombinante BRD4 Protein wurde in *E. coli* exprimiert und mittels (Ni-NTA) Affinitäts- und (Sephadex G-75) Größenausschlusschromatografie gereinigt. Das Ac-H4 Peptid kann von z.B. Biosyntan (Berlin, Deutschland) gekauft werden. Im Assay wurden typischerweise 11 verschiedene Konzentrationen von jeder Substanz (0.1 nM, 0.33 nM, 1.1 nM, 3.8 nM, 13 nM, 44 nM, 0.15 µM, 0.51 µM, 1.7 µM, 5.9 µM und 20 µM) als Duplikate auf derselben Mikrotiter-Platte gemessen. Dafür wurden 100-fach konzentrierte Lösungen in DMSO vorbereitet durch serielle Verdünnungen (1:3.4) einer 2 mM Stammlösung in eine klare, 384-Well Mikrotiter-Platte (Greiner Bio-One, Frickenhausen, Germany). Daraus wurden 50 nl in eine schwarze Testplatte (Greiner Bio-One, Frickenhausen, Germany) überführt. Der Test wurde gestartet durch die Zufuhr von 2 µl einer 2.5-fach konzentrierten BRD4-Lösung (üblicherweise 10 nM Endkonzentration in den 5 µl des Reaktionsvolums) in wässrigem Assaypuffer [50 mM HEPES pH 7.5, 50 mM Natriumchlorid (NaCl), 0.25 mM CHAPS und 0.05% Serumalbumin (BSA)] zu den Substanzen in der Testplatte. Darauf folgte ein 10-minütiger Inkubationsschritt bei 22°C für die Voräquilibrierung von putativen Komplexen zwischen BRD4 und den Substanzen. Anschließend wurden 3 µl einer 1.67-fach konzentrierten Lösung (im Assaypuffer) bestehend aus Ac-H4 Peptid (83.5 nM) und TR-FRET Detektionsreagenzien [16.7 nM Anti-6His-XL665 und 3.34 nM Streptavidin-Kryptat (beide von Cisbio Bioassays, Codolet, France), so wie 668 mM Kaliumfluorid (KF)] zugegeben.

Die Mischung wurde dann im Dunkeln für eine Stunde bei 22°C und anschließend für mindestens 3 Stunden und maximal über Nacht bei 4°C inkubiert. Die Bildung von BRD4 / Ac-H4 Komplexen wurde bestimmt durch die Messung des Resonanzenergietransfers von dem Streptavidin-EuKryptat zum anti-6His-XL665 Antikörper der sich in der Reaktion befindet. Dafür wurden die Fluoreszenzemission bei 620 nm und 665 nm nach Anregung bei 330-350 nm in einem TR-FRET Messgerät, z.B. ein Rubystar oder Pherastar (beide von BMG Lab Technologies, Offenburg, Germany) oder ein Viewlux (Perkin-Elmer), gemessen. Das Verhältnis der Emission bei 665 nm und bei 622 nm (Ratio) wurde als Indikator für die Menge der gebildeten BRD4/Ac-H4 Komplexe genommen.

Die erhaltenen Daten (Ratio) wurden normalisiert, wobei 0% Inhibition dem Mittelwert aus den Messwerten eines Satzes von Kontrollen (üblicherweise 32 Datenpunkte) entsprach, bei denen alle Reagenzien enthalten waren. Dabei wurden anstatt von Testsubstanzen 50 nl DMSO (100%) eingesetzt. Inhibition von 100% entsprach dem Mittelwert aus den Messwerten eines Satzes von Kontrollen (üblicherweise 32 Datenpunkte), bei denen alle Reagenzien außer BRD4 enthalten waren. Die Bestimmung des IC₅₀ Wertes erfolgte durch Regressionsanalyse auf Basis einer 4-Parameter Gleichung (Minimum, Maximum, IC₅₀, Hill; Y = Max + (Min - Max) / (1 + (X/IC₅₀)^{Hill})).

### 1.2 Zell-Assays

### Zellproliferationsassays

In Übereinstimmung mit der Erfindung, wurde die Fähigkeit der Substanzen die Zellproliferation zu hemmen bestimmt. Die Zellviabilität wurde mittels des alamarBlue® Reagenz (Invitrogen) in einem Victor X3 Multilabel Reader (Perkin Elmer) bestimmt. Die Anregungswellenlänge war 530 nm und die Emissionswellenlänge 590 nM.

Die MOLM-13-Zellen (DSMZ, ACC 554) wurden zu einer Konzentration von 4000 Zellen/Well in 100µl Wachstumsmedium (RPMI1640, 10% FCS) auf 96well Microtiterplatten ausgesät.

Die MV4-11-Zellen (ATCC, CRL 9591) wurden zu einer Konzentration von 5000 Zellen/Well in 100µl Wachstumsmedium (RPMI1640, 10% FCS) auf 96well Microtiterplatten ausgesät.

Die B16F10-Zellen (ATCC, CRL-6475) wurden zu einer Konzentration von 300-500 Zellen/Well in 100µl Wachstumsmedium (DMEM mit Phenolrot, 10% FCS) auf 96well Microtiterplatten ausgesät.

Die LOX-IMVI-Zellen (NCI-60) wurden zu einer Konzentration von 1000 Zellen/Well in 100µl Wachstumsmedium (RPMI1640, 10% FCS) auf 96well Microtiterplatten ausgesät.

Die MOLP-8-Zellen (DSMZ, ACC 569) wurden zu einer Konzentration von 4000 Zellen/Well in 100µl Wachstumsmedium (RPMI1640, 20% FCS) auf 96well Microtiterplatten ausgesät.

Die KMS-12-PE-Zellen (DSMZ, ACC 606) wurden zu einer Konzentration von 4000 Zellen/Well in 100µl Wachstumsmedium (RPMI1640, 20% FCS) auf 96well Microtiterplatten ausgesät.

Die LAPC-4-Zellen (ATCC, PTA-1441TM) wurden zu einer Konzentration von 4000 Zellen/Well in 100µl Wachstumsmedium (RPMI1640, 2 mM L-Glutamin, 10% cFCS) auf 96well Microtiterplatten ausgesät. Ein Tag später wurden die LAPC-4-Zellen mit 1 nM Methyltrienolone und verschiedene Substanzverdünungen behandelt.

Die MDA-MB-231-Zellen (DSMZ, ACC 732) wurden zu einer Konzentration von 4000 Zellen/Well in 100µl Wachstumsmedium (DMEM/Ham's F12 Medium, 10% FCS) auf 96well Microtiterplatten ausgesät.

Die Caov-3-Zellen (ATCC, HTB-75) wurden zu einer Konzentration von 2000 Zellen/Well in 100µl Wachstumsmedium (MEM Earle's Medium, 10% FCS) auf 96well Microtiterplatten ausgesät.

Nach einer Übernachtinkubation bei 37°C wurden die Fluoreszenzwerte bestimmt (CI Werte). Dann wurden die Platten mit verschiedenen Substanzverdünnungen behandelt (1E-5 M, 3E-6 M, 1E-6M, 3E-7 M, 1E-7 M, 3E-8 M, 1E-8 M) und während 72 (MV4-11-, LOX-IMVI-Zellen), 96 (MOLM-13-, B16F10-, MDA-MB-431-, Caov-3-Zellen), 120 (MOLP-8-, KMS-12-PE-Zellen) oder 168 (LAPC-4-Zellen) Stunden bei 37°C inkubiert. Anschließend wurden die Fluoreszenzwerte bestimmt (CO Werte). Für die Datenanalyse wurden die CI Werte von den CO Werten abgezogen und die Ergebnisse verglichen zwischen Zellen, die mit verschiedenen Verdünnungen der Substanz oder nur mit Pufferlösung behandelt wurden. Die IC₅₀-Werte (Substanzkonzentration, die für eine 50%ige Hemmung der Zellproliferation notwendig ist) wurden daraus berechnet.

Die Substanzen wurden in den Zelllinien der Tabelle 1 untersucht, die beispielhaft die angegebenen Indikationen vertreten:

**Tabelle 1**

| Zelllinie | Quelle | Indikation |
|---|---|---|
| MOLM-13 | DSMZ | Akute myeloische Leukämie |
| MV4-11 | ATCC | Akute myeloische Leukämie |
| B16F10 | ATCC | Melanom (BRAF Wild-Typ) |
| LOX IMVI | NCI-60 | Melanom (BRAF mutiert) |
| MOLP-8 | DSMZ | Multiples Myelom |
| KMS-12-PE | DSMZ | Multiples Myelom |
| LAPC-4 | ATCC | Prostatakrebs |
| MDA-MB-231 | DSMZ | Mammakarzinom |
| Caov-3 | ATCC | Ovarkarzinom |

### 2. Ergebnisse:

### 2.1 Bindunssassay

Die Tabelle 2 zeigt die Ergebnisse aus dem BRD4 (BD1) Bindungsassay.

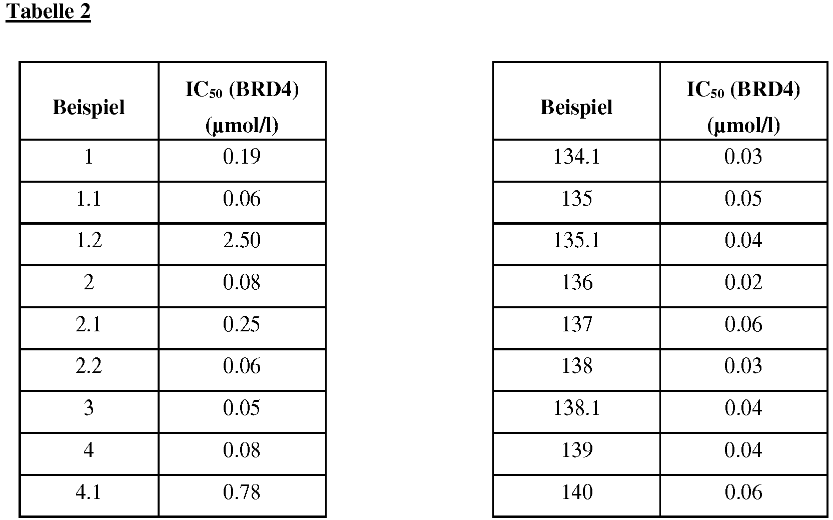

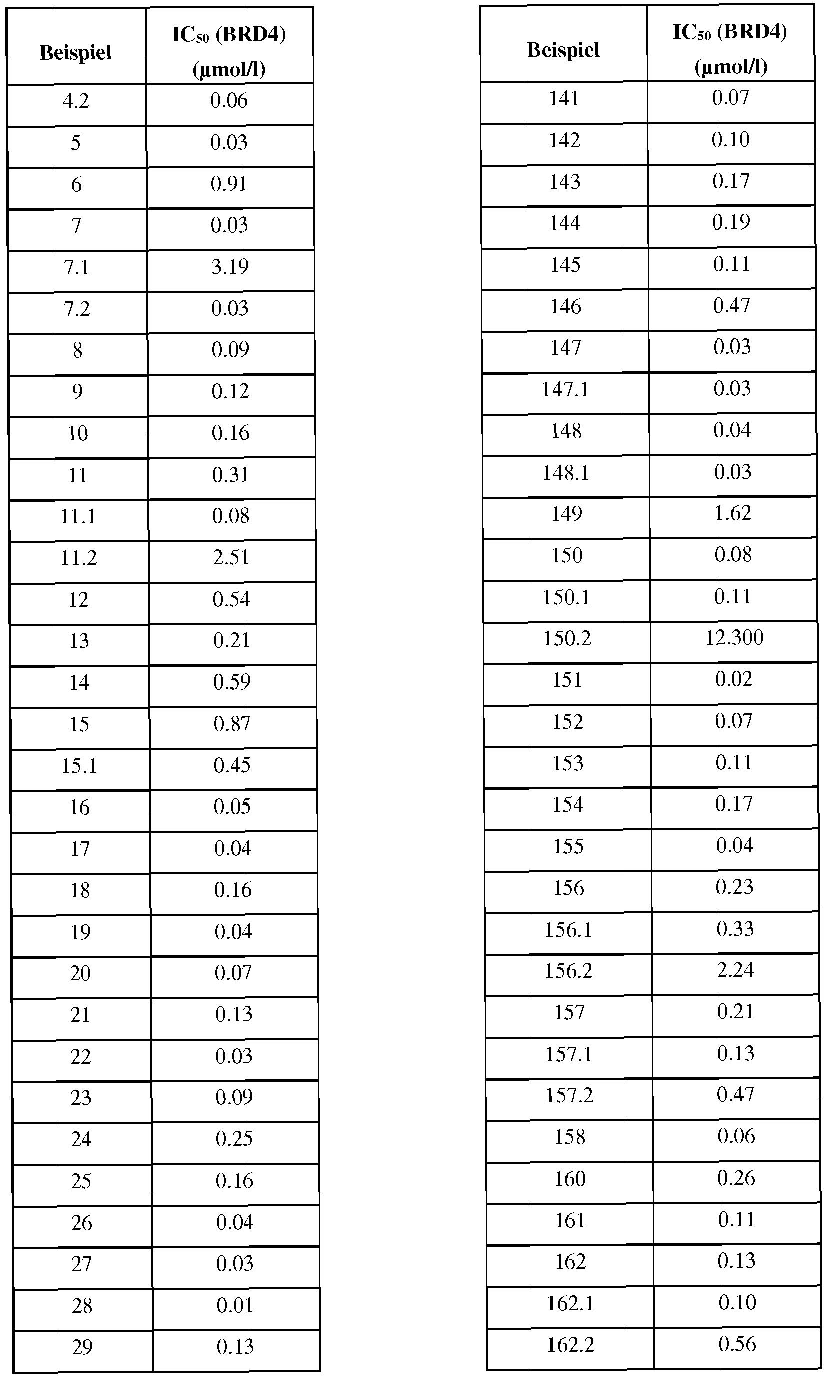

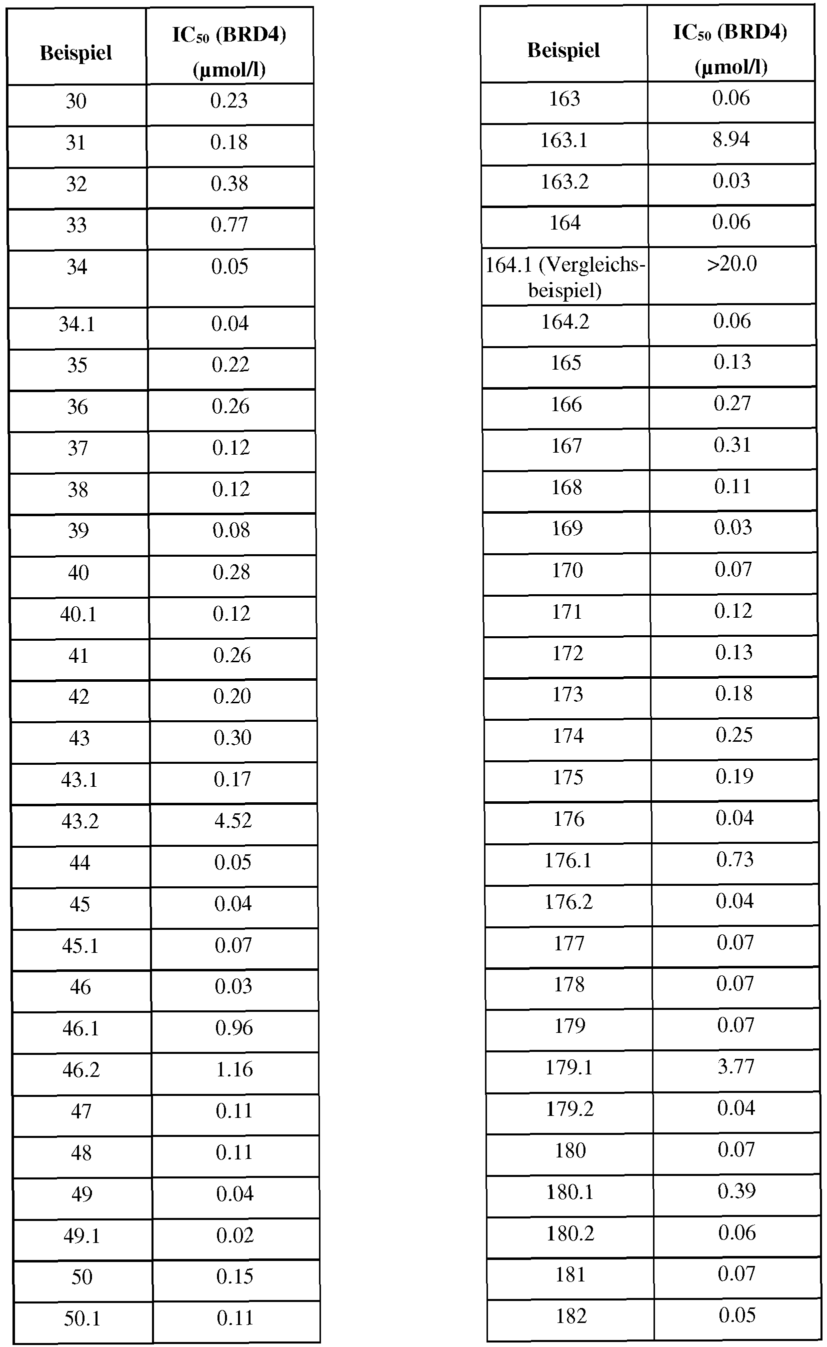

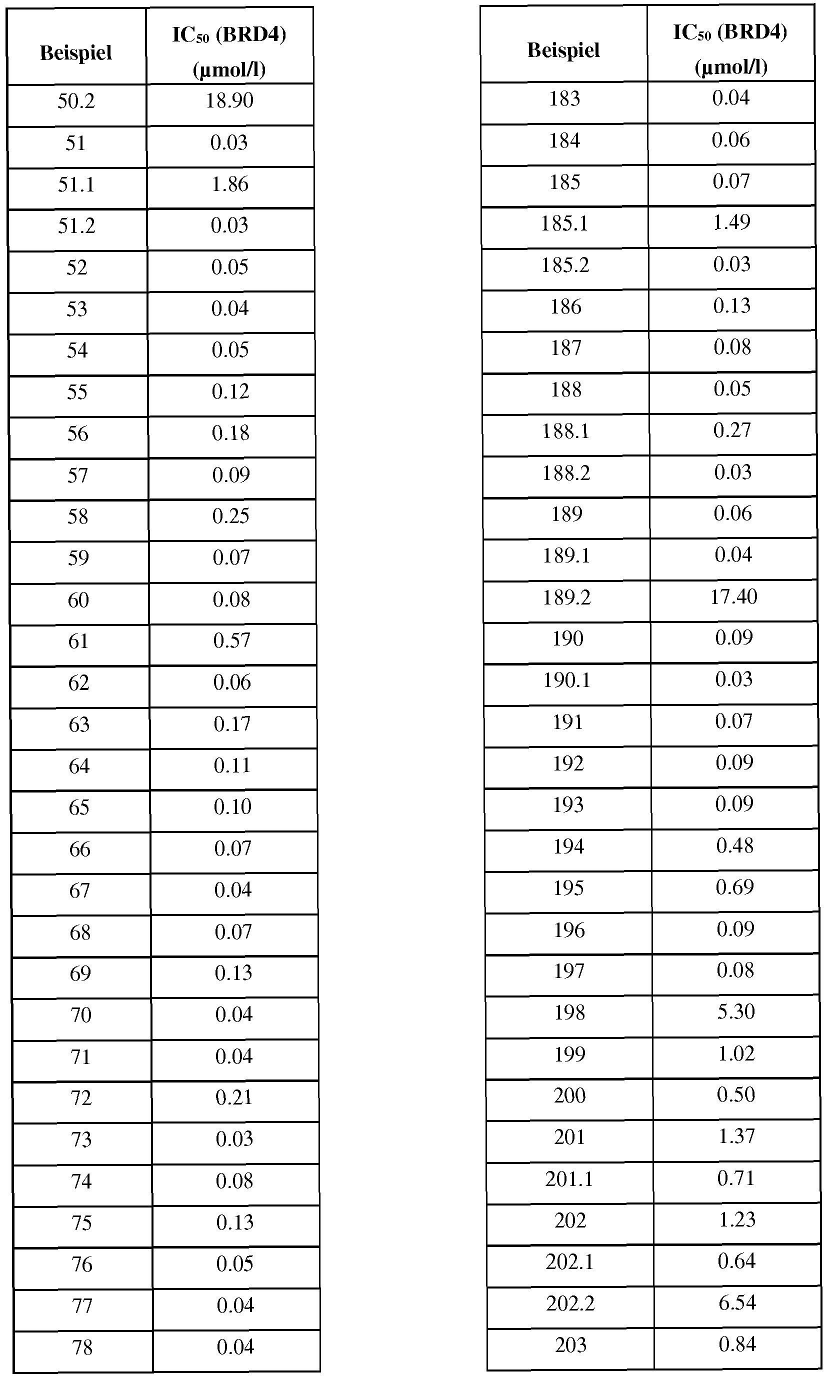

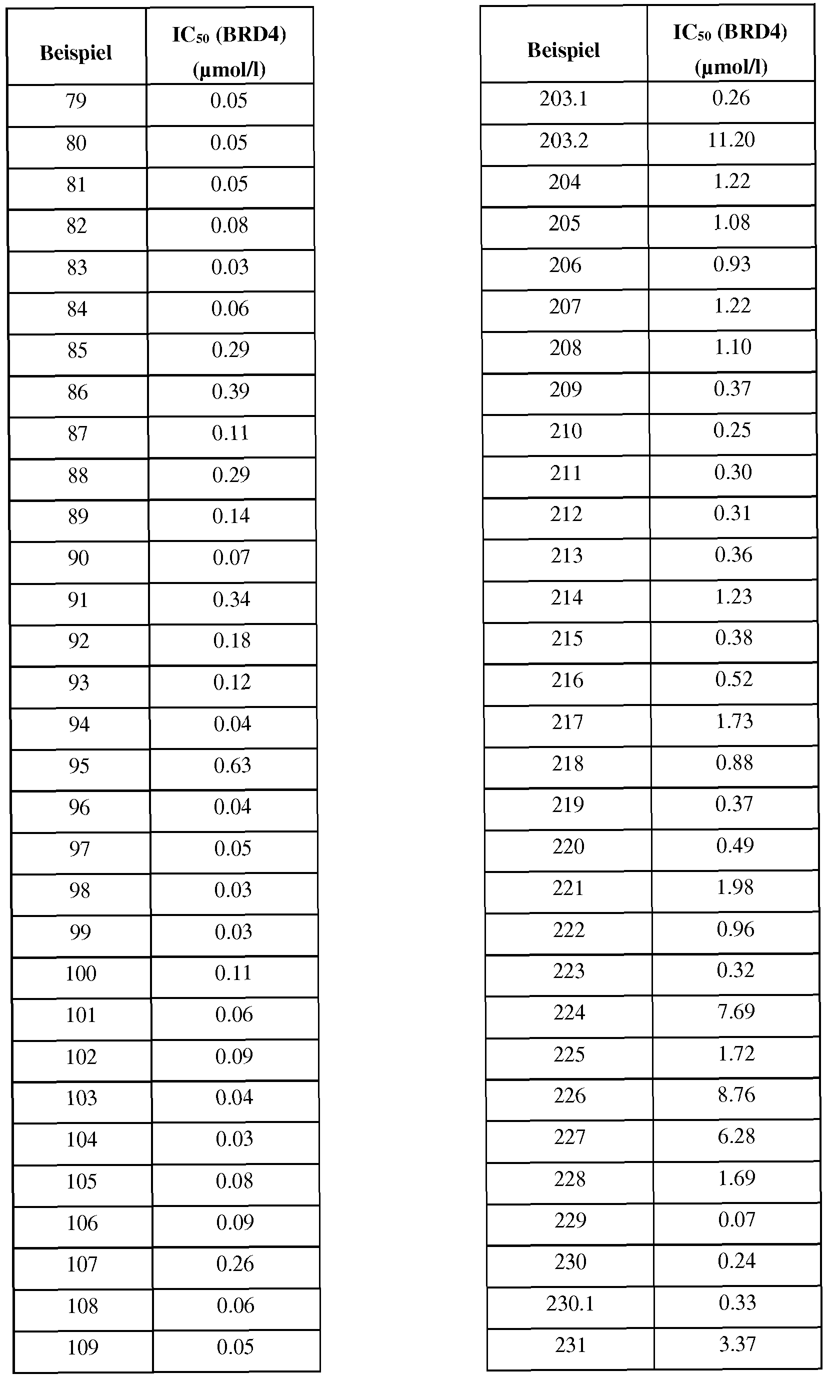

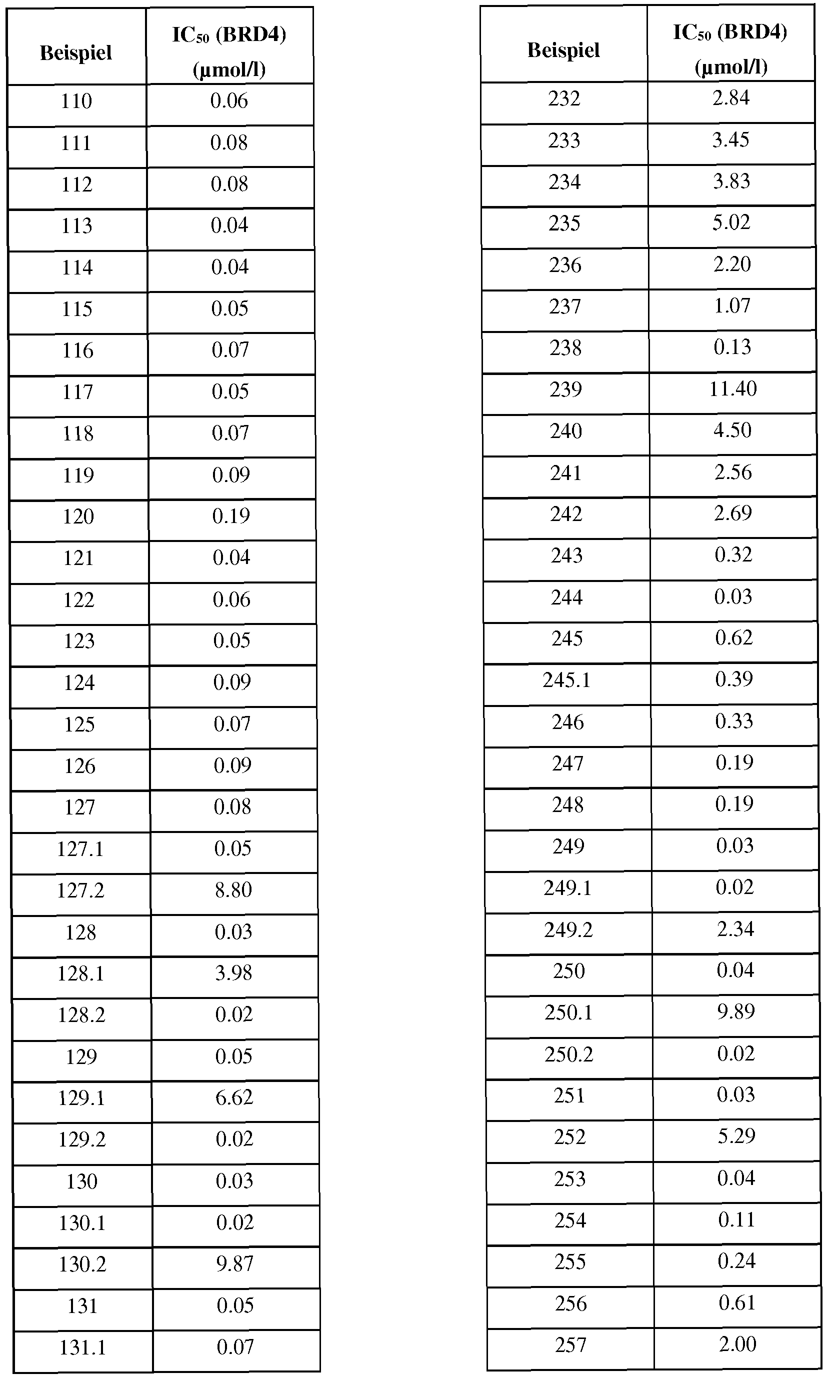

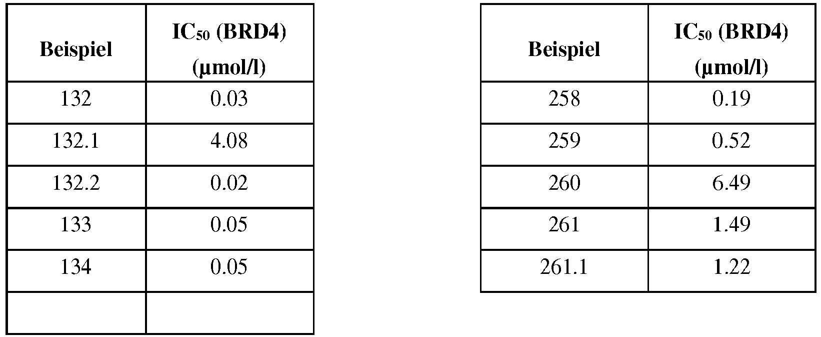

### 2.2 Zell-Assays

Die Tabellen 3A und 3B zeigen die Ergebnisse aus verschiedenen Zellproliferationsassays. Die mit den jeweiligen Zell-Linien korrespondierenden Indikationen können Tabelle 1 entnommen werden.

**Tabelle 3A**

| **Beispiel** | **MOLM-13 IC₅₀ (µmol/l)** | **MV4-11 IC₅₀ (µmol/l)** | **B16F10 IC₅₀ (µmol/l)** | **LOX IMVI IC₅₀ (µmol/l)** | **MOLP-8 IC₅₀ (µmol/l)** | **KMS-12-PE IC₅₀ (µmol/l)** |
|---|---|---|---|---|---|---|
| 1 | 1.26 | | 0.69 | | | |
| 1.1 | 0.39 | | 0.39 | | 0.51 | |
| 1.2 | >10 | | >10 | | | |
| 2 | 0.17 | 0.11 | 0.18 | 0.43 | 0.12 | 0.09 |
| 2.1 | 1.51 | | 1.05 | | | |
| 2.2 | 0.13 | 0.10 | 0.11 | 0.33 | 0.12 | 0.11 |
| 3 | 0.24 | 0.26 | 0.11 | 0.95 | 0.28 | 0.23 |
| 4 | 0.19 | 0.12 | 0.11 | 0.45 | 0.17 | 0.11 |
| 4.1 | 2.94 | | | | | |
| 4.2 | 0.18 | | 0.08 | | | |
| 5 | 0.82 | | 0.69 | | | |
| 7 | 0.20 | 0.17 | 0.14 | 0.57 | 0.12 | 0.09 |
| 7.1 | >10 | | | | | |
| 7.2 | 0.10 | 0.06 | | 0.19 | 0.05 | 0.05 |
| 8 | 0.38 | | 0.19 | | | |
| 9 | 0.65 | | 0.54 | | | |
| 10 | 0.31 | | 0.27 | | | |
| 11 | 1.91 | | 1.12 | | | |
| 11.1 | 0.57 | | | | | |
| 11.2 | 7.74 | | | | | |
| 12 | 1.61 | | 1.32 | | | |
| 13 | 1.44 | | 0.92 | | | |
| 14 | 3.07 | | 2.63 | | | |
| 15 | 3.45 | | | | | |
| 15.1 | 3.05 | | | | | |
| 16 | 0.22 | 0.27 | | 0.64 | 0.29 | 0.25 |
| 17 | 0.17 | 0.13 | | 0.30 | 0.11 | 0.11 |
| 18 | 0.25 | 0.30 | | 0.76 | 0.34 | 0.35 |
| 19 | 0.17 | 0.27 | | 0.65 | 0.27 | 0.27 |
| 20 | 0.19 | 0.14 | | 0.33 | 0.12 | 0.11 |
| 21 | 0.56 | | | | | |
| 22 | 0.08 | 0.88 | 0.10 | 0.24 | 0.09 | 0.11 |
| 23 | 0.27 | | | | | |
| 24 | 1.17 | | | | | |
| 25 | 0.74 | | | | | |
| 26 | 0.43 | | | | | |
| 27 | 0.63 | | | | | |
| 28 | 0.04 | 0.03 | | 0.04 | 0.02 | 0.03 |
| 29 | 0.36 | | | | | |
| 30 | 0.79 | | | | | |
| 31 | 0.27 | | | | | |
| 32 | 1.18 | | | | | |
| 33 | 3.31 | | | | | |
| 34 | 0.35 | | | | | |
| 34.1 | 0.24 | 0.12 | | 0.44 | 0.08 | 0.08 |
| 35 | 1.47 | | | | | |
| 36 | 1.16 | | | | | |
| 37 | 1.13 | | | | | |
| 38 | 0.73 | | | | | |
| 39 | 0.70 | | | | | |
| 40 | 1.47 | | | | | |
| 40.1 | 0.96 | 0.76 | 0.79 | 0.33 | 0.59 | 0.08 |
| 41 | 1.35 | | | | | |
| 42 | 1.29 | | | | | |
| 43 | 0.84 | | | | | |
| 43.1 | 0.81 | | | | | |
| 43.2 | 9.85 | | | | | |
| 44 | 0.21 | | | | | |
| 45 | 0.23 | 0.14 | | 0.35 | 0.13 | 0.13 |
| 45.1 | 0.22 | | | | | |
| 46 | 0.19 | 0.11 | | 0.37 | 0.16 | 0.12 |
| 46.1 | 5.14 | | | | | |
| 46.2 | >10 | | | | 9.47 | |
| 47 | 0.53 | | | | | |
| 48 | 0.19 | 0.22 | | 0.65 | 0.25 | 0.26 |
| 49 | 0.42 | | | | | |
| 49.1 | 0.23 | | | | | |
| 50 | 0.56 | | | | | |
| 50.1 | 0.37 | | | | | |
| 51 | 0.21 | 0.12 | | 0.35 | 0.12 | 0.09 |
| 51.1 | >10 | | | | | |
| 51.2 | 0.11 | 0.05 | | 0.17 | 0.05 | 0.04 |
| 52 | 0.24 | 0.14 | | 0.45 | 0.15 | 0.13 |
| 53 | 0.35 | | | | | |
| 54 | 0.44 | | | | | |
| 55 | 0.90 | | | | | |
| 56 | 0.90 | | | | | |
| 57 | 0.79 | | | | | |
| 58 | 0.99 | | | | | |
| 59 | 0.62 | | | | | |
| 60 | 0.48 | | | | | |
| 61 | 2.11 | | | | | |
| 62 | 0.65 | | | | | |
| 63 | 0.77 | | | | | |
| 64 | 0.88 | | | | | |
| 65 | 0.63 | | | | | |
| 66 | 0.63 | | | | | |
| 67 | 0.47 | | | | | |
| 68 | 1.17 | | | | | |
| 69 | 1.21 | | | | | |
| 70 | 0.30 | | | | | |
| 71 | 0.25 | 0.05 | | 0.15 | 0.09 | 0.08 |
| 72 | 0.66 | | | | | |
| 73 | 0.30 | | | | | |
| 74 | 0.55 | | | | | |
| 75 | 0.66 | | | | | |
| 76 | 0.75 | | | | | |
| 77 | 0.49 | | | | | |
| 78 | 0.37 | | | | | |
| 79 | 0.46 | | | | | |
| 80 | 0.56 | | | | | |
| 81 | 0.30 | | | | | |
| 82 | 0.55 | | | | | |
| 83 | 0.45 | | | | | |
| 84 | 0.34 | | | | | |
| 85 | 1.08 | | | | | |
| 86 | 1.43 | | | | | |
| 87 | 0.56 | | | | | |
| 88 | 0.67 | | | | | |
| 89 | 0.38 | | | | | |
| 90 | 0.46 | | | | | |
| 91 | 0.55 | | | | | |
| 92 | 0.85 | | | | | |
| 93 | 0.46 | | | | | |
| 94 | 0.38 | | | | | |
| 95 | 2.76 | | | | | |
| 96 | 0.25 | 0.07 | | 0.22 | 0.09 | 0.07 |
| 97 | 0.45 | | | | | |
| 98 | 0.27 | | | | | |
| 99 | 0.27 | | | | | |
| 100 | 0.53 | | | | | |
| 101 | 0.65 | | | | | |
| 102 | 0.96 | | | | | |
| 103 | 0.35 | | | | | |
| 104 | 0.26 | | | | | |
| 105 | 0.67 | | | | | |
| 106 | 2.55 | | | | | |
| 107 | 0.63 | | | | | |
| 108 | 1.56 | | | | | |
| 109 | 1.16 | | | | | |
| 110 | 0.36 | | | | | |
| 111 | 0.81 | | | | | |
| 112 | 0.63 | | | | | |
| 113 | 1.42 | | | | | |
| 114 | 0.30 | | | | | |
| 115 | 0.46 | | | | | |
| 116 | 0.49 | | | | | |
| 117 | 0.38 | | | | | |
| 118 | 0.50 | | | | | |
| 119 | 0.41 | | | | | |
| 120 | 0.80 | | | | | |
| 121 | 0.27 | | | | | |
| 122 | 0.66 | | | | | |
| 123 | 0.63 | | | | | |
| 124 | 1.07 | | | | | |
| 125 | 1.16 | | | | | |
| 126 | 0.74 | | | | | |
| 127 | 0.18 | 0.12 | | 0.25 | 0.09 | 0.09 |
| 127.1 | 0.11 | 0.07 | 0.10 | 0.29 | 0.06 | 0.09 |
| 127.2 | >10 | | | | | |
| 128 | 0.23 | | | 0.39 | 0.10 | 0.10 |
| 128.1 | >10 | | | | | |
| 128.2 | 0.15 | 0.07 | | 0.19 | 0.05 | 0.05 |
| 129 | 0.22 | 0.18 | | 0.58 | 0.15 | 0.12 |
| 129.1 | >10 | | | | | |
| 129.2 | 0.15 | | | | 0.08 | |
| 130 | 0.17 | | | | | |
| 130.1 | 0.07 | 0.05 | 0.10 | 0.19 | 0.05 | 0.07 |
| 131 | 0.11 | 0.04 | | 0.07 | 0.03 | 0.03 |
| 131.1 | 0.11 | | 0.06 | | | |
| 132 | 0.20 | 0.13 | | 0.42 | 0.12 | 0.11 |
| 132.1 | 7.56 | | | | | |
| 132.2 | 0.23 | | | | | |
| 133 | 0.18 | 0.08 | | 0.17 | 0.07 | 0.08 |
| 134 | 0.36 | | | | | |
| 134.1 | 0.08 | | | | | |
| 135 | 0.20 | 0.12 | | 0.36 | 0.13 | 0.12 |
| 135.1 | 0.15 | | 0.08 | | | |
| 136 | 0.20 | | | | | |
| 137 | 0.49 | | | | | |
| 138 | 0.28 | | | | | |
| 138.1 | 0.18 | | | | | |
| 139 | 0.21 | 0.13 | | 0.38 | 0.13 | 0.11 |
| 140 | 0.47 | | | | | |
| 141 | 0.55 | | | | | |
| 142 | 0.68 | | | | | |
| 143 | 0.84 | | | | | |
| 144 | 0.96 | | | | | |
| 145 | 0.83 | | | | | |
| 146 | 1.15 | | | | | |
| 147 | 0.07 | 0.05 | | 0.18 | 0.08 | 0.06 |
| 147.1 | 0.06 | 0.02 | | 0.51 | 0.02 | 0.03 |
| 148 | 0.08 | 0.07 | | 0.25 | 0.09 | 0.08 |
| 148.1 | 0.08 | 0.05 | | 0.09 | 0.05 | 0.05 |
| 149 | 3.00 | | | | | |
| 150 | 0.48 | | | | | |
| 150.1 | 0.23 | 0.13 | | 0.34 | 0.23 | 0.18 |
| 150.2 | 9.87 | | | | | |
| 151 | 1.31 | | | | | |
| 152 | 0.43 | | | | | |
| 153 | 0.48 | | | | | |
| 154 | 2.08 | | | | | |
| 155 | 0.38 | | | | | |
| 156 | 0.79 | | | | | |
| 156.1 | 0.56 | | | | | |
| 156.2 | >10 | | | | | |
| 157 | 0.09 | 0.19 | | 0.58 | 0.12 | 0.24 |
| 157.1 | 0.12 | | | | | |
| 157.2 | 2.19 | | | | | |
| 158 | 0.43 | | | | | |
| 160 | 0.40 | | | | | |
| 161 | 0.28 | | | | | |
| 162 | 0.38 | | | | | |
| 162.1 | 0.21 | | | | | |
| 162.2 | 1.68 | | | | | |
| 163 | 0.21 | | | | | |
| 163.1 | 7.08 | | | | | |
| 163.2 | 0.26 | | | | | |
| 164 | 0.58 | | | | | |
| 164.2 | 0.42 | | | | | |
| 165 | 0.88 | | | | | |
| 166 | 0.67 | | | | | |
| 167 | 3.24 | | | | 2.95 | |
| 168 | 0.39 | 0.22 | | | | |
| 169 | 0.24 | | | 0.41 | 0.12 | 0.12 |
| 170 | 0.52 | | | | | |
| 171 | 1.91 | | | | | |
| 172 | 1.91 | | | | | |
| 173 | 1.21 | | | | | |
| 174 | 2.34 | | | | 2.03 | |
| 175 | 0.44 | | | | | |
| 176 | 0.52 | | | | | |
| 176.1 | 2.19 | | | | | |
| 176.2 | 0.25 | | | | | |
| 177 | 0.49 | | | | | |
| 178 | 0.18 | | | | | |
| 179 | 0.29 | | | | | |
| 179.2 | 0.08 | | | | | |
| 180 | 0.37 | | | | | |
| 180.1 | 2.70 | | | | | |
| 180.2 | 0.05 | | | | | |
| 181 | 0.30 | | 0.17 | | | |
| 182 | 0.40 | | | | | |
| 184 | 0.30 | | 0.13 | | | |
| 185 | 0.28 | | 0.16 | | | |
| 185,2 | 0.10 | | | | | |
| 186 | 0.33 | | 0.24 | | | |
| 187 | 0.33 | | 0.17 | | | |
| 188 | 0.20 | | 0.10 | | | |
| 188.1 | 1.13 | | | | | |
| 188.2 | 0.07 | | | | | |
| 189 | 0.44 | | 0.21 | | | |
| 189.1 | 0.21 | | 0.10 | | 0.08 | |
| 190 | 0.08 | | | | | |
| 191 | 0.17 | | | | | |
| 192 | 0.27 | | | | | |
| 193 | 0.11 | | | | | |
| 200 | 0.18 | | | | | |
| 201.1 | 1.99 | | | | | |
| 202.1 | 0.58 | | | | | |
| 203 | 3.31 | | | | | |
| 203.1 | 1.27 | | | | | |
| 209 | 0.19 | | | | | |
| 210 | 0.17 | | | | | |
| 212 | 0.82 | | | | | |
| 213 | 0.86 | | | | | |
| 215 | 2.27 | | | | | |
| 216 | 1.03 | | | | | |
| 218 | 0.30 | | | | | |
| 219 | 0.57 | | | | | |
| 220 | 2.40 | | | | | |
| 222 | 2.12 | | | | | |
| 229 | 0.14 | | | | | |
| 230 | 0.23 | | | | | |
| 243 | 0.34 | | | | | |
| 245 | 0.86 | | | | | |
| 246 | 2.06 | | | | | |
| 247 | 0.57 | | | | | |
| 248 | 0.14 | | | | | |
| 249.1 | 0.06 | | | | | |
| 250 | 0.14 | | 0.10 | | | |
| 250.2 | 0.05 | | 0.04 | | | |
| 251 | 0.10 | | | | | |
| 253 | 0.06 | | | | | |
| 254 | 0.17 | | | | | |

**Tabelle 3B**

| **Beispiel** | **LAPC-4 IC₅₀ (µmol/l)** | **MDA-MB-231 IC₅₀ (µmol/l)** | **Caov-3 IC₅₀ (µmol/l)** |
|---|---|---|---|
| 1 | 0.60 | 0.90 | |
| 1.1 | 0.60 | 0.53 | |
| 1.2 | 5.17 | 10.00 | |
| 2 | 0.24 | 0.31 | 0.31 |
| 2.1 | 0.71 | 1.39 | |
| 2.2 | 0.09 | 0.20 | 0.25 |
| 3 | 0.11 | 0.21 | 0.55 |
| 4 | 0.09 | 0.17 | 0.32 |
| 5 | 0.27 | 1.35 | |
| 7 | 0.09 | 0.25 | 0.40 |
| 8 | 0.18 | 0.28 | |
| 9 | 0.43 | 0.73 | |
| 10 | 0.12 | 0.26 | |
| 11 | 0.45 | 2.58 | |
| 11.1 | 0.31 | 1.11 | |
| 11.2 | | 10.00 | |
| 12 | 0.87 | 1.85 | |
| 13 | 0.90 | 1.30 | |
| 14 | 1.65 | 3.64 | |
| 15 | | 6.23 | |
| 16 | 0.11 | 0.47 | 0.37 |
| 17 | 0.10 | 0.21 | 0.22 |
| 18 | | 0.28 | 0.48 |
| 19 | 0.11 | | 0.41 |
| 20 | 0.12 | 0.22 | 0.24 |
| 21 | | | |
| 22 | 0.06 | 0.15 | 0.11 |
| 23 | 0.19 | | |
| 26 | 0.13 | | |
| 27 | 0.18 | | |
| 28 | 0.02 | | 0.04 |
| 34.1 | | | 0.31 |
| 40.1 | 0.29 | 1.28 | |
| 45 | | | 0.24 |
| 46 | | | 0.28 |
| 48 | | | 0.47 |
| 51 | | | 0.28 |
| 51.2 | | | 0.13 |
| 52 | | | 0.35 |
| 71 | | | 0.15 |
| 96 | | | 0.17 |
| 127 | | | 0.24 |
| 127.1 | 0.04 | 0.13 | 0.17 |
| 128 | | | 0.32 |
| 128.2 | | | 0.16 |
| 129 | | | 0.42 |
| 130.1 | 0.05 | 0.14 | 0.003 |
| 131 | | | 0.05 |
| 132 | | | 0.32 |
| 133 | | | 0.18 |
| 135 | | | 0.30 |
| 139 | | | 0.31 |
| 147 | | | 0.18 |
| 147.1 | | | 0.08 |
| 148 | | | 0.30 |
| 148.1 | | | 0.13 |
| 150.1 | | | 0.34 |
| 157 | | | 0.42 |
| 169 | | | 0.37 |

## Patentansprüche

1. Verbindungen der allgemeinen Formel I 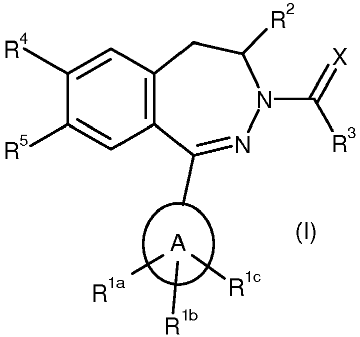 in der
X für ein Sauerstoff- oder Schwefelatom steht, und
A für einen monozyklischen Heteroarylring mit 5 oder 6 Ringatomen oder für einen Phenylring steht, und
R^{1a} für Wasserstoff, Halogen, Cyano, Carboxy, Amino oder Aminosulfonyl steht,
oder
für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino-, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
oder
für einen monozyklischen Heterocyclylrest mit 3 bis 8 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, Phenyl-, Halophenyl-, Phenyl-C₁-C₆-Alkyl-, Pyridinyl-, -NR⁶C(=O)-R⁹, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder einem monocyclischen Heterocyclylrest mit 3 bis 8 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 3 bis 8 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden, mit Halogen, C₁-C₃-Alkyl und/oder C₁-C₃-Alkoxy-substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, -C(=O)NR⁶R⁷, -C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl- und/oder einem monocyclischen Heterocyclylrest mit 3 bis 8 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl und/oder C₁-C₃-Alkoxy-, und
R^{1b} und R^{1c} unabhängig voneinander für Wasserstoff, Halogen, Hydroxy, Cyano, Nitro und/oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl-Rest und/oder einen monocyclischen Heterocyclylrest mit 3 bis 8 Ringatomen stehen, und
R² für einen C₁-C₃-Alkyl- oder Trifluormethyl- oder einen C₃- oder C₄-Cycloalkylrest steht, und
R³ für Cyclopropyl-, C₁-C₃-Alkyl-, C₁-C₃-Alkoxy-, Amino-, Cyclopropylamino-oder C₁-C₃-Alkylamino- steht, und
R⁴ und R⁵ unabhängig voneinander stehen für Wasserstoff, Hydroxy, Cyano, Nitro, Amino, Aminocarbonyl-, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die gegebenenfalls ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, monocyclischem Heterocyclyl- mit 3 bis 8 Ringatomen und/oder monocyclischem Heteroarylmit 5 oder 6 Ringatomen, worin die genannten monocyclischen Heterocyclyl- und Heteroarylreste ihrerseits gegebenenfalls einfach substituiert sein können durch C₁-C₃-Alkyl,
oder
für C₃-C₁₀-Cycloalkyl-, das gegebenenfalls ein- oder mehrfach, gleich oder verschieden substituiert sein kann mit Halogen, Amino, Hydroxy, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆- Alkyl-, Halogen-C₁-C₆-Alkoxy-, und/oder einem monocyclischen Heterocyclylrest mit 3 bis 8 Ringatomen,
oder
für monozyklisches Heteroaryl- mit 5 oder 6 Ringatomen, das gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷, und/oder einem monocyclischen Heterocyclylrest mit 3 bis 8 Ringatomen,
oder
für monozyklisches Heterocyclyl- mit 3 bis 8 Ringatomen, das gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, - C(=O)R⁸,
-S(=O)₂R⁹, -NR⁶R⁷, und/oder einem monocyclischen Heterocyclylrest mit 3 bis 8 Ringatomen,
oder
für Phenyl-, das gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl-, C₁-C₆-Alkylaminosulfonyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-und/oder einem monocyclischen Heterocyclylrest mit 3 bis 8 Ringatomen, und
R⁶und R⁷ unabhängig voneinander für Wasserstoff, C₁-C₃-Alkyl-, Cyclopropyl-, Di-C₁-C₃-Alkyl-Amino-C₁-C₃-Alkyl- oder Fluorpyridyl- stehen, und
R⁸ für Hydroxy, C₁-C₆-Alkyl-, Halogen-C₁-C₃-Alkyl-, Hydroxy-C₁-C₃-Alkyl-, C₁-C₃-Alkoxy-C₁-C₃-Alkyl-, C₃-C₈-Cycloalkyl-, Phenyl-, monocyclisches Heterocyclyl- mit 3 bis 8 Ringatomen oder monocyclisches Heteroaryl- mit 5 oder 6 Ringatomen steht, worin Phenyl-, Heteroaryl- und Heterocyclylgegebenenfalls ein- oder zweifach substituiert sein können durch Halogen, C₁-C₃-Alkoxy- oder C₁-C₃-Alkyl-, und
R⁹ für Wasserstoff, C₁-C₆-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze,
mit der Maßgabe, dass,
wenn A für einen Phenylring steht und R⁴ und R⁵ unabhängig voneinander stehen für Wasserstoff, Hydroxy, Cyano, Nitro, Amino, Aminocarbonyl-, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, oder für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die gegebenenfalls ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, monocyclischem Heterocyclyl- mit 3 bis 8 Ringatomen und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl,
R^{1a} nicht für Wasserstoff, Halogen, Cyano, Carboxy, Amino oder Aminosulfonyl, oder für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino-, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht, und
wobei die Maßgabe nicht solche Verbindungen der allgemeinen Formel (I) umfasst, in der A für Phenyl steht und R⁴ für Wasserstoff, Fluor, Chlor oder Brom steht und R⁵ für C₁-C₆-Alkoxy-, das ein- oder mehrfach, gleich oder verschieden mit Halogen substituiert ist, steht, und R^{1a} für Halogen steht,
und auch nicht solche Verbindungen der allgemeinen Formel (I), in der A für Phenyl steht und R⁴ für Wasserstoff steht und R⁵ für C₁-C₆-Alkoxy steht, welches ein- oder mehrfach, gleich oder verschieden mit einem monocyclischen Heterocyclylrest mit 3 bis 8 Ringatomen, und/oder einem monocyclischen Heteroarylrest mit 5 oder 6 Ringatomen substituiert ist, worin die genannten monocyclischen Heterocyclyl- und Heteroarylreste ihrerseits gegebenenfalls einfach substituiert sein können durch C₁-C₃-Alkyl, und R^{1a} für Halogen steht.

2. Verbindungen der allgemeinen Formel (I), gemäß Anspruch 1, in der
X für ein Sauerstoffatom steht, und
A für einen Phenyl- oder Pyridylring steht, und
R^{1a} für Wasserstoff, Halogen, Cyano, Carboxy, Amino oder Aminosulfonyl steht, oder
für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino, C₁-C₆-Alkylamino-C₁-C₆-Alkyl-, *N*-(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-, Phenyl-, Halophenyl-, Phenyl-C₁-C₆-Alkyl-, Pyridinyl-, -NR⁶C(=O)R⁹, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, C₃-C₁₀-Cycloalkyl-,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen und/oder mit einem monocyclischen Heteroarylrest mit 5 oder 6 Ringatomen und/oder mit einem Phenylrest, der seinerseits gegebenenfalls ein- oder mehrfach, gleich oder verschieden mit Halogen, C₁-C₃-Alkyl- und/oder C₁-C₃-Alkoxysubstituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Halogen-C₁-C₆-Alkyl-, Halogen-C₁-C₆-Alkoxy-, Hydroxy-C₁-C₆-Alkyl-, C₃-C₁₀-Cycloalkyl- und/oder einem monocyclischen Heterocyclylrest mit 4 bis 7 Ringatomen und/oder einem monocyclischen Heteroarylrest mit 5 oder 6 Ringatomen, der seinerseits gegebenenfalls ein- oder mehrfach, gleich oder verschieden substituiert sein kann mit Halogen, C₁-C₃-Alkyl- und/oder C₁-C₃-Alkoxy-, und
R^{1b} und R^{1c} unabhängig voneinander für Wasserstoff, Halogen, Hydroxy, Cyano, Nitro oder für einen C₁-C₆-Alkyl-, C₁-C₆-Alkoxy-, C₁-C₆-Alkoxy-C₁-C₆-Alkyl-, Halogen-C₁-C₆-Alkyl-, Halogen-C₁-C₆-Alkoxy-, C₃-C₁₀-Cycloalkyl-Rest, und/oder einen monocyclischen Heterocyclylrest mit 4 bis 7 Ringatomen stehen, und
R² für Methyl-, Ethyl- oder Isopropyl- steht, und
R³ für Cyclopropyl-, C₁-C₃-Alkyl-, C₁-C₃-Alkoxy-, Amino-, Cyclopropylamino- oder C₁-C₃-Alkylamino- steht, und
R⁴ und R⁵ unabhängig voneinander stehen für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die gegebenenfalls ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, monocyclischem Heterocyclyl- mit 4 bis 7 Ringatomen, und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl-,
oder
für einen C₃-C₁₀-Cycloalkylrest, der gegebenenfalls ein- oder mehrfach, gleich oder verschieden substituiert sein kann mit Halogen, Amino, Hydroxy, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Halogen-C₁-C₆-Alkyl-, Halogen-C₁-C₆-Alkoxy-, und/oder einem monocyclischen Heterocyclylrest mit 4 bis 7 Ringatomen,
oder
für monozyklisches Heteroaryl- mit 5 oder 6 Ringatomen, das gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, Hydroxy-C₁-C₆-Alkyl-, C₁-C₆-Alkylamino-, Amino-C₁-C₆-Alkyl-, C₁-C₆-Alkylamino-C₁-C₆-Alkyl, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷, und/oder einem monocyclischen Heterocyclylrest mit 4 bis 7 Ringatomen,
oder
für monozyklisches Heterocyclyl- mit 4 bis 7 Ringatomen, das gegebenenfalls ein- oder mehrfach, gleich oder verschieden substituiert sein kann mit Halogen, Amino, Hydroxy, Cyano, Oxo, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, -C(=O)R⁸, -S(=O)₂R⁹, -NR⁶R⁷ und/oder einem monocyclischen Heterocyclylrest mit 4 bis 7 Ringatomen,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, C₁-C₆-Alkylaminocarbonyl-, C₁-C₆-Alkylaminosulfonyl-, C₁-C₆-Alkylamino-C₁-C₆-Alkyl-, Hydroxy-C₁-C₆-Alkyl-, Halogen-C₁-C₆-Alkyl-, Halogen-C₁-C₆-Alkoxy-, C₃-C₁₀-Cycloalkyl-, und/oder einem monocyclischen Heterocyclylrest mit 4 bis 7 Ringatomen,
und
R⁶ und R⁷ unabhängig voneinander für Wasserstoff, C₁-C₃-Alkyl-, Cyclopropyl-, Di-C₁-C₃-Alkyl-Amino-C₁-C₃-Alkyl- oder Fluorpyridyl stehen, und
R⁸ für Hydroxy, C₁-C₆-Alkyl-, Halogen-C₁-C₃-Alkyl-, Hydroxy-C₁-C₃-Alkyl-, C₁-C₃-Alkoxy-C₁-C₃-Alkyl-, C₃-C₈-Cycloalkyl-, Phenyl-, monocyclisches Heterocyclyl- mit 5 oder 6 Ringatomen steht, und
R⁹ für Wasserstoff, C₁-C₆-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze,
mit der Maßgabe, dass, wenn A für einen Phenylring steht und R⁴ und R⁵ unabhängig voneinander stehen für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, oder für C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, die gegebenenfalls ein- oder mehrfach, gleich oder verschieden substituiert sein können mit Halogen, Amino, Hydroxy, Carboxy, Hydroxy-C₁-C₆-Alkyl-, C₁-C₆-Alkoxy-, C₁-C₆-Alkoxy-C₁-C₆-Alkyl-, C₁-C₆-Alkylamino-, Amino-C₁-C₆-Alkyl-, monocyclischem Heterocyclyl- mit 4 bis 7 Ringatomen, und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl-,
R^{1a} nicht für Wasserstoff, Halogen, Cyano, Carboxy, Amino oder Aminosulfonyl, oder für einen C₁-C₆-Alkoxy-, C₁-C₃-Alkoxy-C₁-C₃-Alkyl-, C₁-C₃-Alkoxy-C₂-C₃-Alkoxy-, C₁-C₆-Alkylamino-, C₁-C₆-Alkylcarbonylamino, C₁-C₆-Alkylamino-C₁-C₆-Alkyl-, *N-*(Heterocyclyl)-C₁-C₆-Alkyl-, *N*-(Heterocyclyl)-C₁-C₆-Alkoxy-, Hydroxy-C₁-C₆-Alkyl-, Hydroxy-C₁-C₆-Alkoxy-, Halogen-C₁-C₆-Alkyl-, Halogen-C₁-C₆-Alkoxy-, C₁-C₆-Alkylcarbonyl- oder C₁-C₆-Alkoxycarbonyl-Rest steht, und
wobei die Maßgabe nicht solche Verbindungen der allgemeinen Formel (I) umfasst, in der A für Phenyl steht und R⁴ für Wasserstoff, Fluor, Chlor oder Brom steht und R⁵ für C₁-C₆-Alkoxy-, das ein- oder mehrfach, gleich oder verschieden mit Halogen substituiert ist, steht, und R^{1a} für Halogen steht,
und auch nicht solche Verbindungen der allgemeinen Formel (I), in der A für Phenyl steht und R⁴ für Wasserstoff steht und R⁵ für C₁-C₆-Alkoxy steht, welches ein- oder mehrfach, gleich oder verschieden mit einem monocyclischen Heterocyclylrest mit 4 bis 7 Ringatomen, und/oder einem monocyclischen Heteroarylrest mit 5 oder 6 Ringatomen substituiert ist, worin die genannten monocyclischen Heterocyclyl- und Heteroarylreste ihrerseits gegebenenfalls einfach substituiert sein können durch C₁-C₃-Alkyl, und R^{1a} für Halogen steht.

3. Verbindungen der allgemeinen Formel (I), gemäß den Ansprüchen 1 und 2, in der
X für ein Sauerstoffatom steht, und
A für einen Phenyl- oder Pyridylring steht, und
R^{1a} für Wasserstoff, Halogen, Cyano, Carboxy, Amino oder Aminosulfonyl steht, oder
für einen C₁-C₆-Alkoxy-, C₁-C₃-Alkoxy-C₁-C₃-Alkyl-, C₁-C₃-Alkoxy-C₂-C₃-Alkoxy-, C₁-C₃-Alkylamino-, C₁-C₃-Alkylcarbonylamino, C₁-C₃-Alkylamino-C₁-C₃-Alkyl-, Hydroxy-C₁-C₃-Alkyl-, Fluor-C₁-C₃-Alkyl-, Fluor-C₁-C₃-Alkoxy-, C₁-C₃-Alkylcarbonyl- oder C₁-C₄-Alkoxycarbonyl-Rest steht,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, Phenyl-, Halophenyl-, Phenyl-C₁-C₃-Alkyl-, Pyridinyl-, -NR⁶C(=O)-R⁹,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Fluor, Chlor, Brom, Methyl- und/oder Methoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl, C₁-C₃-Alkoxy-, C₁-C₂-Alkoxy-C₁-C₂-Alkyl-, C₁-C₃-Alkylamino-, Amino-C₁-C₃-Alkyl-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Fluor-C₁-C₃-Alkyl-, Fluor-C₁-C₃-Alkoxy-, Hydroxy-C₁-C₃-Alkyl-, C₃-C₆-Cycloalkyl- und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Fluor, Chlor, Brom, Methyl- und/oder Methoxy-, und
R^{1b} für Wasserstoff, Halogen, Hydroxy, Cyano, Nitro oder für einen C₁-C₃-Alkyl-, C₁-C₃-Alkoxy-, Fluor-C₁-C₃-Alkyl- oder Fluor-C₁-C₃-Alkoxy-Rest steht, und
R^{1c} für Wasserstoff, Fluor, Chlor, Brom oder Cyano steht, und
R² für Methyl-, Ethyl- oder Isopropyl- steht, und
R³ für Cyclopropyl-, C₁-C₃-Alkyl-, C₁-C₃-Alkoxy-, Cyclopropylamino- oder C₁-C₃-Alkylamino- steht, und
R⁴ und R⁵ unabhängig voneinander stehen für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₃-Alkyl-, C₁-C₃-Alkoxy-, C₁-C₃-Alkylamino-, die ein- oder mehrfach, gleich oder verschieden substituiert sein können mit Halogen, Amino, Hydroxy, Carboxy, Hydroxy-C₁-C₃-Alkyl-, C₁-C₃-Alkoxy-, C₁-C₃-Alkylamino-, Amino-C₁-C₃-Alkyl-, monocyclischem Heterocyclyl- mit 4 bis 7 Ringatomen, und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl,
oder
für einen C₃-C₇-Cycloalkylrest, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen,
oder
für monozyklisches Heteroaryl- mit 5 oder 6 Ringatomen, das gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für monozyklisches Heterocyclyl- mit 4 bis 7 Ringatomen, das gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylaminocarbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen, und
R⁶ und R⁷ unabhängig voneinander für Wasserstoff, C₁-C₃-Alkyl-, Cyclopropyl-, Di-C₁-C₃-Alkyl-Amino-C₁-C₃-Alkyl- oder Fluorpyridyl- stehen, und
R⁸ für Hydroxy, C₁-C₆-Alkyl-, Hydroxy-C₁-C₃-Alkyl-, Fluor-C₁-C₃-Alkyl-, C₁-C₃-Alkoxy-C₁-C₃-Alkyl-, C₃-C₈-Cycloalkyl- oder monocyclisches Heterocyclyl- mit 5 oder 6 Ringatomen steht, und
R⁹ für Wasserstoff, C₁-C₆-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze,
mit der Maßgabe, dass,
wenn A für einen Phenylring und R⁴ und R⁵ unabhängig voneinander stehen für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-, oder für C₁-C₃-Alkyl-, C₁-C₃-Alkoxy-, C₁-C₃-Alkylamino-, die gegebenenfalls ein- oder mehrfach, gleich oder verschieden substituiert sein können mit Halogen, Amino, Hydroxy, Carboxy, Hydroxy-C₁-C₃-Alkyl-, C₁-C₃-Alkoxy-, C₁-C₃-Alkylamino-, Amino-C₁-C₃-Alkyl-, monocyclischem Heterocyclyl- mit 4 bis 7 Ringatomen, und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl, R^{1a} nicht für Wasserstoff, Halogen, Cyano, Carboxy, Amino oder Aminosulfonyl, oder für einen C₁-C₆-Alkoxy-, C₁-C₃-Alkoxy-C₁-C₃-Alkyl-, C₁-C₃-Alkoxy-C₂-C₃-Alkoxy-, C₁-C₃-Alkylamino-, C₁-C₃-Alkylcarbonylamino, C₁-C₃-Alkylamino-C₁-C₃-Alkyl-, Hydroxy-C₁-C₃-Alkyl-, Fluor-C₁-C₃-Alkyl-, Fluor-C₁-C₃-Alkoxy-, C₁-C₃-Alkylcarbonyl- oder C₁-C₄-Alkoxycarbonyl-Rest steht, und
wobei die Maßgabe nicht solche Verbindungen der allgemeinen Formel (I) umfasst, in der A für Phenyl steht und R⁴ für Wasserstoff, Fluor, Chlor oder Brom steht und R⁵ für C₁-C₃-Alkoxy-, das ein- oder mehrfach, gleich oder verschieden mit Halogen substituiert ist, steht, und R^{1a} für Halogen steht,
und auch nicht solche Verbindungen der allgemeinen Formel (I), in der A für Phenyl steht und R⁴ für Wasserstoff steht und R⁵ für C₁-C₃-Alkoxy steht, welches ein- oder mehrfach, gleich oder verschieden mit einem monocyclischen Heterocyclylrest mit 4 bis 7 Ringatomen, und/oder einem monocyclischen Heteroarylrest mit 5 oder 6 Ringatomen substituiert ist, worin die genannten monocyclischen Heterocyclyl- und Heteroarylreste ihrerseits gegebenenfalls einfach substituiert sein können durch C₁-C₃-Alkyl, und R^{1a} für Halogen steht.

4. Verbindungen der allgemeinen Formel (I), gemäß den Ansprüchen 1 bis 3, in der
X für ein Sauerstoffatom steht, und
A für einen Phenyl- oder Pyridylring steht, und
R^{1a} für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, Phenyl-, Halophenyl-, Phenyl-C₁-C₃-Alkyl-, Pyridinyl-, -NR⁶C(=O)-R⁹,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Fluor, Chlor, Brom, Methyl- und/oder Methoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl, C₁-C₃-Alkoxy-, C₁-C₂-Alkoxy-C₁-C₂-Alkyl-, C₁-C₃-Alkylamino-, Amino-C₁-C₃-Alkyl-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Fluor-C₁-C₃-Alkyl-, Fluor-C₁-C₃-Alkoxy-, Hydroxy-C₁-C₃-Alkyl-, C₃-C₆-Cycloalkyl- und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Fluor, Chlor, Brom, Methyl- oder Methoxy-, und
R^{1b} für Wasserstoff, Halogen, Hydroxy, Cyano, Nitro oder für einen C₁-C₃-Alkyl-, C₁-C₃-Alkoxy-, Fluor-C₁-C₃-Alkyl- oder Fluor-C₁-C₃-Alkoxy-Rest steht, und
R^{1c} für Wasserstoff, Fluor, Chlor, Brom oder Cyano steht, und
R² für Methyl-, Ethyl- oder Isopropyl- steht, und
R³ für Cyclopropyl-, C₁-C₃-Alkyl-, C₁-C₃-Alkoxy-, Cyclopropylamino- oder C₁-C₃-Alkylamino- steht, und
R⁴ und R⁵ unabhängig voneinander stehen für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl-,
oder
für C₁-C₃-Alkyl-, C₁-C₃-Alkoxy-, C₁-C₃-Alkylamino-, die ein- oder mehrfach, gleich oder verschieden substituiert sein können mit Halogen, Amino, Hydroxy, Carboxy, Hydroxy-C₁-C₃-Alkyl-, C₁-C₃-Alkoxy-, C₁-C₃-Alkylamino-, Amino-C₁-C₃-Alkyl-, monocyclischem Heterocyclyl- mit 4 bis 7 Ringatomen, und/oder monocyclischem Heteroaryl- mit 5 oder 6 Ringatomen, worin die genannten monocyclischen Heterocyclyl- und Heteroarylreste ihrerseits gegebenenfalls einfach substituiert sein können durch C₁-C₃-Alkyl,
oder
für einen C₃-C₇-Cycloalkylrest, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen,
oder
für monozyklisches Heteroaryl- mit 5 oder 6 Ringatomen, das gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für monozyklisches Heterocyclyl- mit 4 bis 7 Ringatomen, das gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen Phenylrest, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylaminocarbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen, und
R⁶ und R⁷ unabhängig voneinander für Wasserstoff, C₁-C₃-Alkyl-, Cyclopropyl-, Di-C₁-C₃-Alkyl-Amino-C₁-C₃-Alkyl- oder Fluorpyridyl- stehen, und
R⁸ für Hydroxy, C₁-C₆-Alkyl-, Hydroxy-C₁-C₃-Alkyl-, Fluor-C₁-C₃-Alkyl-, C₁-C₃-Alkoxy-C₁-C₃-Alkyl-, C₃-C₈-Cycloalkyl- oder monocyclisches Heterocyclyl- mit 5 oder 6 Ringatomen steht, und
R⁹ für Wasserstoff, C₁-C₆-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze.

5. Verbindungen der allgemeinen Formel (I), gemäß den Ansprüchen 1 bis 3, in der
X für ein Sauerstoffatom steht, und
A für einen Phenyl- oder Pyridylring steht, und
R^{1a} für Wasserstoff, Halogen, Cyano, Carboxy, Amino oder Aminosulfonyl steht, oder
für einen C₁-C₆-Alkoxy-, C₁-C₃-Alkoxy-C₁-C₃-Alkyl-, C₁-C₃-Alkoxy-C₂-C₃-Alkoxy-, C₁-C₃-Alkylamino-, C₁-C₃-Alkylcarbonylamino, C₁-C₃-Alkylamino-C₁-C₃-Alkyl-, Hydroxy-C₁-C₃-Alkyl-, Fluor-C₁-C₃-Alkyl-, Fluor-C₁-C₃-Alkoxy-, C₁-C₃-Alkylcarbonyl- oder C₁-C₄-Alkoxycarbonyl-Rest steht,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, Phenyl-, Halophenyl-, Phenyl-C₁-C₃-Alkyl-, Pyridinyl-, -NR⁶C(=O)-R⁹,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Fluor, Chlor, Brom, Methyl- und/oder Methoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl, C₁-C₃-Alkoxy-, C₁-C₂-Alkoxy-C₁-C₂-Alkyl-, C₁-C₃-Alkylamino-, Amino-C₁-C₃-Alkyl-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C1-C₃-Alkylaminosulfonyl-, C₃-C₆-Cycloalkylaminosulfonyl-, Fluor-C₁-C₃-Alkyl-, Fluor-C₁-C₃-Alkoxy-, Hydroxy-C₁-C₃-Alkyl-, C₃-C₆-Cycloalkyl- und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Fluor, Chlor, Brom, Methyl- und/oder Methoxy-, und
R^{1b} für Wasserstoff, Halogen, Hydroxy, Cyano, Nitro oder für einen C₁-C₃-Alkyl-, C₁-C₃-Alkoxy-, Fluor-C₁-C₃-Alkyl- oder Fluor-C₁-C₃-Alkoxy-Rest steht, und
R^{1c} für Wasserstoff, Fluor, Chlor, Brom oder Cyano steht, und
R² für Methyl-, Ethyl- oder Isopropyl- steht, und
R³ für Cyclopropyl-, C₁-C₃-Alkyl-, C₁-C₃-Alkoxy-, Cyclopropylamino- oder C₁-C₃-Alkylamino- steht, und
R⁴ für einen C₃-C₇-Cycloalkylrest steht, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen,
oder
für monozyklisches Heteroaryl- mit 5 oder 6 Ringatomen steht, das gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für monozyklisches Heterocyclyl- mit 4 bis 7 Ringatomen steht, das gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylamino-carbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen, und
R⁵ für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylamino-sulfonyl- steht, und
R⁶ und R⁷ unabhängig voneinander für Wasserstoff, C₁-C₃-Alkyl-, Cyclopropyl-, Di-C₁-C₃-Alkyl-Amino-C₁-C₃-Alkyl- oder Fluorpyridyl- stehen, und
R⁸ für Hydroxy, C₁-C₆-Alkyl-, Hydroxy-C₁-C₃-Alkyl-, Fluor-C₁-C₃-Alkyl-, C₁-C₃-Alkoxy-C₁-C₃-Alkyl-, C₃-C₈-Cycloalkyl- oder monocyclisches Heterocyclyl- mit 5 oder 6 Ringatomen steht, und
R⁹ für Wasserstoff, C₁-C₆-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze.

6. Verbindungen der allgemeinen Formel (I), gemäß den Ansprüchen 1 bis 3, in der
X für ein Sauerstoffatom steht, und
A für einen Phenyl- oder Pyridylring steht, und
R^{1a} für Wasserstoff, Halogen, Cyano, Carboxy, Amino oder Aminosulfonyl steht, oder
für einen C₁-C₆-Alkoxy-, C₁-C₃-Alkoxy-C₁-C₃-Alkyl-, C₁-C₃-Alkoxy-C₂-C₃-Alkoxy-, C₁-C₃-Alkylamino-, C₁-C₃-Alkylcarbonylamino, C₁-C₃-Alkylamino-C₁-C₃-Alkyl-, Hydroxy-C₁-C₃-Alkyl-, Fluor-C₁-C₃-Alkyl-, Fluor-C₁-C₃-Alkoxy-, C₁-C₃-Alkylcarbonyl- oder C₁-C₄-Alkoxycarbonyl-Rest steht,
oder
für einen monozyklischen Heterocyclylrest mit 4 bis 7 Ringatomen steht, der gegebenenfalls ein- oder mehrfach, gleich oder verschieden substituiert sein kann mit Halogen, Cyano, Nitro, Hydroxy, Amino, Oxo, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, Phenyl-, Halophenyl-, Phenyl-C₁-C₃-Alkyl-, Pyridinyl-, -NR⁶C(=O)-R⁹,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen,
oder
für einen monozyklischen Heteroarylrest mit 5 oder 6 Ringatomen steht, der gegebenenfalls ein- oder mehrfach, gleich oder verschieden substituiert sein kann mit Halogen, Hydroxy, Amino, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, und/oder mit einem monocyclischen Heterocyclylrest mit 4 bis 7 Ringatomen, und/oder mit einem monocyclischen Heteroarylrest mit 5 oder 6 Ringatomen, und/oder mit einem Phenylrest, der seinerseits gegebenenfalls ein- oder mehrfach, gleich oder verschieden mit Fluor, Chlor, Brom, Methyl- und/oder Methoxy- substituiert sein kann,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl, C₁-C₃-Alkoxy-, C₁-C₂-Alkoxy-C₁-C₂-Alkyl-, C₁-C₃-Alkylamino-, Amino-C₁-C₃-Alkyl-, -C(=O)NR⁶R⁷, -C(=O)R⁸, C₁-C₃-Alkylsulfinyl-, C₁-C₃-Alkylsulfonyl-, -S(=O)₂NH₂, C₁-C₃-Alkyl-sulfonylamino-, C₁-C₃-Alkylaminosulfonyl-, C₃-C₆-Cycloalkylamino-sulfonyl-, Fluor-C₁-C₃-Alkyl-, Fluor-C₁-C₃-Alkoxy-, Hydroxy-C₁-C₃-Alkyl-, C₃-C₆-Cycloalkyl- und/oder einem monocyclischen Heterocyclylrest mit 4 bis 7 Ringatomen, und/oder einem monocyclischen Heteroarylrest mit 5 oder 6 Ringatomen, der seinerseits gegebenenfalls ein- oder mehrfach, gleich oder verschieden substituiert sein kann mit Fluor, Chlor, Brom, Methyl- und/oder Methoxy-, und
R^{1b} für Wasserstoff, Halogen, Hydroxy, Cyano, Nitro oder für einen C₁-C₃-Alkyl-, C₁-C₃-Alkoxy-, Fluor-C₁-C₃-Alkyl- oder Fluor-C₁-C₃-Alkoxy-Rest steht, und
R^{1c} für Wasserstoff, Fluor, Chlor, Brom oder Cyano steht, und
R² für Methyl-, Ethyl- oder Isopropyl- steht, und
R³ für Cyclopropyl-, C₁-C₃-Alkyl-, C₁-C₃-Alkoxy-, Cyclopropylamino- oder C₁-C₃-Alkylamino- steht, und
R⁴ für Wasserstoff, Hydroxy, Cyano, Nitro, Amino, Aminocarbonyl, Fluor, Chlor, Brom, C₁-C₆-Alkyl-, C₁-C₆-Alkoxy-, C₁-C₆-Alkylamino-, C₁-C₆-Alkylcarbonylamino-, C₁-C₆-Alkylaminocarbonyl- oder C₁-C₆-Alkylaminosulfonyl- steht, und
R⁵ für einen C₃-C₇-Cycloalkylrest steht, der gegebenenfalls ein- oder mehrfach, gleich oder verschieden substituiert sein kann mit Halogen, Amino, Hydroxy, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Fluor-C₁-C₃-Alkyl-, Fluor-C₁-C₃-Alkoxy-, und/ oder einem monocyclischen Heterocyclylrest mit 4 bis 7 Ringatomen,
oder
für monozyklisches Heteroaryl- mit 5 oder 6 Ringatomen steht, das gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Hydroxy-C₁-C₃-Alkyl-, C₁-C₃-Alkylamino-, Amino-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für monozyklisches Heterocyclyl- mit 4 bis 7 Ringatomen steht, das gegebenenfalls ein- oder mehrfach, gleich oder verschieden substituiert sein kann mit Halogen, Amino, Hydroxy, Cyano, Oxo, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, Hydroxy-C₁-C₃-Alkyl-, Fluor-C₁-C₃-Alkyl-, Fluor-C₁-C₃-Alkoxy-, C₃-C₆-Cycloalkyl-, -C(=O)R⁸, -S(=O)₂R⁹, -NR⁶R⁷, und/oder einem monocyclischen Heterocyclylrest mit 4 bis 7 Ringatomen,
oder
für einen Phenylrest steht, der gegebenenfalls ein- oder mehrfach, gleich oder verschieden substituiert sein kann mit Halogen, Amino, Hydroxy, Cyano, Nitro, Carboxy, C₁-C₃-Alkyl-, C₁-C₃-Alkoxy-, C₁-C₂-Alkoxy-C₁-C₂-Alkyl-, C₁-C₃-Alkylamino-, Amino-C₁-C₃-Alkyl-, C₁-C₃-Alkylamino-carbonyl, C₁-C₃-Alkylaminosulfonyl-, Hydroxy-C₁-C₃-Alkyl-, Fluor-C₁-C₃-Alkyl-, Fluor-C₁-C₃-Alkoxy-, C₃-C₆-Cycloalkyl-, und/oder einem monocyclischen Heterocyclylrest mit 4 bis 7 Ringatomen, und
R⁶ und R⁷ unabhängig voneinander für Wasserstoff, C₁-C₃-Alkyl-, Cyclopropyl-, Di-C₁-C₃-Alkyl-Amino-C₁-C₃-Alkyl- oder Fluorpyridyl- stehen, und
R⁸ für Hydroxy, C₁-C₆-Alkyl-, Hydroxy-C₁-C₃-Alkyl-, Fluor-C₁-C₃-Alkyl-, C₁-C₃-Alkoxy-C₁-C₃-Alkyl-, C₃-C₈-Cycloalkyl- oder monocyclisches Heterocyclyl- mit 5 oder 6 Ringatomen steht, und
R⁹ für Wasserstoff, C₁-C₆-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze.

7. Verbindungen der allgemeinen Formel (I), gemäß den Ansprüchen 1 bis 3, in der
X für ein Sauerstoffatom steht, und
A für einen Phenyl- oder 3-Pyridylring steht, und
R^{1a} für Wasserstoff oder Chlor steht,
oder
für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl-, die gegebenenfalls ein- oder mehrfach, gleich oder verschieden substituiert sein können mit Fluor, Chlor, Cyano, Nitro, Hydroxy, Oxo, C₁-C₃-Alkyl-, C₁-C₃-Alkoxy-, C₁-C₂-Alkoxy-C₁-C₂-Alkyl-, Hydroxy-C₁-C₃-Alkyl-, Dimethylamino-, Trifluormethyl-, Difluorethyl, Trifluorethyl-, Trifluormethoxy-, Cyclopropyl-, Phenyl-, Fluorphenyl-, Phenyl-C₁-C₃-Alkyl-, Pyridinyl-, -NR⁶C(=O)-R⁹, -C(=O)-NR⁶R⁷, -C(=O)-R⁸, -S(=O)₂-NR⁶R⁷, -S(=O)-R⁹, -S(=O)₂-R⁹ und/oder -NH-S(=O)₂-R⁹ steht,
oder
für Tetrazolyl- steht,
oder
für Isoxazolyl-, Pyrazolyl-, Thienyl-, Thiazolyl-, Imidazolyl-, Triazolyl-, Pyrrolyl-, Oxadiazolyl-, Pyridinyl- oder Pyrimidinyl- steht, die gegebenenfalls ein- oder mehrfach, gleich oder verschieden substituiert sein können mit Fluor, Chlor, Hydroxy, Amino, Cyano, Nitro, C₁-C₃-Alkyl-, C₁-C₃-Alkoxy-, C₁-C₂-Alkoxy-C₁-C₂-Alkyl-, Dimethylamino-, Trifluormethyl-, Difluorethyl-, Trifluorethyl-, Trifluormethoxy-, Cyclopropyl, Pyridinyl-, Phenyl-, Fluorphenyl-, -C(=O)-NR⁶R⁷, -C(=O)-R⁸, -S(=O)₂-NR⁶R⁷, -S(=O)-R⁹, -S(=O)₂-R⁹, und/oder -NH-S(=O)₂-R⁹,
oder
für einen Phenylrest steht, der gegebenenfalls ein- oder mehrfach, gleich oder verschieden substituiert sein kann mit Fluor, Chlor, Amino, Hydroxy, Cyano, Nitro, Carboxy, C₁-C₃-Alkyl-, C₁-C₃-Alkoxy-, C₁-C₂-Alkoxy-C₁-C₂-Alkyl-, Dimethylamino-, -C(=O)NR⁶R⁷, -C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Trifluormethyl-, Difluorethyl-, Trifluorethyl-, Trifluormethoxy-, Hydroxy-C₁-C₃-Alkyl-, Cyclopropyl-, Chlorthienyl-, Morpholino- und/oder Pyridinyl-, und
R^{1b} für Wasserstoff, Fluor, Brom oder Cyano steht,
R^{1c} für Wasserstoff oder Brom steht, und
R² für Methyl-, Ethyl- oder Isopropyl- steht, und
R³ für Cyclopropyl-, Methyl-, Ethyl-, Methoxy-, Ethoxy-, Cyclopropylamino-, Methylamino- oder Ethylamino- steht, und
R⁴ und R⁵ unabhängig voneinander stehen für Wasserstoff, Hydroxy, Cyano, Amino, Chlor, C₁-C₆-Alkyl-, Methoxy-, Ethoxy- oder C₁-C₃-Alkylcarbonylamino-,
oder
für Difluormethoxy- oder Trifluormethoxy-, oder für C₁-C₃-Alkoxy-, welches mit Pyridinyl-, Morpholinyl-, Pyrrolidinyl-oder Piperazinyl- substituiert sein kann, worin Pyridinyl- und Piperazinyl- ihrerseits gegebenenfalls substituiert sein können mit C₁-C₃-Alkyl-,
oder
für Cyclopropyl-,
oder
für Pyridinyl-, Pyrazolyl-, Triazolyl- oder Isoxazolyl-, die gegebenenfalls ein- oder mehrfach, gleich oder verschieden substituiert sein können mit Hydroxy und/oder Methyl-,
oder
für Pyrrolidinyl-, Morpholinyl-, Piperidinyl-, Piperazinyl-, Oxazolidinyl-, Thiomorpholinyl-, die gegebenenfalls ein- oder mehrfach substituiert sein können mit Oxo, Methyl-, und/oder -S(=O)₂R⁹,
oder
für gegebenenfalls mit C₁-C₃-Alkylaminosulfonyl- oder Fluor substituiertes Phenyl, und
R⁶ und R⁷ unabhängig voneinander für Wasserstoff, C₁-C₃-Alkyl-, Cyclopropyl-, Di-C₁-C₃-Alkyl-Amino-C₁-C₃-Alkyl- oder Fluorpyridyl stehen, und
R⁸ für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
R⁹ für C₁-C₄-Alkyl- oder C₁-C₄-Alkoxy- steht,
sowie deren Polymorphe, Enantiomere, Diastereomere, Razemate, Tautomere, Solvate, physiologisch verträglichen Salze und Solvate dieser Salze,
mit der Maßgabe, dass,
wenn A für einen Phenylring steht und R⁴ und R⁵ unabhängig voneinander stehen für Wasserstoff, Hydroxy, Cyano, Amino, Chlor, C₁-C₆-Alkyl-, Methoxy-, Ethoxy- oder C₁-C₃-Alkylcarbonylamino-, oder für Difluormethoxy- oder Trifluormethoxy-, oder für C₁-C₃-Alkoxy-, welches mit Pyridinyl-, Morpholinyl-, Pyrrolidinyl- oder Piperazinyl- substituiert sein kann,
worin Pyridinyl- und Piperazinyl- ihrerseits gegebenenfalls mit C₁-C₃-Alkyl- substituiert sein können,
R^{1a} nicht für Wasserstoff oder Chlor steht, und
wobei die Maßgabe nicht solche Verbindungen der allgemeinen Formel (I) umfasst, in der A für Phenyl steht und R⁴ für Wasserstoff oder Chlor steht und R⁵ für Trifluormethoxy steht, und R^{1a} für Chlor steht,
und auch nicht solche Verbindungen der allgemeinen Formel (I), in der A für Phenyl steht und R⁴ für Wasserstoff steht und R⁵ für C₁-C₃-Alkoxy steht, welches mit Morpholinyl-, Pyrrolidinyl-, Piperazinyl- oder Pyridyl- substituiert ist, worin das Piperazinyl- und Pyridinyl- selbst durch C₁-C₃-Alkyl- substituiert sein kann, und R^{1a} für Chlor steht.

8. Verbindungen der allgemeinen Formel (I), gemäß den Ansprüchen 1 bis 3 und 7, in der
X für ein Sauerstoffatom steht, und
A für einen Phenyl- oder 3-Pyridylring steht, und
R^{1a} für Wasserstoff oder Chlor steht,
oder
für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl- steht, die gegebenenfalls ein- oder mehrfach, gleich oder verschieden substituiert sein können mit Fluor, Hydroxy, Oxo, C₁-C₃-Alkyl-, Methoxy, Hydroxy-C₁-C₃-Alkyl-, Dimethylamino-, Difluorethyl-, Trifluorethyl-, Benzyl-, -NR⁶C(=O)-R⁹, -C(=O)-NR⁶R⁷, -C(=O)-R⁸, und/oder -S(=O)₂-R⁹,
oder
für Tetrazolyl- steht,
oder
für Isoxazolyl-, Pyrazolyl-, Thienyl-, Thiazolyl-, Imidazolyl-, Triazolyl-, Pyrrolyl-, Oxadiazolyl-, Pyridinyl- oder Pyrimidinyl- steht, die gegebenenfalls ein- oder mehrfach, gleich oder verschieden substituiert sein können mit Fluor, Chlor, Hydroxy, Cyano, C₁-C₂-Alkyl-, Methoxy-, Methoxymethyl-, Trifluormethyl-, Cyclopropyl, Pyridinyl-, Phenyl-, Fluorphenyl- und/oder -C(=O)-R⁸,
oder
für einen Phenylrest steht, der gegebenenfalls ein- oder mehrfach, gleich oder verschieden substituiert sein kann mit Fluor, Chlor, Hydroxy, Cyano, Nitro, Carboxy, C₁-C₃-Alkyl-, Methoxy-, -C(=O)NR⁶R⁷, -C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Trifluormethyl-, Trifluormethoxy-, Hydroxy-C₁-C₃-Alkyl-, Cyclopropyl-, Chlorthienyl- und/oder Morpholino-, und
R^{1b} für Wasserstoff, Fluor, Brom oder Cyano steht, und
R^{1c} für Wasserstoff oder Brom steht, und
R² für Methyl-, Ethyl- oder Isopropyl- steht, und
R³ für Cyclopropyl-, Methyl-, Ethyl-, Methoxy-, Ethoxy-, Cyclopropylamino-, Methylamino- oder Ethylamino- steht, und
R⁴ und R⁵ unabhängig voneinander stehen für Wasserstoff, Hydroxy, Cyano, Amino, Chlor, C₁-C₆-Alkyl-, Methoxy-, Ethoxy- oder C₁-C₃-Alkylcarbonylamino-,
oder
für Difluormethoxy- oder Trifluormethoxy-,
oder für C₁-C₃-Alkoxy-, welches mit Pyridinyl-, Morpholinyl-, Pyrrolidinyl- oder Piperazinyl- substituiert sein kann,
worin Pyridinyl- und Piperazinyl- ihrerseits gegebenenfalls substituiert sein können mit C₁-C₃-Alkyl-,
oder
für Cyclopropyl-,
oder
für Pyridinyl-, Pyrazolyl-, Triazolyl- oder Isoxazolyl-, die gegebenenfalls ein- oder mehrfach, gleich oder verschieden substituiert sein können mit Hydroxy und/oder Methyl-,
oder
für Pyrrolidinyl-, Morpholinyl-, Piperidinyl-, Piperazinyl-, Oxazolidinyl-, Thiomorpholinyl-, die gegebenenfalls ein- oder mehrfach substituiert sein können mit Methyl-, Oxo, und/oder -S(=O)₂R⁹,
oder
für gegebenenfalls mit C₁-C₃-Alkylaminosulfonyl- oder Fluor substituiertes Phenyl, und
R⁶ und R⁷ unabhängig voneinander für Wasserstoff, C₁-C₃-Alkyl-, Cyclopropyl-, Di-C₁-C₃-Alkyl-Amino-C₁-C₃-Alkyl- oder Fluorpyridyl stehen, und
R⁸ für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
R⁹ für C₁-C₄-Alkyl- oder C₁-C₄-Alkoxy- steht, sowie deren Polymorphe,
Enantiomere, Diastereomere, Razemate, Tautomere, Solvate, physiologisch verträglichen Salze und Solvate dieser Salze,
mit der Maßgabe, dass,
wenn A für einen Phenylring steht und R⁴ und R⁵ unabhängig voneinander stehen für Wasserstoff, Hydroxy, Cyano, Amino, Chlor, C₁-C₆-Alkyl-, Methoxy-, Ethoxy- oder C₁-C₃-Alkylcarbonylamino-, oder für Difluormethoxy- oder Trifluormethoxy-, oder für C₁-C₃-Alkoxy-, welches mit Pyridinyl-, Morpholinyl-, Pyrrolidinyl- oder Piperazinyl- substituiert sein kann,
worin Pyridinyl- und Piperazinyl- ihrerseits gegebenenfalls mit C₁-C₃-Alkyl- substituiert sein können,
R^{1a} nicht für Wasserstoff oder Chlor steht, und
wobei die Maßgabe nicht solche Verbindungen der allgemeinen Formel (I) umfasst, in der A für Phenyl steht und R⁴ für Wasserstoff oder Chlor steht und R⁵ für Trifluormethoxy steht, und R^{1a} für Chlor steht,
und auch nicht solche Verbindungen der allgemeinen Formel (I), in der A für Phenyl steht und R⁴ für Wasserstoff steht und R⁵ für C₁-C₃-Alkoxy steht, welches mit Morpholinyl-, Pyrrolidinyl-, Piperazinyl- oder Pyridyl- substituiert ist, worin das Piperazinyl- und Pyridinyl- selbst durch C₁-C₃-Alkyl- substituiert sein kann, und R^{1a} für Chlor steht.

9. Verbindungen der allgemeinen Formel (I), gemäß den Ansprüchen 1 bis 3 und 7 bis 8, in der
X für ein Sauerstoffatom steht, und
A für einen Phenylring steht, und
R^{1a} für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl- steht, die gegebenenfalls ein- oder mehrfach, gleich oder verschieden substituiert sein können mit Fluor, Hydroxy, Oxo, C₁-C₃-Alkyl-, Methoxy, Hydroxy-C₁-C₃-Alkyl-, Dimethylamino-, Difluorethyl-, Trifluorethyl-, Benzyl-, -NR⁶C(=O)-R⁹, -C(=O)-NR⁶R⁷, -C(=O)-R⁸, und/oder -S(=O)₂-R⁹,
oder
für Tetrazolyl- steht,
oder
für Isoxazolyl-, Pyrazolyl-, Thienyl-, Thiazolyl-, Imidazolyl-, Triazolyl-, Pyrrolyl-, Oxadiazolyl-, Pyridinyl- oder Pyrimidinyl- steht, die gegebenenfalls ein- oder mehrfach, gleich oder verschieden substituiert sein können mit Fluor, Chlor, Hydroxy, Cyano, C₁-C₂-Alkyl-, Methoxy-, Methoxymethyl-, Trifluormethyl-, Cyclopropyl, Pyridinyl-, Phenyl-, Fluorphenyl- und/oder -C(=O)-R⁸,
oder
für einen Phenylrest steht, der gegebenenfalls ein- oder mehrfach, gleich oder verschieden substituiert sein kann mit Fluor, Chlor, Hydroxy, Cyano, Nitro, Carboxy, C₁-C₃-Alkyl-, Methoxy-, -C(=O)NR⁶R⁷, C(=O)R⁸, C₁-C₃-Alkylsulfinyl-, C₁-C₃-Alkylsulfonyl-, -S(=O)₂NH₂, C₁-C₃-Alkylsulfonylamino-, C₁-C₃-Alkylaminosulfonyl-, C₃-C₆-Cycloalkylaminosulfonyl-, Trifluormethyl-, Trifluormethoxy-, Hydroxy-C₁-C₃-Alkyl-, Cyclopropyl-, Chlorthienyl- und/oder Morpholino-,
R^{1b} für Wasserstoff, Fluor, Brom oder Cyano steht, und
R^{1c} für Wasserstoff steht, und
R² für Methyl- oder Ethyl- steht, und
R³ für Methylamino- steht, und
R⁴ und R⁵ unabhängig voneinander für Wasserstoff, Hydroxy, Cyano, Chlor, C₁-C₆-Alkyl-, Methoxy-, Ethoxy- oder C₁-C₃-Alkylcarbonylamino-,
oder
Difluormethoxy- oder Trifluormethoxy- stehen, und
R⁶ und R⁷ unabhängig voneinander für Wasserstoff, C₁-C₃-Alkyl-, Cyclopropyl- oder Di-C₁-C₃-Alkyl-Amino-C₁-C₃-Alkyl- stehen, und
R⁸ für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
R⁹ für C₁-C₄-Alkyl- oder C₁-C₄-Alkoxy- steht,
sowie deren Polymorphe, Tautomere, Solvate, physiologisch verträglichen Salze und Solvate dieser Salze, und
wobei das Stereozentrum, welches durch das an R² gebundene Kohlenstoffatom des Benzodiazepin-Gerüsts repräsentiert wird, entweder racemisch oder überwiegend oder vollständig in der (*S*)-Konfiguration vorliegt.

10. Verbindungen der allgemeinen Formel (I), gemäß den Ansprüchen 1 bis 3 und 7 bis 8, in der
X für ein Sauerstoffatom steht, und
A für einen Phenylring steht, und
R^{1a} für Wasserstoff oder Chlor steht, und
R^{1b} für Wasserstoff, Fluor, Brom oder Cyano steht, und
R^{1c} für Wasserstoff steht, und
R² für Methyl- oder Ethyl- steht, und
R³ für Methylamino- steht, und
R⁴ für Cyclopropyl- steht,
oder
für Pyridinyl-, Pyrazolyl-, Triazolyl- oder Isoxazolyl- steht, die gegebenenfalls ein- oder mehrfach, gleich oder verschieden substituiert sein können mit Hydroxy und/oder Methyl-,
oder
für Pyrrolidinyl-, Morpholinyl-, Piperidinyl-, Piperazinyl-, Oxazolidinyl- oder Thiomorpholinyl- steht, die gegebenenfalls ein- oder mehrfach substituiert sein können mit Methyl-, Oxo und/oder -S(=O)₂R⁹,
oder
für gegebenenfalls mit C₁-C₃-Alkylaminosulfonyl- oder Fluor substituiertes Phenyl- steht, und
R⁵ für Wasserstoff, Hydroxy, Cyano, Chlor, C₁-C₆-Alkyl-, Methoxy-, Ethoxy- oder C₁-C₃-Alkylcarbonylamino- steht,
oder
für Difluormethoxy- oder Trifluormethoxy- steht, und
R⁶ und R⁷ unabhängig voneinander für Wasserstoff, C₁-C₃-Alkyl-, Cyclopropyl- oder Di-C₁-C₃-Alkyl-Amino-C₁-C₃-Alkyl- stehen, und
R⁸ für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
R⁹ für C₁-C₄-Alkyl- oder C₁-C₄-Alkoxy- steht,
sowie deren Polymorphe, Tautomere, Solvate, physiologisch verträglichen Salze und Solvate dieser Salze, und
wobei das Stereozentrum, welches durch das an R² gebundene Kohlenstoffatom des Benzodiazepin-Gerüsts repräsentiert wird, entweder racemisch oder überwiegend oder vollständig in der (*S*)-Konfiguration vorliegt.

11. Verbindungen der allgemeinen Formel (I), gemäß den Ansprüchen 1 bis 3 und 7 bis 8, in der
X für ein Sauerstoffatom steht, und
A für einen Phenylring steht, und
R^{1a} für Wasserstoff oder Chlor steht, und
R^{1b} für Wasserstoff, Fluor, Brom oder Cyano steht, und
R^{1c} für Wasserstoff steht, und
R² für Methyl- oder Ethyl- steht, und
R³ für Methylamino- steht, und
R⁴ für Wasserstoff, Chlor, Methoxy- oder Ethoxy- steht,
oder
für Difluormethoxy- oder Trifluormethoxy- steht, und
R⁵ für Cyclopropyl- steht,
oder
für Pyridinyl- oder Pyrazolyl- steht, die gegebenenfalls ein- oder mehrfach, mit Methyl- substituiert sein können,
oder
für Morpholinyl-, Piperidinyl-, Piperazinyl- oder Thiomorpholinyl- steht, die gegebenenfalls ein- oder mehrfach substituiert sein können mit Methyl-, Oxo und/oder -S(=O)₂R⁹,
oder
für mit C₁-C₃-Alkylaminosulfonyl- substituiertes Phenyl steht, und
R⁶ und R⁷ unabhängig voneinander für Wasserstoff, C₁-C₃-Alkyl-, Cyclopropyl- oder Di-C₁-C₃-Alkyl-Amino-C₁-C₃-Alkyl- stehen, und
R⁸ für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
R⁹ für C₁-C₄-Alkyl- oder C₁-C₄-Alkoxy- steht,
sowie deren Polymorphe, Tautomere, Solvate, physiologisch verträglichen Salze und Solvate dieser Salze, und
wobei das Stereozentrum, welches durch das an R² gebundene Kohlenstoffatom des Benzodiazepin-Gerüsts repräsentiert wird, entweder racemisch oder überwiegend oder vollständig in der (*S*)-Konfiguration vorliegt.

12. Verbindungen der allgemeinen Formel (I), gemäß den Ansprüchen 1 bis 3 und 7 bis 8, in der
X für ein Sauerstoffatom steht, und
A für einen Phenylring steht, und
R^{1a} für Piperazinyl-, Pyrrolidinyl-, Piperidinyl-, Diazepanyl-, Oxazinanyl-, Oxazolidinyl-, Morpholinyl-, Thiomorpholinyl, Thiazolidinyl- oder Azetidinyl- steht, die gegebenenfalls ein- oder mehrfach, gleich oder verschieden substituiert sein können mit Fluor, Hydroxy, Oxo, C₁-C₃-Alkyl-, Methoxy, Hydroxy-C₁-C₃-Alkyl-, Dimethylamino-, Difluorethyl-, Trifluorethyl-, Benzyl-, -NR⁶C(=O)-R⁹, -C(=O)-NR⁶R⁷, -C(=O)-R⁸, und/oder -S(=O)₂-R⁹,
oder
für Isoxazolyl- oder Pyrazolyl- steht, die gegebenenfalls ein- oder mehrfach, gleich oder verschieden mit C₁-C₂-Alkyl-substituiert sein können, und
R^{1b} für Wasserstoff, Fluor, Brom oder Cyano steht, und
R^{1c} für Wasserstoff steht, und
R² für Methyl- steht, und
R³ für Methylamino- steht, und
R⁴ und R⁵ unabhängig voneinander für Wasserstoff, Hydroxy, Cyano, Chlor, C₁-C₆-Alkyl-, Methoxy-, Ethoxy- oder C₁-C₃-Alkylcarbonylamino- stehen,
oder
für Difluormethoxy- oder Trifluormethoxy- stehen, und
R⁶ und R⁷ unabhängig voneinander für Wasserstoff, C₁-C₃-Alkyl-, Cyclopropyl- oder Di-C₁-C₃-Alkyl-Amino-C₁-C₃-Alkyl- stehen, und
R⁸ für Hydroxy, C₁-C₃-Alkyl-, Hydroxy-C₁-C₃-Alkyl-, Trifluormethyl-, Pyrrolidinyl-, Morpholinyl- oder Piperidinyl- steht, und
R⁹ für C₁-C₄-Alkyl- oder C₁-C₄-Alkoxy- steht,
sowie deren Polymorphe, Tautomere, Solvate, physiologisch verträglichen Salze und Solvate dieser Salze, und
wobei das Stereozentrum, welches durch das an R² gebundene Kohlenstoffatom des Benzodiazepin-Gerüsts repräsentiert wird, entweder racemisch oder überwiegend oder vollständig in der (*S*)-Konfiguration vorliegt.

13. Verbindungen der allgemeinen Formel (I), gemäß den Ansprüchen 1 bis 3, 7 bis 8 und 12 in der
X für ein Sauerstoffatom steht, und
A für einen Phenylring steht, und
R^{1a} für Piperazinyl-, Pyrrolidinyl-, Piperidinyl-, Morpholinyl-, Thiomorpholinyl- oder Azetidinyl- steht, die gegebenenfalls ein- oder mehrfach, gleich oder verschieden substituiert sein können mit Hydroxy, Oxo, C₁-C₃-Alkyl-, Methoxy-, Dimethylamino-, Difluorethyl-, Trifluorethyl-, -NR⁶C(=O)-R⁹, - C(=O)-NR⁶R⁷, und/oder -C(=O)-R⁸, und
R^{1b} für Wasserstoff, Fluor, Brom oder Cyano steht, und
R^{1c} für Wasserstoff steht, und
R² für Methyl- steht, und
R³ für Methylamino- steht, und
R⁴ und R⁵ unabhängig voneinander für Wasserstoff, Chlor, Methoxy- oder Ethoxystehen,
oder
für Difluormethoxy- oder Trifluormethoxy- stehen, und
R⁶ und R⁷ unabhängig voneinander für Wasserstoff oder C₁-C₃-Alkyl- stehen, und
R⁸ für Methyl- steht, und
R⁹ für Methyl- steht,
sowie deren Polymorphe, Tautomere, Solvate, physiologisch verträglichen Salze und Solvate dieser Salze, und
wobei das Stereozentrum, welches durch das an R² gebundene Kohlenstoffatom des Benzodiazepin-Gerüsts repräsentiert wird, entweder racemisch oder überwiegend oder vollständig in der (*S*)-Konfiguration vorliegt.

14. Verbindungen der allgemeinen Formel (I) gemäß den Ansprüchen 1 bis 3, in der A für einen Phenylring steht und R⁴ für Wasserstoff oder Chlor steht und R⁵ für Trifluormethoxy steht, und R^{1a} für Chlor steht,
und Verbindungen der allgemeinen Formel (I), in der A für einen Phenylring steht und R⁴ für Wasserstoff steht und R⁵ für C₁-C₃-Alkoxy, welches mit Morpholinyl-, Pyrrolidinyl-, Piperazinyl- oder Pyridyl- substituiert sein kann, und das Piperazinyl- und Pyridinyl- selbst mit C₁-C₃-Alkyl- substituiert sein kann, steht, und R^{1a} für Chlor steht.

15. Verbindungen der allgemeinen Formel (I), gemäß den Ansprüchen 1 bis 14,
- (±)-1-(4-Chlorphenyl)-*N*,4-dimethyl-8-(trifluormethoxy)-4,5-dihydro-3*H*-2,3-benzodiazepin-3-carboxamid
- (4*S*)-1-(4-Chlorphenyl)-*N*,4-dimethyl-8-(trifluormethoxy)-4,5-dihydro-3*H*-2,3-benzodiazepin-3-carboxamid
- (4*R*)-1-(4-Chlorphenyl)-*N*,4-dimethyl-8-(trifluormethoxy)-4,5-dihydro-3*H*-2,3-benzodiazepin-3-carboxamid
- (±)-1-[4-(3,5-Dimethyl-4-isoxazolyl)phenyl]-7,8-dimethoxy-N,4-dimethyl-4,5-dihydro-3*H-*2,3-benzodiazepin-3-carboxamid
- (4*R*)-1-[4*-*(3,5-Dimethyl-4-isoxazolyl)phenyl]-7,8-dimethoxy-*N*,4*-*dimethyl-4,5-dihydro-3*H*-2,3-benzodiazepin-3-carboxamid
- (4*S*)-1-[4-(3,5-Dimethyl-4-isoxazolyl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4-(1*H*-pyrazol-3-yl)phenyl]-4,5-dihydro-3*H*-2,3-benzodiazepin-3-carboxamid
- (±)-1-[4-(2-Chlorpyridin-3-yl)phenyl]-7,8-dimethoxy-*N*,4-dimethyl-4,5-dihydro-3*H*-2,3-benzodiazepin-3-carboxamid
- (±)-5-(4-{7,8-Dimethoxy-4-methyl-3-[(methylamino)carbonyl]-4,5-dihydro-3*H*-2,3-benzodiazepin-1-yl}phenyl)thiophen-2-carbonsäure
- (±)-4'-{7,8-Dimethoxy-4-methyl-3-[(methylamino)carbonyl]-4,5-dihydro-3*H*-2,3-benzodiazepin-1-yl}biphenyl-2-carbonsäure
- (±)-7,8-Dimethoxy-*N*,4-dimethyl-1-[4-(morpholin-4-yl)phenyl]-4,5-dihydro-3*H*-2,3-benzodiazepin-3-carboxamid
- (±)-1-(4-Chlorphenyl)-*N*,4-dimethyl-8-(pyridin-4-yl)-4,5-dihydro-3*H*-2,3-benzodiazepin-3-carboxamid
- (±)-1-(4-Chlorphenyl)-8-cyclopropyl-*N*,4-dimethyl-4,5-dihydro-3*H*-2,3-benzodiazepin-3-carboxamid
- (±)-1-(4-Chlorphenyl)-*N*,4-dimethyl-8-{4-[(methylamino)sulfonyl]phenyl}-4,5-dihydro-3*H*-2,3-benzodiazepin-3-carboxamid
- (±)-1-(4-Chlorphenyl)-*N*,4-dimethyl-8-(morpholin-4-yl)-4,5-dihydro-3*H*-2,3-benzodiazepin-3-carboxamid
- (4*S*)-1-(4-Chlorphenyl)-*N*,4-dimethyl-8-(morpholin-4-yl)-4,5-dihydro-3*H*-2,3-benzodiazepin-3-carboxamid
- (4*R*)-1-(4-Chlorphenyl)-*N*,4-dimethyl-8-(morpholin-4-yl)-4,5-dihydro-3*H*-2,3-benzodiazepin-3-carboxamid
- (±)-1-(4-Chlorphenyl)-*N*,4-dimethyl-8-(4-methylpiperazin-1-yl)-4,5-dihydro-3*H*-2,3-benzodiazepin-3-carboxamid
- (±)-1-(4-Chlorphenyl)-*N*,4-dimethyl-8-(piperidin-1-yl)-4,5-dihydro-3*H*-2,3-benzodiazepin-3-carboxamid
- (±)-8-Methoxy-*N*,4-dimethyl-1-(pyridin-3-yl)-4,5-dihydro-3*H*-2,3-benzodiazepin-3-carboxamid
- (±)-7-Chlor-1-(4-chlorphenyl)-*N*,4-dimethyl-8-(trifluormethoxy)-4,5-dihydro-3*H*-2,3-benzodiazepin-3-carboxamid
- 7-Chlor-1-(4-chlorphenyl)-*N*,4-dimethyl-8-(trifluormethoxy)-4,5-dihydro-3*H*-2,3-benzodiazepin-3-carboxamid, Enantiomer 1
- (4*S*)-1-[4-(3,5-Dimethyl-1*H*-pyrazol-1-yl)phenyl]-8-methoxy-*N*,4-dimethyl-4,5-dihydro-3*H*-2,3-benzodiazepin-3-carboxamid
- (4*S*)-8-Methoxy-*N*,4-dimethyl-1-[4-(morpholin-4-yl)phenyl]-4,5-dihydro-3*H*-2,3-benzodiazepin-3-carboxamid
- (4*S*)-1-[4-(4-Isoxazolyl)phenyl]-8-methoxy-*N*,4-dimethyl-4,5-dihydro-3*H*-2,3-benzodiazepin-3-carboxamid
- (4*S*)-8-Methoxy-*N*,4-dimethyl-1-[4-(1-methyl-1H-pyrazol-5-yl)phenyl]-4,5-dihydro-3*H-*2,3-benzodiazepin-3-carboxamid
- (4*S*)-1-[4-(3,5-Dimethyl-4-isoxazolyl)phenyl]-8-methoxy-*N*,4-dimethyl-4,5-dihydro-3*H-*2,3-benzodiazepin-3-carboxamid
- (4*S*)-8-Methoxy-*N*,4-dimethyl-1-[4-(1-methyl-1*H*-pyrazol-4-yl)phenyl]-4,5-dihydro-3*H-*2,3-benzodiazepin-3-carboxamid
- (4*S*)-8-Methoxy-*N*,4-dimethyl-1-[4-(1,3,5-trimethyl-1*H*-pyrazol-4-yl)phenyl]-4,5-dihydro-3*H*-2,3-benzodiazepin-3-carboxamid
- (4*S*)-8-Methoxy-*N*,4-dimethyl-1-[4-(1*H*-pyrazol-5-yl)phenyl]-4,5-dihydro-3*H*-2,3-benzodiazepin-3-carboxamid
- (4*S*)-1-[4-(3-Cyclopropyl-5-ethyl-1*H*-pyrazol-1-yl)phenyl]-8-methoxy-*N*,4-dimethyl-4,5-dihydro-3*H*-2,3-benzodiazepin-3-carboxamid
- (4*S*)-1-[4-(5-Cyclopropyl-3-ethyl-1*H*-pyrazol-1-yl)phenyl]-8-methoxy-*N*,4-dimethyl-4,5-dihydro-3*H*-2,3-benzodiazepin-3-carboxamid
- (4*S*)-8-Methoxy-1-{4-[3-(methoxymethyl)-5-methyl-1*H*-pyrazol-1-yl]phenyl}-*N*,4-dimethyl-4,5-dihydro-3*H*-2,3-benzodiazepin-3-carboxamid
- (4*S*)-8-Methoxy-1-{4-[5-(methoxymethyl)-3-methyl-1*H*-pyrazol-1-yl]phenyl}-*N*,4-dimethyl-4,5-dihydro-3*H*-2,3-benzodiazepin-3-carboxamid
- (4*S*)-1-{4-[5-Cyclopropyl-3-(pyridin-2-yl)-1*H*-pyrazol-1-yl]phenyl}-8-methoxy-*N*,4-dimethyl-4,5-dihydro-3*H*-2,3-benzodiazepin-3-carboxamid
- (4*S*)-1-{4-[3-Cyclopropyl-5-(pyridin-2-yl)-1*H*-pyrazol-1-yl]phenyl}-8-methoxy-*N*,4-dimethyl-4,5-dihydro-3*H*-2,3-benzodiazepin-3-carboxamid
- (4*S*)-8-Methoxy-*N*,4-dimethyl-1-[4-(1*H*-tetrazol-1-yl)phenyl]-4,5-dihydro-3*H*-2,3-benzodiazepin-3-carboxamid
- (±)-1-[3-(3,5-Dimethylisoxazol-4-yl)phenyl]-7,8-dimethoxy-*N*,4-dimethyl-4,5-dihydro-3*H-*2,3-benzodiazepin-3-carboxamid
- (±)-1-(4-Chlorphenyl)-*N*,4-dimethyl-8-[2-(morpholin-4-yl)ethoxy]-4,5-dihydro-3*H*-2,3-benzodiazepin-3-carboxamid
- (±)-1-(4-Chlorphenyl)-*N*,4-dimethyl-8-[2-(pyrrolidin-1-yl)ethoxy]-4,5-dihydro-3*H*-2,3-benzodiazepin-3-carboxamid
- (±)-7,8-Dimethoxy-*N*,4-dimethyl-1-[4-(2-oxooxazolidin-3-yl)phenyl]-4,5-dihydro-3*H*-2,3-benzodiazepin-3-carboxamid
- (4*S*)-7,8-Dimethoxy-*N*,4-dimethyl-1-[4-(2-oxooxazolidin-3-yl)phenyl]-4,5-dihydro-3*H*-2,3-benzodiazepin-3-carboxamid
- (±)-7,8-Dimethoxy-*N*,4-dimethyl-1-[4-(2-oxopiperidin-1-yl)phenyl]-4,5-dihydro-3*H*-2,3-benzodiazepin-3-carboxamid
- (±)-1-[4-(4-Benzyl-2-oxopiperazin-1-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4-(4-methyl-2-oxo-1,4-diazepan-1-yl)phenyl]-4,5-dihydro-3*H*-2,3-benzodiazepin-3-carboxamid
- (±)-7,8-Dimethoxy-*N*,4-dimethyl-1-[4-(2-oxo-1,3-oxazinan-3-yl)phenyl]-4,5-dihydro-3*H-*2,3-benzodiazepin-3-carboxamid
- (±)-7,8-Dimethoxy-*N*,4-dimethyl-1-[4-(2-oxopyrrolidin-1-yl)phenyl]-4,5-dihydro-3*H*-2,3-benzodiazepin-3-carboxamid
- (±)-7,8-Dimethoxy-*N*,4-dimethyl-1-[4-(3-oxomorpholin-4-yl)phenyl]-4,5-dihydro-3*H*-2,3-benzodiazepin-3-carboxamid
- (4*S*)-7,8-Dimethoxy-*N*,4-dimethyl-1-[4-(3-oxomorpholin-4-yl)phenyl]-4,5-dihydro-3*H*-2,3-benzodiazepin-3-carboxamid
- (±)-7,8-Dimethoxy-*N*,4-dimethyl-1-[4-(2-methyl-5-oxomorpholin-4-yl)phenyl]-4,5-dihydro-3*H*-2,3-benzodiazepin-3-carboxamid (Stereoisomerengemisch)
- (±)-7,8-Dimethoxy-*N*,4-dimethyl-1-[4-(2-methyl-3-oxomorpholin-4-yl)phenyl]-4,5-dihydro-3*H*-2,3-benzodiazepin-3-carboxamid (Stereoisomerengemisch)
- (±)-7,8-Dimethoxy-*N*,4-dimethyl-1-[4-(4-methyl-2-oxopiperazin-1-yl)phenyl]-4,5-dihydro-3*H*-2,3-benzodiazepin-3-carboxamid
- (4*S*)-7,8-Dimethoxy-*N*,4-dimethyl-1-[4-(4-methyl-2-oxopiperazin-1-yl)phenyl]-4,5-dihydro-3*H*-2,3-benzodiazepin-3-carboxamid
- (4*R*)-7,8-Dimethoxy-*N*,4-dimethyl-1-[4-(4-methyl-2-oxopiperazin-1-yl)phenyl]-4,5-dihydro-3*H*-2,3-benzodiazepin-3-carboxamid
- (±)-7,8-Dimethoxy-*N*,4-dimethyl-1-[4-(2-oxopiperazin-1-yl)phenyl]-4,5-dihydro-3*H*-2,3-benzodiazepin-3-carboxamid
- (±)-7,8-Dimethoxy-*N*,4-dimethyl-1-[4-(1-methyl-1*H*-1,2,3-triazol-4-yl)phenyl]-4,5-dihydro-3*H*-2,3-benzodiazepin-3-carboxamid
- (4*S*)-7,8-Dimethoxy-*N*,4-dimethyl-1-[4-(1-methyl-1*H*-1,2,3-triazol-4-yl)phenyl]-4,5-dihydro-3*H*-2,3-benzodiazepin-3-carboxamid
- (±)-1-[4-(2,4-Dimethylthiazol-5-yl)phenyl]-7,8-dimethoxy-*N*,4-dimethyl-4,5-dihydro-3*H-*2,3-benzodiazepin-3-carboxamid
- (4*S*)-1-[4-(2,4-Dimethylthiazol-5-yl)phenyl]-7,8-dimethoxy-*N*,4-dimethyl-4,5-dihydro-3*H-*2,3-benzodiazepin-3-carboxamid
- (4*R*)-1-[4-(2,4-Dimethylthiazol-5-yl)phenyl]-7,8-dimethoxy-*N*,4-dimethyl-4,5-dihydro-3*H-*2,3-benzodiazepin-3-carboxamid
- (±)-1-[4-(1,2-Dimethyl-1*H*-imidazol-5-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-{4-[2-(trifluormethyl)pyridin-3-yl]phenyl}-4,5-dihydro-3*H*-2,3-benzodiazepin-3-carboxamid
- (±)-1-[4-(6-Hydroxypyridin-3-yl)phenyl]-7,8-dimethoxy-*N*,4-dimethyl-4,5-dihydro-3*H*-2,3-benzodiazepin-3-carboxamid
- (4*S*)-1-[4-(6-Hydroxypyridin-3-yl)phenyl]-7,8-dimethoxy-*N*,4-dimethyl-4,5-dihydro-3*H-*2,3-benzodiazepin-3-carboxamid
- (±)-7,8-Dimethoxy-*N*,4-dimethyl-1-{4-[6-(trifluormethyl)pyridin-3-yl]phenyl}-4,5-dihydro-3*H*-2,3-benzodiazepin-3-carboxamid
- (±)-7,8-Dimethoxy-*N*,4-dimethyl-1-[4-(1,3,5-trimethyl-1*H*-pyrazol-4-yl)phenyl]-4,5-dihydro-3*H*-2,3-benzodiazepin-3-carboxamid
- (4*R*)-7,8-Dimethoxy-*N*,4-dimethyl-1-[4-(1,3,5-trimethyl-1*H*-pyrazol-4-yl)phenyl]-4,5-dihydro-3*H*-2,3-benzodiazepin-3-carboxamid
- (4*S*)-7,8-Dimethoxy-*N*,4-dimethyl-1-[4-(1,3,5-trimethyl-1*H*-pyrazol-4-yl)phenyl]-4,5-dihydro-3*H*-2,3-benzodiazepin-3-carboxamid
- (±)-1-[4-(Isoxazol-4-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[3-(1,3,5-trimethyl-1*H*-pyrazol-4-yl)phenyl]-4,5-dihydro-3*H*-2,3-benzodiazepin-3-carboxamid
- (±)-7,8-Dimethoxy-*N*,4-dimethyl-1-[3-(1-methyl-1*H*-pyrazol-4-yl)phenyl]-4,5-dihydro-3*H-*2,3-benzodiazepin-3-carboxamid
- (±)-7,8-Dimethoxy-*N*,4-dimethyl-1-[3-(1-methyl-1*H*-pyrazol-5-yl)phenyl]-4,5-dihydro-3*H-*2,3-benzodiazepin-3-carboxamid
- (±)-1-[4-Fluoro-3-(1,3,5-trimethyl-1*H*-pyrazol-4-yl)phenyl]-7,8-dimethoxy-*N*,4-dimethyl-4,5-dihydro-3*H*-2,3-benzodiazepin-3-carboxamid
- (±)-1-[4-Fluoro-3-(1-methyl-1*H*-pyrazol-4-yl)phenyl]-7,8-dimethoxy-*N*,4-dimethyl-4,5-dihydro-3*H*-2,3-benzodiazepin-3-carboxamid
- (±)-1-[3-(3,5-Dimethylisoxazol-4-yl)-4-fluorophenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-(3'-nitrobiphenyl-4-yl)-4,5-dihydro-3*H*-2,3-benzodiazepin-3-carboxamid
- (±)-1-(Biphenyl-4-yl)-7,8-dimethoxy-*N*,4-dimethyl-4,5-dihydro-3*H*-2,3-benzodiazepin-3-carboxamid
- (±)-1-(2',4'-Dichlorbiphenyl-4-yl)-7,8-dimethoxy-*N*,4-dimethyl-4,5-dihydro-3*H*-2,3-benzodiazepin-3-carboxamid
- (±)-1-(4'-Fluorbiphenyl-4-yl)-7,8-dimethoxy-*N*,4-dimethyl-4,5-dihydro-3*H*-2,3-benzodiazepin-3-carboxamid
- (±)-1-(4'-Chlorbiphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-(4'-methylbiphenyl-4-yl)-4,5-dihydro-3*H*-2,3-benzodiazepin-3-carboxamid
- (±)-7,8-Dimethoxy-1-(4'-methoxybiphenyl-4-yl)-*N*,4-dimethyl-4,5-dihydro-3*H*-2,3-benzodiazepin-3-carboxamid
- (±)-7,8-Dimethoxy-1-[4-(6-methoxypyridin-3-yl)phenyl]-*N*,4-dimethyl-4,5-dihydro-3*H-*2,3-benzodiazepin-3-carboxamid
- (±)-7,8-Dimethoxy-*N*,4-dimethyl-1-[4'-(methylsulfinyl)biphenyl-4-yl]-4,5-dihydro-3*H*-2,3-benzodiazepin-3-carboxamid
- (±)-7,8-Dimethoxy-*N*,4-dimethyl-1-{2'-[(methylsulfonyl)amino]biphenyl-4-yl}-4,5-dihydro-3*H*-2,3-benzodiazepin-3-carboxamid
- (±)-7,8-Dimethoxy-*N*,4-dimethyl-1-[2'-(methylsulfonyl)biphenyl-4-yl]-5-dihydro-3*H*-2,3-benzodiazepin-3-carboxamid
- (±)-7,8-Dimethoxy-*N*,4-dimethyl-1-1-4'-[(methylsulfonyl)amino]biphenyl-4-yl}-4,5-dihydro-3*H*-2,3-benzodiazepin-3-carboxamid
- (±)-7,8-Dimethoxy-*N*,4-dimethyl-1-{3'-[(methylsulfonyl)amino]biphenyl-4-yl}-4,5-dihydro-3*H*-2,3-benzodiazepin-3-carboxamid
- (±)-7,8-Dimethoxy-*N*,4-dimethyl-1-(2'-methylbiphenyl-4-yl)-4,5-dihydro-3*H*-2,3-benzodiazepin-3-carboxamid
- (±)-7,8-Dimethoxy-*N*,4-dimethyl-1-[3'-(methylsulfonyl)biphenyl-4-yl]-4,5-dihydro-3*H*-2,3-benzodiazepin-3-carboxamid
- (±)-7,8-Dimethoxy-1-[4-(2-methoxypyrimidin-5-yl)phenyl]-*N*,4-dimethyl-4,5-dihydro-3*H-*2,3-benzodiazepin-3-carboxamid
- (±)-1-(3'-Cyan-4'-fluorbiphenyl-4-yl)-7,8-dimethoxy-*N*,4-dimethyl-4,5-dihydro-3*H*-2,3-benzodiazepin-3-carboxamid
- (±)-7,8-Dimethoxy-1-[4-(2-methoxypyridin-3-yl)phenyl]-*N*,4-dimethyl-4,5-dihydro-3*H-*2,3-benzodiazepin-3-carboxamid
- (±)-1-(3'-Carbamoylbiphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4'-(pyrrolidin-1-ylcarbonyl)biphenyl-4-yl]-4,5-dihydro-3*H*-2,3-benzodiazepine-3-carboxamide
- (±)-7,8-Dimethoxy-*N*,4-dimethyl-1-[4'-(morpholin-4-ylcarbonyl)biphenyl-4-yl]-4,5-dihydro-3*H*-2,3-benzodiazepin-3-carboxamid
- (±)-7,8-Dimethoxy-1-[4-(5-methoxypyridin-3-yl)phenyl]-*N*,4-dimethyl-4,5-dihydro-3*H-*2,3-benzodiazepin-3-carboxamid
- (±)-7,8-Dimethoxy-*N*,4-dimethyl-1-[4-(5-methylpyridin-3-yl)phenyl]-4,5-dihydro-3*H*-2,3-benzodiazepin-3-carboxamid
- (±)-7,8-Dimethoxy-*N*,4-dimethyl-1-[4-(4-methylpyridin-3-yl)phenyl]-4,5-dihydro-3*H*-2,3-benzodiazepin-3-carboxamid
- (±)-1-[4'-(Cyclopropylcarbamoyl)biphenyl-4-yl]-7,8-dimethoxy-*N*,4-dimethyl-4,5-dihydro-3*H*-2,3-benzodiazepin-3-carboxamid
- (±)-1-[4-(3-Fluorpyridin-4-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[3'-(trifluormethyl)biphenyl-4-yl]-4,5-dihydro-3*H*-2,3-benzodiazepin-3-carboxamid
- (±)-7,8-Dimethoxy-*N*,4-dimethyl-1-[4'-(trifluormethyl)biphenyl-4-yl]-4,5-dihydro-3*H*-2,3-benzodiazepin-3-carboxamid
- (±)-7,8-Dimethoxy-1-(3'-methoxybiphenyl-4-yl)-*N*,4-dimethyl-4,5-dihydro-3*H*-2,3-benzodiazepin-3-carboxamid
- (±)-1-[4'-(5-Chlorthien-2-yl)biphenyl-4-yl]-7,8-dimethoxy-*N*,4-dimethyl-4,5-dihydro-3*H-*2,3-benzodiazepin-3-carboxamid
- (±)-1-(3'-Fluorbiphenyl-4-yl)-7,8-dimethoxy-*N*,4-dimethyl-4,5-dihydro-3*H*-2,3-benzodiazepin-3-carboxamid
- (±)-7,8-Dimethoxy-1-(2'-methoxybiphenyl-4-yl)-*N*,4-40dimethyl-4,5-dihydro-3*H*-2,3-benzodiazepin-3-carboxamid
- (±)-7,8-Dimethoxy-*N*,4-dimethyl-1-[2'-(trifluormethyl)biphenyl-4-yl]-4,5-dihydro-3*H*-2,3-benzodiazepin-3-carboxamid
- (±)-1-(2'-Chlorbiphenyl-4-yl)-7,8-dimethoxy-*N*,4-dimethyl-4,5-dihydro-3*H*-2,3-benzodiazepin-3-carboxamid
- (±)-1-(2'-Fluorbiphenyl-4-yl)-7,8-dimethoxy-*N*,4-dimethyl-4,5-dihydro-3*H*-2,3-benzodiazepin-3-carboxamid
- (±)-1-[4'-(Hydroxymethyl)biphenyl-4-yl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4'-(trifluormethoxy)biphenyl-4-yl]-4,5-dihydro-3*H-*2,3-benzodiazepin-3-carboxamid
- (±)-7,8-Dimethoxy-*N*,4-dimethyl-1-[3'-(pyrrolidin-1-ylcarbonyl)biphenyl-4-yl]-4,5-dihydro-3*H*-2,3-benzodiazepin-3-carboxamid
- (±)-7,8-Dimethoxy-*N*,4-dimethyl-1-[3'-(piperidin-1-ylcarbonyl)biphenyl-4-yl]-4,5-dihydro-3*H*-2,3-benzodiazepin-3-carboxamid
- (±)-7,8-Dimethoxy-*N*,4-dimethyl-1-[3'-(morpholin-4-ylcarbonyl)biphenyl-4-yl]-4,5-dihydro-3*H*-2,3-benzodiazepin-3-carboxamid
- (±)-1-[3'-(Cyclopropylcarbamoyl)biphenyl-4-yl]-7,8-dimethoxy-*N*,4-dimethyl-4,5-dihydro-3*H*-2,3-benzodiazepin-3-carboxamid
- (±)-1-(2',4'-Difluorbiphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4-(1-methyl-1*H*-pyrazol-5-yl)phenyl]-4,5-dihydro-3*H-*2,3-benzodiazepin-3-carboxamid
- (±)-7,8-Dimethoxy-*N*,4-dimethyl-1-(4'-nitrobiphenyl-4-yl)-4,5-dihydro-3*H*-2,3-benzodiazepin-3-carboxamid
- (±)-7,8-Dimethoxy-*N*,4-dimethyl-1-[4-(pyridin-3-yl)phenyl]-4,5-dihydro-3*H*-2,3-benzodiazepin-3-carboxamid
- (±)-7,8-Dimethoxy-1-[4-(4-methoxypyridin-3-yl)phenyl]-*N*,4-dimethyl-4,5-dihydro-3*H-*2,3-benzodiazepin-3-carboxamid
- (±)-1-(3'-Cyanbiphenyl-4-yl)-7,8-dimethoxy-*N*,4-dimethyl-4,5-dihydro-3*H*-2,3-benzodiazepin-3-carboxamid
- (±)-1-(4'-Cyanbiphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[2'-(trifluormethoxy)biphenyl-4-yl]-4,5-dihydro-3*H-*2,3-benzodiazepin-3-carboxamid
- (±)-7,8-Dimethoxy-*N*,4-dimethyl-1-[4'-(methylsulfonyl)biphenyl-4-yl]-4,5-dihydro-3*H*-2,3-benzodiazepin-3-carboxamid
- (±)-1-(2'-Cyanbiphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4'-(morpholin-4-yl)biphenyl-4-yl]-4,5-dihydro-3*H*-2,3-benzodiazepin-3-carboxamid
- (±)-7,8-Dimethoxy-*N*,4-dimethyl-1-[4-(pyrimidin-5-yl)phenyl]-4,5-dihydro-3*H*-2,3-benzodiazepin-3-carboxamid
- (±)-1-[2'-(Hydroxymethyl)biphenyl-4-yl]-7,8-dimethoxy-*N*,4-dimethyl-4,5-dihydro-3*H*-2,3-benzodiazepie-3-carboxamid
- (±)-1-(3'-{[2-(Dimethylamino)ethyl]carb-amoyl}biphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-(3'-sulfamoylbiphenyl-4-yl)-4,5-dihydro-3*H*-2,3-benzodiazepin-3-carboxamid
- (±)-7,8-Dimethoxy-*N*,4-dimethyl-1-[4'-(methylsulfamoyl)biphenyl-4-yl]-4,5-dihydro-3*H-*2,3-benzodiazepin-3-carboxamid
- (±)-7,8-Dimethoxy-*N*,4-dimethyl-1-[4-(1-methyl-1*H*-pyrrol-2-yl)phenyl]-4,5-dihydro-3*H-*2,3-benzodiazepin-3-carboxamid
- (±)-7,8-Dimethoxy-*N*,4-dimethyl-1-[4-(6-methylpyridin-3-yl)phenyl]-4,5-dihydro-3*H*-2,3-benzodiazepin-3-carboxamid
- (±)-1-[4'-(Cyclopropylsulfamoyl)biphenyl-4-yl]-7,8-dimethoxy-*N*,4-dimethyl-4,5-dihydro-3*H*-2,3-benzodiazepin-3-carboxamid
- (±)-1-(3'-Fluor-5'-hydroxybiphenyl-4-yl)-7,8-dimethoxy-*N*,4-dimethyl-4,5-dihydro-3*H*-2,3-benzodiazepin-3-carboxamid
- (±)-1-(3'-Fluor-5'-methylbiphenyl-4-yl)-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[3'-(methylsulfamoyl)biphenyl-4-yl]-4,5-dihydro-3*H-*2,3-benzodiazepin-3-carboxamid
- (±)-1-[4-(5-Fluorpyridin-3-yl)phenyl]-7,8-dimethoxy-*N*,4-dimethyl-4,5-dihydro-3*H*-2,3-benzodiazepin-3-carboxamid
- (±)-1-[4-(4-Fluorpyridin-3-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4-(2-methylpyridin-3-yl)phenyl]-4,5-dihydro-3*H*-2,3-benzodiazepin-3-carboxamid
- (±)-7,8-Dimethoxy-1-[4-(2-methoxypyridin-4-yl)phenyl]-*N*,4-dimethyl-4,5-dihydro-3*H-*2,3-benzodiazepin-3-carboxamid
- (±)-1-[4-(5-Cyanpyridin-3-yl)phenyl]-7,8-dimethoxy-*N*,4-dimethyl-4,5-dihydro-3*H*-2,3-benzodiazepin-3-carboxamid
- (4*R*)-7,8-Dimethoxy-*N*,4-dimethyl-1-[4-(morpholin-4-yl)phenyl]-4,5-dihydro-3*H*-2,3-benzodiazepin-3-carboxamid
- (4*S*)-7,8-Dimethoxy-*N*,4-dimethyl-1-[4-(morpholin-4-yl)phenyl]-4,5-dihydro-3*H*-2,3-benzodiazepin-3-carboxamid
- (4*S*)-7,8-Dimethoxy-*N*,4-dimethyl-1-[4-(4-methylpiperazin-1-yl)phenyl]-4,5-dihydro-3*H-*2,3-benzodiazepin-3-carboxamid
- (±)-1-[4-(Azetidin-1-yl)phenyl]-7,8-dimethoxy-*N*,4-dimethyl-4,5-dihydro-3*H*-2,3-benzodiazepin-3-carboxamid
- (4*R*)-1-[4-(Azetidin-1-yl)phenyl]-7,8-dimethoxy-*N*,4-dimethyl-4,5-dihydro-3*H*-2,3-benzodiazepin-3-carboxamid
- (4*S*)-1-[4-(Azetidin-1-yl)phenyl]-7,8-dimethoxy-*N*,4-dimethyl-4,5-dihydro-3*H*-2,3-benzodiazepin-3-carboxamid
- (±)-1-[4-(3-Fluorazetidin-1-yl)phenyl]-7,8-dimethoxy-*N*,4-dimethyl-4,5-dihydro-3*H*-2,3-benzodiazepin-3-carboxamid
- (4*R*)-1-[4-(3-Fluorazetidin-1-yl)phenyl]-7,8-dimethoxy-*N*,4-dimethyl-4,5-dihydro-3*H*-2,3-benzodiazepin-3-carboxamid
- (4*S*)-1-[4-(3-Fluorazetidin-1-yl)phenyl]-7,8-dimethoxy-*N*,4-dimethyl-4,5-dihydro-3*H*-2,3-benzodiazepin-3-carboxamid
- (±)-1-[4-(4-Hydroxypiperidin-1-yl)phenyl]-7,8-dimethoxy-*N*,4-dimethyl-4,5-dihydro-3*H-*2,3-benzodiazepin-3-carboxamid
- (4*S*)-1-[4-(4-Hydroxypiperidin-1-yl)phenyl]-7,8-dimethoxy-*N*,4-dimethyl-4,5-dihydro-3*H-*2,3-benzodiazepin-3-carboxamid
- (±)-7,8-Dimethoxy-*N*,4-dimethyl-1-[4-(piperazin-1-yl)phenyl]-4,5-dihydro-3*H*-2,3-benzodiazepin-3-carboxamid
- (4*S*)-7,8-Dimethoxy-*N*,4-dimethyl-1-[4-(piperazin-1-yl)phenyl]-4,5-dihydro-3*H*-2,3-benzodiazepin-3-carboxamid
- (±)-7,8-Dimethoxy-*N*,4-dimethyl-1-[4-(4-methylpiperazin-1-yl)phenyl]-4,5-dihydro-3*H-*2,3-benzodiazepin-3-carboxamid
- (±)-1-[4-(4-Acetylpiperazin-1-yl)phenyl]-7,8-dimethoxy-*N*,4-dimethyl-4,5-dihydro-3*H*-2,3-benzodiazepin-3-carboxamid
- (4*R*)-1-[4-(4-Acetylpiperazin-1-yl)phenyl]-7,8-dimethoxy-*N*,4-dimethyl-4,5-dihydro-3*H-*2,3-benzodiazepin-3-carboxamid
- (4*S*)-1-[4-(4-Acetylpiperazin-1-yl)phenyl]-7,8-dimethoxy-*N*,4-dimethyl-4,5-dihydro-3*H-*2,3-benzodiazepin-3-carboxamid
- (±)-7,8-Dimethoxy-*N*,4-dimethyl-1-{4-[4-(trifluoracetyl)piperazin-1-yl]phenyl}-4,5-dihydro-3*H*-2,3-benzodiazepin-3-carboxamid
- (±)-1-{4-[4-(2-Hydroxy-2-methylpropanoyl)piperazin-1-yl]phenyl}-7,8-dimethoxy-*N*,4-dimethyl-4,5-dihydro-3*H*-2,3-benzodiazepin-3-carboxamid
- (±)-7,8-Dimethoxy-*N*,4-dimethyl-1-{4-[4-(methylsulfonyl)piperazin-1-yl]phenyl}-4,5-dihydro-3*H*-2,3-benzodiazepin-3-carboxamid
- (4*S*)-1-[4-(1,-Dioxidothiomorpholin-4-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4-(3-oxopiperazin-1-yl)phenyl]-4,5-dihydro-3*H*-2,3-benzodiazepin-3-carboxamid
- (±)-7,8-Dimethoxy-*N*,4-dimethyl-1-[4-(4-methyl-3-oxopiperazin-1-yl)phenyl]-4,5-dihydro-3*H*-2,3-benzodiazepin-3-carboxamid
- (±)-7,8-Dimethoxy-*N*,4-dimethyl-1-[4-(piperidin-1-yl)phenyl]-4,5-dihydro-3*H*-2,3-benzodiazepin-3-carboxamid
- (±)-7,8-Dimethoxy-*N*,4-dimethyl-1-[3-(morpholin-4-yl)phenyl]-4,5-dihydro-3*H*-2,3-benzodiazepin-3-carboxamid
- (±)-1-[3-(3,3-Difluorazetidin-1-yl)phenyl]-7,8-dimethoxy-*N*,4-dimethyl-4,5-dihydro-3*H-*2,3-benzodiazepin-3-carboxamid
- (±)-1-[3-(Azetidin-1-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[3-(4-methylpiperazin-1-yl)phenyl]-4,5-dihydro-3*H-*2,3-benzodiazepin-3-carboxamid
- (±)-1-[4-Fluor-3-(morpholin-4-yl)phenyl]-7,8-dimethoxy-*N*,4-dimethyl-4,5-dihydro-3*H-*2,3-benzodiazepin-3-carboxamid
- (±)-1-[3-(3,3-Difluorazetidin-1-yl)-4-fluorphenyl]-7,8-dimethoxy-*N*,4-dimethyl-4,5-dihydro-3*H*-2,3-benzodiazepin-3-carboxamid
- (±)-1-[4-Fluor-3-(4-hydroxypiperidin-1-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-[4-(5-methyl-3-phenyl-1*H*-pyrazol-1-yl)phenyl]-4,5-dihydro-3*H*-2,3-benzodiazepin-3-carboxamid
- (45)-7,8-Dimethoxy-*N*,4-dimethyl-1-[4-(5-methyl-3-phenyl-1*H*-pyrazol-1-yl)phenyl]-4,5-dihydro-3*H*-2,3-benzodiazepin-3-carboxamid
- (±)-1-[4-(5-Cyclopropyl-3-phenyl-1*H*-pyrazol-1-yl)phenyl]-7,8-dimethoxy-*N*,4-dimethyl-4,5-dihydro-3*H*-2,3-benzodiazepin-3-carboxamid
- (4*S*)-1-[4-(5-Cyclopropyl-3-phenyl-1*H*-pyrazol-1-yl)phenyl]-7,8-dimethoxy-*N*,4-dimethyl-4,5-dihydro-3*H*-2,3-benzodiazepin-3-carboxamid
- (±)-7,8-Dimethoxy-*N*,4-dimethyl-1-{4-[3-phenyl-5-(trifluormethyl)-1*H*-pyrazol-1-yl]phenyl}-4,5-dihydro-3*H*-2,3-benzodiazepin-3-carboxamid
- (±)-1-{4-[3-(4-Fluorphenyl)-1*H*-pyrazol-1-yl]phenyl}-7,8-dimethoxy-*N*,4-dimethyl-4,5-dihydro-3*H*-2,3-benzodiazepin-3-carboxamid
- (4*S*)-1-{4-[3-(4-Fluorphenyl)-1*H*-pyrazol-1-yl]phenyl}-7,8-dimethoxy-*N*,4-dimethyl-4,5-dihydro-3*H*-2,3-benzodiazepin-3-carboxamid
- (4*R*)-1-{4-[3-(4-Fluorphenyl)-1*H*-pyrazol-1-yl]phenyl}-7,8-dimethoxy-*N*,4-dimethyl-4,5-dihydro-3*H*-2,3-benzodiazepin-3-carboxamid
- (±)-7,8-Dimethoxy-*N*,4-dimethyl-1-{4-[5-methyl-3-(trifluormethyl)-1*H*-pyrazol-1-yl]phenyl}-4,5-dihydro-3*H*-2,3-benzodiazepin-3-carboxamid
- (±)-7,8-Dimethoxy-*N*,4-dimethyl-1-[4-(1*H*-1,2,4-triazol-1-yl)phenyl]-4,5-dihydro-3*H*-2,3-benzodiazepin-3-carboxamid
- (±)-7,8-Dimethoxy-*N*,4-dimethyl-1-[4-(5-methyl-1*H*-1,2,4-triazol-1-yl)phenyl]-4,5-dihydro-3*H*-2,3-benzodiazepin-3-carboxamid
- (±)-1-[4-(3,5-Dimethyl-1*H*-1,2,4-triazol-1-yl)phenyl]-7,8-dimethoxy-*N*,4-dimethyl-4,5-dihydro-3*H*-2,3-benzodiazepin-3-carboxamid
- (±)-8-*tert*-Butyl-1-[4-(3,5-dimethylisoxazol-4-yl)phenyl]-*N*,4-dimethyl-4,5-dihydro-3*H-*2,3-benzodiazepin-3-carboxamid
- (±)-7-Chlor-1-[4-(3,5-dimethylisoxazol-4-yl)phenyl]-*N*,4-dimethyl-8-(trifluormethoxy)-4,5-dihydro-3*H*-2,3-benzodiazepin-3-carboxamid
- (4*S*)-7-Chlor-1-[4-(3,5-dimethylisoxazol-4-yl)phenyl]-*N*,4-dimethyl-8-(trifluormethoxy)-4,5-dihydro-3*H*-2,3-benzodiazepin-3-carboxamid
- (4*R*)-7-Chlor-1-[4-(3,5-dimethylisoxazol-4-yl)phenyl]-*N*,4-dimethyl-8-(trifluormethoxy)-4,5-dihydro-3*H*-2,3-benzodiazepin-3-carboxamid
- (±)-8-Chlor-1-[4-(3,5-dimethylisoxazol-4-yl)phenyl]-7-methoxy-*N*,4-dimethyl-4,5-dihydro-3*H*-2,3-benzodiazepin-3-carboxamid
- (4*S*)-8-Chlor-1-[4-(3,5-dimethylisoxazol-4-yl)phenyl]-7-methoxy-*N*,4-dimethyl-4,5-dihydro-3*H*-2,3-benzodiazepin-3-carboxamid
- (4*R*)-8-Chlor-1-[4-(3,5-dimethylisoxazol-4-yl)phenyl]-7-methoxy-*N*,4-dimethyl-4,5-dihydro-3*H*-2,3-benzodiazepin-3-carboxamid
- (±)-1-[4-(3,5-Dimethylisoxazol-4-yl)phenyl]-*N*,4,8-trimethyl-4,5-dihydro-3*H*-2,3-benzodiazepin-3-carboxamid
- (±)-7,8-Bis(difluormethoxy)-1-[4-(3,5-dimethylisoxazol-4-yl)phenyl]-*N*,4-dimethyl-4,5-dihydro-3*H*-2,3-benzodiazepin-3-carboxamid
- (±)-1-[4-(3,5-Dimethylisoxazol-4-yl)phenyl]-7,8-diethoxy-*N*,4-dimethyl-4,5-dihydro-3*H-*2,3-benzodiazepin-3-carboxamid
- (±)-7-(Difluormethoxy)-1-[4-(3,5-dimethylisoxazol-4-yl)phenyl]-8-methoxy-*N*,4-dimethyl-4,5-dihydro-3*H*-2,3-benzodiazepin-3-carboxamid
- (4*S*)-7-(Difluormethoxy)-1-[4-(3,5-dimethylisoxazol-4-yl)phenyl]-8-methoxy-*N*,4-dimethyl-4,5-dihydro-3*H*-2,3-benzodiazepin-3-carboxamid
- (4*R*)-7-(Difluormethoxy)-1-[4-(3,5-dimethylisoxazol-4-yl)phenyl]-8-methoxy-*N*,4-dimethyl-4,5-dihydro-3*H*-2,3-benzodiazepin-3-carboxamid
- (±)-7-(Difluormethoxy)-8-methoxy-*N*,4-dimethyl-1-[4-(4-methylpiperazin-1-yl)phenyl]-4,5-dihydro-3*H*-2,3-benzodiazepin-3-carboxamid
- (4*R*)-7-(Difluormethoxy)-8-methoxy-*N*,4-dimethyl-1-[4-(4-methylpiperazin-1-yl)phenyl]-4,5-dihydro-3*H*-2,3-benzodiazepin-3-carboxamid
- (4*S*)-7-(Difluormethoxy)-8-methoxy-*N*,4-dimethyl-1-[4-(4-methylpiperazin-1-yl)phenyl]-4,5-dihydro-3*H*-2,3-benzodiazepin-3-carboxamid
- (±)-1-(4-Chlorphenyl)-*N*,4-dimethyl-8-[3-(4-methylpiperazin-1-yl)propoxy]-4,5-dihydro-3*H*-2,3-benzodiazepin-3-carboxamid
- (4*S*)-1-(4-Chlorphenyl)-*N*,4-dimethyl-8-[3-(4-methylpiperazin-1-yl)propoxy]-4,5-dihydro-3*H*-2,3-benzodiazepin-3-carboxamid
- (±)-1-(4-Chlorphenyl)-*N*,4-dimethyl-8-[3-(morpholin-4-yl)propoxy]-4,5-dihydro-3*H*-2,3-benzodiazepin-3-carboxamid
- (±)-1-(4-Chlorphenyl)-*N*,4-dimethyl-8-[2-(4-methylpiperazin-1-yl)ethoxy]-4,5-dihydro-3*H-*2,3-benzodiazepin-3-carboxamid
- (±)-1-(4-Chlorphenyl)-*N*,4-dimethyl-8-[(6-methylpyridin-2-yl)methoxy]-4,5-dihydro-3*H-*2,3-benzodiazepin-3-carboxamid
- (±)-1-[4-(3,5-Dimethylisoxazol-4-yl)phenyl]-8-hydroxy-*N*,4-dimethyl-4,5-dihydro-3*H*-2,3-benzodiazepin-3-carboxamid
- (±)-1-[4-(3,5-Dimethylisoxazol-4-yl)phenyl]-*N*,4-dimethyl-8-[3-(morpholin-4-yl)propoxy]-4,5-dihydro-3*H*-2,3-benzodiazepin-3-carboxamid
- (±)-7-Cyan-1-[4-(3,5-dimethylisoxazol-4-yl)phenyl]-8-methoxy-*N*,4-dimethyl-4,5-dihydro-3*H*-2,3-benzodiazepin-3-carboxamid
- (±)-8-Acetamido-*N*,4-dimethyl-1-[4-(morpholin-4-yl)phenyl]-4,5-dihydro-3*H*-2,3-benzodiazepin-3-carboxamid
- (±)-8-Acetamido-*N*,4-dimethyl-1-[4-(4-methylpiperazin-1-yl)phenyl]-4,5-dihydro-3*H*-2,3-benzodiazepin-3-carboxamid
- (±)-8-Acetamido-1-[4-(3,5-dimethylisoxazol-4-yl)phenyl]-*N*,4-dimethyl-4,5-dihydro-3*H-*2,3-benzodiazepin-3-carboxamid
- (±)-1-(4-Chlorphenyl)-8-(3,5-dimethyl-1*H*-pyrazol-1-yl)-*N*,4-dimethyl-4,5-dihydro-3*H*-2,3-benzodiazepin-3-carboxamid
- (±)-1-[4-(3,5-Dimethylisoxazol-4-yl)phenyl]-8-(3,5-dimethyl-1*H*-pyrazol-1-yl)-*N*,4-dimethyl-4,5-dihydro-3*H*-2,3-benzodiazepin-3-carboxamid
- (±)-1-[4-(3,5-Dimethylisoxazol-4-yl)phenyl]-*N*,4-dimethyl-8-(morpholin-4-yl)-4,5-dihydro-3*H*-2,3-benzodiazepin-3-carboxamid
- (4*R*)-1-[4-(3,5-Dimethylisoxazol-4-yl)phenyl]-*N*,4-dimethyl-8-(morpholin-4-yl)-4,5-dihydro-3*H*-2,3-benzodiazepin-3-carboxamid
- (4*S*)-1-[4-(3,5-Dimethylisoxazol-4-yl)phenyl]-*N*,4-dimethyl-8-(morpholin-4-yl)-4,5-dihydro-3*H*-2,3-benzodiazepin-3-carboxamid
- (4*S*)-8-Methoxy-*N*,4-dimethyl-1-[4-(3-oxomorpholin-4-yl)phenyl]-4,5-dihydro-3*H*-2,3-benzodiazepin-3-carboxamid
- (±)-1-{4-[4-(2-Hydroxyethyl)piperazin-1-yl]phenyl}-7,8-dimethoxy-N,4-dimethyl-4,5-dihydro-3*H*-2,3-benzodiazepin-3-carboxamid
- (±)-7,8-Dimethoxy-1-[4-(4-methoxypiperidin-1-yl)phenyl]-N,4-dimethyl-4,5-dihydro-3*H-*2,3-benzodiazepin-3-carboxamid
- (4*R*)-7,8-Dimethoxy-1-[4-(4-methoxypiperidin-1-yl)phenyl]-N,4-dimethyl-4,5-dihydro-3*H-*2,3-benzodiazepin-3-carboxamid
- (4*S*)-7,8-Dimethoxy-1-[4-(4-methoxypiperidin-1-yl)phenyl]-N,4-dimethyl-4,5-dihydro-3*H-*2,3-benzodiazepin-3-carboxamid
- (±)-1-{4-[4-(Dimethylamino)piperidin-1-yl]phenyl}-7,8-dimethoxy-N,4-dimethyl-4,5-dihydro-3*H*-2,3-benzodiazepin-3-carboxamid
- (4*R*)-1-{4-[4-(Dimethylamino)piperidin-1-yl]phenyl}-7,8-dimethoxy-N,4-dimethyl-4,5-dihydro-3*H*-2,3-benzodiazepin-3-carboxamid
- (4*S*)-1-{4-[4-(Dimethylamino)piperidin-1-yl]phenyl}-7,8-dimethoxy-N,4-dimethyl-4,5-dihydro-3*H*-2,3-benzodiazepin-3-carboxamid
- (±)-1-[4-(3,3-Difluorazetidin-1-yl)phenyl]-7,8-dimethoxy-N,4-dimethyl-4,5-dihydro-3H-2,3-benzodiazepin-3-carboxamid
- (±)-1-[4-(4-Acetamidopiperidin-1-yl)phenyl]-7,8-dimethoxy-N,4-dimethyl-4,5-dihydro-3H-2,3-benzodiazepin-3-carboxamid
- (±)-1-4-[4-(2-Hydroxyethyl)piperidin-1-yl]phenyl}-7,8-dimethoxy-N,4-dimethyl-4,5-dihydro-3H-2,3-benzodiazepin-3-carboxamid
- (±)-1-[4-(3-Hydroxyazetidin-1-yl)phenyl]-7,8-dimethoxy-N,4-dimethyl-4,5-dihydro-3H-2,3-benzodiazepin-3-carboxamid
- (±)-1-[4-(3-Hydroxy-3-methylazetidin-1-yl)phenyl]-7,8-dimethoxy-N,4-dimethyl-4,5-dihydro-3H-2,3-benzodiazepin-3-carboxamid
- (4*R*)-1-[4-(3-Hydroxy-3-methylazetidin-1-yl)phenyl]-7,8-dimethoxy-N,4-dimethyl-4,5-dihydro-3H-2,3-benzodiazepin-3-carboxamid
- (4*S*)-1-[4-(3-Hydroxy-3-methylazetidin-1-yl)phenyl]-7,8-dimethoxy-N,4-dimethyl-4,5-dihydro-3H-2,3-benzodiazepin-3-carboxamid
- (±)-1-[4-(4-Isopropylpiperazin-1-yl)phenyl]-7,8-dimethoxy-N,4-dimethyl-4,5-dihydro-3H-2,3-benzodiazepin-3-carboxamid
- (±)-7,8-Dimethoxy-1-[4-(3-methoxyazetidin-1-yl)phenyl]-N,4-dimethyl-4,5-dihydro-3H-2,3-benzodiazepin-3-carboxamid
- (±)-1-[4-(4-Hydroxy-4-methylpiperidin-1-yl)phenyl]-7,8-dimethoxy-N,4-dimethyl-4,5-dihydro-3H-2,3-benzodiazepin-3-carboxamid
- (4*R*)-1-[4-(4-Hydroxy-4-methylpiperidin-1-yl)phenyl]-7,8-dimethoxy-N,4-dimethyl-4,5-dihydro-3H-2,3-benzodiazepin-3-carboxamid
- (4*S*)-1-[4-(4-Hydroxy-4-methylpiperidin-1-yl)phenyl]-7,8-dimethoxy-N,4-dimethyl-4,5-dihydro-3H-2,3-benzodiazepin-3-carboxamid
- (±)-7,8-Dimethoxy-N,4-dimethyl-1-{4-[4-(methylcarbamoyl)piperidin-1-yl]phenyl}-4,5-dihydro-3H-2,3-benzodiazepin-3-carboxamid
- (4*S*)-7,8-Dimethoxy-N,4-dimethyl-1-{4-[4-(methylcarbamoyl)piperidin-1-yl]phenyl}-4,5-dihydro-3H-2,3-benzodiazepin-3-carboxamid
- (*4R*)-7,8-Dimethoxy-N,4-dimethyl-1-{4-[4-(methylcarbamoyl)piperidin-1-yl]phenyl}-4,5-dihydro-3H-2,3-benzodiazepin-3-carboxamid
- (±)-1-{4-[(3S)-3-Hydroxypyrrolidin-1-yl]phenyl}-7,8-dimethoxy-N,4-dimethyl-4,5-dihydro-3H-2,3-benzodiazepin-3-carboxamid
- (4S)-1-{4-[(3S)-3-Hydroxypyrrolidin-1-yl]phenyl}-7,8-dimethoxy-N,4-dimethyl-4,5-dihydro-3H-2,3-benzodiazepin-3-carboxamid
- (±)-tert-Butyl-(1-{4-[7,8-dimethoxy-4-methyl-3-(methylcarbamoyl)-4,5-dihydro-3H-2,3-benzodiazepin-1-yl]phenyl}-4-methylpiperidin-4-yl)carbamat
- (±)-1-{4-[(2S,5R)-2,5-Dimethylpiperazin-1-yl]phenyl}-7,8-dimethoxy-N,4-dimethyl-4,5-dihydro-3H-2,3-benzodiazepin-3-carboxamid
- (±)-1-{4-[4-(2,2-Difluorethyl)piperazin-1-yl]phenyl}-7,8-dimethoxy-N,4-dimethyl-4,5-dihydro-3H-2,3-benzodiazepin-3-carboxamid
- (4S)-7,8-Dimethoxy-N,4-dimethyl-1-[4-(3-oxopiperazin-1-yl)phenyl]-4,5-dihydro-3H-2,3-benzodiazepin-3-carboxamid
- (4S)-1-{4-[(2R,6S)-2,6-Dimethylmorpholin-4-yl]phenyl}-7,8-dimethoxy-N,4-dimethyl-4,5-dihydro-3H-2,3-benzodiazepin-3-carboxamid
- (±)-7,8-Dimethoxy-N,4-dimethyl-1-[4-(4-oxopiperidin-1-yl)phenyl]-4,5-dihydro-3H-2,3-benzodiazepin-3-carboxamid
- (±)-7,8-Dimethoxy-N,4-dimethyl-1-{4-[4-(2,2,2-trifluorethyl)piperazin-1-yl]phenyl}-4,5-dihydro-3H-2,3-benzodiazepin-3-carboxamid
- (4S)-1-{4-[(3R,5S)-3,5-Dimethylpiperazin-1-yl]phenyl}-7,8-dimethoxy-N,4-dimethyl-4,5-dihydro-3H-2,3-benzodiazepin-3-carboxamid
- (±)-7,8-Dihydroxy-N,4-dimethyl-1-[4-(4-methylpiperazin-1-yl)phenyl]-4,5-dihydro-3H-2,3-benzodiazepin-3-carboxamid
- (±)-7,8-Diethoxy-N,4-dimethyl-1-[4-(4-methylpiperazin-1-yl)phenyl]-4,5-dihydro-3H-2,3-benzodiazepin-3-carboxamid
- (±)-4-Ethyl-7,8-dimethoxy-N-methyl-1-[4-(4-methylpiperazin-1-yl)phenyl]-4,5-dihydro-3H-2,3-benzodiazepin-3-carboxamid
- (4S)-4-Ethyl-7,8-dimethoxy-N-methyl-1-[4-(4-methylpiperazin-1-yl)phenyl]-4,5-dihydro-3H-2,3-benzodiazepin-3-carboxamid
- (±)-4-Ethyl-7,8-dimethoxy-N-methyl-1-[4-(piperazin-1-yl)phenyl]-4,5-dihydro-3H-2,3-benzodiazepin-3-carboxamid
- (4S)-4-Ethyl-7,8-dimethoxy-N-methyl-1-[4-(piperazin-1-yl)phenyl]-4,5-dihydro-3H-2,3-benzodiazepin-3-carboxamid
- (4R)-4-Ethyl-7,8-dimethoxy-N-methyl-1-[4-(piperazin-1-yl)phenyl]-4,5-dihydro-3H-2,3-benzodiazepin-3-carboxamid
- (±)-4-Ethyl-7,8-dimethoxy-N-methyl-1-{4-[4-(methylsulfonyl)piperazin-1-yl]phenyl}-4,5-dihydro-3H-2,3-benzodiazepin-3-carboxamid
- (4S)-4-Ethyl-7,8-dimethoxy-N-methyl-1-{4-[4-(methylsulfonyl)piperazin-1-yl]phenyl}-4,5-dihydro-3H-2,3-benzodiazepin-3-carboxamid
- (4R)-4-Ethyl-7,8-dimethoxy-N-methyl-1-{4-[4-(methylsulfonyl)piperazin-1-yl]phenyl}-4,5-dihydro-3H-2,3-benzodiazepin-3-carboxamid
- (±)-4-Ethyl-1-[4-(3-fluorazetidin-1-yl)phenyl]-7,8-dimethoxy-N-methyl-4,5-dihydro-3H-2,3-benzodiazepin-3-carboxamid
- (±)-1-[4-(4-Acetylpiperazin-1-yl)phenyl]-4-ethyl-7,8-dimethoxy-N-methyl-4,5-dihydro-3H-2,3-benzodiazepine-3-carboxamid
- (+)-1-[4-(1,1-Dioxidothiomorpholin-4-yl)phenyl]-4-ethyl-7,8-dimethoxy-N-methyl-4,5-dihydro-3H-2,3-benzodiazepin-3-carboxamid
- (±)-4-Ethyl-1-[4-(4-hydroxypiperidin-1-yl)phenyl]-7,8-dimethoxy-N-methyl-4,5-dihydro-3H-2,3-benzodiazepin-3-carboxamid
- (±)-4-Ethyl-7,8-dimethoxy-N-methyl-1-[4-(morpholin-4-yl)phenyl]-4,5-dihydro-3H-2,3-benzodiazepin-3-carboxamid
- (4S)-4-Ethyl-7,8-dimethoxy-N-methyl-1-[4-(morpholin-4-yl)phenyl]-4,5-dihydro-3H-2,3-benzodiazepin-3-carboxamid
- (±)-8-Chlor-1-[4-(1,1-dioxidothiomorpholin-4-yl)phenyl]-N,4-dimethyl-7-(trifluormethoxy)-4,5-dihydro-3H-2,3-benzodiazepin-3-carboxamid
- (±)-8-Chlor-N,4-dimethyl-1-[4-(piperazin-1-yl)phenyl]-7-(trifluormethoxy)-4,5-dihydro-3H-2,3-benzodiazepin-3-carboxamid
- (±)-8-Chlor-1-[4-(4-hydroxypiperidin-1-yl)phenyl]-N,4-dimethyl-7-(trifluormethoxy)-4,5-dihydro-3H-2,3-benzodiazepin-3-carboxamid
- (±)-8-Chlor-N,4-dimethyl-1-[4-(4-methylpiperazin-1-yl)phenyl]-7-(trifluormethoxy)-4,5-dihydro-3H-2,3-benzodiazepin-3-carboxamid
- (±)-8-Chlor-N,4-dimethyl-1-{4-[4-(methylsulfonyl)piperazin-1-yl]phenyl}-7-(trifluormethoxy)-4,5-dihydro-3H-2,3-benzodiazepin-3-carboxamid
- (±)-8-(1,1-Dioxidothiomorpholin-4-yl)-1-[4-(1,1-dioxidothiomorpholin-4-yl)phenyl]-N,4-dimethyl-7-(trifluormethoxy)-4,5-dihydro-3H-2,3-benzodiazepin-3-carboxamid
- (±)-1-(4-Chlorphenyl)-8-(1,1-dioxidothiomorpholin-4-yl)-N,4-dimethyl-7-(trifluormethoxy)-4,5-dihydro-3H-2,3-benzodiazepin-3-carboxamid
- (±)-1-[4-(4-Acetylpiperazin-1-yl)phenyl]-N,4-dimethyl-7-(trifluormethoxy)-4,5-dihydro-3H-2,3-benzodiazepin-3-carboxamid
- (±)-1-{4-[4-(2-Hydroxyethyl)piperazin-1-yl]phenyl}-N,4-dimethyl-7-(trifluormethoxy)-4,5-dihydro-3H-2,3-benzodiazepin-3-carboxamid
- (±)-8-Methoxy-N,4-dimethyl-1-[4-(4-methylpiperazin-1-yl)phenyl]-7-(trifluormethoxy)-4,5-dihydro-3H-2,3-benzodiazepin-3-carboxamid
- (±)-4-Ethyl-7,8-dimethoxy-N-methyl-1-4-(1,3,5-trimethyl-1H-pyrazol-4-yl)phenyl]-4,5-dihydro-3H-2,3-benzodiazepin-3-carboxamid
- (±)-4-Ethyl-1-(4'-fluorbiphenyl-4-yl)-7,8-dimethoxy-N-methyl-4,5-dihydro-3H-2,3-benzodiazepin-3-carboxamid
- (±)-4-Ethyl-7,8-dimethoxy-N-methyl-1-[4-(1-methyl-1H-1,2,3-triazol-4-yl)phenyl]-4,5-dihydro-3H-2,3-benzodiazepin-3-carboxamid
- (±)-1-[4-(3,5-Dimethyl-1,2-oxazol-4-yl)phenyl]-4-ethyl-7,8-dimethoxy-N-methyl-4,5-dihydro-3H-2,3-benzodiazepin-3-carboxamid
- (±)-4-Isopropyl-7,8-dimethoxy-N-methyl-1-[4-(1,3,5-trimethyl-1H-pyrazol-4-yl)phenyl]-4,5-dihydro-3H-2,3-benzodiazepin-3-carboxamid
- (±)-4-Ethyl-7,8-dimethoxy-N-methyl-1-[4-(2-oxopyrrolidin-1-yl)phenyl]-4,5-dihydro-3H-2,3-benzodiazepin-3-carboxamid
- (±)-4-Ethyl-7,8-dimethoxy-N-methyl-1-[4-(3-oxomorpholin-4-yl)phenyl]-4,5-dihydro-3H-2,3-benzodiazepin-3-carboxamid
- (±)-4-Ethyl-7,8-dimethoxy-N-methyl-1-[4-(2-oxopiperidin-1-yl)phenyl]-4,5-dihydro-3H-2,3-benzodiazepin-3-carboxamid
- (±)-4-Ethyl-7,8-dimethoxy-N-methyl-1-[4-(2-oxo-1,3-oxazolidin-3-yl)phenyl]-4,5-dihydro-3H-2,3-benzodiazepin-3-carboxamid
- (4S)-7,8-Dimethoxy-N,4-dimethyl-1-[4-(4-methyl-2-oxo-1,4-diazepan-1-yl)phenyl]-4,5-dihydro-3H-2,3-benzodiazepin-3-carboxamid
- (±)-1-(4-Chlorphenyl)-N,4-dimethyl-7-(2-oxo-1,3-oxazolidin-3-yl)-4,5-dihydro-3H-2,3-benzodiazepin-3-carboxamid
- (4S)-1-(4-Chlorphenyl)-N,4-dimethyl-7-(2-oxo-1,3-oxazolidin-3-yl)-4,5-dihydro-3H-2,3-benzodiazepin-3-carboxamid
- (±)-1-(4-Chlorphenyl)-N,4-dimethyl-7-(2-oxopiperidin-1-yl)-4,5-dihydro-3H-2,3-benzodiazepin-3-carboxamid
- (±)-1-(4-Chlorphenyl)-N,4-dimethyl-7-(3-oxomorpholin-4-yl)-4,5-dihydro-3H-2,3-benzodiazepin-3-carboxamid
- (±)-1-(4-Chlorphenyl)-N,4-dimethyl-7-(morpholin-4-yl)-4,5-dihydro-3H-2,3-benzodiazepin-3-carboxamid
- (±)-1-(4-Chlorphenyl)-N,4-dimethyl-7-(pyrrolidin-1-yl)-4,5-dihydro-3H-2,3-benzodiazepin-3-carboxamid
- (±)-1-(4-Chlorphenyl)-7-(1,1-dioxidothiomorpholin-4-yl)-N,4-dimethyl-4,5-dihydro-3H-2,3-benzodiazepin-3-carboxamid
- (±)-1-(4-Chlorphenyl)-N,4-dimethyl-7-(4-methylpiperazin-1-yl)-4,5-dihydro-3H-2,3-benzodiazepin-3-carboxamid
- (±)-N,4-Dimethyl-7-(4-methylpiperazin-1-yl)-1-[4-(4-methylpiperazin-1-yl)phenyl]-4,5-dihydro-3H-2,3-benzodiazepin-3-carboxamid
- (±)-N,4-Dimethyl-7-(4-methyl-3-oxopiperazin-1-yl)-1-[4-(4-methyl-3-oxopiperazin-1-yl)phenyl]-4,5-dihydro-3H-2,3-benzodiazepin-3-carboxamid
- (±)-1-(4-Chlorphenyl)-7-(4-fluorphenyl)-N,4-dimethyl-4,5-dihydro-3H-2,3-benzodiazepin-3-carboxamid
- (±)-1-(4-Chlorphenyl)-N,4-dimethyl-7-(pyridin-4-yl)-4,5-dihydro-3H-2,3-benzodiazepin-3-carboxamid
- (±)-1-(4-Chlorphenyl)-7-(6-hydroxypyridin-3-yl)-N,4-dimethyl-4,5-dihydro-3H-2,3-benzodiazepin-3-carboxamid
- (±)-1-(4-Chlorphenyl)-7-(3,5-dimethyl-1,2-oxazol-4-yl)-N,4-dimethyl-4,5-dihydro-3H-2,3-benzodiazepin-3-carboxamid
- (±)-1-(4-Chlorphenyl)-N,4-dimethyl-7-(1-methyl-1H-1,2,3-triazol-4-yl)-4,5-dihydro-3H-2,3-benzodiazepin-3-carboxamid
- (4R)-1-[4-(4-Hydroxypiperidin-1-yl)phenyl]-7,8-dimethoxy-N,4-dimethyl-4,5-dihydro-3H-2,3-benzodiazepin-3-carboxamid
- (4R)-7,8-Dimethoxy-N,4-dimethyl-1-[4-(4-methylpiperazin-1-yl)phenyl]-4,5-dihydro-3H-2,3-benzodiazepin-3-carboxamid
- (±)-1-[4-(1,1-Dioxido-1,2-thiazolidin-2-yl)phenyl]-7,8-dimethoxy-N,4-dimethyl-4,5-dihydro-3H-2,3-benzodiazepin-3-carboxamid
- (±)-1-{7,8-Dimethoxy-4-methyl-1-[4-(4-methylpiperazin-1-yl)phenyl]-4,5-dihydro-3H-2,3-benzodiazepin-3-yl}ethanon
- 1-{(4S)-7,8-Dimethoxy-4-methyl-1-[4-(4-methylpiperazin-1-yl)phenyl]-4,5-dihydro-3H-2,3-benzodiazepin-3-yl}ethanon
- (±)-1-{1-[4-(3,5-Dimethyl-1,2-oxazol-4-yl)phenyl]-7,8-dimethoxy-4-methyl-4,5-dihydro-3H-2,3-benzodiazepin-3-yl}ethanon
- (±)-7,8-Dimethoxy-N,4-dimethyl-1-[4-(5-methyl-1,3,4-oxadiazol-2-yl)phenyl]-4,5-dihydro-3H-2,3-benzodiazepin-3-carboxamid
- (±)-7,8-Dimethoxy-N,4-dimethyl-1-[4-(3-methyl-1,2,4-oxadiazol-5-yl)phenyl]-4,5-dihydro-3H-2,3-benzodiazepin-3-carboxamid
- (±)-N,4-Dimethyl-8-(trifluormethoxy)-1-[4-(1,3,5-trimethyl-1H-pyrazol-4-yl)phenyl]-4,5-dihydro-3H-2,3-benzodiazepin-3-carboxamid
- (4S)-N,4-Dimethyl-8-(trifluormethoxy)-1-[4-(1,3,5-trimethyl-1H-pyrazol-4-yl)phenyl]-4,5-dihydro-3H-2,3-benzodiazepin-3-carboxamid
- (4R)-N,4-Dimethyl-8-(trifluormethoxy)-1-[4-(1,3,5-trimethyl-1H-pyrazol-4-yl)phenyl]-4,5-dihydro-3H-2,3-benzodiazepin-3-carboxamid
- (±)-N,4-Dimethyl-1-[4-(4-methyl-1-piperazinyl)phenyl]-8-(trifluormethoxy)-4,5-dihydro-3H-2,3-benzodiazepin-3-carboxamid
- (4R)-N,4-Dimethyl-1-[4-(4-methyl-1-piperazinyl)phenyl]-8-(trifluormethoxy)-4,5-dihydro-3H-2,3-benzodiazepin-3-carboxamid
- (4S)-N,4-Dimethyl-1-[4-(4-methyl-1-piperazinyl)phenyl]-8-(trifluormethoxy)-4,5-dihydro-3H-2,3-benzodiazepin-3-carboxamid
- (±)-1-[4-(4-Hydroxy-1-piperidinyl)phenyl]-N,4-dimethyl-8-(trifluormethoxy)-4,5-dihydro-3H-2,3-benzodiazepin-3-carboxamid
- (±)-1-[2,4-Dibrom-5-(4-methylpiperazin-1-yl)phenyl]-7,8-dimethoxy-N,4-dimethyl-4,5-dihydro-3H-2,3-benzodiazepin-3-carboxamid
- (4S)-1-[3-Brom-4-(4-methylpiperazin-1-yl)phenyl]-7,8-dimethoxy-N,4-dimethyl-4,5-dihydro-3H-2,3-benzodiazepin-3-carboxamid
- (4S)-1-[3-Cyan-4-(4-methylpiperazin-1-yl)phenyl]-7,8-dimethoxy-N,4-dimethyl-4,5-dihydro-3H-2,3-benzodiazepin-3-carboxamid
- (±)-7,8-Dimethoxy-4-methyl-1-[4-(4-methylpiperazin-1-yl)phenyl]-3-(1-oxopropyl)-4,5-
- yl)phenyl]-4,5-dihydro-3H-2,3-benzodiazepin
- (±)-N-Cyclopropyl-7,8-dimethoxy-4-methyl-1-[4-(4-methylpiperazin-1-yl)phenyl]-4,5-dihydro-3H-2,3-benzodiazepin
- (±)-7,8-Dimethoxy-N,4-dimethyl-1-[4-(4-methylpiperazin-1-yl)phenyl]-4,5-dihydro-3H-2,3-benzodiazepin-3-carbothioamid
- Methyl-(±)-7,8-dimethoxy-4-methyl-1-[4-(4-methylpiperazin-1-yl)phenyl]-4,5-dihydro-3H-2,3-benzodiazepin-3-carboxylat
- Ethyl-(±)-7,8-dimethoxy-4-methyl-1-[4-(4-methylpiperazin-1-yl)phenyl]-4,5-dihydro-3H-2,3-benzodiazepin-3-carboxylat
- (±)-N-Ethyl-7,8-dimethoxy-4-methyl-1-[4-(4-methylpiperazin-1-yl)phenyl]-4,5-dihydro-3H-2,3-benzodiazepin-3-carboxamid
- (4S)-N-Ethyl-7,8-dimethoxy-4-methyl-1-[4-(4-methylpiperazin-1-yl)phenyl]-4,5-dihydro-3H-2,3-benzodiazepin-3-carboxamid.

16. Verbindungen gemäß einem der Ansprüche 1 bis 15 zur Verwendung in der Prophylaxe und/oder Therapie von hyper-proliferativen Erkrankungen, benignen Hyperplasien, inflammatorischen Erkrankungen, Autoimmunerkrankungen, Sepsis, viralen Infektionen, Gefäßerkrankungen, atherosklerotischen Erkrankungen und neurodegenerativen Erkrankungen.

17. Verbindung gemäß einem der Ansprüche 1 bis 15 zur Verwendung in der Prophylaxe und/oder Therapie von Tumorerkrankungen.

18. Verbindung gemäß einem der Ansprüche 1 bis 15, zur Verwendung in der männlichen Fertilitätskontrolle.

19. Verbindung gemäß einem der Ansprüche 1 bis 15 zur Verwendung in der Prophylaxe und/oder Therapie von Leukämien, Prostatakarzinomen, Mammakarzinomen, Melanomen oder Multiplen Myelomen.

20. Verwendung einer Verbindung gemäß Anspruch 1 bis 15 zur Herstellung eines Medikamentes.

21. Verbindung gemäß einem der Ansprüche 1 bis 15 zur Verwendung in der Prophylaxe und/oder Therapie von Erkrankungen des Menschen oder eines anderen Säugetiers.

22. Verbindung gemäß einem der Ansprüche 1 bis 15 in Kombination mit einem weiteren Wirkstoff.

23. Pharmazeutische Formulierung enthaltend eine Verbindung gemäß einem der Ansprüche 1 bis 15.

24. Zwischenverbindungen der allgemeinen Formel (Ia), 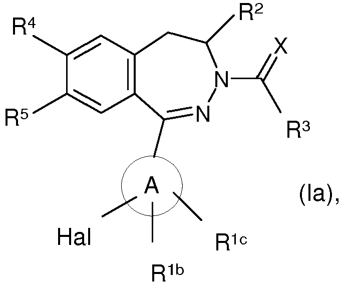 in der X, A, R^{1b}, R^{1c}, R², R³, R⁴ und R⁵ die in der allgemeinen Formel (I) angegebenen Bedeutungen haben und Hal für Brom steht, zur Herstellung der Verbindungen der allgemeinen Formel (I).

25. Zwischenverbindungen:
(4R)-1-(4-Bromphenyl)-7,8-dimethoxy-N,4-dimethyl-4,5-dihydro-3H-2,3-benzodiazepin-3-carboxamid
(4S)-1-(4-Bromphenyl)-7,8-dimethoxy-N,4-dimethyl-4,5-dihydro-3H-2,3-benzodiazepin-3-carboxamid
(±)-1-(4-Bromphenyl)-7,8-dimethoxy-N,4-dimethyl-4,5-dihydro-3H-2,3-benzodiazepin-3-carboxamid
(±)-1-(4-Chlorphenyl)-8-methoxy-N,4-dimethyl-4,5-dihydro-3H-2,3-benzodiazepin-3-carboxamid
(±)-1-(3-Bromphenyl)-7,8-dimethoxy-N,4-dimethyl-4,5-dihydro-3H-2,3-benzodiazepin-3-carboxamid
(±)-8-Brom-1-(4-chlorphenyl)-N,4-dimethyl-4,5-dihydro-3H-2,3-benzodiazepin-3-carboxamid
(4R)-8-Brom-1-(4-chlorphenyl)-N,4-dimethyl-4,5-dihydro-3H-2,3-benzodiazepin-3-carboxamid
(4S)-8-Brom-1-(4-chlorphenyl)-N,4-dimethyl-4,5-dihydro-3H-2,3-benzodiazepin-3-carboxamid
(4S)-1-(4-Bromphenyl)-8-methoxy-N,4-dimethyl-4,5-dihydro-3H-2,3-benzodiazepin-3-carboxamid
(±)-1-(4-Chlorphenyl)-8-hydroxy-N,4-dimethyl-4,5-dihydro-3H-2,3-benzodiazepin-3-carboxamid
(±)-1-(3-Brom-4-fluorphenyl)-7,8-dimethoxy-N,4-dimethyl-4,5-dihydro-3H-2,3-benzodiazepin-3-carboxamid
(±)-8-tert-Butyl-1-(4-chlorphenyl)-N,4-dimethyl-4,5-dihydro-3H-2,3-benzodiazepin-3-carboxamid
(4S)-8-tert-Butyl-1-(4-chlorphenyl)-N,4-dimethyl-4,5-dihydro-3H-2,3-benzodiazepin-3-carboxamid
(4R)-8-tert-Butyl-1-(4-chlorphenyl)-N,4-dimethyl-4,5-dihydro-3H-2,3-benzodiazepin-3-carboxamid
(±)-8-Chlor-1-(4-chlorphenyl)-7-methoxy-N,4-dimethyl-4,5-dihydro-3H-2,3-benzodiazepin-3-carboxamid
(4S)-8-Chlor-1-(4-chlorphenyl)-7-methoxy-N,4-dimethyl-4,5-dihydro-3H-2,3-benzodiazepin-3-carboxamid
(4R)-8-Chlor-1-(4-chlorphenyl)-7-methoxy-N,4-dimethyl-4,5-dihydro-3H-2,3-benzodiazepin-3-carboxamid
(±)-1-(4-Chlorphenyl)-N,4,8-trimethyl-4,5-dihydro-3H-2,3-benzodiazepin-3-carboxamid
(4S)-1-(4-Chlorphenyl)-N,4,8-trimethyl-4,5-dihydro-3H-2,3-benzodiazepin-3-carboxamid
(4R)-1-(4-Chlorphenyl)-N,4,8-trimethyl-4,5-dihydro-3H-2,3-benzodiazepin-3-carboxamid
(±)-8-Amino-1-(4-chlorphenyl)-N,4-dimethyl-4,5-dihydro-3H-2,3-benzodiazepin-3-carboxamid
(±)-1-[8-tert-Butyl-1-(4-chlorphenyl)-4-methyl-4,5-dihydro-3H-2,3-benzodiazepin-3-yl]ethanon
(±)-8-Acetamido-1-(4-chlorphenyl)-N,4-dimethyl-4,5-dihydro-3H-2,3-benzodiazepin-3-carboxamid
(±)-1-(4-Bromphenyl)-7-hydroxy-8-methoxy-N,4-dimethyl-4,5-dihydro-3H-2,3-benzodiazepin-3-carboxamid
(±)-1-(4-Bromphenyl)-7,8-dihydroxy-N,4-dimethyl-4,5-dihydro-3H-2,3-benzodiazepin-3-carboxamid
(±)-1-(4-Bromphenyl)-7,8-bis(difluormethoxy)-N,4-dimethyl-4,5-dihydro-3H-2,3-benzodiazepin-3-carboxamid
(±)-1-(4-Bromphenyl)-7,8-diethoxy-N,4-dimethyl-4,5-dihydro-3H-2,3-benzodiazepin-3-carboxamid
(±)-7-Brom-1-(4-chlorphenyl)-8-methoxy-N,4-dimethyl-4,5-dihydro-3H-2,3-benzodiazepin-3-carboxamid
(±)-1-(4-Chlorphenyl)-7-cyan-8-methoxy-N,4-dimethyl-4,5-dihydro-3H-2,3-benzodiazepin-3-carboxamid
(±)-1-(4-Bromphenyl)-7-(difluormethoxy)-8-methoxy-N,4-dimethyl-4,5-dihydro-3H-2,3-benzodiazepin-3-carboxamid
(±)-1-(4-Bromphenyl)-4-ethyl-7,8-dimethoxy-N-methyl-4,5-dihydro-3H-2,3-benzodiazepin-3-carboxamid
(±)-1-(4-Bromphenyl)-4-isopropyl-7,8-dimethoxy-N-methyl-4,5-dihydro-3H-2,3-benzodiazepin-3-carboxamid
(±)-7-Brom-1-(4-chlorphenyl)-N,4-dimethyl-4,5-dihydro-3H-2,3-benzodiazepin-3-carboxamid
(±)-8-Chlor-1-(4-chlorphenyl)-N,4-dimethyl-7-(trifluormethoxy)-4,5-dihydro-3H-2,3-benzodiazepin-3-carboxamid
(4S)-8-Chlor-1-(4-chlorphenyl)-N,4-dimethyl-7-(trifluormethoxy)-4,5-dihydro-3H-2,3-benzodiazepin-3-carboxamid
(±)-1-(4-Chlorphenyl)-8-methoxy-N,4-dimethyl-7-(trifluormethoxy)-4,5-dihydro-3H-2,3-benzodiazepin-3-carboxamid
(±)-1-[1-(4-Bromphenyl)-7,8-dimethoxy-4-methyl-4,5-dihydro-3H-2,3-benzodiazepin-3-yl]ethanon
(±)-1-(4-Bromphenyl)-N-ethyl-7,8-dimethoxy-4-methyl-4,5-dihydro-3H-2,3-benzodiazepin-3-carboxamid.

## Claims

1. Compounds of the general formula I 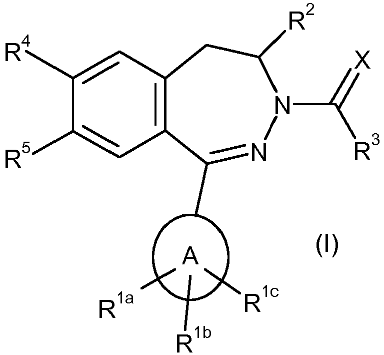 in which
X represents an oxygen or sulphur atom, and
A represents a monocyclic heteroaryl ring which has 5 or 6 ring atoms or represents a phenyl ring, and
R^{1a} represents hydrogen, halogen, cyano, carboxyl, amino or aminosulphonyl,
or
represents a C₁-C₆-alkoxy, C₁-C₃-alkoxy-C₁-C₃-alkyl, C₁-C₃-alkoxy-C₂-C₃-alkoxy, C₁-C₆-alkylamino, C₁-C₆-alkylcarbonylamino, C₁-C₆-alkylamino-C₁-C₆-alkyl, *N-*(heterocyclyl)-C₁-C₆-alkyl, *N-*(heterocyclyl)-C₁-C₆-alkoxy, hydroxy-C₁-C₆-alkyl, hydroxy-C₁-C₆-alkoxy, halo-C₁-C₆-alkyl, halo-C₁-C₆-alkoxy, C₁-C₆-alkylcarbonyl or C₁-C₆-alkoxycarbonyl radical,
or
represents a monocyclic heterocyclyl radical which has 3 to 8 ring atoms and may optionally be mono- or polysubstituted by identical or different substituents from the group consisting of halogen, cyano, nitro, hydroxy, amino, oxo, carboxyl, C₁-C₆-alkyl, C₁-C₆-alkoxy, C₁-C₆-alkoxy-C₁-C₆-alkyl, hydroxy-C₁-C₆-alkyl, C₁-C₆-alkylamino, amino-C₁-C₆-alkyl, C₁-C₆-alkylamino-C₁-C₆-alkyl, halo-C₁-C₆-alkyl, halo-C₁-C₆-alkoxy, C₃-C₁₀-cycloalkyl, phenyl, halophenyl, phenyl-C₁-C₆-alkyl, pyridinyl, -NR⁶C (=O)-R⁹, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, and/or a monocyclic heterocyclyl radical which has 3 to 8 ring atoms,
or
represents a monocyclic heteroaryl radical which has 5 or 6 ring atoms and may optionally be mono- or polysubstituted by identical or different substituents from the group consisting of halogen, hydroxy, amino, cyano, nitro, carboxyl, C₁-₆-alkyl, C₁-₆-alkoxy, C₁-C₆-alkoxy-C₁-C₆-alkyl, C₁-C₆-alkylamino, amino-C₁-C₆-alkyl, C₁-C₆-alkylamino-C₁-C₆-alkyl, halo-C₁-C₆-alkyl, halo-C₁-C₆-alkoxy, C₃-C₁₀-cycloalkyl, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, and/or by a monocyclic heterocyclyl radical which has 3 to 8 ring atoms, and/or by a monocyclic heteroaryl radical which has 5 or 6 ring atoms, and/or by a phenyl radical which for its part may optionally be mono- or polysubstituted by halogen, C₁-C₃-alkyl and/or C₁-C₃-alkoxy,
or
represents a phenyl radical which may optionally be mono- or polysubstituted by identical or different substituents from the group consisting of halogen, amino, hydroxy, cyano, nitro, carboxyl, C₁-C₆-alkyl, C₁-C₆-alkoxy, C₁-C₆-alkoxy-C₁-C₆-alkyl, C₁-C₆-alkylamino, amino-C₁-C₆-alkyl, C₁-C₆-alkylamino-C₁-C₆-alkyl, -C(=O)NR⁶R⁷, -C(=O)R⁸, C₁-C₃-alkylsulphinyl, C₁-C₃-alkylsulphonyl, -S(=O)₂NH₂, C₁-C₃-alkylsulphonylamino, C₁-C₃-alkylaminosulphonyl, C₃-C₆-cycloalkylaminosulphonyl, halo-C₁-C₆-alkyl, halo-C₁-C₆-alkoxy, hydroxy-C₁-C₆-alkyl, C₃-C₁₀-cycloalkyl, and/or a monocyclic heterocyclyl radical which has 3 to 8 ring atoms, and/or a monocyclic heteroaryl radical which has 5 or 6 ring atoms and which for its part may optionally be mono- or polysubstituted by identical or different substituents from the group consisting of halogen, C₁-C₃-alkyl and C₁-C₃-alkoxy, and
R^{1b} and R^{1c} independently of one another represent hydrogen, halogen, hydroxy, cyano, nitro and/or represent a C₁-C₆-alkyl, C₁-C₆-alkoxy, C₁-C₆-alkoxy-C₁-C₆-alkyl, halo-C₁-C₆-alkyl, halo-C₁-C₆-alkoxy, C₃-C₁₀-cycloalkyl radical and/or a monocyclic heterocyclyl radical which has 3 to 8 ring atoms, and
R² represents a C₁-C₃-alkyl or trifluoromethyl or a C₃- or C₄-cycloalkyl radical, and
R³ represents cyclopropyl, C₁-C₃-alkyl, C₁-C₃-alkoxy, amino, cyclopropylamino or C₁-C₃-alkylamino, and
R⁴ and R⁵ independently of one another represent hydrogen, hydroxy, cyano, nitro, amino, aminocarbonyl, fluorine, chlorine, bromine, C₁-C₆-alkyl, C₁-C₆-alkoxy, C₁-C₆-alkylamino, C₁-C₆-alkylcarbonylamino, C₁-C₆-alkylaminocarbonyl or C₁-C₆-alkylaminosulphonyl,
or
represent C₁-C₆-alkyl, C₁-C₆-alkoxy, C₁-C₆-alkylamino, C₁-C₆-alkylcarbonylamino, C₁-C₆-alkylaminocarbonyl or C₁-C₆-alkylaminosulphonyl which may optionally be mono- or polysubstituted by identical or different substituents from the group consisting of halogen, amino, hydroxy, carboxyl, hydroxy-C₁-C₆-alkyl, C₁-C₆-alkoxy, C₁-C₆-alkoxy-C₁-C₆-alkyl, C₁-C₆-alkylamino, amino-C₁-C₆-alkyl, monocyclic heterocyclyl which has 3 to 8 ring atoms and/or monocyclic heteroaryl which has 5 or 6 ring atoms, where the monocyclic heterocyclyl and heteroaryl radicals mentioned for their part may optionally be monosubstituted by C₁-C₃-alkyl,
or
represent C₃-C₁₀-cycloalkyl which may optionally be mono- or polysubstituted by identical or different substituents from the group consisting of halogen, amino, hydroxy, carboxyl, C₁-C₆-alkyl, C₁-C₆-alkoxy, C₁-C₆-alkoxy-C₁-C₆-alkyl, C₁-C₆-alkylamino, amino-C₁-C₆-alkyl, C₁-C₆-alkylamino-C₁-C₆-alkyl, halo-C₁-C₆-alkyl, halo-C₁-C₆-alkoxy, and/or a monocyclic heterocyclyl radical which has 3 to 8 ring atoms,
or
represent monocyclic heteroaryl which has 5 or 6 ring atoms and which may optionally be mono- or polysubstituted by identical or different substituents from the group consisting of halogen, amino, hydroxy, cyano, nitro, carboxyl, C₁-C₆-alkyl, C₁-C₆-alkoxy, C₁-C₆-alkoxy-C₁-C₆-alkyl, hydroxy-C₁-C₆-alkyl, C₁-C₆-alkylamino, amino-C₁-C₆-alkyl, C₁-C₆-alkylamino-C₁-C₆-alkyl, halo-C₁-C₆-alkyl, halo-C₁-C₆-alkoxy, C₃-C₁₀-cycloalkyl, -C(=O)R⁸, -S(=O)²R⁹, -NR⁶R⁷, and/or a monocyclic heterocyclyl radical which has 3 to 8 ring atoms,
or
represent monocyclic heterocyclyl which has 3 to 8 ring atoms and which may optionally be mono- or polysubstituted by identical or different substituents from the group consisting of halogen, amino, hydroxy, cyano, oxo, carboxyl, C₁-C₆-alkyl, C₁-C₆-alkoxy, C₁-C₆-alkoxy-C₁-C₆-alkyl, C₁-C₆-alkylamino, amino-C₁-C₆-alkyl, C₁-C₆-alkylamino-C₁-C₆-alkyl, hydroxy-C₁-C₆-alkyl, halo-C₁-C₆-alkyl, halo-C₁-C₆-alkoxy, C₃-C₁₀-cycloalkyl, -C(=O)R¹, -S(=O)₂R⁹, -NR⁶R⁷, and/or a monocyclic heterocyclyl radical which has 3 to 8 ring atoms,
or
represent phenyl which may optionally be mono- or polysubstituted by identical or different substituents from the group consisting of halogen, amino, hydroxy, cyano, nitro, carboxyl, C₁-C₆-alkyl, C₁-C₆-alkoxy, C₁-C₆-alkoxy-C₁-C₆-alkyl, C₁-C₆-alkylamino, amino-C₁-C₆-alkyl, C₁-C₆-alkylaminocarbonyl, C₁-C₆-alkylaminosulphonyl, C₁-C₆-alkylamino-C₁-C₆-alkyl, hydroxy-C₁-C₆-alkyl, halo-C₁-C₆-alkyl, halo-C₁-C₆-alkoxy, C₃-C₁₀-cycloalkyl and/or a monocyclic heterocyclyl radical which has 3 to 8 ring atoms, and
R⁶ and R⁷ independently of one another represent hydrogen, C₁-C₃-alkyl, cyclopropyl, di-C₁-C₃-alkyl-amino-C₁-C₃-alkyl or fluoropyridyl, and
R⁸ represents hydroxy, C₁-C₆-alkyl, halo-C₁-C₃-alkyl, hydroxy-C₁-C₃-alkyl, C₁-C₃-alkoxy-C₁-C₃-alkyl, C₃-C₈-cycloalkyl, phenyl, monocyclic heterocyclyl which has 3 to 8 ring atoms or monocyclic heteroaryl which has 5 or 6 ring atoms, where phenyl, heteroaryl and heterocyclyl may optionally be mono- or disubstituted by halogen, C₁-C₃-alkoxy or C₁-C₃-alkyl,
and
R⁹ represents hydrogen, C₁-C₆-alkyl or C₁-C₄-alkoxy,
and their polymorphs, enantiomers, diastereomers, racemates, tautomers, solvates, physiologically acceptable salts and solvates of these salts,
with the proviso that,
if A represents a phenyl ring and R⁴ and R⁵ independently of one another represent hydrogen, hydroxy, cyano, nitro, amino, aminocarbonyl, fluorine, chlorine, bromine, C₁-C₆-alkyl, C₁-C₆-alkoxy, C₁-C₆-alkylamino, C₁-C₆-alkylcarbonylamino, C₁-C₆-alkylamino-carbonyl or C₁-C₆-alkylaminosulphonyl, or represent C₁-C₆-alkyl, C₁-C₆-alkoxy, C₁-C₆-alkylamino, C₁-C₆-alkylcarbonylamino, C₁-C₆-alkylaminocarbonyl or C₁-C₆-alkylaminosulphonyl which may optionally be mono- or polysubstituted by identical or different substituents from the group consisting of halogen, amino, hydroxy, carboxyl, hydroxy-C₁-C₆-alkyl, C₁-C₆-alkoxy, C₁-C₆-alkoxy-C₁-C₆-alkyl, C₁-C₆-alkylamino, amino-C₁-C₆-alkyl, monocyclic heterocyclyl which has 3 to 8 ring atoms and/or monocyclic heteroaryl which has 5 or 6 ring atoms, where the monocyclic heterocyclyl and heteroaryl radicals mentioned for their part may optionally be monosubstituted by C₁-C₃-alkyl,
R^{1a} does not represent hydrogen, halogen, cyano, carboxyl, amino or aminosulphonyl, or represent a C₁-C₆-alkoxy, C₁-C₃-alkoxy-C₁-C₃-alkyl, C₁-C₃-alkoxy-C₂-C₃-alkoxy, C₁-C₆-alkylamino, C₁-C₆-alkylcarbonylamino, C₁-C₆-alkylamino-C₁-C₆-alkyl, N-(heterocyclyl)-C₁-C₆-alkyl, *N*-(heterocyclyl)-C₁-C₆-alkoxy, hydroxy-C₁-C₆-alkyl, hydroxy-C₁-C₆-alkoxy, halo-C₁-C₆-alkyl, halo-C₁-C₆-alkoxy, C₁-C₆-alkylcarbonyl or C₁-C₆-alkoxycarbonyl radical, and
where the proviso does not encompass those compounds of the general formula (I) in which A is phenyl and R⁴ is hydrogen, fluorine, chlorine or bromine and R⁵ is C₁-C₆-alkoxy which is substituted one or more times by identical or different halogens, and R^{1a} is halogen,
and also not those compounds of the general formula (I) in which A is phenyl and R⁴ is hydrogen and R⁵ is C₁-C₆-alkoxy which is substituted one or more times by identical or different monocyclic heterocyclyl radicals having 3 to 8 ring atoms and/or monocyclic heteroaryl radicals having 5 or 6 ring atoms, it being possible for the stated monocyclic heterocyclyl and heteroaryl radicals in turn to be optionally monosubstituted by C₁-C₃-alkyl, and R^{1a} is halogen.

2. Compounds of the general formula (I) according to Claim 1 in which
X represents an oxygen atom, and
A represents a phenyl or pyridyl ring, and
R^{1a} represents hydrogen, halogen, cyano, carboxyl, amino or aminosulphonyl,
or
represents a C₁-C₆-alkoxy, C₁-C₃-alkoxy-C₁-C₃-alkyl, C₁-C₃-alkoxy-C₂-C₃-alkoxy, C₁-C₆-alkylamino, C₁-C₆-alkylcarbonylamino, C₁-C₆-alkylamino-C₁-C₆-alkyl, *N-*(heterocyclyl)-C₁-C₆-alkyl, *N-*(heterocyclyl)-C₁-C₆-alkoxy, hydroxy-C₁-C₆-alkyl, hydroxy-C₁-C₆-alkoxy, halo-C₁-C₆-alkyl, halo-C₁-C₆-alkoxy, C₁-C₆-alkylcarbonyl or C₁-C₆-alkoxycarbonyl radical,
or
represents a monocyclic heterocyclyl radical which has 4 to 7 ring atoms and may optionally be mono- or polysubstituted by identical or different substituents from the group consisting of halogen, cyano, nitro, hydroxy, amino, oxo, carboxyl, C₁-C₆-alkyl, C₁-C₆-alkoxy, C₁-C₆-alkoxy-C₁-C₆-alkyl, hydroxy-C₁-C₆-alkyl, C₁-C₆-alkylamino, amino-C₁-C₆-alkyl, C₁-C₆-alkylamino-C₁-C₆-alkyl, halo-C₁-C₆-alkyl, halo-C₁-C₆-alkoxy, C₃-C₁₀-cycloalkyl, phenyl, halophenyl, phenyl-C₁-C₆-alkyl, pyridinyl, -NR⁶C(=O)R⁹, -C(=O)-NR⁶R⁷, -C(=O)-R⁸, -S(=O)₂-NR⁶R ⁷, -S(=O)-R⁹, -S(=O)₂-R⁹, -NH-S(=O)₂-R⁹, and/or by a monocyclic heterocyclyl radical which has 4 to 7 ring atoms,
or
represents a monocyclic heteroaryl radical which has 5 or 6 ring atoms and may optionally be mono- or polysubstituted by identical or different substituents from the group consisting of halogen, hydroxy, amino, cyano, nitro, carboxyl, C₁-C₆-alkyl, C₁-C₆-alkoxy, C₁-C₆-alkoxy-C₁-C₆-alkyl, C₁-C₆-alkylamino, amino-C₁-C₆-alkyl, C₁-C₆-alkylamino-C₁-C₆-alkyl, halo-C₁-C₆-alkyl, halo-C₁-C₆-alkoxy, C₃-C₁₀-cycloalkyl, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, and/or by a monocyclic heterocyclyl radical which has 4 to 7 ring atoms and/or by a monocyclic heteroaryl radical which has 5 or 6 ring atoms and/or by a phenyl radical which for its part may optionally be mono- or polysubstituted by halogen, C₁-C₃-alkyl and/or C₁-C₃-alkoxy,
or
represents a phenyl radical which may optionally be mono- or polysubstituted by identical or different substituents from the group consisting of halogen, amino, hydroxy, cyano, nitro, carboxyl, C₁-C₆-alkyl, C₁-C₆-alkoxy, C₁-C₆-alkoxy-C₁-C₆-alkyl, C₁-C₆-alkylamino, amino-C₁-C₆-alkyl, C₁-C₆-alkylamino-C₁-C₆-alkyl, -C(=O)NR⁶R⁷, C(=O)R⁸, C₁-C₃-alkylsulphinyl, C₁-C₃-alkylsulphonyl, -S(=O)₂NH₂, C₁-C₃-alkylsulphonylamino, C₁-C₃-alkylaminosulphonyl, C₃-C₆-cycloalkylaminosulphonyl, halo-C₁-C₆-alkyl, halo-C₁-C₆-alkoxy, hydroxy-C₁-C₆-alkyl, C₃-C₁₀-cycloalkyl, and/or a monocyclic heterocyclyl radical which has 4 to 7 ring atoms and/or a monocyclic heteroaryl radical which has 5 or 6 ring atoms and which for its part may optionally be mono- or polysubstituted by identical or different substituents from the group consisting of halogen, C₁-C₃-alkyl and C₁-C₃-alkoxy, and
R^{1b} and R^{1c} independently of one another represent hydrogen, halogen, hydroxy, cyano, nitro or represent a C₁-C₆-alkyl, C₁-C₆-alkoxy, C₁-C₆-alkoxy-C₁-C₆-alkyl, halo-C₁-C₆-alkyl, halo-C₁-C₆-alkoxy, C₃-C₁₀-cycloalkyl radical, and/or a monocyclic heterocyclyl radical which has 4 to 7 ring atoms, and
R² represents methyl, ethyl or isopropyl, and
R³ represents cyclopropyl, C₁-C₃-alkyl, C₁-C₃-alkoxy, amino, cyclopropylamino or C₁-C₃-alkylamino, and
R⁴ and R⁵ independently of one another represent hydrogen, hydroxy, cyano, nitro, amino, aminocarbonyl, fluorine, chlorine, bromine, C₁-C₆-alkyl, C₁-C₆-alkoxy, C₁-C₆-alkylamino, C₁-C₆-alkylcarbonylamino, C₁-C₆-alkylaminocarbonyl or C₁-C₆-alkylaminosulphonyl,
or
represent C₁-C₆-alkyl, C₁-C₆-alkoxy, C₁-C₆-alkylamino, C₁-C₆-alkylcarbonylamino, C₁-C₆-alkylaminocarbonyl or C₁-C₆-alkylaminosulphonyl which may optionally be mono- or polysubstituted by identical or different substituents from the group consisting of halogen, amino, hydroxy, carboxyl, hydroxy-C₁-C₆-alkyl, C₁-C₆-alkoxy, C₁-C₆-alkoxy-C₁-C₆-alkyl, C₁-C₆-alkylamino, amino-C₁-C₆-alkyl, monocyclic heterocyclyl which has 4 to 7 ring atoms, and/or monocyclic heteroaryl which has 5 or 6 ring atoms, where the monocyclic heterocyclyl and heteroaryl radicals mentioned for their part may optionally be monosubstituted by C₁-C₃-alkyl,
or
represent a C₃-C₁₀-cycloalkyl radical which may optionally be mono- or polysubstituted by identical or different substituents from the group consisting of halogen, amino, hydroxy, carboxyl, C₁-C₆-alkyl, C₁-C₆-alkoxy, C₁-C₆-alkoxy-C₁-C₆-alkyl, C₁-C₆-alkylamino, amino-C₁-C₆-alkyl, C₁-C₆-alkylamino-C₁-C₆-alkyl, halo-C₁-C₆-alkyl, halo-C₁-C₆-alkoxy, and/or a monocyclic heterocyclyl radical which has 4 to 7 ring atoms,
or
represent monocyclic heteroaryl which has 5 or 6 ring atoms and which may optionally be mono- or polysubstituted by identical or different substituents from the group consisting of halogen, amino, hydroxy, cyano, nitro, carboxyl, C₁-C₆-alkyl, C₁-C₆-alkoxy, C₁-C₆-alkoxy-C₁-C₆-alkyl, hydroxy-C₁-C₆-alkyl, C₁-C₆-alkylamino, amino-C₁-C₆-alkyl, C₁-C₆-alkylamino-C₁-C₆-alkyl, halo-C₁-C₆-alkyl, halo-C₁-C₆-alkoxy, C₃-C₁₀-cycloalkyl, -C(=O)R⁸, -S(=O)₂R⁹, -NR⁶R⁷, and/or a monocyclic heterocyclyl radical which has 4 to 7 ring atoms,
or
represent monocyclic heterocyclyl which has 4 to 7 ring atoms and which may optionally be mono- or polysubstituted by identical or different substituents from the group consisting of halogen, amino, hydroxy, cyano, oxo, carboxyl, C₁-C₆-alkyl, C₁-C₆-alkoxy, C₁-C₆-alkoxy-C₁-C₆-alkyl, C₁-C₆-alkylamino, amino-C₁-C₆-alkyl, C₁-C₆-alkylamino-C₁-C₆-alkyl, hydroxy-C₁-C₆-alkyl, halo-C₁-C₆-alkyl, halo-C₁-C₆-alkoxy, C₃-C₁₀-cycloalkyl, -C(=O)R⁸, -S(=O)₂R⁹, -NR⁶R⁷ and/or a monocyclic heterocyclyl radical which has 4 to 7 ring atoms,
or
represent a phenyl radical which may optionally be mono- or polysubstituted by identical or different substituents from the group consisting of halogen, amino, hydroxy, cyano, nitro, carboxyl, C₁-C₆-alkyl, C₁-C₆-alkoxy, C₁-C₆-alkoxy-C₁-C₆-alkyl, C₁-C₆-alkylamino, amino-C₁-C₆-alkyl, C₁-C₆-alkylaminocarbonyl, C₁-C₆-alkylaminosulphonyl, C₁-C₆-alkylamino-C₁-C₆-alkyl, hydroxy-C₁-C₆-alkyl, halo-C₁-C₆-alkyl, halo-C₁-C₆-alkoxy, C₃-C₁₀-cycloalkyl, and/or a monocyclic heterocyclyl radical which has 4 to 7 ring atoms, and
R⁶ and R⁷ independently of one another represent hydrogen, C₁-C₃-alkyl, cyclopropyl, di-C₁-C₃-alkyl-amino-C₁-C₃-alkyl or fluoropyridyl, and
R⁸ represents hydroxy, C₁-C₆-alkyl, halo-C₁-C₃-alkyl, hydroxy-C₁-C₃-alkyl, C₁-C₃-alkoxy-C₁-C₃-alkyl, C₃-C₈-cycloalkyl, phenyl, monocyclic heterocyclyl having 5 or 6 ring atoms, and
R⁹ represents hydrogen, C₁-C₆-alkyl or C₁-C₄-alkoxy,
and their polymorphs, enantiomers, diastereomers, racemates, tautomers, solvates, physiologically acceptable salts and solvates of these salts,
with the proviso that, if A represents a phenyl ring and R⁴ and R⁵ independently of one another represent hydrogen, hydroxy, cyano, nitro, amino, aminocarbonyl, fluorine, chlorine, bromine, C₁-C₆-alkyl, C₁-C₆-alkoxy, C₁-C₆-alkylamino, C₁-C₆-alkylcarbonylamino, C₁-C₆-alkylaminocarbonyl or C₁-C₆-alkylaminosulphonyl, or represent C₁-C₆-alkyl, C₁-C₆-alkoxy, C₁-C₆-alkylamino, C₁-C₆-alkylcarbonylamino, C₁-C₆-alkylaminocarbonyl or C₁-C₆-alkylaminosulphonyl which may optionally be mono- or polysubstituted by identical or different substituents from the group consisting of halogen, amino, hydroxy, carboxyl, hydroxy-C₁-C₆-alkyl, C₁-C₆-alkoxy, C₁-C₆-alkoxy-C₁-C₆-alkyl, C₁-C₆-alkylamino, amino-C₁-C₆-alkyl, monocyclic heterocyclyl which has 4 to 7 ring atoms and/or monocyclic heteroaryl which has 5 or 6 ring atoms, where the monocyclic heterocyclyl and heteroaryl radicals mentioned for their part may optionally be monosubstituted by C₁-C₃-alkyl,
R^{1a} does not represent hydrogen, halogen, cyano, carboxyl, amino or aminosulphonyl, or represent a C₁-C₆-alkoxy, C₁-C₃-alkoxy-C₁-C₃-alkyl, C₁-C₃-alkoxy-C₂-C₃-alkoxy, C₁-C₆-alkylamino, C₁-C₆-alkylcarbonylamino, C₁-C₆-alkylamino-C₁-C₆-alkyl, N-(heterocyclyl)-C₁-C₆-alkyl, *N*-(heterocyclyl)-C₁-C₆-alkoxy, hydroxy-C₁-C₆-alkyl, hydroxy-C₁-C₆-alkoxy, halo-C₁-C₆-alkyl, halo-C₁-C₆-alkoxy, C₁-C₆-alkylcarbonyl or C₁-C₆-alkoxycarbonyl radical, and
where the proviso does not embrace those compounds of the general formula (I) in which A is phenyl and R⁴ is hydrogen, fluorine, chlorine or bromine and R⁵ is C₁-C₆-alkoxy which is substituted one or more times by identical or different halogens, and
R^{1a} is halogen,
and also not those compounds of the general formula (I) in which A is phenyl and R⁴ is hydrogen and R⁵ is C₁-C₆-alkoxy which is substituted one or more times by identical or different monocyclic heterocyclyl radicals having 4 to 7 ring atoms and/or monocyclic heteroaryl radicals having 5 or 6 ring atoms, it being possible for the stated monocyclic heterocyclyl and heteroaryl radicals in turn to be optionally monosubstituted by C₁-C₃-alkyl, and R^{1a} is halogen.

3. Compounds of the general formula (I) according to Claim 1 or 2 in which
X represents an oxygen atom, and
A represents a phenyl or pyridyl ring, and
R^{1a} represents hydrogen, halogen, cyano, carboxyl, amino or aminosulphonyl, or represents a C₁-C₆-alkoxy, C₁-C₃-alkoxy-C₁-C₃-alkyl, C₁-C₃-alkoxy-C₂-C₃-alkoxy, C₁-C₃-alkylamino, C₁-C₃-alkylcarbonylamino, C₁-C₃-alkylamino-C₁-C₃-alkyl, hydroxy-C₁-C₃-alkyl, fluoro-C₁-C₃-alkyl, fluoro-C₁-C₃-alkoxy, C₁-C₃-alkylcarbonyl or C₁-C₄-alkoxycarbonyl radical,
or
represents a monocyclic heterocyclyl radical which has 4 to 7 ring atoms and may optionally be mono- or polysubstituted by identical or different substituents from the group consisting of halogen, cyano, nitro, hydroxy, amino, oxo, carboxyl, C₁-C₃-alkyl, C₁-C₃-alkoxy, C₁-C₂-alkoxy-C₁-C₂-alkyl, hydroxy-C₁-C₃-alkyl, C₁-C₃-alkylamino, amino-C₁-C₃-alkyl, fluoro-C₁-C₃-alkyl, fluoro-C₁-C₃-alkoxy, C₃-C₆-cycloalkyl, phenyl, halophenyl, phenyl-C₁-C₃-alkyl, pyridinyl, -NR⁶C(=O)-R⁹, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, and/or by a monocyclic heterocyclyl radical which has 4 to 7 ring atoms,
or
represents a monocyclic heteroaryl radical which has 5 or 6 ring atoms and may optionally be mono- or polysubstituted by identical or different substituents from the group consisting of halogen, hydroxy, amino, cyano, nitro, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, fluoro-C₁-C₃-alkyl, fluoro-C₁-C₃-alkoxy, C₃-C₆-cycloalkyl, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, and/or by a monocyclic heterocyclyl radical which has 4 to 7 ring atoms, and/or by a monocyclic heteroaryl radical which has 5 or 6 ring atoms, and/or by a phenyl radical which for its part may be mono- or polysubstituted by identical or different substituents from the group consisting of fluorine, chlorine, bromine, methyl and/or methoxy,
or
represents a phenyl radical which may optionally be mono- or polysubstituted by identical or different substituents from the group consisting of halogen, amino, hydroxy, cyano, nitro, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, -C(=O)NR⁶R⁷, C(=O)R⁸, C₁-C₃-alkylsulphinyl, C₁-C₃-alkylsulphonyl, -S(=O)₂NH₂, C₁-C₃-alkylsulphonylamino, C₁-C₃-alkylaminosulphonyl, C₃-C₆-cycloalkylaminosulphonyl, fluoro-C₁-C₃-alkyl, fluoro-C₁-C₃-alkoxy, hydroxy-C₁-C₃-alkyl, C₃-C₆-cycloalkyl and/or a monocyclic heterocyclyl radical which has 4 to 7 ring atoms, and/or a monocyclic heteroaryl radical which has 5 or 6 ring atoms and which for its part may optionally be mono- or polysubstituted by identical or different substituents from the group consisting of fluorine, chlorine, bromine, methyl and/or methoxy,
and
R^{1b} represents hydrogen, halogen, hydroxy, cyano, nitro or represents a C₁-C₃-alkyl, C₁-C₃-alkoxy, fluoro-C₁-C₃-alkyl or fluoro-C₁-C₃-alkoxy radical, and
R^{1c} represents hydrogen, fluorine, chlorine, bromine or cyano, and
R² represents methyl, ethyl or isopropyl, and
R³ represents cyclopropyl, C₁-C₃-alkyl, C₁-C₃-alkoxy, cyclopropylamino or C₁-C₃-alkylamino, and
R⁴ and R⁵ independently of one another represent hydrogen, hydroxy, cyano, nitro, amino, aminocarbonyl, fluorine, chlorine, bromine, C₁-C₆-alkyl, C₁-C₆-alkoxy, C₁-C₆-alkylamino, C₁-C₆-alkylcarbonylamino, C₁-C₆-alkylaminocarbonyl or C₁-C₆-alkylaminosulphonyl,
or
represent C₁-C₃-alkyl, C₁-C₃-alkoxy, C₁-C₃-alkylamino which may be mono- or polysubstituted by identical or different substituents from the group consisting of halogen, amino, hydroxy, carboxyl, hydroxy-C₁-C₃-alkyl, C₁-C₃-alkoxy, C₁-C₃-alkylamino, amino-C₁-C₃-alkyl, monocyclic heterocyclyl which has 4 to 7 ring atoms, and/or monocyclic heteroaryl which has 5 or 6 ring atoms, where the monocyclic heterocyclyl and heteroaryl radicals mentioned for their part may optionally be monosubstituted by C₁-C₃-alkyl,
or
represent a C₃-C₇-cycloalkyl radical which may optionally be mono- or polysubstituted by identical or different substituents from the group consisting of halogen, amino, hydroxy, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, fluoro-C₁-C₃-alkyl, fluoro-C₁-C₃-alkoxy, and/or a monocyclic heterocyclyl radical which has 4 to 7 ring atoms,
or
represent monocyclic heteroaryl which has 5 or 6 ring atoms and which may optionally be mono- or polysubstituted by identical or different substituents from the group consisting of halogen, amino, hydroxy, cyano, nitro, carboxyl, C₁-C₃-alkyl, C₁-C₃-alkoxy, C₁-C₂-alkoxy-C₁-C₂-alkyl, hydroxy-C₁-C₃-alkyl, C₁-C₃-alkylamino, amino-C₁-C₃-alkyl, fluoro-C₁-C₃-alkyl, fluoro-C₁-C₃-alkoxy, C₃-C₆-cycloalkyl, -C(=O)R⁸, -S(=O)₂R⁹, -NR⁶R⁷, and/or a monocyclic heterocyclyl radical which has 4 to 7 ring atoms,
or
represent monocyclic heterocyclyl which has 4 to 7 ring atoms and which may optionally be mono- or polysubstituted by identical or different substituents from the group consisting of halogen, amino, hydroxy, cyano, oxo, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, hydroxy-C₁-C₃-alkyl, fluoro-C₁-C₃-alkyl, fluoro-C₁-C₃-alkoxy, C₃-C₆-cycloalkyl, -C(=O)R⁸, -S(=O)₂R⁹, -NR⁶R⁷, and/or a monocyclic heterocyclyl radical which has 4 to 7 ring atoms,
or
represent a phenyl radical which may optionally be mono- or polysubstituted by identical or different substituents from the group consisting of halogen, amino, hydroxy, cyano, nitro, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, C₁-C₃-alkylaminocarbonyl, C₁-C₃-alkylaminosulphonyl, hydroxy-C₁-C₃-alkyl, fluoro-C₁-C₃-alkyl, fluoro-C₁-C₃-alkoxy, C₃-C₆-cycloalkyl, and/or a monocyclic heterocyclyl radical which has 4 to 7 ring atoms, and
R⁶ and R⁷ independently of one another represent hydrogen, C₁-C₃-alkyl, cyclopropyl, di-C₁-C₃-alkyl-amino-C₁-C₃-alkyl or fluoropyridyl, and
R⁸ represents hydroxy, C₁-C₆-alkyl, hydroxy-C₁-C₃-alkyl, fluoro-C₁-C₃-alkyl, C₁-C₃-alkoxy-C₁-C₃-alkyl, C₃-C₈-cycloalkyl or monocyclic heterocyclyl which has 5 or 6 ring atoms, and
R⁹ represents hydrogen, C₁-C₆-alkyl or C₁-C₄-alkoxy,
and their polymorphs, enantiomers, diastereomers, racemates, tautomers, solvates, physiologically acceptable salts and solvates of these salts,
with the proviso that,
if A represents a phenyl ring and R⁴ and R⁵ independently of one another represent hydrogen, hydroxy, cyano, nitro, amino, aminocarbonyl, fluorine, chlorine, bromine, C₁-C₆-alkyl, C₁-C₆-alkoxy, C₁-C₆-alkylamino, C₁-C₆-alkylcarbonylamino, C₁-C₆-alkylaminocarbonyl or C₁-C₆-alkylaminosulphonyl, or represent C₁-C₃-alkyl, C₁-C₃-alkoxy, C₁-C₃-alkylamino, which may optionally be mono- or polysubstituted by identical or different substituents from the group consisting of halogen, amino, hydroxy, carboxyl, hydroxy-C₁-C₃-alkyl, C₁-C₃-alkoxy, C₁-C₃-alkylamino, amino-C₁-C₃-alkyl, monocyclic heterocyclyl which has 4 to 7 ring atoms and/or monocyclic heteroaryl which has 5 or 6 ring atoms, where the monocyclic heterocyclyl and heteroaryl radicals mentioned for their part may optionally be monosubstituted by C₁-C₃-alkyl,
R^{1a} does not represent hydrogen, halogen, cyano, carboxyl, amino or aminosulphonyl, or represent a C₁-C₆-alkoxy, C₁-C₃-alkoxy-C₁-C₃-alkyl, C₁-C₃-alkoxy-C₂-C₃-alkoxy, C₁-C₃-alkylamino, C₁-C₃-alkylcarbonylamino, C₁-C₃-alkylamino-C₁-C₃-alkyl, hydroxy-C₁-C₃-alkyl, fluoro-C₁-C₃-alkyl, fluoro-C₁-C₃-alkoxy, C₁-C₃-alkylcarbonyl or C₁-C₄-alkoxycarbonyl radical, and
where the proviso does not embrace those compounds of the general formula (I) in which A is phenyl and R⁴ is hydrogen, fluorine, chlorine or bromine and R⁵ is C₁-C₃-alkoxy which is substituted one or more times by identical or different halogens, and
R^{1a} is halogen,
and also not those compounds of the general formula (I) in which A is phenyl and R⁴ is hydrogen and R⁵ is C₁-C₃-alkoxy which is substituted one or more times by identical or different monocyclic heterocyclyl radicals having 4 to 7 ring atoms and/or monocyclic heteroaryl radicals having 5 or 6 ring atoms, it being possible for the stated monocyclic heterocyclyl and heteroaryl radicals in turn to be optionally monosubstituted by C₁-C₃-alkyl, and R^{1a} is halogen.

4. Compounds of the general formula (I) according to any of Claims 1 to 3 in which
X represents an oxygen atom, and
A represents a phenyl or pyridyl ring, and
R^{1a} represents a monocyclic heterocyclyl radical which has 4 to 7 ring atoms and may optionally be mono- or polysubstituted by identical or different substituents from the group consisting of halogen, cyano, nitro, hydroxy, amino, oxo, carboxyl, C₁-C₃-alkyl, C₁-C₃-alkoxy, C₁-C₂-alkoxy-C₁-C₂-alkyl, hydroxy-C₁-C₃-alkyl, C₁-C₃-alkylamino, amino-C₁-C₃-alkyl, fluoro-C₁-C₃-alkyl, fluoro-C₁-C₃-alkoxy, C₃-C₆-cycloalkyl, phenyl, halophenyl, phenyl-C₁-C₃-alkyl, pyridinyl, -NR⁶C(=O)-R⁹, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, and/or by a monocyclic heterocyclyl radical which has 4 to 7 ring atoms,
or
represents a monocyclic heteroaryl radical which has 5 or 6 ring atoms and may optionally be mono- or polysubstituted by identical or different substituents from the group consisting of halogen, hydroxy, amino, cyano, nitro, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, fluoro-C₁-C₃-alkyl, fluoro-C₁-C₃-alkoxy, C₃-C₆-cycloalkyl, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, and/or by a monocyclic heterocyclyl radical which has 4 to 7 ring atoms, and/or by a monocyclic heteroaryl radical which has 5 or 6 ring atoms, and/or by a phenyl radical which for its part may be mono- or polysubstituted by identical or different substituents from the group consisting of fluorine, chlorine, bromine, methyl and/or methoxy,
or
represents a phenyl radical which may optionally be mono- or polysubstituted by identical or different substituents from the group consisting of halogen, amino, hydroxy, cyano, nitro, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, -C(=O)NR⁶R⁷, C(=O)R⁸, C₁-C₃-alkylsulphinyl, C₁-C₃-alkylsulphonyl, -S(=O)₂NH₂, C₁-C₃-alkylsulphonylamino, C₁-C₃-alkylaminosulphonyl, C₃-C₆-cycloalkylaminosulphonyl, fluoro-C₁-C₃-alkyl, fluoro-C₁-C₃-alkoxy, hydroxy-C₁-C₃-alkyl, C₃-C₆-cycloalkyl and/or a monocyclic heterocyclyl radical which has 4 to 7 ring atoms, and/or a monocyclic heteroaryl radical which has 5 or 6 ring atoms which for its part may optionally be mono- or polysubstituted by identical or different substituents from the group consisting of fluorine, chlorine, bromine, methyl or methoxy, and
R^{1b} represents hydrogen, halogen, hydroxy, cyano, nitro or represents a C₁-C₃-alkyl, C₁-C₃-alkoxy, fluoro-C₁-C₃-alkyl or fluoro-C₁-C₃-alkoxy radical, and
R^{1c} represents hydrogen, fluorine, chlorine, bromine or cyano, and
R² represents methyl, ethyl or isopropyl, and
R³ represents cyclopropyl, C₁-C₃-alkyl, C₁-C₃-alkoxy, cyclopropylamino or C₁-C₃-alkylamino, and
R⁴ and R⁵ independently of one another represent hydrogen, hydroxy, cyano, nitro, amino, aminocarbonyl, fluorine, chlorine, bromine, C₁-C₆-alkyl, C₁-C₆-alkoxy, C₁-C₆-alkylamino, C₁-C₆-alkylcarbonylamino, C₁-C₆-alkylaminocarbonyl or C₁-C₆-alkylaminosulphonyl,
or
represent C₁-C₃-alkyl, C₁-C₃-alkoxy, C₁-C₃-alkylamino which may be mono- or polysubstituted by identical or different substituents from the group consisting of halogen, amino, hydroxy, carboxyl, hydroxy-C₁-C₃-alkyl, C₁-C₃-alkoxy, C₁-C₃-alkylamino, amino-C₁-C₃-alkyl, monocyclic heterocyclyl which has 4 to 7 ring atoms, and/or monocyclic heteroaryl which has 5 or 6 ring atoms, where the monocyclic heterocyclyl and heteroaryl radicals mentioned for their part may optionally be monosubstituted by C₁-C₃-alkyl,
or
represent a C₃-C₇-cycloalkyl radical which may optionally be mono- or polysubstituted by identical or different substituents from the group consisting of halogen, amino, hydroxy, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, fluoro-C₁-C₃-alkyl, fluoro-C₁-C₃-alkoxy, and/or a monocyclic heterocyclyl radical which has 4 to 7 ring atoms,
or
represent monocyclic heteroaryl which has 5 or 6 ring atoms and may optionally be mono- or polysubstituted by identical or different substituents from the group consisting of halogen, amino, hydroxy, cyano, nitro, carboxyl, C₁-C₃-alkyl, C₁-C₃-alkoxy, C₁-C₂-alkoxy-C₁-C₂-alkyl, hydroxy-C₁-C₃-alkyl, C₁-C₃-alkylamino, amino-C₁-C₃-alkyl, fluoro-C₁-C₃-alkyl, fluoro-C₁-C₃-alkoxy, C₃-C₆-cycloalkyl, -C(=O)R⁸, -S(=O)₂R⁹, -NR⁶R⁷, and/or a monocyclic heterocyclyl radical which has 4 to 7 ring atoms,
or
represent monocyclic heterocyclyl which has 4 to 7 ring atoms and may optionally be mono- or polysubstituted by identical or different substituents from the group consisting of halogen, amino, hydroxy, cyano, oxo, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, hydroxy-C₁-C₃-alkyl, fluoro-C₁-C₃-alkyl, fluoro-C₁-C₃-alkoxy, C₃-C₆-cycloalkyl, -C(=O)R⁸, -S(=O)₂R⁹, -NR⁶R⁷, and/or a monocyclic heterocyclyl radical which has 4 to 7 ring atoms,
or
represent a phenyl radical which may optionally be mono- or polysubstituted by identical or different substituents from the group consisting of halogen, amino, hydroxy, cyano, nitro, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, C₁-C₃-alkylaminocarbonyl, C₁-C₃-alkylaminosulphonyl, hydroxy-C₁-C₃-alkyl, fluoro-C₁-C₃-alkyl, fluoro-C₁-C₃-alkoxy, C₃-C₆-cycloalkyl, and/or a monocyclic heterocyclyl radical which has 4 to 7 ring atoms, and
R⁶ and R⁷ independently of one another represent hydrogen, C₁-C₃-alkyl, cyclopropyl, di-C₁-C₃-alkyl-amino-C₁-C₃-alkyl or fluoropyridyl, and
R⁸ represents hydroxy, C₁-C₆-alkyl, hydroxy-C₁-C₃-alkyl, fluoro-C₁-C₃-alkyl, C₁-C₃-alkoxy-C₁-C₃-alkyl, C₃-C₈-cycloalkyl or monocyclic heterocyclyl which has 5 or 6 ring atoms, and
R⁹ represents hydrogen, C₁-C₆-alkyl or C₁-C₄-alkoxy,
and their polymorphs, enantiomers, diastereomers, racemates, tautomers, solvates, physiologically acceptable salts and solvates of these salts.

5. Compounds of the general formula (I) according to any of Claims 1 to 3 in which
X represents an oxygen atom, and
A represents a phenyl or pyridyl ring, and
R^{1a} represents hydrogen, halogen, cyano, carboxyl, amino or aminosulphonyl, or represents a C₁-C₆-alkoxy, C₁-C₃-alkoxy-C₁-C₃-alkyl, C₁-C₃-alkoxy-C₂-C₃-alkoxy, C₁-C₃-alkylamino, C₁-C₃-alkylcarbonylamino, C₁-C₃-alkylamino-C₁-C₃-alkyl, hydroxy-C₁-C₃-alkyl, fluoro-C₁-C₃-alkyl, fluoro-C₁-C₃-alkoxy, C₁-C₃-alkylcarbonyl or C₁-C₄-alkoxycarbonyl radical,
or
represents a monocyclic heterocyclyl radical which has 4 to 7 ring atoms and may optionally be mono- or polysubstituted by identical or different substituents from the group consisting of halogen, cyano, nitro, hydroxy, amino, oxo, carboxyl, C₁-C₃-alkyl, C₁-C₃-alkoxy, C₁-C₂-alkoxy-C₁-C₂-alkyl, hydroxy-C₁-C₃-alkyl, C₁-C₃-alkylamino, amino-C₁-C₃-alkyl, fluoro-C₁-C₃-alkyl, fluoro-C₁-C₃-alkoxy, C₃-C₆-cycloalkyl, phenyl, halophenyl, phenyl-C₁-C₃-alkyl, pyridinyl, -NR⁶C(=O)-R⁹, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, and/or by a monocyclic heterocyclyl radical which has 4 to 7 ring atoms,
or
represents a monocyclic heteroaryl radical which has 5 or 6 ring atoms and may optionally be mono- or polysubstituted by identical or different substituents from the group consisting of halogen, hydroxy, amino, cyano, nitro, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, fluoro-C₁-C₃-alkyl, fluoro-C₁-C₃-alkoxy, C₃-C₆-cycloalkyl, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, and/or by a monocyclic heterocyclyl radical which has 4 to 7 ring atoms, and/or by a monocyclic heteroaryl radical which has 5 or 6 ring atoms, and/or by a phenyl radical which for its part may be mono-or polysubstituted by identical or different substituents from the group consisting of fluorine, chlorine, bromine, methyl and/or methoxy,
or
represents a phenyl radical which may optionally be mono- or polysubstituted by identical or different substituents from the group consisting of halogen, amino, hydroxy, cyano, nitro, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, -C(=O)NR⁶R⁷, C(=O)R⁸, C₁-C₃-alkylsulphinyl, C₁-C₃-alkylsulphonyl, -S(=O)₂NH₂, C₁-C₃-alkylsulphonylamino, C₁-C₃-alkylaminosulphonyl, C₃-C₆-cycloalkylaminosulphonyl, fluoro-C₁-C₃-alkyl, fluoro-C₁-C₃-alkoxy, hydroxy-C₁-C₃-alkyl, C₃-C₆-cycloalkyl and/or a monocyclic heterocyclyl radical which has 4 to 7 ring atoms, and/or a monocyclic
heteroaryl radical which has 5 or 6 ring atoms which for its part may optionally be mono- or polysubstituted by identical or different substituents from the group consisting of fluorine, chlorine, bromine, methyl and/or methoxy, and
R^{1b} represents hydrogen, halogen, hydroxy, cyano, nitro or represents a C₁-C₃-alkyl, C₁-C₃-alkoxy, fluoro-C₁-C₃-alkyl, fluoro-C₁-C₃-alkoxy radical, and
R^{1c} represents hydrogen, fluorine, chlorine, bromine or cyano, and
R² represents methyl, ethyl or isopropyl, and
R³ represents cyclopropyl, C₁-C₃-alkyl, C₁-C₃-alkoxy, cyclopropylamino or C₁-C₃-alkylamino, and
R⁴ represents a C₃-C₇-cycloalkyl radical which may optionally be mono- or polysubstituted by identical or different substituents from the group consisting of halogen, amino, hydroxy, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, fluoro-C₁-C₃-alkyl, fluoro-C₁-C₃-alkoxy, and/or a monocyclic heterocyclyl radical which has 4 to 7 ring atoms,
or
represents monocyclic heteroaryl which has 5 or 6 ring atoms and may optionally be mono- or polysubstituted by identical or different substituents from the group consisting of halogen, amino, hydroxy, cyano, nitro, carboxyl, C₁-C₃-alkyl, C₁-C₃-alkoxy, C₁-C₂-alkoxy-C₁-C₂-alkyl, hydroxy-C₁-C₃-alkyl, C₁-C₃-alkylamino, amino-C₁-C₃-alkyl, fluoro-C₁-C₃-alkyl, fluoro-C₁-C₃-alkoxy, C₃-C₆-cycloalkyl, -C(=O)R⁸, -S(=O)₂R⁹, -NR⁶R⁷, and/or a monocyclic heterocyclyl radical which has 4 to 7 ring atoms,
or
represents monocyclic heterocyclyl which has 4 to 7 ring atoms and may optionally be mono- or polysubstituted by identical or different substituents from the group consisting of halogen, amino, hydroxy, cyano, oxo, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, hydroxy-C₁-C₃-alkyl, fluoro-C₁-C₃-alkyl, fluoro-C₁-C₃-alkoxy, C₃-C₆-cycloalkyl, -C(=O)R⁸, -S(=O)₂R⁹, -NR⁶R⁷, and/or a monocyclic heterocyclyl radical which has 4 to 7 ring atoms,
or
represents a phenyl radical which may optionally be mono- or polysubstituted by identical or different substituents from the group consisting of halogen, amino, hydroxy, cyano, nitro, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, C₁-C₃-alkylaminocarbonyl, C₁-C₃-alkylaminosulphonyl, hydroxy-C₁-C₃-alkyl, fluoro-C₁-C₃-alkyl, fluoro-C₁-C₃-alkoxy, C₃-C₆-cycloalkyl, and/or a monocyclic heterocyclyl radical which has 4 to 7 ring atoms,
R⁵ represents hydrogen, hydroxy, cyano, nitro, amino, aminocarbonyl, fluorine, chlorine, bromine, C₁-C₆-alkyl, C₁-C₆-alkoxy, C₁-C₆-alkylamino, C₁-C₆-alkylcarbonylamino, C₁-C₆-alkylaminocarbonyl or C₁-C₆-alkylaminosulphonyl, and
R⁶ and R⁷ independently of one another represent hydrogen, C₁-C₃-alkyl, cyclopropyl, di-C₁-C₃-alkyl-amino-C₁-C₃-alkyl or fluoropyridyl, and
R⁸ represents hydroxy, C₁-C₆-alkyl, hydroxy-C₁-C₃-alkyl, fluoro-C₁-C₃-alkyl, C₁-C₃-alkoxy-C₁-C₃-alkyl, C₃-C₈-cycloalkyl or monocyclic heterocyclyl which has 5 or 6 ring atoms, and
R⁹ represents hydrogen, C₁-C₆-alkyl or C₁-C₄-alkoxy,
and their polymorphs, enantiomers, diastereomers, racemates, tautomers, solvates, physiologically acceptable salts and solvates of these salts.

6. Compounds of the general formula (I) according to any of Claims 1 to 3 in which
X represents an oxygen atom, and
A represents a phenyl or pyridyl ring, and
R^{1a} represents hydrogen, halogen, cyano, carboxyl, amino or aminosulphonyl, or represents a C₁-C₆-alkoxy, C₁-C₃-alkoxy-C₁-C₃-alkyl, C₁-C₃-alkoxy-C₂-C₃-alkoxy, C₁-C₃-alkylamino, C₁-C₃-alkylcarbonylamino, C₁-C₃-alkylamino-C₁-C₃-alkyl, hydroxy-C₁-C₃-alkyl, fluoro-C₁-C₃-alkyl, fluoro-C₁-C₃-alkoxy, C₁-C₃-alkylcarbonyl or C₁-C₄-alkoxycarbonyl radical,
or
represents a monocyclic heterocyclyl radical which has 4 to 7 ring atoms and may optionally be mono- or polysubstituted by identical or different substituents from the group consisting of halogen, cyano, nitro, hydroxy, amino, oxo, carboxyl, C₁-C₃-alkyl, C₁-C₃-alkoxy, C₁-C₂-alkoxy-C₁-C₂-alkyl, hydroxy-C₁-C₃-alkyl, C₁-C₃-alkylamino, amino-C₁-C₃-alkyl, fluoro-C₁-C₃-alkyl, fluoro-C₁-C₃-alkoxy, C₃-C₆-cycloalkyl, phenyl, halophenyl, phenyl-C₁-C₃-alkyl, pyridinyl, -NR⁶C(=O)-R⁹, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, and/or by a monocyclic heterocyclyl radical which has 4 to 7 ring atoms,
or
represents a monocyclic heteroaryl radical which has 5 or 6 ring atoms and may optionally be mono- or polysubstituted by identical or different substituents from the group consisting of halogen, hydroxy, amino, cyano, nitro, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, fluoro-C₁-C₃-alkyl, fluoro-C₁-C₃-alkoxy, C₃-C₆-cycloalkyl, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, and/or by a monocyclic heterocyclyl radical which has 4 to 7 ring atoms, and/or by a monocyclic heteroaryl radical which has 5 or 6 ring atoms, and/or by a phenyl radical which for its part may be mono- or polysubstituted by identical or different substituents from the group consisting of fluorine, chlorine, bromine, methyl and/or methoxy,
or
represents a phenyl radical which may optionally be mono- or polysubstituted by identical or different substituents from the group consisting of halogen, amino, hydroxy, cyano, nitro, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, -C(=O)NR⁶R⁷, -C(=O)R⁸, C₁-C₃-alkylsulphinyl, C₁-C₃-alkylsulphonyl, - S(=O)₂NH₂, C₁-C₃-alkylsulphonylamino, C₁-C₃-alkylaminosulphonyl, C₃-C₆-cycloalkylaminosulphonyl, fluoro-C₁-C₃-alkyl, fluoro-C₁-C₃-alkoxy, hydroxy-C₁-C₃-alkyl, C₃-C₆-cycloalkyl and/or a monocyclic heterocyclyl radical which has 4 to 7 ring atoms, and/or a monocyclic heteroaryl radical which has 5 or 6 ring atoms which for its part may optionally be mono- or polysubstituted by identical or different substituents from the group consisting of fluorine, chlorine, bromine, methyl and/or methoxy, and
R^{1b} represents hydrogen, halogen, hydroxy, cyano, nitro or represents a C₁-C₃-alkyl, C₁-C₃-alkoxy, fluoro-C₁-C₃-alkyl or fluoro-C₁-C₃-alkoxy radical, and
R^{1c} represents hydrogen, fluorine, chlorine, bromine or cyano, and
R² represents methyl, ethyl or isopropyl, and
R³ represents cyclopropyl, C₁-C₃-alkyl, C₁-C₃-alkoxy, cyclopropylamino or C₁-C₃-alkylamino, and
R⁴ represents hydrogen, hydroxy, cyano, nitro, amino, aminocarbonyl, fluorine, chlorine, bromine, C₁-C₆-alkyl, C₁-C₆-alkoxy, C₁-C₆-alkylamino, C₁-C₆-alkylcarbonylamino, C₁-C₆-alkylaminocarbonyl or C₁-C₆-alkyl-aminosulphonyl, and
R⁵ represents a C₃-C₇-cycloalkyl radical which may optionally be mono- or polysubstituted by identical or different substituents from the group consisting of halogen, amino, hydroxy, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, fluoro-C₁-C₃-alkyl, fluoro-C₁-C₃-alkoxy, and/or a monocyclic heterocyclyl radical which has 4 to 7 ring atoms,
or
represents monocyclic heteroaryl which has 5 or 6 ring atoms and may optionally be mono- or polysubstituted by identical or different substituents from the group consisting of halogen, amino, hydroxy, cyano, nitro, carboxyl, C₁-C₃-alkyl, C₁-C₃-alkoxy, C₁-C₂-alkoxy-C₁-C₂-alkyl, hydroxy-C₁-C₃-alkyl, C₁-C₃-alkylamino, amino-C₁-C₃-alkyl, fluoro-C₁-C₃-alkyl, fluoro-C₁-C₃-alkoxy, C₃-C₆-cycloalkyl, -C(=O)R⁸, - S(=O)₂R⁹, -NR⁶R⁷, and/or a monocyclic heterocyclyl radical which has 4 to 7 ring atoms,
or
represents monocyclic heterocyclyl which has 4 to 7 ring atoms and may optionally be mono- or polysubstituted by identical or different substituents from the group consisting of halogen, amino, hydroxy, cyano, oxo, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, hydroxy-C₁-C₃-alkyl, fluoro-C₁-C₃-alkyl, fluoro-C₁-C₃-alkoxy, C₃-C₆-cycloalkyl, -C(=O)R⁸, - S(=O)₂R⁹, -NR⁶R⁷, and/or a monocyclic heterocyclyl radical which has 4 to 7 ring atoms,
or
represents a phenyl radical which may optionally be mono- or polysubstituted by identical or different substituents from the group consisting of halogen, amino, hydroxy, cyano, nitro, carboxyl, C₁-C₃-alkyl, C₁-C₃-alkoxy, C₁-C₂-alkoxy-C₁-C₂-alkyl, C₁-C₃-alkylamino, amino-C₁-C₃-alkyl, C₁-C₃-alkylaminocarbonyl, C₁-C₃-alkylaminosulphonyl, hydroxy-C₁-C₃-alkyl, fluoro-C₁-C₃-alkyl, fluoro-C₁-C₃-alkoxy, C₃-C₆-cycloalkyl, and/or a monocyclic heterocyclyl radical which has 4 to 7 ring atoms, and
R⁶ and R⁷ independently of one another represent hydrogen, C₁-C₃-alkyl, cyclopropyl, di-C₁-C₃-alkyl-amino-C₁-C₃-alkyl or fluoropyridyl, and
R⁸ represents hydroxy, C₁-C₆-alkyl, hydroxy-C₁-C₃-alkyl, fluoro-C₁-C₃-alkyl, C₁-C₃-alkoxy-C₁-C₃-alkyl, C₃-C₈-cycloalkyl or monocyclic heterocyclyl which has 5 or 6 ring atoms, and
R⁹ represents hydrogen, C₁-C₆-alkyl or C₁-C₄-alkoxy,
and their polymorphs, enantiomers, diastereomers, racemates, tautomers, solvates, physiologically acceptable salts and solvates of these salts.

7. Compounds of the general formula (I) according to any of Claims 1 to 3 in which
X represents an oxygen atom, and
A represents a phenyl or 3-pyridyl ring,
and R^{1a} represents hydrogen or chlorine,
or
represents piperazinyl, pyrrolidinyl, piperidinyl, diazepanyl, oxazinanyl, oxazolidinyl, morpholinyl, thiomorpholinyl, thiazolidinyl or azetidinyl, which may optionally be mono-or polysubstituted by identical or
different substituents from the group consisting of fluorine, chlorine, cyano, nitro, hydroxy, oxo, C₁-C₃-alkyl, C₁-C₃-alkoxy, C₁-C₂-alkoxy-C₁-C₂-alkyl, hydroxy-C₁-C₃-alkyl, dimethylamino, trifluoromethyl, difluoroethyl, trifluoroethyl, trifluoromethoxy, cyclopropyl, phenyl, fluorophenyl, phenyl-C₁-C₃-alkyl, pyridinyl, -NR⁶C(=O)-R⁹, -C(=O)-NR⁶R⁷, -C(=O)-R⁸, -S(=O)₂-NR⁶R⁷, -S(=O)-R⁹, -S(=O)₂-R⁹ and/or -NH-S (=O)₂-R⁹,
or
represents tetrazolyl,
or
represents isoxazolyl, pyrazolyl, thienyl, thiazolyl, imidazolyl, triazolyl, pyrrolyl, oxadiazolyl, pyridinyl or pyrimidinyl, which may optionally be mono- or polysubstituted by identical or different substituents from the group consisting of fluorine, chlorine, hydroxy, amino, cyano, nitro, C₁-C₃-alkyl, C₁-C₃-alkoxy, C₁-C₂-alkoxy-C₁-C₂-alkyl, dimethylamino, trifluoromethyl, difluoroethyl, trifluoroethyl, trifluoromethoxy, cyclopropyl, pyridinyl, phenyl, fluorophenyl, -C(=O)-NR⁶R⁷, - C(=O)-R⁸, -S(=O)₂-NR⁶R⁷, -S(=O)-R⁹, -S(=O)₂-R⁹, and/or -NH-S(=O)₂-R⁹,
or
represents a phenyl radical which may optionally be mono- or polysubstituted by identical or different substituents from the group consisting of fluorine, chlorine, amino, hydroxy, cyano, nitro, carboxyl, C₁-C₃-alkyl, C₁-C₃-alkoxy, C₁-C₂-alkoxy-C₁-C₂-alkyl, dimethylamino, -C(=O)NR⁶R⁷, -C(=O)R⁸, C₁-C₃-alkylsulphinyl, C₁-C₃-alkylsulphonyl, - S(=O)₂NH₂, C₁-C₃-alkylsulphonylamino, C₁-C₃-alkylaminosulphonyl, C₃-C₆-cycloalkylaminosulphonyl, trifluoromethyl, difluoroethyl, trifluoroethyl, trifluoromethoxy, hydroxy-C₁-C₃-alkyl, cyclopropyl, chlorothienyl, morpholino and/or pyridinyl, and
R^{1b} represents hydrogen, fluorine, bromine or cyano, and
R^{1c} represents hydrogen or bromine, and
R² represents methyl, ethyl or isopropyl, and
R³ represents cyclopropyl, methyl, ethyl, methoxy, ethocy, cyclopropylamino, methylamino or ethylamino, and
R⁴ and R⁵ independently of one another represent hydrogen, hydroxy, cyano, amino, chlorine, C₁-C₆-alkyl, methoxy, ethoxy or C₁-C₃-alkylcarbonylamino,
or
represent difluoromethoxy or trifluoromethoxy, or represent C₁-C₃-alkoxy which may be substituted by pyridinyl, morpholinyl, pyrrolidinyl or piperazinyl, in which pyridinyl and piperazinyl in turn may be optionally be substituted by C₁-C₃-alkyl,
or
represents cyclopropyl,
or
represents pyridinyl, pyrazolyl, triazolyl or isoxazolyl, which may be optionally mono- or polysubstituted by identical or different substituents from the group consting of hydroxy and/or methyl,
or
represents pyrrolidinyl, morpholinyl, piperidinyl, piperazinyl, oxazolidinyl, thiomorpholinyl, which may optionally be mono- or polysubstituted by oxo, methyl and/or -S(=O)²R⁹,
or
represents phenyl optionally substituted by C₁-C₃-alkylaminosulphonyl or fluorine, and
R⁶ and R⁷ independently of one another represent hydrogen, C₁-C₃-alkyl, cyclopropyl, di-C₁-C₃-alkyl-amino-C₁-C₃-alkyl or fluoropyridyl, and
R⁸ represents hydroxy, C₁-C₃-alkyl, hydroxy-C₁-C₃-alkyl, trifluoromethyl, pyrrolidinyl, morpholinyl or piperidinyl, and
R⁹ represents C₁-C₄-alkyl or C₁-C₄-alkoxy,
and their polymorphs, enantiomers, diastereomers, racemates, tautomers, solvates, physiologically acceptable salts and solvates of these salts, with the proviso that, if A represents a phenyl ring and R⁴ and R⁵ independently of one another represent hydrogen, hydroxy, cyano, amino, chlorine, C₁-C₆-alkyl, methoxy, ethoxy or C₁-C₃-alkylcarbonylamino, or represent difluoromethoxy or trifluoromethoxy,
or represent C₁-C₃-alkoxy which may be substituted by pyridinyl, morpholinyl, pyrrolidinyl or piperazinyl,
in which pyridinyl and piperazinyl in turn may be optionally substituted by C₁-C₃-alkyl,
R^{1a} does not represent hydrogen or chlorine, and
where the proviso does not encompass those compounds of the general formula (I) in which A represents phenyl and R⁴ represents hydrogen or chlorine and R⁵ represents trifluoromethoxy, and
R^{1a} represents chlorine,
and also not those compounds of the general formula (I) in which
A represents phenyl and R⁴ represents hydrogen and R⁵ represents C₁-C₃-alkoxy which is substituted by morpholinyl, pyrrolidinyl, piperazinyl or pyridyl, in which the piperazinyl and pyridinyl itself may be substituted by C₁-C₃-alkyl, and R^{1a} represents chlorine.

8. Compounds of the general formula (I) according to any of Claims 1 to 3 and 7 in which
X represents an oxygen atom, and
A represents a phenyl or 3-pyridyl ring,
and R^{1a} represents hydrogen or chlorine,
or
represents piperazinyl, pyrrolidinyl, piperidinyl, diazepanyl, oxazinanyl, oxazolidinyl, morpholinyl, thiomorpholinyl, thiazolidinyl or azetidinyl, which may optionally be mono-or polysubstituted by identical or different substituents from the group consisting of fluorine, hydroxy, oxo, C₁-C₃-alkyl, methoxy, hydroxy-C₁-C₃-alkyl, dimethylamino, difluoroethyl, trifluoroethyl, benzyl, -NR⁶C(=O)-R⁹,-C(=O)-NR⁶R⁷, -C(=O)-R⁸, and/or -S(=O)₂-R⁹, or
represents tetrazolyl,
or
represents isoxazolyl, pyrazolyl, thienyl, thiazolyl, imidazolyl, triazolyl, pyrrolyl, oxadiazolyl, pyridinyl or pyrimidinyl, which may optionally be mono- or polysubstituted by identical or different substituents from the group consisting of fluorine, chlorine, hydroxy, cyano, C₁-C₂-alkyl, methoxy, methoxymethyl, trifluoromethyl, cyclopropyl, pyridinyl, phenyl, fluorophenyl and/or -C(=O)-R⁸,
or
represents a phenyl radical which may optionally be mono- or polysubstituted by identical or different substituents from the group consisting of fluorine, chlorine, hydroxy, cyano, nitro, carboxyl, C₁-C₃-alkyl, methoxy, -C(=O)NR⁶R⁷, -C(=O)R⁸, C₁-C₃-alkylsulphinyl, C₁-C₃-alkylsulphonyl,-S(=O)₂NH₂, C₁-C₃-alkylsulphonylamino, C₁-C₃-alkylaminosulphonyl, C₃-C₆-cycloalkylaminosulphonyl,
trifluoromethyl, trifluoromethoxy, hydroxy-C₁-C₃-alkyl, cyclopropyl, chlorotheinyl and/or morpholino, and
R^{1b} represents hydrogen, fluorine, bromine or cyano, and
R^{1c} represents hydrogen or bromine, and
R² represents methyl, ethyl or isopropyl, and
R³ represents cyclopropyl, methyl, ethyl, methoxy, ethoxy, cyclopropylamino, methylamino or ethylamino, and
R⁴ and R⁵ independently of one another represent hydrogen, hydroxy, cyano, amino, chlorine, C₁-C₆-alkyl, methoxy, ethoxy or C₁-C₃-alkylcarbonylamino,
or
represent difluoromethoxy or trifluoromethoxy,
or represent C₁-C₃-alkoxy which may be substituted by pyridinyl, morpholinyl, pyrrolidinyl or piperazinyl,
in which the pyridinyl and piperazinyl may in turn optionally be substituted by C₁-C₃-alkyl,
or
represent cyclopropyl,
or
represent pyridinyl, pyrazolyl, triazolyl or isoxazolyl, which may optionally be mono- or polysubstituted by identical or different substituents from the group consisting of hydroxy and/or methyl,
or
represent pyrrolidinyl, morpholinyl, piperidinyl, piperazinyl, oxazolidinyl, thiomorpholinyl, which may optionally be mono- or polysubstituted by methyl, oxo, and/or -S(=O)₂R⁹,
or
represent phenyl optionally substituted by C₁-C₃-alkylaminosulphonyl or fluorine, and
R⁶ and R⁷ independently of one another represent hydrogen, C₁-C₃-alkyl, cyclopropyl, di-C₁-C₃-alkyl-amino-C₁-C₃-alkyl or fluoropyridyl, and
R⁸ represents hydroxy, C₁-C₃-alkyl, hydroxy-C₁-C₃-alkyl, trifluoromethyl, pyrrolidinyl, morpholinyl or piperidinyl, and
R⁹ represents C₁-C₄-alkyl or C₁-C₄-alkoxy, and
their polymorphs, enantiomers, diastereomers, racemates, tautomers, solvates, physiologically acceptable salts and solvates of these salts, with the proviso that,
if A represents a phenyl ring and R⁴ and R⁵ independently of one another represent hydrogen, hydroxy, cyano, amino, chlorine, C₁-C₆-alkyl, methoxy, ethoxy or C₁-C₃-alkylcarbonylamino, or represent difluoromethoxy or trifluoromethoxy, or represent C₁-C₃-alkoxy which may be substituted by pyridinyl, morpholinyl, pyrrolidinyl or
piperazinyl,
in which the pyridinyl and piperazinyl may in turn optionally be substituted by C₁-C₃-alkyl,
R^{1a} does not represent hydrogen or chlorine, and
where the proviso does not encompass those compounds of the general formula (I) in which A represents phenyl and R⁴ represents hydrogen or chlorine and R⁵ represents trifluoromethoxy, and
R^{1a} represents chlorine,
and also not those compounds of the general formula (I) in which
A represents phenyl and R⁴ represents hydrogen and
R⁵ represents C₁-C₃-alkoxy which is substituted by morpholinyl, pyrrolidinyl, piperazinyl or pyridyl, in which the piperazinyl and pyridinyl itself may be substituted by C₁-C₃-alkyl, and R^{1a} represents chlorine.

9. Compounds of the general formula (I) according to any of Claims 1 to 3 and 7 to 8 in which
X represents an oxygen atom, and
A represents a phenyl ring, and
R^{1a} represents piperazinyl, pyrrolidinyl, piperidinyl, diazepanyl, oxazinanyl, oxazolidinyl, morpholinyl, thiomorpholinyl, thiazolidinyl or azetidinyl, which may optionally be mono-or polysubstituted by identical or different substituents from the group consisting of fluorine, hydroxy, oxo, C₁-C₃-alkyl, methoxy, hydroxy-C₁-C₃-alkyl, dimethylamino, difluoroethyl, trifluoroethyl, benzyl, -NR⁶C(=O)-R⁹, - C(=O)-NR⁶R⁷, -C(=O)-R⁸, and/or -S(=O)₂-R⁹,
or
represents tetrazolyl,
or
represents isoxazolyl, pyrazolyl, thienyl, thiazolyl, imidazolyl, triazolyl, pyrrolyl, oxadiazolyl, pyridinyl or pyrimidinyl, which may optionally be mono- or polysubstituted by identical or different substituents from the group consisting of fluorine, chlorine, hydroxy, cyano, C₁-C₂-alkyl, methoxy, methoxymethyl, trifluoromethyl, cyclopropyl, pyridinyl, phenyl, fluorophenyl and/or -C(=O)-R⁸,
or
represents a phenyl radical, which may optionally be mono- or polysubstituted by identical or different substituents from the group consisting of fluorine, chlorine, hydroxy, cyano, nitro, carboxyl, C₁-C₃-alkyl, methoxy, - C (=O)NR⁶R⁷, C(=O)R⁸, C₁-C₃-alkylsulphinyl, C₁-C₃-alkylsulphonyl, -S(=O)₂NH₂, C₁-C₃-alkylsulphonylamino, C₁-C₃-alkylaminosulphonyl, C₃-C₆-cycloalkylaminosulphonyl, trifluoromethyl, trifluoromethoxy, hydroxy-C₁-C₃-alkyl, cyclopropyl, chlorothienyl and/or morpholino,
R^{1b} represents hydrogen, fluorine, bromine or cyano, and
R^{1c} represents hydrogen, and
R² represents methyl or ethyl, and
R³ represents methylamino, and
R⁴ and R⁵ independently of one another represent hydrogen, hydroxy, cyano, chlorine, C₁-C₆-alkyl, methoxy, ethoxy or C₁-C₃-alkylcarbonylamino,
or
difluoromethoxy or trifluoromethoxy, and
R⁶ and R⁷ independently of one another represent hydrogen, C₁-C₃-alkyl, cyclopropyl or di-C₁-C₃-alkyl-amino-C₁-C₃-alkyl, and
R⁸ represents hydroxy, C₁-C₃-alkyl, hydroxy-C₁-C₃-alkyl, trifluoromethyl, pyrrolidinyl, morpholinyl or piperidinyl, and
R⁹ represents C₁-C₄-alkyl or C₁-C₄-alkoxy,
and their polymorphs, tautomers, solvates, physiologically acceptable salts and solvates of these salts, and
where the stereocentre, which is represented by the carbon atom of the benzodiazepine scaffold which is bound to R², is present either in racemic form or predominantly or completely in the (*S*) configuration.

10. Compounds of the general formula (I) according to any of Claims 1 to 3 and 7 to 8 in which
X represents an oxygen atom, and
A represents a phenyl ring, and
R^{1a} represents hydrogen or chlorine, and
R^{1b} represents hydrogen, fluorine, bromine or cyano, and
R^{1c} represents hydrogen, and
R² represents methyl or ethyl, and
R³ represents methylamino, and
R⁴ represents cyclopropyl,
or
represents pyridinyl, pyrazolyl, triazolyl or isoxazolyl, which may optionally be mono- or polysubstituted by identical or different substituents from the group consisting of hydroxy and/or methyl,
or
represents pyrrolidinyl, morpholinyl, piperidinyl, piperazinyl, oxazolidinyl or thiomorpholinyl, which may optionally be mono- or polysubstituted by methyl, oxo and/or -S(=O)₂R⁹,
or
represents phenyl which is optionally substituted by C₁-C₃-alkylaminosulphonyl or fluorine, and
R⁵ represents hydrogen, hydroxy, cyano, chlorine, C₁-C₆-alkyl, methoxy, ethoxy or C₁-C₃-alkylcarbonylamino,
or
represents difluoromethoxy or trifluoromethoxy, and
R⁶ and R⁷ independently of one another represent hydrogen, C₁-C₃-alkyl, cyclopropyl or di-C₁-C₃-alkyl-amino-C₁-C₃-alkyl, and
R⁸ represents hydroxy, C₁-C₃-alkyl, hydroxy-C₁-C₃-alkyl, trifluoromethyl, pyrrolidinyl, morpholinyl or piperidinyl, and
R⁹ represents C₁-C₄-alkyl or C₁-C₄-alkoxy,
and their polymorphs, tautomers, solvates, physiologically acceptable salts and solvates of these salts, and
where the stereocentre, which is represented by the carbon atom of the benzodiazepine scaffold which is bound to R², is present either in racemic form or predominantly or completely in the (*S*) configuration.

11. Compounds of the general formula (I), according to Claims 1 to 3 and 7 to 8, in which
X represents an oxygen atom, and
A represents a phenyl ring, and
R^{1a} represents hydrogen or chlorine, and
R^{1b} represents hydrogen, fluorine, bromine or cyano, and
R^{1c} represents hydrogen, and
R² represents methyl or ethyl, and
R³ represents methylamino, and
R⁴ represents hydrogen, chlorine, methoxy or ethoxy,
or
represents difluoromethoxy or trifluoromethoxy, and
R⁵ represents cyclopropyl,
or
represents pyridinyl or pyrazolyl, which may optionally be substituted one or more times by methyl,
or
represents morpholinyl, piperidinyl, piperazinyl or thiomorpholinyl, which may optionally be mono- or polysubstituted by methyl, oxo and/or -S(=O)₂R⁹,
or
represents phenyl which is substituted by C₁-C₃-alkylaminosulphonyl, and
R⁶ and R⁷ independently of one another represent hydrogen, C₁-C₃-alkyl, cyclopropyl or di-C₁-C₃-alkylamino-C₁-C₃-alkyl, and
R⁸ represents hydroxy, C₁-C₃-alkyl, hydroxy-C₁-C₃-alkyl, trifluoromethyl, pyrrolidinyl, morpholinyl or piperidinyl, and
R⁹ represents C₁-C₄-alkyl or C₁-C₄-alkoxy,
and their polymorphs, tautomers, solvants, physiologically acceptable salts and solvates of these salts, and
where the stereocentre, which is represented by the carbon atom of the benzodiazepine scaffold which is bound to R², is present either in racemic form or predominantly or completely in the (*S*) configuration.

12. Compounds of the general formula (I), according to Claims 1 to 3 and 7 to 8, in which
X represents an oxygen atom, and
A represents a phenyl ring, and
R^{1a} represents piperazinyl, pyrrolidinyl, piperidinyl, diazepanyl, oxazinanyl, oxazolidinyl, morpholinyl, thiomorpholinyl, thiazolidinyl or azetidinyl, which may optionally be mono-or polysubstituted by identical or different substituents from the group consisting of fluorine, hydroxy, oxo, C₁-C₃-alkyl, methoxy, hydroxy-C₁-C₃-alkyl, dimethylamino, difluoroethyl, trifluoroethyl, benzyl, -NR⁶C(=O)-R⁹, - C(=O)-NR⁶R⁷, -C(=O)-R⁸, and/or -S(=O)₂-R⁹,
or
represents isoxazolyl or pyrazolyl, which may optionally be substituted one or more times by identical or different C₁-C₂-alkyls, and
R^{1b} represents hydrogen, fluorine, bromine or cyano, and
R^{1c} represents hydrogen, and
R² represents methyl, and
R³ represents methylamino, and
R⁴ and R⁵ independently of one another represent hydrogen, hydroxy, cyano, chlorine, C₁-C₆-alkyl, methoxy, ethoxy or C₁-C₃-alkylcarbonylamino, or represent difluoromethoxy or trifluoromethoxy, and
R⁶ and R⁷ independently of one another represent hydrogen, C₁-C₃-alkyl, cyclopropyl or di-C₁-C₃-alkylamino-C₁-C₃-alkyl, and
R⁸ represents hydroxyl, C₁-C₃-alkyl, hydroxy-C₁-C₃-alkyl, trifluoromethyl, pyrrolidinyl, morpholinyl or piperidinyl, and
R⁹ represents C₁-C₄-alkyl or C₁-C₄-alkoxy,
and their polymorphs, tautomers, solvates, physiologically acceptable salts and solvates of these salts, and
where the stereocentre, which is represented by the carbon atom of the benzodiazepine scaffold which is bound to R², is present either in racemic form or predominantly or completely in the (*S*) configuration.

13. Compounds of the general formula (I), according to Claims 1 to 3, 7 to 8 and 12 in which
X represents an oxygen atom, and
A represents a phenyl ring, and
R^{1a} represents piperazinyl, pyrrolidinyl, piperidinyl, morpholinyl, thiomorpholinyl or azetidinyl, which may optionally be mono- or polysubstituted by identical or different substituents from the group consisting of hydoxy, oxo, C₁-C₃-alkyl, methoxy, dimethylamino, difluoroethyl, trifluoroethyl, -NR⁶C(=O)-R⁹, -C(=O)-NR⁶R⁷, and/or -C(=O)-R⁸, and
R^{1b} represents hydrogen, fluorine, bromine or cyano, and
R^{1c} represents hydrogen, and
R² represents methyl, and
R³ represents methylamino, and
R⁴ and R⁵ independently of one another represent hydrogen, chlorine, methoxy or ethoxy, or represent difluoromethoxy or trifluoromethoxy, and
R⁶ and R⁷ independently of one another represent hydrogen or C₁-C₃-alkyl, and
R⁸ represents methyl, and
R⁹ represents methyl,
and their polymorphs, tautomers, solvates, physiologically acceptable salts and solvates of these salts, and
where the stereocentre, which is represented by the carbon atom of the benzodiazepine scaffold which is bound to R², is present either in racemic form or predominantly or completely in the (*S*) configuration.

14. Compounds of the general formula (I) according to any of Claims 1 to 3 in which
A represents a phenyl ring and R⁴ represents hydrogen or chlorine and R⁵ represents trifluoromethoxy and R^{1a} represents chlorine, and compounds of the general formula (I) in which A represents a phenyl ring and R⁴ represents hydrogen and R⁵ represents C₁-C₃-alkoxy which may be substituted by morpholinyl, pyrrolidinyl, piperazinyl or pyridyl, where the piperazinyl or pyridinyl for its part may be substituted by C₁-C₃-alkyl, and R^{1a} represents chlorine.

15. Compounds of the general formula (I) according to any of Claims 1 to 14,
- (±)-1-(4-chlorophenyl)-N,4-dimethyl-8-(trifluoromethoxy)-4,5-dihydro-3*H*-2,3-benzodiazepine-3-carboxamide
- (4*S*)-1-(4-chlorophenyl)-N,4-dimethyl-8-(trifluoromethoxy)-4,5-dihydro-3*H*-2,3-benzodiazepine-3-carboxamide
- (4*R*)-1-(4-chlorophenyl)-N,4-dimethyl-8-(trifluoromethoxy)-4,5-dihydro-3*H*-2,3-benzodiazepine-3-carboxamide
- (±)-1-[4-(3,5-dimethyl-4-isoxazolyl)phenyl]-7,8-dimethoxy-*N*,4-dimethyl-4,5-dihydro-3*H*-2,3-benzodiazepine-3-carboxamide
- (4*R*)-1-[4-(3,5-dimethyl-4-isoxazolyl)phenyl]-7,8-dimethoxy-*N*,4-dimethyl-4,5-dihydro-3*H*-2,3-benzodiazepine-3-carboxamide
- (4*S*)-1-[4-(3,5-dimethyl-4-isoxazolyl)phenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-[4-(1*H*-pyrazol-3-yl)phenyl]-4,5-dihydro-3*H*-2,3-benzodiazepine-3-carboxamide
- (±)-1-[4-(2-chloropyridin-3-yl)phenyl]-7,8-dimethoxy-*N*,4-dimethyl-4,5-dihydro-3*H*-2,3-benzodiazepine-3-carboxamide
- (±)-5-(4-{7,8-dimethoxy-4-methyl-3-[(methylamino)carbonyl]-4,5-dihydro-3*H*-2,3-benzodiazepin-1-yl}phenyl)thiophen-2-carboxylic acid
- (±)-4'-{7,8-dimethoxy-4-methyl-3-[(methylamino)carbonyl]-4,5-dihydro-3*H*-2,3-benzodiazepin-1-yl}biphenyl-2-carboxylic acid
- (±)-7,8-dimethoxy-*N*,4-dimethyl-1-[4-(morpholin-4-yl)phenyl]-4,5-dihydro-3*H*-2,3-benzodiazepine-3-carboxamide
- (±)-1-(4-chlorophenyl)-*N*,4-dimethyl-8-(pyridin-4-yl)-4,5-dihydro-3*H*-2,3-benzodiazepine-3-carboxamide
- (±)-1-(4-chlorophenyl)-8-cyclopropyl-*N*,4-dimethyl-4,5-dihydro-3*H*-2,3-benzodiazepine-3-carboxamide
- (±)-1-(4-chlorophenyl)-*N*,4-dimethyl-8-{4-[(methylamino)sulphonyl]phenyl}-4,5-dihydro-3*H-*2,3-benzodiazepine-3-carboxamide
- (±)-1-(4-chlorophenyl)-*N*,4-dimethyl-8-(morpholin-4-yl)-4,5-dihydro-3*H*-2,3-benzodiazepine-3-carboxamide
- (4*S*)-1-(4-chlorophenyl)-*N*,4-dimethyl-8-(morpholin-4-yl)-4,5-dihydro-3*H*-2,3-benzodiazepine-3-carboxamide
- (4*R*)-1-(4-chlorophenyl)-*N*,4-dimethyl-8-(morpholin-4-yl)-4,5-dihydro-3*H*-2,3-benzodiazepine-3-carboxamide
- (±)-1-(4-chlorophenyl)-*N*,4-dimethyl-8-(4-methylpiperazin-1-yl)-4,5-dihydro-3*H*-2,3-benzodiazepine-3-carboxamide
- (±)-1-(4-chlorophenyl)-*N*,4-dimethyl-8-(piperidin-1-yl)-4,5-dihydro-3*H*-2,3-benzodiazepine-3-carboxamide
- (±)-8-methoxy-*N*,4-dimethyl-1-(pyridin-3-yl)-4,5-dihydro-3*H*-2,3-benzodiazepine-3-carboxamide
- (±)-7-chloro-1-(4-chlorophenyl)-N,4-dimethyl-8-(trifluoromethoxy)-4,5-dihydro-3*H*-2,3-benzodiazepine-3-carboxamide
- 7-chloro-1-(4-chlorophenyl)-N,4-dimethyl-8-(trifluoromethoxy)-4,5-dihydro-3*H*-2,3-benzodiazepine-3-carboxamide, enantiomer 1
- (4*S*)-1-[4-(3,5-dimethyl-1*H*-pyrazol-1-yl)phenyl]-8-methoxy-*N*,4-dimethyl-4,5-dihydro-3*H*-2,3-benzodiazepine-3-carboxamide
- (4*S*)-8-methoxy-*N*,4-dimethyl-1-[4-(morpholin-4-yl)phenyl]-4,5-dihydro-3*H*-2,3-benzodiazepine-3-carboxamide
- (4*S*)-1-[4-(4-isoxazolyl)phenyl]-8-methoxy-*N*,4-dimethyl-4,5-dihydro-3*H*-2,3-benzodiazepine-3-carboxamide
- (4*S*)-8-methoxy-N,4-dimethyl-1-[4-(1-methyl-1H-pyrazol-5-yl)phenyl]-4,5-dihydro-3*H*-2,3-benzodiazepine-3-carboxamide
- (4*S*)-1-[4-(3,5-dimethyl-4-isoxazolyl)phenyl]-8-methoxy-*N*,4-dimethyl-4,5-dihydro-3*H*-2,3-benzodiazepine-3-carboxamide
- (4*S*)-8-methoxy-*N*,4-dimethyl-1-[4-(1-methyl-1*H-*pyrazol-4-yl)phenyl]-4,5-dihydro-3*H*-2,3-benzodiazepine-3-carboxamide
- (4*S*)-8-methoxy-*N*,4-dimethyl-1-[4-(1,3,5-trimethyl-1*H*-pyrazol-4-yl)phenyl]-4,5-dihydro-3*H*-2,3-benzodiazepine-3-carboxamide
- (4*S*)-8-methoxy-*N*,4-dimethyl-1-[4-(1*H*-pyrazol-5-yl)phenyl]-4,5-dihydro-3*H*-2,3-benzodiazepine-3-carboxamide
- (4*S*)-1-[4-(3-cyclopropyl-5-ethyl-1*H*-pyrazol-1-yl)phenyl]-8-methoxy-*N*,4-dimethyl-4,5-dihydro-3*H-*2,3-benzodiazepine-3-carboxamide
- (4*S*)-1-[4-(5-cyclopropyl-3-ethyl-1*H*-pyrazol-1-yl)phenyl]-8-methoxy-*N*,4-dimethyl-4,5-dihydro-3*H-*2,3-benzodiazepine-3-carboxamide
- (4*S*)-8-methoxy-1-{4-[3-(methoxymethyl)-5-methyl-1*H*-pyrazol-1-yl]phenyl}-*N*,4-dimethyl-4,5-dihydro-3*H*-2,3-benzodiazepine-3-carboxamide
- (4*S*)-8-methoxy-1-{4-[5-(methoxymethyl)-3-methyl-1*H*-pyrazol-1-yl]phenyl}-*N*,4-dimethyl-4,5-dihydro-3*H*-2,3-benzodiazepine-3-carboxamide
- (4*S*)-1-{4-[5-cyclopropyl-3-(pyridin-2-yl)-1*H-*pyrazol-1-yl]phenyl}-8-methoxy-*N*,4-dimethyl-4,5-dihydro-3*H*-2,3-benzodiazepine-3-carboxamide
- (4*S*)-1-{4-[3-cyclopropyl-5-(pyridin-2-yl)-1*H-*pyrazol-1-yl]phenyl}-8-methoxy-*N*,4-dimethyl-4,5-dihydro-3*H*-2,3-benzodiazepine-3-carboxamide
- (4*S*)-8-methoxy-*N*,4-dimethyl-1-[4-(1*H*-tetrazol-1-yl)phenyl]-4,5-dihydro-3*H*-2,3-benzodiazepine-3-carboxamide
- (±)-1-[3-(3,5-dimethylisoxazol-4-yl)phenyl]-7,8-dimethoxy-*N*,4-dimethyl-4,5-dihydro-3*H*-2,3-benzodiazepine-3-carboxamide
- (±)-1-(4-chlorophenyl)-*N*,4-dimethyl-8-[2-(morpholin-4-yl)ethoxy]-4,5-dihydro-3*H*-2,3-benzodiazepine-3-carboxamide
- (±)-1-(4-chlorophenyl)-*N*,4-dimethyl-8-[2-(pyrrolidin-1-yl)ethoxy]-4,5-dihydro-3*H*-2,3-benzodiazepine-3-carboxamide
- (±)-7,8-dimethoxy-*N*,4-dimethyl-1-[4-(2-oxooxazolidin-3-yl)phenyl]-4,5-dihydro-3*H*-2,3-benzodiazepine-3-carboxamide
- (4*S*)-7,8-dimethoxy-*N*,4-dimethyl-1-[4-(2-oxooxazolidin-3-yl)phenyl]-4,5-dihydro-3*H*-2,3-benzodiazepine-3-carboxamide
- (±)-7,8-dimethoxy-*N*,4-dimethyl-1-[4-(2-oxopiperidin-1-yl)phenyl]-4,5-dihydro-3*H*-2,3-benzodiazepine-3-carboxamide
- (±)-1-[4-(4-benzyl-2-oxopiperazin-1-yl)phenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-[4-(4-methyl-2-oxo-1,4-diazepan-1-yl)phenyl]-4,5-dihydro-3*H*-2,3-benzodiazepine-3-carboxamide
- (±)-7,8-dimethoxy-*N*,4-dimethyl-1-[4-(2-oxo-1,3-oxazinan-3-yl)phenyl]-4,5-dihydro-3*H*-2,3-benzodiazepine-3-carboxamide
- (±)-7,8-dimethoxy-*N*,4-dimethyl-1-[4-(2-oxopyrrolidin-1-yl)phenyl]-4,5-dihydro-3*H*-2,3-benzodiazepine-3-carboxamide
- (±)-7,8-dimethoxy-*N*,4-dimethyl-1-[4-(3-oxomorpholin-4-yl)phenyl]-4,5-dihydro-3*H*-2,3-benzodiazepine-3-carboxamide
- (4*S*)-7,8-dimethoxy-*N*,4-dimethyl-1-[4-(3-oxomorpholin-4-yl)phenyl]-4,5-dihydro-3*H*-2,3-benzodiazepine-3-carboxamide
- (±)-7,8-dimethoxy-*N*,4-dimethyl-1-[4-(2-methyl-5-oxomorpholin-4-yl)phenyl]-4,5-dihydro-3*H*-2,3-benzodiazepine-3-carboxamide (mixture of stereoisomers)
- (±)-7,8-dimethoxy-*N*,4-dimethyl-1-[4-(2-methyl-3-oxomorpholin-4-yl)phenyl]-4,5-dihydro-3*H*-2,3-benzodiazepine-3-carboxamide (mixture of stereoisomers)
- (±)-7,8-dimethoxy-*N*,4-dimethyl-1-[4-(4-methyl-2-oxopiperazin-1-yl)phenyl]-4,5-dihydro-3*H*-2,3-benzodiazepine-3-carboxamide
- (4*S*)-7,8-dimethoxy-*N*,4-dimethyl-1-[4-(4-methyl-2-oxopiperazin-1-yl)phenyl]-4,5-dihydro-3*H*-2,3-benzodiazepine-3-carboxamide
- (4*R*)-7,8-dimethoxy-*N*,4-dimethyl-1-[4-(4-methyl-2-oxopiperazin-1-yl)phenyl]-4,5-dihydro-3*H*-2,3-benzodiazepine-3-carboxamide
- (±)-7,8-dimethoxy-*N*,4-dimethyl-1-[4-(2-oxopiperazin-1-yl)phenyl]-4,5-dihydro-3*H*-2,3-benzodiazepine-3-carboxamide
- (±)-7,8-dimethoxy-*N*,4-dimethyl-1-[4-(1-methyl-1*H-*1,2,3-triazol-4-yl)phenyl]-4,5-dihydro-3*H*-2,3-benzodiazepine-3-carboxamide
- (4*S*)-7,8-dimethoxy-*N*,4-dimethyl-1-[4-(1-methyl-1*H-*1,2,3-triazol-4-yl)phenyl]-4,5-dihydro-3*H*-2,3-benzodiazepine-3-carboxamide
- (±)-1-[4-(2,4-dimethylthiazol-5-yl)phenyl]-7,8-dimethoxy-*N*,4-dimethyl-4,5-dihydro-3*H*-2,3-benzodiazepine-3-carboxamide
- (4*S*)-1-[4-(2,4-dimethylthiazol-5-yl)phenyl]-7,8-dimethoxy-*N*,4-dimethyl-4,5-dihydro-3*H*-2,3-benzodiazepine-3-carboxamide
- (4*R*)-1-[4-(2,4-dimethylthiazol-5-yl)phenyl]-7,8-dimethoxy-*N*,4-dimethyl-4,5-dihydro-3*H*-2,3-benzodiazepine-3-carboxamide
- (±)-1-[4-(1,2-dimethyl-1*H*-imidazol-5-yl)phenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-{4-[2-(trifluoromethyl)pyridin-3-yl]phenyl}-4,5-dihydro-3*H*-2,3-benzodiazepine-3-carboxamide
- (±)-1-[4-(6-hydroxypyridin-3-yl)phenyl]-7,8-dimethoxy-*N*,4-dimethyl-4,5-dihydro-3*H*-2,3-benzodiazepine-3-carboxamide
- (4*S*)-1-[4-(6-hydroxypyridin-3-yl)phenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-{4-[6-(trifluoromethyl)pyridin-3-yl]phenyl}-4,5-dihydro-3*H*-2,3-benzodiazepine-3-carboxamide
- (±)-7,8-dimethoxy-*N*,4-dimethyl-1-[4-(1,3,5-trimethyl-1*H*-pyrazol-4-yl)phenyl]-4,5-dihydro-3*H-*2,3-benzodiazepine-3-carboxamide
- (4*R*)-7,8-dimethoxy-*N*,4-dimethyl-1-[4-(1,3,5-trimethyl-1*H*-pyrazol-4-yl)phenyl]-4,5-dihydro-3*H-*2,3-benzodiazepine-3-carboxamide
- (4*S*)-7,8-dimethoxy-*N*,4-dimethyl-1-[4-(1,3,5-trimethyl-1*H*-pyrazol-4-yl)phenyl]-4,5-dihydro-3*H-*2,3-benzodiazepine-3-carboxamide
- (±)-1-[4-(isoxazol-4-yl)phenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-[3-(1,3,5-trimethyl-1*H*-pyrazol-4-yl)phenyl]-4,5-dihydro-3*H-*2,3-benzodiazepine-3-carboxamide
- (±)-7,8-dimethoxy-*N*,4-dimethyl-1-[3-(1-methyl-1*H-*pyrazol-4-yl)phenyl]-4,5-dihydro-3*H*-2,3-benzodiazepine-3-carboxamide
- (±)-7,8-dimethoxy-*N*,4-dimethyl-1-[3-(1-methyl-1*H-*pyrazol-5-yl)phenyl]-4,5-dihydro-3*H*-2,3-benzodiazepine-3-carboxamide
- (±)-1-[4-fluoro-3-(1,3,5-trimethyl-1*H*-pyrazol-4-yl)phenyl]-7,8-dimethoxy-*N*,4-dimethyl-4,5-dihydro-3*H*-2,3-benzodiazepine-3-carboxamide
- (±)-1-[4-fluoro-3-(1-methyl-1*H*-pyrazol-4-yl)phenyl]-7,8-dimethoxy-*N*,4-dimethyl-4,5-dihydro-3*H*-2,3-benzodiazepine-3-carboxamide
- (±)-1-[3-(3,5-dimethylisoxazol-4-yl)-4-fluorophenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-(3'-nitrobiphenyl-4-yl)-4,5-dihydro-3*H*-2,3-benzodiazepine-3-carboxamide
- (±)-1-(biphenyl-4-yl)-7,8-dimethoxy-*N*,4-dimethyl-4,5-dihydro-3*H*-2,3-benzodiazepine-3-carboxamide
- (±)-1-(2',4'-dichlorobiphenyl-4-yl)-7,8-dimethoxy-*N*,4-dimethyl-4,5-dihydro-3*H*-2,3-benzodiazepine-3-carboxamide
- (±)-1-(4'-fluorobiphenyl-4-yl)-7,8-dimethoxy-*N*,4-dimethyl-4,5-dihydro-3*H*-2,3-benzodiazepine-3-carboxamide
- (±)-1-(4'-chlorobiphenyl-4-yl)-7,8-dimethoxy-*N*,4-dimethyl-4,5-dihydro-3*H*-2,3-benzodiazepine-3-carboxamide
- (±)-7,8-dimethoxy-*N*,4-dimethyl-1-(4'-methylbiphenyl-4-yl)-4,5-dihydro-3*H*-2,3-benzodiazepine-3-carboxamide
- (±)-7,8-dimethoxy-1-(4'-methoxybiphenyl-4-yl)-*N*,4-dimethyl-4,5-dihydro-3*H*-2,3-benzodiazepine-3-carboxamide
- (±)-7,8-dimethoxy-1-[4-(6-methoxypyridin-3-yl)phenyl]-*N*,4-dimethyl-4,5-dihydro-3*H*-2,3-benzodiazepine-3-carboxamide
- (±)-7,8-dimethoxy-*N*,4-dimethyl-1-[4'-(methylsulphinyl)biphenyl-4-yl]-4,5-dihydro-3*H-*2,3-benzodiazepine-3-carboxamide
- (±)-7,8-dimethoxy-*N*,4-dimethyl-1-{2'-[(methylsulphonyl)amino]biphenyl-4-yl}-4,5-dihydro-3*H*-2,3-benzodiazepine-3-carboxamide
- (±)-7,8-dimethoxy-*N*,4-dimethyl-1-[2'-(methylsulphonyl)biphenyl-4-yl]-4,5-dihydro-3*H-*2,3-benzodiazepine-3-carboxamide
- (±)-7,8-dimethoxy-*N*,4-dimethyl-1-{4'-[(methylsulphonyl)amino]biphenyl-4-yl}-4,5-dihydro-3*H*-2,3-benzodiazepine-3-carboxamide
- (±)-7,8-dimethoxy-*N*,4-dimethyl-1-{3'-[(methylsulphonyl)amino]biphenyl-4-yl}-4,5-dihydro-3*H*-2,3-benzodiazepine-3-carboxamide
- (±)-7,8-dimethoxy-*N*,4-dimethyl-1-(2'-methylbiphenyl-4-yl)-4,5-dihydro-3*H*-2,3-benzodiazepine-3-carboxamide
- (±)-7,8-dimethoxy-*N*,4-dimethyl-1-[3'-(methylsulphonyl)biphenyl-4-yl]-4,5-dihydro-3*H-*2,3-benzodiazepine-3-carboxamide
- (±)-7,8-dimethoxy-1-[4-(2-methoxypyrimidin-5-yl)phenyl]-*N*,4-dimethyl-4,5-dihydro-3*H*-2,3-benzodiazepine-3-carboxamide
- (±)-1-(3'-cyano-4'-fluorobiphenyl-4-yl)-7,8-dimethoxy-*N*,4-dimethyl-4,5-dihydro-3*H*-2,3-benzodiazepine-3-carboxamide
- (±)-7,8-dimethoxy-1-[4-(2-methoxypyridin-3-yl)phenyl]-N,4-dimethyl-4,5-dihydro-3*H*-2,3-benzodiazepine-3-carboxamide
- (±)-1-(3'-carbamoylbiphenyl-4-yl)-7,8-dimethoxy-*N*,4-dimethyl-4,5-dihydro-3*H*-2,3-benzodiazepine-3-carboxamide
- (±)-7,8-dimethoxy-*N*,4-dimethyl-1-[4'-(pyrrolidin-1-ylcarbonyl)biphenyl-4-yl]-4,5-dihydro-3*H*-2,3-benzodiazepine-3-carboxamide
- (±)-7,8-dimethoxy-*N*,4-dimethyl-1-[4'-(morpholin-4-ylcarbonyl)biphenyl-4-yl]-4,5-dihydro-3*H*-2,3-benzodiazepine-3-carboxamide
- (±)-7,8-dimethoxy-1-[4-(5-methoxypyridin-3-yl)phenyl]-*N*,4-dimethyl-4,5-dihydro-3*H*-2,3-benzodiazepine-3-carboxamide
- (±)-7,8-dimethoxy-*N*,4-dimethyl-1-[4-(5-methylpyridin-3-yl)phenyl]-4,5-dihydro-3*H*-2,3-benzodiazepine-3-carboxamide
- (±)-7,8-dimethoxy-*N*,4-dimethyl-1-[4-(4-methylpyridin-3-yl)phenyl]-4,5-dihydro-3*H*-2,3-benzodiazepine-3-carboxamide
- (±)-1-[4'-(cyclopropylcarbamoyl)biphenyl-4-yl]-7,8-dimethoxy-*N*,4-dimethyl-4,5-dihydro-3*H*-2,3-benzodiazepine-3-carboxamide
- (±)-1-[4-(3-fluoropyridin-4-yl)phenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-[3'-(trifluoromethyl)biphenyl-4-yl]-4,5-dihydro-3*H-*2,3-benzodiazepine-3-carboxamide
- (±)-7,8-dimethoxy-*N*,4-dimethyl-1-[4'-(trifluoromethyl)biphenyl-4-yl]-4,5-dihydro-3*H-*2,3-benzodiazepine-3-carboxamide
- (±)-7,8-dimethoxy-1-(3'-methoxybiphenyl-4-yl)-*N*,4-dimethyl-4,5-dihydro-3*H*-2,3-benzodiazepine-3-carboxamide
- (±)-1-[4'-(5-chlorothien-2-yl)biphenyl-4-yl]-7,8-dimethoxy-*N*,4-dimethyl-4,5-dihydro-3*H*-2,3-benzodiazepine-3-carboxamide
- (±)-1-(3'-fluorobiphenyl-4-yl)-7,8-dimethoxy-*N*,4-dimethyl-4,5-dihydro-3*H*-2,3-benzodiazepine-3-carboxamide
- (±)-7,8-dimethoxy-1-(2'-methoxybiphenyl-4-yl)-*N*,4-40dimethyl-4,5-dihydro-3*H*-2,3-benzodiazepine-3-carboxamide
- (±)-7,8-dimethoxy-*N*,4-dimethyl-1-[2'-(trifluoromethyl)biphenyl-4-yl]-4,5-dihydro-3*H-*2,3-benzodiazepine-3-carboxamide
- (±)-1-(2'-chlorobiphenyl-4-yl)-7,8-dimethoxy-*N*,4-dimethyl-4,5-dihydro-3*H*-2,3-benzodiazepine-3-carboxamide
- (±)-1-(2'-fluorobiphenyl-4-yl)-7,8-dimethoxy-*N*,4-dimethyl-4,5-dihydro-3*H*-2,3-benzodiazepine-3-carboxamide
- (±)-1-[4'-(hydroxymethyl)biphenyl-4-yl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-[4'-(trifluoromethoxy)biphenyl-4-yl]-4,5-dihydro-3*H-*2,3-benzodiazepine-3-carboxamide
- (±)-7,8-dimethoxy-*N*,4-dimethyl-1-[3'-(pyrrolidin-1-ylcarbonyl)biphenyl-4-yl]-4,5-dihydro-3*H*-2,3-benzodiazepine-3-carboxamide
- (±)-7,8-dimethoxy-*N*,4-dimethyl-1-[3'-(piperidin-1-ylcarbonyl)biphenyl-4-yl]-4,5-dihydro-3*H*-2,3-benzodiazepine-3-carboxamide
- (±)-7,8-dimethoxy-*N*,4-dimethyl-1-[3'-(morpholin-4-ylcarbonyl)biphenyl-4-yl]-4,5-dihydro-3*H*-2,3-benzodiazepine-3-carboxamide
- (±)-1-[3'-(cyclopropylcarbamoyl)biphenyl-4-yl]-7,8-dimethoxy-*N*,4-dimethyl-4,5-dihydro-3*H*-2,3-benzodiazepine-3-carboxamide
- (±)-1-(2',4'-difluorobiphenyl-4-yl)-7,8-dimethoxy-*N*,4-dimethyl-4,5-dihydro-3*H*-2,3-benzodiazepine-3-carboxamide
- (±)-7,8-dimethoxy-*N*,4-dimethyl-1-[4-(1-methyl-1*H-*pyrazol-5-yl)phenyl]-4,5-dihydro-3*H*-2,3-benzodiazepine-3-carboxamide
- (±)-7,8-dimethoxy-*N*,4-dimethyl-1-(4'-nitrobiphenyl-4-yl)-4,5-dihydro-3*H*-2,3-benzodiazepine-3-carboxamide
- (±)-7,8-dimethoxy-*N*,4-dimethyl-1-[4-(pyridin-3-yl)phenyl]-4,5-dihydro-3*H*-2,3-benzodiazepine-3-carboxamide
- (±)-7,8-dimethoxy-1-[4-(4-methoxypyridin-3-yl)phenyl]-*N*,4-dimethyl-4,5-dihydro-3*H*-2,3-benzodiazepine-3-carboxamide
- (±)-1-(3'-cyanobiphenyl-4-yl)-7,8-dimethoxy-*N*,4-dimethyl-4,5-dihydro-3*H*-2,3-benzodiazepine-3-carboxamide
- (±)-1-(4'-cyanobiphenyl-4-yl)-7,8-dimethoxy-*N*,4-dimethyl-4,5-dihydro-3*H*-2,3-benzodiazepine-3-carboxamide
- (±)-7,8-dimethoxy-*N*,4-dimethyl-1-[2'-(trifluoromethoxy)biphenyl-4-yl]-4,5-dihydro-3*H-*2,3-benzodiazepine-3-carboxamide
- (±)-7,8-dimethoxy-*N*,4-dimethyl-1-[4'-(methylsulphonyl)biphenyl-4-yl]-4,5-dihydro-3*H-*2,3-benzodiazepine-3-carboxamide
- (±)-1-(2'-cyanobiphenyl-4-yl)-7,8-dimethoxy-*N*,4-dimethyl-4,5-dihydro-3*H*-2,3-benzodiazepine-3-carboxamide
- (±)-7,8-dimethoxy-*N*,4-dimethyl-1-[4'-(morpholin-4-yl)biphenyl-4-yl]-4,5-dihydro-3*H*-2,3-benzodiazepine-3-carboxamide
- (±)-7,8-dimethoxy-*N*,4-dimethyl-1-[4-(pyrimidin-5-yl)phenyl]-4,5-dihydro-3*H*-2,3-benzodiazepine-3-carboxamide
- (±)-1-[2'-(hydroxymethyl)biphenyl-4-yl]-7,8-dimethoxy-*N*,4-dimethyl-4,5-dihydro-3*H*-2,3-benzodiazepine-3-carboxamide
- (±)-1-(3'-{[2-(dimethylamino)ethyl]carbamoyl}biphenyl-4-yl)-7,8-dimethoxy-*N*,4-dimethyl-4,5-dihydro-3*H*-2,3-benzodiazepine-3-carboxamide
- (±)-7,8-dimethoxy-*N*,4-dimethyl-1-(3'-sulphamoylbiphenyl-4-yl)-4,5-dihydro-3*H*-2,3-benzodiazepine-3-carboxamide
- (±)-7,8-dimethoxy-*N*,4-dimethyl-1-[4'-(methylsulphamoyl)biphenyl-4-yl]-4,5-dihydro-3*H-*2,3-benzodiazepine-3-carboxamide
- (±)-7,8-dimethoxy-*N*,4-dimethyl-1-[4-(1-methyl-1*H-*pyrrol-2-yl)phenyl]-4,5-dihydro-3*H*-2,3-benzodiazepine-3-carboxamide
- (±)-7,8-dimethoxy-*N*,4-dimethyl-1-[4-(6-methylpyridin-3-yl)phenyl]-4,5-dihydro-3*H*-2,3-benzodiazepine-3-carboxamide
- (±)-1-[4'-(cyclopropylsulphamoyl)biphenyl-4-yl]-7,8-dimethoxy-*N*,4-dimethyl-4,5-dihydro-3*H*-2,3-benzodiazepine-3-carboxamide
- (±)-1-(3'-fluoro-5'-hydroxybiphenyl-4-yl)-7,8-dimethoxy-*N*,4-dimethyl-4,5-dihydro-3*H*-2,3-benzodiazepine-3-carboxamide
- (±)-1-(3'-fluoro-5'-methylbiphenyl-4-yl)-7,8-dimethoxy-*N*,4-dimethyl-4,5-dihydro-3*H*-2,3-benzodiazepine-3-carboxamide
- (±)-7,8-dimethoxy-*N*,4-dimethyl-1-[3'-(methylsulphamoyl)biphenyl-4-yl]-4,5-dihydro-3*H-*2,3-benzodiazepine-3-carboxamide
- (±)-1-[4-(5-fluoropyridin-3-yl)phenyl]-7,8-dimethoxy-*N*,4-dimethyl-4,5-dihydro-3*H*-2,3-benzodiazepine-3-carboxamide
- (±)-1-[4-(4-fluoropyridin-3-yl)phenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-[4-(2-methylpyridin-3-yl)phenyl]-4,5-dihydro-3*H*-2,3-benzodiazepine-3-carboxamide
- (±)-7,8-dimethoxy-1-[4-(2-methoxypyridin-4-yl)phenyl]-*N*,4-dimethyl-4,5-dihydro-3*H*-2,3-benzodiazepine-3-carboxamide
- (±)-1-[4-(5-cyanopyridin-3-yl)phenyl]-7,8-dimethoxy-*N*,4-dimethyl-4,5-dihydro-3*H*-2,3-benzodiazepine-3-carboxamide
- (4*R*)-7,8-dimethoxy-*N*,4-dimethyl-1-[4-(morpholin-4-yl)phenyl]-4,5-dihydro-3*H*-2,3-benzodiazepine-3-carboxamide
- (4*S*)-7,8-dimethoxy-*N*,4-dimethyl-1-[4-(morpholin-4-yl)phenyl]-4,5-dihydro-3*H*-2,3-benzodiazepine-3-carboxamide
- (4*S*)-7,8-dimethoxy-*N*,4-dimethyl-1-[4-(4-methylpiperazin-1-yl)phenyl]-4,5-dihydro-3*H*-2,3-benzodiazepine-3-carboxamide
- (±)-1-[4-(azetidin-1-yl)phenyl]-7,8-dimethoxy-*N*,4-dimethyl-4,5-dihydro-3*H*-2,3-benzodiazepine-3-carboxamide
- (4*R*)-1-[4-(azetidin-1-yl)phenyl]-7,8-dimethoxy-*N*,4-dimethyl-4,5-dihydro-3*H*-2,3-benzodiazepine-3-carboxamide
- (4*S*)-1-[4-(azetidin-1-yl)phenyl]-7,8-dimethoxy-*N*,4-dimethyl-4,5-dihydro-3*H*-2,3-benzodiazepine-3-carboxamide
- (±)-1-[4-(3-fluoroazetidin-1-yl)phenyl]-7,8-dimethoxy-*N*,4-dimethyl-4,5-dihydro-3*H*-2,3-benzodiazepine-3-carboxamide
- (4*R*)-1-[4-(3-fluoroazetidin-1-yl)phenyl]-7,8-dimethoxy-*N*,4-dimethyl-4,5-dihydro-3*H*-2,3-benzodiazepine-3-carboxamide
- (4*S*)-1-[4-(3-fluoroazetidin-1-yl)phenyl]-7,8-dimethoxy-*N*,4-dimethyl-4,5-dihydro-3*H*-2,3-benzodiazepine-3-carboxamide
- (±)-1-[4-(4-hydroxypiperidin-1-yl)phenyl]-7,8-dimethoxy-*N*,4-dimethyl-4,5-dihydro-3*H*-2,3-benzodiazepine-3-carboxamide
- (4*S*)-1-[4-(4-hydroxypiperidin-1-yl)phenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-[4-(piperazin-1-yl)phenyl]-4,5-dihydro-3*H*-2,3-benzodiazepine-3-carboxamide
- (4*S*)-7,8-dimethoxy-*N*,4-dimethyl-1-[4-(piperazin-1-yl)phenyl]-4,5-dihydro-3*H*-2,3-benzodiazepine-3-carboxamide
- (±)-7,8-dimethoxy-*N*,4-dimethyl-1-[4-(4-methylpiperazin-1-yl)phenyl]-4,5-dihydro-3*H*-2,3-benzodiazepine-3-carboxamide
- (±)-1-[4-(4-acetylpiperazin-1-yl)phenyl]-7,8-dimethoxy-*N*,4-dimethyl-4,5-dihydro-3*H*-2,3-benzodiazepine-3-carboxamide
- (4*R*)-1-[4-(4-acetylpiperazin-1-yl)phenyl]-7,8-dimethoxy-*N*,4-dimethyl-4,5-dihydro-3*H*-2,3-benzodiazepine-3-carboxamide
- (4*S*)-1-[4-(4-acetylpiperazin-1-yl)phenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-{4-[4-(trifluoroacetyl)piperazin-1-yl]phenyl}-4,5-dihydro-3*H*-2,3-benzodiazepine-3-carboxamide
- (±)-1-{4-[4-(2-hydroxy-2-methylpropanoyl)piperazin-1-yl]phenyl}-7,8-dimethoxy-*N*,4-dimethyl-4,5-dihydro-3*H*-2,3-benzodiazepine-3-carboxamide
- (±)-7,8-dimethoxy-*N*,4-dimethyl-1-{4-[4-(methylsulphonyl)piperazin-1-yl]phenyl}-4,5-dihydro-3*H*-2,3-benzodiazepine-3-carboxamide
- (4*S*)-1-[4-(1,1-dioxidothiomorpholin-4-yl)phenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-[4-(3-oxopiperazin-1-yl)phenyl]-4,5-dihydro-3*H*-2,3-benzodiazepine-3-carboxamide
- (±)-7,8-dimethoxy-*N*,4-dimethyl-1-[4-(4-methyl-3-oxopiperazin-1-yl)phenyl]-4,5-dihydro-3*H*-2,3-benzodiazepine-3-carboxamide
- (±)-7,8-dimethoxy-*N*,4-dimethyl-1-[4-(piperidin-1-yl)phenyl]-4,5-dihydro-3*H*-2,3-benzodiazepine-3-carboxamide
- (±)-7,8-dimethoxy-*N*,4-dimethyl-1-[3-(morpholin-4-yl)phenyl]-4,5-dihydro-3*H*-2,3-benzodiazepine-3-carboxamide
- (±)-1-[3-(3,3-difluoroazetidin-1-yl)phenyl]-7,8-dimethoxy-*N*,4-dimethyl-4,5-dihydro-3*H*-2,3-benzodiazepine-3-carboxamide
- (±)-1-[3-(azetidin-1-yl)phenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-[3-(4-methylpiperazin-1-yl)phenyl]-4,5-dihydro-3*H*-2,3-benzodiazepine-3-carboxamide
- (±)-1-[4-fluoro-3-(morpholin-4-yl)phenyl]-7,8-dimethoxy-*N*,4-dimethyl-4,5-dihydro-3*H*-2,3-benzodiazepine-3-carboxamide
- (±)-1-[3-(3,3-difluoroazetidin-1-yl)-4-fluorophenyl]-7,8-dimethoxy-*N*,4-dimethyl-4,5-dihydro-3*H*-2,3-benzodiazepine-3-carboxamide
- (±)-1-[4-fluoro-3-(4-hydroxypiperidin-1-yl)phenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-[4-(5-methyl-3-phenyl-1*H*-pyrazol-1-yl)phenyl]-4,5-dihydro-3*H*-2,3-benzodiazepine-3-carboxamide
- (4*S*)-7,8-dimethoxy-*N*,4-dimethyl-1-[4-(5-methyl-3-phenyl-1*H*-pyrazol-1-yl)phenyl]-4,5-dihydro-3*H*-2,3-benzodiazepine-3-carboxamide
- (±)-1-[4-(5-cyclopropyl-3-phenyl-1*H*-pyrazol-1-yl)phenyl]-7,8-dimethoxy-*N*,4-dimethyl-4,5-dihydro-3*H*-2,3-benzodiazepine-3-carboxamide
- (4*S*)-1-[4-(5-cyclopropyl-3-phenyl-1*H*-pyrazol-1-yl)phenyl]-7,8-dimethoxy-*N*,4-dimethyl-4,5-dihydro-3*H*-2,3-benzodiazepine-3-carboxamide
- (±)-7,8-dimethoxy-*N*,4-dimethyl-1-{4-[3-phenyl-5-(trifluoromethyl)-1*H*-pyrazol-1-yl]phenyl}-4,5-dihydro-3*H*-2,3-benzodiazepine-3-carboxamide
- (±)-1-{4-[3-(4-fluorophenyl)-1*H*-pyrazol-1-yl]phenyl}-7,8-dimethoxy-*N*,4-dimethyl-4,5-dihydro-3*H*-2,3-benzodiazepine-3-carboxamide
- (4*S*)-1-{4-[3-(4-fluorophenyl)-1*H*-pyrazol-1-yl]phenyl}-7,8-dimethoxy-*N*,4-dimethyl-4,5-dihydro-3*H*-2,3-benzodiazepine-3-carboxamide
- (4*R*)-1-{4-[3-(4-fluorophenyl)-1*H*-pyrazol-1-yl]phenyl}-7,8-dimethoxy-*N*,4-dimethyl-4,5-dihydro-3*H*-2,3-benzodiazepine-3-carboxamide
- (±)-7,8-dimethoxy-*N*,4-dimethyl-1-{4-[5-methyl-3-(trifluoromethyl)-1*H*-pyrazol-1-yl]phenyl}-4,5-dihydro-3*H*-2,3-benzodiazepine-3-carboxamide
- (±)-7,8-dimethoxy-*N*,4-dimethyl-1-[4-(1*H*-1,2,4-triazol-1-yl)phenyl]-4,5-dihydro-3*H*-2,3-benzodiazepine-3-carboxamide
- (±)-7,8-dimethoxy-*N*,4-dimethyl-1-[4-(5-methyl-1*H-*1,2,4-triazol-1-yl)phenyl]-4,5-dihydro-3*H*-2,3-benzodiazepine-3-carboxamide
- (±)-1-[4-(3,5-dimethyl-1*H*-1,2,4-triazol-1-yl)phenyl]-7,8-dimethoxy-*N*,4-dimethyl-4,5-dihydro-3*H*-2,3-benzodiazepine-3-carboxamide
- (±)-8-*tert*-butyl-1-[4-(3,5-dimethylisoxazol-4-yl)phenyl]-*N*,4-dimethyl-4,5-dihydro-3*H*-2,3-benzodiazepine-3-carboxamide
- (±)-7-chloro-1-[4-(3,5-dimethylisoxazol-4-yl)phenyl]-*N*,4-dimethyl-8-(trifluoromethoxy)-4,5-dihydro-3*H*-2,3-benzodiazepine-3-carboxamide
- (4*S*)-7-chloro-1-[4-(3,5-dimethylisoxazol-4-yl)phenyl]-*N*,4-dimethyl-8-(trifluoromethoxy)-4,5-dihydro-3*H*-2,3-benzodiazepine-3-carboxamide
- (4*R*)-7-chloro-1-[4-(3,5-dimethylisoxazol-4-yl)phenyl]-*N*,4-dimethyl-8-(trifluoromethoxy)-4,5-dihydro-3*H*-2,3-benzodiazepine-3-carboxamide
- (±)-8-chloro-1-[4-(3,5-dimethylisoxazol-4-yl)phenyl]-7-methoxy-*N*,4-dimethyl-4,5-dihydro-3*H-*2,3-benzodiazepine-3-carboxamide
- (4*S*)-8-chloro-1-[4-(3,5-dimethylisoxazol-4-yl)phenyl]-7-methoxy-*N*,4-dimethyl-4,5-dihydro-3*H-*2,3-benzodiazepine-3-carboxamide
- (4*R*)-8-chloro-1-[4-(3,5-dimethylisoxazol-4-yl)phenyl]-7-methoxy-*N*,4-dimethyl-4,5-dihydro-3*H-*2,3-benzodiazepine-3-carboxamide
- (±)-1-[4-(3,5-dimethylisoxazol-4-yl)phenyl]-*N*,4,8-trimethyl-4,5-dihydro-3*H*-2,3-benzodiazepine-3-carboxamide
- (±)-7,8-bis(difluoromethoxy)-1-[4-(3,5-dimethylisoxazol-4-yl)phenyl]-*N*,4-dimethyl-4,5-dihydro-3*H*-2,3-benzodiazepine-3-carboxamide
- (±)-1-[4-(3,5-dimethylisoxazol-4-yl)phenyl]-7,8-diethoxy-*N*,4-dimethyl-4,5-dihydro-3*H*-2,3-benzodiazepine-3-carboxamide
- (±)-7-(difluoromethoxy)-1-[4-(3,5-dimethylisoxazol-4-yl)phenyl]-8-methoxy-*N*,4-dimethyl-4,5-dihydro-3*H*-2,3-benzodiazepine-3-carboxamide
- (4*S*)-7-(difluoromethoxy)-1-[4-(3,5-dimethylisoxazol-4-yl)phenyl]-8-methoxy-*N*,4-dimethyl-4,5-dihydro-3*H*-2,3-benzodiazepine-3-carboxamide
- (4*R*)-7-(difluoromethoxy)-1-[4-(3,5-dimethylisoxazol-4-yl)phenyl]-8-methoxy-*N*,4-dimethyl-4,5-dihydro-3*H*-2,3-benzodiazepine-3-carboxamide
- (±)-7-(difluoromethoxy)-8-methoxy-*N*,4-dimethyl-1-[4-(4-methylpiperazin-1-yl)phenyl]-4,5-dihydro-3*H-*2,3-benzodiazepine-3-carboxamide
- (4*R*)-7-(difluoromethoxy)-8-methoxy-*N*,4-dimethyl-1-[4-(4-methylpiperazin-1-yl)phenyl]-4,5-dihydro-3*H-*2,3-benzodiazepine-3-carboxamide
- (4*S*)-7-(difluoromethoxy)-8-methoxy-*N*,4-dimethyl-1-[4-(4-methylpiperazin-1-yl)phenyl]-4,5-dihydro-3*H-*2,3-benzodiazepine-3-carboxamide
- (±)-1-(4-chlorophenyl)-*N*,4-dimethyl-8-[3-(4-methylpiperazin-1-yl)propoxyl-4,5-dihydro-3*H*-2,3-benzodiazepine-3-carboxamide
- (4*S*)-1-(4-chlorophenyl)-*N*,4-dimethyl-8-[3-(4-methylpiperazin-1-yl)propoxyl-4,5-dihydro-3*H*-2,3-benzodiazepine-3-carboxamide
- (±)-1-(4-chlorophenyl)-*N*,4-dimethyl-8-[3-(morpholin-4-yl)propoxy]-4,5-dihydro-3*H*-2,3-benzodiazepine-3-carboxamide
- (±)-1-(4-chlorophenyl)-*N*,4-dimethyl-8-[2-(4-methylpiperazin-1-yl)ethoxy]-4,5-dihydro-3*H*-2,3-benzodiazepine-3-carboxamide
- (±)-1-(4-chlorophenyl)-*N*,4-dimethyl-8-[(6-methylpyridin-2-yl)methoxy]-4,5-dihydro-3*H*-2,3-benzodiazepine-3-carboxamide
- (±)-1-[4-(3,5-dimethylisoxazol-4-yl)phenyl]-8-hydroxy-*N*,4-dimethyl-4,5-dihydro-3*H*-2,3-benzodiazepine-3-carboxamide
- (±)-1-[4-(3,5-dimethylisoxazol-4-yl)phenyl]-*N*,4-dimethyl-8-[3-(morpholin-4-yl)propoxy]-4,5-dihydro-3*H*-2,3-benzodiazepine-3-carboxamide
- (±)-7-cyano-1-[4-(3,5-dimethylisoxazol-4-yl)phenyl]-8-methoxy-*N*,4-dimethyl-4,5-dihydro-3*H-*2,3-benzodiazepine-3-carboxamide
- (±)-8-acetamido-*N*,4-dimethyl-1-[4-(morpholin-4-yl)phenyl]-4,5-dihydro-3*H*-2,3-benzodiazepine-3-carboxamide
- (±)-8-acetamido-*N*,4-dimethyl-1-[4-(4-methylpiperazin-1-yl)phenyl]-4,5-dihydro-3*H*-2,3-benzodiazepine-3-carboxamide
- (±)-8-acetamido-1-[4-(3,5-dimethylisoxazol-4-yl)phenyl]-*N*,4-dimethyl-4,5-dihydro-3*H*-2,3-benzodiazepine-3-carboxamide
- (±)-1-(4-chlorophenyl)-8-(3,5-dimethyl-1*H*-pyrazol-1-yl)-*N*,4-dimethyl-4,5-dihydro-3*H*-2,3-benzodiazepine-3-carboxamide
- (±)-1-[4-(3,5-dimethylisoxazol-4-yl)phenyl]-8-(3,5-dimethyl-1*H*-pyrazol-1-yl)-*N*,4-dimethyl-4,5-dihydro-3*H*-2,3-benzodiazepine-3-carboxamide
- (±)-1-[4-(3,5-dimethylisoxazol-4-yl)phenyl]-*N*,4-dimethyl-8-(morpholin-4-yl)-4,5-dihydro-3*H*-2,3-benzodiazepine-3-carboxamide
- (4*R*)-1-[4-(3,5-dimethylisoxazol-4-yl)phenyl]-*N*,4-dimethyl-8-(morpholin-4-yl)-4,5-dihydro-3*H*-2,3-benzodiazepine-3-carboxamide
- (4*S*)-1-[4-(3,5-dimethylisoxazol-4-yl)phenyl]-*N*,4-dimethyl-8-(morpholin-4-yl)-4,5-dihydro-3*H*-2,3-benzodiazepine-3-carboxamide
- (4*S*)-8-methoxy-*N*,4-dimethyl-1-[4-(3-oxomorpholin-4-yl)phenyl]-4,5-dihydro-3*H*-2,3-benzodiazepine-3-carboxamide
- (±)-1-{4-[4-(2-hydroxyethyl)piperazin-1-yl]phenyl}-7,8-dimethoxy-N,4-dimethyl-4,5-dihydro-3*H*-2,3-benzodiazepine-3-carboxamide
- (±)-7,8-dimethoxy-1-[4-(4-methoxypiperidin-1-yl)phenyl]-N,4-dimethyl-4,5-dihydro-3*H*-2,3-benzodiazepine-3-carboxamide
- (4*R*)-7,8-dimethoxy-1-[4-(4-methoxypiperidin-1-yl)phenyl]-N,4-dimethyl-4,5-dihydro-3*H*-2,3-benzodiazepine-3-carboxamide
- (4*S*)-7,8-dimethoxy-1-[4-(4-methoxypiperidin-1-yl)phenyl]-N,4-dimethyl-4,5-dihydro-3*H*-2,3-benzodiazepine-3-carboxamide
- (±)-1-{4-[4-(dimethylamino)piperidin-1-yl]phenyl}-7,8-dimethoxy-N,4-dimethyl-4,5-dihydro-3*H*-2,3-benzodiazepine-3-carboxamide
- (4*R*)-1-{4-[4-(dimethylamino)piperidin-1-yl]phenyl}-7,8-dimethoxy-N,4-dimethyl-4,5-dihydro-3*H*-2,3-benzodiazepine-3-carboxamide
- (4*S*)-1-{4-[4-(dimethylamino)piperidin-1-yl]phenyl}-7,8-dimethoxy-N,4-dimethyl-4,5-dihydro-3*H*-2,3-benzodiazepine-3-carboxamide
- (±)-1-[4-(3,3-difluoroazetidin-1-yl)phenyl]-7,8-dimethoxy-N,4-dimethyl-4,5-dihydro-3H-2,3-benzodiazepine-3-carboxamide
- (±)-1-[4-(4-acetamidopiperidin-1-yl)phenyl]-7,8-dimethoxy-N,4-dimethyl-4,5-dihydro-3H-2,3-benzodiazepine-3-carboxamide
- (±)-1-{4-[4-(2-hydroxyethyl)piperidin-1-yl]phenyl}-7,8-dimethoxy-N,4-dimethyl-4,5-dihydro-3H-2,3-benzodiazepine-3-carboxamide
- (±)-1-{4-[4-(2-hydroxyethyl)piperidin-1-yl]phenyl}-7,8-dimethoxy-N,4-dimethyl-4,5-dihydro-3H-2,3-benzodiazepine-3-carboxamide
- (±)-1-[4-(3-hydroxyazetidin-1-yl)phenyl]-7,8-dimethoxy-N,4-dimethyl-4,5-dihydro-3H-2,3-benzodiazepine-3-carboxamide
- (±)-1-[4-(3-hydroxy-3-methylazetidin-1-yl)phenyl]-7,8-dimethoxy-N,4-dimethyl-4,5-dihydro-3H-2,3-benzodiazepine-3-carboxamide
- (4*R*)-1-[4-(3-hydroxy-3-methylazetidin-1-yl)phenyl]-7,8-dimethoxy-N,4-dimethyl-4,5-dihydro-3H-2,3-benzodiazepine-3-carboxamide
- (4*S*)-1-[4-(3-hydroxy-3-methylazetidin-1-yl)phenyl]-7,8-dimethoxy-N,4-dimethyl-4,5-dihydro-3H-2,3-benzodiazepine-3-carboxamide
- (±)-1-[4-(4-isopropylpiperazin-1-yl)phenyl]-7,8-dimethoxy-N,4-dimethyl-4,5-dihydro-3H-2,3-benzodiazepine-3-carboxamide
- (±)-7,8-dimethoxy-1-[4-(3-methoxyazetidin-1-yl)phenyl]-N,4-dimethyl-4,5-dihydro-3H-2,3-benzodiazepine-3-carboxamide
- (±)-1-[4-(4-hydroxy-4-methylpiperidin-1-yl)phenyl]-7,8-dimethoxy-N,4-dimethyl-4,5-dihydro-3H-2,3-benzodiazepine-3-carboxamide
- (4*R*)-1-[4-(4-hydroxy-4-methylpiperidin-1-yl)phenyl]-7,8-dimethoxy-N,4-dimethyl-4,5-dihydro-3H-2,3-benzodiazepine-3-carboxamide
- (4*S*)-1-[4-(4-hydroxy-4-methylpiperidin-1-yl)phenyl]-7,8-dimethoxy-N,4-dimethyl-4,5-dihydro-3H-2,3-benzodiazepine-3-carboxamide
- (±)-7,8-dimethoxy-N,4-dimethyl-1-{4-[4-(methylcarbamoyl)piperidin-1-yl]phenyl}-4,5-dihydro-3H-2,3-benzodiazepine-3-carboxamide
- (4S)-7,8-dimethoxy-N,4-dimethyl-1-{4-[4-(methylcarbamoyl)piperidin-1-yl]phenyl}-4,5-dihydro-3H-2,3-benzodiazepine-3-carboxamide
- (4*R*)-7,8-dimethoxy-N,4-dimethyl-1-{4-[4-(methylcarbamoyl)piperidin-1-yl]phenyl}-4,5-dihydro-3H-2,3-benzodiazepine-3-carboxamide
- (±)-1-{4-[(3S)-3-hydroxypyrrolidin-1-yl]phenyl}-7,8-dimethoxy-N,4-dimethyl-4,5-dihydro-3H-2,3-benzodiazepine-3-carboxamide
- (4S)-1-{4-[(3S)-3-hydroxypyrrolidin-1-yl]phenyl}-7,8-dimethoxy-N,4-dimethyl-4,5-benzodiazepin-1-yl]phenyl}-4-methylpiperidin-4-yl)carbamate
- (±)-1-{4-[(2S,5R)-2,5-dimethylpiperazin-1-yl]phenyl}-7,8-dimethoxy-N,4-dimethyl-4,5-dihydro-3H-2,3-benzodiazepine-3-carboxamide
- (±)-1-{4-[4-(2,2-difluoroethyl)piperazin-1-yl]phenyl}-7,8-dimethoxy-N,4-dimethyl-4,5-dihydro-3H-2,3-benzodiazepine-3-carboxamide
- (4S)-7,8-dimethoxy-N,4-dimethyl-1-[4-(3-oxopiperazin-1-yl)phenyl]-4,5-dihydro-3H-2,3-benzodiazepine-3-carboxamide
- (4S)-1-{4-[(2R,6S)-2,6-dimethylmorpholin-4-yl]phenyl}-7,8-dimethoxy-N,4-dimethyl-4,5-dihydro-3H-2,3-benzodiazepine-3-carboxamide
- (±)-7,8-dimethoxy-N,4-dimethyl-1-[4-(4-oxopiperidin-1-yl)phenyl]-4,5-dihydro-3H-2,3-benzodiazepine-3-carboxamide
- (±)-7,8-dimethoxy-N,4-dimethyl-1-{4-[4-(2,2,2-trifluoroethyl)piperazin-1-yl]phenyl}-4,5-dihydro-3H-2,3-benzodiazepine-3-carboxamide
- (4S)-1-{4-[(3R,5S)-3,5-dimethylpiperazin-1-yl]phenyl}-7,8-dimethoxy-N,4-dimethyl-4,5-dihydro-3H-2,3-benzodiazepine-3-carboxamide
- (±)-7,8-dihydroxy-N,4-dimethyl-1-[4-(4-methylpiperazin-1-yl)phenyl]-4,5-dihydro-3H-2,3-benzodiazepine-3-carboxamide
- (±)-7,8-diethoxy-N,4-dimethyl-1-[4-(4-methylpiperazin-1-yl)phenyl]-4,5-dihydro-3H-2,3-benzodiazepine-3-carboxamide
- (±)-4-ethyl-7,8-dimethoxy-N-methyl-1-[4-(4-methylpiperazin-1-yl)phenyl]-4,5-dihydro-3H-2,3-benzodiazepine-3-carboxamide
- (4S)-4-ethyl-7,8-dimethoxy-N-methyl-1-[4-(4-methylpiperazin-1-yl)phenyl]-4,5-dihydro-3H-2,3-benzodiazepine-3-carboxamide
- (±)-4-ethyl-7,8-dimethoxy-N-methyl-1-[4-(piperazin-1-yl)phenyl]-4,5-dihydro-3H-2,3-benzodiazepine-3-carboxamide
- (4S)-4-ethyl-7,8-dimethoxy-N-methyl-1-[4-(piperazin-1-yl)phenyl]-4,5-dihydro-3H-2,3-benzodiazepine-3-carboxamide
- (4R)-4-ethyl-7,8-dimethoxy-N-methyl-1-[4-(piperazin-1-yl)phenyl]-4,5-dihydro-3H-2,3-benzodiazepine-3-carboxamide
- (±)-4-ethyl-7,8-dimethoxy-N-methyl-1-{4-[4-(methylsulphonyl)piperazin-1-yl]phenyl}-4,5-dihydro-3H-2,3-benzodiazepine-3-carboxamide
- (4S)-4-ethyl-7,8-dimethoxy-N-methyl-1-{4-[4-(methylsulphonyl)piperazin-1-yl]phenyl}-4,5-dihydro-3H-2,3-benzodiazepine-3-carboxamide
- (4R)-4-ethyl-7,8-dimethoxy-N-methyl-1-{4-[4-(methylsulphonyl)piperazin-1-yl]phenyl}-4,5-dihydro-3H-2,3-benzodiazepine-3-carboxamide
- (±)-4-ethyl-1-[4-(3-fluoroazetidin-1-yl)phenyl]-7,8-dimethoxy-N-methyl-4,5-dihydro-3H-2,3-benzodiazepine-3-carboxamide
- (±)-1-[4-(4-acetylpiperazin-1-yl)phenyl]-4-ethyl-7,8-dimethoxy-N-methyl-4,5-dihydro-3H-2,3-benzodiazepine-3-carboxamide
- (±)-1-[4-(1,1-dioxidothiomorpholin-4-yl)phenyl]-4-ethyl-7,8-dimethoxy-N-methyl-4,5-dihydro-3H-2,3-benzodiazepine-3-carboxamide
- (±)-4-ethyl-1-[4-(4-hydroxypiperidin-1-yl)phenyl]-7,8-dimethoxy-N-methyl-4,5-dihydro-3H-2,3-benzodiazepine-3-carboxamide
- (±)-4-ethyl-7,8-dimethoxy-N-methyl-1-[4-(morpholin-4-yl)phenyl]-4,5-dihydro-3H-2,3-benzodiazepine-3-carboxamide
- (4S)-4-ethyl-7,8-dimethoxy-N-methyl-1-[4-(morpholin-4-yl)phenyl]-4,5-dihydro-3H-2,3-benzodiazepine-3-carboxamide
- (±)-8-chloro-1-[4-(1,1-dioxidothiomorpholin-4-yl)phenyl]-N,4-dimethyl-7-(trifluoromethoxy)-4,5-dihydro-3H-2,3-benzodiazepine-3-carboxamide
- (±)-8-chloro-N,4-dimethyl-1-[4-(piperazin-1-yl)phenyl]-7-(trifluoromethoxy)-4,5-dihydro-3H-2,3-benzodiazepine-3-carboxamide
- (±)-8-chloro-1-[4-(4-hydroxypiperidin-1-yl)phenyl]-N,4-dimethyl-7-(trifluoromethoxy)-4,5-dihydro-3H-2,3-benzodiazepine-3-carboxamide
- (±)-8-chloro-N,4-dimethyl-1-[4-(4-methylpiperazin-1-yl)phenyl]-7-(trifluoromethoxy)-4,5-dihydro-3H-2,3-benzodiazepine-3-carboxamide
- (±)-8-chloro-N,4-dimethyl-1-{4-[4-(methylsulphonyl)piperazin-1-yl]phenyl}-7-(trifluoromethoxy)-4,5-dihydro-3H-2,3-benzodiazepine-3-carboxamide
- (±)-8-(1,1-dioxidothiomorpholin-4-yl)-1-[4-(1,1-dioxidothiomorpholin-4-yl)phenyl]-N,4-dimethyl-7-(trifluoromethoxy)-4,5-dihydro-3H-2,3-benzodiazepine-3-carboxamide
- (±)-1-(4-chlorophenyl)-8-(1,1-dioxidothiomorpholin-4-yl)-N,4-dimethyl-7-(trifluoromethoxy)-4,5-dihydro-3H-2,3-benzodiazepine-3-carboxamide
- (±)-1-[4-(4-acetylpiperazin-1-yl)phenyl]-N,4-dimethyl-7-(trifluoromethoxy)-4,5-dihydro-3H-2,3-benzodiazepine-3-carboxamide
- (±)-1-{4-[4-(2-hydroxyethyl)piperazin-1-yl]phenyl}-N,4-dimethyl-7-(trifluoromethoxy)-4,5-dihydro-3H-2,3-benzodiazepine-3-carboxamide
- (±)-8-methoxy-N,4-dimethyl-1-[4-(4-methylpiperazin-1-yl)phenyl]-7-(trifluoromethoxy)-4,5-dihydro-3H-2,3-benzodiazepine-3-carboxamide
- (±)-4-ethyl-7,8-dimethoxy-N-methyl-1-[4-(1,3,5-trimethyl-1H-pyrazol-4-yl)phenyl]-4,5-dihydro-3H-2,3-benzodiazepine-3-carboxamide
- (±)-4-ethyl-1-(4'-fluorobiphenyl-4-yl)-7,8-dimethoxy-N-methyl-4,5-dihydro-3H-2,3-benzodiazepine-3-carboxamide
- (±)-4-ethyl-7,8-dimethoxy-N-methyl-1-[4-(1-methyl-1H-1,2,3-triazol-4-yl)phenyl]-4,5-dihydro-3H-2,3-benzodiazepine-3-carboxamide
- (±)-1-[4-(3,5-dimethyl-1,2-oxazol-4-yl)phenyl]-4-ethyl-7,8-dimethoxy-N-methyl-4,5-dihydro-3H-2,3-benzodiazepine-3-carboxamide
- (±)-4-isopropyl-7,8-dimethoxy-N-methyl-1-[4-(1,3,5-trimethyl-1H-pyrazol-4-yl)phenyl]-4,5-dihydro-3H-2,3-benzodiazepine-3-carboxamide
- (±)-4-ethyl-7,8-dimethoxy-N-methyl-1-[4-(2-oxopyrrolidin-1-yl)phenyl]-4,5-dihydro-3H-2,3-benzodiazepine-3-carboxamide
- (±)-4-ethyl-7,8-dimethoxy-N-methyl-1-[4-(3-oxomorpholin-4-yl)phenyl]-4,5-dihydro-3H-2,3-benzodiazepine-3-carboxamide
- (±)-4-ethyl-7,8-dimethoxy-N-methyl-1-[4-(2-oxopiperidin-1-yl)phenyl]-4,5-dihydro-3H-2,3-benzodiazepine-3-carboxamide
- (±)-4-ethyl-7,8-dimethoxy-N-methyl-1-[4-(2-oxo-1,3-oxazolidin-3-yl)phenyl]-4,5-dihydro-3H-2,3-benzodiazepine-3-carboxamide
- (4S)-7,8-dimethoxy-N,4-dimethyl-1-[4-(4-methyl-2-oxo-1,4-diazepan-1-yl)phenyl]-4,5-dihydro-3H-2,3-benzodiazepine-3-carboxamide
- (±)-1-(4-chlorophenyl)-N,4-dimethyl-7-(2-oxo-1,3-oxazolidin-3-yl)-4,5-dihydro-3H-2,3-benzodiazepine-3-carboxamide
- (4S)-1-(4-chlorophenyl)-N,4-dimethyl-7-(2-oxo-1,3-oxazolidin-3-yl)-4,5-dihydro-3H-2,3-benzodiazepine-3-carboxamide
- (±)-1-(4-chlorophenyl)-N,4-dimethyl-7-(2-oxopiperidin-1-yl)-4,5-dihydro-3H-2,3-benzodiazepine-3-carboxamide
- (±)-1-(4-chlorophenyl)-N,4-dimethyl-7-(3-oxomorpholin-4-yl)-4,5-dihydro-3H-2,3-benzodiazepine-3-carboxamide
- (±)-1-(4-chlorophenyl)-N,4-dimethyl-7-(morpholin-4-yl)-4,5-dihydro-3H-2,3-benzodiazepine-3-carboxamide
- (±)-1-(4-chlorophenyl)-N,4-dimethyl-7-(pyrrolidin-1-yl)-4,5-dihydro-3H-2,3-benzodiazepine-3-carboxamide
- (±)-1-(4-chlorophenyl)-7-(1,1-dioxidothiomorpholin-4-yl)-N,4-dimethyl-4,5-dihydro-3H-2,3-benzodiazepine-3-carboxamide
- (±)-1-(4-chlorophenyl)-N,4-dimethyl-7-(4-methylpiperazin-1-yl)-4,5-dihydro-3H-2,3-benzodiazepine-3-carboxamide
- (±)-N,4-dimethyl-7-(4-methylpiperazin-1-yl)-1-[4-(4-methylpiperazin-1-yl)phenyl]-4,5-dihydro-3H-2,3-benzodiazepine-3-carboxamide
- (±)-N,4-dimethyl-7-(4-methyl-3-oxopiperazin-1-yl)-1-[4-(4-methyl-3-oxopiperazin-1-yl)phenyl]-4,5-dihydro-3H-2,3-benzodiazepine-3-carboxamide
- (±)-1-(4-chlorophenyl)-7-(4-fluorophenyl)-N,4-dimethyl-4,5-dihydro-3H-2,3-benzodiazepine-3-carboxamide
- (±)-1-(4-chlorophenyl)-N,4-dimethyl-7-(pyridin-4-yl)-4,5-dihydro-3H-2,3-benzodiazepine-3-carboxamide
- (±)-1-(4-chlorophenyl)-7-(6-hydroxypyridin-3-yl)-N,4-dimethyl-4,5-dihydro-3H-2,3-benzodiazepine-3-carboxamide
- (±)-1-(4-chlorophenyl)-7-(3,5-dimethyl-1,2-oxazol-4-yl)-N,4-dimethyl-4,5-dihydro-3H-2,3-benzodiazepine-3-carboxamide
- (±)-1-(4-chlorophenyl)-N,4-dimethyl-7-(1-methyl-1H-1,2,3-triazol-4-yl)-4,5-dihydro-3H-2,3-benzodiazepine-3-carboxamide
- (4R)-1-[4-(4-hydroxypiperidin-1-yl)phenyl]-7,8-dimethoxy-N,4-dimethyl-4,5-dihydro-3H-2,3-benzodiazepine-3-carboxamide
- (4R)-7,8-dimethoxy-N,4-dimethyl-1-[4-(4-methylpiperazin-1-yl)phenyl]-4,5-dihydro-3H-2,3-benzodiazepine-3-carboxamide
- (±)-1-[4-(1,1-dioxido-1,2-thiazolidin-2-yl)phenyl]-7,8-dimethoxy-N,4-dimethyl-4,5-dihydro-3H-2,3-benzodiazepine-3-carboxamide
- (±)-1-{7,8-dimethoxy-4-methyl-1-[4-(4-methylpiperazin-1-yl)phenyl]-4,5-dihydro-3H-2,3-benzodiazepin-3-yl}ethanone
- 1-{(4S)-7,8-dimethoxy-4-methyl-1-[4-(4-methylpiperazin-1-yl)phenyl]-4,5-dihydro-3H-2,3-benzodiazepin-3-yl}ethanone
- (±)-1-{1-[4-(3,5-dimethyl-1,2-oxazol-4-yl)phenyl]-7,8-dimethoxy-4-methyl-4,5-dihydro-3H-2,3-benzodiazepin-3-yl}ethanone
- (±)-7,8-dimethoxy-N,4-dimethyl-1-[4-(5-methyl-1,3,4-oxadiazol-2-yl)phenyl]-4,5-dihydro-3H-2,3-benzodiazepine-3-carboxamide
- (±)-7,8-dimethoxy-N,4-dimethyl-1-[4-(3-methyl-1,2,4-oxadiazol-5-yl)phenyl]-4,5-dihydro-3H-2,3-benzodiazepine-3-carboxamide
- (±)-N,4-dimethyl-8-(trifluoromethoxy)-1-[4-(1,3,5-trimethyl-1H-pyrazol-4-yl)phenyl]-4,5-dihydro-3H-2,3-benzodiazepine-3-carboxamide
- (4S)-N,4-dimethyl-8-(trifluoromethoxy)-1-[4-(1,3,5-trimethyl-1H-pyrazol-4-yl)phenyl]-4,5-dihydro-3H-2,3-benzodiazepine-3-carboxamide
- (4R)-N,4-dimethyl-8-(trifluoromethoxy)-1-[4-(1,3,5-trimethyl-1H-pyrazol-4-yl)phenyl]-4,5-dihydro-3H-2,3-benzodiazepine-3-carboxamide
- (±)-N,4-dimethyl-1-[4-(4-methyl-1-piperazinyl)phenyl]-8-(trifluoromethoxy)-4,5-dihydro-3H-2,3-benzodiazepine-3-carboxamide
- (4R)-N,4-dimethyl-1-[4-(4-methyl-1-piperazinyl)phenyl]-8-(trifluoromethoxy)-4,5-dihydro-3H-2,3-benzodiazepine-3-carboxamide
- (4S)-N,4-dimethyl-1-[4-(4-methyl-1-piperazinyl)phenyl]-8-(trifluoromethoxy)-4,5-dihydro-3H-2,3-benzodiazepine-3-carboxamide
- (±)-1-[4-(4-hydroxy-1-piperidinyl)phenyl]-N,4-dimethyl-8-(trifluoromethoxy)-4,5-dihydro-3H-2,3-benzodiazepine-3-carboxamide
- (±)-1-[2,4-dibromo-5-(4-methylpiperazin-1-yl)phenyl]-7,8-dimethoxy-N,4-dimethyl-4,5-dihydro-3H-2,3-benzodiazepine-3-carboxamide
- (4S)-1-[3-bromo-4-(4-methylpiperazin-1-yl)phenyl]-7,8-dimethoxy-N,4-dimethyl-4,5-dihydro-3H-2,3-benzodiazepine-3-carboxamide
- (4S)-1-[3-cyano-4-(4-methylpiperazin-1-yl)phenyl]-7,8-dimethoxy-N,4-dimethyl-4,5-dihydro-3H-2,3-benzodiazepine-3-carboxamide
- (±)-7,8-dimethoxy-4-methyl-1-[4-(4-methylpiperazin-1-yl)phenyl]-3-(1-oxopropyl)-4,5-dihydro-3H-2,3-benzodiazepine
- (±)-3-(cyclopropylcarbonyl)-7,8-dimethoxy-4-methyl-1-[4-(4-methylpiperazin-1-yl)phenyl]-4,5-dihydro-3H-2,3-benzodiazepine
- (±)-N-cyclopropyl-7,8-dimethoxy-4-methyl-1-[4-(4-methylpiperazin-1-yl)phenyl]-4,5-dihydro-3H-2,3-benzodiazepine
- (±)-7,8-dimethoxy-N,4-dimethyl-1-[4-(4-methylpiperazin-1-yl)phenyl]-4,5-dihydro-3H-2,3-benzodiazepin-3-carbothioamide
- methyl (±)-7,8-dimethoxy-4-methyl-l-[4-(4-methylpiperazin-1-yl)phenyl]-4,5-dihydro-3H-2,3-benzodiazepine-3-carboxylate
- ethyl (±)-7,8-dimethoxy-4-methyl-1-[4-(4-methylpiperazin-1-yl)phenyl]-4,5-dihydro-3H-2,3-benzodiazepine-3-carboxylate
- (±)-N-ethyl-7,8-dimethoxy-4-methyl-1-[4-(4-methylpiperazin-1-yl)phenyl]-4,5-dihydro-3H-2,3-benzodiazepine-3-carboxamide
- (4S)-N-ethyl-7,8-dimethoxy-4-methyl-1-[4-(4-methylpiperazin-1-yl)phenyl]-4,5-dihydro-3H-2,3-benzodiazepine-3-carboxamide.

16. Compounds according to any of Claims 1 to 15 for use in the prophylaxis and/or therapy of hyperproliferative disorders, benign hyperplasias, inflammatory disorders, autoimmune disorders, sepsis, viral infections, vascular disorders, atherosclerotic disorders and neurodegenerative disorders.

17. Compounds according to any of Claims 1 to 15 for use in the prophylaxis and/or therapy of tumour disorders.

18. Compounds according to any of Claims 1 to 15 for use in the control of male fertility.

19. Compounds according to any of Claims 1 to 15 use in for the prophylaxis and/or therapy of leukaemias, prostate carcinomas, breast carcinomas, melanomas or multiple myelomas.

20. Use of a compound according to any of Claims 1 to 15 for preparing a medicament.

21. Compound according to any of Claims 1 to 15 for use in the prophylaxis and/or therapy of disorders of man or any other mammal.

22. Compound according to any of Claims 1 to 15 in combination with a further active compound.

23. Pharmaceutical formulation comprising a compound according to any of Claims 1 to 15.

24. Intermediates of the general formula (Ia), 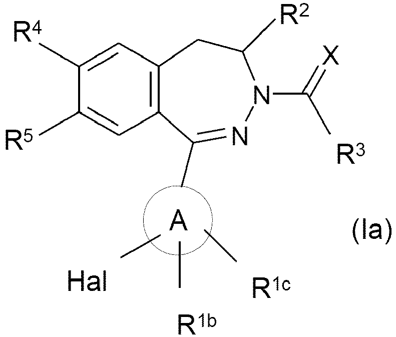 in which X, A, R^{1b}, R^{1c} R², R³, R⁴ and R⁵ have the meanings given in the general formula (I) and Hal represents bromine for preparing the compounds of the general formula (I).

25. Intermediates:
(4R)-1-(4-bromophenyl)-7,8-dimethoxy-N,4-dimethyl-4,5-dihydro-3H-2,3-benzodiazepine-3-carboxamide (4S)-1-(4-bromophenyl)-7,8-dimethoxy-N,4-dimethyl-4,5-dihydro-3H-2,3-benzodiazepine-3-carboxamide (±)-1-(4-bromophenyl)-7,8-dimethoxy-N,4-dimethyl-4,5-dihydro-3H-2,3-benzodiazepine-3-carboxamide (±)-1-(4-chlorophenyl)-8-methoxy-N,4-dimethyl-4,5-dihydro-3H-2,3-benzodiazepine-3-carboxamide (±)-1-(3-bromophenyl)-7,8-dimethoxy-N,4-dimethyl-4,5-dihydro-3H-2,3-benzodiazepine-3-carboxamide (±)-8-bromo-1-(4-chlorophenyl)-N,4-dimethyl-4,5-dihydro-3H-2,3-benzodiazepine-3-carboxamide (4R)-8-bromo-1-(4-chlorophenyl)-N,4-dimethyl-4,5-dihydro-3H-2,3-benzodiazepine-3-carboxamide (4S)-8-bromo-1-(4-chlorophenyl)-N,4-dimethyl-4,5-dihydro-3H-2,3-benzodiazepine-3-carboxamide (4S)-1-(4-bromophenyl)-8-methoxy-N,4-dimethyl-4,5-dihydro-3H-2,3-benzodiazepine-3-carboxamide (±)-1-(4-chlorophenyl)-8-hydroxy-N,4-dimethyl-4,5-dihydro-3H-2,3-benzodiazepine-3-carboxamide (±)-1-(3-bromo-4-fluorophenyl)-7,8-dimethoxy-N,4-dimethyl-4,5-dihydro-3H-2,3-benzodiazepine-3-carboxamide (±)-8-tert-butyl-1-(4-chlorophenyl)-N,4-dimethyl-4,5-dihydro-3H-2,3-benzodiazepine-3-carboxamide (4S)-8-tert-butyl-1-(4-chlorophenyl)-N,4-dimethyl-4,5-dihydro-3H-2,3-benzodiazepine-3-carboxamide (4R)-8-tert-butyl-1-(4-chlorophenyl)-N,4-dimethyl-4,5-dihydro-3H-2,3-benzodiazepine-3-carboxamide (±)-8-chloro-1-(4-chlorophenyl)-7-methoxy-N,4-dimethyl-4,5-dihydro-3H-2,3-benzodiazepine-3-carboxamide (4S)-8-chloro-1-(4-chlorophenyl)-7-methoxy-N,4-dimethyl-4,5-dihydro-3H-2,3-benzodiazepine-3-carboxamide (4R)-8-chloro-1-(4-chlorophenyl)-7-methoxy-N,4-dimethyl-4,5-dihydro-3H-2,3-benzodiazepine-3-carboxamide (±)-1-(4-chlorophenyl)-N,4,8-trimethyl-4,5-dihydro-3H-2,3-benzodiazepine-3-carboxamide (4S)-1-(4-chlorophenyl)-N,4,8-trimethyl-4,5-dihydro-3H-2,3-benzodiazepine-3-carboxamide (4R)-1-(4-chlorophenyl)-N,4,8-trimethyl-4,5-dihydro-3H-2,3-benzodiazepine-3-carboxamide (±)-8-amino-1-(4-chlorophenyl)-N,4-dimethyl-4,5-dihydro-3H-2,3-benzodiazepine-3-carboxamide (±)-1-[8-tert-butyl-1-(4-chlorophenyl)-4-methyl-4,5-dihydro-3H-2,3-benzodiazepin-3-yl]ethanone (±)-8-acetamido-1-(4-chlorophenyl)-N,4-dimethyl-4,5-dihydro-3H-2,3-benzodiazepine-3-carboxamide (±)-1-(4-bromophenyl)-7-hydroxy-8-methoxy-N,4-dimethyl-4,5-dihydro-3H-2,3-benzodiazepine-3-carboxamide (±)-1-(4-bromophenyl)-7,8-dihydroxy-N,4-dimethyl-4,5-dihydro-3H-2,3-benzodiazepine-3-carboxamide (±)-1-(4-bromophenyl)-7,8-bis(difluoromethoxy)-N,4-dimethyl-4,5-dihydro-3H-2,3-benzodiazepine-3-carboxamide (±)-1-(4-bromophenyl)-7,8-diethoxy-N,4-dimethyl-4,5-dihydro-3H-2,3-benzodiazepine-3-carboxamide (±)-7-bromo-1-(4-chlorophenyl)-8-methoxy-N,4-dimethyl-4,5-dihydro-3H-2,3-benzodiazepine-3-carboxamide (±)-1-(4-chlorophenyl)-7-cyano-8-methoxy-N,4-dimethyl-4,5-dihydro-3H-2,3-benzodiazepine-3-carboxamide (±)-1-(4-bromophenyl)-7-(difluoromethoxy)-8-methoxy-N,4-dimethyl-4,5-dihydro-3H-2,3-benzodiazepine-3-carboxamide (±)-1-(4-bromophenyl)-4-ethyl-7,8-dimethoxy-N-methyl-4,5-dihydro-3H-2,3-benzodiazepine-3-carboxamide (±)-1-(4-bromophenyl)-4-isopropyl-7,8-dimethoxy-N-methyl-4,5-dihydro-3H-2,3-benzodiazepine-3-carboxamide (±)-7-bromo-1-(4-chlorophenyl)-N,4-dimethyl-4,5-dihydro-3H-2,3-benzodiazepine-3-carboxamide (±)-8-chloro-1-(4-chlorophenyl)-N,4-dimethyl-7-(trifluoromethoxy)-4,5-dihydro-3H-2,3-benzodiazepine-3-carboxamide (4S)-8-chloro-1-(4-chlorophenyl)-N,4-dimethyl-7-(trifluoromethoxy)-4,5-dihydro-3H-2,3-benzodiazepine-3-carboxamide (±)-1-(4-chlorophenyl)-8-methoxy-N,4-dimethyl-7-(trifluoromethoxy)-4,5-dihydro-3H-2,3-benzodiazepine-3-carboxamide (±)-1-[1-(4-bromophenyl)-7,8-dimethoxy-4-methyl-4,5-dihydro-3H-2,3-benzodiazepin-3-yl]ethanone (±)-1-(4-bromophenyl)-N-ethyl-7,8-dimethoxy-4-methyl-4,5-dihydro-3H-2,3-benzodiazepine-3-carboxamide.

## Revendications

1. Composés de formule générale (I) 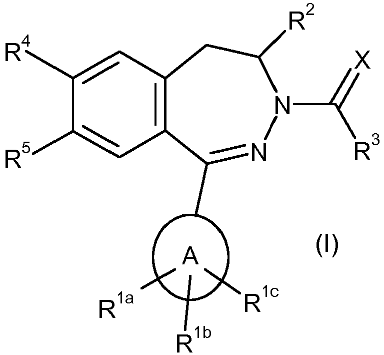 dans laquelle
X représente un atome d'oxygène ou de soufre, et
A représente un cycle hétéroaryle monocyclique à 5 ou 6 atomes de cycle ou un cycle phényle, et
R^{1a} représente hydrogène, halogène, cyano, carboxy, amino ou aminosulfonyle,
ou
un radical alcoxy en C₁-C₆, alcoxy en C₁-C₃-alkyle en C₁-C₃, alcoxy en C₁-C₃-alcoxy en C₂-C₃
alkylamino en C₁-C₆, alkylcarbonylamino en C₁-C₆, alkylamino en C₁-C₆-alkyle en C₁-C₆, *N*-(hétérocyclyl)-alkyle en C₁-C₆, *N-*(hétérocyclyl)-alcoxy en C₁-C₆, hydroxy-alkyle en C₁-C₆, hydroxy-alcoxy en C₁-C₆, halogéno-alkyle en C₁-C₆, halogéno-alcoxy en C₁-C₆, alkylcarbonyle en C₁-C₆ ou alcoxycarbonyle en C₁-C₆,
ou
un radical hétérocyclyle monocyclique de 3 à 8 atomes de cycle, qui peut éventuellement être substitué une ou plusieurs fois, de manière identique ou différente, avec halogène, cyano, nitro, hydroxy, amino, oxo, carboxy, alkyle en C₁-C₆, alcoxy en C₁-C₆, alcoxy en C₁-C₆-alkyle en C₁-C₆, hydroxy-alkyle en C₁-C₆, alkylamino en C₁-C₆, amino-alkyle en C₁-C₆, alkylamino en C₁-C₆-alkyle en C₁-C₆, halogéno-alkyle en C₁-C₆, halogéno-alcoxy en C₁-C₆, cycloalkyle en C₃-C₁₀, phényle, halophényle, phényl-alkyle en C₁-C₆, pyridinyle, -NR⁶C(=O)-R⁹, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, et/ou un radical hétérocyclyle monocyclique de 3 à 8 atomes de cycle,
ou
un radical hétéroaryle monocyclique à 5 ou 6 atomes de cycle, qui peut éventuellement être substitué une ou plusieurs fois, de manière identique ou différente, avec halogène, hydroxy, amino, cyano, nitro, carboxy, alkyle en C₁-C₆, alcoxy en C₁-C₆, alcoxy en C₁-C₆-alkyle en C₁-C₆, alkylamino en C₁-C₆, amino-alkyle en C₁-C₆, alkylamino en C₁-C₆-alkyle en C₁-C₆, halogéno-alkyle en C₁-C₆, halogéno-alcoxy en C₁-C₆, cycloalkyle en C₃-C₁₀, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, et/ou avec un radical hétérocyclyle monocyclique de 3 à 8 atomes de cycle, et/ou avec un radical hétéroaryle monocyclique à 5 ou 6 atomes de cycle, et/ou avec un radical phényle, qui peut de son côté éventuellement être substitué une ou plusieurs fois, de manière identique ou différente, avec halogène, alkyle en C₁-C₃ et/ou alcoxy en C₁-C₃,
ou
un radical phényle, qui peut éventuellement être substitué une ou plusieurs fois, de manière identique ou différente, avec halogène, amino, hydroxy, cyano, nitro, carboxy, alkyle en C₁-C₆, alcoxy en C₁-C₆, alcoxy en C₁-C₆-alkyle en C₁-C₆, alkylamino en C₁-C₆, amino-alkyle en C₁-C₆, alkylamino en C₁-C₆-alkyle en C₁-C₆, -C(=O)NR⁶R⁷, -C(=O)R⁸, alkylsulfinyle en C₁-C₃, alkylsulfonyle en C₁-C₃, -S(=O)₂NH₂, alkylsulfonylamino en C₁-C₃, alkylaminosulfonyle en C₁-C₃, cycloalkylaminosulfonyle en C₃-C₆, halogéno-alkyle en C₁-C₆, halogéno-alcoxy en C₁-C₆, hydroxy-alkyle en C₁-C₆, cycloalkyle en C₃-C₁₀ et/ou un radical hétérocyclyle monocyclique de 3 à 8 atomes de cycle, et/ou un radical hétéroaryle monocyclique à 5 ou 6 atomes de cycle, qui peut de son côté éventuellement être substitué une ou plusieurs fois, de manière identique ou différente, avec halogène, alkyle en C₁-C₃ et/ou alcoxy en C₁-C₃, et
R^{1b} et R^{1c} représentent indépendamment l'un de l'autre hydrogène, halogène, hydroxy, cyano, nitro et/ou un radical alkyle en C₁-C₆, alcoxy en C₁-C₆, alcoxy en C₁-C₆-alkyle en C₁-C₆, halogéno-alkyle en C₁-C₆, halogéno-alcoxy en C₁-C₆, cycloalkyle en C₃-C₁₀ et/ou un radical hétérocyclyle monocyclique de 3 à 8 atomes de cycle, et
R² représente un radical alkyle en C₁-C₃ ou trifluorométhyle, ou cycloalkyle en C₃ ou C₄, et
R³ représente cyclopropyle, alkyle en C₁-C₃, alcoxy en C₁-C₃, amino, cyclopropylamino ou alkylamino en C₁-C₃, et
R⁴ et R⁵ représentent indépendamment l'un de l'autre hydrogène, hydroxy, cyano, nitro, amino, aminocarbonyle, fluor, chlore, brome, alkyle en C₁-C₆, alcoxy en C₁-C₆, alkylamino en C₁-C₆, alkylcarbonylamino en C₁-C₆, alkylaminocarbonyle en C₁-C₆ ou alkylaminosulfonyle en C₁-C₆,
ou
alkyle en C₁-C₆, alcoxy en C₁-C₆, alkylamino en C₁-C₆, alkylcarbonylamino en C₁-C₆, alkylaminocarbonyle en C₁-C₆ ou alkylaminosulfonyle en C₁-C₆, qui sont éventuellement substitués une ou plusieurs fois, de manière identique ou différente, avec halogène, amino, hydroxy, carboxy, hydroxy-alkyle en C₁-C₆, alcoxy en C₁-C₆, alcoxy en C₁-C₆-alkyle en C₁-C₆, alkylamino en C₁-C₆, amino-alkyle en C₁-C₆, hétérocyclyle monocyclique de 3 à 8 atomes de cycle et/ou hétéroaryle monocyclique à 5 ou 6 atomes de cycle, les radicaux hétérocyclyle et hétéroaryle monocycliques mentionnés pouvant éventuellement de leur côté être substitués une fois par alkyle en C₁-C₃,
ou
cycloalkyle en C₃-C₁₀, qui peut éventuellement être substitué une ou plusieurs fois, de manière identique ou différente, avec halogène, amino, hydroxy, carboxy, alkyle en C₁-C₆, alcoxy en C₁-C₆, alcoxy en C₁-C₆-alkyle en C₁-C₆, alkylamino en C₁-C₆, amino-alkyle en C₁-C₆, alkylamino en C₁-C₆-alkyle en C₁-C₆, halogéno-alkyle en C₁-C₆, halogéno-alcoxy en C₁-C₆, et/ou un radical hétérocyclyle monocyclique de 3 à 8 atomes de cycle,
ou
hétéroaryle monocyclique à 5 ou 6 atomes de cycle, qui peut éventuellement être substitué une ou plusieurs fois, de manière identique ou différente, avec halogène, amino, hydroxy, cyano, nitro, carboxy, alkyle en C₁-C₆, alcoxy en C₁-C₆, alcoxy en C₁-C₆-alkyle en C₁-C₆, hydroxy-alkyle en C₁-C₆, alkylamino en C₁-C₆, amino-alkyle en C₁-C₆, alkylamino en C₁-C₆-alkyle en C₁-C₆, halogéno-alkyle en C₁-C₆, halogéno-alcoxy en C₁-C₆, cycloalkyle en C₃-C₁₀,
-C(=O)R⁸, -S(=O)₂R⁹, -NR⁶R⁷, et/ou un radical hétérocyclyle monocyclique de 3 à 8 atomes de cycle,
ou
hétérocyclyle monocyclique de 3 à 8 atomes de cycle, qui peut éventuellement être substitué une ou plusieurs fois, de manière identique ou différente, avec halogène, amino, hydroxy, cyano, oxo, carboxy, alkyle en C₁-C₆, alcoxy en C₁-C₆, alcoxy en C₁-C₆-alkyle en C₁-C₆, alkylamino en C₁-C₆, amino-alkyle en C₁-C₆, alkylamino en C₁-C₆-alkyle en C₁-C₆, hydroxy-alkyle en C₁-C₆, halogéno-alkyle en C₁-C₆, halogéno-alcoxy en C₁-C₆, cycloalkyle en C₃-C₁₀, -C(=O)R⁸, -S(=O)₂R⁹, -NR⁶R⁷, et/ou un radical hétérocyclyle monocyclique de 3 à 8 atomes de cycle,
ou
phényle, qui peut éventuellement être substitué une ou plusieurs fois, de manière identique ou différente, avec halogène, amino, hydroxy, cyano, nitro, carboxy, alkyle en C₁-C₆, alcoxy en C₁-C₆, alcoxy en C₁-C₆-alkyle en C₁-C₆, alkylamino en C₁-C₆, amino-alkyle en C₁-C₆, alkylaminocarbonyle en C₁-C₆, alkylaminosulfonyle en C₁-C₆, alkylamino en C₁-C₆-alkyle en C₁-C₆, hydroxy-alkyle en C₁-C₆, halogéno-alkyle en C₁-C₆, halogéno-alcoxy en C₁-C₆, cycloalkyle en C₃-C₁₀ et/ou un radical hétérocyclyle monocyclique de 3 à 8 atomes de cycle, et
R⁶ et R⁷ représentent indépendamment l'un de l'autre hydrogène, alkyle en C₁-C₃, cyclopropyle, di-alkyle en C₁-C₃-amino-alkyle en C₁-C₃ ou fluoropyridyle, et
R⁸ représente hydroxy, alkyle en C₁-C₆, halogéno-alkyle en C₁-C₃, hydroxy-alkyle en C₁-C₃, alcoxy en C₁-C₃-alkyle en C₁-C₃, cycloalkyle en C₃-C₈, phényle, hétérocyclyle monocyclique de 3 à 8 atomes de cycle ou hétéroaryle monocyclique à 5 ou 6 atomes de cycle, le phényle, l'hétéroaryle et l'hétérocyclyle pouvant éventuellement être substitués une ou deux fois par halogène, alcoxy en C₁-C₃ ou alkyle en C₁-C₃, et
R⁹ représente hydrogène, alkyle en C₁-C₆ ou alcoxy en C₁-C₄,
ainsi que leur polymorphes, énantiomères, diastéréomères, racémats, tautomères, solvates, sels physiologiquement acceptables et solvates de ces sels,
à condition que
lorsque A représente un cycle phényle et R⁴ et R⁵ représentent indépendamment l'un de l'autre hydrogène, hydroxy, cyano, nitro, amino, aminocarbonyle, fluor, chlore, brome, alkyle en C₁-C₆, alcoxy en C₁-C₆, alkylamino en C₁-C₆, alkylcarbonylamino en C₁-C₆, alkylaminocarbonyle en C₁-C₆ ou alkylaminosulfonyle en C₁-C₆, ou alkyle en C₁-C₆, alcoxy en C₁-C₆, alkylamino en C₁-C₆, alkylcarbonylamino en C₁-C₆, alkylaminocarbonyle en C₁-C₆ ou alkylaminosulfonyle en C₁-C₆, qui peuvent éventuellement être substitués une ou plusieurs fois, de manière identique ou différente, avec halogène, amino, hydroxy, carboxy, hydroxy-alkyle en C₁-C₆, alcoxy en C₁-C₆, alcoxy en C₁-C₆-alkyle en C₁-C₆, alkylamino en C₁-C₆, amino-alkyle en C₁-C₆, hétérocyclyle monocyclique de 3 à 8 atomes de cycle et/ou hétéroaryle monocyclique à 5 ou 6 atomes de cycle, les radicaux hétérocyclyle et hétéroaryle mentionnés pouvant de leur côté éventuellement être substitués une fois par alkyle en C₁-C₃,
R^{1a} ne représente pas hydrogène, halogène, cyano, carboxy, amino ou aminosulfonyle, ou un radical alcoxy en C₁-C₆, alcoxy en C₁-C₃-alkyle en C₁-C₃, alcoxy en C₁-C₃-alcoxy en C₂-C₃, alkylamino en C₁-C₆, alkylcarbonylamino en C₁-C₆, alkylamino en C₁-C₆-alkyle en C₁-C₆, *N*-(hétérocyclyl)-alkyle en C₁-C₆, *N*-(hétérocyclyl)-alcoxy en C₁-C₆, hydroxy-alkyle en C₁-C₆, hydroxy-alcoxy en C₁-C₆, halogéno alkyle en C₁-C₆, halogéno-alcoxy en C₁-C₆, alkylcarbonyle en C₁-C₆- ou alcoxycarbonyle en C₁-C₆, et
la condition ne comprenant pas les composés de formule générale (I) dans laquelle A représente phényle et R⁴ représente hydrogène, fluor, chlore ou brome, et R⁵ représente alcoxy en C₁-C₆, qui est substitué une ou plusieurs fois, de manière identique ou différente, avec halogène, et
R^{1a} représente halogène,
ni les composés de formule générale (I) dans laquelle A représente phényle et R⁴ représente hydrogène, et R⁵ représente alcoxy en C₁-C₆, qui est substitué une ou plusieurs fois, de manière identique ou différente, avec un radical hétérocyclyle monocyclique de 3 à 8 atomes de cycle et/ou un radical hétéroaryle monocyclique à 5 ou 6 atomes de cycle, les radicaux hétérocyclyle et hétéroaryle monocycliques mentionnés pouvant de leur côté éventuellement être substitués une fois par alkyle en C₁-C₃, et R^{1a} représentant halogène.

2. Composés de formule générale (I) selon la revendication 1, dans lesquels
X représente un atome d'oxygène, et
A représente un cycle phényle ou pyridyle, et
R^{1a} représente hydrogène, halogène, cyano, carboxy, amino ou aminosulfonyle, ou un radical alcoxy en C₁-C₆, alcoxy en C₁-C₃-alkyle en C₁-C₃, alcoxy en C₁-C₃-alcoxy en C₂-C₃, alkylamino en C₁-C₆, alkylcarbonylamino en C₁-C₆, alkylamino en C₁-C₆-alkyle en C₁-C₆, *N*-(hétérocyclyl)-alkyle en C₁-C₆, N-(hétérocyclyl)-alcoxy en C₁-C₆, hydroxy-alkyle en C₁-C₆, hydroxy-alcoxy en C₁-C₆, halogéno-alkyle en C₁-C₆, halogéno-alcoxy en C₁-C₆, alkylcarbonyle en C₁-C₆- ou alcoxycarbonyle en C₁-C₆, et
ou
un radical hétérocyclyle monocyclique de 4 à 7 atomes de cycle, qui peut éventuellement être substitué une ou plusieurs fois, de manière identique ou différente, avec halogène, cyano, nitro, hydroxy, amino, oxo, carboxy, alkyle en C₁-C₆, alcoxy en C₁-C₆, alcoxy en C₁-C₆-alkyle en C₁-C₆, hydroxy-alkyle en C₁-C₆, alkylamino en C₁-C₆, amino-alkyle en C₁-C₆, alkylamino en C₁-C₆- alkyle en C₁-C₆, halogéno-alkyle en C₁-C₆, halogéno-alcoxy en C₁-C₆, cycloalkyle en C₃-C₁₀, phényle, halophényle, phényl-alkyle en C₁-C₆, pyridinyle, -NR⁶C(=O)R⁹, -C(=O)-NR⁶R⁷, -C(=O)-R⁸, -S(=O)₂-NR⁶R⁷, -S(=O)-R⁹, - S(=O)₂-R⁹, -NH-S(=O)₂-R⁹, et/ou avec un radical hétérocyclyle monocyclique de 4 à 7 atomes de cycle,
ou
un radical hétéroaryle monocyclique à 5 ou 6 atomes de cycle, qui peut éventuellement être substitué une ou plusieurs fois, de manière identique ou différente, avec halogène, hydroxy, amino, cyano, nitro, carboxy, alkyle en C₁-C₆, alcoxy en C₁-C₆, alcoxy en C₁-C₆-alkyle en C₁-C₆, alkylamino en C₁-C₆, amino-alkyle en C₁-C₆, alkylamino en C₁-C₆-alkyle en C₁-C₆, halogéno-alkyle en C₁-C₆, halogéno-alcoxy en C₁-C₆, cycloalkyle en C₃-C₁₀, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, et/ou avec un radical hétérocyclyle monocyclique de 4 à 7 atomes de cycle, et/ou avec un radical hétéroaryle monocyclique à 5 ou 6 atomes de cycle, et/ou avec un radical phényle, qui peut de son côté éventuellement être substitué une ou plusieurs fois, de manière identique ou différente, avec halogène, alkyle en C₁-C₃ et/ou alcoxy en C₁-C₃,
ou
un radical phényle, qui peut éventuellement être substitué une ou plusieurs fois, de manière identique ou différente, avec halogène, amino, hydroxy, cyano, nitro, carboxy, alkyle en C₁-C₆, alcoxy en C₁-C₆, alcoxy en C₁-C₆-alkyle en C₁-C₆, alkylamino en C₁-C₆, amino-alkyle en C₁-C₆, alkylamino en C₁-C₆-alkyle en C₁-C₆, -C(=O)NR⁶R⁷, -C(=O)R⁸, alkylsulfinyle en C₁-C₃, alkylsulfonyle en C₁-C₃, -S(=O)₂NH₂, alkylsulfonylamino en C₁-C₃, alkylaminosulfonyle en C₁-C₃, cycloalkylaminosulfonyle en C₃-C₆, halogéno-alkyle en C₁-C₆, halogéno-alcoxy en C₁-C₆, hydroxy-alkyle en C₁-C₆, cycloalkyle en C₃-C₁₀ et/ou un radical hétérocyclyle monocyclique de 4 à 7 atomes de cycle, et/ou un radical hétéroaryle monocyclique à 5 ou 6 atomes de cycle, qui peut de son côté éventuellement être substitué une ou plusieurs fois, de manière identique ou différente, avec halogène, alkyle en C₁-C₃ et/ou alcoxy en C₁-C₃, et
R^{1b} et R^{1c} représentent indépendamment l'un de l'autre hydrogène, halogène, hydroxy, cyano, nitro ou un radical alkyle en C₁-C₆, alcoxy en C₁-C₆, alcoxy en C₁-C₆-alkyle en C₁-C₆, halogéno-alkyle en C₁-C₆, halogéno-alcoxy en C₁-C₆, cycloalkyle en C₃-C₁₀ et/ou un radical hétérocyclyle monocyclique de 4 à 7 atomes de cycle, et
R² représente méthyle, éthyle ou isopropyle, et
R³ représente cyclopropyle, alkyle en C₁-C₃, alcoxy en C₁-C₃, amino, cyclopropylamino ou alkylamino en C₁-C₃, et
R⁴ et R⁵ représentent indépendamment l'un de l'autre hydrogène, hydroxy, cyano, nitro, amino, aminocarbonyle, fluor, chlore, brome, alkyle en C₁-C₆, alcoxy en C₁-C₆, alkylamino en C₁-C₆, alkylcarbonylamino en C₁-C₆, alkylaminocarbonyle en C₁-C₆ ou alkylaminosulfonyle en C₁-C₆,
ou
alkyle en C₁-C₆, alcoxy en C₁-C₆, alkylamino en C₁-C₆, alkylcarbonylamino en C₁-C₆, alkylaminocarbonyle en C₁-C₆ ou alkylaminosulfonyle en C₁-C₆, qui sont éventuellement substitués une ou plusieurs fois, de manière identique ou différente, avec halogène, amino, hydroxy, carboxy, hydroxy-alkyle en C₁-C₆, alcoxy en C₁-C₆, alcoxy en C₁-C₆-alkyle en C₁-C₆, alkylamino en C₁-C₆, amino-alkyle en C₁-C₆, hétérocyclyle monocyclique de 4 à 7 atomes de cycle et/ou hétéroaryle monocyclique à 5 ou 6 atomes de cycle, les radicaux hétérocyclyle et hétéroaryle monocycliques mentionnés pouvant éventuellement de leur côté être substitués une fois par alkyle en C₁-C₃,
ou
un radical cycloalkyle en C₃-C₁₀, qui peut éventuellement être substitué une ou plusieurs fois, de manière identique ou différente, avec halogène, amino, hydroxy, carboxy, alkyle en C₁-C₆, alcoxy en C₁-C₆, alcoxy en C₁-C₆-alkyle en C₁-C₆, alkylamino en C₁-C₆, amino-alkyle en C₁-C₆, alkylamino en C₁-C₆-alkyle en C₁-C₆, halogéno-alkyle en C₁-C₆, halogéno-alcoxy en C₁-C₆, et/ou un radical hétérocyclyle monocyclique de 4 à 7 atomes de cycle,
ou
hétéroaryle monocyclique à 5 ou 6 atomes de cycle, qui peut éventuellement être substitué une ou plusieurs fois, de manière identique ou différente, avec halogène, amino, hydroxy, cyano, nitro, carboxy, alkyle en C₁-C₆, alcoxy en C₁-C₆, alcoxy en C₁-C₆-alkyle en C₁-C₆, hydroxy-alkyle en C₁-C₆, alkylamino en C₁-C₆, amino-alkyle en C₁-C₆, alkylamino en C₁-C₆-alkyle en C₁-C₆, halogéno-alkyle en C₁-C₆, halogéno-alcoxy en C₁-C₆, cycloalkyle en C₃-C₁₀, -C(=O)R⁸, -S(=O)₂R⁹, -NR⁶R⁷, et/ou un radical hétérocyclyle monocyclique de 4 à 7 atomes de cycle,
ou
un radical hétérocyclyle monocyclique de 4 à 7 atomes de cycle, qui peut éventuellement être substitué une ou plusieurs fois, de manière identique ou différente, avec halogène, amino, hydroxy, cyano, oxo, carboxy, alkyle en C₁-C₆, alcoxy en C₁-C₆, alcoxy en C₁-C₆-alkyle en C₁-C₆, alkylamino en C₁-C₆, amino-alkyle en C₁-C₆, alkylamino en C₁-C₆-alkyle en C₁-C₆, hydroxy-alkyle en C₁-C₆, halogéno-alkyle en C₁-C₆, halogéno-alcoxy en C₁-C₆, cycloalkyle en C₃-C₁₀, -C(=O)R⁸, -S(=O)₂R⁹, -NR⁶R⁷ et/ou un radical hétérocyclyle monocyclique de 4 à 7 atomes de cycle,
ou
un radical phényle, qui peut éventuellement être substitué une ou plusieurs fois, de manière identique ou différente, avec halogène, amino, hydroxy, cyano, nitro, carboxy, alkyle en C₁-C₆, alcoxy en C₁-C₆, alcoxy en C₁-C₆-alkyle en C₁-C₆, alkylamino en C₁-C₆, amino-alkyle en C₁-C₆, alkylaminocarbonyle en C₁-C₆, alkylaminosulfonyle en C₁-C₆, alkylamino en C₁-C₆-alkyle en C₁-C₆, hydroxy-alkyle en C₁-C₆, halogéno-alkyle en C₁-C₆, halogéno-alcoxy en C₁-C₆, cycloalkyle en C₃-C₁₀ et/ou un radical hétérocyclyle monocyclique de 4 à 7 atomes de cycle, et
R⁶ et R⁷ représentent indépendamment l'un de l'autre hydrogène, alkyle en C₁-C₃, cyclopropyle, di-alkyle en C₁-C₃-amino-alkyle en C₁-C₃ ou fluoropyridyle, et
R⁸ représente hydroxy, alkyle en C₁-C₆, halogéno-alkyle en C₁-C₃, hydroxy-alkyle en C₁-C₃, alcoxy en C₁-C₃-alkyle en C₁-C₃, cycloalkyle en C₃-C₈, phényle, hétérocyclyle monocyclique à 5 ou 6 atomes de cycle, et
R⁹ représente hydrogène, alkyle en C₁-C₆ ou alcoxy en C₁-C₄,
ainsi que leur polymorphes, énantiomères, diastéréomères, racémats, tautomères, solvates, sels physiologiquement acceptables et solvates de ces sels,
à condition que lorsque A représente un cycle phényle et R⁴ et R⁵ représentent indépendamment l'un de l'autre hydrogène, hydroxy, cyano, nitro, amino, aminocarbonyle, fluor, chlore, brome, alkyle en C₁-C₆, alcoxy en C₁-C₆, alkylamino en C₁-C₆, alkylcarbonylamino en C₁-C₆, alkylaminocarbonyle en C₁-C₆ ou alkylaminosulfonyle en C₁-C₆, ou alkyle en C₁-C₆, alcoxy en C₁-C₆, alkylamino en C₁-C₆, alkylcarbonylamino en C₁-C₆, alkylaminocarbonyle en C₁-C₆ ou alkylaminosulfonyle en C₁-C₆, qui peuvent éventuellement être substitués une ou plusieurs fois, de manière identique ou différente, avec halogène, amino, hydroxy, carboxy, hydroxy-alkyle en C₁-C₆, alcoxy en C₁-C₆, alcoxy en C₁-C₆-alkyle en C₁-C₆, alkylamino en C₁-C₆, amino-alkyle en C₁-C₆, hétérocyclyle monocyclique de 4 à 7 atomes de cycle et/ou hétéroaryle monocyclique à 5 ou 6 atomes de cycle, les radicaux hétérocyclyle et hétéroaryle mentionnés pouvant de leur côté éventuellement être substitués une fois par alkyle en C₁-C₃,
R^{1a} ne représente pas hydrogène, halogène, cyano, carboxy, amino ou aminosulfonyle, ou un radical alcoxy en C₁-C₆, alcoxy en C₁-C₃-alkyle en C₁-C₃, alcoxy en C₁-C₃-alcoxy en C₂-C₃, alkylamino en C₁-C₆, alkylcarbonylamino en C₁-C₆, alkylamino en C₁-C₆-alkyle en C₁-C₆, *N*-(hétérocyclyl)-alkyle en C₁-C₆, N-(hétérocyclyl)-alcoxy en C₁-C₆, hydroxy-alkyle en C₁-C₆, hydroxy-alcoxy en C₁-C₆, halogéno alkyle en C₁-C₆, halogéno-alcoxy en C₁-C₆, alkylcarbonyle en C₁-C₆- ou alcoxycarbonyle en C₁-C₆, et
la condition ne comprenant pas les composés de formule générale (I) dans laquelle
A représente phényle et R⁴ représente hydrogène, fluor, chlore ou brome, et R⁵ représente alcoxy en C₁-C₆, qui est substitué une ou plusieurs fois, de manière identique ou différente, avec halogène, et
R^{1a} représente halogène,
ni les composés de formule générale (I) dans laquelle A représente phényle et R⁴ représente hydrogène, et R⁵ représente alcoxy en C₁-C₆, qui est substitué une ou plusieurs fois, de manière identique ou différente, avec un radical hétérocyclyle monocyclique de 4 à 7 atomes de cycle et/ou un radical hétéroaryle monocyclique à 5 ou 6 atomes de cycle, les radicaux hétérocyclyle et hétéroaryle monocycliques mentionnés pouvant de leur côté éventuellement être substitués une fois par alkyle en C₁-C₃, et R^{1a} représentant halogène.

3. Composés de formule générale (I) selon les revendications 1 et 2, dans lesquels
X représente un atome d'oxygène, et
A représente un cycle phényle ou pyridyle, et
R^{1a} représente hydrogène, halogène, cyano, carboxy, amino ou aminosulfonyle, ou un radical alcoxy en C₁-C₆, alcoxy en C₁-C₃-alkyle en C₁-C₃, alcoxy en C₁-C₃-alcoxy en C₂-C₃, alkylamino en C₁-C₃, alkylcarbonylamino en C₁-C₃, alkylamino en C₁-C₃-alkyle en C₁-C₃, hydroxy-alkyle en C₁-C₃, fluoro-alkyle en C₁-C₃, fluoro-alcoxy en C₁-C₃, alkylcarbonyle en C₁-C₃ ou alcoxycarbonyle en C₁-C₄,
ou
un radical hétérocyclyle monocyclique de 4 à 7 atomes de cycle, qui peut éventuellement être substitué une ou plusieurs fois, de manière identique ou différente, avec halogène, cyano, nitro, hydroxy, amino, oxo, carboxy, alkyle en C₁-C₃, alcoxy en C₁-C₃, alcoxy en C₁-C₂-alkyle en C₁-C₂, hydroxy-alkyle en C₁-C₃, alkylamino en C₁-C₃, amino-alkyle en C₁-C₃, fluoro-alkyle en C₁-C₃, fluoro-alcoxy en C₁-C₃, cycloalkyle en C₃-C₆, phényle, halophényle, phényl-alkyle en C₁-C₃, pyridinyle, -NR⁶C(=O)-R⁹, -C(=O)-NR⁶R⁷, - C(=O)-R⁸, -S(=O)₂-NR⁶R⁷, -S(=O)-R⁹, -S(=O)₂-R⁹, -NH-S(=O)₂-R⁹, et/ou un radical hétérocyclyle monocyclique de 4 à 7 atomes de cycle,
ou
un radical hétéroaryle monocyclique à 5 ou 6 atomes de cycle, qui peut éventuellement être substitué une ou plusieurs fois, de manière identique ou différente, avec halogène, hydroxy, amino, cyano, nitro, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, fluoro-alkyle en C₁-C₃, fluoro-alcoxy C₁-C₃, cycloalkyle en C₃-C₆, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, et/ou un radical hétérocyclyle monocyclique de 4 à 7 atomes de cycle, et/ou un radical hétéroaryle monocyclique à 5 ou 6 atomes de cycle et/ou un radical phényle, qui peut de son côté éventuellement être substitué une ou plusieurs fois, de manière identique ou différente, avec fluor, chlore, brome, méthyle et/ou méthoxy,
ou
un radical phényle, qui peut éventuellement être substitué une ou plusieurs fois, de manière identique ou différente, avec halogène, amino, hydroxy, cyano, nitro, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, -C(=O)NR⁶R⁷, C(=O)R⁸, alkylsulfinyle en C₁-C₃, alkylsulfonyle en C₁-C₃, -S(=O)₂NH₂, alkylsulfonylamino en C₁-C₃, alkylaminosulfonyle en C₁-C₃, cycloalkylaminosulfonyle en C₃-C₆, fluoro-alkyle en C₁-C₃, fluoro-alcoxy en C₁-C₃, hydroxy-alkyle en C₁-C₃, cycloalkyle en C₃-C₆, et/ou un radical hétérocyclyle monocyclique de 4 à 7 atomes de cycle, et/ou un radical hétéroaryle monocyclique à 5 ou 6 atomes de cycle, qui peut de son côté éventuellement être substitué une ou plusieurs fois, de manière identique ou différente, avec fluor, chlore, brome, méthyle et/ou méthoxy, et
R^{1b} représente hydrogène, halogène, hydroxy, cyano, nitro ou un radical alkyle en C₁-C₃, alcoxy en C₁-C₃, fluoro-alkyle en C₁-C₃ ou fluoro-alcoxy en C₁-C₃, et
R^{1c} représente hydrogène, fluor, chlore, brome ou cyano, et
R² représente méthyle, éthyle ou isopropyle, et
R³ représente cyclopropyle, alkyle en C₁-C₃, alcoxy en C₁-C₃, amino, cyclopropylamino ou alkylamino en C₁-C₃, et
R⁴ et R⁵ représentent indépendamment l'un de l'autre hydrogène, hydroxy, cyano, nitro, amino, aminocarbonyle, fluor, chlore, brome, alkyle en C₁-C₆, alcoxy en C₁-C₆, alkylamino en C₁-C₆, alkylcarbonylamino en C₁-C₆, alkylaminocarbonyle en C₁-C₆ ou alkylaminosulfonyle en C₁-C₆,
ou
alkyle en C₁-C₃, alcoxy en C₁-C₃, alkylamino en C₁-C₃, qui peuvent être substitués une ou plusieurs fois, de manière identique ou différente, avec halogène, amino, hydroxy, carboxy, hydroxy-alkyle en C₁-C₃, alcoxy en C₁-C₃, alkylamino en C₁-C₃, amino-alkyle en C₁-C₃, hétérocyclyle monocyclique de 4 à 7 atomes de cycle et/ou hétéroaryle monocyclique à 5 ou 6 atomes de cycle, les radicaux hétérocyclyle et hétéroaryle monocycliques mentionnés pouvant de leur côté éventuellement être substitués par alkyle en C₁-C₃,
ou
un radical cycloalkyle en C₃-C₇, qui peut éventuellement être substitué une ou plusieurs fois, de manière identique ou différente, avec halogène, amino, hydroxy, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, alkylamino en C₁-C₃-alkyle en C₁-C₃, fluoro-alkyle en C₁-C₃, fluoro-alcoxy en C₁-C₃, et/ou un radical hétérocyclyle monocyclique de 4 à 7 atomes de cycle,
ou
hétéroaryle monocyclique à 5 ou 6 atomes de cycle, qui peut éventuellement être substitué une ou plusieurs fois, de manière identique ou différente, avec halogène, amino, hydroxy, cyano, nitro, carboxy, alkyle en C₁-C₃, alcoxy en C₁-C₃, alcoxy en C₁-C₂-alkyle en C₁-C₂, hydroxy-alkyle en C₁-C₃, alkylamino en C₁-C₃, amino-alkyle en C₁-C₃, fluoro-alkyle en C₁-C₃, fluoro-alcoxy en C₁-C₃, cycloalkyle en C₃-C₆, -C(=O)R⁸, -S(=O)₂R⁹, -NR⁶R⁷, et/ou un radical hétérocyclyle monocyclique de 4 à 7 atomes de cycle,
ou
un radical hétérocyclyle monocyclique de 4 à 7 atomes de cycle, qui peut éventuellement être substitué une ou plusieurs fois, de manière identique ou différente, avec halogène, amino, hydroxy, cyano, oxo, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, hydroxy-alkyle en C₁-C₃, fluoro-alkyle en C₁-C₃, fluoro-alcoxy en C₁-C₃, cycloalkyle en C₃-C₆,-C(=O)R⁸, -S(=O)₂R⁹, -NR⁶R⁷, et/ou un radical hétérocyclyle monocyclique de 4 à 7 atomes de cycle,
ou
un radical phényle, qui peut éventuellement être substitué une ou plusieurs fois, de manière identique ou différente, avec halogène, amino, hydroxy, cyano, nitro, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, alkylaminocarbonyle en C₁-C₃, alkylaminosulfonyle en C₁-C₃, hydroxy-alkyle en C₁-C₃, fluoro-alkyle en C₁-C₃, fluoro-alcoxy en C₁-C₃, cycloalkyle en C₃-C₆ et/ou un radical hétérocyclyle monocyclique de 4 à 7 atomes de cycle, et
R⁶ et R⁷ représentent indépendamment l'un de l'autre hydrogène, alkyle en C₁-C₃, cyclopropyle, di-alkyle en C₁-C₃-amino-alkyle en C₁-C₃ ou fluoropyridyle, et
R⁸ représente hydroxy, alkyle en C₁-C₆, hydroxy-alkyle en C₁-C₃, fluoro-alkyle en C₁-C₃, alcoxy en C₁-C₃-alkyle en C₁-C₃, cycloalkyle en C₃-C₈, phényle ou hétérocyclyle monocyclique à 5 ou 6 atomes de cycle, et
R⁹ représente hydrogène, alkyle en C₁-C₆ ou alcoxy en C₁-C₄,
ainsi que leur polymorphes, énantiomères, diastéréomères, racémats, tautomères, solvates, sels physiologiquement acceptables et solvates de ces sels,
à condition que
lorsque A représente un cycle phényle et R⁴ et R⁵ représentent indépendamment l'un de l'autre hydrogène, hydroxy, cyano, nitro, amino, aminocarbonyle, fluor, chlore, brome, alkyle en C₁-C₆, alcoxy en C₁-C₆, alkylamino en C₁-C₆, alkylcarbonylamino en C₁-C₆, alkylaminocarbonyle en C₁-C₆ ou alkylaminosulfonyle en C₁-C₆, ou alkyle en C₁-C₃, alcoxy en C₁-C₃, alkylamino en C₁-C₃, qui peuvent éventuellement être substitués une ou plusieurs fois, de manière identique ou différente, avec halogène, amino, hydroxy, carboxy, hydroxy-alkyle en C₁-C₃, alcoxy en C₁-C₃, alkylamino en C₁-C₃, amino-alkyle en C₁-C₃, hétérocyclyl monocyclique de 4 à 7 atomes de cycle, et/ou hétéroaryle monocyclique à 5 ou 6 atomes de cycle, les radicaux hétérocyclyle et hétéroaryle monocycliques mentionnés pouvant de leur côté éventuellement être substitués une fois par alkyle en C₁-C₃,
R^{1a} ne représente pas hydrogène, halogène, cyano, carboxy, amino ou aminosulfonyle, ou un radical alcoxy en C₁-C₆, alcoxy en C₁-C₃-alkyle en C₁-C₃, alcoxy en C₁-C₃-alcoxy en C₂-C₃, alkylamino en C₁-C₃, alkylcarbonylamino en C₁-C₃, alkylamino en C₁-C₃-alkyle en C₁-C₃, hydroxy-alkyle en C₁-C₃, fluoro-alkyle en C₁-C₃, fluoro-alcoxy en C₁-C₃, alkylcarbonyle en C₁-C₃- ou alcoxycarbonyle en C₁-C₄, et
la condition ne comprenant pas les composés de formule générale (I) dans laquelle
A représente phényle et R⁴ représente hydrogène, fluor, chlore ou brome, et R⁵ représente alcoxy en C₁-C₃, qui est substitué une ou plusieurs fois, de manière identique ou différente, avec halogène, et
R^{1a} représente halogène,
ni les composés de formule générale (I) dans laquelle
A représente phényle et R⁴ représente hydrogène, et
R⁵ représente alcoxy en C₁-C₃, qui est substitué une ou plusieurs fois, de manière identique ou différente, avec un radical hétérocyclyle monocyclique de 4 à 7 atomes de cycle et/ou un radical hétéroaryle monocyclique à 5 ou 6 atomes de cycle, les radicaux hétérocyclyle et hétéroaryle monocycliques mentionnés pouvant de leur côté éventuellement être substitués une fois par alkyle en C₁-C₃, et R^{1a} représentant halogène.

4. Composés de formule générale (I) selon les revendications 1 à 3, dans lesquels
X représente un atome d'oxygène, et
A représente un cycle phényle ou pyridyle, et
R^{1a} représente un radical hétérocyclyle monocyclique de 4 à 7 atomes de cycle, qui peut éventuellement être substitué une ou plusieurs fois, de manière identique ou différente, avec halogène, cyano, nitro, hydroxy, amino, oxo, carboxy, alkyle en C₁-C₃, alcoxy en C₁-C₃, alcoxy en C₁-C₂-alkyle en C₁-C₂, hydroxy-alkyle en C₁-C₃, alkylamino en C₁-C₃, amino-alkyle en C₁-C₃, fluoro-alkyle en C₁-C₃, fluoro-alcoxy en C₁-C₃, cycloalkyle en C₃-C₆, phényle, halophényle, phényl-alkyle en C₁-C₃, pyridinyle, -NR⁶C(=O)-R⁹,-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, et/ou un radical hétérocyclyle monocyclique de 4 à 7 atomes de cycle,
ou
un radical hétéroaryle monocyclique à 5 ou 6 atomes de cycle, qui peut éventuellement être substitué une ou plusieurs fois, de manière identique ou différente, avec halogène, hydroxy, amino, cyano, nitro, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, fluoro-alkyle en C₁-C₃, fluoro-alcoxy C₁-C₃, cycloalkyle en C₃-c₆, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, et/ou un radical hétérocyclyle monocyclique de 4 à 7 atomes de cycle, et/ou un radical hétéroaryle monocyclique à 5 ou 6 atomes de cycle et/ou un radical phényle, qui peut de son côté éventuellement être substitué une ou plusieurs fois, de manière identique ou différente, avec fluor, chlore, brome, méthyle et/ou méthoxy,
ou
un radical phényle, qui peut éventuellement être substitué une ou plusieurs fois, de manière identique ou différente, avec halogène, amino, hydroxy, cyano, nitro, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, -C(=O)NR⁶R⁷, C(=O)R⁸, alkylsulfinyle en C₁-C₃, alkylsulfonyle en C₁-C₃, -S(=O)₂NH₂, alkylsulfonylamino en C₁-C₃, alkylaminosulfonyle en C₁-C₃, cycloalkylaminosulfonyle en C₃-C₆, fluoro-alkyle en C₁-C₃, fluoro-alcoxy en C₁-C₃, hydroxy-alkyle en C₁-C₃, cycloalkyle en C₃-C₆, et/ou un radical hétérocyclyle monocyclique de 4 à 7 atomes de cycle, et/ou un radical hétéroaryle monocyclique à 5 ou 6 atomes de cycle, qui peut de son côté éventuellement être substitué une ou plusieurs fois, de manière identique ou différente, avec fluor, chlore, brome, méthyle ou méthoxy, et
R^{1b} représente hydrogène, halogène, hydroxy, cyano, nitro ou un radical alkyle en C₁-C₃, alcoxy en C₁-C₃, fluoro-alkyle en C₁-C₃ ou fluoro-alcoxy en C₁-C₃, et
R^{1c} représente hydrogène, fluor, chlore, brome ou cyano, et
R² représente méthyle, éthyle ou isopropyle, et
R³ représente cyclopropyle, alkyle en C₁-C₃, alcoxy en C₁-C₃, amino, cyclopropylamino ou alkylamino en C₁-C₃, et
R⁴ et R⁵ représentent indépendamment l'un de l'autre hydrogène, hydroxy, cyano, nitro, amino, aminocarbonyle, fluor, chlore, brome, alkyle en C₁-C₆, alcoxy en C₁-C₆, alkylamino en C₁-C₆, alkylcarbonylamino en C₁-C₆, alkylaminocarbonyle en C₁-C₆ ou alkylaminosulfonyle en C₁-C₆,
ou
alkyle en C₁-C₃, alcoxy en C₁-C₃, alkylamino en C₁-C₃, qui peuvent être substitués une ou plusieurs fois, de manière identique ou différente, avec halogène, amino, hydroxy, carboxy, hydroxy-alkyle en C₁-C₃, alcoxy en C₁-C₃, alkylamino en C₁-C₃, amino-alkyle en C₁-C₃, hétérocyclyle monocyclique de 4 à 7 atomes de cycle et/ou hétéroaryle monocyclique à 5 ou 6 atomes de cycle, les radicaux hétérocyclyle et hétéroaryle monocycliques mentionnés pouvant de leur côté éventuellement être substitués par alkyle en C₁-C₃,
ou
un radical cycloalkyle en C₃-C₇, qui peut éventuellement être substitué une ou plusieurs fois, de manière identique ou différente, avec halogène, amino, hydroxy, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, alkylamino en C₁-C₃-alkyle en C₁-C₃, fluoro-alkyle en C₁-C₃, fluoro-alcoxy en C₁-C₃, et/ou un radical hétérocyclyle monocyclique de 4 à 7 atomes de cycle,
ou
hétéroaryle monocyclique à 5 ou 6 atomes de cycle, qui peut éventuellement être substitué une ou plusieurs fois, de manière identique ou différente, avec halogène, amino, hydroxy, cyano, nitro, carboxy, alkyle en C₁-C₃, alcoxy en C₁-C₃, alcoxy en C₁-C₂-alkyle en C₁-C₂, hydroxy-alkyle en C₁-C₃, alkylamino en C₁-C₃, amino-alkyle en C₁-C₃, fluoro-alkyle en C₁-C₃, fluoro-alcoxy en C₁-C₃, cycloalkyle en C₃-C₆, -C(=O)R⁸, -S(=O)₂R⁹, -NR⁶R⁷, et/ou un radical hétérocyclyle monocyclique de 4 à 7 atomes de cycle,
ou
un radical hétérocyclyle monocyclique de 4 à 7 atomes de cycle, qui peut éventuellement être substitué une ou plusieurs fois, de manière identique ou différente, avec halogène, amino, hydroxy, cyano, oxo, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, hydroxy-alkyle en C₁-C₃, fluoro-alkyle en C₁-C₃, fluoro-alcoxy en C₁-C₃, cycloalkyle en C₃-C₆,-C(=O)R⁸, -S(=O)₂R⁹, -NR⁶R⁷, et/ou un radical hétérocyclyle monocyclique de 4 à 7 atomes de cycle,
ou
un radical phényle, qui peut éventuellement être substitué une ou plusieurs fois, de manière identique ou différente, avec halogène, amino, hydroxy, cyano, nitro, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, alkylaminocarbonyle en C₁-C₃, alkylaminosulfonyle en C₁-C₃, hydroxy-alkyle en C₁-C₃, fluoro-alkyle en C₁-C₃, fluoro-alcoxy en C₁-C₃, cycloalkyle en C₃-C₆ et/ou un radical hétérocyclyle monocyclique de 4 à 7 atomes de cycle, et
R⁶ et R⁷ représentent indépendamment l'un de l'autre hydrogène, alkyle en C₁-C₃, cyclopropyle, di-alkyle en C₁-C₃-amino-alkyle en C₁-C₃ ou fluoropyridyle, et
R⁸ représente hydroxy, alkyle en C₁-C₆, hydroxy-alkyle en C₁-C₃, fluoro-alkyle en C₁-C₃, alcoxy en C₁-C₃-alkyle en C₁-C₃, cycloalkyle en C₃-C₈, phényle ou hétérocyclyle monocyclique à 5 ou 6 atomes de cycle, et
R⁹ représente hydrogène, alkyle en C₁-C₆ ou alcoxy en C₁-C₄,
ainsi que leur polymorphes, énantiomères, diastéréomères, racémats, tautomères, solvates, sels physiologiquement acceptables et solvates de ces sels.

5. Composés de formule générale (I) selon les revendications 1 à 3, dans lesquels
X représente un atome d'oxygène, et
A représente un cycle phényle ou pyridyle, et
R^{1a} représente hydrogène, halogène, cyano, carboxy, amino ou aminosulfonyle, ou un radical alcoxy en C₁-C₆, alcoxy en C₁-C₃-alkyle en C₁-C₃, alcoxy en C₁-C₃-alcoxy en C₂-C₃, alkylamino en C₁-C₃, alkylcarbonylamino en C₁-C₃, alkylamino en C₁-C₃-alkyle en C₁-C₃, hydroxy-alkyle en C₁-C₃, fluoro-alkyle en C₁-C₃, fluoro-alcoxy en C₁-C₃, alkylcarbonyle en C₁-C₃ ou alcoxycarbonyle en C₁-C₄,
ou
un radical hétérocyclyle monocyclique de 4 à 7 atomes de cycle, qui peut éventuellement être substitué une ou plusieurs fois, de manière identique ou différente, avec halogène, cyano, nitro, hydroxy, amino, oxo, carboxy, alkyle en C₁-C₃, alcoxy en C₁-C₃, alcoxy en C₁-C₂-alkyle en C₁-C₂, hydroxy-alkyle en C₁-C₃, alkylamino en C₁-C₃, amino-alkyle en C₁-C₃, fluoro-alkyle en C₁-C₃, fluoro-alcoxy en C₁-C₃, cycloalkyle en C₃-C₆, phényle, halophényle, phényl-alkyle en C₁-C₃, pyridinyle, -NR⁶C(=O) -R⁹,-C(=O) -NR⁶R⁷,-C(=O)-R⁸, -S(=O)₂-NR⁶R⁷, -S(=O)-R⁹, -S(=O)₂-R⁹, -NH-S(=O)₂-R⁹, et/ou un radical hétérocyclyle monocyclique de 4 à 7 atomes de cycle,
ou
un radical hétéroaryle monocyclique à 5 ou 6 atomes de cycle, qui peut éventuellement être substitué une ou plusieurs fois, de manière identique ou différente, avec halogène, hydroxy, amino, cyano, nitro, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, fluoro-alkyle en C₁-C₃, fluoro-alcoxy C₁-C₃, cycloalkyle en C₃-C₆, -C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, et/ou un radical hétérocyclyle monocyclique de 4 à 7 atomes de cycle, et/ou un radical hétéroaryle monocyclique à 5 ou 6 atomes de cycle et/ou un radical phényle, qui peut de son côté éventuellement être substitué une ou plusieurs fois, de manière identique ou différente, avec fluor, chlore, brome, méthyle et/ou méthoxy,
ou
un radical phényle, qui peut éventuellement être substitué une ou plusieurs fois, de manière identique ou différente, avec halogène, amino, hydroxy, cyano, nitro, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, -C(=O)NR⁶R⁷, C(=O)R⁸, alkylsulfinyle en C₁-C₃, alkylsulfonyle en C₁-C₃, -S(=O)₂NH₂, alkylsulfonylamino en C₁-C₃, alkylaminosulfonyle en C₁-C₃, cycloalkylaminosulfonyle en C₃-C₆, fluoro-alkyle en C₁-C₃, fluoro-alcoxy en C₁-C₃, hydroxy-alkyle en C₁-C₃, cycloalkyle en C₃-C₆, et/ou un radical hétérocyclyle monocyclique de 4 à 7 atomes de cycle, et/ou un radical hétéroaryle monocyclique à 5 ou 6 atomes de cycle, qui peut de son côté éventuellement être substitué une ou plusieurs fois, de manière identique ou différente, avec fluor, chlore, brome, méthyle et/ou méthoxy, et
R^{1b} représente hydrogène, halogène, hydroxy, cyano, nitro ou un radical alkyle en C₁-C₃, alcoxy en C₁-C₃, fluoro-alkyle en C₁-C₃ ou fluoro-alcoxy en C₁-C₃, et
R^{1c} représente hydrogène, fluor, chlore, brome ou cyano, et
R² représente méthyle, éthyle ou isopropyle, et
R³ représente cyclopropyle, alkyle en C₁-C₃, alcoxy en C₁-C₃, amino, cyclopropylamino ou alkylamino en C₁-C₃, et
R⁴ représente un radical cycloalkyle en C₃-C₇, qui peut éventuellement être substitué une ou plusieurs fois, de manière identique ou différente, avec halogène, amino, hydroxy, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, alkylamino en C₁-C₃-alkyle en C₁-C₃, fluoro-alkyle en C₁-C₃, fluoro-alcoxy en C₁-C₃, et/ou un radical hétérocyclyle monocyclique de 4 à 7 atomes de cycle,
ou
hétéroaryle monocyclique à 5 ou 6 atomes de cycle, qui peut éventuellement être substitué une ou plusieurs fois, de manière identique ou différente, avec halogène, amino, hydroxy, cyano, nitro, carboxy, alkyle en C₁-C₃, alcoxy en C₁-C₃, alcoxy en C₁-C₂-alkyle en C₁-C₂, hydroxy-alkyle en C₁-C₃, alkylamino en C₁-C₃, amino-alkyle en C₁-C₃, fluoro-alkyle en C₁-C₃, fluoro-alcoxy en C₁-C₃, cycloalkyle en C₃-C₆, -C(=O)R⁸, -S(=O)₂R⁹, -NR⁶R⁷, et/ou un radical hétérocyclyle monocyclique de 4 à 7 atomes de cycle,
ou
un radical hétérocyclyle monocyclique de 4 à 7 atomes de cycle, qui peut éventuellement être substitué une ou plusieurs fois, de manière identique ou différente, avec halogène, amino, hydroxy, cyano, oxo, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, hydroxy-alkyle en C₁-C₃, fluoro-alkyle en C₁-C₃, fluoro-alcoxy en C₁-C₃, cycloalkyle en C₃-C₆,-C(=O)R⁸, -S(=O)₂R⁹, -NR⁶R⁷, et/ou un radical hétérocyclyle monocyclique de 4 à 7 atomes de cycle,
ou
un radical phényle, qui peut éventuellement être substitué une ou plusieurs fois, de manière identique ou différente, avec halogène, amino, hydroxy, cyano, nitro, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, alkylaminocarbonyle en C₁-C₃, alkylaminosulfonyle en C₁-C₃, hydroxy-alkyle en C₁-C₃, fluoro-alkyle en C₁-C₃, fluoro-alcoxy en C₁-C₃, cycloalkyle en C₃-C₆ et/ou un radical hétérocyclyle monocyclique de 4 à 7 atomes de cycle, et
R⁵ représente hydrogène, hydroxy, cyano, nitro, amino, aminocarbonyle, fluor, chlore, brome, alkyle en C₁-C₆, alcoxy en C₁-C₆, alkylamino en C₁-C₆, alkylcarbonylamino en C₁-C₆, alkylaminocarbonyle en C₁-C₆ ou alkylaminosulfonyle en C₁-C₆, et
R⁶ et R⁷ représentent indépendamment l'un de l'autre hydrogène, alkyle en C₁-C₃, cyclopropyle, di-alkyle en C₁-C₃-amino-alkyle en C₁-C₃ ou fluoropyridyle, et
R⁸ représente hydroxy, alkyle en C₁-C₆, hydroxy-alkyle en C₁-C₃, fluoro-alkyle en C₁-C₃, alcoxy en C₁-C₃-alkyle en C₁-C₃, cycloalkyle en C₃-C₈, phényle ou hétérocyclyle monocyclique à 5 ou 6 atomes de cycle, et
R⁹ représente hydrogène, alkyle en C₁-C₆ ou alcoxy en C₁-C₄,
ainsi que leur polymorphes, énantiomères, diastéréomères, racémats, tautomères, solvates, sels physiologiquement acceptables et solvates de ces sels.

6. Composés de formule générale (I) selon les revendications 1 à 3, dans lesquels
X représente un atome d'oxygène, et
A représente un cycle phényle ou pyridyle, et
R^{1a} représente hydrogène, halogène, cyano, carboxy, amino ou aminosulfonyle, ou un radical alcoxy en C₁-C₆, alcoxy en C₁-C₃-alkyle en C₁-C₃, alcoxy en C₁-C₃-alcoxy en C₂-C₃, alkylamino en C₁-C₃, alkylcarbonylamino en C₁-C₃, alkylamino en C₁-C₃-alkyle en C₁-C₃, hydroxy-alkyle en C₁-C₃, fluoro-alkyle en C₁-C₃, fluoro-alcoxy en C₁-C₃, alkylcarbonyle en C₁-C₃ ou alcoxycarbonyle en C₁-C₄,
ou
un radical hétérocyclyle monocyclique de 4 à 7 atomes de cycle, qui peut éventuellement être substitué une ou plusieurs fois, de manière identique ou différente, avec halogène, cyano, nitro, hydroxy, amino, oxo, carboxy, alkyle en C₁-C₃, alcoxy en C₁-C₃, alcoxy en C₁-C₂-alkyle en C₁-C₂, hydroxy-alkyle en C₁-C₃, alkylamino en C₁-C₃, amino-alkyle en C₁-C₃, fluoro-alkyle en C₁-C₃, fluoro-alcoxy en C₁-C₃, cycloalkyle en C₃-C₆, phényle, halophényle, phényl-alkyle en C₁-C₃, pyridinyle, -NR⁶C(=O)-R⁹,-C(=O)-NR⁶R⁷,-C(=O)-R⁸, -S(=O)₂-NR⁶R⁷, -S(=O)-R⁹, -S(=O)₂-R⁹, -NH-S(=O)₂-R⁹, et/ou un radical hétérocyclyle monocyclique de 4 à 7 atomes de cycle,
ou
un radical hétéroaryle monocyclique à 5 ou 6 atomes de cycle, qui peut éventuellement être substitué une ou plusieurs fois, de manière identique ou différente, avec halogène, hydroxy, amino, cyano, nitro, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, fluoro-alkyle en C₁-C₃, fluoro-alcoxy C₁-C₃, cycloalkyle en C₃-C₆,
-C(=O)-NR⁶R⁷, -C(=O)-R⁸, -S(=O)₂-NR⁶R⁷, -S(=O)-R⁹, -S(=O)₂-R⁹, -NH-S(=O)₂-R⁹, et/ou un radical hétérocyclyle monocyclique de 4 à 7 atomes de cycle, et/ou un radical hétéroaryle monocyclique à 5 ou 6 atomes de cycle et/ou un radical phényle, qui peut de son côté éventuellement être substitué une ou plusieurs fois, de manière identique ou différente, avec fluor, chlore, brome, méthyle et/ou méthoxy,
ou
un radical phényle, qui peut éventuellement être substitué une ou plusieurs fois, de manière identique ou différente, avec halogène, amino, hydroxy, cyano, nitro, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, -C(=O)NR⁶R⁷, C(=O)R⁸, alkylsulfinyle en C₁-C₃, alkylsulfonyle en C₁-C₃, -S(=O)₂NH₂, alkylsulfonylamino en C₁-C₃, alkylaminosulfonyle en C₁-C₃, cycloalkylaminosulfonyle en C₃-C₆, fluoro-alkyle en C₁-C₃, fluoro-alcoxy en C₁-C₃, hydroxy-alkyle en C₁-C₃, cycloalkyle en C₃-C₆, et/ou un radical hétérocyclyle monocyclique de 4 à 7 atomes de cycle, et/ou un radical hétéroaryle monocyclique à 5 ou 6 atomes de cycle, qui peut de son côté éventuellement être substitué une ou plusieurs fois, de manière identique ou différente, avec fluor, chlore, brome, méthyle et/ou méthoxy, et
R^{1b} représente hydrogène, halogène, hydroxy, cyano, nitro ou un radical alkyle en C₁-C₃, alcoxy en C₁-C₃, fluoro-alkyle en C₁-C₃ ou fluoro-alcoxy en C₁-C₃, et
R^{1c} représente hydrogène, fluor, chlore, brome ou cyano, et
R² représente méthyle, éthyle ou isopropyle, et
R³ représente cyclopropyle, alkyle en C₁-C₃, alcoxy en C₁-C₃, amino, cyclopropylamino ou alkylamino en C₁-C₃, et
R⁴ représente hydrogène, hydroxy, cyano, nitro, amino, aminocarbonyle, fluor, chlore, brome, alkyle en C₁-C₆, alcoxy en C₁-C₆, alkylamino en C₁-C₆, alkylcarbonylamino en C₁-C₆, alkylaminocarbonyle en C₁-C₆ ou alkylaminosulfonyle en C₁-C₆, et
R⁵ représente un radical cycloalkyle en C₃-C₇, qui peut éventuellement être substitué une ou plusieurs fois, de manière identique ou différente, avec halogène, amino, hydroxy, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, alkylamino en C₁-C₃-alkyle en C₁-C₃, fluoro-alkyle en C₁-C₃, fluoro-alcoxy en C₁-C₃, et/ou un radical hétérocyclyle monocyclique de 4 à 7 atomes de cycle,
ou
hétéroaryle monocyclique à 5 ou 6 atomes de cycle, qui peut éventuellement être substitué une ou plusieurs fois, de manière identique ou différente, avec halogène, amino, hydroxy, cyano, nitro, carboxy, alkyle en C₁-C₃, alcoxy en C₁-C₃, alcoxy en C₁-C₂-alkyle en C₁-C₂, hydroxy-alkyle en C₁-C₃, alkylamino en C₁-C₃, amino-alkyle en C₁-C₃, fluoro-alkyle en C₁-C₃, fluoro-alcoxy en C₁-C₃, cycloalkyle en C₃-C₆, -C(=O)R⁸, -S(=O)₂R⁹, -NR⁶R⁷, et/ou un radical hétérocyclyle monocyclique de 4 à 7 atomes de cycle,
ou
un radical hétérocyclyle monocyclique de 4 à 7 atomes de cycle, qui peut éventuellement être substitué une ou plusieurs fois, de manière identique ou différente, avec halogène, amino, hydroxy, cyano, oxo, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, hydroxy-alkyle en C₁-C₃, fluoro-alkyle en C₁-C₃, fluoro-alcoxy en C₁-C₃, cycloalkyle en C₃-C₆,-C(=O)R⁸, -S(=O)₂R⁹, -NR⁶R⁷, et/ou un radical hétérocyclyle monocyclique de 4 à 7 atomes de cycle,
ou un radical phényle, qui peut éventuellement être substitué une ou plusieurs fois, de manière identique ou différente, avec halogène, amino, hydroxy, cyano, nitro, carboxy, alkyle en C₁-C₃, alcoxy en C₁-C₃, alcoxy en C₁-C₂-alkyle en C₁-C₂, alkylamino en C₁-C₃, amino-alkyle en C₁-C₃, alkylaminocarbonyle en C₁-C₃, alkylaminosulfonyle en C₁-C₃, hydroxy-alkyle en C₁-C₃, fluoro-alkyle en C₁-C₃, fluoro-alcoxy en C₁-C₃, cycloalkyle en C₃-C₆ et/ou un radical hétérocyclyle monocyclique de 4 à 7 atomes de cycle, et
R⁶ et R⁷ représentent indépendamment l'un de l'autre hydrogène, alkyle en C₁-C₃, cyclopropyle, di-alkyle en C₁-C₃-amino-alkyle en C₁-C₃ ou fluoropyridyle, et
R⁸ représente hydroxy, alkyle en C₁-C₆, hydroxy-alkyle en C₁-C₃, fluoro-alkyle en C₁-C₃, alcoxy en C₁-C₃-alkyle en C₁-C₃, cycloalkyle en C₃-C₈, phényle ou hétérocyclyle monocyclique à 5 ou 6 atomes de cycle, et
R⁹ représente hydrogène, alkyle en C₁-C₆ ou alcoxy en C₁-C₄,
ainsi que leur polymorphes, énantiomères, diastéréomères, racémats, tautomères, solvates, sels physiologiquement acceptables et solvates de ces sels.

7. Composés de formule générale (I) selon les revendications 1 à 3, dans lesquels
X représente un atome d'oxygène, et
A représente un cycle phényle ou 3-pyridyle, et
R^{1a} représente hydrogène ou chlore,
ou
pipérazinyle, pyrrolidinyle, pipéridinyle, diazépanyle, oxazinanyle, oxazolidinyle, morpholinyle, thiomorpholinyle, thiazolidinyle ou azétidinyle, qui peuvent éventuellement être substitués une ou plusieurs fois, de manière identique ou différente, avec fluor, chlore, cyano, nitro, hydroxy, oxo, alkyle en C₁-C₃, alcoxy en C₁-C₃, alcoxy en C₁-C₂-alkyle en C₁-C₂, hydroxy-alkyle en C₁-C₃, diméthylamino, trifluorométhyle, difluoroéthyle, trifluoroéthyle, trifluorométhoxy, cyclopropyle, phényle, fluorophényle, phényl-alkyle en C₁-C₃, pyridinyle,-NR⁶C(=O)-R⁹, -C(=O)-NR⁶R⁷, -C(=O)-R⁸,-S(=O)₂-NR⁶R⁷, -S(=O)-R⁹, -S(=O)₂-R⁹ et/ou-NH-S (=O)₂-R⁹,
ou
tétrazolyle,
ou
isoxazolyle, pyrazolyle, thiényle, thiazolyle, imidazolyle, triazolyle, pyrrolyle, oxadiazolyle, pyridinyle ou pyrimidinyle, qui peuvent éventuellement être substitués une ou plusieurs fois, de manière identique ou différente, avec fluor, chlore, hydroxy, amino, cyano, nitro, alkyle en C₁-C₃, alcoxy en C₁-C₃, alcoxy en C₁-C₂-alkyle en C₁-C₂, diméthylamino, trifluorométhyle, difluoroéthyle, trifluoroéthyle, trifluorométhoxy, cyclopropyle, pyridinyle, phényle, fluorophényle,-C (=O) -NR⁶R⁷, -C(=O)-R⁸, -S(=O)₂-NR⁶R⁷,-S(=O)-R⁹, -S(=O)₂-R⁹ et/ou -NH-S(=O)₂-R⁹,
ou
représente un radical phényle, qui peut éventuellement être substitué une ou plusieurs fois, de manière identique ou différente, avec fluor, chlore, amino, hydroxy, cyano, nitro, carboxy, alkyle en C₁-C₃, alcoxy en C₁-C₃, alcoxy en C₁-C₂-alkyle en C₁-C₂, diméthylamino,-C(=O)NR⁶R⁷, -C(=O)R⁸, alkylsulfinyle en C₁-C₃, alkylsulfonyle en C₁-C₃, -S(=O)₂NH₂, alkylsulfonylamino en C₁-C₃, alkylaminosulfonyle en C₁-C₃, cycloalkylaminosulfonyle en C₃-C₆, trifluorométhyle, difluoroéthyle, trifluoroéthyle, trifluorométhoxy, hydroxy-alkyle en C₁-C₃, cyclopropyle, chlorothiényle, morpholino et/ou pyridinyle, et
R^{1b} représente hydrogène, fluor, brome ou cyano,
R^{1c} représente hydrogène ou brome, et
R² représente méthyle, éthyle ou isopropyle, et
R³ représente cyclopropyle, méthyle, éthyle, méthoxy, éthoxy, cyclopropylamino, méthylamino ou éthylamino, et
R⁴ et R⁵ représentent indépendamment l'un de l'autre hydrogène, hydroxy, cyano, amino, chlore, alkyle en C₁-C₆, méthoxy, éthoxy ou alkylcarbonylamino en C₁-C₃,
ou
difluorométhoxy ou trifluorométhoxy, ou alcoxy en C₁-C₃, qui peut être substitué avec pyridinyle, morpholinyle, pyrrolidinyle ou pipérazinyle, pyridinyle et pipérazinyle pouvant de leur côté éventuellement être substitués avec alkyle en C₁-C₃,
ou
cyclopropyle,
ou
pyridinyle, pyrazolyle, triazolyle ou isoxazolyle, qui peuvent éventuellement être substitués une ou plusieurs fois, de manière identique ou différente, avec hydroxy et/ou méthyle,
ou
pyrrolidinyle, morpholinyle, pipéridinyle, pipérazinyle, oxazolidinyle, thiomorpholinyle, qui peuvent éventuellement être substitués une ou plusieurs fois avec oxo, méthyle et/ou -S(=O)₂R⁹,
ou
phényle éventuellement substitué avec alkylaminosulfonyle en C₁-C₃ ou fluor, et
R⁶ et R⁷ représentent indépendamment l'un de l'autre hydrogène, alkyle en C₁-C₃, cyclopropyle, di-alkyle en C₁-C₃-amino-alkyle en C₁-C₃ ou fluoropyridyle, et
R⁸ représente hydroxy, alkyle en C₁-C₃, hydroxy-alkyle en C₁-C₃, trifluorométhyle, pyrrolidinyle, morpholinyle ou pipéridinyle, et
R⁹ représente alkyle en C₁-C₄ ou alcoxy en C₁-C₄,
ainsi que leur polymorphes, énantiomères, diastéréomères, racémats, tautomères, solvates, sels physiologiquement acceptables et solvates de ces sels,
à condition que
lorsque A représente un cycle phényle et R⁴ et R⁵ représentent indépendamment l'un de l'autre hydrogène, hydroxy, cyano, amino, chlore, alkyle en C₁-C₆, méthoxy, éthoxy ou alkylcarbonylamino en C₁-C₃, ou difluorométhoxy ou trifluorométhoxy, ou alcoxy en C₁-C₃, qui peut être substitué avec pyridinyle, morpholinyle, pyrrolidinyle ou pipérazinyle,
le pyridinyle et le pipérazinyle pouvant de leur côté éventuellement être substitués avec alkyle en C₁-C₃,
R^{1a} ne représente pas hydrogène ou chlore, et
la condition ne comprenant pas les composés de formule générale (I) dans laquelle
A représente phényle et R⁴ représente hydrogène ou chlore, et R⁵ représente trifluorométhoxy, et R^{1a} représente chlore,
ni les composés de formule générale (I) dans laquelle
A représente phényle et R⁴ représente hydrogène, et
R⁵ représente alcoxy en C₁-C₃, qui est substitué avec morpholinyle, pyrrolidinyle, pipérazinyle ou pyridyle, le pipérazinyle et le pyridinyle pouvant eux-mêmes être substitués par alkyle en C₁-C₃, R^{1a} représente chlore.

8. Composés de formule générale (I) selon les revendications 1 à 3 et 7, dans lesquels
X représente un atome d'oxygène, et
A représente un cycle phényle ou 3-pyridyle, et
R^{1a} représente hydrogène ou chlore,
ou
pipérazinyle, pyrrolidinyle, pipéridinyle, diazépanyle, oxazinanyle, oxazolidinyle, morpholinyle, thiomorpholinyle, thiazolidinyle ou azétidinyle, qui peuvent éventuellement être substitués une ou plusieurs fois, de manière identique ou différente, avec fluor, hydroxy, oxo, alkyle en C₁-C₃, méthoxy, hydroxy-alkyle en C₁-C₃, diméthylamino, difluoroéthyle, trifluoroéthyle, benzyle, -NR⁶C(=O)-R⁹,-C(=O)-NR⁶R⁷, -C(=O)-R⁸ et/ou -S(=O)₂-R⁹,
ou
tétrazolyle,
ou
isoxazolyle, pyrazolyle, thiényle, thiazolyle, imidazolyle, triazolyle, pyrrolyle, oxadiazolyle, pyridinyle ou pyrimidinyle, qui peuvent éventuellement être substitués une ou plusieurs fois, de manière identique ou différente, avec fluor, chlore, hydroxy, cyano, alkyle en C₁-C₂, méthoxy, méthoxyméthyle, trifluorométhyle, cyclopropyle, pyridinyle, phényle, fluorophényle et/ou -C(=O)-R⁸,
ou
représente un radical phényle, qui peut éventuellement être substitué une ou plusieurs fois, de manière identique ou différente, avec fluor, chlore, hydroxy, cyano, nitro, carboxy, alkyle en C₁-C₃, méthoxy, -C(=O)NR⁶R⁷, -C(=O)R⁸, alkylsulfinyle en C₁-C₃, alkylsulfonyle en C₁-C₃, -S(=O)₂NH₂,
alkylsulfonylamino en C₁-C₃, alkylaminosulfonyle en C₁-C₃, cycloalkylaminosulfonyle en C₃-C₆, trifluorométhyle, trifluorométhoxy, hydroxy-alkyle en C₁-C₃, cyclopropyle, chlorothiényle et/ou morpholino, et
R^{1b} représente hydrogène, fluor, brome ou cyano, et
R^{1c} représente hydrogène ou brome, et
R² représente méthyle, éthyle ou isopropyle, et
R³ représente cyclopropyle, méthyle, éthyle, méthoxy, éthoxy, cyclopropylamino, méthylamino ou éthylamino, et
R⁴ et R⁵ représentent indépendamment l'un de l'autre hydrogène, hydroxy, cyano, amino, chlore, alkyle en C₁-C₆, méthoxy, éthoxy ou alkylcarbonylamino en C₁-C₃,
ou
difluorométhoxy ou trifluorométhoxy,
ou alcoxy en C₁-C₃, qui peut être substitué avec pyridinyle, morpholinyle, pyrrolidinyle ou pipérazinyle, pyridinyle et pipérazinyle pouvant de leur côté éventuellement être substitués avec alkyle en C₁-C₃,
ou
cyclopropyle,
ou
pyridinyle, pyrazolyle, triazolyle ou isoxazolyle, qui peuvent éventuellement être substitués une ou plusieurs fois, de manière identique ou différente, avec hydroxy et/ou méthyle,
ou
pyrrolidinyle, morpholinyle, pipéridinyle, pipérazinyle, oxazolidinyle, thiomorpholinyle, qui peuvent éventuellement être substitués une ou plusieurs fois avec méthyle, oxo et/ou -S(=O)₂R⁹,
ou
phényle éventuellement substitué avec alkylaminosulfonyle en C₁-C₃ ou fluor, et
R⁶ et R⁷ représentent indépendamment l'un de l'autre hydrogène, alkyle en C₁-C₃, cyclopropyle, di-alkyle en C₁-C₃-amino-alkyle en C₁-C₃ ou fluoropyridyle, et
R⁸ représente hydroxy, alkyle en C₁-C₃, hydroxy-alkyle en C₁-C₃, trifluorométhyle, pyrrolidinyle, morpholinyle ou pipéridinyle, et
R⁹ représente alkyle en C₁-C₄ ou alcoxy en C₁-C₄, ainsi que leurs polymorphes, énantiomères, diastéréomères, racémats, tautomères, solvates, sels physiologiquement compatibles et solvates de ces sels,
à condition que
lorsque A représente un cycle phényle, et R⁴ et R⁵ représentent indépendamment l'un de l'autre hydrogène, hydroxy, cyano, amino, chlore, alkyle en C₁-C₆, méthoxy, éthoxy ou alkylcarbonylamino en C₁-C₃ ou difluorométhoxy ou trifluorométhoxy, ou alcoxy en C₁-C₃, qui peut être substitué avec pyridinyle, morpholinyle, pyrrolidinyle ou pipérazinyle,
le pyridinyle et le pipérazinyle pouvant de leur côté éventuellement être substitués avec alkyle en C₁-C₃,
R^{1a} ne représente pas hydrogène ou chlore, et la condition ne comprenant pas les composés de formule générale (I) dans laquelle
A représente phényle et R⁴ représente hydrogène ou chlore, et R⁵ représente trifluorométhoxy, et R^{1a} représente chlore,
ni les composés de formule générale (I) dans laquelle
A représente phényle et R⁴ représente hydrogène, et
R⁵ représente alcoxy en C₁-C₃, qui est substitué avec morpholinyle, pyrrolidinyle, pipérazinyle ou pyridyle, le pipérazinyle et le pyridinyle pouvant eux-mêmes être substitués par alkyle en C₁-C₃, R^{1a} représente chlore.

9. Composés de formule générale (I) selon les revendications 1 à 3 et 7 à 8, dans lesquels
X représente un atome d'oxygène, et
A représente un cycle phényle, et
R^{1a} représente pipérazinyle, pyrrolidinyle, pipéridinyle, diazépanyle, oxazinanyle, oxazolidinyle, morpholinyle, thiomorpholinyle, thiazolidinyle ou azétidinyle, qui peuvent éventuellement être substitués une ou plusieurs fois, de manière identique ou différente, avec fluor, hydroxy, oxo, alkyle en C₁-C₃, méthoxy, hydroxy-alkyle en C₁-C₃, diméthylamino, difluoroéthyle, trifluoroéthyle, benzyle, -NR⁶C(=O)-R⁹, - C(=O)-NR⁶R⁷, -C(=O)-R⁸ et/ou -S(=O)₂-R⁹,
ou
tétrazolyle,
ou
isoxazolyle, pyrazolyle, thiényle, thiazolyle, imidazolyle, triazolyle, pyrrolyle, oxadiazolyle, pyridinyle ou pyrimidinyle, qui peuvent éventuellement être substitués une ou plusieurs fois, de manière identique ou différente, avec fluor, chlore, hydroxy, cyano, alkyle en C₁-C₂, méthoxy, méthoxyméthyle, trifluorométhyle, cyclopropyle, pyridinyle, phényle, fluorophényle et/ou -C(=O)-R⁸,
ou
représente un radical phényle, qui peut éventuellement être substitué une ou plusieurs fois, de manière identique ou différente, avec fluor, chlore, hydroxy, cyano, nitro, carboxy, alkyle en C₁-C₃, méthoxy, -C(=O)NR⁶R⁷, C(=O)R⁸, alkylsulfinyle en C₁-C₃, alkylsulfonyle en C₁-C₃, -S(=O)₂NH₂, alkylsulfonylamino en C₁-C₃, alkylaminosulfonyle en C₁-C₃, cycloalkylaminosulfonyle en C₃-C₆, trifluorométhyle, trifluorométhoxy, hydroxy-alkyle en C₁-C₃, cyclopropyle, chlorothiényle et/ou morpholino,
R^{1b} représente hydrogène, fluor, brome ou cyano, et
R^{1c} représente hydrogène, et
R² représente méthyle ou éthyle, et
R³ représente méthylamino, et
R⁴ et R⁵ représentent indépendamment l'un de l'autre hydrogène, hydroxy, cyano, chlore, alkyle en C₁-C₆, méthoxy, éthoxy ou alkylcarbonylamino en C₁-C₃,
ou
difluorométhoxy ou trifluorométhoxy, et
R⁶ et R⁷ représentent indépendamment l'un de l'autre hydrogène, alkyle en C₁-C₃, cyclopropyle ou di-alkyle en C₁-C₃-amino-alkyle en C₁-C₃, et
R⁸ représente hydroxy, alkyle en C₁-C₃, hydroxy-alkyle en C₁-C₃, trifluorométhyle, pyrrolidinyle, morpholinyle ou pipéridinyle, et
R⁹ représente alkyle en C₁-C₄ ou alcoxy en C₁-C₄,
ainsi que leur polymorphes, tautomères, solvates, sels physiologiquement acceptables et solvates de ces sels, et
le stéréocentre, qui est représenté par l'atome de carbone relié à R² du squelette benzodiazépine, se présentant soit sous forme racémique, soit principalement, soit en totalité sous la configuration (S).

10. Composés de formule générale (I) selon les revendications 1 à 3 et 7 à 8, dans lesquels
X représente un atome d'oxygène, et
A représente un cycle phényle, et
R^{1a} représente hydrogène ou chlore, et
R^{1b} représente hydrogène, fluor, brome ou cyano, et
R^{1c} représente hydrogène, et
R² représente méthyle ou éthyle, et
R³ représente méthylamino, et
R⁴ représente cyclopropyle,
ou
pyridinyle, pyrazolyle, triazolyle ou isoxazolyle, qui peuvent éventuellement être substitués une ou plusieurs fois, de manière identique ou différente, avec hydroxy et/ou méthyle,
ou
pyrrolidinyle, morpholinyle, pipéridinyle, pipérazinyle, oxazolidinyle ou thiomorpholinyle, qui peuvent éventuellement être substitués une ou plusieurs fois avec méthyle, oxo et/ou -S (=O)₂R⁹,
ou
phényle éventuellement substitué avec alkylaminosulfonyle en C₁-C₃ ou fluor, et
R⁵ représente hydrogène, hydroxy, cyano, chlore, alkyle en C₁-C₆, méthoxy, éthoxy ou alkylcarbonylamino en C₁-C₃,
ou
difluorométhoxy ou trifluorométhoxy, et
R⁶ et R⁷ représentent indépendamment l'un de l'autre hydrogène, alkyle en C₁-C₃, cyclopropyle ou di-alkyle en C₁-C₃-amino-alkyle en C₁-C₃, et
R⁸ représente hydroxy, alkyle en C₁-C₃, hydroxy-alkyle en C₁-C₃, trifluorométhyle, pyrrolidinyle, morpholinyle ou pipéridinyle, et
R⁹ représente alkyle en C₁-C₄ ou alcoxy en C₁-C₄,
ainsi que leur polymorphes, tautomères, solvates, sels physiologiquement acceptables et solvates de ces sels, et
le stéréocentre, qui est représenté par l'atome de carbone relié à R² du squelette benzodiazépine, se présentant soit sous forme racémique, soit principalement, soit en totalité sous la configuration (S).

11. Composés de formule générale (I) selon les revendications 1 à 3 et 7 à 8, dans lesquels
X représente un atome d'oxygène, et
A représente un cycle phényle, et
R^{1a} représente hydrogène ou chlore, et
R^{1b} représente hydrogène, fluor, brome ou cyano, et
R^{1c} représente hydrogène, et
R² représente méthyle ou éthyle, et
R³ représente méthylamino, et
R⁴ représente hydrogène, chlore, méthoxy ou éthoxy,
ou
difluorométhoxy ou trifluorométhoxy, et
R⁵ représente cyclopropyle,
ou
pyridinyle ou pyrazolyle, qui peuvent éventuellement être substitués une ou plusieurs fois avec méthyle,
ou
morpholinyle, pipéridinyle, pipérazinyle ou thiomorpholinyle, qui peuvent éventuellement être substitués une ou plusieurs fois avec méthyle, oxo et/ou *-*S(=O)₂R⁹,
ou
phényle éventuellement substitué avec alkylaminosulfonyle en C₁-C₃, et
R⁶ et R⁷ représentent indépendamment l'un de l'autre hydrogène, alkyle en C₁-C₃, cyclopropyle ou di-alkyle en C₁-C₃-amino-alkyle en C₁-C₃, et
R⁸ représente hydroxy, alkyle en C₁-C₃, hydroxy-alkyle en C₁-C₃, trifluorométhyle, pyrrolidinyle, morpholinyle ou pipéridinyle, et
R⁹ représente alkyle en C₁-C₄ ou alcoxy en C₁-C₄,
ainsi que leur polymorphes, tautomères, solvates, sels physiologiquement acceptables et solvates de ces sels, et
le stéréocentre, qui est représenté par l'atome de carbone relié à R² du squelette benzodiazépine, se présentant soit sous forme racémique, soit principalement, soit en totalité sous la configuration (S).

12. Composés de formule générale (I) selon les revendications 1 à 3 et 7 à 8, dans lesquels
X représente un atome d'oxygène, et
A représente un cycle phényle, et
R^{1a} représente pipérazinyle, pyrrolidinyle, pipéridinyle, diazépanyle, oxazinanyle, oxazolidinyle, morpholinyle, thiomorpholinyle, thiazolidinyle ou azétidinyle, qui peuvent éventuellement être substitués une ou plusieurs fois, de manière identique ou différente, avec fluor, hydroxy, oxo, alkyle en C₁-C₃, méthoxy, hydroxy-alkyle en C₁-C₃, diméthylamino, difluoroéthyle, trifluoroéthyle, benzyle, -NR⁶C(=O)-R⁹, - C(=O)-NR⁶R⁷, -C(=O)-R⁸ et/ou -S(=O)₂-R⁹,
ou
isoxazolyle ou pyrazolyle, qui peuvent éventuellement être substitués une ou plusieurs fois, de manière identique ou différente, avec alkyle en C₁-C₂, et
R^{1b} représente hydrogène, fluor, brome ou cyano, et
R^{1c} représente hydrogène, et
R² représente méthyle, et
R³ représente méthylamino, et
R⁴ et R⁵ représentent indépendamment l'un de l'autre hydrogène, hydroxy, cyano, chlore, alkyle en C₁-C₆, méthoxy, éthoxy ou alkylcarbonylamino en C₁-C₃,
ou
difluorométhoxy ou trifluorométhoxy, et
R⁶ et R⁷ représentent indépendamment l'un de l'autre hydrogène, alkyle en C₁-C₃, cyclopropyle ou di-alkyle en C₁-C₃-amino-alkyle en C₁-C₃, et
R⁸ représente hydroxy, alkyle en C₁-C₃, hydroxy-alkyle en C₁-C₃, trifluorométhyle, pyrrolidinyle, morpholinyle ou pipéridinyle, et
R⁹ représente alkyle en C₁-C₄ ou alcoxy en C₁-C₄,
ainsi que leur polymorphes, tautomères, solvates, sels physiologiquement acceptables et solvates de ces sels, et
le stéréocentre, qui est représenté par l'atome de carbone relié à R² du squelette benzodiazépine, se présentant soit sous forme racémique, soit principalement, soit en totalité sous la configuration (S).

13. Composés de formule générale (I) selon les revendications 1 à 3 et 7 à 8 et 12, dans lesquels
X représente un atome d'oxygène, et
A représente un cycle phényle, et
R^{1a} représente pipérazinyle, pyrrolidinyle, pipéridinyle, morpholinyle, thiomorpholinyle ou azétidinyle, qui peuvent éventuellement être substitués une ou plusieurs fois, de manière identique ou différente, avec hydroxy, oxo, alkyle en C₁-C₃, méthoxy, diméthylamino, difluoroéthyle, trifluoroéthyle, -NR⁶C(=O)-R⁹, -C((=O)-NR⁶R⁷ et/ou -C(=O)-R⁸, et
R^{1b} représente hydrogène, fluor, brome ou cyano, et
R^{1c} représente hydrogène, et
R² représente méthyle, et
R³ représente méthylamino, et
R⁴ et R⁵ représentent indépendamment l'un de l'autre, chlore, méthoxy ou éthoxy,
ou
difluorométhoxy ou trifluorométhoxy, et
R⁶ et R⁷ représentent indépendamment l'un de l'autre hydrogène ou alkyle en C₁-C₃, et
R⁸ représente méthyle, et
R⁹ représente méthyle,
ainsi que leur polymorphes, tautomères, solvates, sels physiologiquement acceptables et solvates de ces sels, et
le stéréocentre, qui est représenté par l'atome de carbone relié à R² du squelette benzodiazépine, se présentant soit sous forme racémique, soit principalement, soit en totalité sous la configuration (S).

14. Composés de formule générale (I) selon les revendications 1 à 3, dans lesquels A représente un cycle phényle et R⁴ représente hydrogène ou chlore, et R⁵ représente trifluorométhoxy, et R^{1a} représente chlore, et composés de formule générale (I) dans laquelle A représente un cycle phényle et R⁴ représente hydrogène, et R⁵ représente alcoxy en C₁-C₃, qui peut être substitué avec morpholinyle, pyrrolidinyle, pipérazinyle ou pyridyle, le pipérazinyle et le pyridinyle pouvant eux-mêmes être substitués par alkyle en C₁-C₃, R^{1a} représente chlore.

15. Composés de formule générale (I) selon les revendications 1 à 14,
- (±)-1-(4-chlorophényl)-*N*,4-diméthyl-8-(trifluorométhoxy)-4,5-dihydro-3*H*-2,3-benzodiazépine-3-carboxamide
- (4*S*)-1-(4-chlorophényl)-*N*,4-diméthyl-8-(trifluorométhoxy)-4,5-dihydro-3*H*-2,3-benzodiazépine-3-carboxamide
- (4*R*)-1-(4-chlorophényl)-*N*,4-diméthyl-8-(trifluorométhoxy)-4,5-dihydro-3*H*-2,3-benzodiazépine-3-carboxamide
- (±)-1-[4-(3,5-diméthyl-4-isoxazolyl)phényl]-7,8-diméthoxy-*N*,4-diméthyl-4,5-dihydro-3*H*-2,3-benzodiazépine-3-carboxamide
- (4*R*)-1-[4-(3,5-diméthyl-4-isoxazolyl)phényl]-7,8-diméthoxy-*N*,4-diméthyl-4,5-dihydro-3*H*-2,3-benzodiazépine-3-carboxamide
- (4*S*)-1-[4-(3,5-diméthyl-4-isoxazolyl)phényl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-[4-(1*H*-pyrazol-3-yl)phényl]-4,5-dihydro-3*H*-2,3-benzodiazépine-3-carboxamide
- (±)-1-[4-(2-chloropyridin-3-yl)phényl]-7,8-diméthoxy-*N*,4-diméthyl-4,5-dihydro-3*H*-2,3-benzodiazépine-3-carboxamide
- acide (±)-5-(4-{7,8-diméthoxy-4-méthyl-3-[(méthylamino)carbonyl]-4,5-dihydro-3*H*-2,3-benzodiazépine-1-yl}phényl)thiophène-2-carboxylique
- acide (±)-4'-{7,8-diméthoxy-4-méthyl-3-[(méthylamino)carbonyl]-4,5-dihydro-3*H*-2,3-benzodiazépine-1-yl}biphényl-2-carboxylique
- (±)-7,8-diméthoxy-*N*,4-diméthyl-1-[4-(morpholin-4-yl)phényl]-4,5-dihydro-3*H*-2,3-benzodiazépine-3-carboxamide
- (±)-1-(4-chlorophényl)-*N*,4-diméthyl-8-(pyridin-4-yl)-4,5-dihydro-3*H*-2,3-benzodiazépine-3-carboxamide
- (±)-1-(4-chlorophényl)-8-cyclopropyl-*N*,4-diméthyl-4,5-dihydro-3*H*-2,3-benzodiazépine-3-carboxamide
- (±)-1-(4-chlorophényl)-*N*,4-diméthyl-8-{4-[(méthylamino)sulfonyl]phényl}-4,5-dihydro-3*H*-2,3-benzodiazépine-3-carboxamide
- (±)-1-(4-chlorophényl)-*N*,4-diméthyl-8-(morpholin-4-yl)-4,5-dihydro-3*H*-2,3-benzodiazépine-3-carboxamide
- (4*S*)-1-(4-chlorophényl)-*N*,4-diméthyl-8-(morpholin-4-yl)-4,5-dihydro-3*H*-2,3-benzodiazépine-3-carboxamide
- (4*R*)-1-(4-chlorophényl)-*N*,4-diméthyl-8-(morpholin-4-yl)-4,5-dihydro-3*H*-2,3-benzodiazépine-3-carboxamide
- (±)-1-(4-chlorophényl)-*N*,4-diméthyl-8-(4-méthylpipérazin-1-yl)-4,5-dihydro-3*H*-2,3-benzodiazépine-3-carboxamide
- (±)-1-(4-chlorophényl)-*N*,4-diméthyl-8-(pipéridin-1-yl)-4,5-dihydro-3*H*-2,3-benzodiazépine-3-carboxamide
- (±)-8-méthoxy-*N*,4-diméthyl-1-(pyridin-3-yl)-4,5-dihydro-3*H*-2,3-benzodiazépine-3-carboxamide
- (±)-7-chloro-1-(4-chlorophényl)-*N*,4-diméthyl-8-(trifluorométhoxy)-4,5-dihydro-3*H*-2,3-benzodiazépine-3-carboxamide
- 7-chloro-1-(4-chlorophényl)-*N*,4-diméthyl-8-(trifluorométhoxy)-4,5-dihydro-3*H*-2,3-benzodiazépine-3-carboxamide, énantiomère 1
- (4*S*)-1-[4-(3,5-diméthyl-1*H*-pyrazol-1-yl)phényl]-8-méthoxy-*N*,4-diméthyl-4,5-dihydro-3*H*-2,3-benzodiazépine-3-carboxamide
- (4*S*)-8-méthoxy-*N*,4-diméthyl-1-[4-(morpholin-4-yl)phényl]-4,5-dihydro-3*H*-2,3-benzodiazépine-3-carboxamide
- (4*S*)-1-[4-(4-isoxazolyl)phényl]-8-méthoxy-*N*,4-diméthyl-4,5-dihydro-3*H*-2,3-benzodiazépine-3-carboxamide
- (4*S*)-8-méthoxy-*N*,4-diméthyl-1-[4-(1-méthyl-1H-pyrazol-5-yl)phényl]-4,5-dihydro-3*H*-2,3-benzodiazépine-3-carboxamide
- (4*S*)-1-[4-(3,5-diméthyl-4-isoxazolyl)phényl]-8-méthoxy-*N*,4-diméthyl-4,5-dihydro-3*H*-2,3-benzodiazépine-3-carboxamide
- (4*S*)-8-méthoxy-*N*,4-diméthyl-1-[4-(1-méthyl-1*H-*pyrazol-4-yl)phényl]-4,5-dihydro-3*H*-2,3-benzodiazépine-3-carboxamide
- (4*S*)-8-méthoxy-*N*,4-diméthyl-1-[4-(1,3,5-triméthyl-1*H*-pyrazol-4-yl)phényl]-4,5-dihydro-3*H*-2,3-benzodiazépine-3-carboxamide
- (4*S*)-8-méthoxy-*N*,4-diméthyl-1-[4-(1*H*-pyrazol-5-yl)phényl]-4,5-dihydro-3*H*-2,3-benzodiazépine-3-carboxamide
- (4*S*)-1-[4-(3-cyclopropyl-5-éthyl-1*H*-pyrazol-1-yl)phényl]-8-méthoxy-*N*,4-diméthyl-4,5-dihydro-3*H-*2,3-benzodiazépine-3-carboxamide
- (4*S*)-1-[4-(5-cyclopropyl-3-éthyl-1*H*-pyrazol-1-yl)phényl]-8-méthoxy-*N*,4-diméthyl-4,5-dihydro-3*H-*2,3-benzodiazépine-3-carboxamide
- (4*S*)-8-méthoxy-1-{4-[3-(méthoxyméthyl)-5-méthyl-1*H*-pyrazol-1-yl]phényl}-*N*,4-diméthyl-4,5-dihydro-3*H*-2,3-benzodiazépine-3-carboxamide
- (4*S*)-8-méthoxy-1-{4-[5-(méthoxyméthyl)-3-méthyl-1*H*-pyrazol-1-yl]phényl}-*N*,4-diméthyl-4,5-dihydro-3*H*-2,3-benzodiazépine-3-carboxamide
- (4*S*)-1-{4-[5-cyclopropyl-3-(pyridin-2-yl)-1*H-*pyrazol-1-yl]phényl}-8-méthoxy-*N*,4-diméthyl-4,5-dihydro-3*H*-2,3-benzodiazépine-3-carboxamide
- (4*S*)-1-{4-[3-cyclopropyl-5-(pyridin-2-yl)-1*H-*pyrazol-1-yl]phényl}-8-méthoxy-*N*,4-diméthyl-4,5-dihydro-3*H*-2,3-benzodiazépine-3-carboxamide
- (4*S*)-8-méthoxy-*N*,4-diméthyl-1-[4-(1*H*-tetrazol-1-yl)phényl]-4,5-dihydro-3*H*-2,3-benzodiazépine-3-carboxamide
- (±)-1-[3-(3,5-diméthylisoxazol-4-yl)phényl]-7,8-diméthoxy-*N*,4-diméthyl-4,5-dihydro-3*H*-2,3-benzodiazépine-3-carboxamide
- (±)-1-(4-chlorophényl)-*N*,4-diméthyl-8-[2-(morpholin-4-yl)éthoxy]-4,5-dihydro-3*H*-2,3-benzodiazépine-3-carboxamide
- (±)-1-(4-chlorophényl)-*N*,4-diméthyl-8-[2-(pyrrolidin-1-yl)éthoxy]-4,5-dihydro-3*H*-2,3-benzodiazépine-3-carboxamide
- (±)-7,8-diméthoxy-*N*,4-diméthyl-1-[4-(2-oxoxazolidin-3-yl)phényl]-4,5-dihydro-3*H*-2,3-benzodiazépine-3-carboxamide
- (4*S*)-7,8-diméthoxy-*N*,4-diméthyl-1-[4-(2-oxoxazolidin-3-yl)phényl]-4,5-dihydro-3*H*-2,3-benzodiazépine-3-carboxamide
- (±)-7,8-diméthoxy-*N*,4-diméthyl-1-[4-(2-oxopipéridin-1-yl)phényl]-4,5-dihydro-3*H*-2,3-benzodiazépine-3-carboxamide
- (±)-1-[4-(4-benzyl-2-oxopipérazin-1-yl)phényl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-[4-(4-méthyl-2-oxo-1,4-diazépan-1-yl)phényl]-4,5-dihydro-3*H*-2,3-benzodiazépine-3-carboxamide
- (±)-7,8-diméthoxy-*N*,4-diméthyl-1-[4-(2-oxo-1,3-oxazinan-3-yl)phényl]-4,5-dihydro-3*H*-2,3-benzodiazépine-3-carboxamide
- (±)-7,8-diméthoxy-*N*,4-diméthyl-1-[4-(2-oxopyrrolidin-1-yl)phényl]-4,5-dihydro-3*H*-2,3-benzodiazépine-3-carboxamide
- (±)-7,8-diméthoxy-*N*,4-diméthyl-1-[4-(3-oxomorpholin-4-yl)phényl]-4,5-dihydro-3*H*-2,3-benzodiazépine-3-carboxamide
- (4*S*)-7,8-diméthoxy-*N*,4-diméthyl-1-[4-(3-oxomorpholin-4-yl)phényl]-4,5-dihydro-3*H*-2,3-benzodiazépine-3-carboxamide
- (±)-7,8-diméthoxy-*N*,4-diméthyl-1-[4-(2-méthyl-5-oxomorpholin-4-yl)phényl]-4,5-dihydro-3*H*-2,3-benzodiazépine-3-carboxamide (mélange de stéréoisomères)
- (±)-7,8-diméthoxy-*N*,4-diméthyl-1-[4-(2-méthyl-3-oxomorpholin-4-yl)phényl]-4,5-dihydro-3*H*-2,3-benzodiazépine-3-carboxamide (mélange de stéréoisomères)
- (±)-7,8-diméthoxy-*N*,4-diméthyl-1-[4-(4-méthyl-2-oxopipérazin-1-yl)phényl]-4,5-dihydro-3*H*-2,3-benzodiazépine-3-carboxamide
- (4*S*)-7,8-diméthoxy-*N*,4-diméthyl-1-[4-(4-méthyl-2-oxopipérazin-1-yl)phényl]-4,5-dihydro-3*H*-2,3-benzodiazépine-3-carboxamide
- (4*R*)-7,8-diméthoxy-*N*,4-diméthyl-1-[4-(4-méthyl-2-oxopipérazin-1-yl)phényl]-4,5-dihydro-3*H*-2,3-benzodiazépine-3-carboxamide
- (±)-7,8-diméthoxy-*N*,4-diméthyl-1-[4-(2-oxopipérazin-1-yl)phényl]-4,5-dihydro-3*H*-2,3-benzodiazépine-3-carboxamide
- (±)-7,8-diméthoxy-*N*,4-diméthyl-1-[4-(1-méthyl-1*H-*1,2,3-triazol-4-yl)phényl]-4,5-dihydro-3*H*-2,3-benzodiazépine-3-carboxamide
- (4*S*)-7,8-diméthoxy-*N*,4-diméthyl-1-[4-(1-méthyl-1*H-*1,2,3-triazol-4-yl)phényl]-4,5-dihydro-3*H*-2,3-benzodiazépine-3-carboxamide
- (±)-1-[4-(2,4-diméthylthiazol-5-yl)phényl]-7,8-diméthoxy-*N*,4-diméthyl-4,5-dihydro-3*H*-2,3-benzodiazépine-3-carboxamide
- (4*S*)-1-[4-(2,4-diméthylthiazol-5-yl)phényl]-7,8-diméthoxy-*N*,4-diméthyl-4,5-dihydro-3*H*-2,3-benzodiazépine-3-carboxamide
- (4*R*)-1-[4-(2,4-diméthylthiazol-5-yl)phényl]-7,8-diméthoxy-*N*,4-diméthyl-4,5-dihydro-3*H*-2,3-benzodiazépine-3-carboxamide
- (±)-1-[4-(1,2-diméthyl-1*H*-imidazol-5-yl)phényl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-{4-[2-(trifluorométhyl)pyridin-3-yl]phényl}-4,5-dihydro-3*H*-2,3-benzodiazépine-3-carboxamide
- (±)-1-[4-(6-hydroxypyridin-3-yl)phényl]-7,8-diméthoxy-*N*,4-diméthyl-4,5-dihydro-3*H*-2,3-benzodiazépine-3-carboxamide
- (4*S*)-1-[4-(6-hydroxypyridin-3-yl)phényl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-{4-[6-(trifluorométhyl)pyridin-3-yl]phényl}-4,5-dihydro-3*H*-2,3-benzodiazépine-3-carboxamide
- (+)-7,8-diméthoxy-*N*,4-diméthyl-1-[4-(1,3,5-triméthyl-1*H*-pyrazol-4-yl)phényl]-4,5-dihydro-3*H-*2,3-benzodiazépine-3-carboxamide
- (4*R*)-7,8-diméthoxy-*N*,4-diméthyl-1-[4-(1,3,5-triméthyl-1*H*-pyrazol-4-yl)phényl]-4,5-dihydro-3*H-*2,3-benzodiazépine-3-carboxamide
- (4*S*)-7,8-diméthoxy-*N*,4-diméthyl-1-[4-(1,3,5-triméthyl-1*H*-pyrazol-4-yl)phényl]-4,5-dihydro-3*H-*2,3-benzodiazépine-3-carboxamide
- (±)-1-[4-(isoxazol-4-yl)phényl]-7,8-diméthoxy-*N*,4-diméthyl-4,5-dihydro-3*H*-2,3-benzodiazépine-3-carboxamide
- (+)-7,8-diméthoxy-*N*,4-diméthyl-1-[3-(1,3,5-triméthyl-1*H*-pyrazol-4-yl)phényl]-4,5-dihydro-3*H-*2,3-benzodiazépine-3-carboxamide
- (±)-7,8-diméthoxy-*N*,4-diméthyl-1-[3-(1-méthyl-1*H-*pyrazol-4-yl)phényl]-4,5-dihydro-3*H*-2,3-benzodiazépine-3-carboxamide
- (±)-7,8-diméthoxy-*N*,4-diméthyl-1-[3-(1-méthyl-1*H-*pyrazol-5-yl)phényl]-4,5-dihydro-3*H*-2,3-benzodiazépine-3-carboxamide
- (±)-1-[4-fluoro-3-(1,3,5-triméthyl-1*H*-pyrazol-4-yl)phényl]-7,8-diméthoxy-*N*,4-diméthyl-4,5-dihydro-3*H*-2,3-benzodiazépine-3-carboxamide
- (±)-1-[4-fluoro-3-(1-méthyl-1*H*-pyrazol-4-yl)phényl]-7,8-diméthoxy-*N*,4-diméthyl-4,5-dihydro-3*H*-2,3-benzodiazépine-3-carboxamide
- (±)-1-[3-(3,5-diméthylisoxazol-4-yl)-4-fluorophényl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-(3'-nitrobiphényl-4-yl)-4,5-dihydro-3*H*-2,3-benzodiazépine-3-carboxamide
- (±)-1-(biphényl-4-yl)-7,8-diméthoxy-*N*,4-diméthyl-4,5-dihydro-3*H*-2,3-benzodiazépine-3-carboxamide
- (±)-1-(2',4'-dichlorobiphényl-4-yl)-7,8-diméthoxy-*N*,4-diméthyl-4,5-dihydro-3*H*-2,3-benzodiazépine-3-carboxamide
- (±)-1-(4'-fluorobiphényl-4-yl)-7,8-diméthoxy-*N*,4-diméthyl-4,5-dihydro-3*H*-2,3-benzodiazépine-3-carboxamide
- (±)-1-(4'-chlorobiphényl-4-yl)-7,8-diméthoxy-*N*,4-diméthyl-4,5-dihydro-3*H*-2,3-benzodiazépine-3-carboxamide
- (±)-7,8-diméthoxy-*N*,4-diméthyl-1-(4'-méthylbiphényl-4-yl)-4,5-dihydro-3*H*-2,3-benzodiazépine-3-carboxamide
- (±)-7,8-diméthoxy-1-(4'-méthoxybiphényl-4-yl)-*N*,4-diméthyl-4,5-dihydro-3*H*-2,3-benzodiazépine-3-carboxamide
- (±)-7,8-diméthoxy-1-[4-(6-méthoxypyridin-3-yl)phényl]-*N*,4-diméthyl-4,5-dihydro-3*H*-2,3-benzodiazépine-3-carboxamide
- (±)-7,8-diméthoxy-*N*,4-diméthyl-1-[4'-(méthylsulfinyl)biphényl-4-yl]-4,5-dihydro-3*H*-2,3-benzodiazépine-3-carboxamide
- (±)-7,8-diméthoxy-*N*,4-diméthyl-1-{2'-[(méthylsulfonyl)amino]biphényl-4-yl}-4,5-dihydro-3*H*-2,3-benzodiazépine-3-carboxamide
- (±)-7,8-diméthoxy-*N*,4-diméthyl-1-[2'-(méthylsulfonyl)biphényl-4-yl]-4,5-dihydro-3*H*-2,3-benzodiazépine-3-carboxamide
- (±)-7,8-diméthoxy-*N*,4-diméthyl-1-{4'-[(méthylsulfonyl)amino]biphényl-4-yl}-4,5-dihydro-3*H*-2,3-benzodiazépine-3-carboxamide
- (±)-7,8-diméthoxy-*N*,4-diméthyl-1-{3'-[(méthylsulfonyl)amino]biphényl-4-yl}-4,5-dihydro-3*H*-2,3-benzodiazépine-3-carboxamide
- (±)-7,8-diméthoxy-*N*,4-diméthyl-1-(2'-méthylbiphényl-4-yl)-4,5-dihydro-3*H*-2,3-benzodiazépine-3-carboxamide
- (±)-7,8-diméthoxy-*N*,4-diméthyl-1-[3'-(méthylsulfonyl)biphényl-4-yl]-4,5-dihydro-3*H*-2,3-benzodiazépine-3-carboxamide
- (±)-7,8-diméthoxy-1-[4-(2-méthoxypyrimidin-5-yl)phényl]-*N*,4-diméthyl-4,5-dihydro-3*H*-2,3-benzodiazépine-3-carboxamide
- (±)-1-(3'-cyano-4'-fluorobiphényl-4-yl)-7,8-diméthoxy-*N*,4-diméthyl-4,5-dihydro-3*H*-2,3-benzodiazépine-3-carboxamide
- (±)-7,8-diméthoxy-1-[4-(2-méthoxypyridin-3-yl)phényl]-*N*,4-diméthyl-4,5-dihydro-3*H*-2,3-benzodiazépine-3-carboxamide
- (±)-1-(3'-carbamoylbiphényl-4-yl)-7,8-diméthoxy-*N*,4-diméthyl-4,5-dihydro-3*H*-2,3-benzodiazépine-3-carboxamide
- (±)-7,8-diméthoxy-*N*,4-diméthyl-1-[4'-(pyrrolidin-1-ylcarbonyl)biphényl-4-yl]-4,5-dihydro-3*H*-2,3-benzodiazépinee-3-carboxamidee
- (±)-7,8-diméthoxy-*N*,4-diméthyl-1-[4'-(morpholin-4-ylcarbonyl)biphényl-4-yl]-4,5-dihydro-3*H*-2,3-benzodiazépine-3-carboxamide
- (±)-7,8-diméthoxy-1-[4-(5-méthoxypyridin-3-yl)phényl]-*N*,4-diméthyl-4,5-dihydro-3*H*-2,3-benzodiazépine-3-carboxamide
- (±)-7,8-diméthoxy-*N*,4-diméthyl-1-[4-(5-méthylpyridin-3-yl)phényl]-4,5-dihydro-3*H*-2,3-benzodiazépine-3-carboxamide
- (±)-7,8-diméthoxy-*N*,4-diméthyl-1-[4-(4-méthylpyridin-3-yl)phényl]-4,5-dihydro-3*H*-2,3-benzodiazépine-3-carboxamide
- (±)-1-[4'-(cyclopropylcarbamoyl)biphényl-4-yl]-7,8-diméthoxy-*N*,4-diméthyl-4,5-dihydro-3*H*-2,3-benzodiazépine-3-carboxamide
- (±)-1-[4-(3-fluoropyridin-4-yl)phényl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-[3'-(trifluorométhyl)biphényl-4-yl]-4,5-dihydro-3*H-*2,3-benzodiazépine-3-carboxamide
- (±)-7,8-diméthoxy-*N*,4-diméthyl-1-[4'-(trifluorométhyl)biphényl-4-yl]-4,5-dihydro-3*H-*2,3-benzodiazépine-3-carboxamide
- (±)-7,8-diméthoxy-1-(3'-méthoxybiphényl-4-yl)-*N*,4-diméthyl-4,5-dihydro-3*H*-2,3-benzodiazépine-3-carboxamide
- (±)-1-[4'-(5-chlorothién-2-yl)biphényl-4-yl]-7,8-diméthoxy-*N*,4-diméthyl-4,5-dihydro-3*H*-2,3-benzodiazépine-3-carboxamide
- (±)-1-(3'-fluorobiphényl-4-yl)-7,8-diméthoxy-*N*,4-diméthyl-4,5-dihydro-3*H*-2,3-benzodiazépine-3-carboxamide
- (±)-7,8-diméthoxy-1-(2'-méthoxybiphényl-4-yl)-*N*,4-40 diméthyl-4,5-dihydro-3*H*-2,3-benzodiazépine-3-carboxamide
- (±)-7,8-diméthoxy-*N*,4-diméthyl-1-[2'-(trifluorométhyl)biphényl-4-yl]-4,5-dihydro-3*H-*2,3-benzodiazépine-3-carboxamide
- (±)-1-(2'-chlorobiphényl-4-yl)-7,8-diméthoxy-*N*,4-diméthyl-4,5-dihydro-3*H*-2,3-benzodiazépine-3-carboxamide
- (±)-1-(2'-fluorobiphényl-4-yl)-7,8-diméthoxy-*N*,4-diméthyl-4,5-dihydro-3*H*-2,3-benzodiazépine-3-carboxamide
- (±)-1-[4'-(hydroxyméthyl)biphényl-4-yl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-[4'-(trifluorométhoxy)biphényl-4-yl]-4,5-dihydro-3*H-*2,3-benzodiazépine-3-carboxamide
- (±)-7,8-diméthoxy-*N*,4-diméthyl-1-[3'-(pyrrolidin-1-ylcarbonyl)biphényl-4-yl]-4,5-dihydro-3*H*-2,3-benzodiazépine-3-carboxamide
- (±)-7,8-diméthoxy-*N*,4-diméthyl-1-[3'-(pipéridin-1-ylcarbonyl)biphényl-4-yl]-4,5-dihydro-3*H*-2,3-benzodiazépine-3-carboxamide
- (±)-7,8-diméthoxy-*N*,4-diméthyl-1-[3'-(morpholin-4-ylcarbonyl)biphényl-4-yl]-4,5-dihydro-3*H*-2,3-benzodiazépine-3-carboxamide
- (±)-1-[3'-(cyclopropylcarbamoyl)biphényl-4-yl]-7,8-diméthoxy-*N*,4-diméthyl-4,5-dihydro-3*H*-2,3-benzodiazépine-3-carboxamide
- (±)-1-(2',4'-difluorobiphényl-4-yl)-7,8-diméthoxy-*N*,4-diméthyl-4,5-dihydro-3*H*-2,3-benzodiazépine-3-carboxamide
- (±)-7,8-diméthoxy-*N*,4-diméthyl-1-[4-(1-méthyl-1*H-*pyrazol-5-yl)phényl]-4,5-dihydro-3*H*-2,3-benzodiazépine-3-carboxamide
- (±)-7,8-diméthoxy-*N*,4-diméthyl-1-(4'-nitrobiphényl-4-yl)-4,5-dihydro-3*H*-2,3-benzodiazépine-3-carboxamide
- (±)-7,8-diméthoxy-*N*,4-diméthyl-1-[4-(pyridin-3-yl)phényl]-4,5-dihydro-3*H*-2,3-benzodiazépine-3-carboxamide
- (±)-7,8-diméthoxy-1-[4-(4-méthoxypyridin-3-yl)phényl]-*N*,4-diméthyl-4,5-dihydro-3*H*-2,3-benzodiazépine-3-carboxamide
- (±)-1-(3'-cyanobiphényl-4-yl)-7,8-diméthoxy-*N*,4-diméthyl-4,5-dihydro-3*H*-2,3-benzodiazépine-3-carboxamide
- (±)-1-(4'-cyanobiphényl-4-yl)-7,8-diméthoxy-*N*,4-diméthyl-4,5-dihydro-3*H*-2,3-benzodiazépine-3-carboxamide
- (±)-7,8-diméthoxy-*N*,4-diméthyl-1-[2'-(trifluorométhoxy)biphényl-4-yl]-4,5-dihydro-3*H-*2,3-benzodiazépine-3-carboxamide
- (±)-7,8-diméthoxy-*N*,4-diméthyl-1-[4'-(méthylsulfonyl)biphényl-4-yl]-4,5-dihydro-3*H*-2,3-benzodiazépine-3-carboxamide
- (±)-1-(2'-cyanobiphényl-4-yl)-7,8-diméthoxy-*N*,4-diméthyl-4,5-dihydro-3*H*-2,3-benzodiazépine-3-carboxamide
- (±)-7,8-diméthoxy-*N*,4-diméthyl-1-[4'-(morpholin-4-yl)biphényl-4-yl]-4,5-dihydro-3*H*-2,3-benzodiazépine-3-carboxamide
- (±)-7,8-diméthoxy-*N*,4-diméthyl-1-[4-(pyrimidin-5-yl)phényl]-4,5-dihydro-3*H*-2,3-benzodiazépine-3-carboxamide
- (±)-1-[2'-(hydroxyméthyl)biphényl-4-yl]-7,8-diméthoxy-*N*,4-diméthyl-4,5-dihydro-3*H*-2,3-benzodiazépie-3-carboxamide
- (±)-1-(3'-{[2-(diméthylamino)éthyl]carb-amoyl}biphényl-4-yl)-7,8-diméthoxy-*N*,4-diméthyl-4,5-dihydro-3*H*-2,3-benzodiazépine-3-carboxamide
- (±)-7,8-diméthoxy-*N*,4-diméthyl-1-(3'-sulfamoylbiphényl-4-yl)-4,5-dihydro-3*H*-2,3-benzodiazépine-3-carboxamide
- (±)-7,8-diméthoxy-*N*,4-diméthyl-1-[4'-(méthylsulfamoyl)biphényl-4-yl]-4,5-dihydro-3*H-*2,3-benzodiazépine-3-carboxamide
- (±)-7,8-diméthoxy-*N*,4-diméthyl-1-[4-(1-méthyl-1*H-*pyrrol-2-yl)phényl]-4,5-dihydro-3*H*-2,3-benzodiazépine-3-carboxamide
- (±)-7,8-diméthoxy-*N*,4-diméthyl-1-[4-(6-méthylpyridin-3-yl)phényl]-4,5-dihydro-3*H*-2,3-benzodiazépine-3-carboxamide
- (±)-1-[4'-(cyclopropylsulfamoyl)biphényl-4-yl]-7,8-diméthoxy-*N*,4-diméthyl-4,5-dihydro-3*H*-2,3-benzodiazépine-3-carboxamide
- (±)-1-(3'-fluoro-5'-hydroxybiphényl-4-yl)-7,8-diméthoxy-*N*,4-diméthyl-4,5-dihydro-3*H*-2,3-benzodiazépine-3-carboxamide
- (±)-1-(3'-fluoro-5'-méthylbiphényl-4-yl)-7,8-diméthoxy-*N*,4-diméthyl-4,5-dihydro-3*H*-2,3-benzodiazépine-3-carboxamide
- (±)-7,8-diméthoxy-*N*,4-diméthyl-1-[3'-(méthylsulfamoyl)biphényl-4-yl]-4,5-dihydro-3*H-*2,3-benzodiazépine-3-carboxamide
- (±)-1-[4-(5-fluoropyridin-3-yl)phényl]-7,8-diméthoxy-*N*,4-diméthyl-4,5-dihydro-3*H*-2,3-benzodiazépine-3-carboxamide
- (±)-1-[4-(4-fluoropyridin-3-yl)phényl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-[4-(2-méthylpyridin-3-yl)phényl]-4,5-dihydro-3*H*-2,3-benzodiazépine-3-carboxamide
- (±)-7,8-diméthoxy-1-[4-(2-méthoxypyridin-4-yl)phényl]-*N*,4-diméthyl-4,5-dihydro-3*H*-2,3-benzodiazépine-3-carboxamide
- (±)-1-[4-(5-cyanopyridin-3-yl)phényl]-7,8-diméthoxy-*N*,4-diméthyl-4,5-dihydro-3*H*-2,3-benzodiazépine-3-carboxamide
- (4*R*)-7,8-diméthoxy-*N*,4-diméthyl-1-[4-(morpholin-4-yl)phényl]-4,5-dihydro-3*H*-2,3-benzodiazépine-3-carboxamide
- (4*S*)-7,8-diméthoxy-*N*,4-diméthyl-1-[4-(morpholin-4-yl)phényl]-4,5-dihydro-3*H*-2,3-benzodiazépine-3-carboxamide
- (4*S*)-7,8-diméthoxy-*N*,4-diméthyl-1-[4-(4-méthylpipérazin-1-yl)phényl]-4,5-dihydro-3*H*-2,3-benzodiazépine-3-carboxamide
- (±)-1-[4-(azétidin-1-yl)phényl]-7,8-diméthoxy-*N*,4-diméthyl-4,5-dihydro-3*H*-2,3-benzodiazépine-3-carboxamide
- (4*R*)-1-[4-(azétidin-1-yl)phényl]-7,8-diméthoxy-*N*,4-diméthyl-4,5-dihydro-3*H*-2,3-benzodiazépine-3-carboxamide
- (4*S*)-1-[4-(azétidin-1-yl)phényl]-7,8-diméthoxy-*N*,4-diméthyl-4,5-dihydro-3*H*-2,3-benzodiazépine-3-carboxamide
- (±)-1-[4-(3-fluoroazétidin-1-yl)phényl]-7,8-diméthoxy-*N*,4-diméthyl-4,5-dihydro-3*H*-2,3-benzodiazépine-3-carboxamide
- (4*R*)-1-[4-(3-fluoroazétidin-1-yl)phényl]-7,8-diméthoxy-*N*,4-diméthyl-4,5-dihydro-3*H*-2,3-benzodiazépine-3-carboxamide
- (4*S*)-1-[4-(3-fluoroazétidin-1-yl)phényl]-7,8-diméthoxy-*N*,4-diméthyl-4,5-dihydro-3*H*-2,3-benzodiazépine-3-carboxamide
- (±)-1-[4-(4-hydroxypipéridin-1-yl)phényl]-7,8-diméthoxy-*N*,4-diméthyl-4,5-dihydro-3*H*-2,3-benzodiazépine-3-carboxamide
- (4*S*)-1-[4-(4-hydroxypipéridin-1-yl)phényl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-[4-(pipérazin-1-yl)phényl]-4,5-dihydro-3*H*-2,3-benzodiazépine-3-carboxamide
- (4*S*)-7,8-diméthoxy-*N*,4-diméthyl-1-[4-(pipérazin-1-yl)phényl]-4,5-dihydro-3*H*-2,3-benzodiazépine-3-carboxamide
- (±)-7,8-diméthoxy-*N*,4-diméthyl-1-[4-(4-méthylpipérazin-1-yl)phényl]-4,5-dihydro-3*H*-2,3-benzodiazépine-3-carboxamide
- (±)-1-[4-(4-acétylpipérazin-1-yl)phényl]-7,8-diméthoxy-*N*,4-diméthyl-4,5-dihydro-3*H*-2,3-benzodiazépine-3-carboxamide
- (4*R*)-1-[4-(4-acétylpipérazin-1-yl)phényl]-7,8-diméthoxy-*N*,4-diméthyl-4,5-dihydro-3*H*-2,3-benzodiazépine-3-carboxamide
- (4*S*)-1-[4-(4-acétylpipérazin-1-yl)phényl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-{4-[4-(trifluoroacétyl)pipérazin-1-yl]phényl}-4,5-dihydro-3*H*-2,3-benzodiazépine-3-carboxamide
- (±)-1-{4-[4-(2-hydroxy-2-méthylpropanoyl)pipérazin-1-yl]phényl}-7,8-diméthoxy-*N*,4-diméthyl-4,5-dihydro-3*H*-2,3-benzodiazépine-3-carboxamide
- (±)-7,8-diméthoxy-*N*,4-diméthyl-1-{4-[4-(méthylsulfonyl)pipérazin-1-yl]phényl}-4,5-dihydro-3*H*-2,3-benzodiazépine-3-carboxamide
- (4*S*)-1-[4-(1,1-dioxidothiomorpholin-4-yl)phényl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-[4-(3-oxopipérazin-1-yl)phényl]-4,5-dihydro-3*H*-2,3-benzodiazépine-3-carboxamide
- (±)-7,8-diméthoxy-*N*,4-diméthyl-1-[4-(4-méthyl-3-oxopipérazin-1-yl)phényl]-4,5-dihydro-3*H*-2,3-benzodiazépine-3-carboxamide
- (±)-7,8-diméthoxy-*N*,4-diméthyl-1-[4-(pipéridin-1-yl)phényl]-4,5-dihydro-3*H*-2,3-benzodiazépine-3-carboxamide
- (±)-7,8-diméthoxy-*N*,4-diméthyl-1-[3-(morpholin-4-yl)phényl]-4,5-dihydro-3*H*-2,3-benzodiazépine-3-carboxamide
- (±)-1-[3-(3,3-difluoroazétidin-1-yl)phényl]-7,8-diméthoxy-*N*,4-diméthyl-4,5-dihydro-3*H*-2,3-benzodiazépine-3-carboxamide
- (±)-1-[3-(azétidin-1-yl)phényl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-[3-(4-méthylpipérazin-1-yl)phényl]-4,5-dihydro-3*H*-2,3-benzodiazépine-3-carboxamide
- (±)-1-[4-fluoro-3-(morpholin-4-yl)phényl]-7,8-diméthoxy-*N*,4-diméthyl-4,5-dihydro-3*H*-2,3-benzodiazépine-3-carboxamide
- (±)-1-[3-(3,3-difluoroazétidin-1-yl)-4-fluorophényl]-7,8-diméthoxy-*N*,4-diméthyl-4,5-dihydro-3*H*-2,3-benzodiazépine-3-carboxamide
- (±)-1-[4-fluoro-3-(4-hydroxypipéridin-1-yl)phényl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-[4-(5-méthyl-3-phényl-1*H*-pyrazol-1-yl)phényl]-4,5-dihydro-3*H*-2,3-benzodiazépine-3-carboxamide
- (4*S*)-7,8-diméthoxy-*N*,4-diméthyl-1-[4-(5-méthyl-3-phényl-1*H*-pyrazol-1-yl)phényl]-4,5-dihydro-3*H*-2,3-benzodiazépine-3-carboxamide
- (±)-1-[4-(5-cyclopropyl-3-phényl-1*H*-pyrazol-1-yl)phényl]-7,8-diméthoxy-*N*,4-diméthyl-4,5-dihydro-3*H*-2,3-benzodiazépine-3-carboxamide
- (4*S*)-1-[4-(5-cyclopropyl-3-phényl-1*H*-pyrazol-1-yl)phényl]-7,8-diméthoxy-*N*,4-diméthyl-4,5-dihydro-3*H*-2,3-benzodiazépine-3-carboxamide
- (±)-7,8-diméthoxy-*N*,4-diméthyl-1-{4-[3-phényl-5-(trifluorométhyl)-1*H*-pyrazol-1-yl]phényl}-4,5-dihydro-3*H*-2,3-benzodiazépine-3-carboxamide
- (±)-1-{4-[3-(4-fluorophényl)-1*H*-pyrazol-1-yl]phényl}-7,8-diméthoxy-*N*,4-diméthyl-4,5-dihydro-3*H*-2,3-benzodiazépine-3-carboxamide
- (4*S*)-1-{4-[3-(4-fluorophényl)-1*H*-pyrazol-1-yl]phényl}-7,8-diméthoxy-*N*,4-diméthyl-4,5-dihydro-3*H*-2,3-benzodiazépine-3-carboxamide
- (4*R*)-1-{4-[3-(4-fluorophényl)-1*H*-pyrazol-1-yl]phényl}-7,8-diméthoxy-*N*,4-diméthyl-4,5-dihydro-3*H*-2,3-benzodiazépine-3-carboxamide
- (+)-7,8-diméthoxy-*N*,4-diméthyl-1-{4-[5-méthyl-3-(trifluorométhyl)-1*H*-pyrazol-1-yl]phényl}-4,5-dihydro-3*H*-2,3-benzodiazépine-3-carboxamide
- (±)-7,8-diméthoxy-*N*,4-diméthyl-1-[4-(1*H*-1,2,4-triazol-1-yl)phényl]-4,5-dihydro-3*H*-2,3-benzodiazépine-3-carboxamide
- (±)-7,8-diméthoxy-*N*,4-diméthyl-1-[4-(5-méthyl-1*H-*1,2,4-triazol-1-yl)phényl]-4,5-dihydro-3*H*-2,3-benzodiazépine-3-carboxamide
- (±)-1-[4-(3,5-diméthyl-1*H*-1,2,4-triazol-1-yl)phényl]-7,8-diméthoxy-*N*,4-diméthyl-4,5-dihydro-3*H*-2,3-benzodiazépine-3-carboxamide
- (±)-8-*tert*-butyl-1-[4-(3,5-diméthylisoxazol-4-yl)phényl]-*N*,4-diméthyl-4,5-dihydro-3*H*-2,3-benzodiazépine-3-carboxamide
- (±)-7-chloro-1-[4-(3,5-diméthylisoxazol-4-yl)phényl]-*N*,4-diméthyl-8-(trifluorométhoxy)-4,5-dihydro-3*H*-2,3-benzodiazépine-3-carboxamide
- (4*S*)-7-chloro-1-[4-(3,5-diméthylisoxazol-4-yl)phényl]-*N*,4-diméthyl-8-(trifluorométhoxy)-4,5-dihydro-3*H*-2,3-benzodiazépine-3-carboxamide
- (4*R*)-7-chloro-1-[4-(3,5-diméthylisoxazol-4-yl)phényl]-*N*,4-diméthyl-8-(trifluorométhoxy)-4,5-dihydro-3*H*-2,3-benzodiazépine-3-carboxamide
- (±)-8-chloro-1-[4-(3,5-diméthylisoxazol-4-yl)phényl]-7-méthoxy-*N*,4-diméthyl-4,5-dihydro-3*H-*2,3-benzodiazépine-3-carboxamide
- (4*S*)-8-chloro-1-[4-(3,5-diméthylisoxazol-4-yl)phényl]-7-méthoxy-*N*,4-diméthyl-4,5-dihydro-3*H-*2,3-benzodiazépine-3-carboxamide
- (4*R*)-8-chloro-1-[4-(3,5-diméthylisoxazol-4-yl)phényl]-7-méthoxy-*N*,4-diméthyl-4,5-dihydro-3*H-*2,3-benzodiazépine-3-carboxamide
- (±)-1-[4-(3,5-diméthylisoxazol-4-yl)phényl]-*N*,4,8-triméthyl-4,5-dihydro-3*H*-2,3-benzodiazépine-3-carboxamide
- (±)-7,8-bis(difluorométhoxy)-1-[4-(3,5-diméthylisoxazol-4-yl)phényl]-*N*,4-diméthyl-4,5-dihydro-3*H*-2,3-benzodiazépine-3-carboxamide
- (±)-1-[4-(3,5-diméthylisoxazol-4-yl)phényl]-7,8-diéthoxy-*N*,4-diméthyl-4,5-dihydro-3*H*-2,3-benzodiazépine-3-carboxamide
- (±)-7-(difluorométhoxy)-1-[4-(3,5-diméthylisoxazol-4-yl)phényl]-8-méthoxy-*N*,4-diméthyl-4,5-dihydro-3*H*-2,3-benzodiazépine-3-carboxamide
- (4*S*)-7-(difluorométhoxy)-1-[4-(3,5-diméthylisoxazol-4-yl)phényl]-8-méthoxy-*N*,4-diméthyl-4,5-dihydro-3*H*-2,3-benzodiazépine-3-carboxamide
- (4*R*)-7-(difluorométhoxy)-1-[4-(3,5-diméthylisoxazol-4-yl)phényl]-8-méthoxy-*N*,4-diméthyl-4,5-dihydro-3*H*-2,3-benzodiazépine-3-carboxamide
- (±)-7-(difluorométhoxy)-8-méthoxy-*N*,4-diméthyl-1-[4-(4-méthylpipérazin-1-yl)phényl]-4,5-dihydro-3*H-*2,3-benzodiazépine-3-carboxamide
- (4*R*)-7-(difluorométhoxy)-8-méthoxy-*N*,4-diméthyl-1-[4-(4-méthylpipérazin-1-yl)phényl]-4,5-dihydro-3*H-*2,3-benzodiazépine-3-carboxamide
- (4*S*)-7-(difluorométhoxy)-8-méthoxy-*N*,4-diméthyl-1-[4-(4-méthylpipérazin-1-yl)phényl]-4,5-dihydro-3*H-*2,3-benzodiazépine-3-carboxamide
- (±)-1-(4-chlorophényl)-*N*,4-diméthyl-8-[3-(4-méthylpipérazin-1-yl)propoxy]-4,5-dihydro-3*H*-2,3-benzodiazépine-3-carboxamide
- (4*S*)-1-(4-chlorophényl)-*N*,4-diméthyl-8-[3-(4-méthylpipérazin-1-yl)propoxy]-4,5-dihydro-3*H*-2,3-benzodiazépine-3-carboxamide
- (±)-1-(4-chlorophényl)-*N*,4-diméthyl-8-[3-(morpholin-4-yl)propoxy]-4,5-dihydro-3*H*-2,3-benzodiazépine-3-carboxamide
- (±)-1-(4-chlorophényl)-*N*,4-diméthyl-8-[2-(4-méthylpipérazin-1-yl)éthoxy]-4,5-dihydro-3*H*-2,3-benzodiazépine-3-carboxamide
- (±)-1-(4-chlorophényl)-*N*,4-diméthyl-8-[(6-méthylpyridin-2-yl)méthoxy]-4,5-dihydro-3*H*-2,3-benzodiazépine-3-carboxamide
- (±)-1-[4-(3,5-diméthylisoxazol-4-yl)phényl]-8-hydroxy-*N*,4-diméthyl-4,5-dihydro-3*H*-2,3-benzodiazépine-3-carboxamide
- (±)-1-[4-(3,5-diméthylisoxazol-4-yl)phényl]-*N*,4-diméthyl-8-[3-(morpholin-4-yl)propoxy]-4,5-dihydro-3*H*-2,3-benzodiazépine-3-carboxamide
- (±)-7-cyano-1-[4-(3,5-diméthylisoxazol-4-yl)phényl]-8-méthoxy-*N*,4-diméthyl-4,5-dihydro-3*H-*2,3-benzodiazépine-3-carboxamide
- (±)-8-acétamido-*N*,4-diméthyl-1-[4-(morpholin-4-yl)phényl]-4,5-dihydro-3*H*-2,3-benzodiazépine-3-carboxamide
- (±)-8-acétamido-*N*,4-diméthyl-1-[4-(4-méthylpipérazin-1-yl)phényl]-4,5-dihydro-3*H*-2,3-benzodiazépine-3-carboxamide
- (±)-8-acétamido-1-[4-(3,5-diméthylisoxazol-4-yl)phényl]-*N*,4-diméthyl-4,5-dihydro-3*H*-2,3-benzodiazépine-3-carboxamide
- (±)-1-(4-chlorophényl)-8-(3,5-diméthyl-1*H*-pyrazol-1-yl)-*N*,4-diméthyl-4,5-dihydro-3*H*-2,3-benzodiazépine-3-carboxamide
- (±)-1-[4-(3,5-diméthylisoxazol-4-yl)phényl]-8-(3,5-diméthyl-1*H*-pyrazol-1-yl)-*N*,4-diméthyl-4,5-dihydro-3*H*-2,3-benzodiazépine-3-carboxamide
- (±)-1-[4-(3,5-diméthylisoxazol-4-yl)phényl]-*N*,4-diméthyl-8-(morpholin-4-yl)-4,5-dihydro-3*H*-2,3-benzodiazépine-3-carboxamide
- (4*R*)-1-[4-(3,5-diméthylisoxazo]-4-yl)phényl]-*N*,4-diméthyl-8-(morpholin-4-yl)-4,5-dihydro-3H-2,3-benzodiazépine-3-carboxamide
- (4*S*)-1-[4-(3,5-diméthylisoxazol-4-yl)phényl]-*N*,4-diméthyl-8-(morpholin-4-yl)-4,5-dihydro-3H-2,3-benzodiazépine-3-carboxamide
- (4S)-8-méthoxy-N,4-diméthyl-1-[4-(3-oxomorpholin-4-yl)phényl]-4,5-dihydro-3H-2,3-benzodiazépine-3-carboxamide
- (±)-1-{4-[4-(2-hydroxyéthyl)pipérazin-1-yl]phényl}-7,8-diméthoxy-N,4-diméthyl-4,5-dihydro-3H-2,3-benzodiazépine-3-carboxamide
- (±)-7,8-diméthoxy-1-[4-(4-méthoxypipéridin-1-yl)phényl]-N,4-diméthyl-4,5-dihydro-3H-2,3-benzodiazépine-3-carboxamide
- (4R)-7,8-diméthoxy-1-[4-(4-méthoxypipéridin-1-yl)phényl]-N,4-diméthyl-4,5-dihydro-3H-benzodiazépine-3-carboxamide
- (4S)-7,8-diméthoxy-1-[4-(4-méthoxypipéridin-1-yl)phényl]-N,4-diméthyl-4,5-dihydro-3H-benzodiazépine-3-carboxamide
- (±)-1-{4-[4-(diméthylamino)pipéridin-1-yl]phényl}-7,8-diméthoxy-N,4-diméthyl-4,5-dihydro-3H-2,3-benzodiazépine-3-carboxamide
- (4R)-1-{4-[4-(diméthylamino)pipéridin-1-yl]phényl}-7,8-diméthoxy-N,4-diméthyl-4,5-dihydro-3H-2,3-benzodiazépine-3-carboxamide
- (4S)-1-{4-[4-(diméthylamino)pipéridin-1-yl]phényl}-7,8-diméthoxy-N,4-diméthyl-4,5-dihydro-3H-2,3-benzodiazépine-3-carboxamide
- (±)-1-[4-(3,3-difluorazétidin-1-yl)phényl]-7,8-diméthoxy-N,4-diméthyl-4,5-dihydro-3H-benzodiazépine-3-carboxamide
- (±)-1-[4-(4-acétamidopipéridin-1-yl)phényl]-7,8-diméthoxy-N,4-diméthyl-4,5-dihydro-3H-benzodiazépine-3-carboxamide
- (±)-1-{4-[4-(2-hydroxyéthyl)pipéridin-1-yl]phényl}-7,8-diméthoxy-N,4-diméthyl-4,5-dihydro-3H-2,3-benzodiazépine-3-carboxamide
- (±)-1-{4-[4-(2-hydroxyéthyl)pipéridin-1-yl]phényl}-7,8-diméthoxy-N,4-diméthyl-4,5-dihydro-3H-2,3-benzodiazépine-3-carboxamide
- (±)-1-[4-(3-hydroxyazétidin-1-yl)phényl]-7,8-diméthoxy-N,4-diméthyl-4,5-dihydro-3H-2,3-benzodiazépine-3-carboxamide
- (±)-1-[4-(3-hydroxy-3-méthylazétidin-1-yl)phényl]-7,8-diméthoxy-N,4-diméthyl-4,5-dihydro-3H-2,3-benzodiazépine-3-carboxamide
- (4*R*)-1-[4-(3-hydroxy-3-méthylazétidin-1-yl)phényl]-7,8-diméthoxy-N,4-diméthyl-4,5-dihydro-3H-2,3-benzodiazépine-3-carboxamide
- (4*S*)-1-[4-(3-hydroxy-3-méthylazétidin-1-yl)phényl]-7,8-diméthoxy-N,4-diméthyl-4,5-dihydro-3H-2,3-benzodiazépine-3-carboxamide
- (±)-1-[4-(4-isopropylpipérazin-1-yl)phényl]-7,8-diméthoxy-N,4-diméthyl-4,5-dihydro-3H-2,3-benzodiazépine-3-carboxamide
- (±)-7,8-diméthoxy-1-[4-(3-méthoxyazétidin-1-yl)phényl]-N,4-diméthyl-4,5-dihydro-3H-2,3-benzodiazépine-3-carboxamide
- (±)-1-[4-(4-hydroxy-4-méthylpipéridin-1-yl)phényl]-7,8-diméthoxy-N,4-diméthyl-4,5-dihydro-3H-2,3-benzodiazépine-3-carboxamide
- (4*R*)-1-[4-(4-hydroxy-4-méthylpipéridin-1-yl)phényl]-7,8-diméthoxy-N,4-diméthyl-4,5-dihydro-3H-2,3-benzodiazépine-3-carboxamide
- (4*S*)-1-[4-(4-hydroxy-4-méthylpipéridin-1-yl)phényl]-7,8-diméthoxy-N,4-diméthyl-4,5-dihydro-3H-2,3-benzodiazépine-3-carboxamide
- (±)-7,8-diméthoxy-N,4-diméthyl-1-{4-[4-(méthylcarbamoyl)pipéridin-1-yl]phényl}-4,5-dihydro-3H-2,3-benzodiazépine-3-carboxamide
- (4*S*)-7,8-diméthoxy-N,4-diméthyl-1-{4-[4-(méthylcarbamoyl)pipéridin-1-yl]phényl}-4,5-dihydro-3H-2,3-benzodiazépine-3-carboxamide
- (4*R*)-7,8-diméthoxy-N,4-diméthyl-1-{4-[4-(méthylcarbamoyl)pipéridin-1-yl]phényl}-4,5-dihydro-3H-2,3-benzodiazépine-3-carboxamide
- (±)-1-{4-[(3S)-3-hydroxypyrrolidin-1-yl]phényl}-7,8-diméthoxy-N,4-diméthyl-4,5-dihydro-3H-2,3-benzodiazépine-3-carboxamide
- (4S)-1-{4-[(3S)-3-hydroxypyrrolidin-1-yl]phényl}-7,8-diméthoxy-N,4-diméthyl-4,5-benzodiazépine-1-yl]phényl}-4-méthylpipéridin-4-yl)carbamat
- (±)-1-{4-[(2S,5R)-2,5-diméthylpipérazin-1-yl]phényl}-7,8-diméthoxy-N,4-diméthyl-4,5-dihydro-3H-2,3-benzodiazépine-3-carboxamide
- (±)-1-{4-[4-(2,2-difluoroéthyl)pipérazin-1-yl]phényl}-7,8-diméthoxy-N,4-diméthyl-4,5-dihydro-3H-2,3-benzodiazépine-3-carboxamide
- (4S)-7,8-diméthoxy-N,4-diméthyl-1-[4-(3-oxopipérazin-1-yl)phényl]-4,5-dihydro-3H-2,3-benzodiazépine-3-carboxamide
- (4S)-1-{4-[(2R,6S)-2,6-diméthylmorpholin-4-yl]phényl}-7,8-diméthoxy-N,4-diméthyl-4,5-dihydro-3H-2,3-benzodiazépine-3-carboxamide
- (±)-7,8-diméthoxy-N,4-diméthyl-1-[4-(4-oxopipéridin-1-yl)phényl]-4,5-dihydro-3H-2,3-benzodiazépine-3-carboxamide
- (±)-7,8-diméthoxy-N,4-diméthyl-1-{4-[4-(2,2,2-trifluoroéthyl)pipérazin-1-yl]phényl}-4,5-dihydro-3H-2,3-benzodiazépine-3-carboxamide
- (4S)-1-{4-[(3R,5S)-3,5-diméthylpipérazin-1-yl]phényl}-7,8-diméthoxy-N,4-diméthyl-4,5-dihydro-3H-2,3-benzodiazépine-3-carboxamide
- (±)-7,8-dihydroxy-N,4-diméthyl-1-[4-(4-méthylpipérazin-1-yl)phényl]-4,5-dihydro-3H-2,3-benzodiazépine-3-carboxamide
- (±)-7,8-diéthoxy-N,4-diméthyl-1-[4-(4-méthylpipérazin-1-yl)phényl]-4,5-dihydro-3H-2,3-benzodiazépine-3-carboxamide
- (±)-4-éthyl-7,8-diméthoxy-N-méthyl-1-[4-(4-méthylpipérazin-1-yl)phényl]-4,5-dihydro-3H-2,3-benzodiazépine-3-carboxamide
- (4S)-4-éthyl-7,8-diméthoxy-N-méthyl-1-[4-(4-méthylpipérazin-1-yl)phényl]-4,5-dihydro-3H-2,3-benzodiazépine-3-carboxamide
- (±)-4-éthyl-7,8-diméthoxy-N-méthyl-1-[4-(pipérazin-1-yl)phényl]-4,5-dihydro-3H-2,3-benzodiazépine-3-carboxamide
- (4S)-4-éthyl-7,8-diméthoxy-N-méthyl-1-[4-(pipérazin-1-yl)phényl]-4,5-dihydro-3H-2,3-benzodiazépine-3-carboxamide
- (4R)-4-éthyl-7,8-diméthoxy-N-méthyl-1-[4-(pipérazin-1-yl)phényl]-4,5-dihydro-3H-2,3-benzodiazépine-3-carboxamide
- (±)-4-éthyl-7,8-diméthoxy-N-méthyl-1-{4-[4-(méthylsulfonyl)pipérazin-1-yl]phényl}-4,5-dihydro-3H-2,3-benzodiazépine-3-carboxamide
- (4S)-4-éthyl-7,8-diméthoxy-N-méthyl-1-{4-[4-(méthylsulfonyl)pipérazin-1-yl]phényl}-4,5-dihydro-3H-2,3-benzodiazépine-3-carboxamide
- (4R)-4-éthyl-7,8-diméthoxy-N-méthyl-1-{4-[4-(méthylsulfonyl)pipérazin-1-yl]phényl}-4,5-dihydro-3H-2,3-benzodiazépine-3-carboxamide
- (±)-4-éthyl-1-[4-(3-fluoroazétidin-1-yl)phényl]-7,8-diméthoxy-N-méthyl-4,5-dihydro-3H-2,3-benzodiazépine-3-carboxamide
- (±)-1-[4-(4-acétylpipérazin-1-yl)phényl]-4-éthyl-7,8-diméthoxy-N-méthyl-4,5-dihydro-3H-2,3-benzodiazépinee-3-carboxamide
- (±)-1-[4-(1,1-dioxidothiomorpholin-4-yl)phényl]-4-éthyl-7,8-diméthoxy-N-méthyl-4,5-dihydro-3H-2,3-benzodiazépine-3-carboxamide
- (±)-4-éthyl-1-[4-(4-hydroxypipéridin-1-yl)phényl]-7,8-diméthoxy-N-méthyl-4,5-dihydro-3H-2,3-benzodiazépine-3-carboxamide
- (±)-4-éthyl-7,8-diméthoxy-N-méthyl-1-[4-(morpholin-4-yl)phényl]-4,5-dihydro-3H-2,3-benzodiazépine-3-carboxamide
- (4S)-4-éthyl-7,8-diméthoxy-N-méthyl-1-[4-(morpholin-4-yl)phényl]-4,5-dihydro-3H-2,3-benzodiazépine-3-carboxamide
- (±)-8-chloro-1-[4-(1,1-dioxidothiomorpholin-4-yl)phényl]-N,4-diméthyl-7-(trifluorométhoxy)-4,5-dihydro-3H-2,3-benzodiazépine-3-carboxamide
- (±)-8-chloro-N,4-diméthyl-1-[4-(pipérazin-1-yl)phényl]-7-(trifluorométhoxy)-4,5-dihydro-3H-2,3-benzodiazépine-3-carboxamide
- (±)-8-chloro-1-[4-(4-hydroxypipéridin-1-yl)phényl]-N,4-diméthyl-7-(trifluorométhoxy)-4,5-dihydro-3H-2,3-benzodiazépine-3-carboxamide
- (±)-8-chloro-N,4-diméthyl-1-[4-(4-méthylpipérazin-1-yl)phényl]-7-(trifluorométhoxy)-4,5-dihydro-3H-2,3-benzodiazépine-3-carboxamide
- (±)-8-chloro-N,4-diméthyl-1-{4-[4-(méthylsulfonyl)pipérazin-1-yl]phényl}-7-(trifluorométhoxy)-4,5-dihydro-3H-2,3-benzodiazépine-3-carboxamide
- (±)-8-(1,1-dioxidothiomorpholin-4-yl)-1-[4-(1,1-dioxidothiomorpholin-4-yl)phényl]-N,4-diméthyl-7-(trifluorométhoxy)-4,5-dihydro-3H-2,3-benzodiazépine-3-carboxamide
- (±)-1-(4-chlorophényl)-8-(1,1-dioxidothiomorpholin-4-yl)-N,4-diméthyl-7-(trifluorométhoxy)-4,5-dihydro-3H-2,3-benzodiazépine-3-carboxamide
- (±)-1-[4-(4-acétylpipérazin-1-yl)phényl]-N,4-diméthyl-7-(trifluorométhoxy)-4,5-dihydro-3H-2,3-benzodiazépine-3-carboxamide
- (±)-1-{4-[4-(2-hydroxyéthyl)pipérazin-1-yl]phényl}-N,4-diméthyl-7-(trifluorométhoxy)-4,5-dihydro-3H-2,3-benzodiazépine-3-carboxamide
- (±)-8-méthoxy-N,4-diméthyl-1-[4-(4-méthylpipérazin-1-yl)phényl]-7-(trifluorométhoxy)-4,5-dihydro-3H-2,3-benzodiazépine-3-carboxamide
- (±)-4-éthyl-7,8-diméthoxy-N-méthyl-1-[4-(1,3,5-triméthyl-1H-pyrazol-4-yl)phényl]-4,5-dihydro-3H-2,3-benzodiazépine-3-carboxamide
- (±)-4-éthyl-1-(4'-fluorobiphényl-4-yl)-7,8-diméthoxy-N-méthyl-4,5-dihydro-3H-2,3-benzodiazépine-3-carboxamide
- (±)-4-éthyl-7,8-diméthoxy-N-méthyl-1-[4-(1-méthyl-1H-1,2,3-triazol-4-yl)phényl]-4,5-dihydro-3H-2,3-benzodiazépine-3-carboxamide
- (±)-1-[4-(3,5-diméthyl-1,2-oxazol-4-yl)phényl]-4-éthyl-7,8-diméthoxy-N-méthyl-4,5-dihydro-3H-2,3-benzodiazépine-3-carboxamide
- (±)-4-isopropyl-7,8-diméthoxy-N-méthyl-1-[4-(1,3,5-triméthyl-1H-pyrazol-4-yl)phényl]-4,5-dihydro-3H-2,3-benzodiazépine-3-carboxamide
- (±)-4-éthyl-7,8-diméthoxy-N-méthyl-1-[4-(2-oxopyrrolidin-1-yl)phényl]-4,5-dihydro-3H-2,3-benzodiazépine-3-carboxamide
- (±)-4-éthyl-7,8-diméthoxy-N-méthyl-1-[4-(3-oxomorpholin-4-yl)phényl]-4,5-dihydro-3H-2,3-benzodiazépine-3-carboxamide
- (±)-4-éthyl-7,8-diméthoxy-N-méthyl-1-[4-(2-oxopipéridin-l-yl)phényl]-4,5-dihydro-3H-2,3-benzodiazépine-3-carboxamide
- (±)-4-éthyl-7,8-diméthoxy-N-méthyl-1-[4-(2-oxo-1,3-oxazolidin-3-yl)phényl]-4,5-dihydro-3H-2,3-benzodiazépine-3-carboxamide
- (4S)-7,8-diméthoxy-N,4-diméthyl-1-[4-(4-méthyl-2-oxo-1,4-diazépan-1-yl)phényl]-4,5-dihydro-3H-2,3-benzodiazépine-3-carboxamide
- (±)-1-(4-chlorophényl)-N,4-diméthyl-7-(2-oxo-1,3-oxazolidin-3-yl)-4,5-dihydro-3H-2,3-benzodiazépine-3-carboxamide
- (4S)-1-(4-chlorophényl)-N,4-diméthyl-7-(2-oxo-1,3-oxazolidin-3-yl)-4,5-dihydro-3H-2,3-benzodiazépine-3-carboxamide
- (±)-1-(4-chlorophényl)-N,4-diméthyl-7-(2-oxopipéridin-1-yl)-4,5-dihydro-3H-2,3-benzodiazépine-3-carboxamide
- (±)-1-(4-chlorophényl)-N,4-diméthyl-7-(3-oxomorpholin-4-yl)-4,5-dihydro-3H-2,3-benzodiazépine-3-carboxamide
- (±)-1-(4-chlorophényl)-N,4-diméthyl-7-(morpholin-4-yl)-4,5-dihydro-3H-2,3-benzodiazépine-3-carboxamide
- (±)-1-(4-chlorophényl)-N,4-diméthyl-7-(pyrrolidin-1-yl)-4,5-dihydro-3H-2,3-benzodiazépine-3-carboxamide
- (±)-1-(4-chlorophényl)-7-(1,1-dioxidothiomorpholin-4-yl)-N,4-diméthyl-4,5-dihydro-3H-2,3-benzodiazépine-3-carboxamide
- (±)-1-(4-chlorophényl)-N,4-diméthyl-7-(4-méthylpipérazin-1-yl)-4,5-dihydro-3H-2,3-benzodiazépine-3-carboxamide
- (±)-N,4-diméthyl-7-(4-méthylpipérazin-1-yl)-1-[4-(4-méthylpipérazin-1-yl)phényl]-4,5-dihydro-3H-2,3-benzodiazépine-3-carboxamide
- (±)-N,4-diméthyl-7-(4-méthyl-3-oxopipérazin-1-yl)-1-[4-(4-méthyl-3-oxopipérazin-1-yl)phényl]-4,5-dihydro-3H-2,3-benzodiazépine-3-carboxamide
- (±)-1-(4-chlorophényl)-7-(4-fluorophényl)-N,4-diméthyl-4,5-dihydro-3H-2,3-benzodiazépine-3-carboxamide
- (±)-1-(4-chlorophényl)-N,4-diméthyl-7-(pyridin-4-yl)-4,5-dihydro-3H-2,3-benzodiazépine-3-carboxamide
- (±)-1-(4-chlorophényl)-7-(6-hydroxypyridin-3-yl)-N,4-diméthyl-4,5-dihydro-3H-2,3-benzodiazépine-3-carboxamide
- (±)-1-(4-chlorophényl)-7-(3,5-diméthyl-1,2-oxazol-4-yl)-N,4-diméthyl-4,5-dihydro-3H-2,3-benzodiazépine-3-carboxamide
- (±)-1-(4-chlorophényl)-N,4-diméthyl-7-(1-méthyl-1H-1,2,3-triazol-4-yl)-4,5-dihydro-3H-2,3-benzodiazépine-3-carboxamide
- (4R)-1-[4-(4-hydroxypipéridin-1-yl)phényl]-7,8-diméthoxy-N,4-diméthyl-4,5-dihydro-3H-2,3-benzodiazépine-3-carboxamide
- (4R)-7,8-diméthoxy-N,4-diméthyl-1-[4-(4-méthylpipérazin-1-yl)phényl]-4,5-dihydro-3H-2,3-benzodiazépine-3-carboxamide
- (±)-1-[4-(1,1-dioxido-1,2-thiazolidin-2-yl)phényl]-7,8-diméthoxy-N,4-diméthyl-4,5-dihydro-3H-2,3-benzodiazépine-3-carboxamide
- (±)-1-{7,8-diméthoxy-4-méthyl-1-[4-(4-méthylpipérazin-1-yl)phényl]-4,5-dihydro-3H-2,3-benzodiazépine-3-yl}éthanon
- 1-{(4S)-7,8-diméthoxy-4-méthyl-1-[4-(4-méthylpipérazin-1-yl)phényl]-4,5-dihydro-3H-2,3-benzodiazépine-3-yl}éthanon
- (±)-1-{1-[4-(3,5-diméthyl-1,2-oxazol-4-yl)phényl]-7,8-diméthoxy-4-méthyl-4,5-dihydro-3H-2,3-benzodiazépine-3-yl}éthanon
- (±)-7,8-diméthoxy-N,4-diméthyl-1-[4-(5-méthyl-1,3,4-oxadiazol-2-yl)phényl]-4,5-dihydro-3H-2,3-benzodiazépine-3-carboxamide
- (±)-7,8-diméthoxy-N,4-diméthyl-1-[4-(3-méthyl-1,2,4-oxadiazol-5-yl)phényl]-4,5-dihydro-3H-2,3-benzodiazépine-3-carboxamide
- (±)-N,4-diméthyl-8-(trifluorométhoxy)-1-[4-(1,3,5-triméthyl-1H-pyrazol-4-yl)phényl]-4,5-dihydro-3H-2,3-benzodiazépine-3-carboxamide
- (4S)-N,4-diméthyl-8-(trifluorométhoxy)-1-[4-(1,3,5-triméthyl-1H-pyrazol-4-yl)phényl]-4,5-dihydro-3H-2,3-benzodiazépine-3-carboxamide
- (4R)-N,4-diméthyl-8-(trifluorométhoxy)-1-[4-(1,3,5-triméthyl-1H-pyrazol-4-yl)phényl]-4,5-dihydro-3H-2,3-benzodiazépine-3-carboxamide
- (±)-N,4-diméthyl-1-[4-(4-méthyl-1-pipérazinyl)phényl]-8-(trifluorométhoxy)-4,5-dihydro-3H-2,3-benzodiazépine-3-carboxamide
- (4R)-N,4-diméthyl-1-[4-(4-méthyl-1-pipérazinyl)phényl]-8-(trifluorométhoxy)-4,5-dihydro-3H-2,3-benzodiazépine-3-carboxamide
- (4S)-N,4-diméthyl-1-[4-(4-méthyl-1-pipérazinyl)phényl]-8-(trifluorométhoxy)-4,5-dihydro-3H-2,3-benzodiazépine-3-carboxamide
- (±)-1-[4-(4-hydroxy-1-pipéridinyl)phényl]-N,4-diméthyl-8-(trifluorométhoxy)-4,5-dihydro-3H-2,3-benzodiazépine-3-carboxamide
- (±)-1-[2,4-dibromo-5-(4-méthylpipérazin-1-yl)phényl]-7,8-diméthoxy-N,4-diméthyl-4,5-dihydro-3H-2,3-benzodiazépine-3-carboxamide
- (4S)-1-[3-bromo-4-(4-méthylpipérazin-1-yl)phényl]-7,8-diméthoxy-N,4-diméthyl-4,5-dihydro-3H-2,3-benzodiazépine-3-carboxamide
- (4S)-1-[3-cyano-4-(4-méthylpipérazin-1-yl)phényl]-7,8-diméthoxy-N,4-diméthyl-4,5-dihydro-3H-2,3-benzodiazépine-3-carboxamide
- (±)-7,8-diméthoxy-4-méthyl-1-[4-(4-méthylpipérazin-1-yl)phényl]-3-(1-oxopropyl)-4,5-dihydro-3H-2,3-benzodiazépine
- (±)-3-(cyclopropylcarbonyl)-7,8-diméthoxy-4-méthyl-1-[4-(4-méthylpipérazin-1-yl)phényl]-4,5-dihydro-3H-2,3-benzodiazépine
- (±)-N-cyclopropyl-7,8-diméthoxy-4-méthyl-1-[4-(4-méthylpipérazin-1-yl)phényl]-4,5-dihydro-3H-2,3-benzodiazépine
- (±)-7,8-diméthoxy-N,4-diméthyl-1-[4-(4-méthylpipérazin-1-yl)phényl]-4,5-dihydro-3H-2,3-benzodiazépine-3-carbothioamid
- (±)-7,8-diméthoxy-4-méthyl-1-[4-(4-méthylpipérazin-1-yl)phényl]-4,5-dihydro-3H-2,3-benzodiazépine-3-carboxylate de méthyle
- (±)-7,8-diméthoxy-4-méthyl-1-[4-(4-méthylpipérazin-1-yl)phényl]-4,5-dihydro-3H-2,3-benzodiazépine-3-carboxylate d'éthyle
- (±)-N-éthyl-7,8-diméthoxy-4-méthyl-1-[4-(4-méthylpipérazin-1-yl)phényl]-4,5-dihydro-3H-2,3-benzodiazépine-3-carboxamide
- (4S)-N-éthyl-7,8-diméthoxy-4-méthyl-1-[4-(4-méthylpipérazin-1-yl)phényl]-4,5-dihydro-3H-2,3-benzodiazépine-3-carboxamide

16. Composés selon l'une quelconque des revendications 1 à 15, destinés à une utilisation pour la prophylaxie et/ou le traitement de maladies hyper-prolifératives, d'hyperplasies bénignes, de maladies inflammatoires, de maladies auto-immunes, du sepsis, d'infections virales, de maladies vasculaires, de maladies athérosclérotiques et de maladies neurodégénératives.

17. Composé selon l'une quelconque des revendications 1 à 15, destiné à une utilisation pour la prophylaxie et/ou le traitement de maladies tumorales.

18. Composé selon l'une quelconque des revendications 1 à 15, destiné à une utilisation pour le contrôle de la fertilité masculine.

19. Composé selon l'une quelconque des revendications 1 à 15, destiné à une utilisation pour la prophylaxie et/ou le traitement de leucémies, de cancers de la prostate, de cancers du sein, de mélanomes ou de myélomes multiples.

20. Utilisation d'un composé selon l'une quelconque des revendications 1 à 15 pour la fabrication d'un médicament.

21. Composé selon l'une quelconque des revendications 1 à 15 pour une utilisation pour la prophylaxie et/ou le traitement de maladies humaines ou d'un autre mammifère.

22. Composé selon l'une quelconque des revendications 1 à 15 en combinaison avec un autre agent actif.

23. Formulation pharmaceutique contenant un composé selon l'une quelconque des revendications 1 à 15.

24. Composés intermédiaires de formule générale (la), 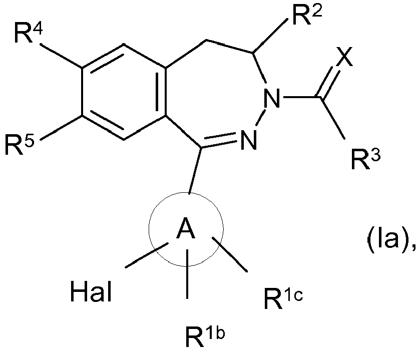 dans laquelle X, A, R^{1b}, R^{1c}, R², R³, R⁴ et R⁵ ont les significations indiquées pour la formule générale (I) et Hal représente bromo, pour la fabrication des composés de formule générale (I).

25. Composés intermédiaires :
(4R)-1-(4-bromophényl)-7,8-diméthoxy-N,4-diméthyl-4,5-dihydro-3H-2,3-benzodiazépine-3-carboxamide (4S)-1-(4-bromophényl)-7,8-diméthoxy-N,4-diméthyl-4,5-dihydro-3H-2,3-benzodiazépine-3-carboxamide (±)-1-(4-bromophényl)-7,8-diméthoxy-N,4-diméthyl-4,5-dihydro-3H-2,3-benzodiazépine-3-carboxamide (±)-1-(4-chlorophényl)-8-méthoxy-N,4-diméthyl-4,5-dihydro-3H-2,3-benzodiazépine-3-carboxamide (±)-1-(3-bromophényl)-7,8-diméthoxy-N,4-diméthyl-4,5-dihydro-3H-2,3-benzodiazépine-3-carboxamide (±)-8-bromo-1-(4-chlorophényl)-N,4-diméthyl-4,5-dihydro-3H-2,3-benzodiazépine-3-carboxamide (4R)-8-bromo-1-(4-chlorophényl)-N,4-diméthyl-4,5-dihydro-3H-2,3-benzodiazépine-3-carboxamide (4S)-8-bromo-1-(4-chlorophényl)-N,4-diméthyl-4,5-dihydro-3H-2,3-benzodiazépine-3-carboxamide (4S)-1-(4-bromophényl)-8-méthoxy-N,4-diméthyl-4,5-dihydro-3H-2,3-benzodiazépine-3-carboxamide (±)-1-(4-chlorophényl)-8-hydroxy-N,4-diméthyl-4,5-dihydro-3H-2,3-benzodiazépine-3-carboxamide (±)-1-(3-bromo-4-fluorophényl)-7,8-diméthoxy-N,4-diméthyl-4,5-dihydro-3H-2,3-benzodiazépine-3-carboxamide (±)-8-tert-butyl-1-(4-chlorophényl)-N,4-diméthyl-4,5-dihydro-3H-2,3-benzodiazépine-3-carboxamide (4S)-8-tert-butyl-1-(4-chlorophényl)-N,4-diméthyl-4,5-dihydro-3H-2,3-benzodiazépine-3-carboxamide (4R)-8-tert-butyl-1-(4-chlorophényl)-N,4-diméthyl-4,5-dihydro-3H-2,3-benzodiazépine-3-carboxamide (±)-8-chloro-1-(4-chlorophényl)-7-méthoxy-N,4-diméthyl-4,5-dihydro-3H-2,3-benzodiazépine-3-carboxamide (4S)-8-chloro-1-(4-chlorophényl)-7-méthoxy-N,4-diméthyl-4,5-dihydro-3H-2,3-benzodiazépine-3-carboxamide (4R)-8-chloro-1-(4-chlorophényl)-7-méthoxy-N,4-diméthyl-4,5-dihydro-3H-2,3-benzodiazépine-3-carboxamide (±)-1-(4-chlorophényl)-N,4,8-triméthyl-4,5-dihydro-3H-2,3-benzodiazépine-3-carboxamide (4S)-1-(4-chlorophényl)-N,4,8-triméthyl-4,5-dihydro-3H-2,3-benzodiazépine-3-carboxamide (4R)-1-(4-chlorophényl)-N,4,8-triméthyl-4,5-dihydro-3H-2,3-benzodiazépine-3-carboxamide (±)-8-amino-1-(4-chlorophényl)-N,4-diméthyl-4,5-dihydro-3H-2,3-benzodiazépine-3-carboxamide (±)-1-[8-tert-butyl-1-(4-chlorophényl)-4-méthyl-4,5-dihydro-3H-2,3-benzodiazépine-3-yl]éthanone (±)-8-acétamido-1-(4-chlorophényl)-N,4-diméthyl-4,5-dihydro-3H-2,3-benzodiazépine-3-carboxamide (±)-1-(4-bromophényl)-7-hydroxy-8-méthoxy-N,4-diméthyl-4,5-dihydro-3H-2,3-benzodiazépine-3-carboxamide (±)-1-(4-bromophényl)-7,8-dihydroxy-N,4-diméthyl-4,5-dihydro-3H-2,3-benzodiazépine-3-carboxamide (±)-1-(4-bromophényl)-7,8-bis(difluorométhoxy)-N,4-diméthyl-4,5-dihydro-3H-2,3-benzodiazépine-3-carboxamide (±)-1-(4-bromophényl)-7,8-diéthoxy-N,4-diméthyl-4,5-dihydro-3H-2,3-benzodiazépine-3-carboxamide (±)-7-bromo-1-(4-chlorophényl)-8-méthoxy-N,4-diméthyl-4,5-dihydro-3H-2,3-benzodiazépine-3-carboxamide (±)-1-(4-chlorophényl)-7-cyano-8-méthoxy-N,4-diméthyl-4,5-dihydro-3H-2,3-benzodiazépine-3-carboxamide (±)-1-(4-bromophényl)-7-(difluorométhoxy)-8-méthoxy-N,4-diméthyl-4,5-dihydro-3H-2,3-benzodiazépine-3-carboxamide (±)-1-(4-bromophényl)-4-éthyl-7,8-diméthoxy-N-méthyl-4,5-dihydro-3H-2,3-benzodiazépine-3-carboxamide (±)-1-(4-bromophényl)-4-isopropyl-7,8-diméthoxy-N-méthyl-4,5-dihydro-3H-2,3-benzodiazépine-3-carboxamide (±)-7-bromo-1-(4-chlorophényl)-N,4-diméthyl-4,5-dihydro-3H-2,3-benzodiazépine-3-carboxamide (±)-8-chloro-1-(4-chlorophényl)-N,4-diméthyl-7-(trifluorométhoxy)-4,5-dihydro-3H-2,3-benzodiazépine-3-carboxamide (4S)-8-chloro-1-(4-chlorophényl)-N,4-diméthyl-7-(trifluorométhoxy)-4,5-dihydro-3H-2,3-benzodiazépine-3-carboxamide (±)-1-(4-chlorophényl)-8-méthoxy-N,4-diméthyl-7-(trifluorométhoxy)-4,5-dihydro-3H-2,3-benzodiazépine-3-carboxamide (±)-1-[1-(4-bromophényl)-7,8-diméthoxy-4-méthyl-4,5-dihydro-3H-2,3-benzodiazépine-3-yl]éthanone (±)-1-(4-bromophényl)-N-éthyl-7,8-diméthoxy-4-méthyl-4,5-dihydro-3H-2,3-benzodiazépine-3-carboxamide.
